(19) Europäisches Patentamt
European Patent Office
Office européen des brevets

(11) EP 4 772 509 A1

(12) EUROPEAN PATENT APPLICATION
published in accordance with Art. 153(4) EPC

(43) Date of publication:
**08.07.2026 Bulletin 2026/28**

(21) Application number: **24870783.8**

(22) Date of filing: **25.09.2024**

(51) International Patent Classification (IPC):
***C07D 471/22*** (2006.01) ***C07D 471/04*** (2006.01)
***C07D 401/14*** (2006.01) ***C07D 413/14*** (2006.01)
***C07D 405/14*** (2006.01) ***A61K 31/497*** (2006.01)
***A61K 31/444*** (2006.01) ***A61P 35/00*** (2006.01)

(52) Cooperative Patent Classification (CPC):
**A61K 31/444; A61K 31/497; A61P 35/00; C07D 401/14; C07D 405/14; C07D 413/14; C07D 471/04; C07D 471/22**

(86) International application number:
**PCT/CN2024/121098**

(87) International publication number:
**WO 2025/067239 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority:
**26.09.2023 CN 202311254609**
**15.11.2023 CN 202311526635**
**29.12.2023 CN 202311870790**
**06.02.2024 CN 202410173580**
**28.03.2024 CN 202410371108**

(71) Applicant: **Shanghai Apeiron Therapeutics Company Limited**
**Shanghai 201210 (CN)**

(72) Inventors:
- **XIAO, Yisong**
  **Shanghai 201210 (CN)**
- **GU, Xiaohui**
  **Shanghai 201210 (CN)**
- **LAI, Kunmin**
  **Shanghai 201210 (CN)**
- **WANG, Minghua**
  **Shanghai 201210 (CN)**
- **HU, Min**
  **Shanghai 201210 (CN)**

(74) Representative: **Bayramoglu et al.**
**Mira Office**
**Kanuni Sultan Süleyman Boulevard 5387**
**Street Beytepe, floor 12, no:50**
**06800 Cankaya, Ankara (TR)**

# (54) KINESIN KIF18A INHIBITOR, AND APPLICATION THEREOF

(57) The present disclosure provides a KIF18 inhibitor and its synthetic method. Compounds of the present disclosure are capable of regulating the KIF18A protein, thereby influencing cell cycle and proliferation processes for the treatment of cancers and cancer-related diseases. The present disclosure also encompasses pharmaceutical compositions containing the compounds and methods for treating conditions associated with KIF18A activity.

EP 4 772 509 A1

## Description

### TECHNICAL FIELD

The present disclosure belongs to the field of medicine and relates to a class of kinesin KIF18A inhibitors and their use for inhibiting cancer cell proliferation and treating cancers.

### BACKGROUND ART

KIF18A is a member of the kinesin-8 family. It moves towards the plus ends of microtubules within cells using microtubules as tracks, relying on the energy released from ATP hydrolysis. Upon reaching the plus ends of microtubules, KIF I 8A regulates the dynamic instability of microtubules and exerts an activity similar to that of microtubule depolymerase. During mitosis, KIF18A regulates spindle microtubule dynamics and chromosome amplitude, playing a critical role in the timely completion of chromosome alignment at mitosis, maintenance of genomic stability and successful completion of mitosis.

The KIF18A gene belongs to the kinesin-8 subfamily and is a plus-end directed motor. KIF18A is believed to influence the dynamics of the plus ends of centromeric microtubules, thereby regulating correct chromosome orientation and spindle tension. Depletion of human KIF18A results in longer spindles, increased chromosome oscillation at metaphase and activated mitotic spindle assembly checkpoint in HeLa cervical cancer cells (MI Mayr et al., Current Biology 17, 488-98, 2007). KIF18A appears to be a viable target for cancer therapy. KIF18A is overexpressed in multiple types of cancers, including but not limited to colon, breast, lung, pancreatic, prostatic, bladder, head, neck, cervical and ovarian cancers. Furthermore, in cancer cell lines, gene deletion or knockout or KIF18A inhibition affects the mitotic spindle apparatus. In particular, inhibition of KIF18A has been found to induce mitotic cell arrest, a known vulnerability that promotes mitotic cell death through apoptosis, mitotic catastrophe, polyploidy-driven lethality, or death following mitotic slippage into interphase. As a result, there is a strong interest in finding inhibitors of KIF 18A protein. Therefore, inhibition of KIF18A ATPase activity is a promising approach to developing new anticancer agents.

### CONTENT OF INVENTION

The present disclosure belongs to the field of medicine and relates to a class of kinesin KIF18A inhibitors, and specifically relates to the compounds or their stereoisomers, tautomers, mesomers, racemates, enantiomers, diastereomers, or mixtures thereof, or pharmaceutically acceptable salts, co-crystals, metabolites, solvates, prodrugs, or isotopically labeled compounds thereof, the preparation methods thereof, pharmaceutical compositions containing such compounds, and their use as therapeutic agents, particularly for inhibiting cancer cell proliferation and treating cancer.

The present disclosure provides a novel class of compounds that regulate KIF18A protein, alone or in microtubule-bound complexes, for the treatment of KIF18A-mediated disorders and/or diseases, including cancer, inflammation or ciliopathy.

The compounds of the present disclosure exhibit MT-based regulatory activity on KIF18A, specifically inhibitory activity on KIF18A. To this end, the present disclosure also provides the use of these compounds and their pharmaceutically acceptable salts in the preparation and manufacture of pharmaceutical compositions or medicines for therapeutic, prophylactic, acute or chronic treatment of KIF18A-mediated diseases and disorders (including but not limited to cancers).

The present disclosure provides an Example:

A compound having the structure of Formula (I), or pharmaceutically acceptable salts, stereoisomers, isotope isomers or tautomers thereof:

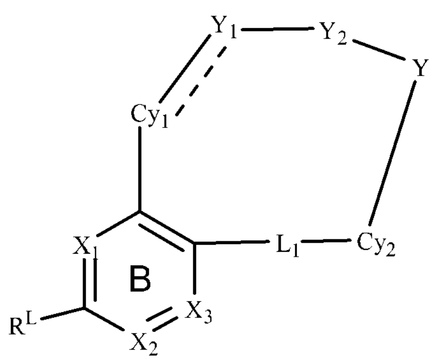

Formula (I)

wherein, $C_{y2}$ represents

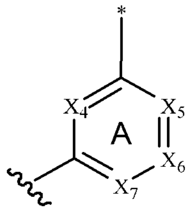 or 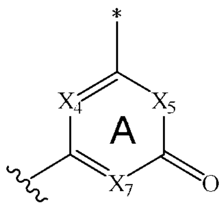 or 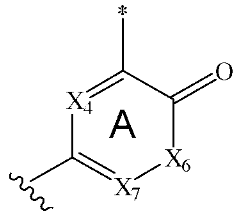 or 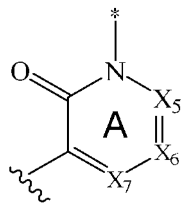

or

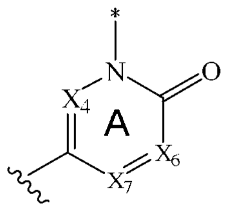

;

wherein, * represents the site of connection with Y, and the wavy line represents the site of connection with $L_1$;
wherein, - - - represents a single bond or double bond;
wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -$S(O)R^a$, -O-$C_1$-$C_{10}$ haloalkyl, -$SR^a$, -$C(O)OR^a$, -$C(O)R^a$, -$OC(O)R^a$, -$NR^aC(O)R^b$ -$C(O)NR^aR^b$, -$NR^aC(O)OR^b$, -$OC(O)NR^aR^b$, -$OC(O)OR^a$, -$SF_5$, -$PO(R^a R^b)$, -$P(R^a R^b)$ or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1, 2 or 3 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -$S(O)R^a$, -O-$C_1$-$C_{10}$ haloalkyl, -$SR^a$, -$C(O)OR^a$, -$C(O)R^a$, -$OC(O)R^a$, -$NR^aC(O)R^b$, -$C(O)NR^aR^b$, -$NR^aC(O)OR^b$, -$OC(O)NR^aR^b$, -$OC(O)OR^a$, -$SF_5$, -$PO(R^a R^b)$, -$P(R^a R^b)$ or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1, 2 or 3 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$,

$-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR^aC(O)OR^b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;

wherein, $L_1$ represents $-C(O)NR^a-$, $-NR^aC(O)-$ or a 5-6-membered heteroaromatic ring;

wherein, Y represents absence, $-CH_2-$, -O-, $-NR^a-$, $-C(O)NR^a$, $-NR^aC(O)-$ or the following groups:

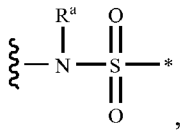 , 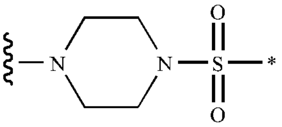

or $C_{y3}$;

wherein, * represents the site of connection with ring A; the wavy line represents the site of connection with $Y_2$;

wherein, $C_{y1}$ and $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein substituents are each independently selected from the following: hydrogen, deuterium, halogen, cyano, nitro, azido, hydroxy $(C_1-C_{10})$ alkyl, $C_1-C_{10}$ alkyl, $C_2-C_{10}$ alkenyl, $C_2-C_{10}$ alkynyl, $C_1-C_{10}$ deuterated alkyl, $C_3-C_{12}$ cycloalkyl, $C_1-C_{10}$ haloalkyl, $C_2-C_{10}$ haloalkenyl, $C_2-C_{10}$ haloalkynyl, $C_3-C_{12}$ halocycloalkyl, 3-12-membered heterocyclyl, $C_6-C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^a-C(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR^aC(O)OR^b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;

wherein, $Y_1$ represents $-(CR^aR^b)_m-$; $Y_2$ represents $-(CR^aR^b)_n-$;

wherein, optionally $-CR^aR^b-$ may be substituted with -O-, -S-, -Se-, $-NR^a-$, $-NR^aSO_2-$, $-NR^aC(=O)-$, $-C(=O)NR^a-$, -OC(=O)-, -C(=O)O-, $-SO_2NR^a-$, $-S(=O)(NR^a)-$, $-P(O)(OR^a)_2-$, $PO(R^a R^b)$, $-NR^aP(O)(OR^a)_2-$ or $-NR^aP(O)(R^a)_2-$, $-CR^a=CR^b-$,

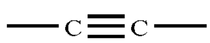 , 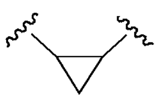 , 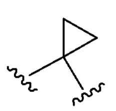 , 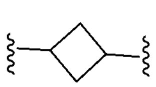 , 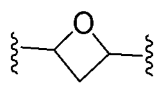 , 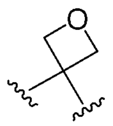 ,

5-6-membered heteroaryl,

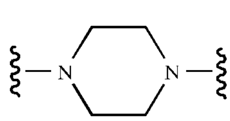 , 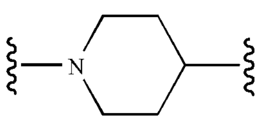 or 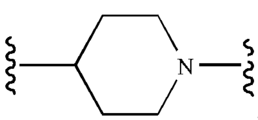 ;

wherein, $R^L$ represents $L_2-R^M$;

wherein, $L_2$ represents absence, $-C_1-C_6$ alkyl-, $-NR^a-$, $-NR^aSO_2-$, $-SO_2NR^a-$, $-NR^aS(=O)(=NH)-$, -S(=O) (=NH)-, -S-, -S(=O)-, $-SO_2-$, $-C_1-C_6$ alkyl-O-, -(C=O)-, $-(C=O)NR^a-$, -C=N(OH)-, $-NR^a$ (C=O), $-P(O)(OR^a)_2$, $-NR^aP(O)(OR^a)_2$ or $-NR^aP(O)(R^a)_2-$;

wherein, $R^M$ represents $C_1-C_6$ alkyl or $C_3-C_6$ cycloalkyl, wherein the $C_1-C_6$ alkyl or $C_3-C_6$ cycloalkyl can be optionally independently substituted with 0-3 substituents selected from halogen, $C_1-C_6$ alkyl, $-OR^a$, $-NR^aR^b$, cyano and $-O-C_1-C_6$ haloalkyl;

alternatively, $L_2-R^M$ represents

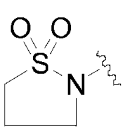 or 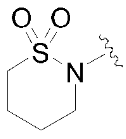 ;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, N and Se.

Furthermore, the present disclosure provides a compound having the structure of Formula (I-1), or pharmaceutically acceptable salts, stereoisomers, isotope isomers or tautomers thereof:

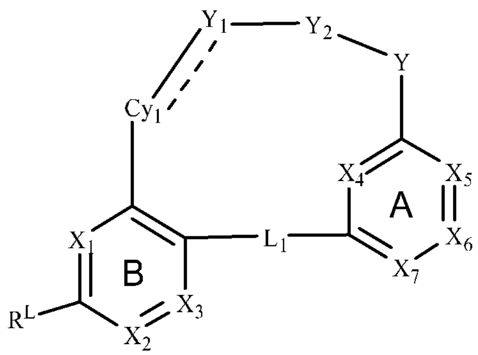

Formula (I-1)

wherein, $\overline{- - -}$ represents a single bond or double bond;

wherein, $X_1$ represents $CR^{W1}$ or N;

wherein, $X_2$ represents $CR^{W2}$ or N;

wherein, $X_3$ represents $CR^{W3}$ or N;

wherein, $X_4$ represents $CR^{W4}$ or N;

wherein, $X_5$ represents $CR^{W5}$ or N;

wherein, $X_6$ represents $CR^{W6}$ or N;

wherein, $X_7$ represents $CR^{W7}$ or N;

wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$ -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)$OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;

alternatively, the chemical bond between $X_2$ and $X_3$ may be fused with ring B to form a 4-8-membered saturated or unsaturated (heterocyclic) ring, which may optionally contain 0, 1, 2 or 3 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$, -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl

$NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;

alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1, 2 or 3 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -$S(O)R^a$, -O-$C_1$-$C_{10}$ haloalkyl, -$SR^a$, -$C(O)OR^a$, -$C(O)R^a$, -$OC(O)R^a$, -$NR^aC(O)R^b$, -$C(O)NR^aR^b$, -$NR^aC(O)OR^b$, -$OC(O)NR^aR^b$, -$OC(O)OR^a$, -$SF_5$, -$PO(R^a R^b)$, -$P(R^a R^b)$ or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^aR^b)$, -$C_0$-$C_{10}$ alkyl $P(R^a R^b)$ or -$C_0$-$C_{10}$ alkyl $NR^aR^b$;

wherein, $L_1$ represents -$C(O)NR^a$-, -$NR^aC(O)$- or a 5-6-membered heteroaromatic ring;

wherein, Y represents absence, -$CH_2$-, -O-, -$NR^a$-, -$C(O)NR^a$, -$NR^aC(O)$- or the following groups:

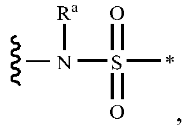 , 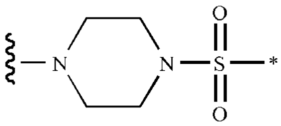

or $C_{y3}$;

wherein, * represents the site of connection with ring A; the wavy line represents the site of connection with $Y_2$;

wherein, $C_{y1}$ and $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein substituents are each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -$S(O)R^a$, -O-$C_1$-$C_{10}$ haloalkyl, -$SR^a$, -$C(O)OR^a$, -$C(O)R^a$, -$OC(O)R^a$, -$NR^aC(O)R^b$ -$C(O)NR^aR^b$, -$NR^a$-$C(O)OR^b$, -$OC(O)NR^aR^b$, -$OC(O)OR^a$, -$SF_5$, -$PO(R^a R^b)$, -$P(R^a R^b)$ or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;

wherein, $Y_1$ represents -$(CR^aR^b)_m$-; $Y_2$ represents -$(CR^aR^b)_n$-;

wherein, optionally -$CR^aR^b$- may be substituted with -O-, -S-, -Se-, -$NR^a$-, -$NR^aSO_2$-, -$NR^aC(=O)$-, -$C(=O)NR^a$-, -$OC(=O)$-, -$C(=O)O$-, -$SO_2NR^a$-, -$S(=O)(NR^a)$-, -$P(O)(OR^a)_2$-, -$NR^aP(O)(OR^a)_2$- or -$NR^aP(O)(R^a)_2$-, -$CR^a$=$CR^b$-,

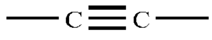 ,

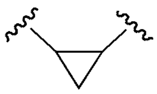 , 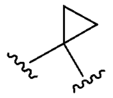 , 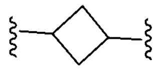 , 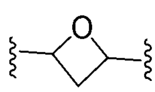 , 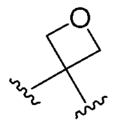 ,

5-6-membered heteroaryl,

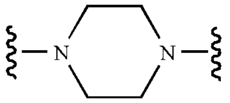 ,

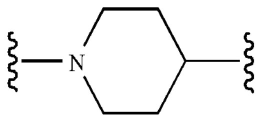, or 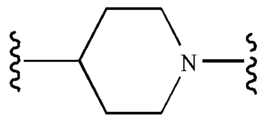;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^aS(=O)(=NH)$-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)_2$, -$NR^a$P(O)$(OR^a)_2$ or -$NR^a$P(O)$(R^a)_2$-;

wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;

alternatively, $L_2$-$R^M$ represents

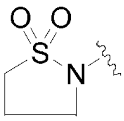 or 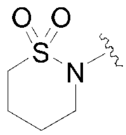;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, N and Se.

wherein, $X_1$ represents CH or N, preferably CH.

In a preferred Embodiment of Formula (I) or Formula (I-1), $X_2$ represents CH or N, preferably CH.

In a preferred Embodiment of Formula (I) or Formula (I-1), $X_3$ represents CH or N, preferably CH.

In a preferred Embodiment of Formula (I) or Formula (I-1), the chemical bond between $X_2$ and $X_3$ is fused with ring B to form a group having the following structure:

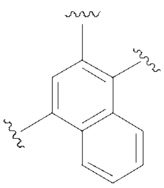 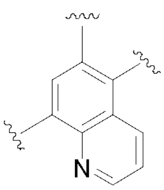 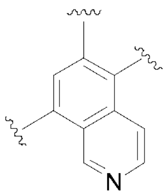 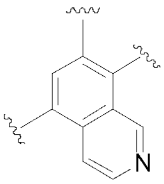 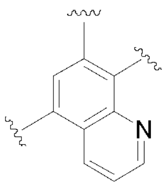 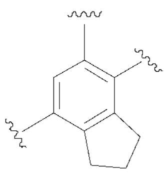

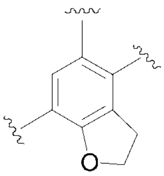 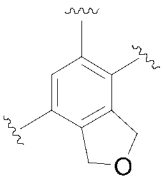 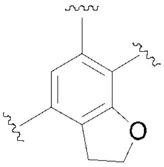 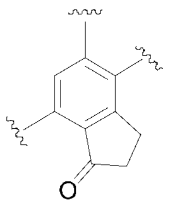 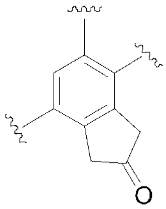 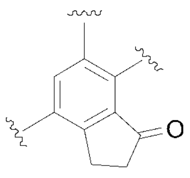

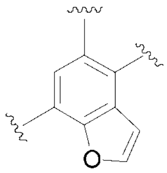 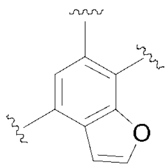 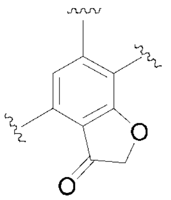 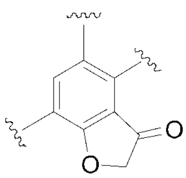

In a preferred Embodiment of Formula (I) or Formula (I-1), $X_4$ represents CH or N, preferably N.

In a preferred Embodiment of Formula (I) or Formula (I-1), $X_5$ represents $CR^{W5}$ or N, wherein $R^{W5}$ represents

hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

In a preferred Embodiment of Formula (I) or Formula (I-1), $X_6$ represents $CR^{W6}$ or N, wherein $R^{W6}$ represents hydrogen, halogen, $-CH_3O$, $-CF_3$, $-CHF_2$, $-CH_2F$, $-NH_2$, $-N(CH_3)_2$, -CN, -OH, $-C(O)CH_3$, $-OC(O)CH_3$, $-C(O)OCH_3$ or $-C(O)NH_2$.

In a preferred Embodiment of Formula (I) or Formula (I-1), $X_7$ represents CH or N, preferably N.

In a preferred Embodiment of Formula (I) or Formula (I-1), the chemical bond between $X_5$ and $X_6$ is fused with ring A to form a group having the following structure:

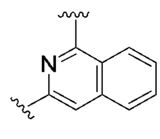 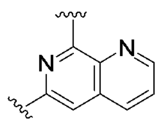 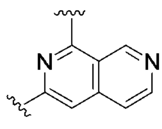 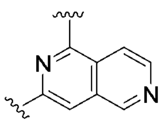 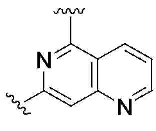 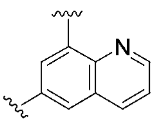

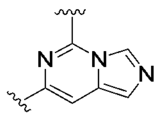 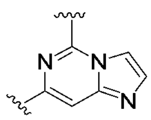 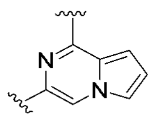 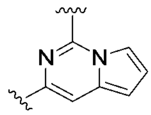 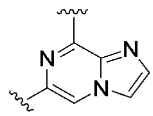 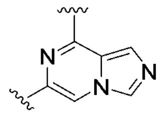 .

In a preferred Embodiment of Formula (I) or Formula (I-1), the chemical bond between $X_6$ and $X_7$ is fused with ring A to form a group having the following structure:

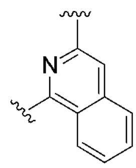 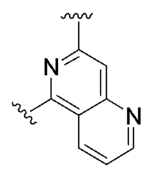 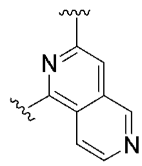 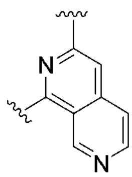 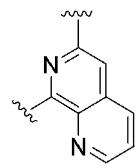 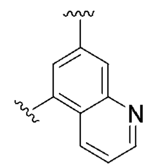

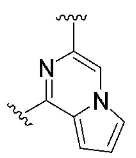 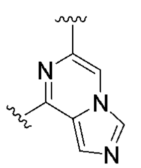 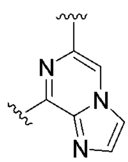 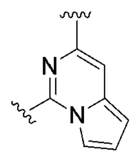 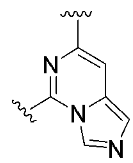 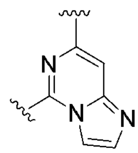 .

In a preferred Embodiment of Formula (I) or Formula (I-1), $C_{y1}$ represents the following groups:

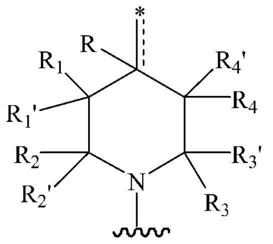 , 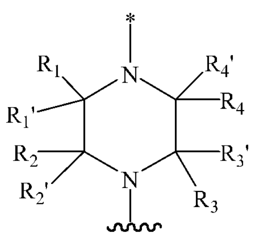 , 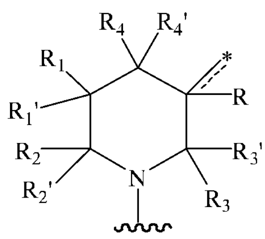 , 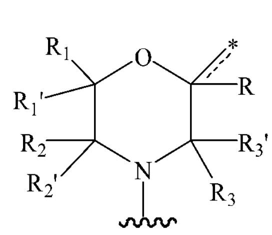 ,

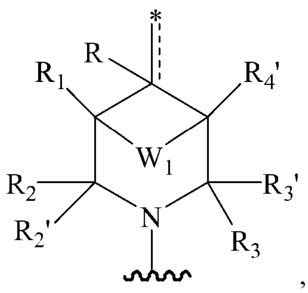 , 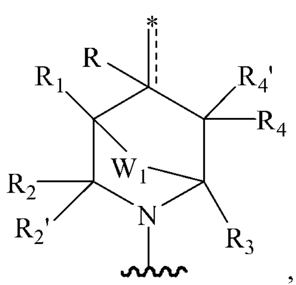 , 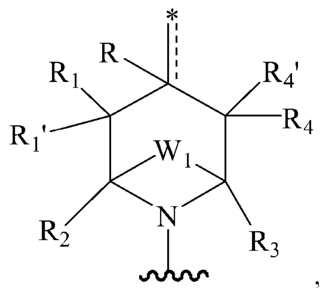 , 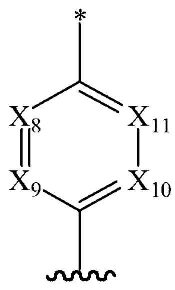 or 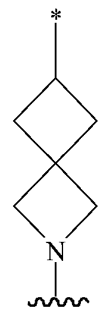

wherein, when $\overline{- - -}$ represents a double bond, R represents absence; wherein, when $\overline{- - -}$ represents a single bond, R represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;

wherein, $R_1$, $R_1'$, $R_2$, $R_2'$, $R_3$, $R_3'$, $R_4$ and $R_4'$ each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;

alternatively, pairs $R_1$ and $R_1'$, pairs $R_2$ and $R_2'$, pairs $R_3$ and $R_3'$ and pairs $R_4$ and $R_4'$, together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, -$OR^a$ or oxo;

wherein, $W_1$ represents -$(CR^sR^t)_o$-;

wherein, $R^s$ and $R^t$ each independently represent hydrogen or $C_1$-$C_6$ alkyl;

wherein, o represents 1 or 2;

wherein, $X_8$ represents $CR^{W8}$ or N;

wherein, $X_9$ represents $CR^{W9}$ or N;

wherein, $X_{10}$ represents $CR^{W10}$ or N;

wherein, $X_{11}$ represents $CR^{W11}$ or N;

wherein, $R^{W8}$, $R^{W9}$, $R^{W10}$ and $R^{W11}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$ -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR_a$C(O)$OR_b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$.

In a preferred Embodiment of Formula (I) or Formula (I-1), $X_8$ represents CH or N.

In a preferred Embodiment of Formula (I) or Formula (1-1), $X_9$ represents CH or N.

In a preferred Embodiment of Formula (I) or Formula (I-1), $X_{10}$ represents CH or N.

In a preferred Embodiment of Formula (I) or Formula (1-1), $X_{11}$ represents CH or N.

In a preferred Embodiment of Formula (I) or Formula (I-1), $R_1$, $R_1'$, $R_2$, $R_2'$, $R_3$, $R_3'$, $R_4$ and $R_4'$ each independently represent hydrogen.

In a preferred Embodiment of Formula (I) or Formula (I-1), $W_1$ represents -$CH_2$ or -$CH_2CH_2$.

In a preferred Embodiment of Formula (I) or Formula (I-1), Y represents absence, -$CH_2$-, -O-, -Se-, -$NR^a$-, -C(O)$NR^a$, -$NR^a$C(O)- or the following groups:

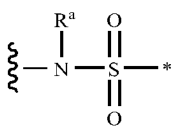
,
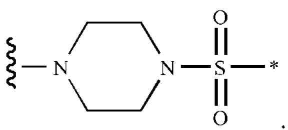
.

In a preferred Embodiment of Formula (I) or Formula (I-1), Y represents -O- or -NH-.

In a preferred Embodiment of Formula (I) or Formula (I-1), Y represents

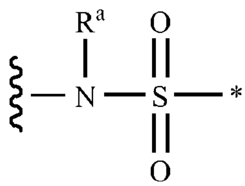
.

In a preferred Embodiment of Formula (I) or Formula (I-1), Y represents

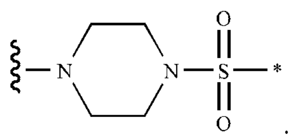
.

In a preferred Embodiment of Formula (I) or Formula (I-1), Y represents $C_{y3}$, wherein $C_{y3}$ represents the following groups:

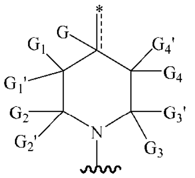 , 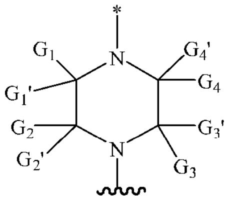 , 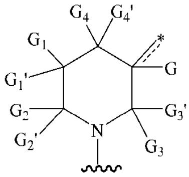 , 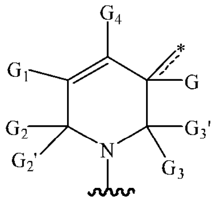 , 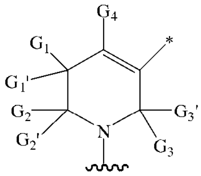 ,

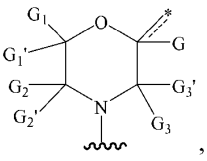 , 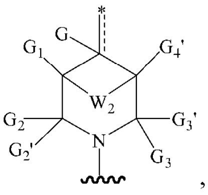 , 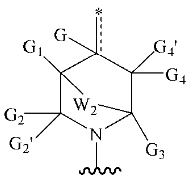 , 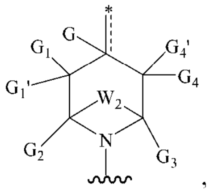 , 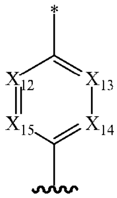 , 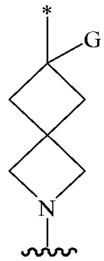 , 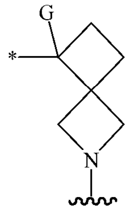 ,

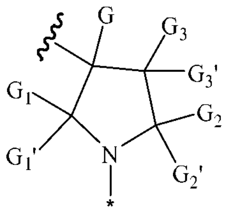 , 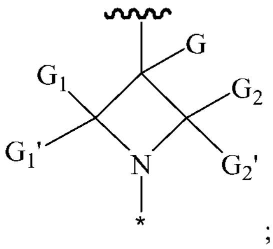 ;

wherein, when --- represents a double bond, G represents absence; wherein, when --- represents a single bond, G represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;

wherein, $G_1$, $G_1$', $G_2$, $G_2$', $G_3$, $G_3$', $G_4$ and $G_4$' each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;

alternatively, pairs $G_1$ and $G_1$', pairs $G_2$ and $G_2$', pairs $G_3$ and $G_3$' and pairs $G_4$ and $G_4$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $OR^a$ or oxo;

wherein, $W_2$ represents -$(CR^iR^j)_p$-;

wherein, $R^i$ and $R^j$ each independently represent hydrogen or $C_1$-$C_6$ alkyl;

wherein, p represents 1 or 2;

wherein, $X_{12}$ represents $CR^{W12}$ or N;

wherein, $X_{13}$ represents $CR^{W13}$ or N;

wherein, $X_{14}$ represents $CR^{W14}$ or N;

wherein, $X_{15}$ represents $CR^{W15}$ or N;

wherein, $R^{W12}$, RW$^{13}$, $R^{W14}$ and $R^{W15}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$ -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR_a$C(O)$OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;

In a preferred Embodiment of Formula (I) or Formula (I-1), $X_{12}$ represents CH or N.

In a preferred Embodiment of Formula (I) or Formula (I-1), $X_{13}$ represents CH or N.

In a preferred Embodiment of Formula (I) or Formula (I-1), $X_{14}$ represents CH or N.

In a preferred Embodiment of Formula (I) or Formula (I-1), $X_{15}$ represents CH or N.

In a preferred Embodiment of Formula (I) or Formula (I-1), $G_1$, $G_1'$, $G_2$, $G_2'$, $G_3$, $G_3'$, $G_4$ and $G_4'$ each independently represent hydrogen.

In a preferred Embodiment of Formula (I) or Formula (I-1), $W_2$ represents $-CH_2$ or $-CH_2CH_2$.

In a preferred Embodiment of Formula (I) or Formula (I-1),

$L_1$ represents $-C(O)NR^a-$, $-NR^aC(O)-$,

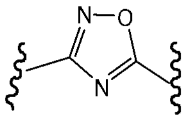, 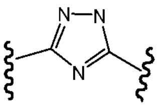, 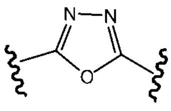, 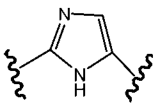, 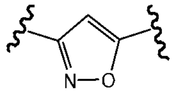,

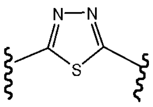, 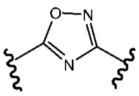, 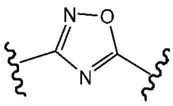, 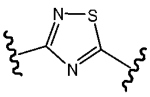, 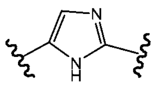, 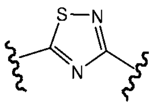, 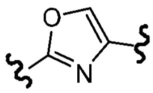,

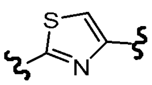, 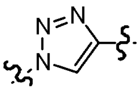, 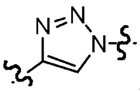, 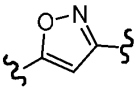, 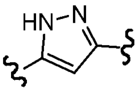, 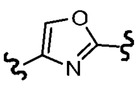, 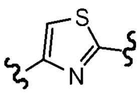, 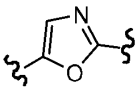,

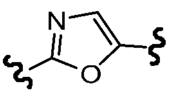, 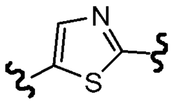 or 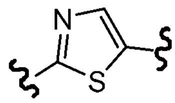.

In a preferred Embodiment of Formula (I) or Formula (I-1), $L_1$ represents -C(O)NH-.

In a preferred Embodiment of Formula (I) or Formula (I-1), $L_1$ represents

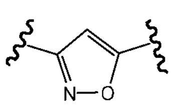, 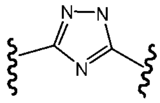,

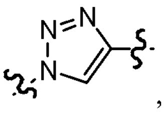, 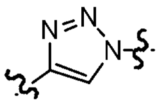, 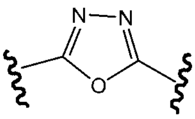 or 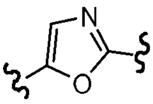.

In a preferred Embodiment of Formula (I) or Formula (I-1), $Y_1$ represents $-(CR^aR^b)_m-$.

In a preferred Embodiment of Formula (I) or Formula (I-1), $Y_1$ represents -NR-C(=O)-, -O-C(=O)- or -C(=O)O-.

In a preferred Embodiment of Formula (I) or Formula (I-1), $Y_1$ represents $-O(CR^aR^b)_m-$ or $-(CR^aR^b)_mO-$.

In a preferred Embodiment of Formula (I) or Formula (I-1), $Y_1$ represents $-CR^a=CR^a-$ or $-CR^aR^b-CR^a=CR^b-$.

In a preferred Embodiment of Formula (I) or Formula (I-1), $Y_2$ represents $-(CR^aR^b)_n-$.

In a preferred Embodiment of Formula (I) or Formula (1-1), $Y_2$ represents $-NR^a-C(=O)-$.

In a preferred Embodiment of Formula (I) or Formula (1-1), $Y_2$ represents $-O(CR^aR^b)_n-$ or $-(CR^aR^b)_nO-$.

In a preferred Embodiment of Formula (I) or Formula (I-1), $Y_2$ represents $-CR^a=CR^a-$, $-CR^a=CR^b-CR^aR^b-$ or $-CR^aR^b-CR^a=CR^b-$.

In a preferred Embodiment of Formula (I) or Formula (I-1), $Y_2$ represents pyrazolyl, oxazolyl, oxadiazolyl, thiazolyl, thiadiazolyl or triazolyl.

In a preferred Embodiment of Formula (I) or Formula (I-1), $-Y_1-Y_2-$ represents $-CR^a=CR^a-$ or $-CR^aR^bCR^a=CR^b-$.

In a preferred Embodiment of Formula (I) or Formula (I-1), $-Y_1-Y_2-$ represents $-CR^aR^b-NR^aC(O)-$.

In a preferred Embodiment of Formula (I) or Formula (I-1), $-Y_1-Y_2-$ represents -

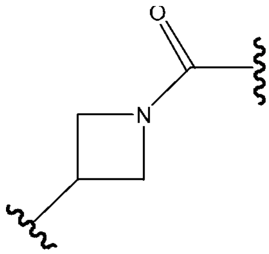.

In a preferred Embodiment of Formula (I) or Formula (I-1), $R^L$ represents $-NHS(O)_2CH_2CH_2OH$,

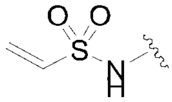,

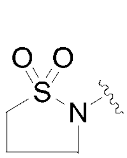, 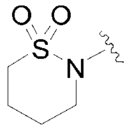, 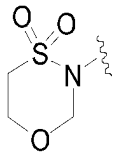,

$-NHS(O)_2CH_2CH_3$ or $-NHS(O)_2CH_3$.

Furthermore, the present disclosure provides a compound having the structure of Formula (II) or Formula (III), or pharmaceutically acceptable salts, stereoisomers, isotope isomers or tautomers thereof,

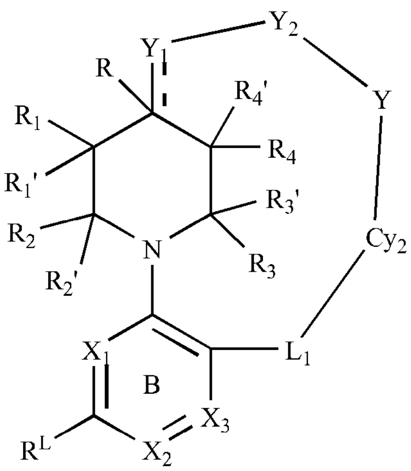

Formula(II)

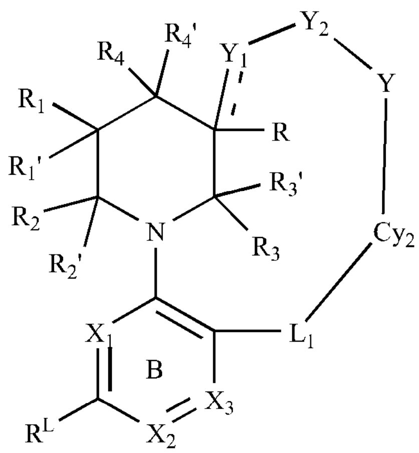

Formula(III)

**wherein,** $C_{y2}$ represents

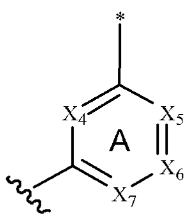 or 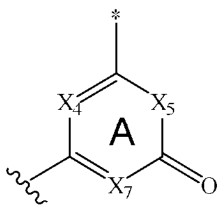 or 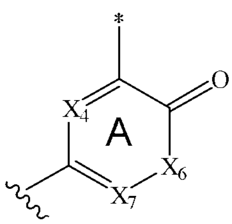 or 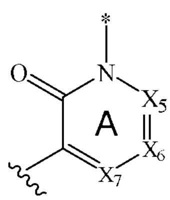

or

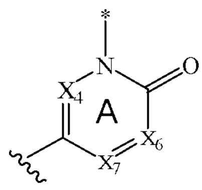

;

wherein, * represents the site of connection with Y, and the wavy line represents the site of connection with $L_1$;
wherein, - - - represents a single bond or double bond;
wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W1}$, $R^{W3}$, $R^{W4}$, $R^{W4}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, -SFs, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents selected from the following: deuterium, halogen, cyano, oxo, nitro, oxo, hydroxy ($C_1$-$C_6$) alkyl, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, $-OR^a$, $-SO_3R^a$, $-S(O)R^a$, -O-$C_1$-$C_6$ haloalkyl, $-SR^a$, $-C(=O)OR^a$, $-C(=O)R^a$, $-C(=O)NR^aR^b$, $-SF_5$ or $-NR^aR^b$;
wherein, $L_1$ represents $-C(O)NR^a-$, $-NR^aC(O)-$ or a 5-6-membered heteroaromatic ring;
wherein, Y represents absence, $-CH_2-$, -O-, $-NR^a-$, $-C(O)NR^a$, $-NR^aC(O)-$ or the following groups:

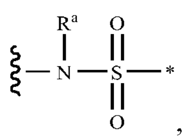

,

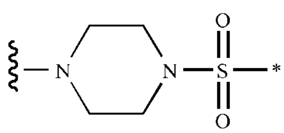

or $C_{y3}$;
wherein, * represents the site of connection with ring A; the wavy line represents the site of connection with $Y_2$;
wherein, when - - - represents a double bond, R represents absence; wherein, when - - - represents a single bond, R represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or $-C(O)CH_3$;

wherein, $R_1$, $R_1$', $R_2$, $R_2$', $R_3$, $R_3$', $R_4$ and $R_4$' each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;

alternatively, pairs $R_1$ and $R_1$', pairs $R_2$ and $R_2$', pairs $R_3$ and $R_3$' and pairs $R_4$ and $R_4$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, -$OR^a$ or oxo;

wherein, $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein the substituents are each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^aC(O)R^b$, -C(O)$NR^aR^b$, -$NR^aC(O)OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^aC(O)R^b$, -$C_0$-$C_{10}$ alkyl C(O)$NR^aR^b$, -$C_0$-$C_{10}$ alkyl $NR^aC(O)OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;

wherein, $Y_1$ represents -$(CR^aR^b)_m$-; $Y_2$ represents -$(CR^aR^b)_n$-;

wherein, optionally -$CR^aR^b$- may be substituted with -O-, -S-, -Se-, -$NR^a$-, -$NR^aSO_2$-, -$NR^aC(=O)$-, -C(=O)$NR^a$-, -OC(=O)-, -C(=O)O-, -$SO_2NR^a$-, -S(=O)($NR^a$)-, -P(O)$(OR^a)_2$-, PO($R^a$ $R^b$), -$NR^aP(O)(OR^a)_2$- or -$NR^aP(O)(R^a)_2$-, -$CR^a=CR^b$-,

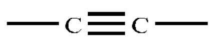, 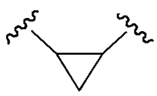, 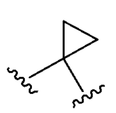, 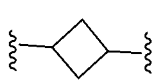, 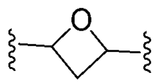, 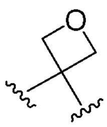,

5-6-membered heteroaryl,

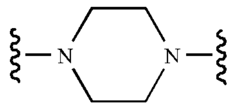, 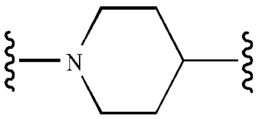 or 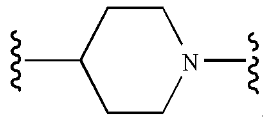;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^aS(=O)(=NH)$-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)_2$, -$NR^aP(O)(OR^a)_2$ or -$NR^aP(O)(R^a)_2$-;

wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;

alternatively, $L_2$-$R^M$ represents

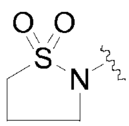 or 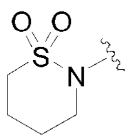;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or

unsaturated heterocyclic ring, which may optionally contain 0-2 heteroatoms selected from O, S, Se and N.

Furthermore, the present disclosure provides a compound having the structure of Formula (II-1)/Formula (III-1), or pharmaceutically acceptable salts, stereoisomers, isotope isomers or tautomers thereof:

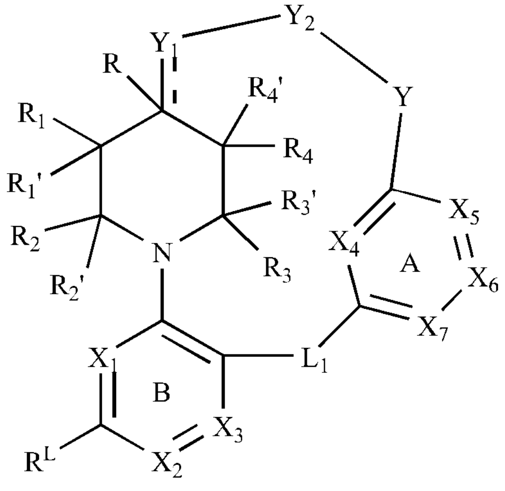

Formula (II-1)

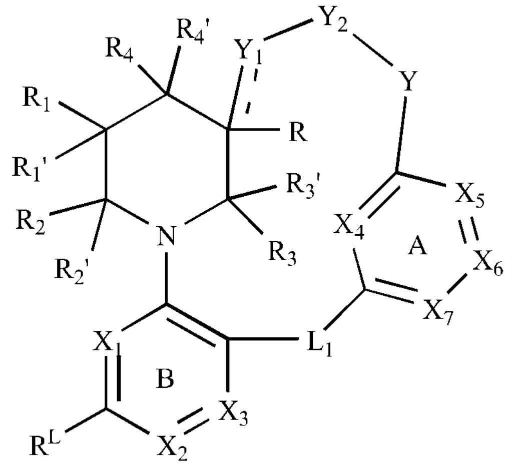

Formula (III-1)

wherein, $\overline{---}$ represents a single bond or double bond;
wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W1}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{19}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents selected from the following:

deuterium, halogen, cyano, nitro, oxo, hydroxy ($C_1$-$C_6$) alkyl, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, $-OR^a$, $-SO_3R^a$, $-S(O)R^a$, $-O-C_1$-$C_6$ haloalkyl, $-SR^a$, $-C(=O)OR^a$, $-C(=O)R^a$, $-C(=O)NR^aR^b$, $-SF_5$ or $-NR^aR^b$;
wherein, $L_1$ represents $-C(O)NR^a$-, $-NR^aC(O)$- or a 5-6-membered heteroaromatic ring;
wherein, Y represents absence, $-CH_2$-, -O-, $-NR^a$-, $-C(O)NR^a$, $-NR^aC(O)$- or the following groups:

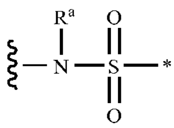 , 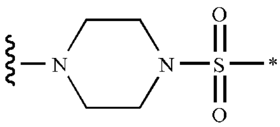

or $C_{y3}$;
wherein, * represents the site of connection with ring A; the wavy line represents the site of connection with $Y_2$;
wherein, when --- represents a double bond, R represents absence; wherein, when --- represents a single bond, R represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or $-C(O)CH_3$;
wherein, $R_1$, $R_1$', $R_2$, $R_2$', $R_3$, $R_3$', $R_4$ and $R_4$' each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;
alternatively, pairs $R_1$ and $R_1$', pairs $R_2$ and $R_2$', pairs $R_3$ and $R_3$' and pairs $R_4$ and $R_4$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $-OR^a$ or oxo;
wherein, $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein the substituents are each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
wherein, $Y_1$ represents $-(CR^aR^b)_m$-; $Y_2$ represents $-(CR^aR^b)_n$-;
wherein, optionally $-CR^aR^b$- may be substituted with -O-, -S-, -Se-, $-NR^a$-, $-NR^aSO_2$-, $-NR^aC(=O)$-, $-C(=O)NR^a$-, -OC(=O)-, -C(=O)O-, $-SO_2NR^a$-, $-S(=O)(NR^a)$-, $-P(O)(OR^a)_2$-, $PO(R^a R^b)$, $-NR^aP(O)(OR^a)_2$- or $-NR^aP(O)(R^a)_2$-, $-CR^a=CR^b$-,

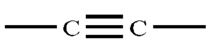 , 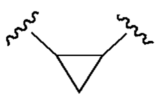 , 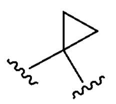 , 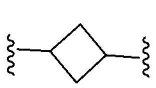 , 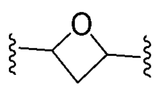 , 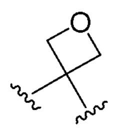 ,

5-6-membered heteroaryl,

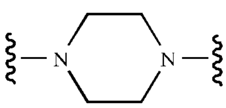 , 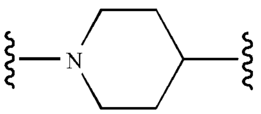 or 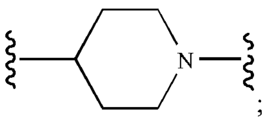 ;

wherein, $R^L$ represents $L_2$-$R^M$;
wherein, $L_2$ represents absence, $-C_1$-$C_6$ alkyl-, $-NR^a$-, $-NR^aSO_2$-, $-SO_2NR^a$-, $-NR^aS(=O)(=NH)$-, -S(=O) (=NH)-, -S-, -S(=O)-, $-SO_2$-, $-C_1$-$C_6$ alkyl-O-, -(C=O)-, $-(C=O)NR^a$-, -C=N(OH)-, $-NR^a$ (C=O), $-P(O)(OR^a)_2$, $-NR^aP(O)(OR^a)_2$ or

$-NR^aP(O)(R^a)_2-$;
wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, $-OR^a$, $-NR^aR^b$, cyano and -O-$C_1$-$C_6$ haloalkyl;
alternatively, $L_2$-$R^M$ represents

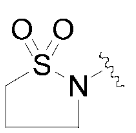 or 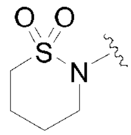 ;

wherein, m and n each independently represent an integer from 0 to 10;
wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, and N.

In a preferred Embodiment of Formula (II) or Formula (II-1) or Formula (III) or Formula (III-1), $X_1$ represents CH or N, preferably CH.

In a preferred Embodiment of Formula (II) or Formula (II-1) or Formula (III) or Formula (III-1), $X_2$ represents CH or N, preferably CH.

In a preferred Embodiment of Formula (II) or Formula (II-1) or Formula (III) or Formula (III-1), $X_3$ represents CH or N, preferably CH.

In a preferred Embodiment of Formula (II) or Formula (II-1) or Formula (III) or Formula (III-1), the chemical bond between $X_2$ and $X_3$ is fused with ring B to form a group having the following structure:

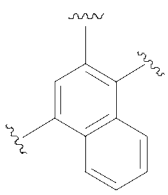 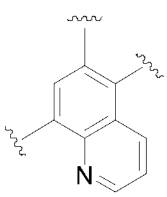 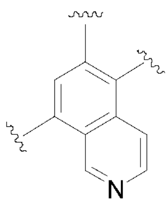 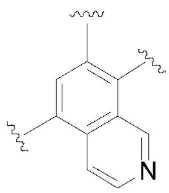 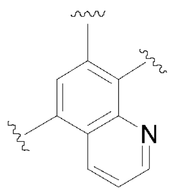 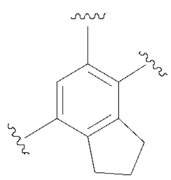

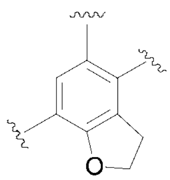 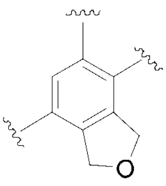 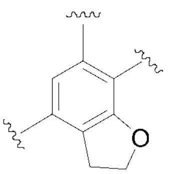 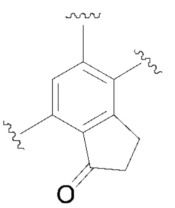 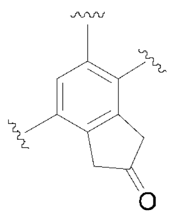 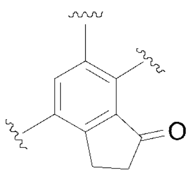

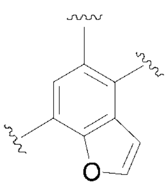 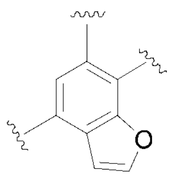 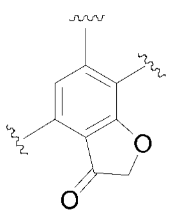 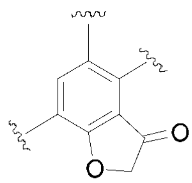

In a preferred Embodiment of Formula (II) or Formula (II-1) or Formula (III) or Formula (III-1), $X_4$ represents CH or N, preferably N.

In a preferred Embodiment of Formula (II) or Formula (II-1) or Formula (III) or Formula (III-1), $X_5$ represents $CR^{W5}$ or N, wherein $R^{W5}$ represents hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

In a preferred Embodiment of Formula (II) or Formula (II-1) or Formula (III) or Formula (III-1), $X_6$ represents $CR^{W6}$ or N, wherein $R^{W6}$ represents hydrogen, halogen, $-CH_3O$, $-CF_3$, $-CHF_2$, $-CH_2F$, $-NH_2$, $-N(CH_3)_2$, -CN, -OH, $-C(O)CH_3$,

-$OC(O)CH_3$, -$C(O)OCH_3$ or -$C(O)NH_2$.

In a preferred Embodiment of Formula (II) or Formula (II-1) or Formula (III) or Formula (III-1), $X_7$ represents CH or N, preferably N.

In a preferred Embodiment of Formula (II) or Formula (II-1) or Formula (III) or Formula (III-1), the chemical bond between $X_5$ and $X_6$ is fused with ring A to form a group having the following structure:

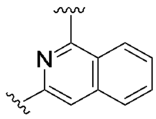 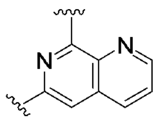 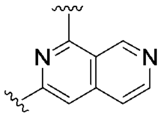 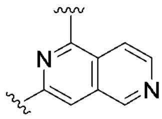 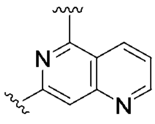 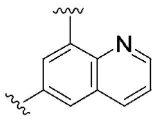

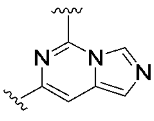 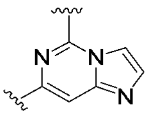 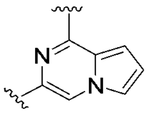 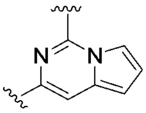 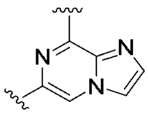 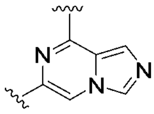 .

In a preferred Embodiment of Formula (II) or Formula (II-1) or Formula (III) or Formula (III-1), the chemical bond between $X_6$ and $X_7$ is fused with ring A to form a group having the following structure:

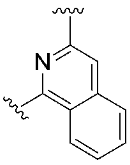 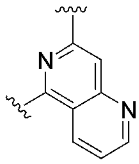 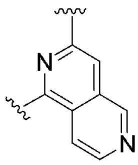 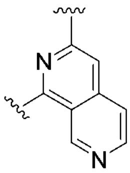 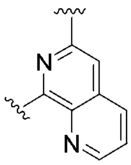 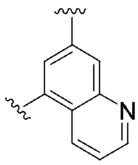

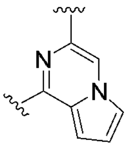 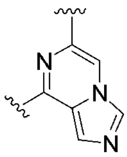 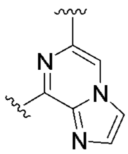 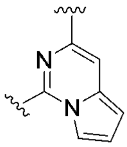 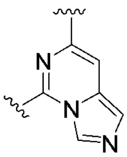 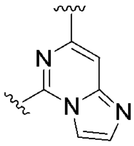 .

In a preferred Embodiment of Formula (II) or Formula (II-1) or Formula (III) or Formula (III-1), Y represents absence, -$CH_2$-, -O-, -Se-, -$NR^a$-, -$C(O)NR^a$, -$NR^aC(O)$- or the following groups:

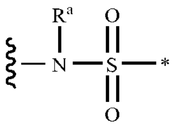 , 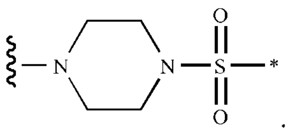 .

In a preferred Embodiment of Formula (II) or Formula (II-1) or Formula (III) or Formula (III-1), Y represents -O-, -Se- or -NH-.

In a preferred Embodiment of Formula (II) or Formula (II-1) or Formula (III) or Formula (III-1), Y represents

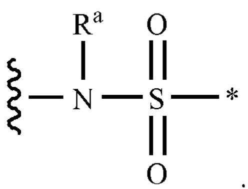 .

In a preferred Embodiment of Formula (II) or Formula (II-1) or Formula (III) or Formula (III-1), Y represents

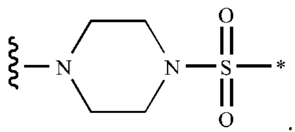.

In a preferred Embodiment of Formula (II) or Formula (II-1) or Formula (III) or Formula (III-1), Y represents $C_{y3}$, wherein $C_{y3}$ represents the following groups:

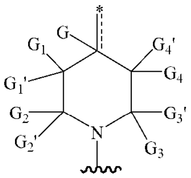, 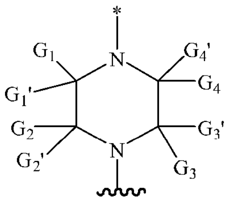, 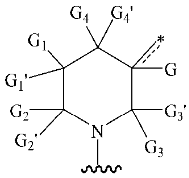, 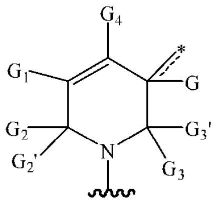, 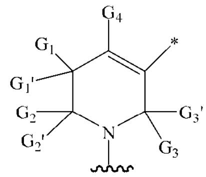,

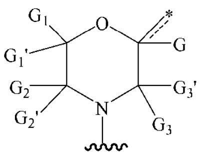, 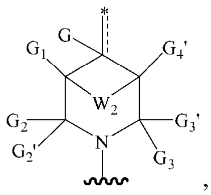, 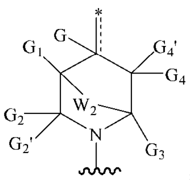, 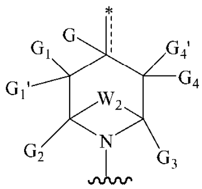, 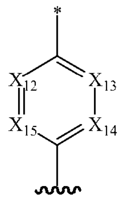, 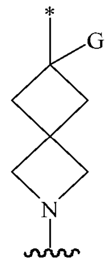, 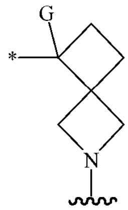,

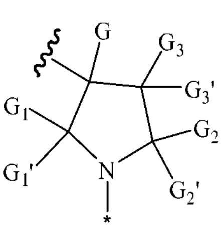, 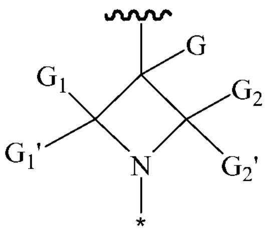;

wherein, when - - - represents a double bond, G represents absence; wherein, when - - - represents a single bond, G represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;

wherein, $G_1$, $G_1$', $G_2$, $G_2$', $G_3$, $G_3$', $G_4$ and $G_4$' each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;

alternatively, pairs $G_1$ and $G_1$', pairs $G_2$ and $G_2$', pairs $G_3$ and $G_3$' and pairs $G_4$ and $G_4$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $OR^a$ or oxo;

wherein, $W_2$ represents $-(CR^iR^j)_p-$;

wherein, $R^i$ and $R^j$ each independently represent hydrogen or $C_1$-$C_6$ alkyl;

wherein, p represents 1 or 2;

wherein, $X_{12}$ represents $CR^{W12}$ or N;

wherein, $X_{13}$ represents $CR^{W13}$ or N;

wherein, $X_{14}$ represents $CR^{W14}$ or N;

wherein, $X_{15}$ represents $CR^{W15}$ or N;

wherein, $R^{W12}$, $R^{W13}$, $R^{W14}$ and $R^{W15}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$,

$C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;

In a preferred Embodiment of Formula (II) or Formula (II-1) or Formula (III) or Formula (III-1), $X_{12}$ represents CH or N.

In a preferred Embodiment of Formula (II) or Formula (II-1) or Formula (III) or Formula (III-1), $X_{13}$ represents CH or N.

In a preferred Embodiment of Formula (II) or Formula (II-1) or Formula (III) or Formula (III-1), $X_{14}$ represents CH or N.

In a preferred Embodiment of Formula (II) or Formula (II-1) or Formula (III) or Formula (III-1), $X_{15}$ represents CH or N.

In a preferred Embodiment of Formula (II) or Formula (II-1) or Formula (III) or Formula (III-1), $G_1$, $G_1$', $G_2$, $G_2$', $G_3$, $G_3$', $G_4$ and $G_4$' each independently represent hydrogen.

In a preferred Embodiment of Formula (II) or Formula (II-1) or Formula (III) or Formula (III-1), $W_2$ represents -$CH_2$ or -$CH_2CH_2$.

In a preferred Embodiment of Formula (II) or Formula (II-1) or Formula (III) or Formula (III-1), $L_1$ represents -$C(O)NR^a$-, -$NR^aC(O)$-,

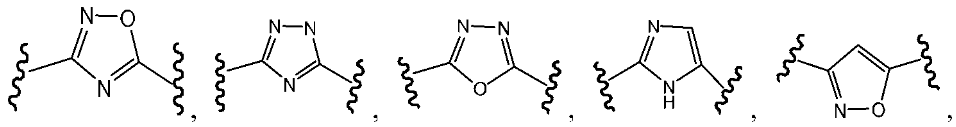
, , , , ,

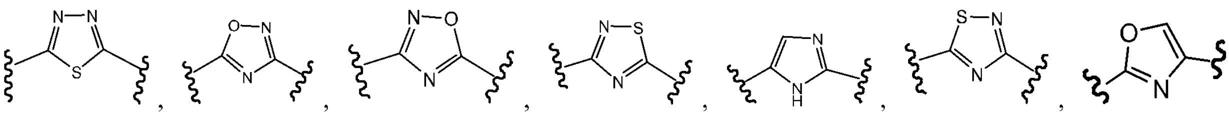
, , , , , , ,

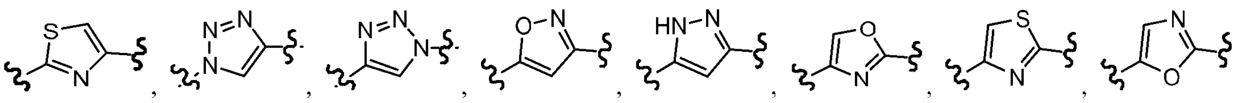
, , , , , , , ,

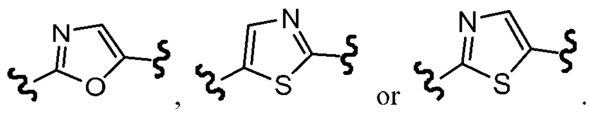
, or .

In a preferred Embodiment of Formula (II) or Formula (II-1) or Formula (III) or Formula (III-1), $L_1$ represents -C(O)NH-.

In a preferred Embodiment of Formula (II) or Formula (II-1) or Formula (III) or Formula (III-1), $L_1$ represents

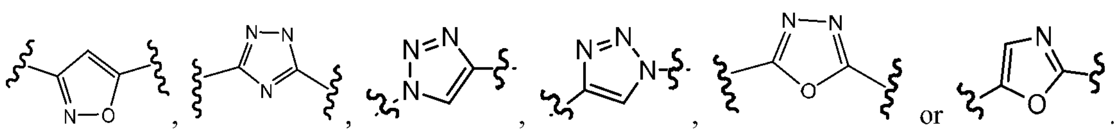
, , , , or .

In a preferred Embodiment of Formula (II) or Formula (II-1) or Formula (III) or Formula (III-1), $Y_1$ represents -$(CR^aR^b)_m$-.

In a preferred Embodiment of Formula (II) or Formula (II-1) or Formula (III) or Formula (III-1), $Y_1$ represents -NR-C(=O)-, -O-C(=O)- or -C(=O)O-.

In a preferred Embodiment of Formula (II) or Formula (II-1) or Formula (III) or Formula (III-1), $Y_1$ represents -$O(CR^aR^b)_m$- or -$(CR^aR^b)_m$O-.

In a preferred Embodiment of Formula (II) or Formula (II-1) or Formula (III) or Formula (III-1), $Y_1$ represents -$CR^a$=$CR^a$- or -$CR^aR^b$-$CR^a$=$CR^b$-.

In a preferred Embodiment of Formula (II) or Formula (II-1) or Formula (III) or Formula (III-1), $Y_2$ represents -$(CR^aR^b)_n$-.

In a preferred Embodiment of Formula (II) or Formula (II-1) or Formula (III) or Formula (III-l), $Y_2$ represents

$-NR^a-C(=O)-$.

In a preferred Embodiment of Formula (II) or Formula (II-1) or Formula (III) or Formula (III-1), $Y_2$ represents $-O(CR^aR^b)_n-$ or $-(CR^aR^b)_nO-$.

In a preferred Embodiment of Formula (II) or Formula (II-1) or Formula (III) or Formula (III-1), $Y_2$ represents $-CR^a=CR^a-$, $-CR^a=CR^b-CR^aR^b-$ or $-CR^aR^b-CR^a=CR^b-$.

In a preferred Embodiment of Formula (II) or Formula (II-1) or Formula (III) or Formula (III-1), $Y^2$ represents pyrazolyl, oxazolyl, oxadiazolyl, thiazolyl, thiadiazolyl or triazolyl.

In a preferred Embodiment of Formula (II) or Formula (II-1) or Formula (III) or Formula (III-1), $-Y_1-Y_2-$ represents $-CR^a=CR^a-$ or $-CR^aR^bCR^a=CR^b-$.

In a preferred Embodiment of Formula (II) or Formula (II-1) or Formula (III) or Formula (III-1), $-Y_1-Y_2-$ represents $-CR^aR^b-NR^aC(O)-$.

In a preferred Embodiment of Formula (II) or Formula (11-1) or Formula (III) or Formula (III-1), $-Y_1-Y_2-$ represents

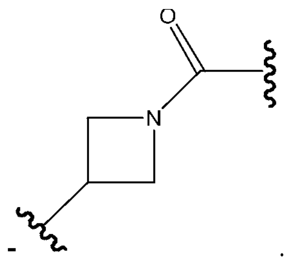

.

In a preferred Embodiment of Formula (II) or Formula (II-1) or Formula (III) or Formula (III-1), $R^L$ represents $-NHS(O)_2CH_2CH_2OH$,

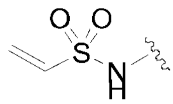 , 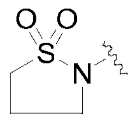 , 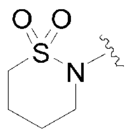 , 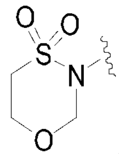 ,

$-NHS(O)_2CH_2CH_3$ or $-NHS(O)_2CH_3$.

Furthermore, the present disclosure provides a compound having the structure of Formula (I-3), or pharmaceutically acceptable salts, stereoisomers, isotope isomers or tautomers thereof,

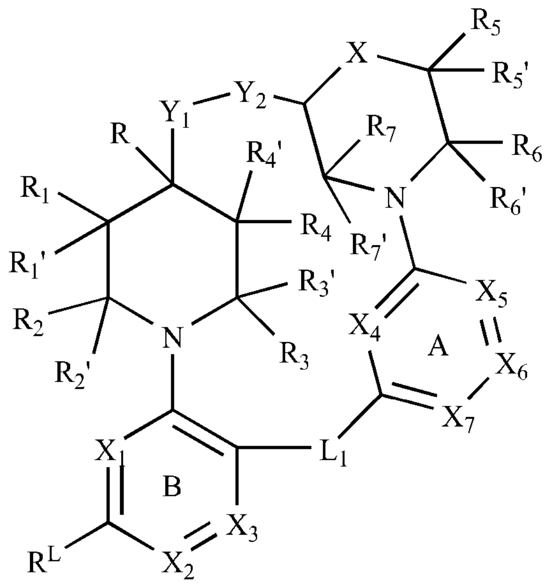

Formula (I-3)

wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$ -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)$OR^b$, -$C_0$-$C_{10}$ alkyl OC(O) $NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, oxo, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$, -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)$OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^aR^b$), -$C_0$-$C_{10}$ alkyl P($R^a$ $R^b$) or -$C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$, -C(O)$NR^aR^b$, -$NR^a$-C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$ $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)$OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;
wherein, $L_1$ represents -C(O)$NR^a$-, -$NR^a$C(O)- or a 5-6-membered heteroaromatic ring;
wherein, R represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;
wherein, X represents $CR_9R_9'$, O or $NR^a$;
wherein, $R_1$, $R_1'$, $R_2$, $R_2'$, $R_3$, $R_3'$, $R_4$, $R_4'$, $R_5$, $R_5'$, $R_6$, $R_6'$, $R_7$, $R_7'$, $R_9$ and $R_9'$ each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;
alternatively, pairs $R_1$ and $R_1'$, pairs $R_2$ and $R_2'$, pairs $R_3$ and $R_3'$, pairs $R_4$ and $R_4'$, pairs $R_5$ and $R_5'$, pairs $R_6$ and $R_6'$, pairs $R_7$ and $R_7'$ and pairs $R_9$ and $R_9'$, together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$haloalkyl, -$OR^a$ or oxo;
wherein, $Y_1$ represents -$(CR^aR^b)_m$-; $Y_2$ represents -$(CR^aR^b)_n$-;
wherein, optionally -$CR^aR^b$- may be substituted with -O-, -S-, -Se-, -$NR^a$-, -$NR^aSO_2$-, -$NR^a$C(=O)-, -C(=O)$NR^a$-, -OC(=O)-, -C(=O)O-, -$SO_2NR^a$-, -S(=O)($NR^a$)-, -P(O)$(OR^a)_2$-, PO($R^a$ $R^b$), -$NR^a$P(O)$(OR^a)_2$- or -$NR^a$P(O)$(R^a)_2$-, -$CR^a$=$CR^b$-,

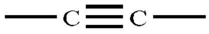, 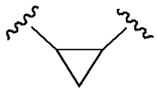, 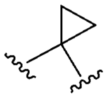, 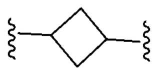, 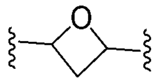, 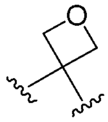,

5-6-membered heteroaryl,

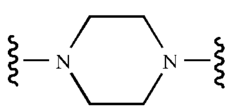, 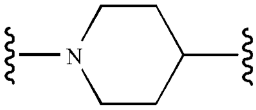 or 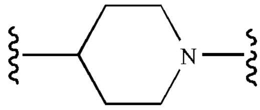;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^aS(=O)(=NH)$-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -$P(O)(OR^a)_2$, -$NR^aP(O)(OR^a)_2$ or -$NR^aP(O)(R^a)_2$-;

wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;

alternatively, $L_2$-$R^M$ represents

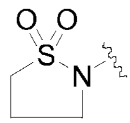 or 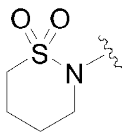;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, Se and N.

In a preferred Embodiment of Formula (1-3), $X_1$ represents CH or N, preferably CH.

In a preferred Embodiment of Formula (1-3), $X_2$ represents CH or N, preferably CH.

In a preferred Embodiment of Formula (1-3), $X_3$ represents CH or N, preferably CH.

In a preferred Embodiment of Formula (I-3), the chemical bond between $X_2$ and $X_3$ is fused with ring B to form a group having the following structure:

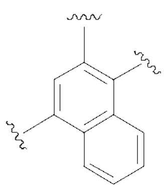 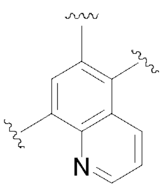 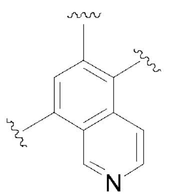 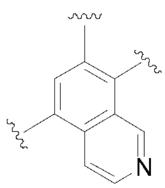 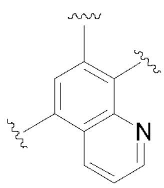 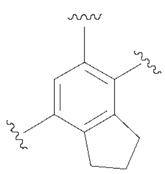

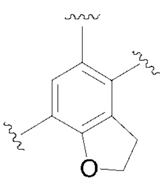 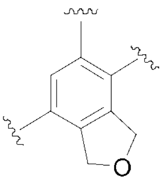 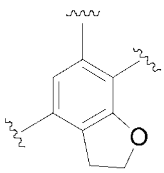 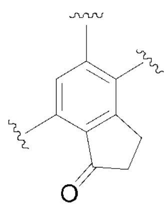 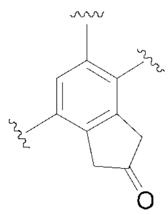 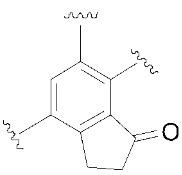

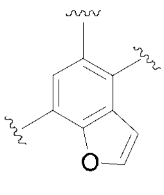 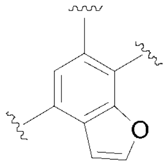 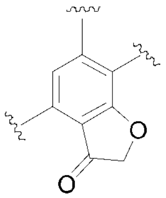 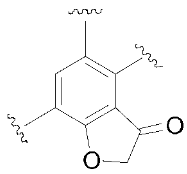

In a preferred Embodiment of Formula (I-3), $X_4$ represents CH or N, preferably N.

In a preferred Embodiment of Formula (I-3), $X_5$ represents $CR^{W5}$ or N, wherein $R^{W5}$ represents hydrogen, halogen, $-CH_3O$, $-CF_3$, $-CHF_2$, $-CH_2F$, $-NH_2$, $-N(CH_3)_2$, -CN, -OH, $-C(O)CH_3$, $-OC(O)CH_3$, $-C(O)OCH_3$ or $-C(O)NH_2$.

In a preferred Embodiment of Formula (I-3), $X_6$ represents $CR^{W6}$ or N, wherein $R^{W6}$ represents hydrogen, halogen, $-CH_3O$, $-CF_3$, $-CHF_2$, $-CH_2F$, $-NH_2$, $-N(CH_3)_2$, -CN, -OH, -C(O)CH3, $-OC(O)CH_3$, $-C(O)OCH_3$ or $-C(O)NH_2$.

In a preferred Embodiment of Formula (1-3), $X_7$ represents CH or N, preferably N.

In a preferred Embodiment of Formula (I-3), the chemical bond between $X_5$ and $X_6$ is fused with ring A to form a group having the following structure:

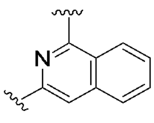 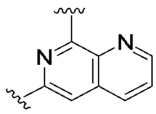 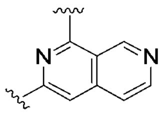 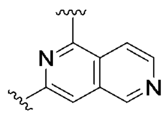 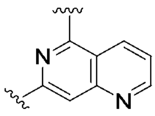 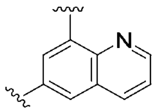

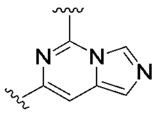 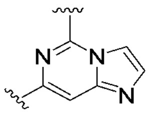 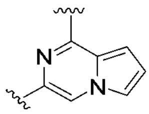 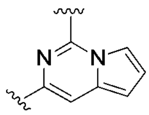 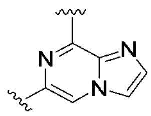 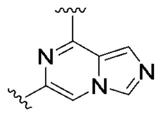 .

In a preferred Embodiment of Formula (I-3), the chemical bond between $X_6$ and $X_7$ is fused with ring A to form a group having the following structure:

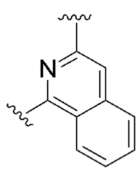 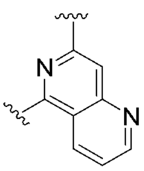 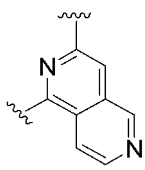 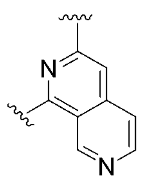 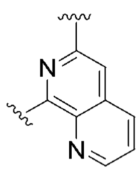 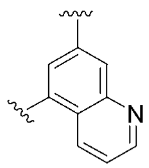

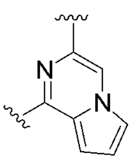 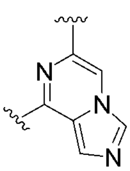 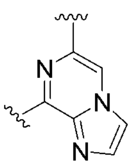 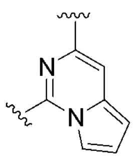 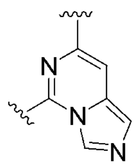 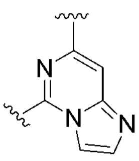 .

In a preferred Embodiment of Formula (1-3),
$L_1$ represents $-C(O)NR^a-$, $-NR^aC(O)-$,

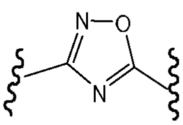, 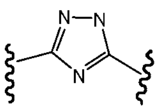, 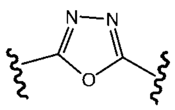, 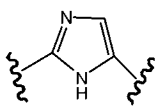, 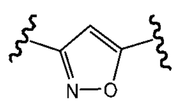,

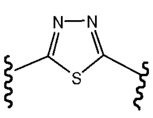, 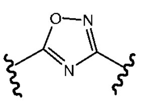, 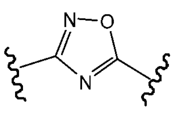, 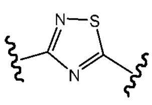, 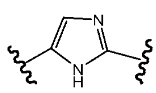, 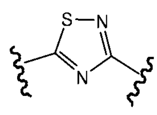, 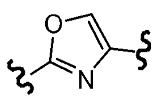,

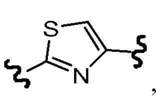, 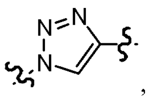, 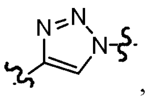, 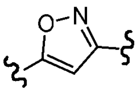, 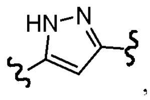, 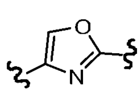, 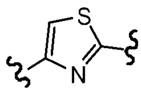, 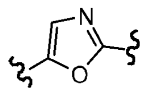,

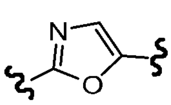, 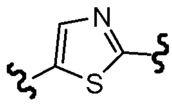 or 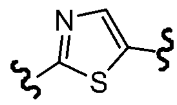.

In a preferred Embodiment of Formula (1-3), $L_1$ represents -C(O)NH-.

In a preferred Embodiment of Formula (I-3), $L_1$ represents

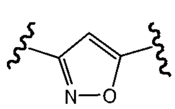, 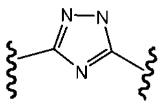, 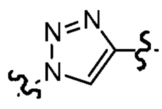,

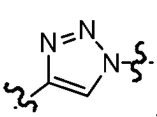, 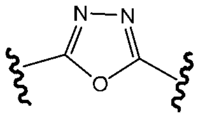 or 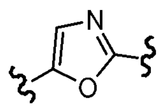.

In a preferred Embodiment of Formula (I-3), $Y_1$ represents $-(CR^aR^b)_m-$.

In a preferred Embodiment of Formula (1-3), $Y_1$ represents -NR-C(=O)-, -O-C(=O)- or -C(=O)O-.

In a preferred Embodiment of Formula (I-3), $Y_1$ represents $-O(CR^aR^b)_m-$ or $-(CR^aR^b)_mO-$.

In a preferred Embodiment of Formula (I-3), $Y_1$ represents $-CR^a=CR^a-$ or $-CR^aR^b-CR^a=CR^b-$.

In a preferred Embodiment of Formula (I-3), $Y_2$ represents $-(CR^aR^b)_n-$.

In a preferred Embodiment of Formula (I-3), $Y_2$ represents $-NR^a-C(=O)-$.

In a preferred Embodiment of Formula (I-3), $Y_2$ represents $-O(CR^aR^b)_n-$ or $-(CR^aR^b)_nO-$.

In a preferred Embodiment of Formula (I-3), $Y_2$ represents $-CR^a=CR^a-$, $-CR^a=CR^b-CR^aR^b-$ or $-CR^aR^b-CR^a=CR^b-$.

In a preferred Embodiment of Formula (I-3), $Y_2$ represents pyrazolyl, oxazolyl, oxadiazolyl, thiazolyl, thiadiazolyl or triazolyl.

In a preferred Embodiment of Formula (I-3), $-Y_1-Y_2-$ represents $-CR^a=CR^a-$ or $-CR^aR^bCR^a=CR^b-$.

In a preferred Embodiment of Formula (I-3), $-Y_1-Y_2-$ represents $-CR^aR^b-NR^aC(O)-$.

In a preferred Embodiment of Formula (I-3), $-Y_1-Y_2-$ represents -

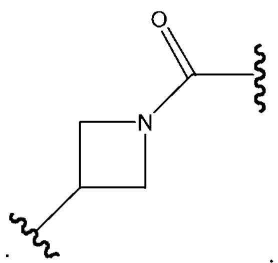.

In a preferred Embodiment of Formula (I-3), $R^L$ represents $-NHS(O)_2CH_2CH_2OH$,

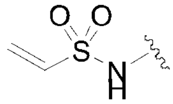, 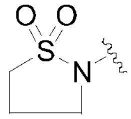,

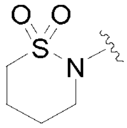, 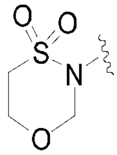,

$-NHS(O)_2CH_2CH_3$ or $-NHS(O)_2CH_3$.

A compound having the structure of Formula (I-1-1) or Formula (I-1-2), or pharmaceutically acceptable salts, stereoisomers, isotope isomers or tautomers thereof,

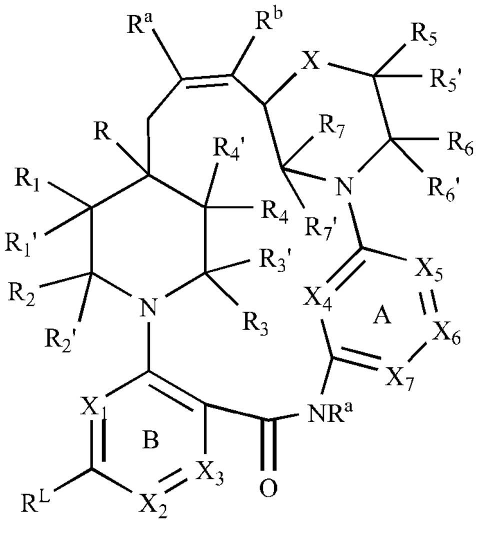

Formula (I-1-1)

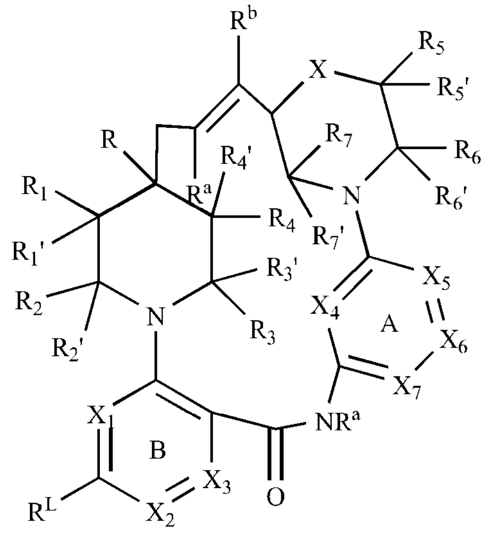

Formula (I-1-2)

wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1-C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
alternatively, the chemical bond between $X_2$ and $X_3$ may be fused with ring B to form a 4-7-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1-C_{10}$ haloalkyl, $-C_0$-$C_{10}$ alkyl $SR^a$, $-C_0$-$C_{10}$ alkyl C(O)

$OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;

alternatively, the chemical bond between $X_5$ and $X_6$ or between $X_6$ and $X_7$ may be fused with ring A to form a 4-7-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^a$-$C(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;

wherein, R represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or $-C(O)CH_3$;

wherein, X represents $CR_9R_9'$, O or $NR^a$;

wherein, $R_1$, $R_1'$, $R_2$, $R_2'$, $R_3$, $R_3'$, $R_4$, $R_4'$, $R_5$, $R_5'$, $R_6$, $R_6'$, $R_7$, $R_7'$, $R_9$ and $R_9'$ each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;

alternatively, pairs $R_1$ and $R_1'$, pairs $R_2$ and $R_2'$, pairs $R_3$ and $R_3'$, pairs $R_4$ and $R_4'$, pairs $R_5$ and $R_5'$, pairs $R_6$ and $R_6'$, pairs $R_7$ and $R_7'$ and pairs $R_9$ and $R_9'$, together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $OR^a$ or oxo;

wherein, $R^L$ represents $L_2$-$R^M$;

wherein, $L_2$ represents absence, $-C_1$-$C_6$ alkyl-, $-NR^a$-, $-NR^aSO_2$-, $-SO_2NR^a$-, $-NR^aS(=O)(=NH)$-, $-S(=O)(=NH)$-, -S-, $-S(=O)$-, $-SO_2$-, $-C_1$-$C_6$ alkyl-O-, -(C=O)-, $-(C=O)NR^a$-, -C=N(OH)-, $-NR^a$ (C=O), $-P(O)(OR^a)_2$, $-NR^aP(O)(OR^a)_2$ or $-NR^aP(O)(R^a)_2$-;

wherein, RM represents $C_1$-$C_6$ alkyl or $C_3$-$C_6$ (hetero) cycloalkyl, wherein the $C_1$-$C_6$ alkyl or $C_3$-$C_6$ (hetero) cycloalkyl can be optionally independently substituted with 0-3 substituents selected from halogen, $C_1$-$C_6$ alkyl, $-OR^a$, $-NR^aR^b$, cyano and $-O-C_1$-$C_6$ haloalkyl;

alternatively, $L_2$-$R^M$ represents

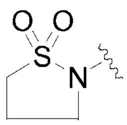 or 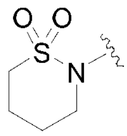 ;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated (heterocyclic) ring which may optionally contain 0-2 heteroatoms selected from O, S, Se and N.

In a preferred Embodiment of Formula (I-1-1) or Formula (I-1-2), $X_1$ represents CH or N, preferably CH.

In a preferred Embodiment of Formula (I-1-1) or Formula (I-1-2), $X_2$ represents CH or N, preferably CH.

In a preferred Embodiment of Formula (I-1-1) or Formula (I-1-2), $X_3$ represents CH or N, preferably CH.

In a preferred Embodiment of Formula (I-1-1) or Formula (I-1-2), the chemical bond between $X_2$ and $X_3$ is fused with ring B to form a group having the following structure:

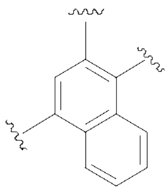 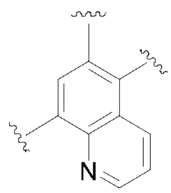 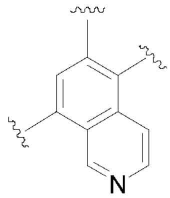 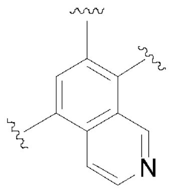 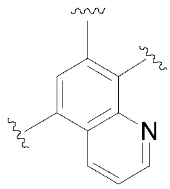 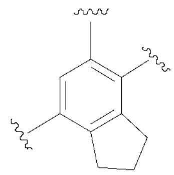

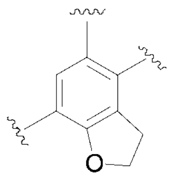 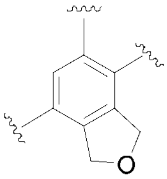 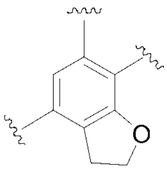 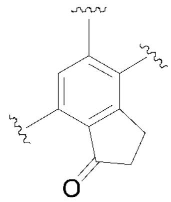 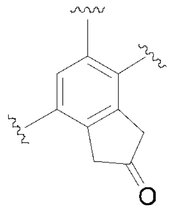 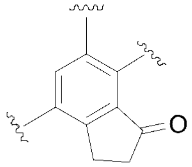

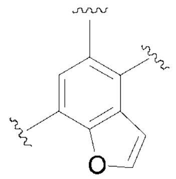 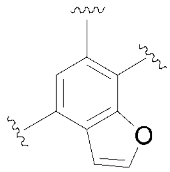 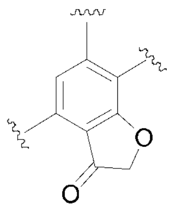 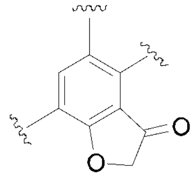

In a preferred Embodiment of Formula (I-1-1) or Formula (I-1-2), $X_4$ represents CH or N, preferably N.

In a preferred Embodiment of Formula (I-1-1) or Formula (I-1-2), $X_5$ represents $CR^{W5}$ or N, wherein $R^{W5}$ represents hydrogen, halogen, $-CH_3O$, $-CF_3$, $-CHF_2$, $-CH_2F$, $-NH_2$, $-N(CH_3)_2$, -CN, -OH, $-C(O)CH_3$, $-OC(O)CH_3$, $-C(O)OCH_3$ or $-C(O)NH_2$.

In a preferred Embodiment of Formula (I-1-1) or Formula (I-1-2), $X_6$ represents $CR^{W6}$ or N, wherein $R^{W6}$ represents hydrogen, halogen, $-CH_3O$, $-CF_3$, $-CHF_2$, $-CH_2F$, $-NH_2$, $-N(CH_3)_2$, -CN, -OH, $-C(O)CH_3$, $-OC(O)CH_3$, $-C(O)OCH_3$ or $-C(O)NH_2$.

In a preferred Embodiment of Formula (I-1-1) or Formula (I-1-2), $X_7$represents CH or N, preferably N.

In a preferred Embodiment of Formula (I-1-1) or Formula (I-1-2), the chemical bond between $X_5$ and $X_6$ is fused with ring A to form a group having the following structure:

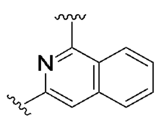 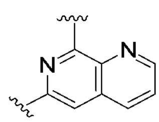 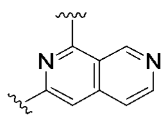 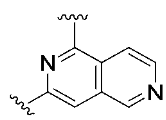 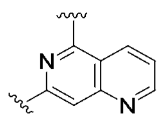 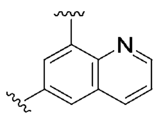

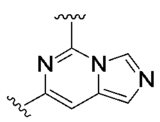 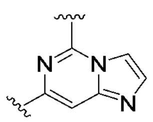 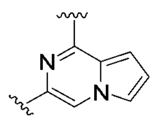 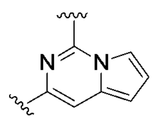 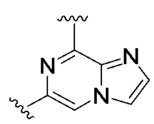 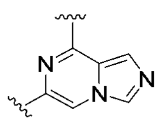 .

In a preferred Embodiment of Formula (I-1-1) or Formula (I-1-2), the chemical bond between $X_6$ and $X_7$ is fused with ring A to form a group having the following structure:

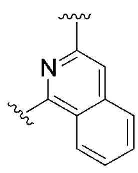 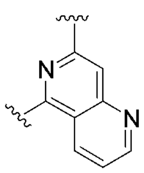 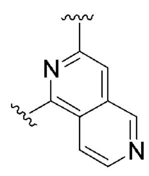 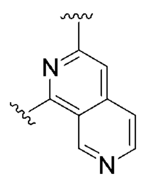 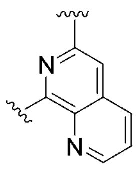 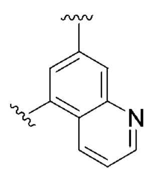

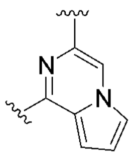 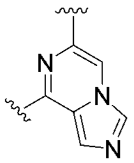 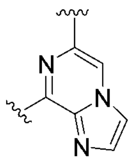 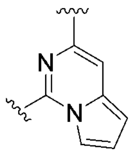 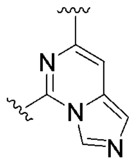 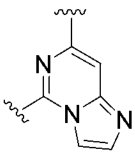 .

In a preferred Embodiment of Formula (I-1-1) or Formula (I-1-2),
$L_1$ represents $-C(O)NR^a-$, $-NR^aC(O)-$,

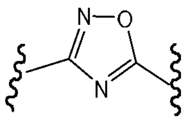, 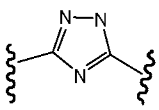, 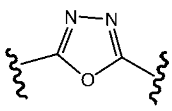, 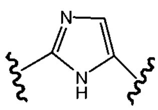, 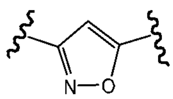,

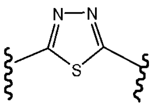, 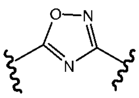, 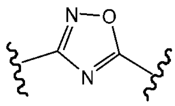, 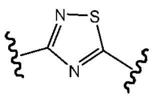, 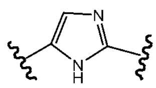, 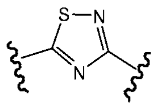, 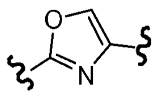,

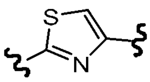, 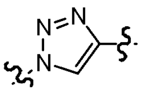, 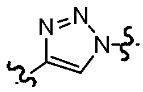, 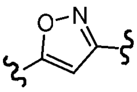, 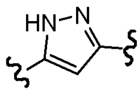, 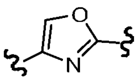, 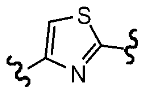, 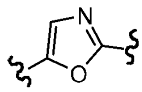,

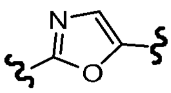, 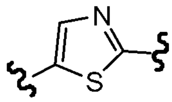 or 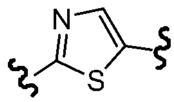.

In a preferred Embodiment of Formula (I-1-1) or Formula (I-1-2), $R^L$ represents $-NHS(O)_2CH_2CH_2OH$,

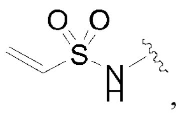, 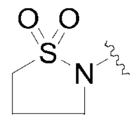, 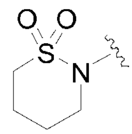, 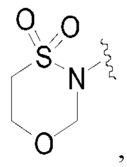,

$-NHS(O)_2CH_2CH_3$ or $-NHS(O)_2CH_3$.

Furthermore, the present disclosure provides a compound having the structure of Formula (I-1-3), or pharmaceutically acceptable salts, stereoisomers, isotope isomers or tautomers thereof,

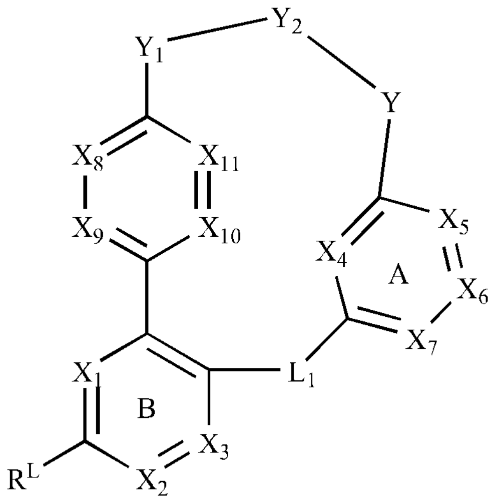

Formula (I-1-3)

wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $X_8$ represents $CR^{W8}$ or N;
wherein, $X_9$ represents $CR^{W9}$ or N;
wherein, $X_{10}$ represents $CR^{W10}$ or N;
wherein, $X_{11}$ represents $CR^{W11}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
wherein, $R^{W8}$, $R^{W9}$, $R^{W10}$ and $R^{W11}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$,

$-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^3$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR^aC(O)OR^b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^aR^b)$, $-C_0-C_{10}$ alkyl $P(R^a R^b)$ or $-C_0-C_{10}$ alkyl $NR^aR^b$;

alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, azido, hydroxy $(C_1-C_{10})$ alkyl, $C_1-C_{10}$ alkyl, $C_2-C_{10}$ alkenyl, $C_2-C_{10}$ alkynyl, $C_1-C_{10}$ deuterated alkyl, $C_3-C_{12}$ cycloalkyl, $C_1-C_{10}$ haloalkyl, $C_2-C_{10}$ haloalkenyl, $C_2-C_{10}$ haloalkynyl, $C_3-C_{12}$ halocycloalkyl, 3-12-membered heterocyclyl, $C_6-C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^a$-$C(O)OR^b$, $-OC(O)NR^aR^b$, $-OC(O)OR^a$, $-SF_5$, $-PO(R^a R^b)$, $-P(R^aR^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR^aC(O)OR^b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;

wherein, $L_1$ represents $-C(O)NR^a-$, $-NR^aC(O)-$ or a 5-6-membered heteroaromatic ring;

wherein, Y represents absence, $-CH_2-$, -O-, $-NR^a-$, $-C(O)NR^a$, $-NR^aC(O)-$ or the following groups:

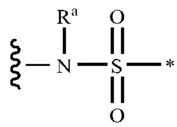 , 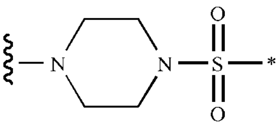

or $C_{y3}$;

wherein, * represents the site of connection with ring A; the wavy line represents the site of connection with $Y_2$;

wherein, $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein the substituents are each independently selected from the following: hydrogen, deuterium, halogen, cyano, nitro, azido, hydroxy $(C_1-C_{10})$ alkyl, $C_1-C_{10}$ alkyl, $C_2-C_{10}$ alkenyl, $C_2-C_{10}$ alkynyl, $C_1-C_{10}$ deuterated alkyl, $C_3-C_{12}$ cycloalkyl, $C_1-C_{10}$ haloalkyl, $C_2-C_{10}$ haloalkenyl, $C_2-C_{10}$ haloalkynyl, $C_3-C_{12}$ halocycloalkyl, 3-12-membered heterocyclyl, $C_6-C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR^aC(O)OR^b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;

wherein, $Y_1$ represents $-(CR^aR^b)_m-$; $Y_2$ represents $-(CR^aR^b)_n-$;

wherein, optionally $-CR^aR^b-$ may be substituted with -O-, -S-, -Se-, $-NR^a-$, $-NR^aSO_2-$, $-NR^aC(=O)-$, $-C(=O)NR^a-$, $-OC(=O)-$, $-C(=O)O-$, $-SO_2NR^a-$, $-S(=O)(NR^a)-$, $-P(O)(OR^a)_2-$, $PO(R^a R^b)$, $-NR^aP(O)(OR^a)_2-$ or $-NR^aP(O)(R^a)_2-$, $-CR^a=CR^b-$,

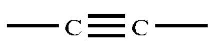 , 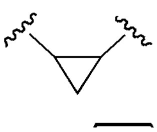 , 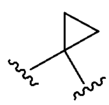 , 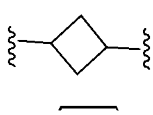 , 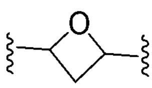 , 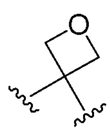 ,

5-6-membered heteroaryl,

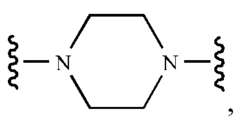, 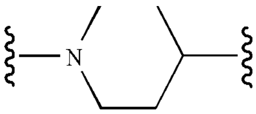 or 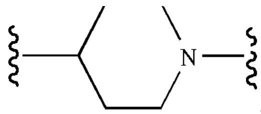;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^a$S(=O)(=NH)-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)_2$, -$NR^a$P(O)$(OR^a)_2$ or -$NR^a$P(O)$(R^a)_2$-;

wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;

alternatively, $L_2$-$R^M$ represents

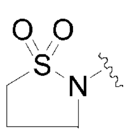 or 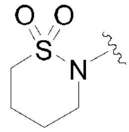;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, and N.

In a preferred Embodiment of Formula (I-1-3), $X_1$ represents CH or N, preferably CH.

In a preferred Embodiment of Formula (I-1-3), $X_2$ represents CH or N, preferably CH.

In a preferred Embodiment of Formula (I-1-3), $X_3$ represents CH or N, preferably CH.

In a preferred Embodiment of Formula (I-1-3), the chemical bond between $X_2$ and $X_3$ is fused with ring B to form a group having the following structure:

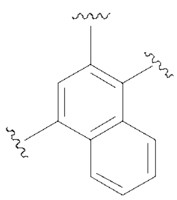 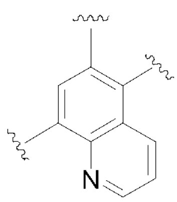 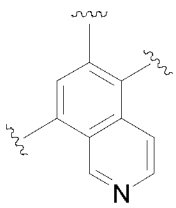 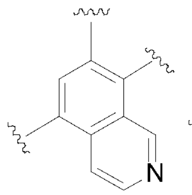 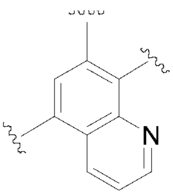 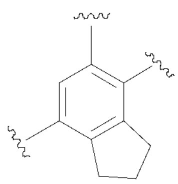

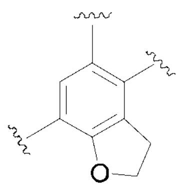 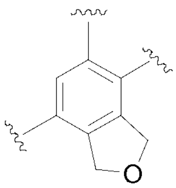 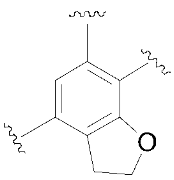 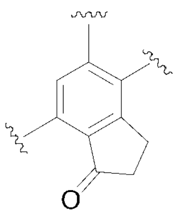 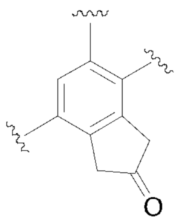 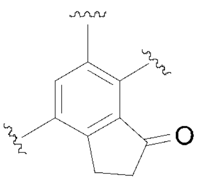

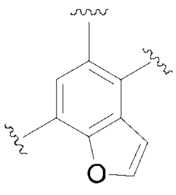 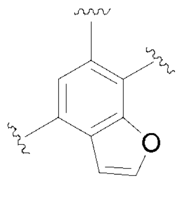 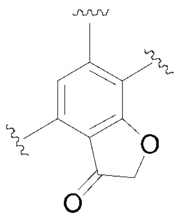 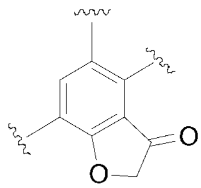

In a preferred Embodiment of Formula (I-1-3), $X_4$ represents CH or N, preferably N.

In a preferred Embodiment of Formula (I-1-3), $X_5$ represents $CR^{W5}$ or N, wherein $R^{W5}$ represents hydrogen, halogen, $CH_3O$, $CF_3$, $CHF_2$, $CH_2F$, $NH_2$, $-N(CH_3)_2$, -CN, -OH, $-C(O)CH_3$, $-OC(O)CH_3$, $-C(O)OCH_3$ or $-C(O)NH_2$.

In a preferred Embodiment of Formula (I-1-3), $X_6$ represents $CR^{W6}$ or N, wherein $R^{W6}$ represents hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

In a preferred Embodiment of Formula (I-1-3), $X_7$ represents CH or N, preferably N.

In a preferred Embodiment of Formula (I-1-3), the chemical bond between $X_5$ and $X_6$ is fused with ring A to form a group having the following structure:

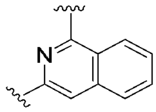 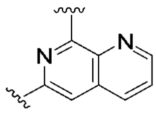 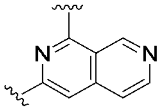 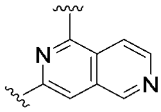 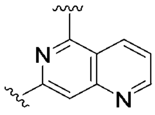 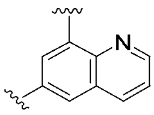

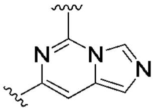 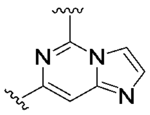 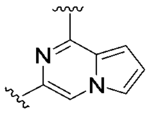 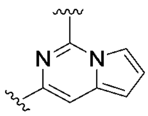 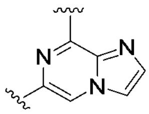 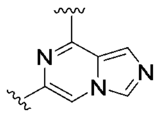 .

In a preferred Embodiment of Formula (I-1-3), the chemical bond between $X_6$ and $X_7$ is fused with ring A to form a group having the following structure:

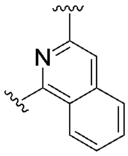 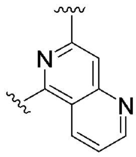 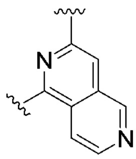 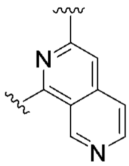 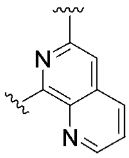 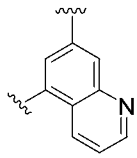

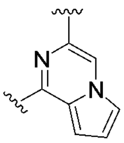 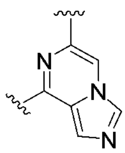 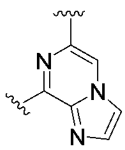 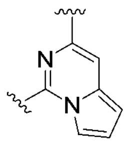 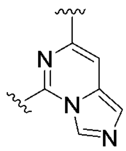 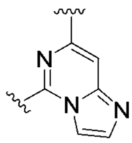 .

In a preferred Embodiment of Formula (I-1-3), Y represents absence, $-CH_2-$, -O-, -Se-, $-NR^a-$, $-C(O)NR^a$, $-NR^aC(O)-$or the following groups:

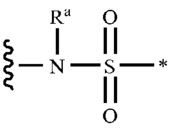 , 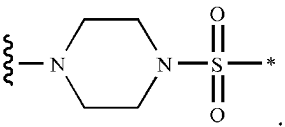 .

In a preferred Embodiment of Formula (I-1-3), Y represents -O- or -NH-.

In a preferred Embodiment of Formula (I-1-3), Y represents

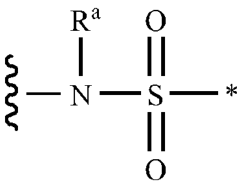 .

In a preferred Embodiment of Formula (I-1-3), Y represents

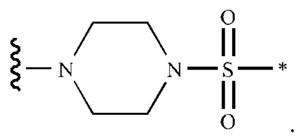.

In a preferred Embodiment of Formula (I-1-3), Y represents $C_{y3}$, wherein $C_{y3}$ represents the following groups:

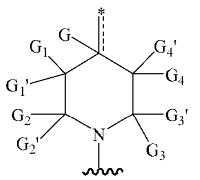, 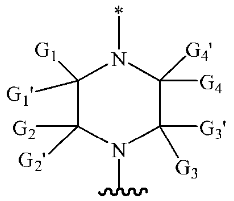, 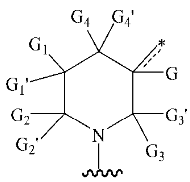, 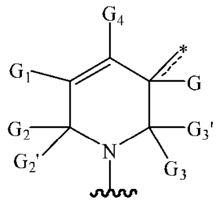, 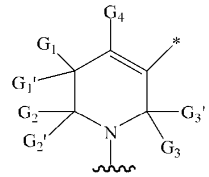,

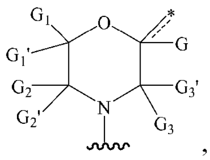, 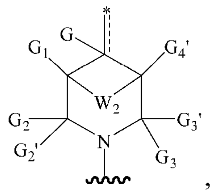, 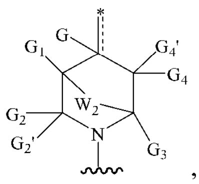, 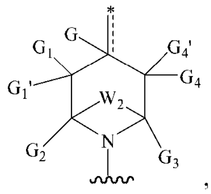, 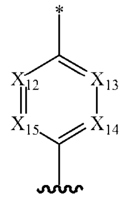, 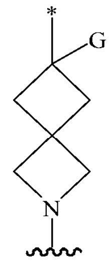, 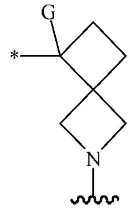,

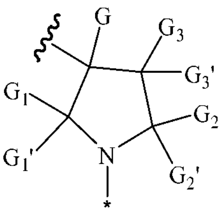, 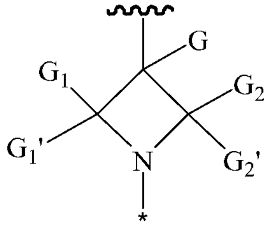;

wherein, when - - - represents a double bond, G represents absence; wherein, when - - - represents a single bond, G represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;

wherein, $G_1$, $G_1$', $G_2$, G2', $G_3$, $G_3$', $G_4$ and $G_4$' each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;

alternatively, pairs $G_1$ and $G_1$', pairs $G_2$ and $G_2$', pairs $G_3$ and $G_3$' and pairs $G_4$ and $G_4$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $OR^a$ or oxo;

wherein, $W_2$ represents -$(CR^iR^j)_p$-;

wherein, $R^i$ and $R^j$ each independently represent hydrogen or $C_1$-$C_6$ alkyl;

wherein, p represents 1 or 2;

wherein, $X_{12}$ represents $CR^{W12}$ or N;

wherein, $X_{13}$ represents $CR^{W13}$ or N;

wherein, $X_{14}$ represents $CR^{W14}$ or N;

wherein, $X_{15}$ represents $CR^{W15}$ or N;

wherein, $R^{W12}$, $R^{W13}$, $R^{W14}$ and $R^{W15}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$ -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{19}$ alkyl $NR^a$C(O)$OR^b$, -$C_0$-$C_{10}$ alkyl OC(O)$NR^aR^b$, -$C_0$-$C_{10}$

alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;

In a preferred Embodiment of Formula (I-1-3), $X_{12}$ represents CH or N.

In a preferred Embodiment of Formula (I-1-3), $X_{13}$ represents CH or N.

In a preferred Embodiment of Formula (I-1-3), $X_{14}$ represents CH or N.

In a preferred Embodiment of Formula (I-1-3), $X_{15}$ represents CH or N.

In a preferred Embodiment of Formula (I-1-3), $G_1$, $G_1$', $G_2$, $G_2$', $G_3$, $G_3$', $G_4$ and $G_4$' each independently represent hydrogen.

In a preferred Embodiment of Formula (I-1-3), $W_2$ represents $CH_2$ or $CH_2CH_2$.

In a preferred Embodiment of Formula (1-1-3),

$L_1$ represents $-C(O)NR^a-$, $-NR^aC(O)-$,

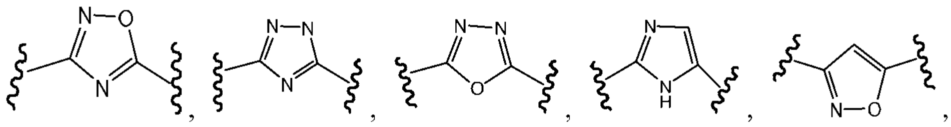

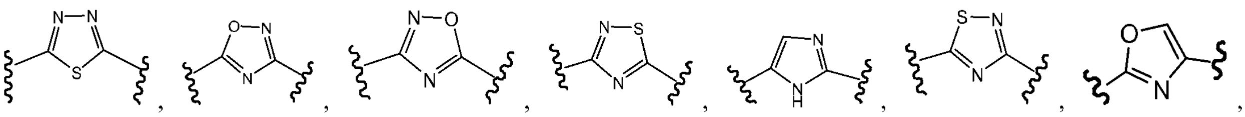

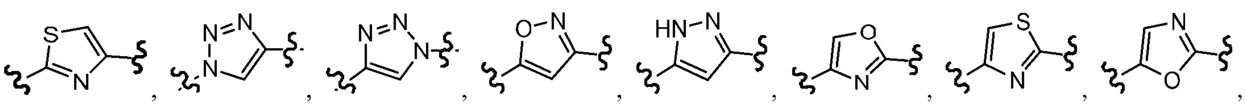

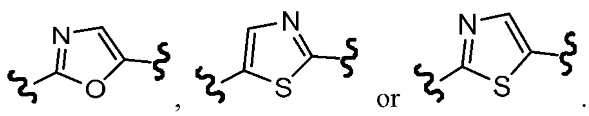

In a preferred Embodiment of Formula (I-1-3), $L_1$ represents -C(O)NH-.

In a preferred Embodiment of Formula (I-1-3), $L_1$ represents

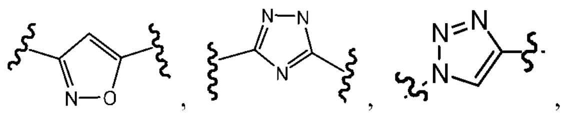

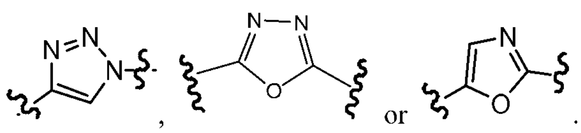

In a preferred Embodiment of Formula (I-1-3), $Y_1$ represents $-(CR^aR^b)_m-$.

In a preferred Embodiment of Formula (I-1-3), $Y_1$ represents -NR-C(=O)-, -O-C(=O)- or -C(=O)O-.

In a preferred Embodiment of Formula (I-1-3), $Y_1$ represents $-O(CR^aR^b)_m-$ or $-(CR^aR^b)_mO-$.

In a preferred Embodiment of Formula (I-1-3), $Y_1$ represents $-CR^a=CR^a-$ or $-CR^aR^b-CR^a=CR^b-$.

In a preferred Embodiment of Formula (I-1-3), $Y_2$ represents $-(CR^aR^b)_n-$.

In a preferred Embodiment of Formula (I-1-3), $Y_2$ represents $-NR^a-C(=O)-$.

In a preferred Embodiment of Formula (I-1-3), $Y_2$ represents $-O(CR^aR^b)_n-$ or $-(CR^aR^b)_{nO}-$.

In a preferred Embodiment of Formula (I-1-3), $Y_2$ represents $-CR^a=CR^a-$, $-CR^a=CR^b-CR^aR^b-$ or $-CR^aR^b-CR^a=CR^b-$.

In a preferred Embodiment of Formula (I-1-3), $Y_2$ represents pyrazolyl, oxazolyl, oxadiazolyl, thiazolyl, thiadiazolyl or triazolyl.

In a preferred Embodiment of Formula (I-1-3), $-Y_1-Y_2-$ represents $-CR^a=CR^a-$ or $-CR^aR^bCR^a=CR^b-$.

In a preferred Embodiment of Formula (I-1-3), $-Y_1-Y_2-$ represents $-CR^aR^b-NR^aC(O)-$.

In a preferred Embodiment of Formula (I-1-3), $-Y_1-Y_2-$ represents -

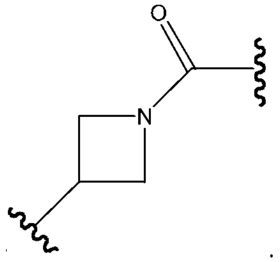

.

In a preferred Embodiment of Formula (I-1-3), $R^L$ represents $-NHS(O)_2CH_2CH_2OH$,

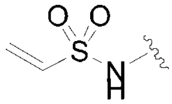 , 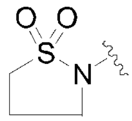 ,

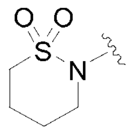 , 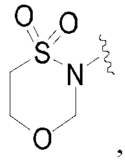 ,

$-NHS(O)_2CH_2CH_3$ or $-NHS(O)_2CH_3$.

Furthermore, the present disclosure provides a compound having the structure of Formula (I-1-4), or pharmaceutically acceptable salts, stereoisomers, isotope isomers or tautomers thereof,

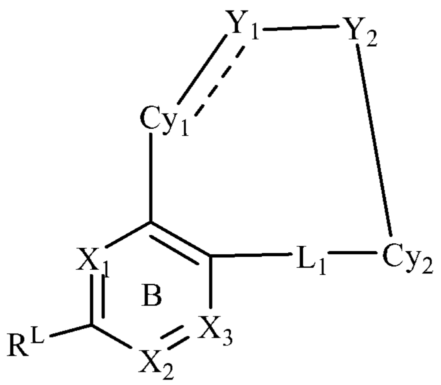

式(I-1-4)

wherein, $X_1$ represents $CR^{W1}$ or N;
wherein, $X_2$ represents $CR^{W2}$ or N;
wherein, $X_3$ represents $CR^{W3}$ or N;
wherein, $R^{W1}$, $R^{W1}$, and $R^{W3}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_2$ and $X_3$ may be fused with ring B to form a 4-8-membered saturated or

unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_1$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -$S(O)R^a$, -O-$C_1$-$C_{10}$ haloalkyl, -$SR^a$, -$C(O)OR^a$, -$C(O)R^a$, -$OC(O)R^a$, -$NR^aC(O)R^b$, -$C(O)NR^aR^b$, -$NR^aC(O)OR^b$, -$OC(O)NR^aR^b$, -$OC(O)OR^a$, -$SF_5$, -$PO(R^a R^b)$, -$P(R^a R^b)$ or -$NR^aR^b$; -$C_0$-$C_{10}$ alkyl -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^aR^b)$, -$C_0$-$C_{10}$ alkyl $P(R^a R^b)$ or -$C_0$-$C_{10}$ alkyl $NR^aR^b$;

wherein, $C_{y1}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein substituents are each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -$S(O)R^a$, -O-$C_1$-$C_{10}$ haloalkyl, -$SR^a$, -$C(O)OR^a$, -$C(O)R^a$, -$OC(O)R^a$, -$NR^aC(O)R^b$, -$C(O)NR^aR^b$, -$NR^aC(O)OR^b$, -$OC(O)NR^aR^b$, -$OC(O)OR^a$, -$SF_5$, -$PO(R^a R^b)$, -$P(R^aR^b)$ or -$NR^aR^b$; -$C_0$-$C_{10}$ alkyl -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;

wherein, $L_1$ represents -$C(O)NR^a$-, -$NR^aC(O)$- or a 5-6-membered heteroaromatic ring;

wherein, $Y_1$ represents -$(CR^aR^b)_m$-; $Y_2$ represents -$(CR^aR^b)_n$-;

wherein, optionally -$CR^aR^b$- may be substituted with -O-, -S-, -Se-, -$NR^a$-, -$NR^aSO_2$-, -$NR^aC(=O)$-, -$C(=O)NR^a$-, -OC(=O)-, -C(=O)O-, -$SO_2NR^a$-, -$S(=O)(NR^a)$-, -$P(O)(OR^a)_2$-, $PO(R^a R^b)$, -$NR^aP(O)(OR^a)_2$- or -$NR^aP(O)(R^a)_2$-, -$CR^a$=$CR^b$-,

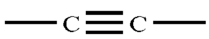 , 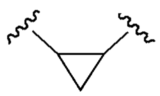 , 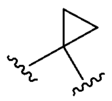 , 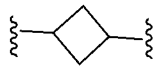 , 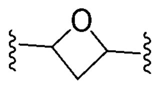 , 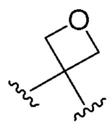 ,

5-6-membered heteroaryl,

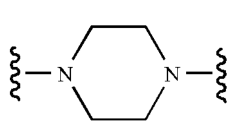 , 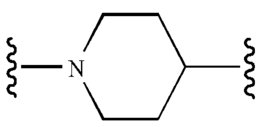 or 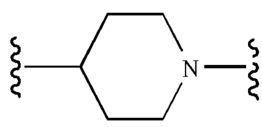 ;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^aS(=O)(=NH)$-, -S(=O) (=NH)-, -S-, -S(=O)-, -$SO_2$-, -$C_1$-$C_6$ alkyl-O-, -(C=O)-, -$(C=O)NR^a$-, -C=N(OH)-, -$NR^a$ (C=O), -$P(O)(OR^a)_2$, -$NR^aP(O)(OR^a)_2$ or -$NR^aP(O)(R^a)_2$-;

wherein, $C_{y2}$ represents

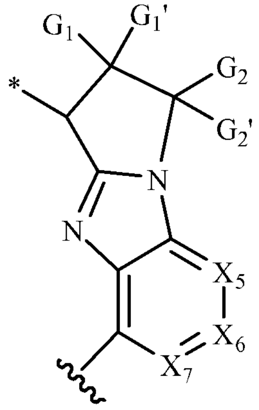 or 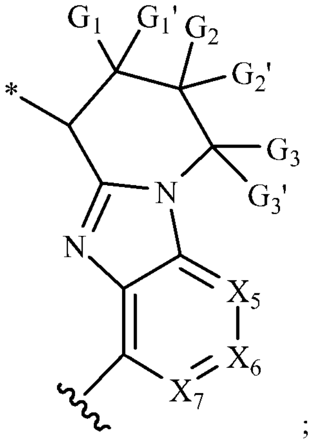 ;

wherein, * represents the site of connection with $Y_2$, and the wavy line represents the site of connection with $L_1$;
wherein, $X_5$ represents $CR^{W5}$ or N;
wherein, $X^6$ represents $CR^{W6}$ or N;
wherein, $X^7$ represents $CR^{W7}$ or N;
wherein, $R^{W5}$, $R^{W6}$, and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^aR^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl O-$C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl O-$C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
wherein, $G_1$, $G_1'$, $G_2$, $G_2'$, $G_3$ and $G_3'$ each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;
alternatively, pairs $G_1$ and $G_1'$, pairs $G_2$ and $G_2'$ and pairs $G_3$ and $G_3'$, together with the carbon atom connected thereto, form a 3-6-membered ring or a 3-6-membered heterocyclic ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $OR^a$ or oxo;
wherein, RM represents $C_1$-$C_6$ alkyl or $C_3$-$C_6$ (hetero) cycloalkyl, wherein the $C_1$-$C_6$ alkyl or $C_3$-$C_6$ (hetero) cycloalkyl can be optionally independently substituted with 0-3 substituents selected from halogen, $C_1$-$C_6$ alkyl, $-OR^a$, $-NR^aR^b$, cyano and $-O-C_1$-$C_6$ haloalkyl;
alternatively, $L_2$-$R^M$ represents

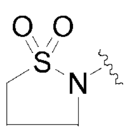 or 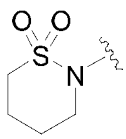 ;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, Se and N.

In a preferred Embodiment of Formula (I-1-4), $X_1$ represents CH or N, preferably CH.

In a preferred Embodiment of Formula (I-1-4), $X_2$ represents CH or N, preferably CH.

In a preferred Embodiment of Formula (I-1-4), $X_3$ represents CH or N, preferably CH.

In a preferred Embodiment of Formula (I-1-4), the chemical bond between $X_2$ and $X_3$ is fused with ring B to form a group having the following structure:

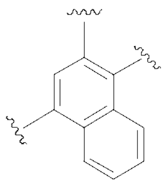 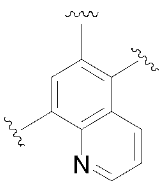 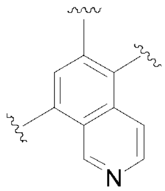 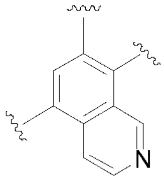 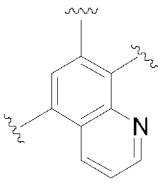 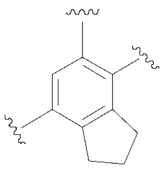

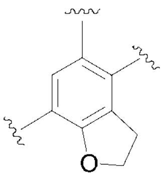 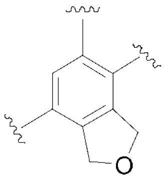 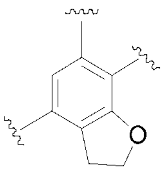 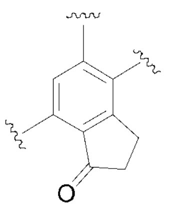 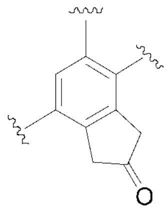 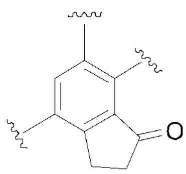

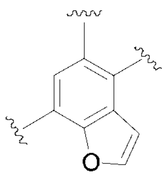 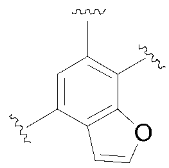 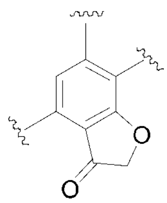 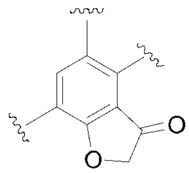

In a preferred Embodiment of Formula (I-1-4), $X_5$ represents $CR^{W5}$ or N, wherein $R^{W5}$ represents hydrogen, halogen, $CH_3O$, $CF_3$, $CHF_2$, $CH_2F$, $NH_2$, $-N(CH_3)_2$, -CN, -OH, $-C(O)CH_3$, $-OC(O)CH_3$, $-C(O)OCH_3$ or $-C(O)NH_2$.

In a preferred Embodiment of Formula (I-1-4), $X_6$ represents $CR^{W6}$ or N, wherein $R^{W6}$ represents hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

In a preferred Embodiment of Formula (I-1-4), $X_7$ represents CH or N, preferably N.

In a preferred Embodiment of Formula (I-1-4), the chemical bond between $X_5$ and $X_6$ is fused with ring A to form a group having the following structure:

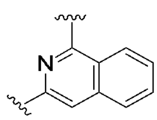 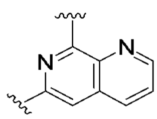 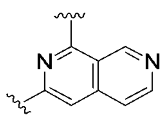 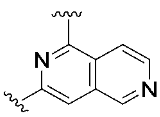 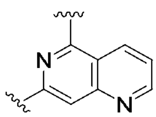 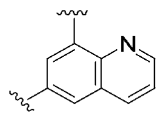

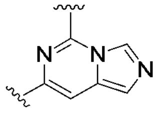 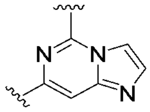 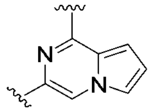 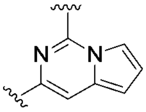 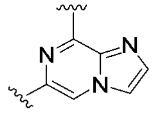 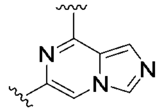.

In a preferred Embodiment of Formula (I-1-4), the chemical bond between $X_6$ and $X_7$ is fused with ring A to form a group having the following structure:

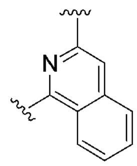 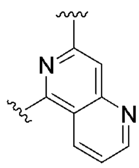 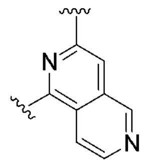 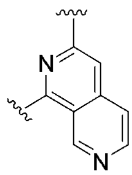 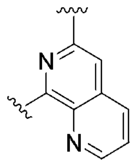 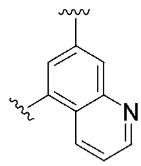

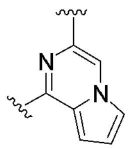 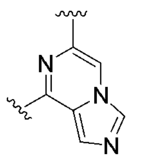 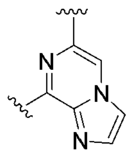 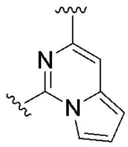 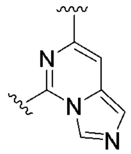 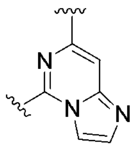.

In a preferred Embodiment of Formula (I-1-4), $L_1$ represents -C(O)NR$^a$-, -NR$^a$C(O)-,

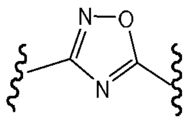, 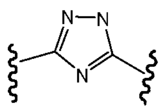, 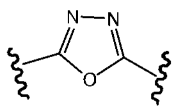, 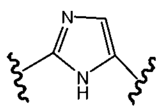, 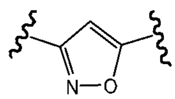,

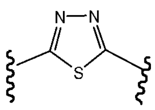, 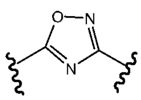, 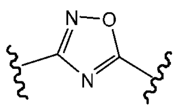, 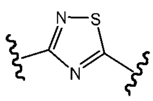, 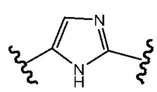, 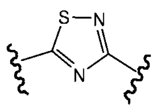, 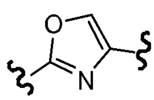,

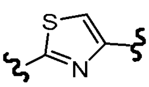, 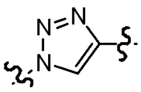, 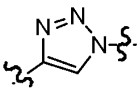, 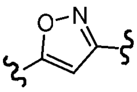, 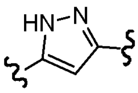, 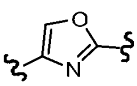, 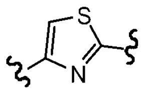, 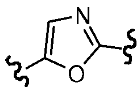,

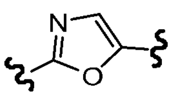, 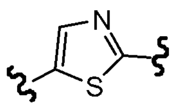 or 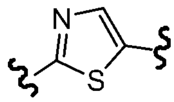.

In a preferred Embodiment of Formula (I-1-4), $L_1$ represents -C(O)NH-.

In a preferred Embodiment of Formula (I-1-4), $L_1$ represents

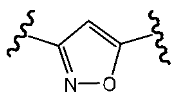, 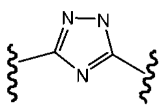, 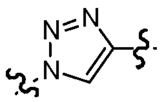,

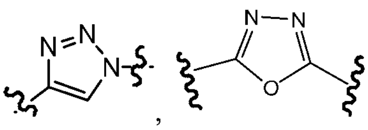 or 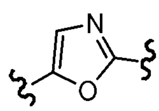.

In a preferred Embodiment of Formula (I-1-4), $Y_1$ represents -(CR$^a$R$^b$)$_m$-.

In a preferred Embodiment of Formula (I-1-4), $Y_1$ represents -NR-C(=O)-, -O-C(=O)- or -C(=O)O-.

In a preferred Embodiment of Formula (I-1-4), $Y_1$ represents -O(CR$^a$R$^b$)$_m$- or -(CR$^a$R$^b$)$_m$O-.

In a preferred Embodiment of Formula (I-1-4), $Y_1$ represents -CR$^a$=CR$^a$- or -CR$^a$R$^b$-CR$^a$=CR$^b$-.

In a preferred Embodiment of Formula (I-1-4), $Y_2$ represents -(CR$^a$R$^b$)$_n$-.

In a preferred Embodiment of Formula (I-1-4), $Y_2$ represents -NR$^a$-C(=O)-.

In a preferred Embodiment of Formula (I-1-4), $Y_2$ represents -O(CR$^a$R$^b$)$_n$- or -(CR$^a$R$^b$)$_n$O-.

In a preferred Embodiment of Formula (I-1-4), $Y_2$ represents -CR$^a$=CR$^a$-, -CR$^a$=CR$^b$-CR$^a$R$^b$- or -CR$^a$R$^b$-CR$^a$=CR$^b$-.

In a preferred Embodiment of Formula (I-1-4), $Y_2$ represents pyrazolyl, oxazolyl, oxadiazolyl, thiazolyl, thiadiazolyl or triazolyl.

In a preferred Embodiment of Formula (I-1-4), $-Y_1-Y_2-$ represents $-CR^a=CR^a-$ or $-CR^aR^bCR^a=CR^b-$.

In a preferred Embodiment of Formula (I-1-4), $-Y_1-Y_2-$ represents $-CR^aR^b-NR^aC(O)-$.

In a preferred Embodiment of Formula (I-1-4), $-Y_1-Y_2-$ represents -

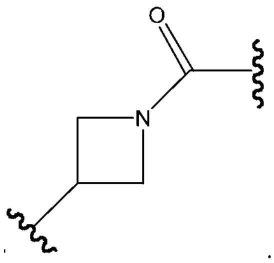
, .

In a preferred Embodiment of Formula (I-1-4), $R^L$ represents $-NHS(O)_2CH_2CH_2OH$,

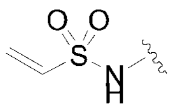
,
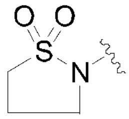
,

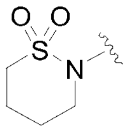
,
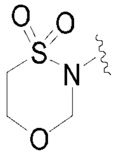
,

$-NHS(O)_2CH_2CH_3$ or $-NHS(O)_2CH_3$.

In particular, the present disclosure provides a compound having the following structures:

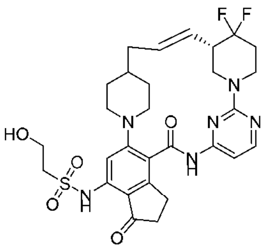
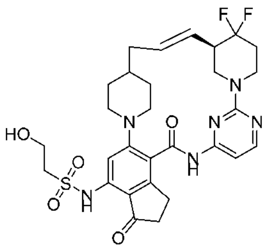
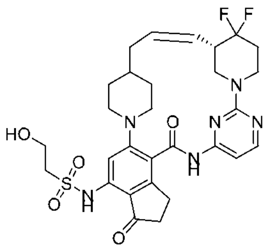
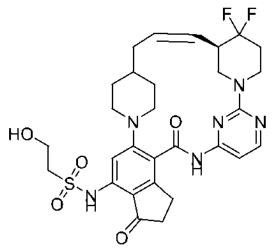

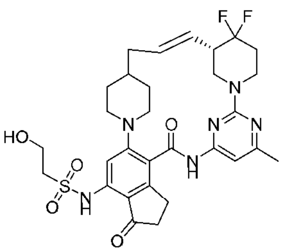
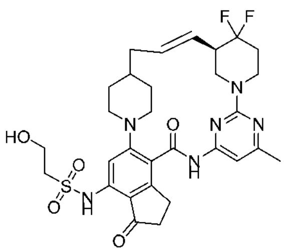
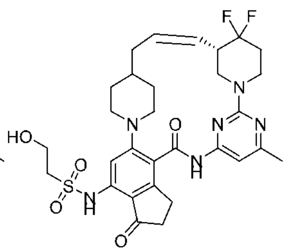
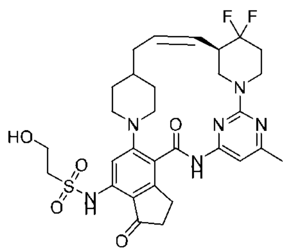

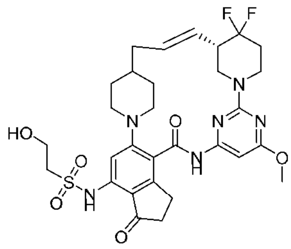
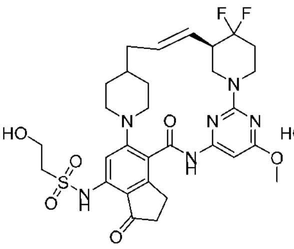
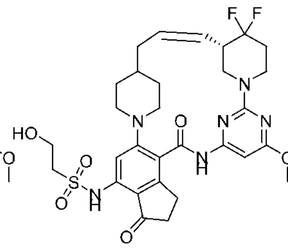
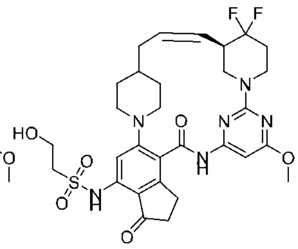

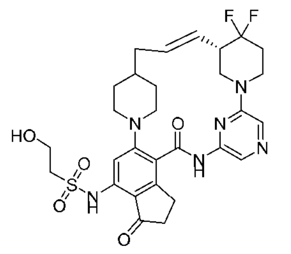
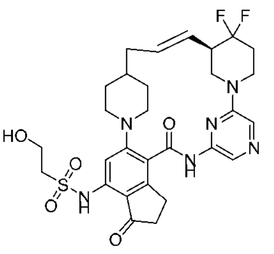
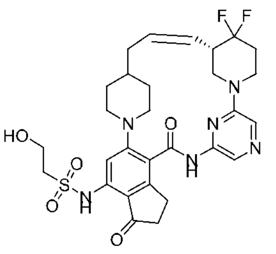
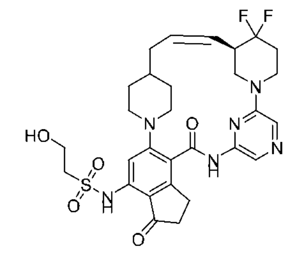

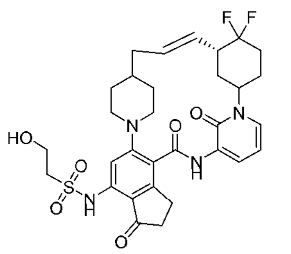
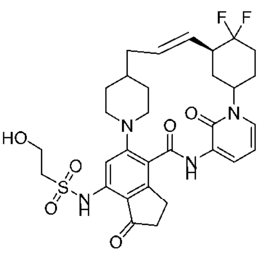
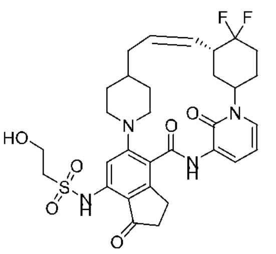
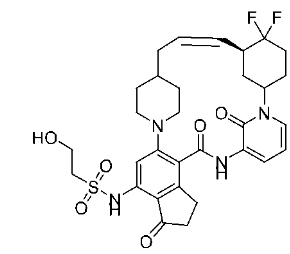

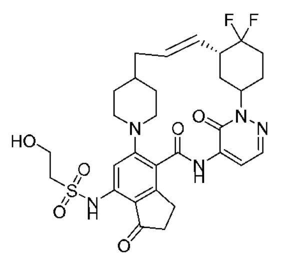
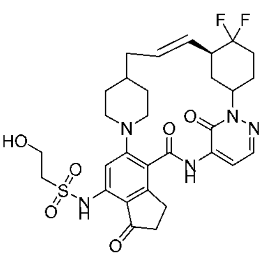
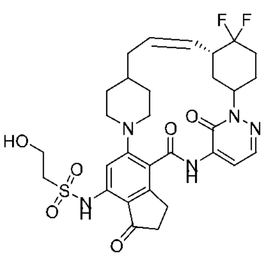
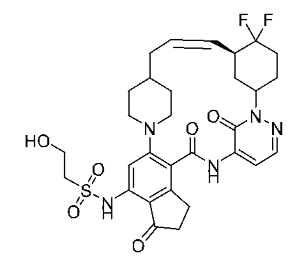

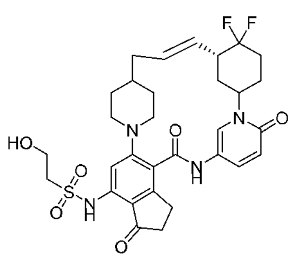
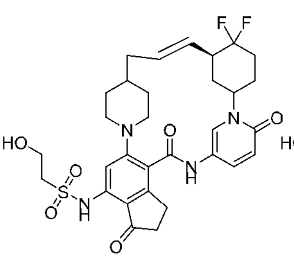
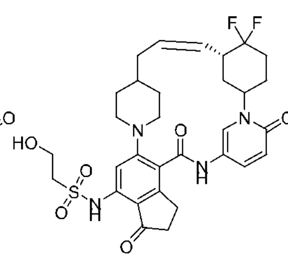
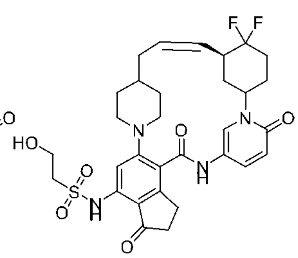

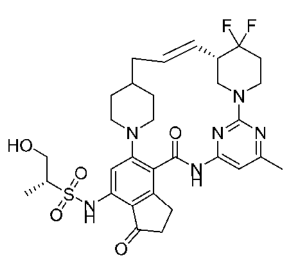
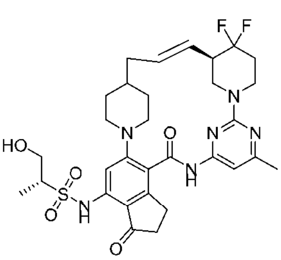
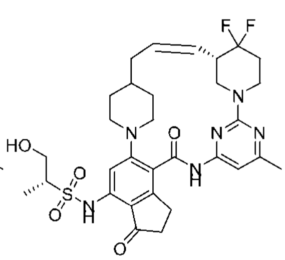
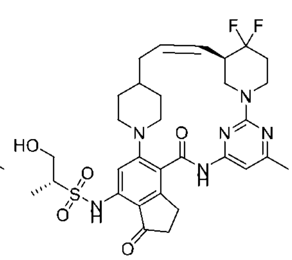

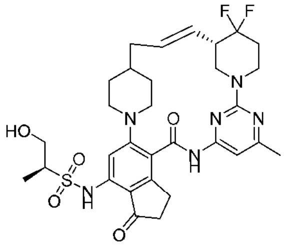
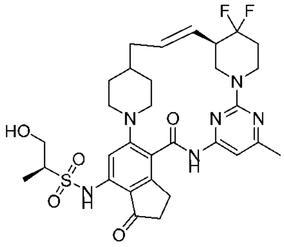
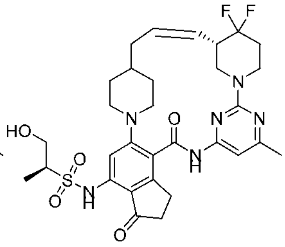
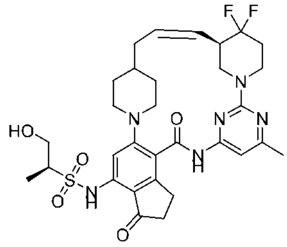

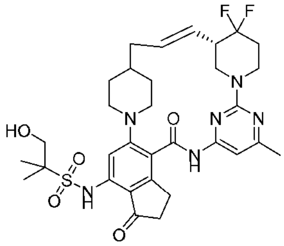
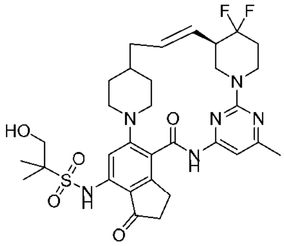
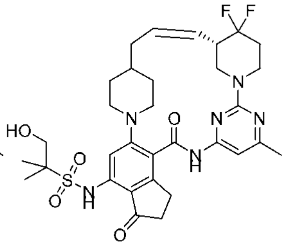
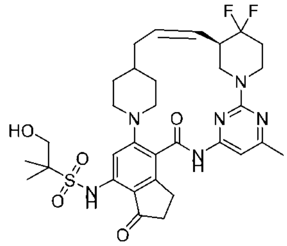

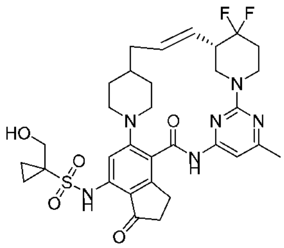
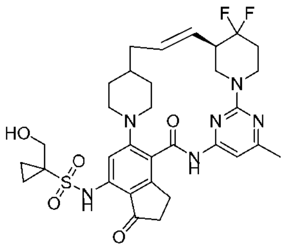
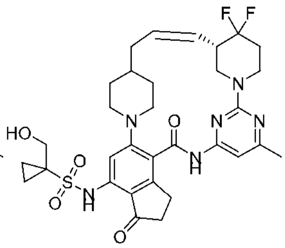
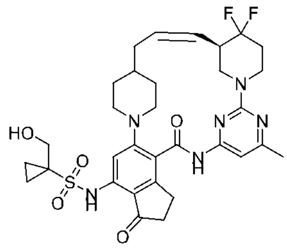

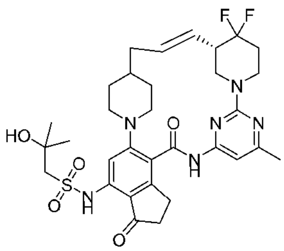
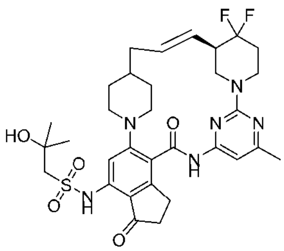
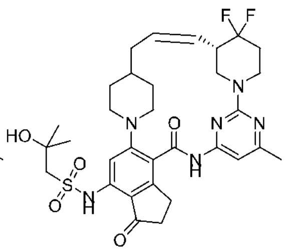
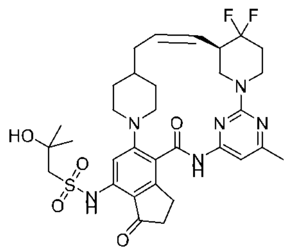

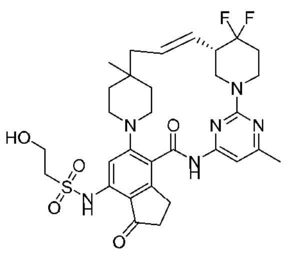
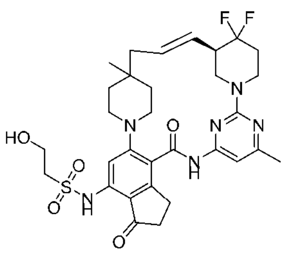
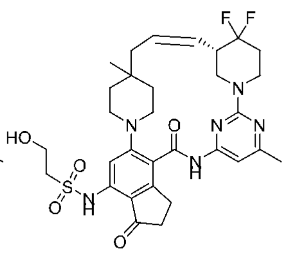
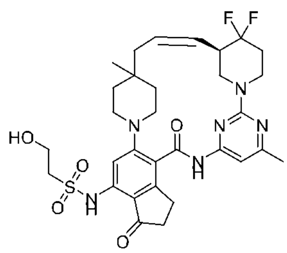

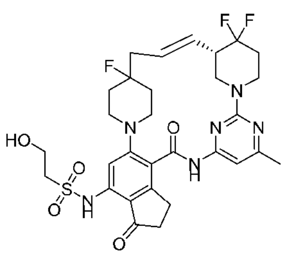
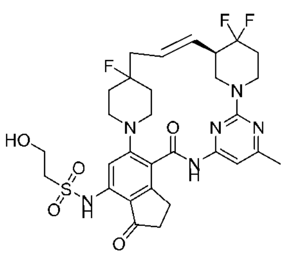
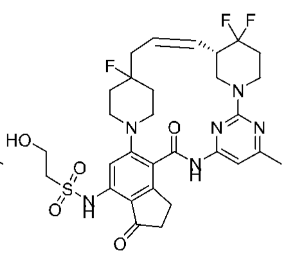
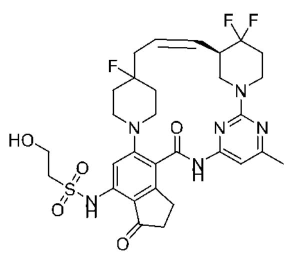

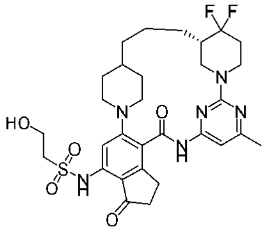
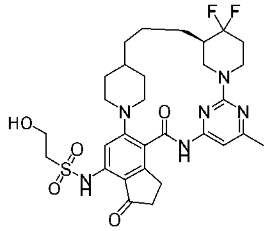
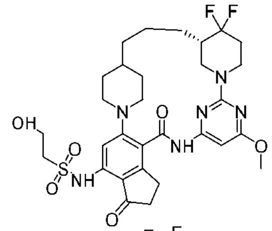
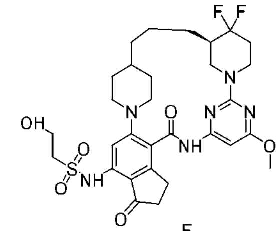
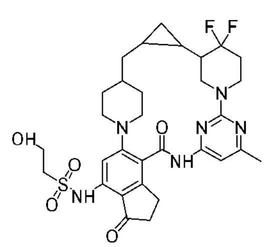
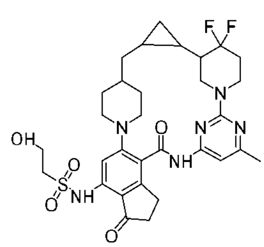
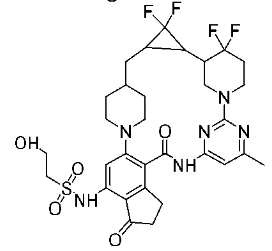
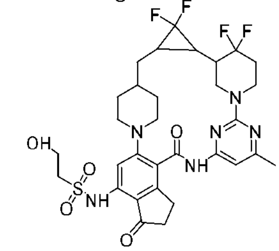
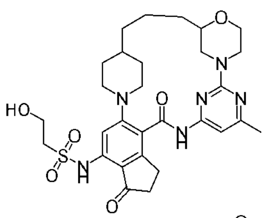
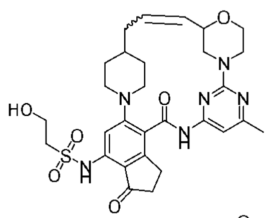
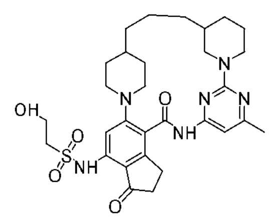
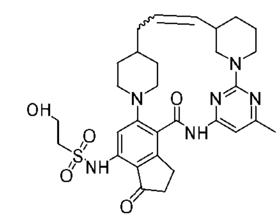
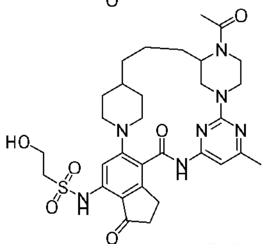
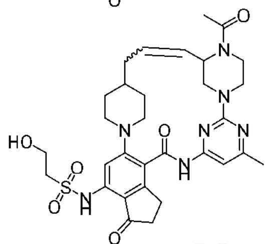
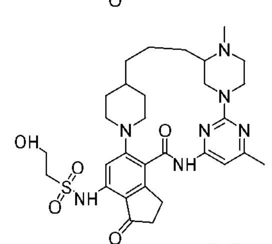
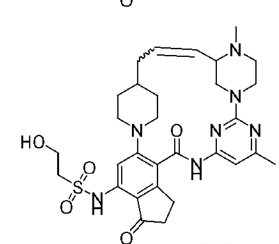
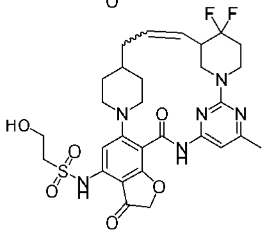
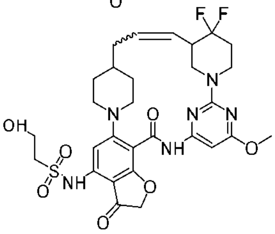
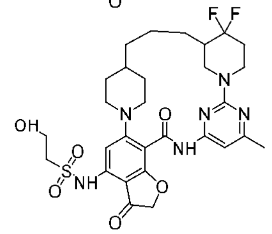
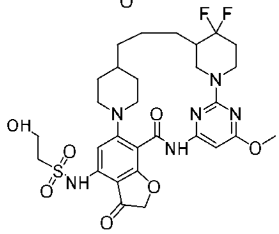
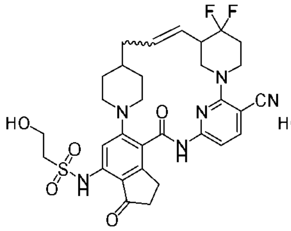
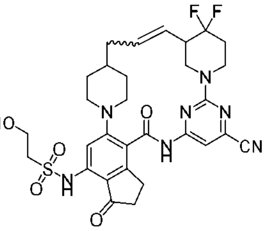
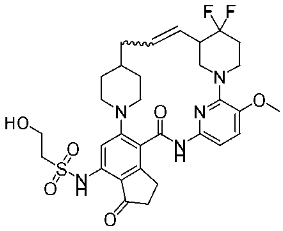
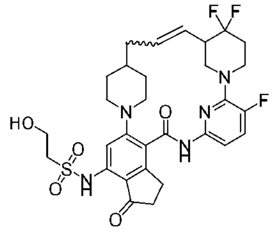

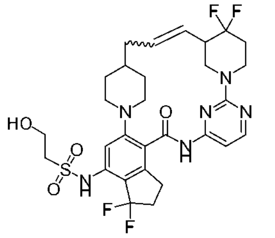
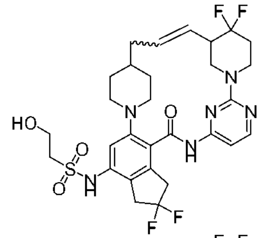
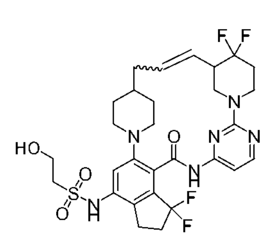
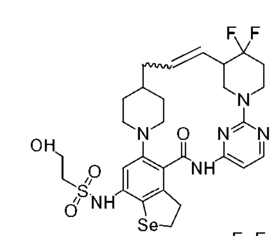
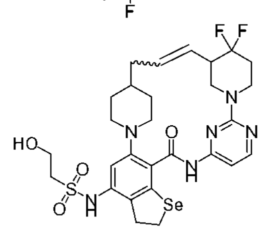
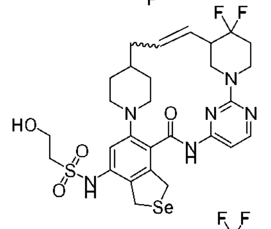
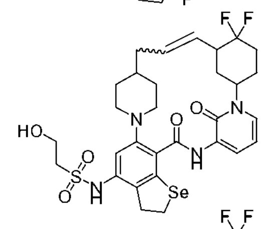
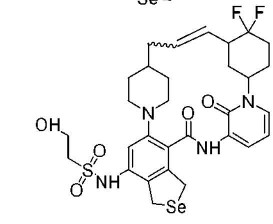
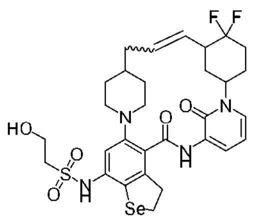
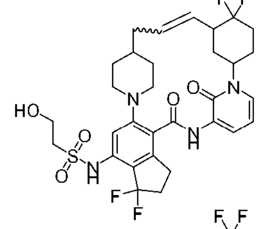
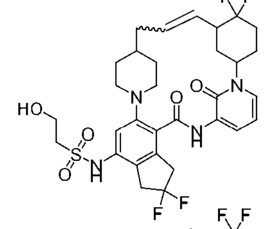
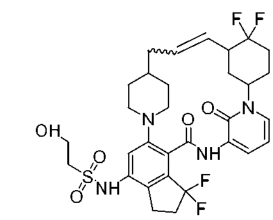
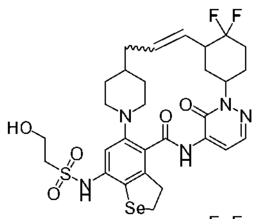
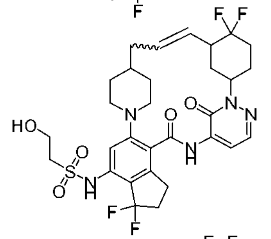
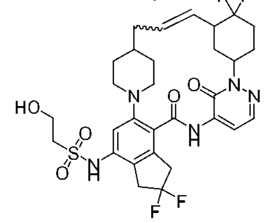
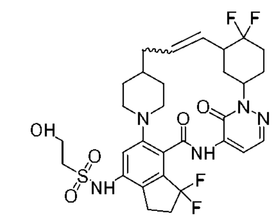
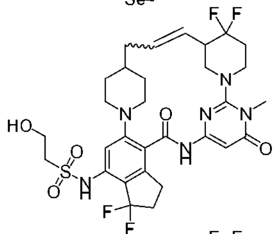
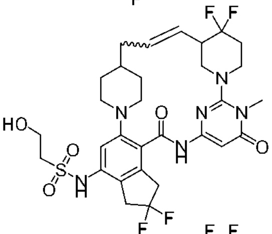
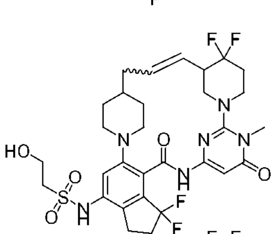
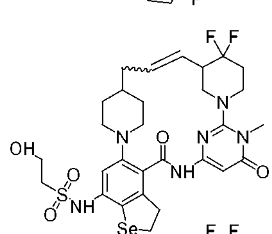
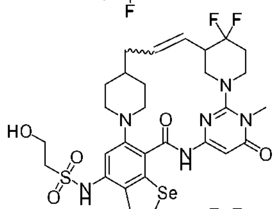
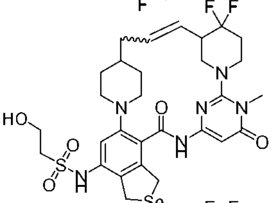
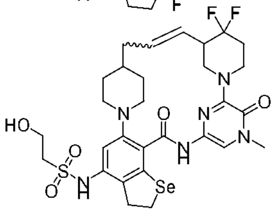
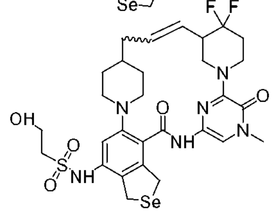
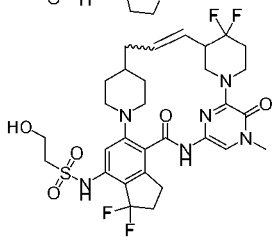
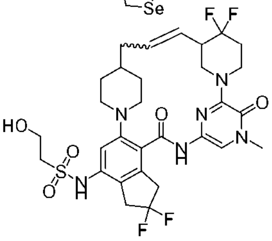
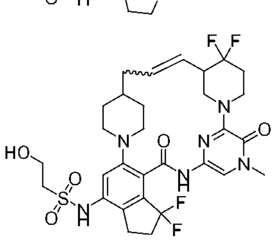
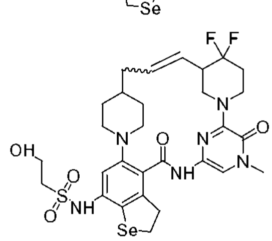

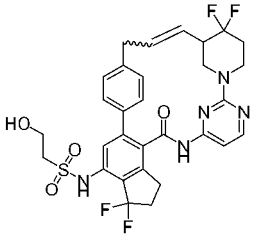
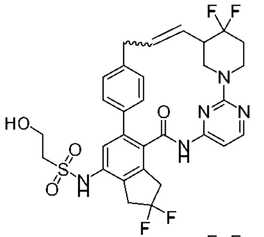
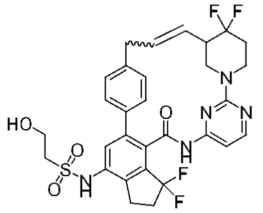
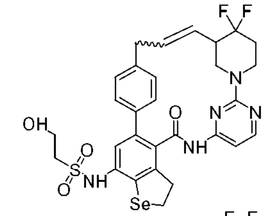
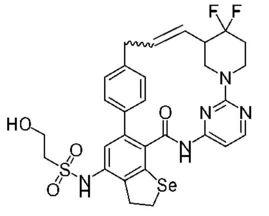
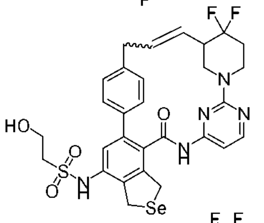
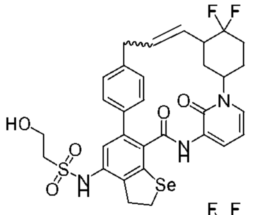
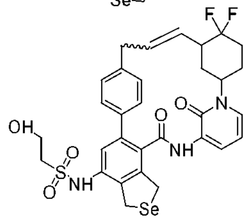
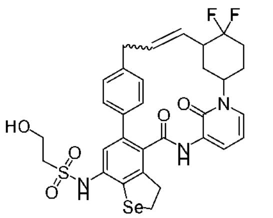
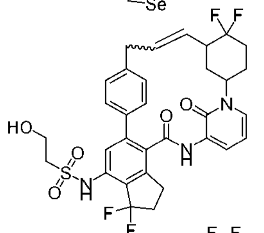
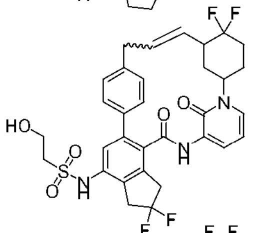
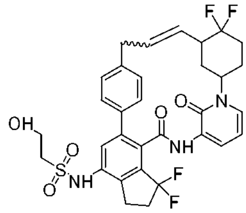
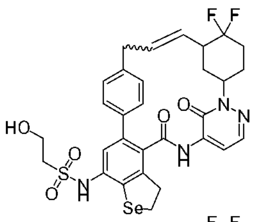
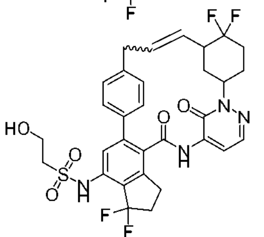
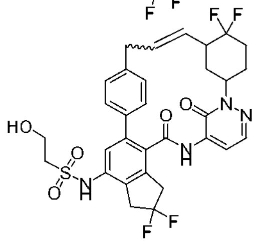
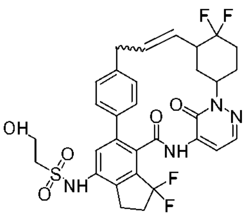
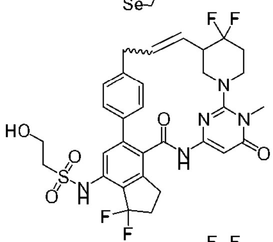
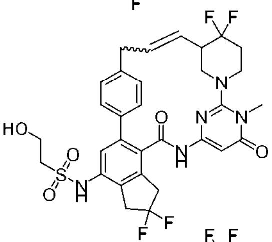
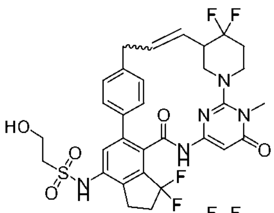
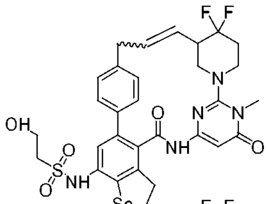
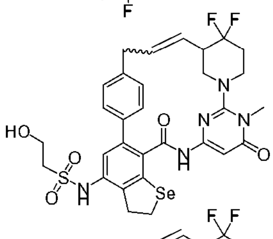
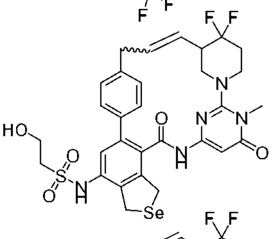
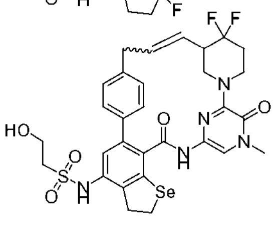
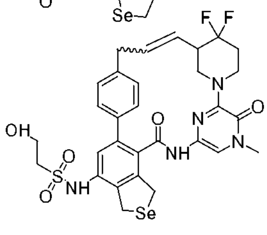
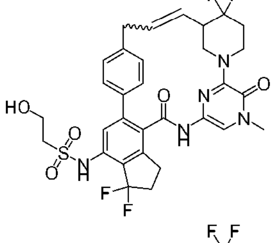
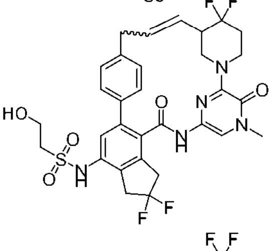
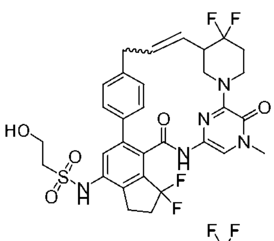
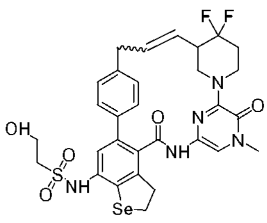
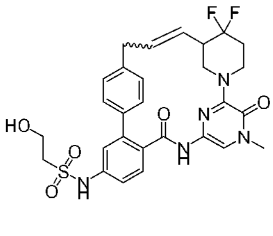
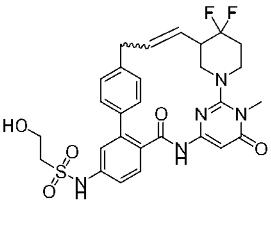
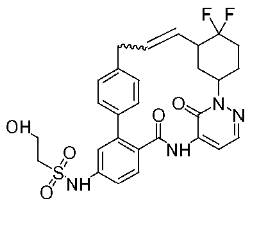
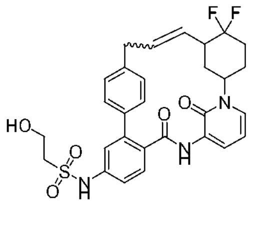

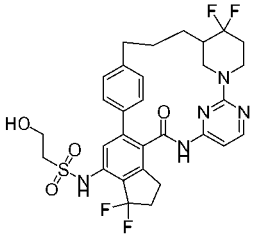 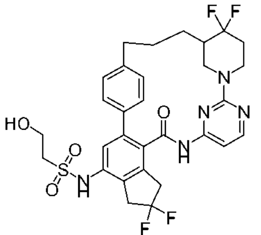 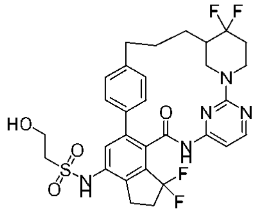 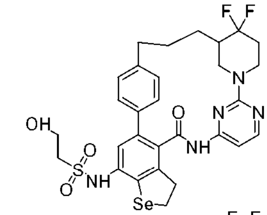

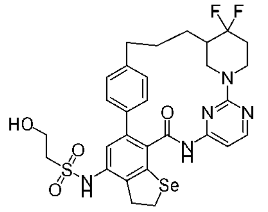 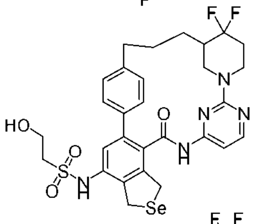 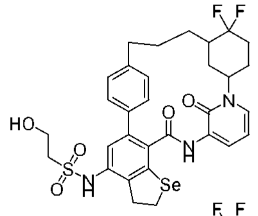 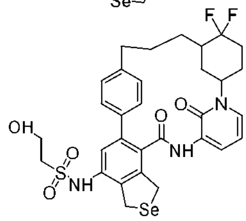

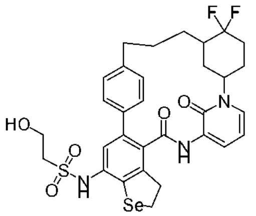 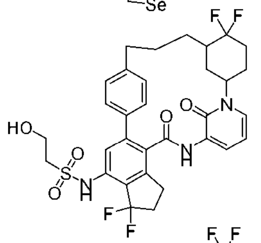 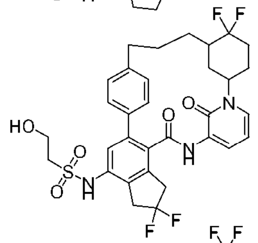 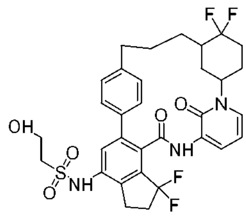

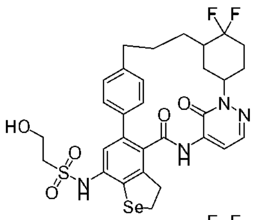 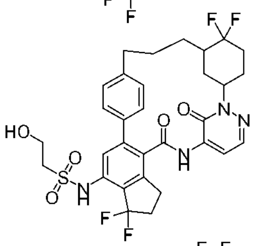 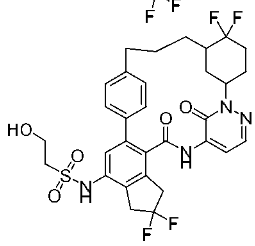 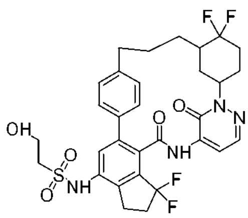

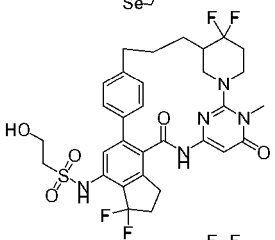 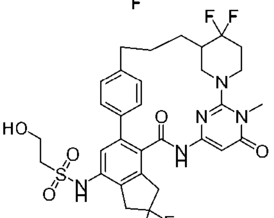 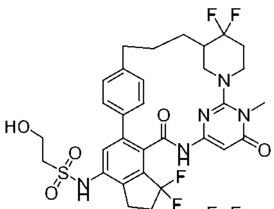 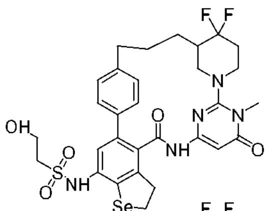

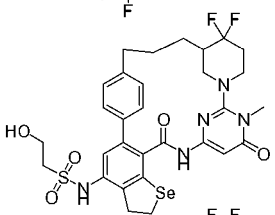 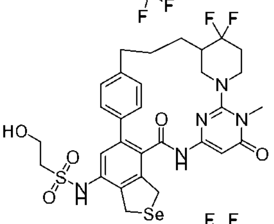 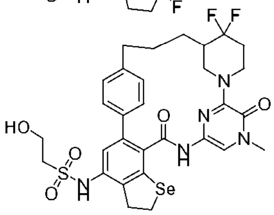 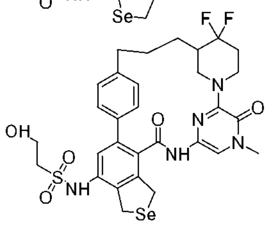

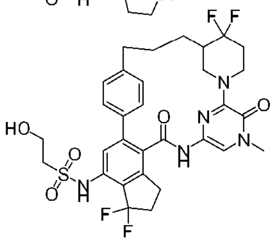 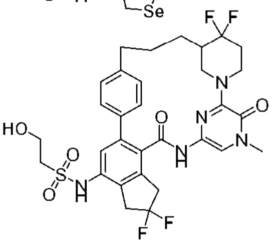 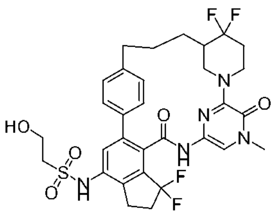 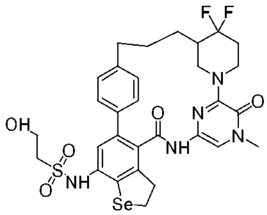

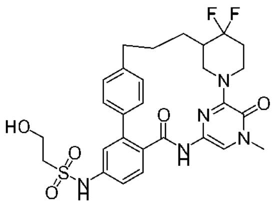 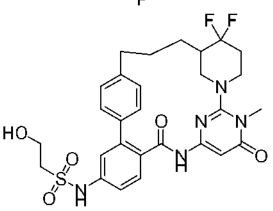 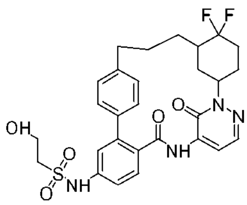 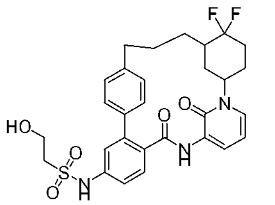

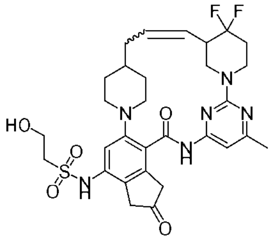
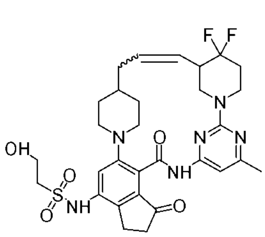
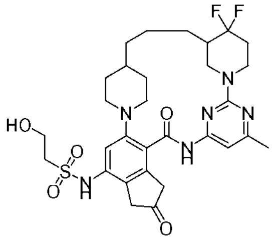
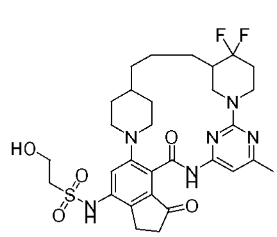

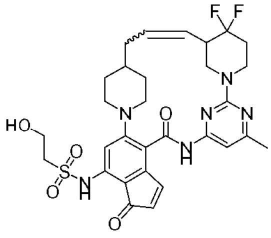
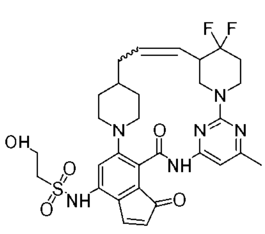
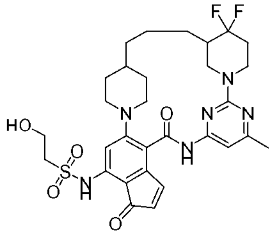
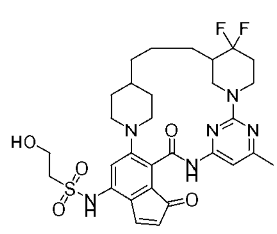

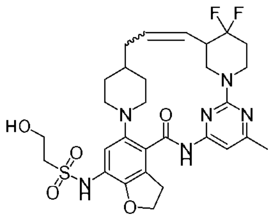
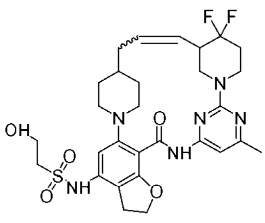
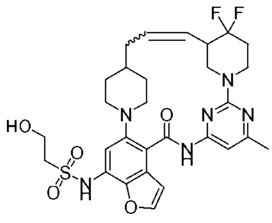
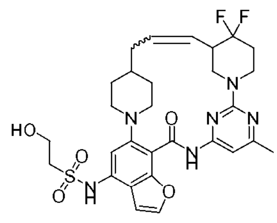

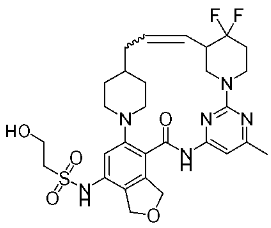
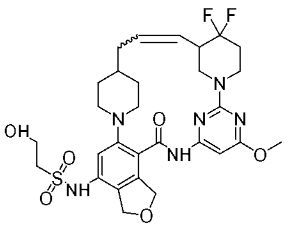
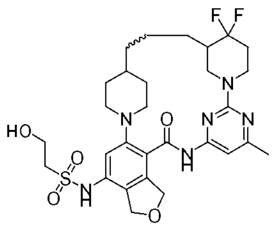
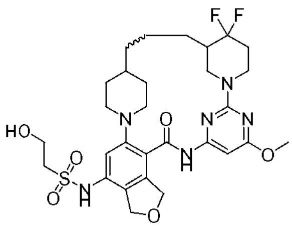

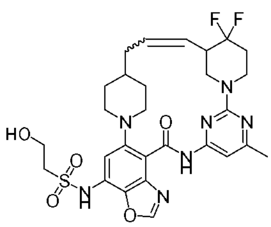
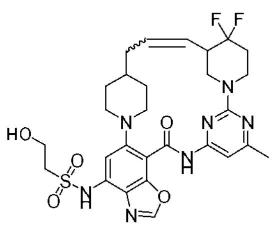
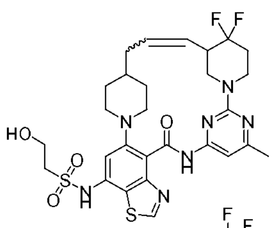
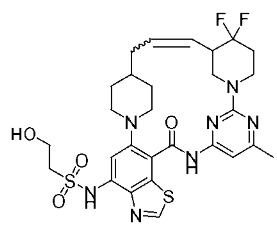

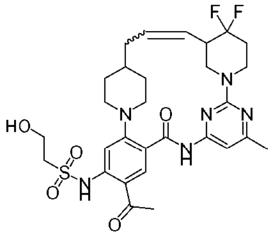
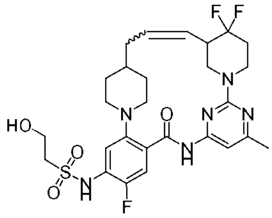
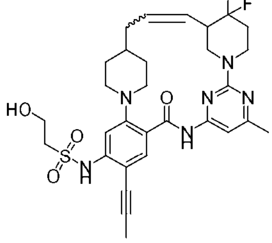
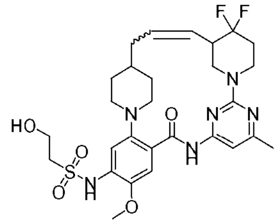

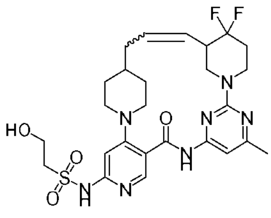
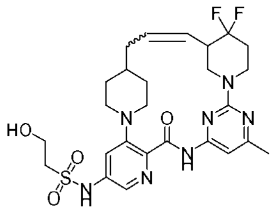
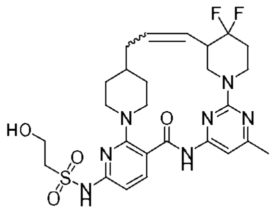
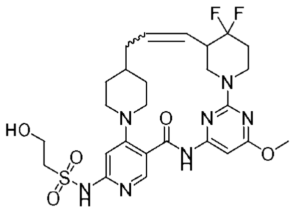

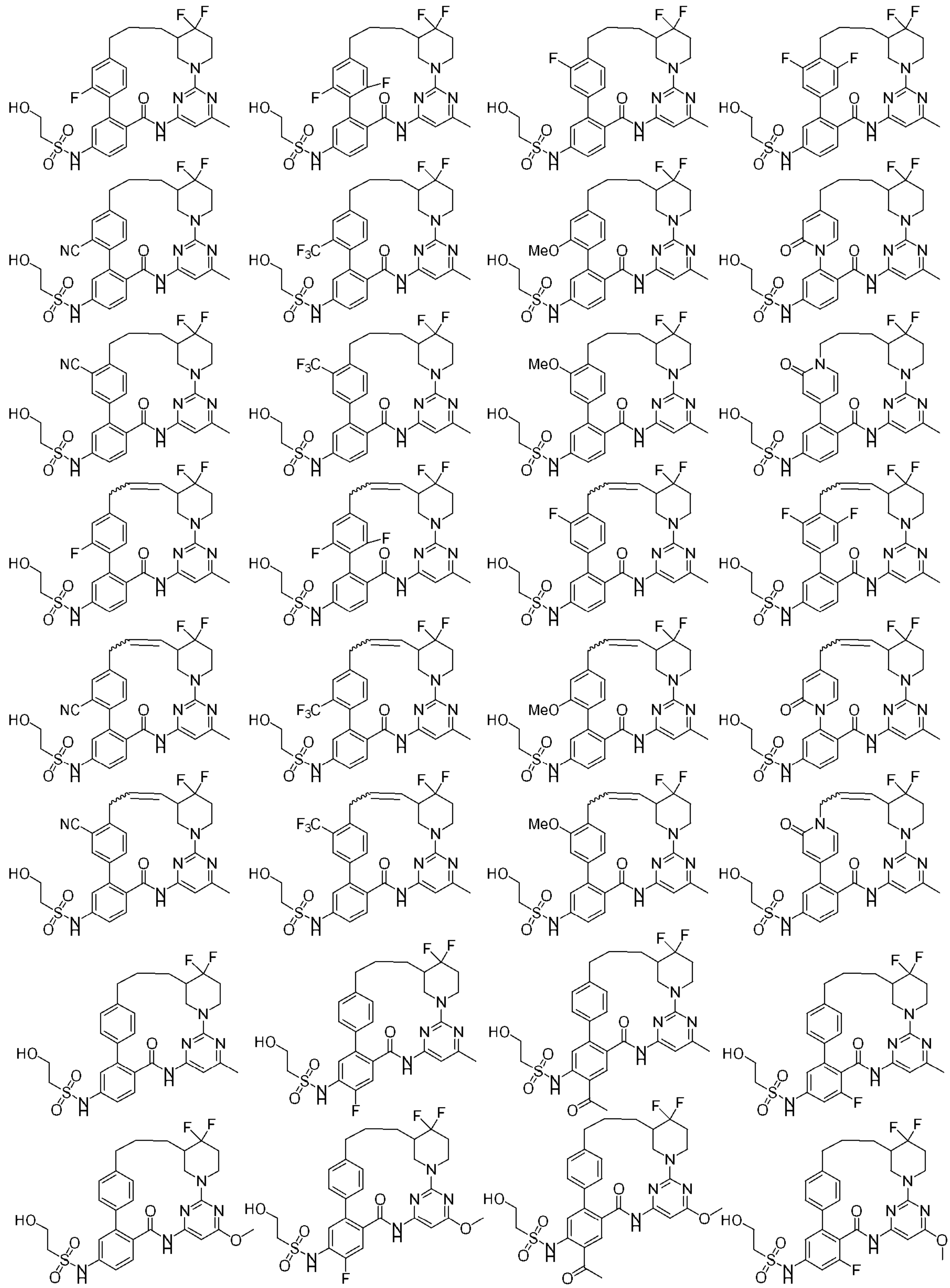

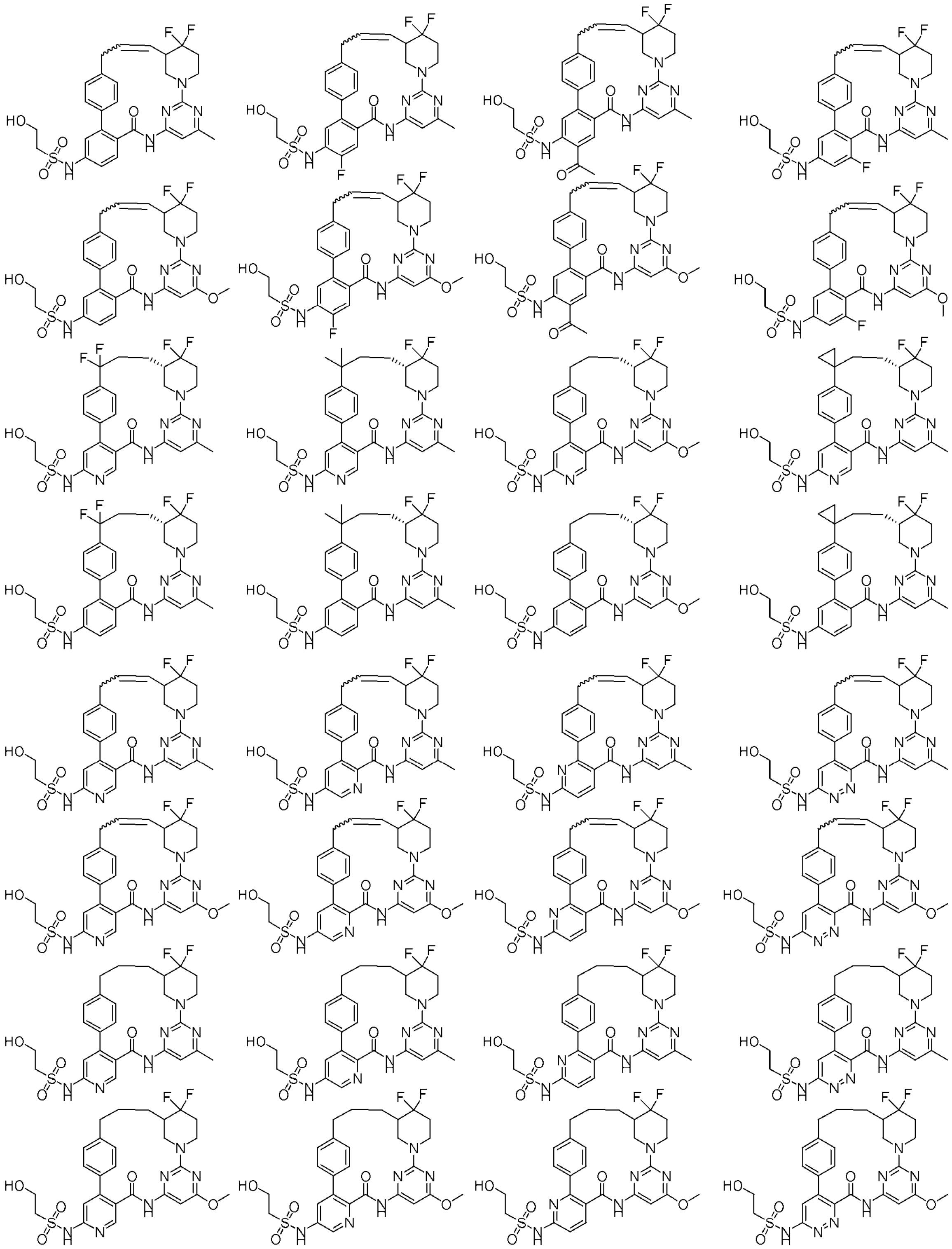

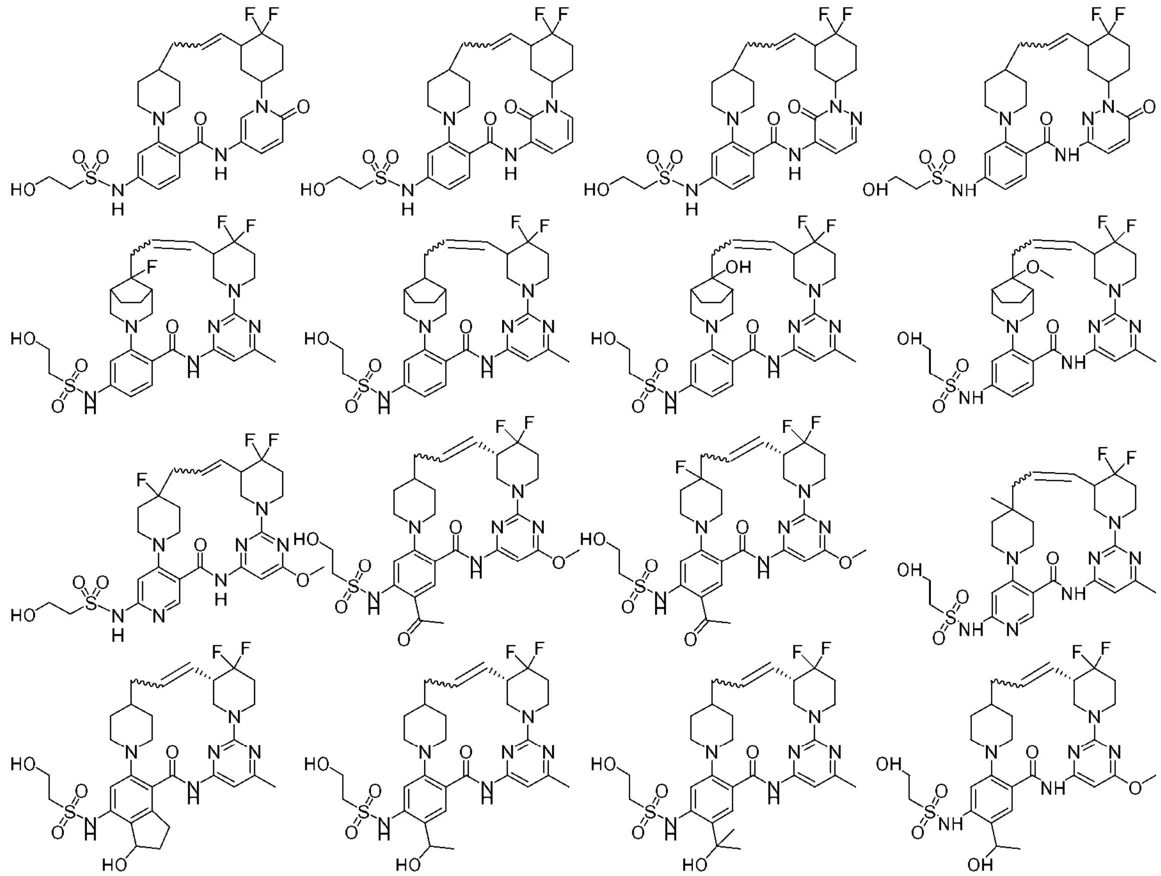

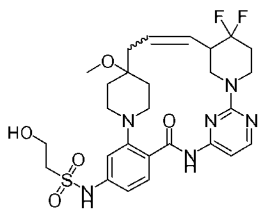
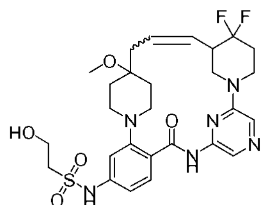
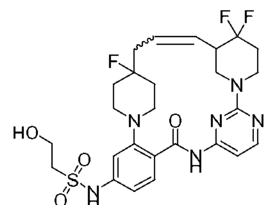
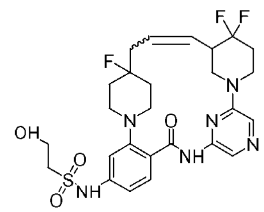

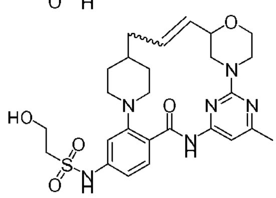
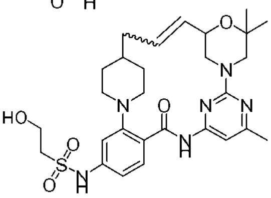
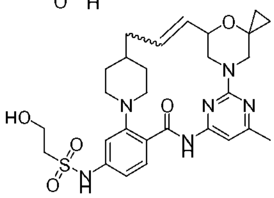
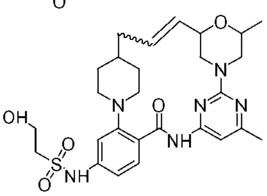

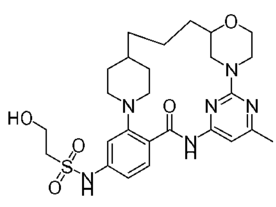
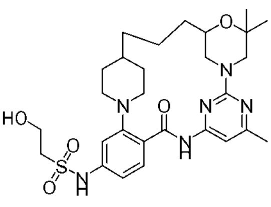
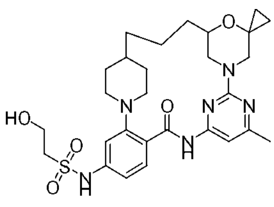
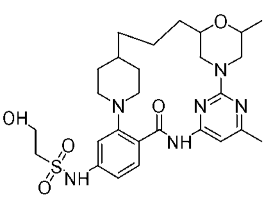

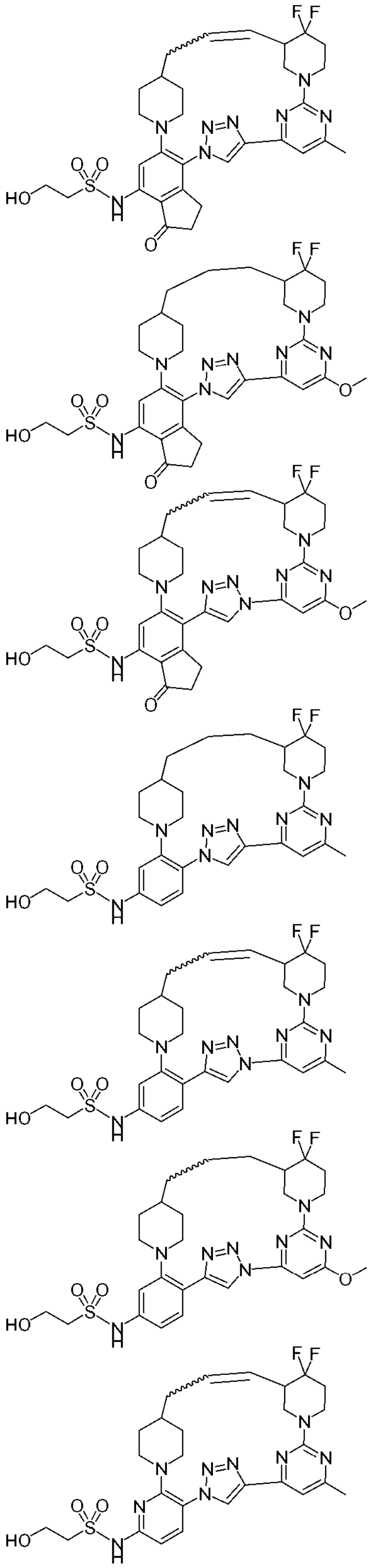

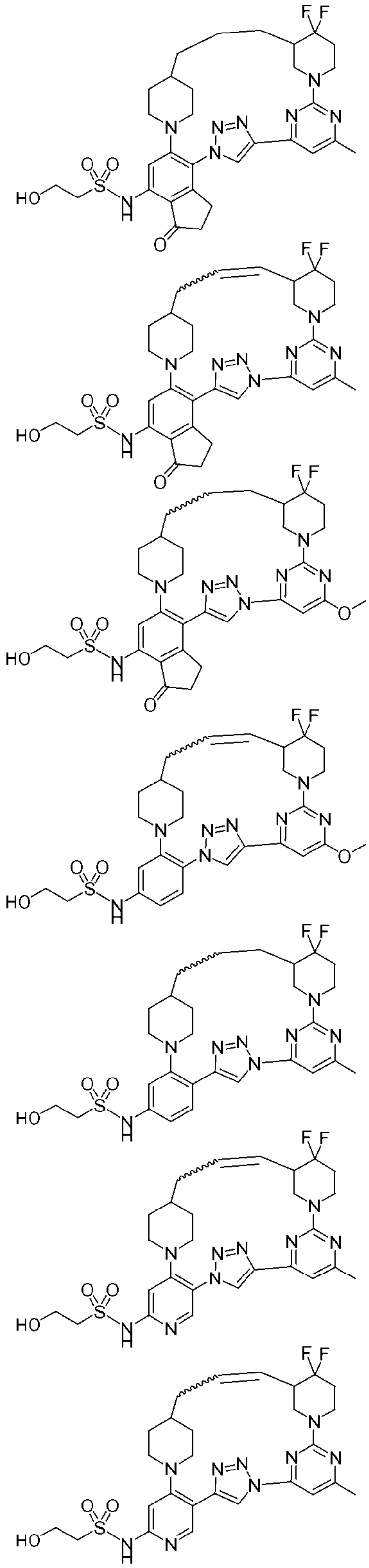

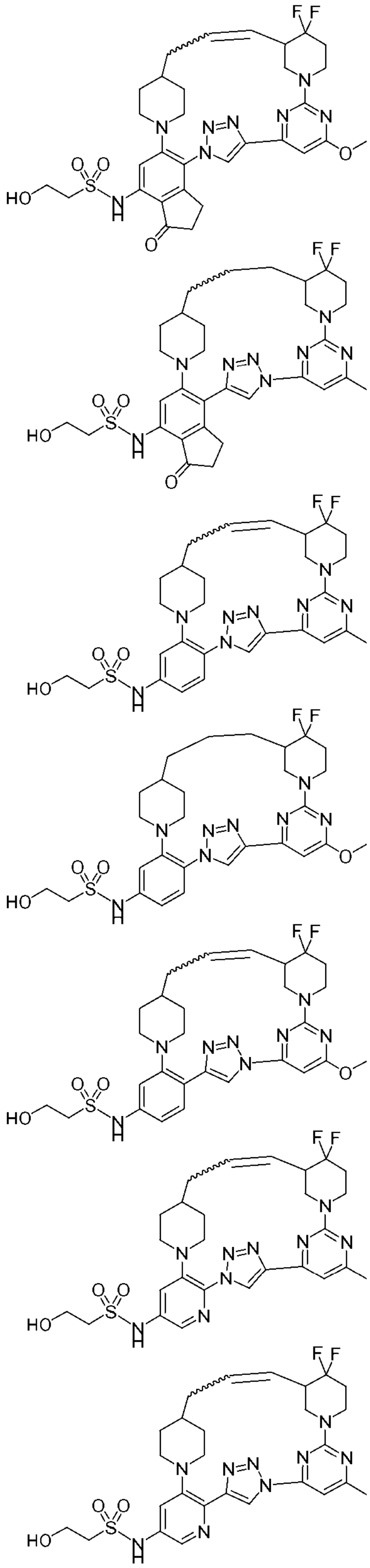

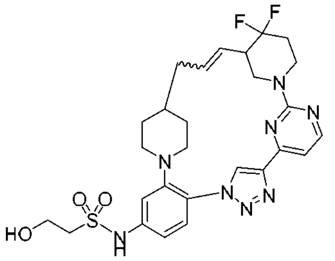

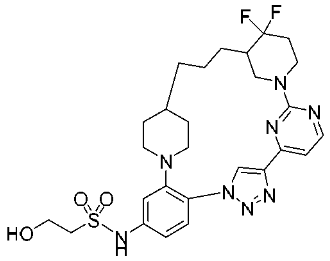

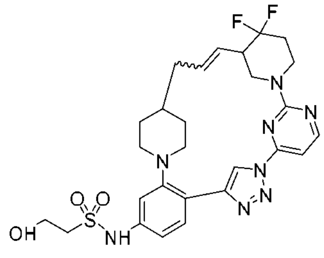

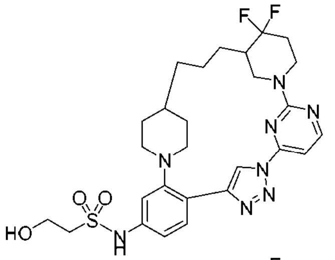

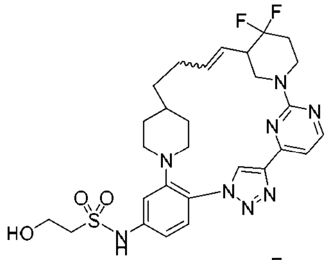

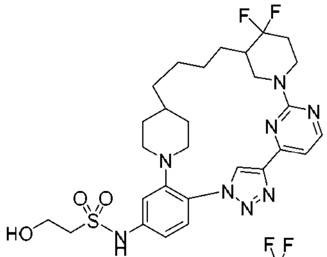

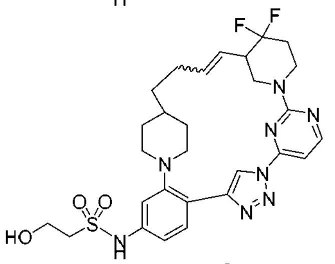

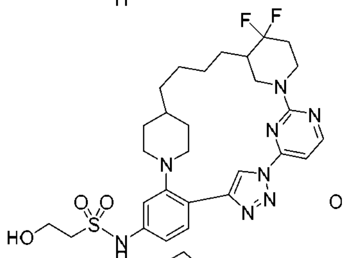

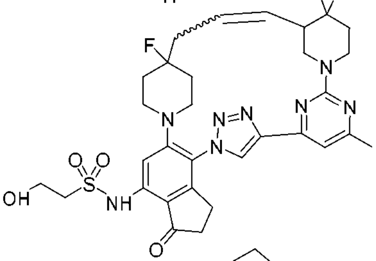

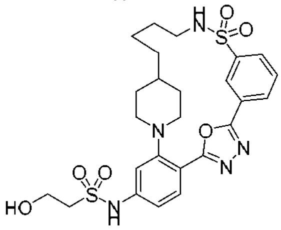

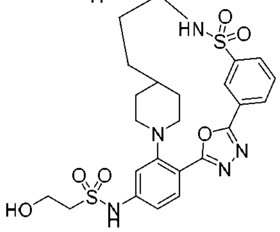

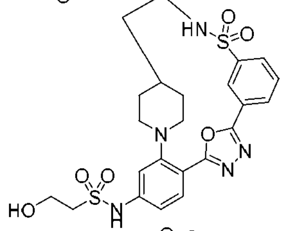

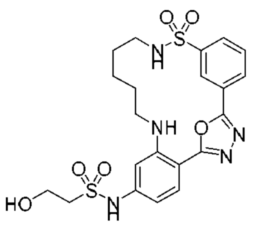

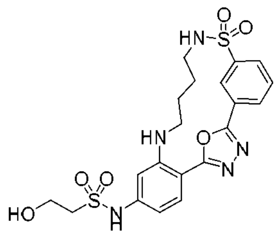

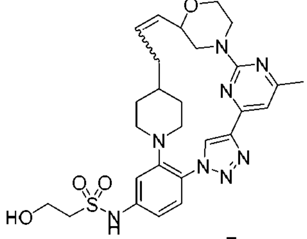

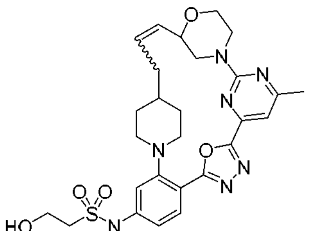

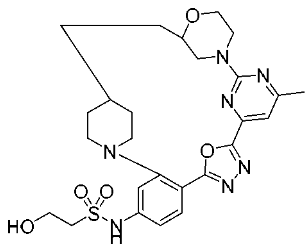

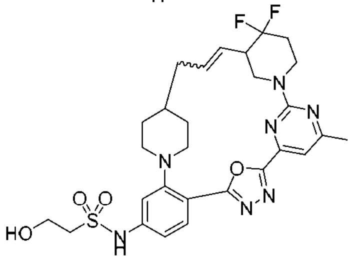

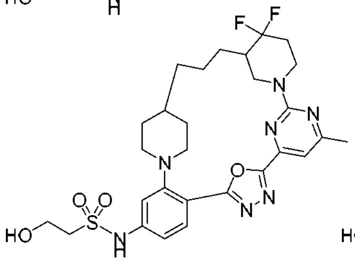

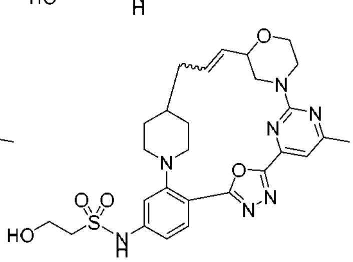

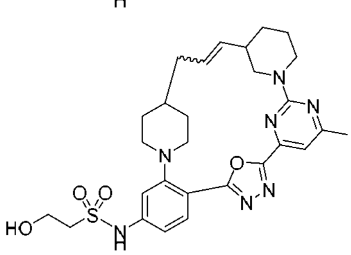

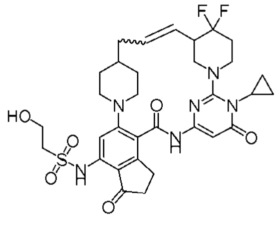
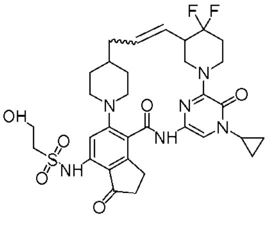
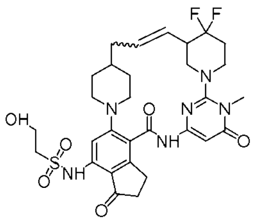
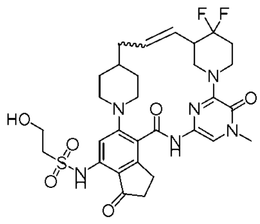

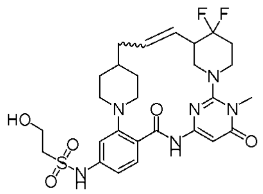
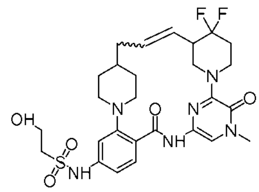
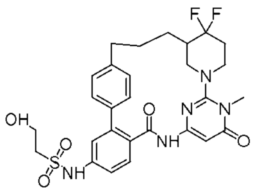
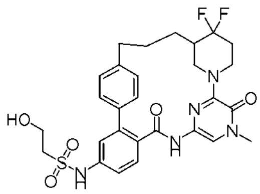

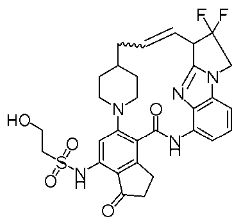
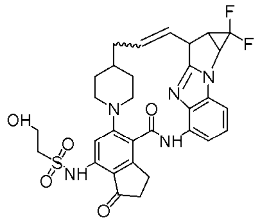
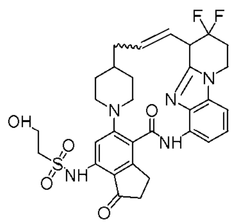
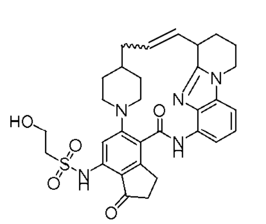

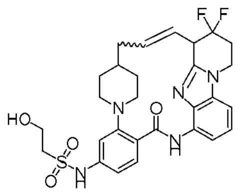
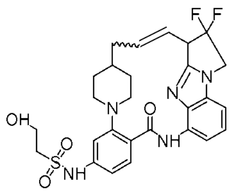
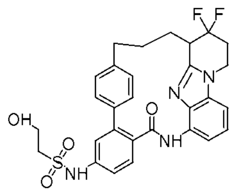
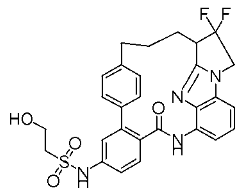

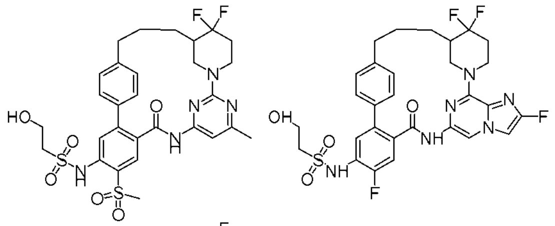
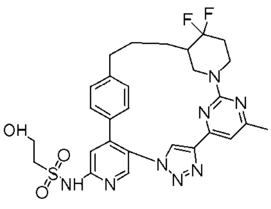
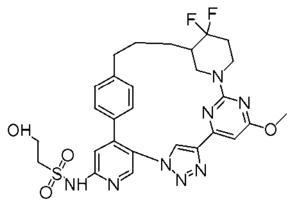

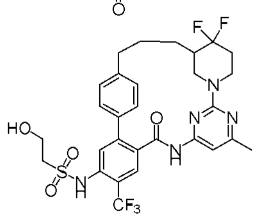
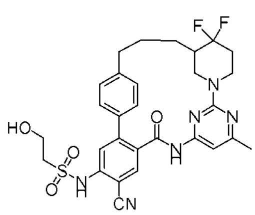
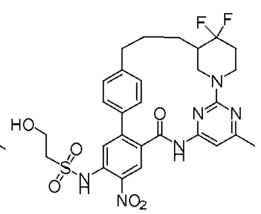
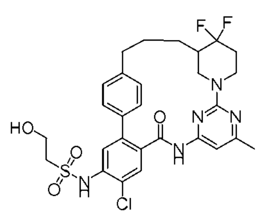

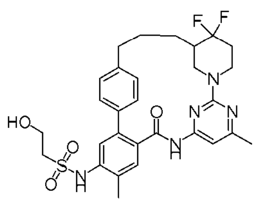
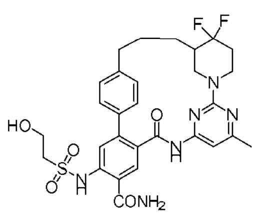
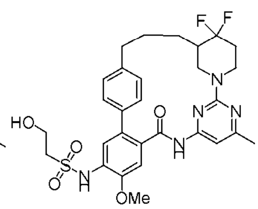
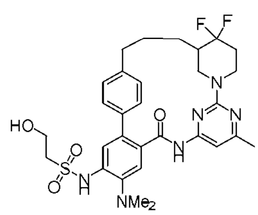

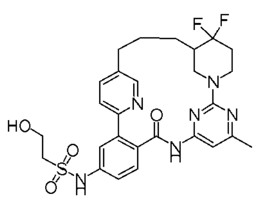
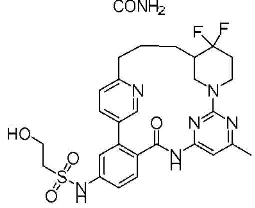
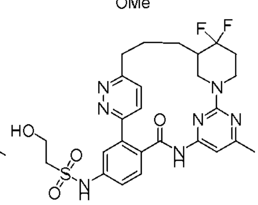
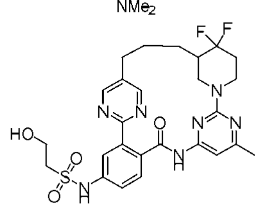

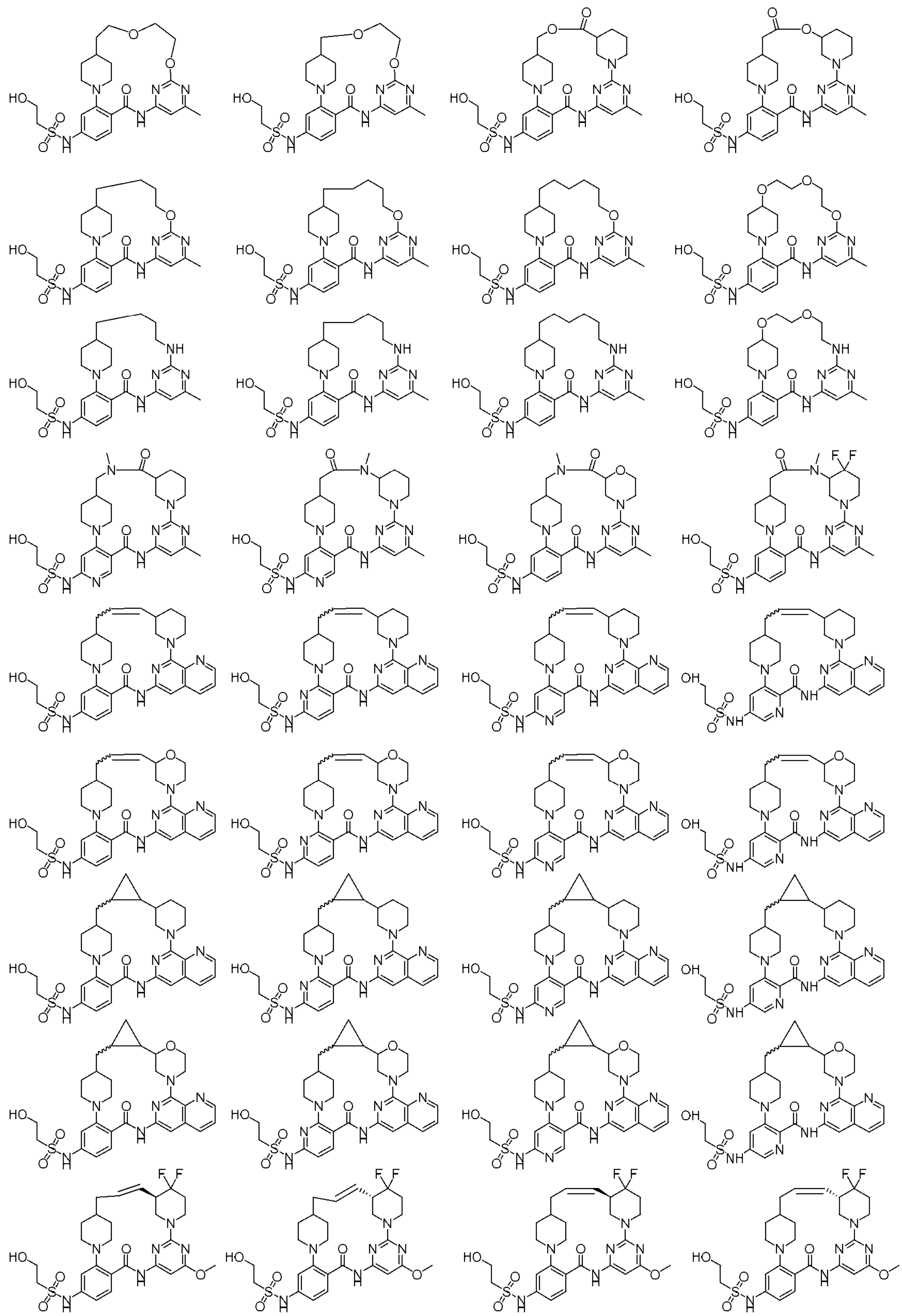

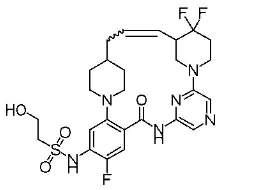 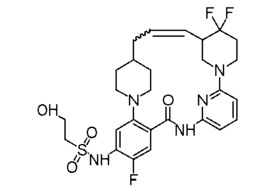 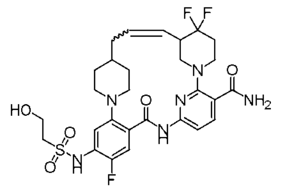 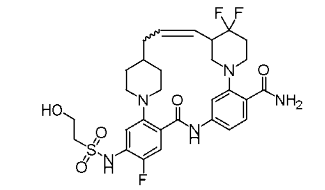

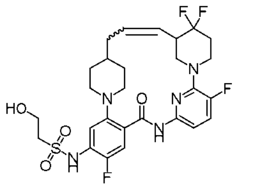 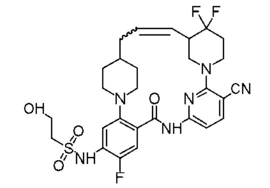 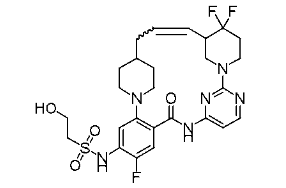 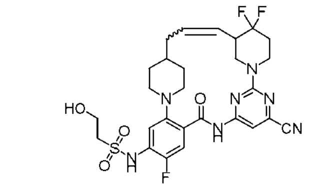

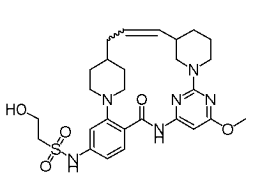 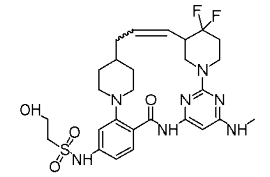 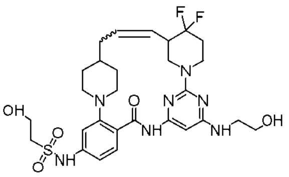 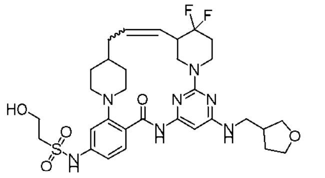

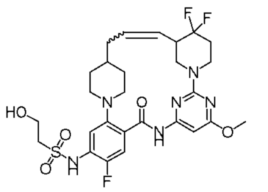 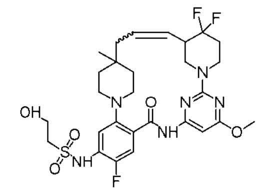 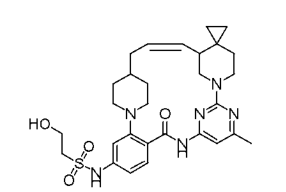 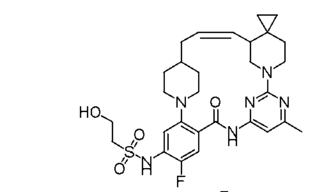

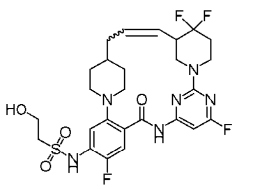 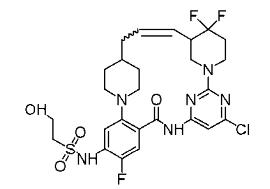 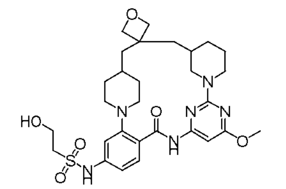 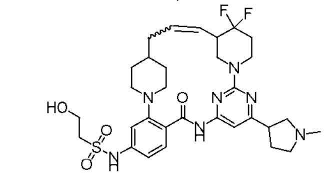

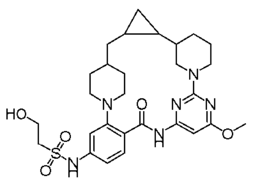 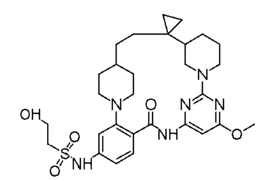 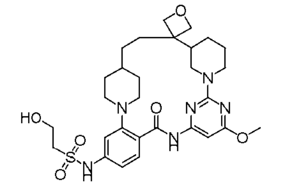 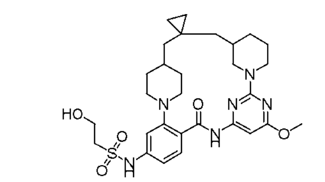

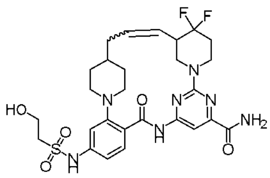 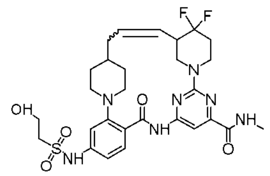 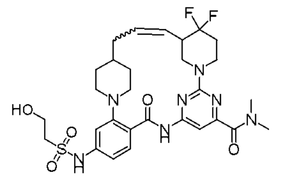 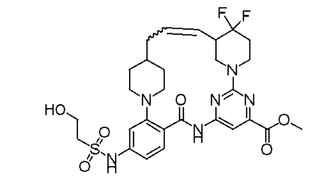

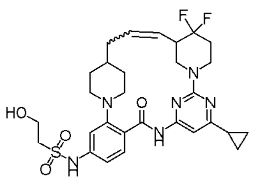 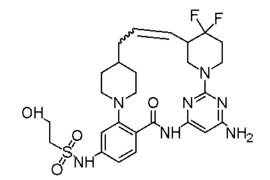 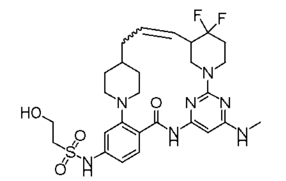 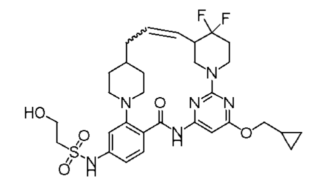

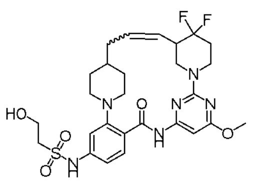 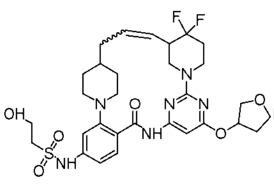 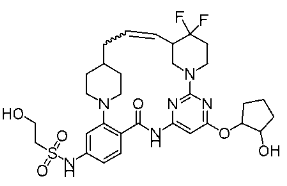 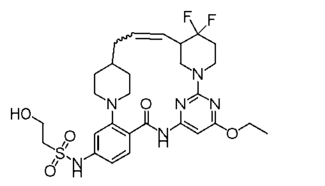

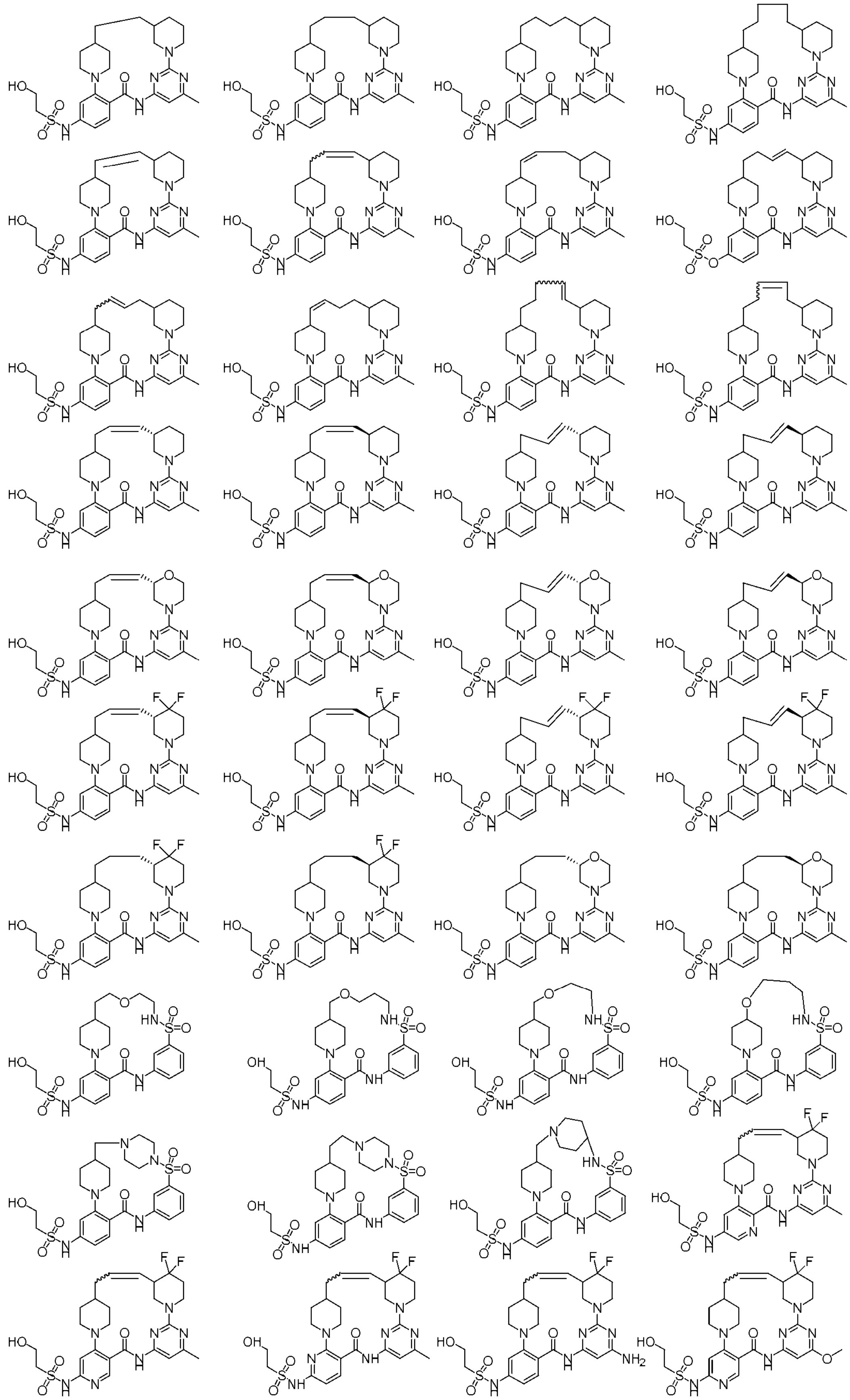

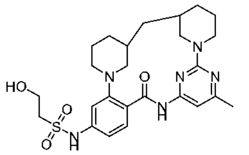
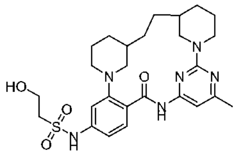
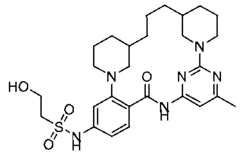
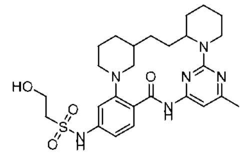
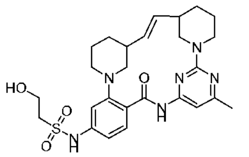
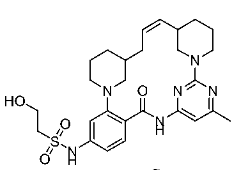
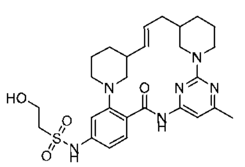
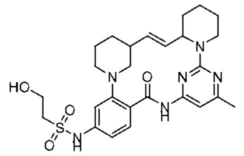
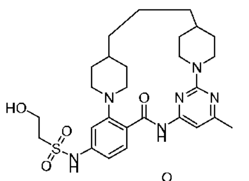
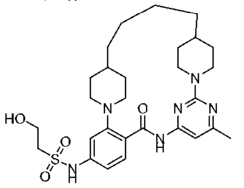
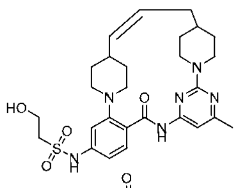
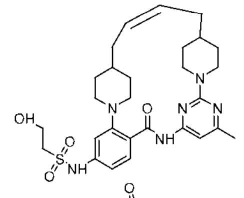
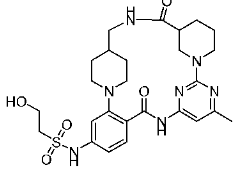
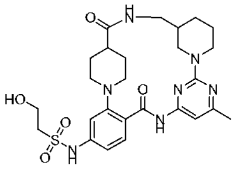
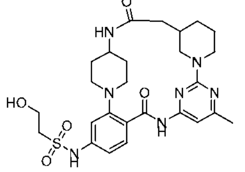
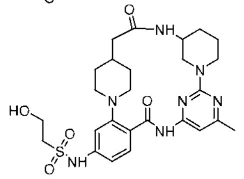
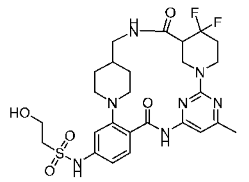
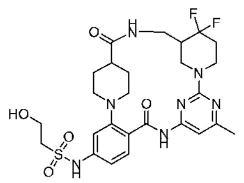
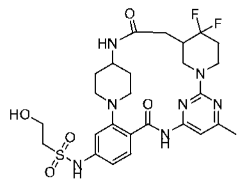
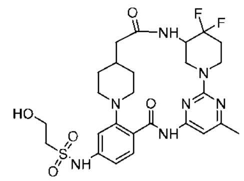
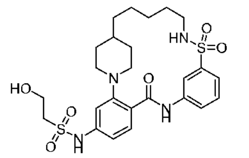
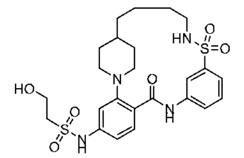
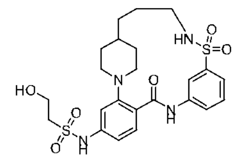
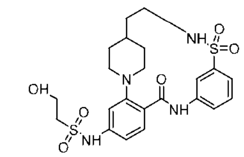
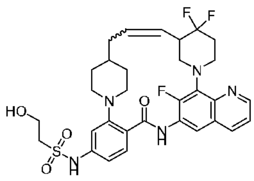
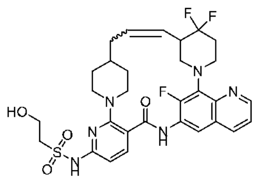
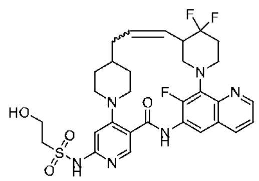
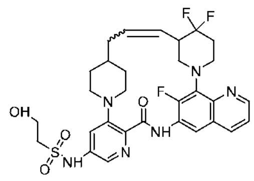
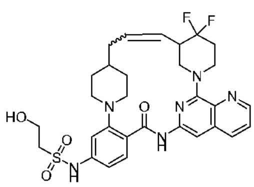
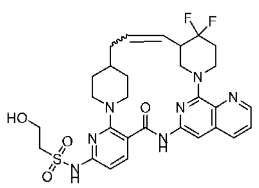
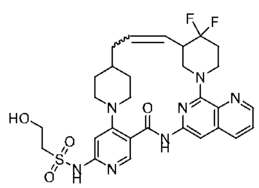
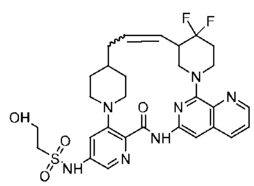
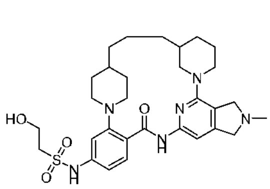
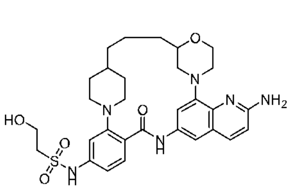
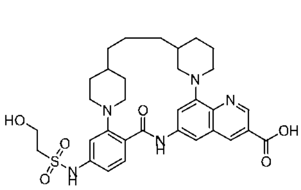
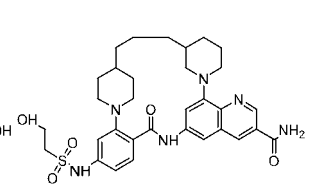

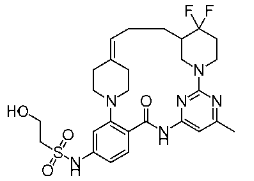
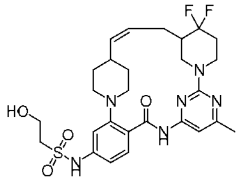
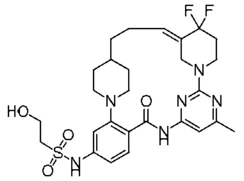
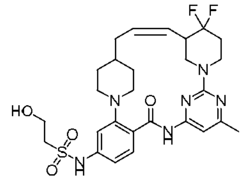
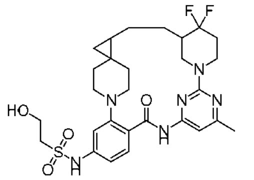
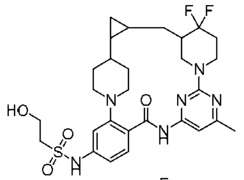
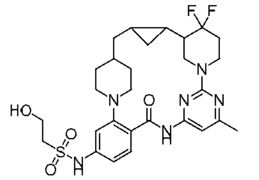
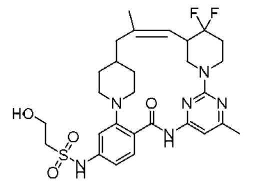
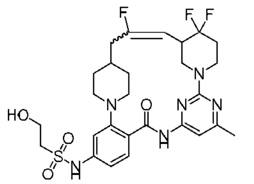
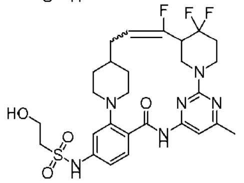
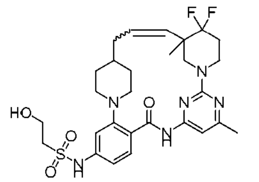
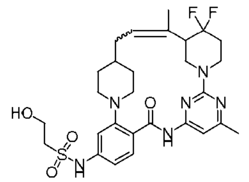
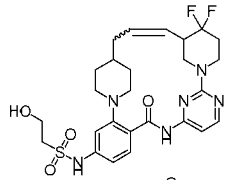
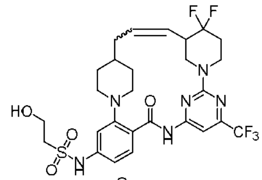
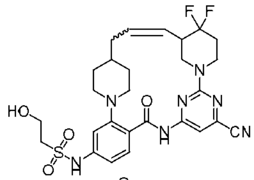
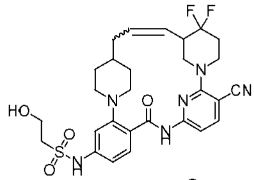
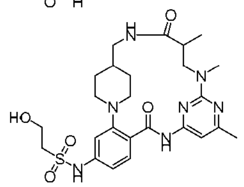
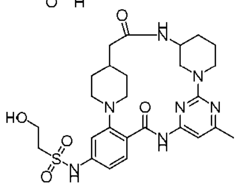
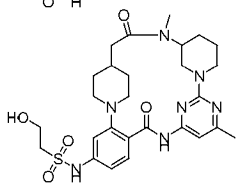
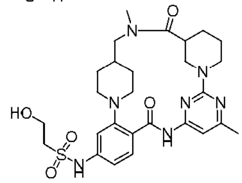
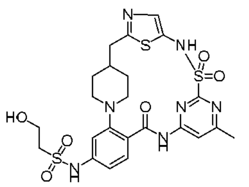
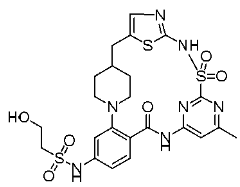
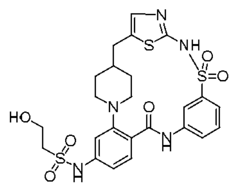
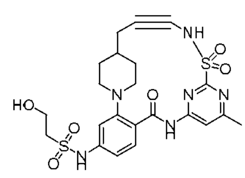
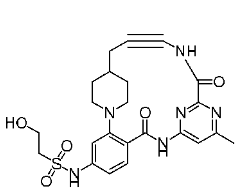
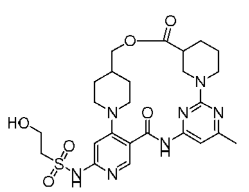
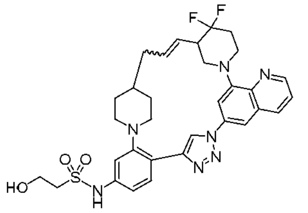
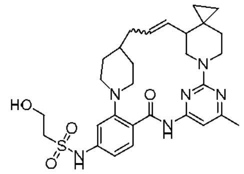
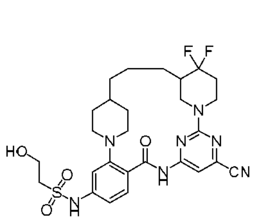
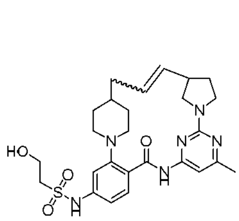
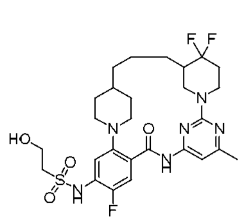
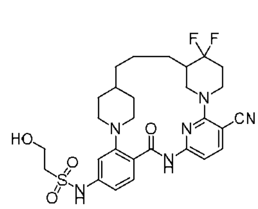
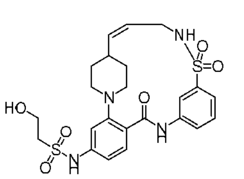
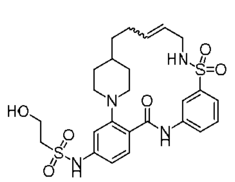
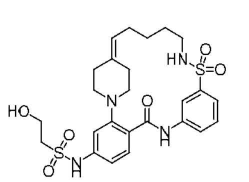
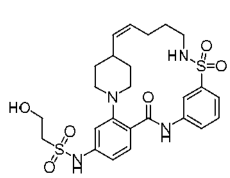

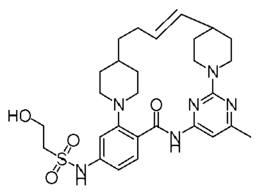
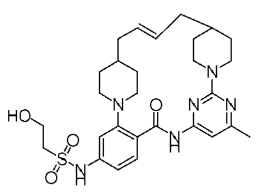
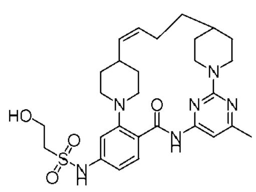
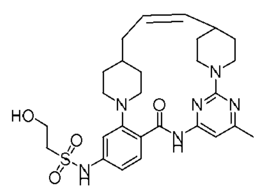

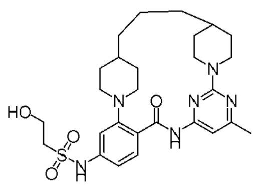
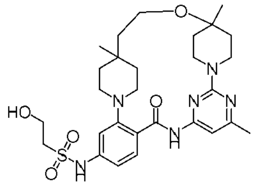
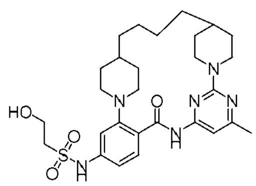
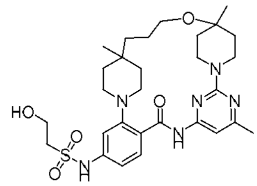

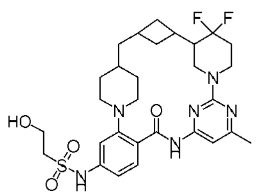
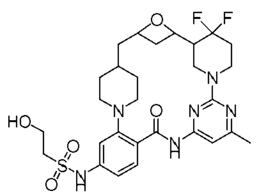
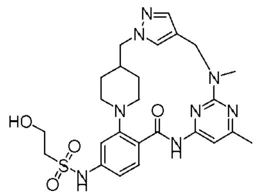
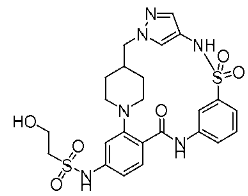

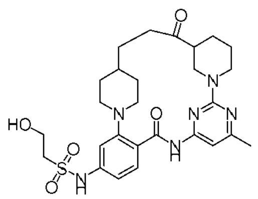
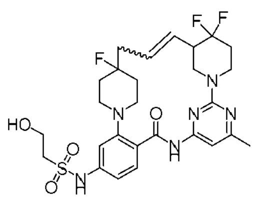
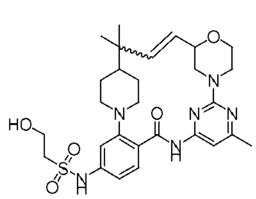
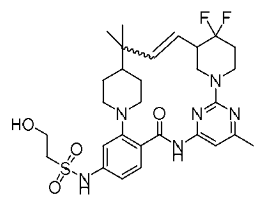

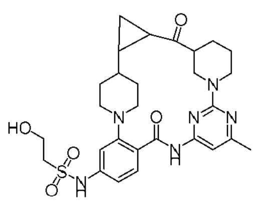
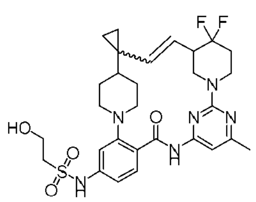
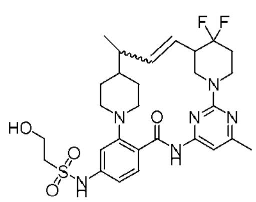
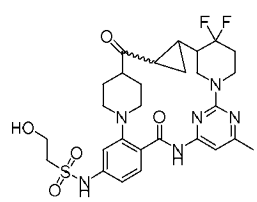

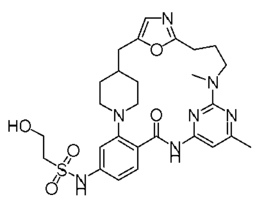
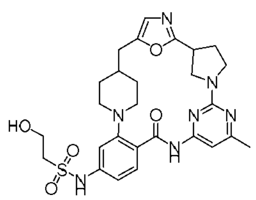
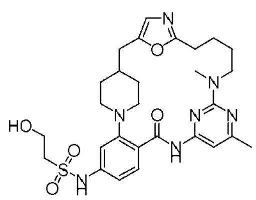
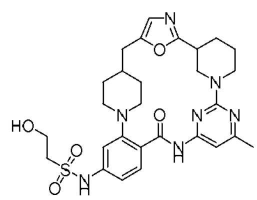

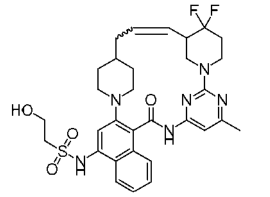
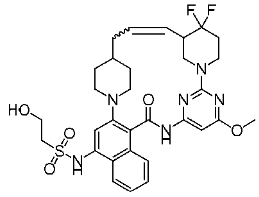
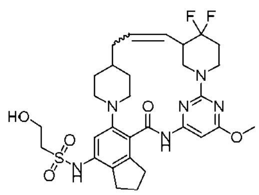
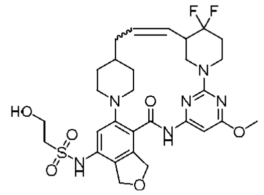

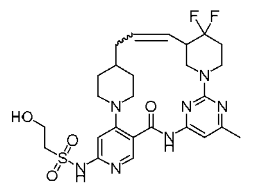
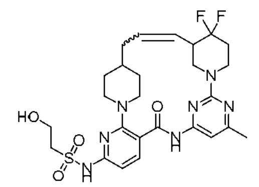
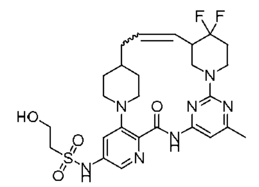
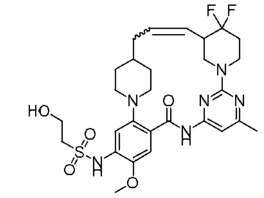

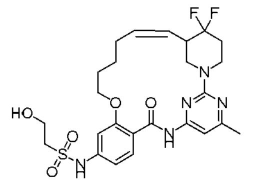
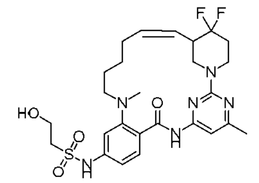
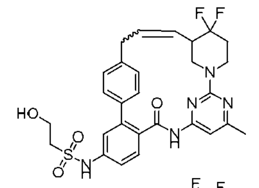
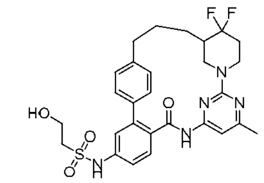

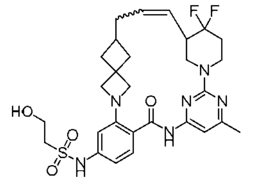
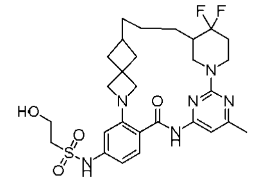
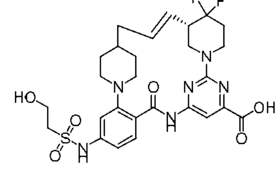
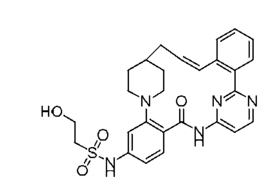

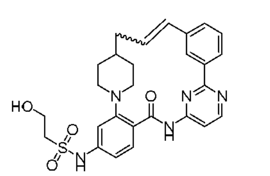
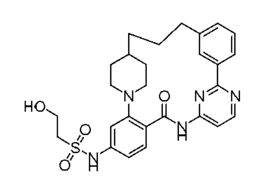
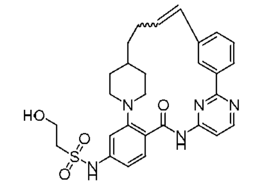
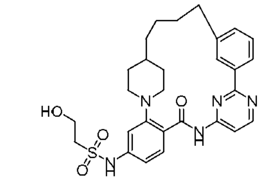

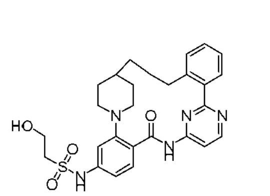
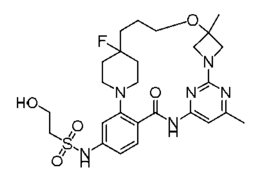
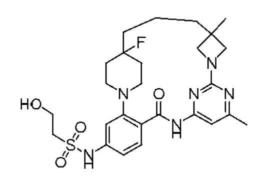
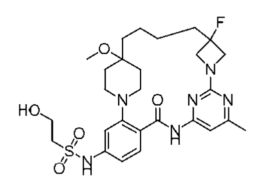

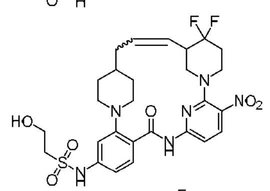
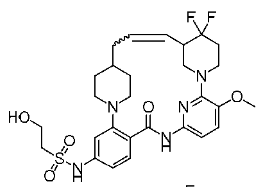
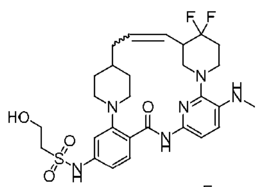
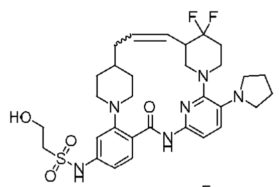

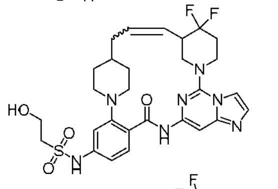
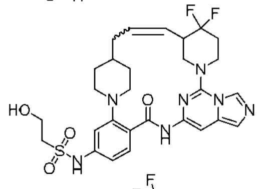
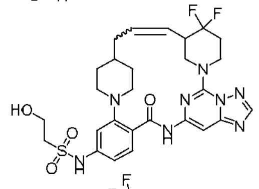
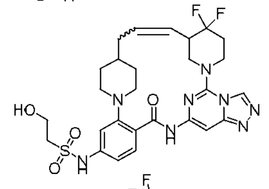

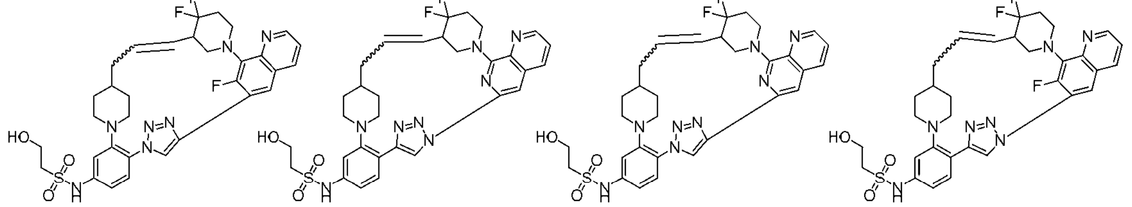

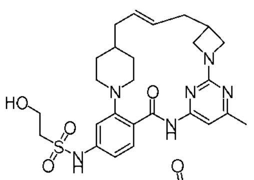
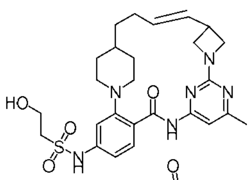
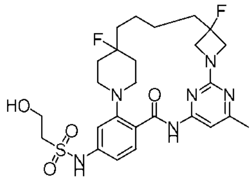
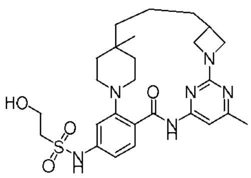

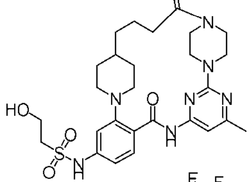
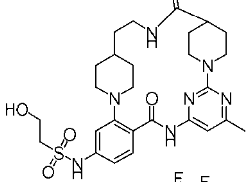
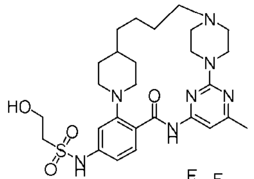
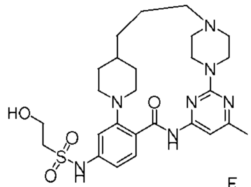

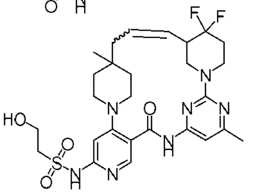
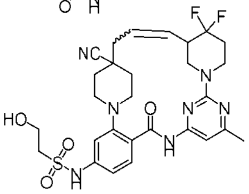
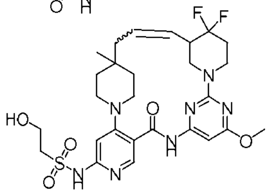
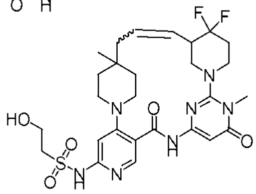

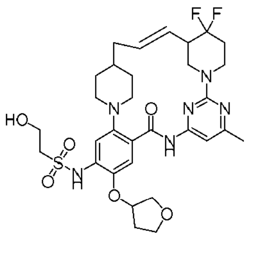
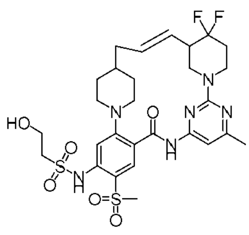
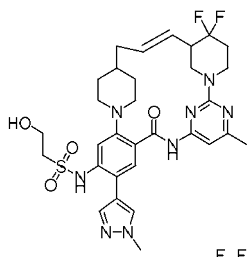
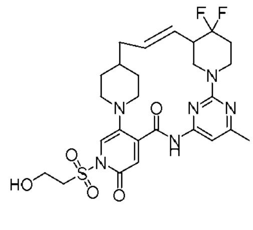

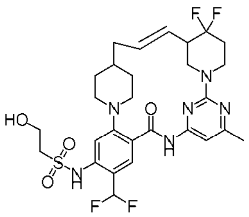
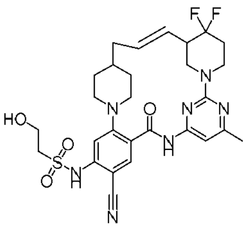
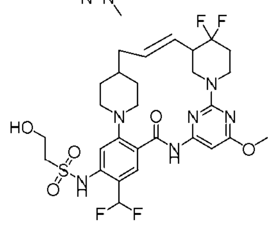
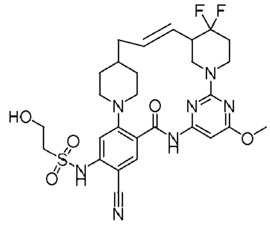

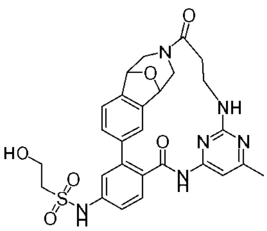
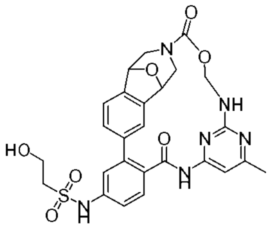
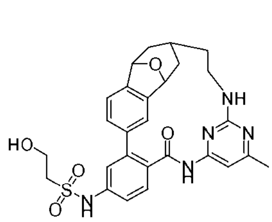
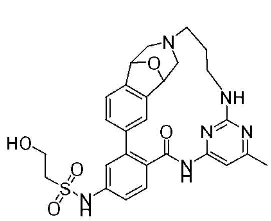

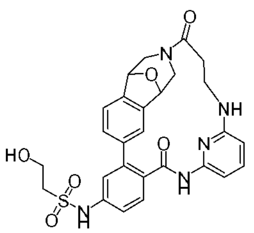
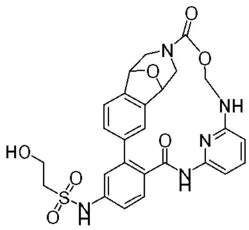
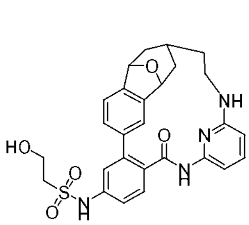
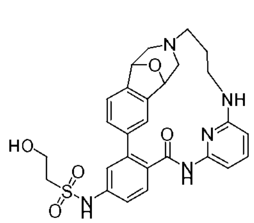

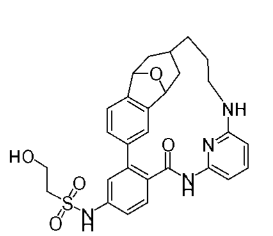
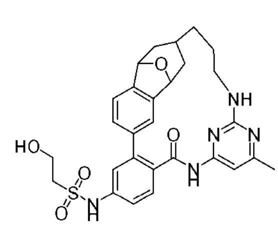
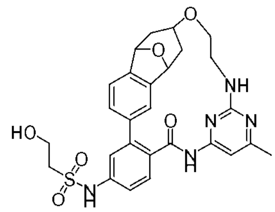
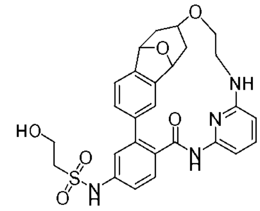

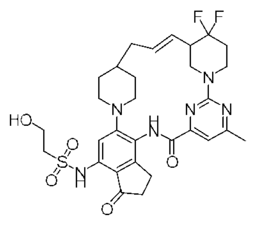
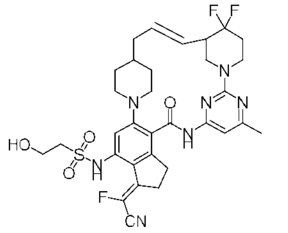
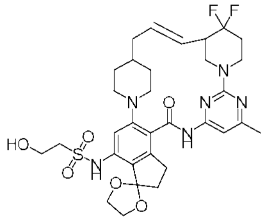
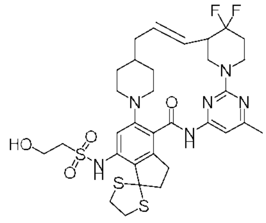

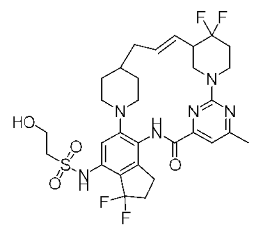
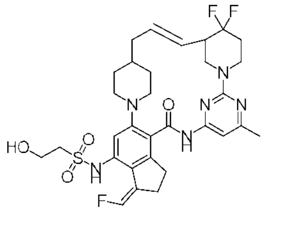
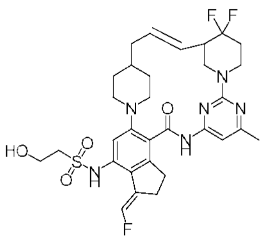
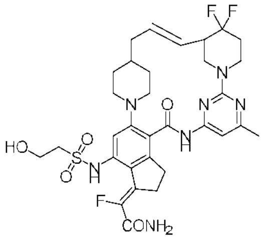

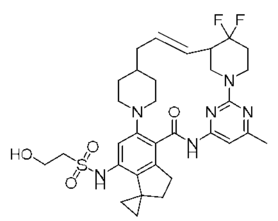
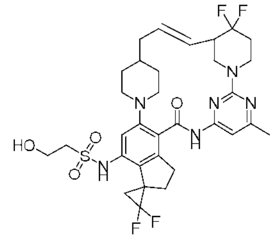
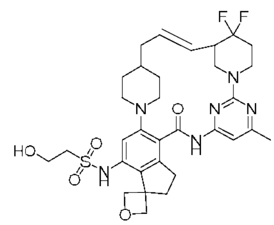
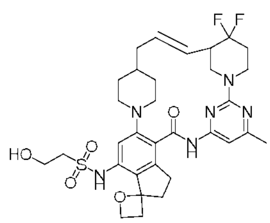

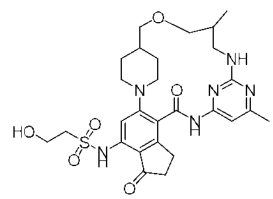
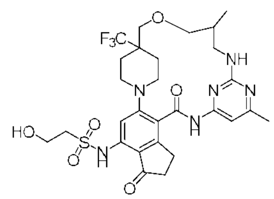
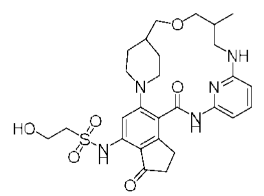
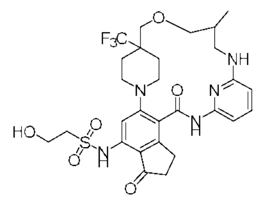

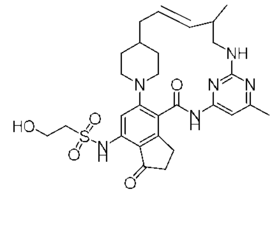
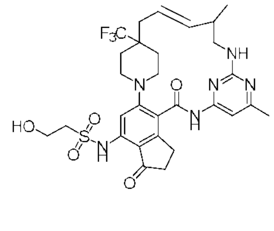
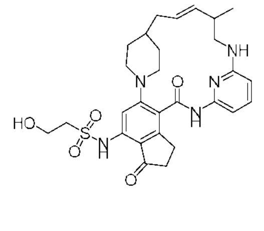
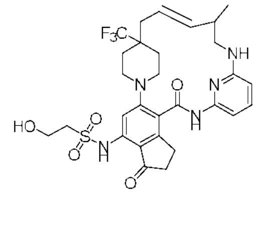

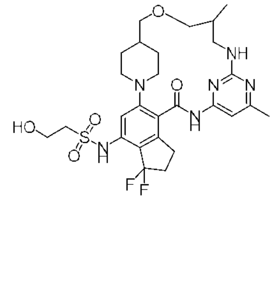
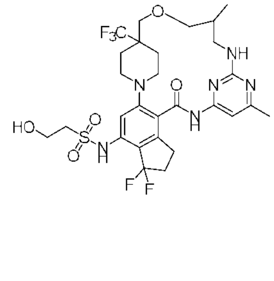
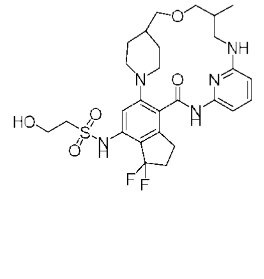
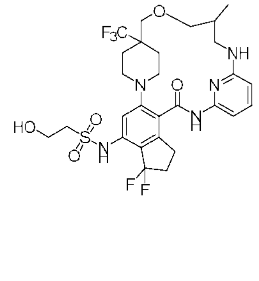

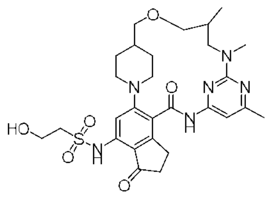 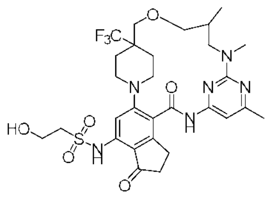 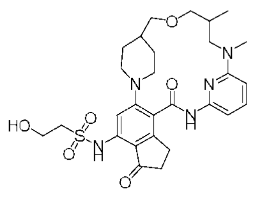 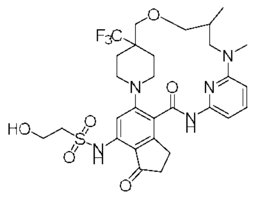

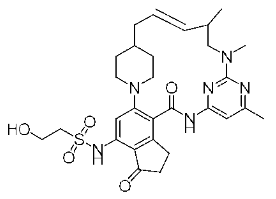 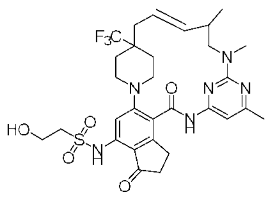 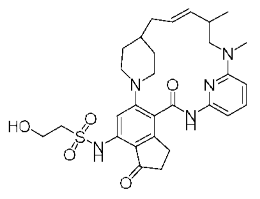 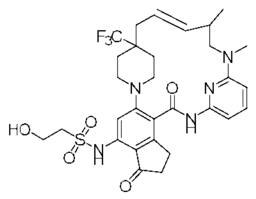

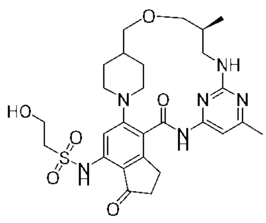 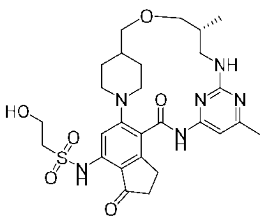 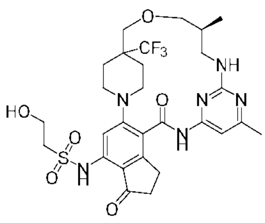 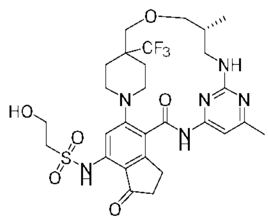

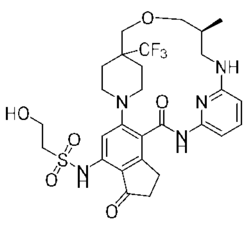 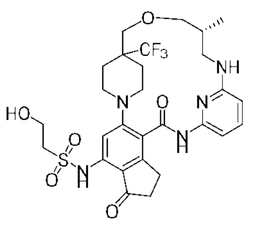 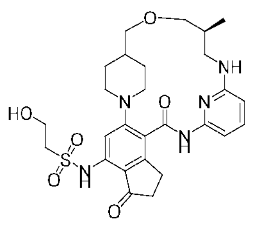 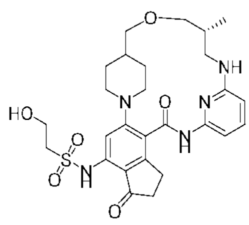

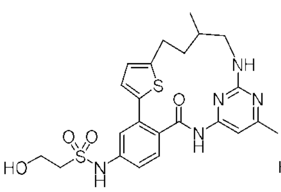 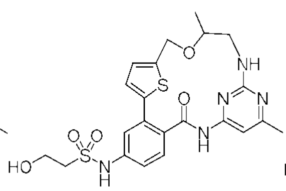 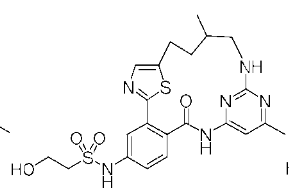 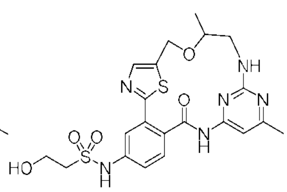

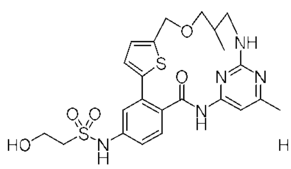 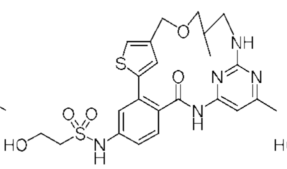 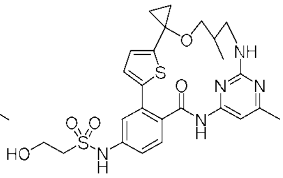 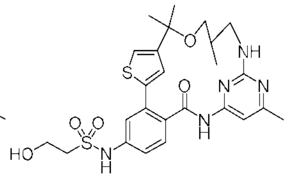

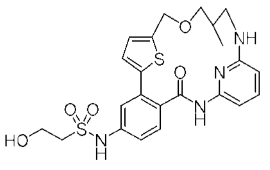 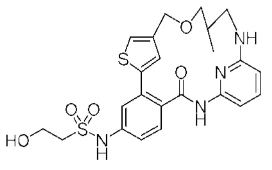 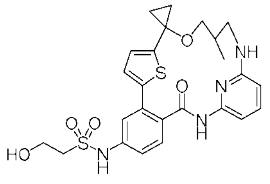 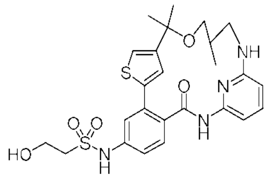

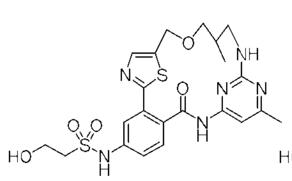 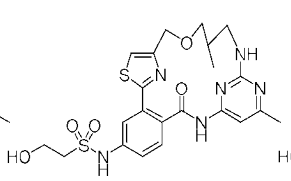 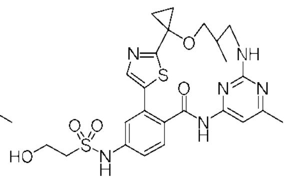 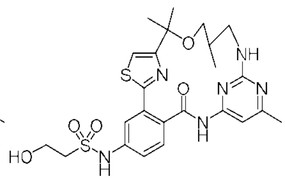

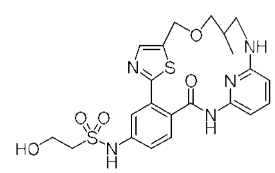 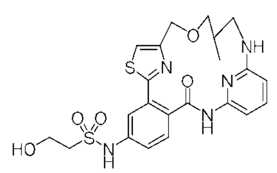 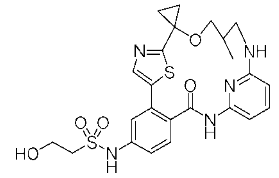 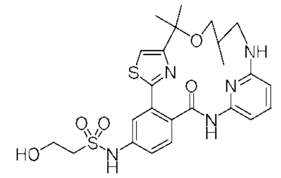

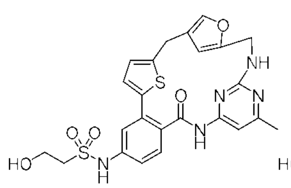 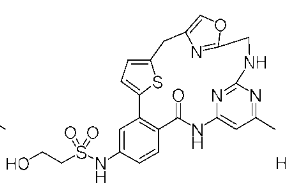 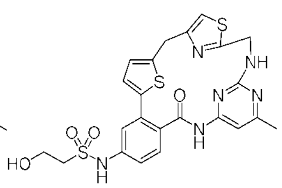 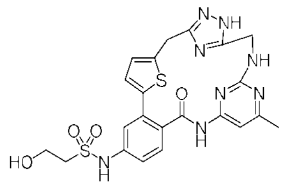

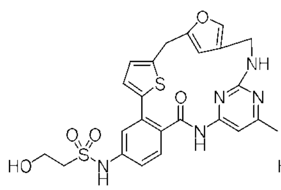 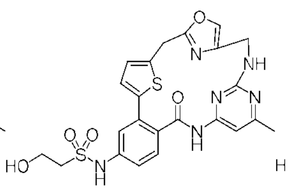 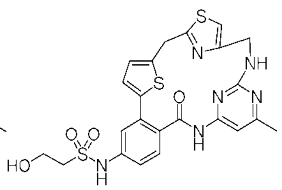 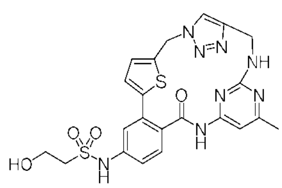

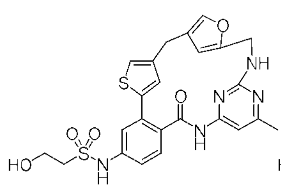 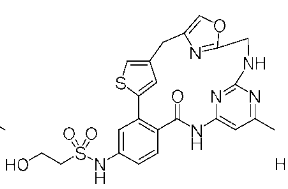 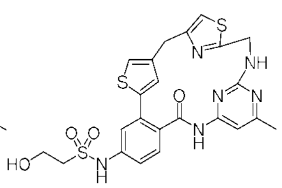 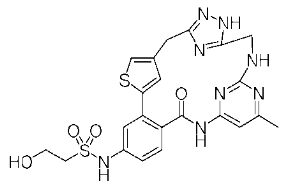

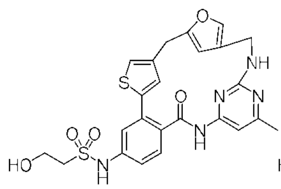 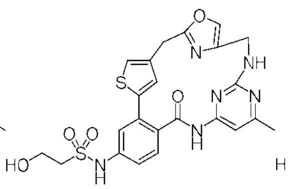 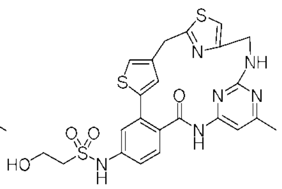 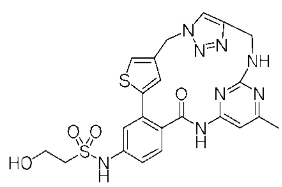

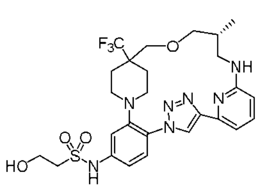 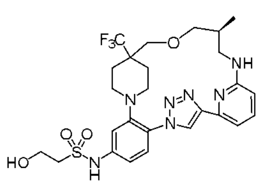 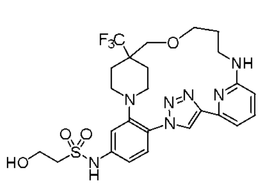 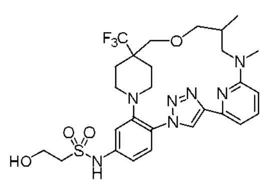

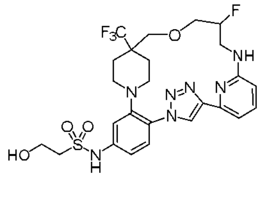 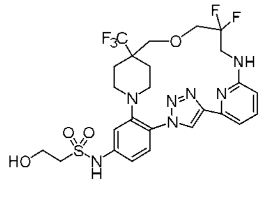 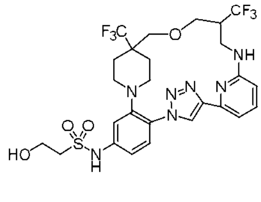 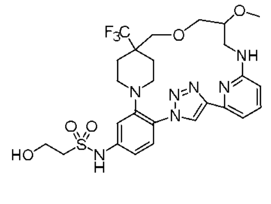

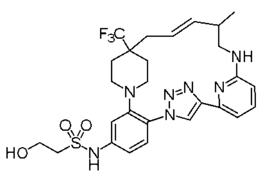
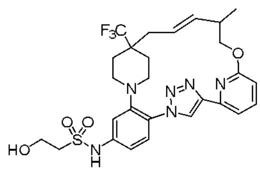
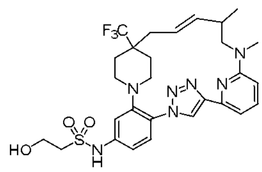
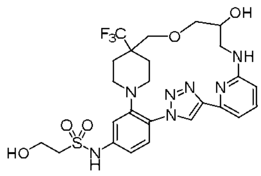
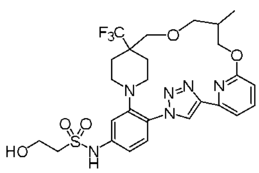
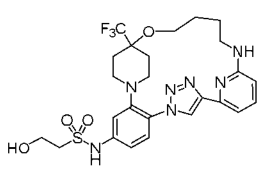
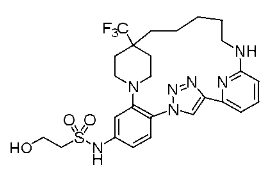
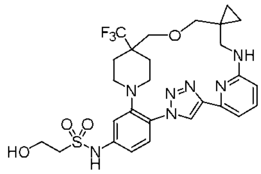
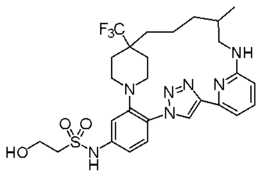
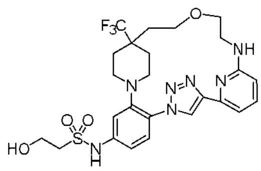
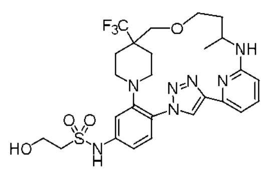
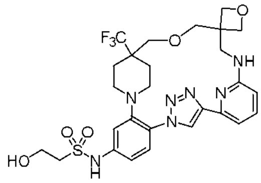
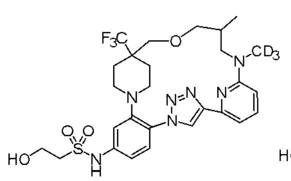
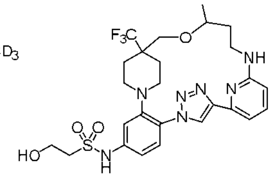
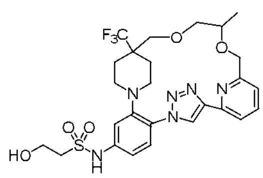
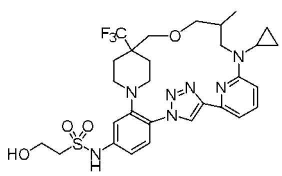
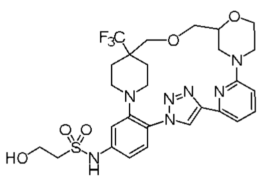
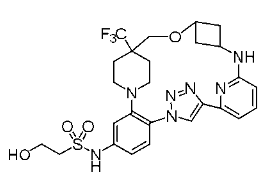
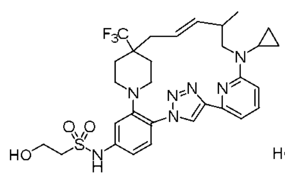
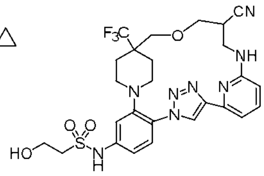
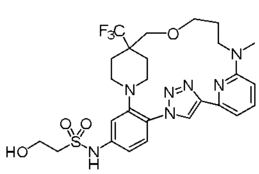
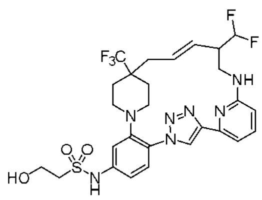
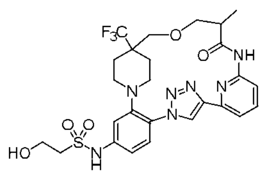
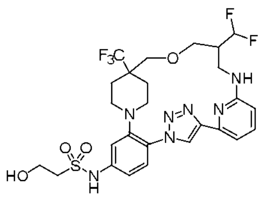
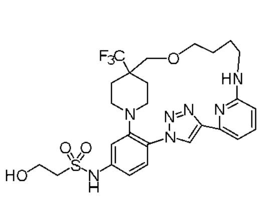
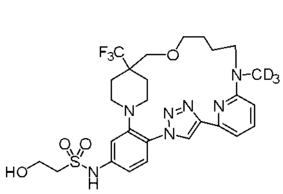
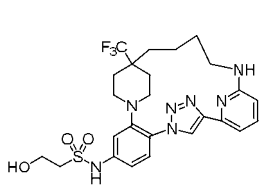
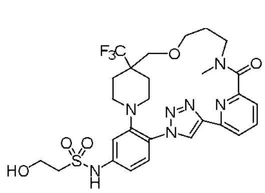
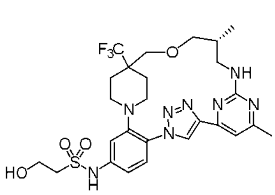
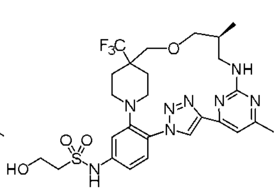
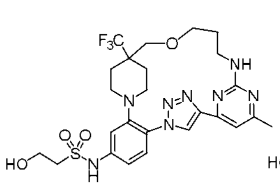
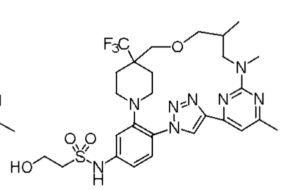

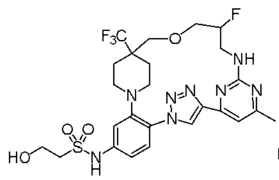 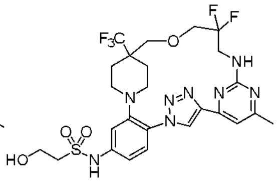 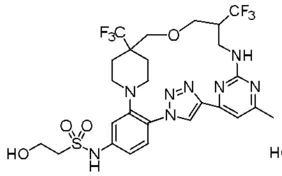 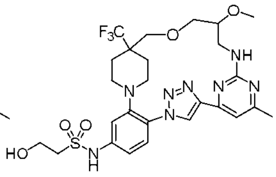

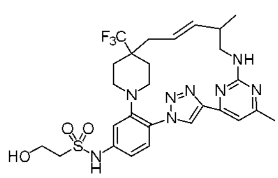 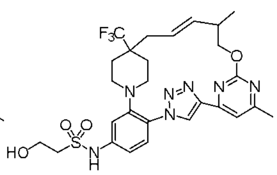 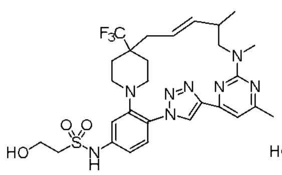 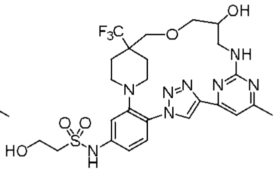

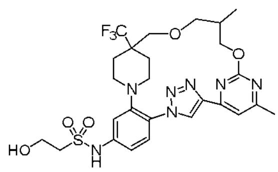 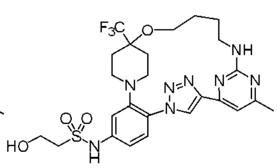 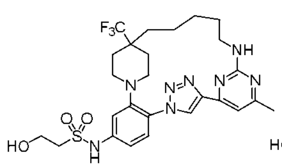 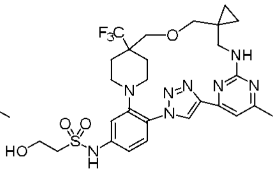

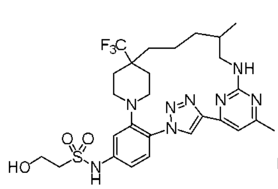 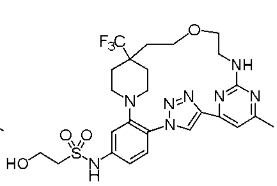 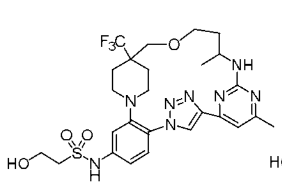 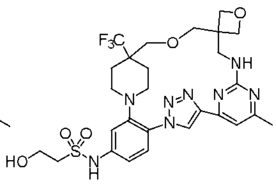

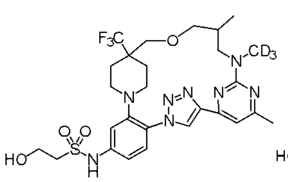 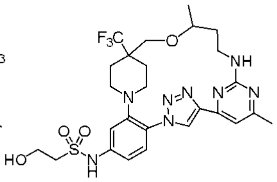 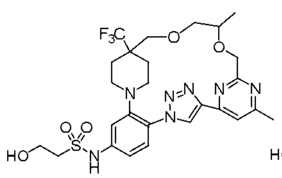 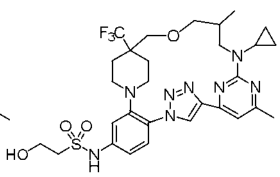

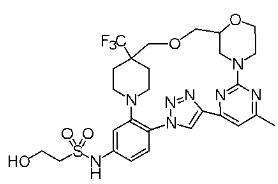 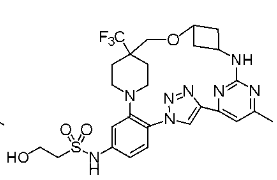 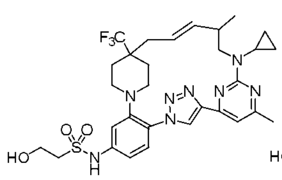 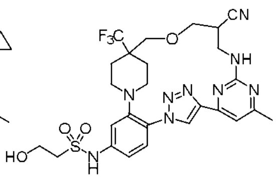

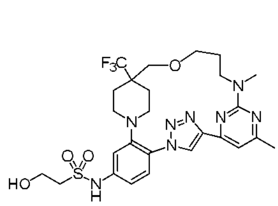 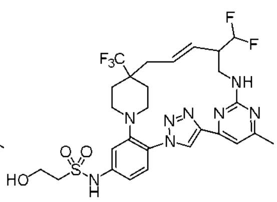 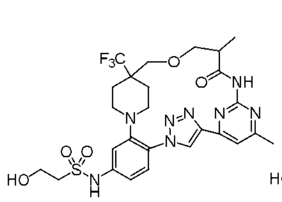 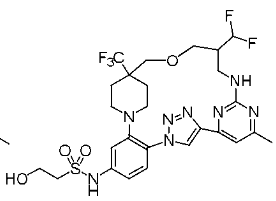

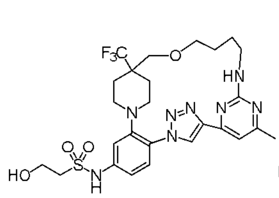 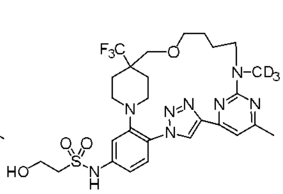 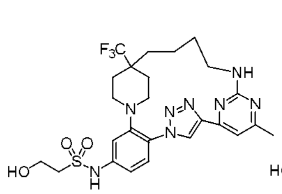 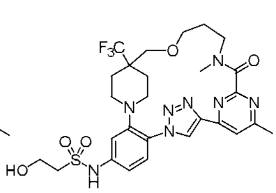

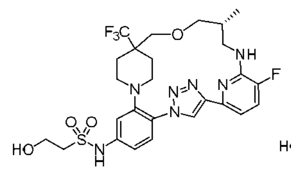
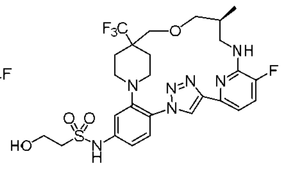
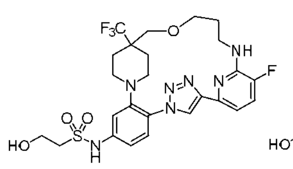
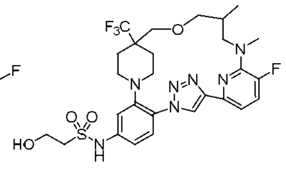

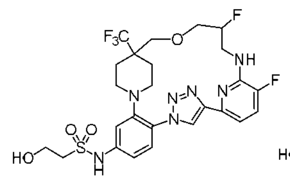
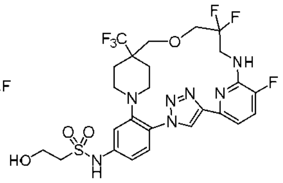
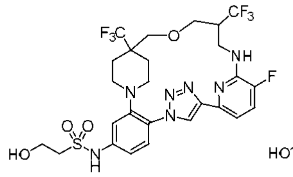
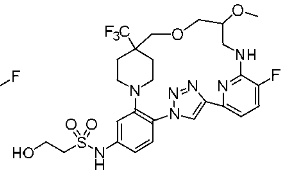

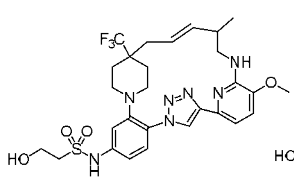
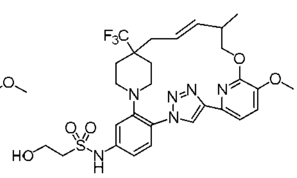
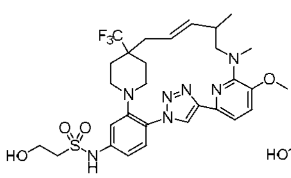
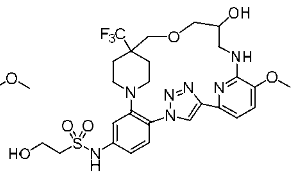

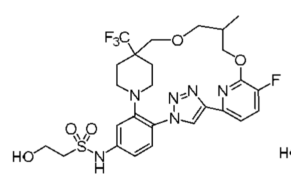
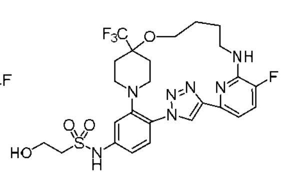
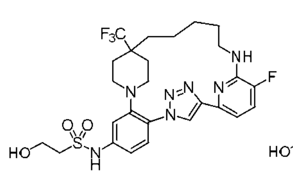
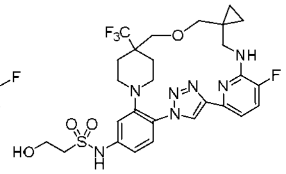

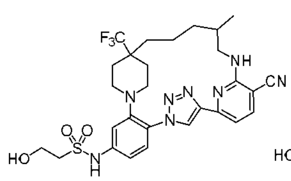
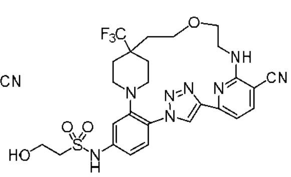
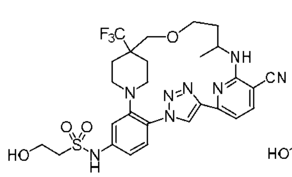
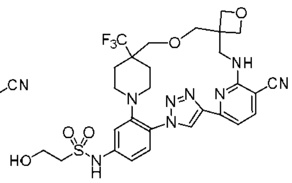

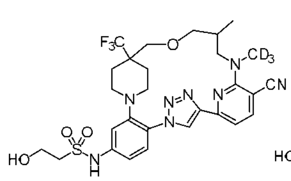
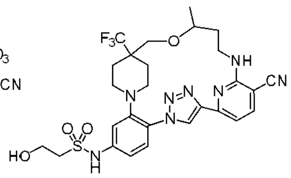
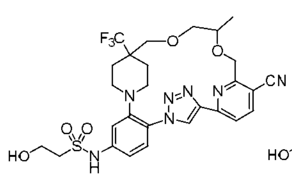
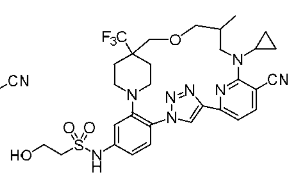

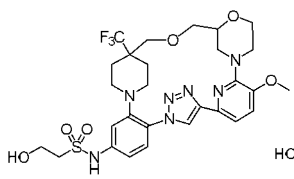
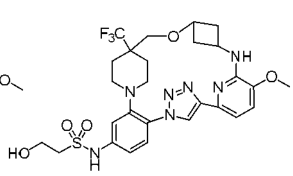
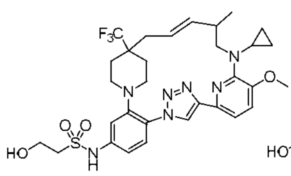
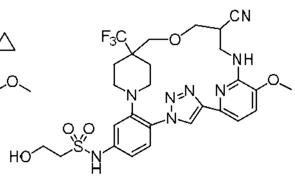

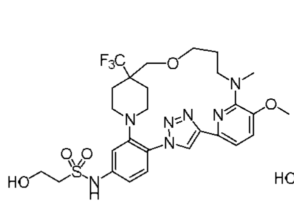
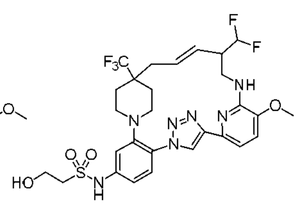
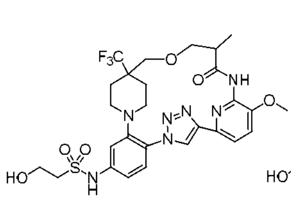
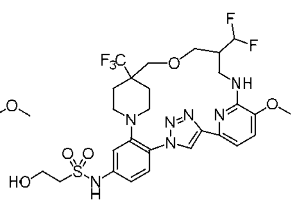

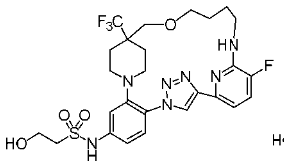 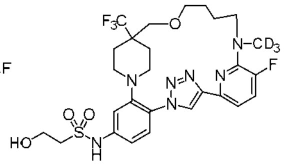 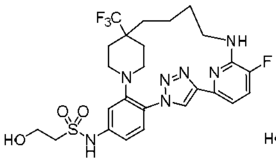 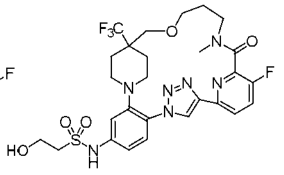

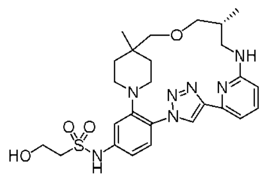 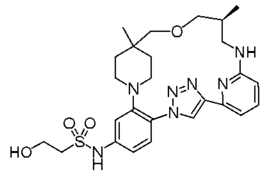 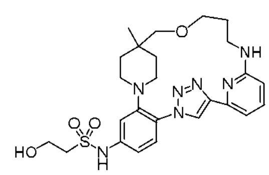 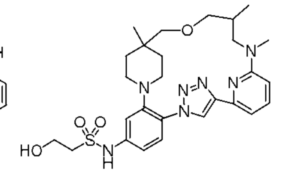

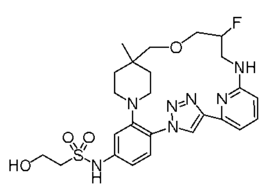 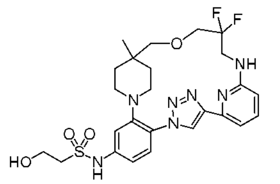 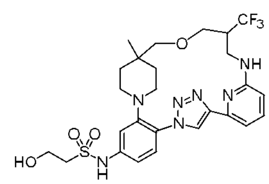 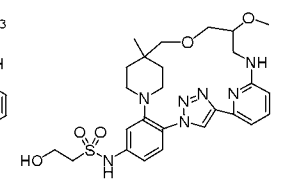

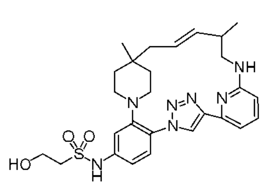 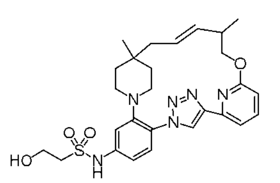 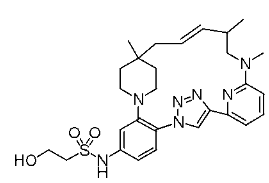 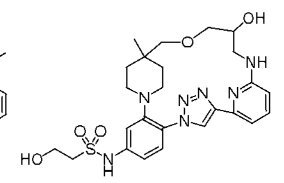

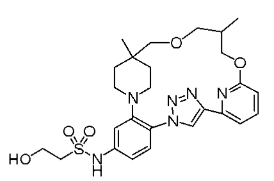 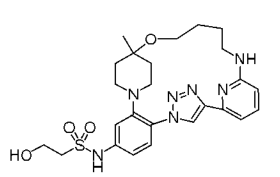 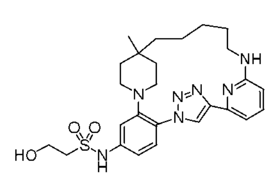 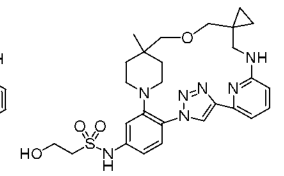

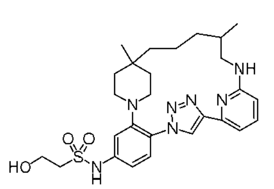 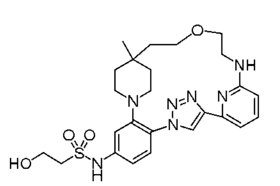 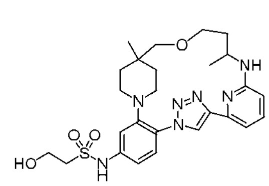 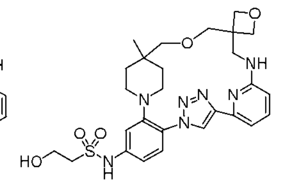

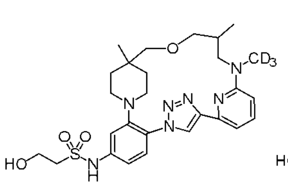 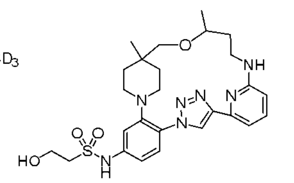 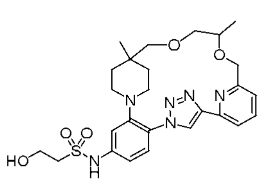 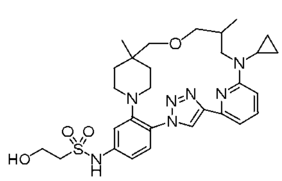

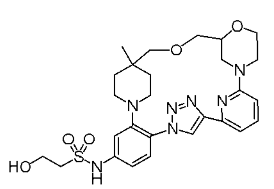 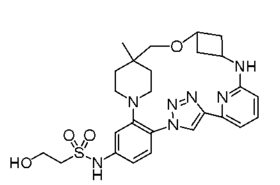 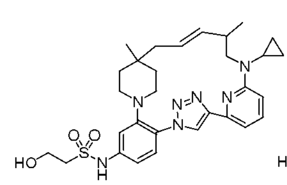 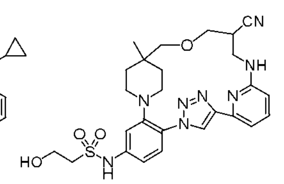

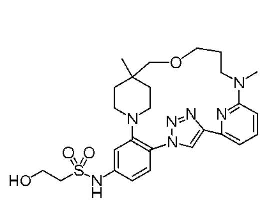
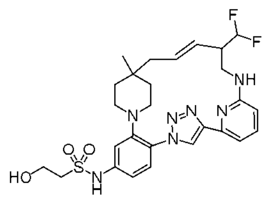
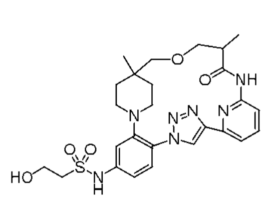
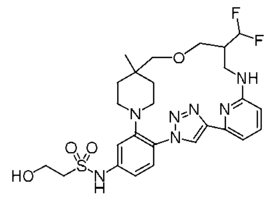

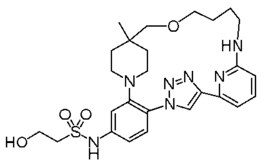
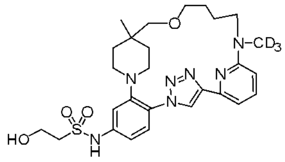
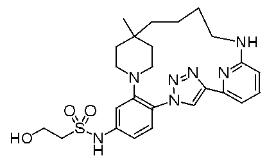
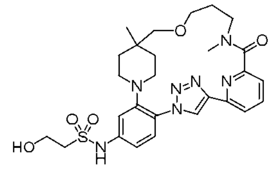

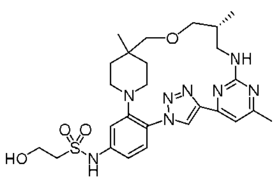
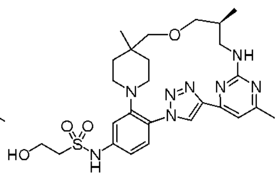
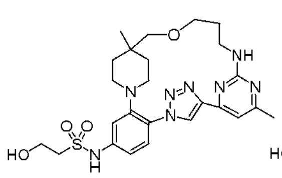
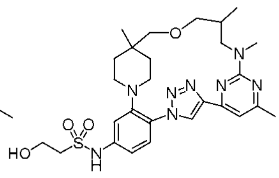

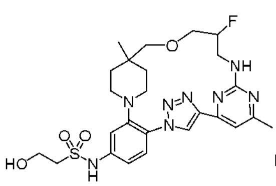
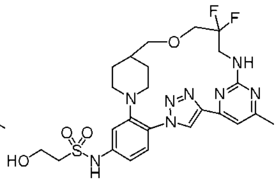
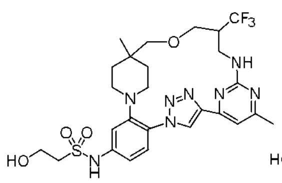
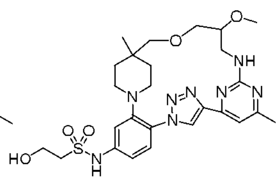

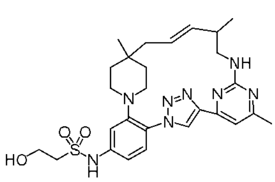
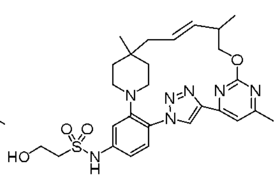
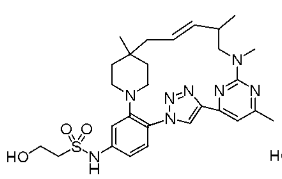
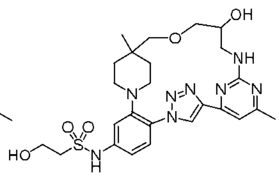

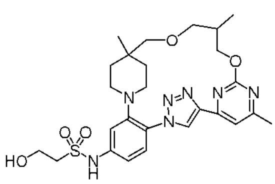
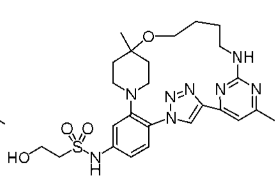
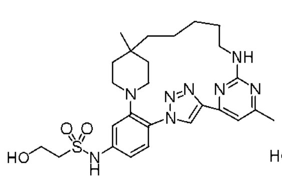
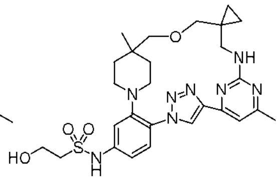

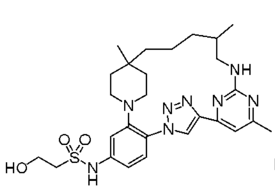
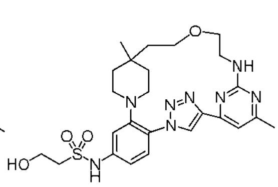
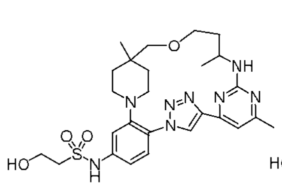
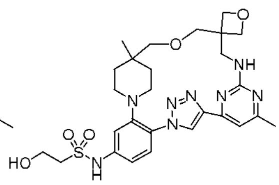

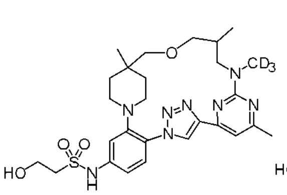
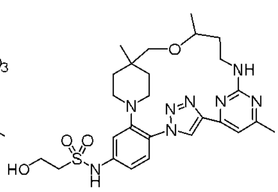
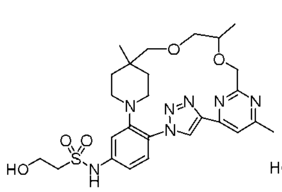
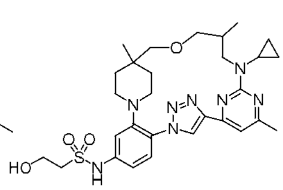

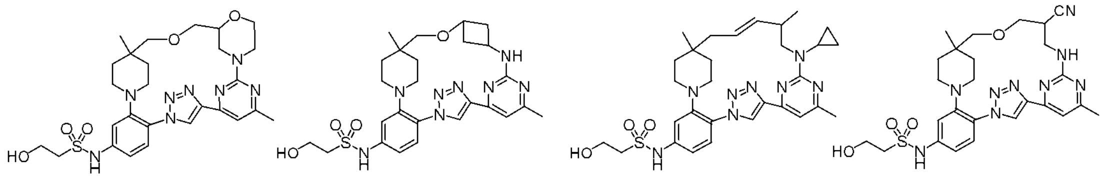

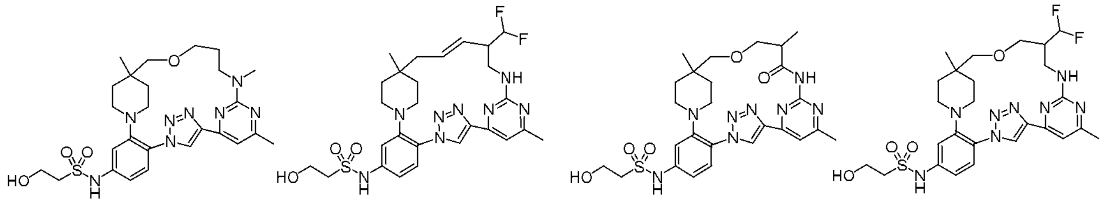

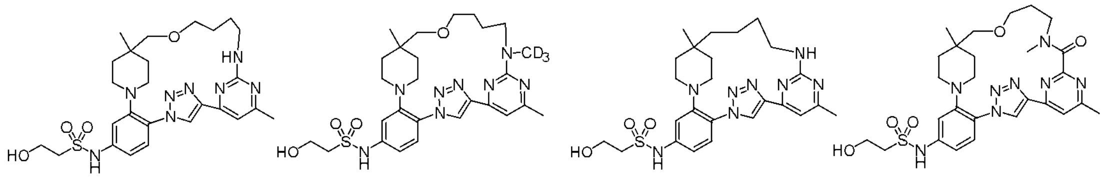

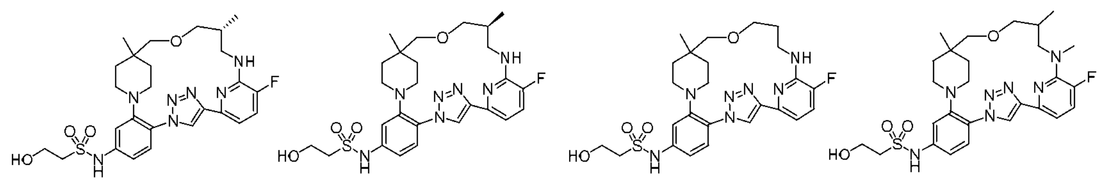

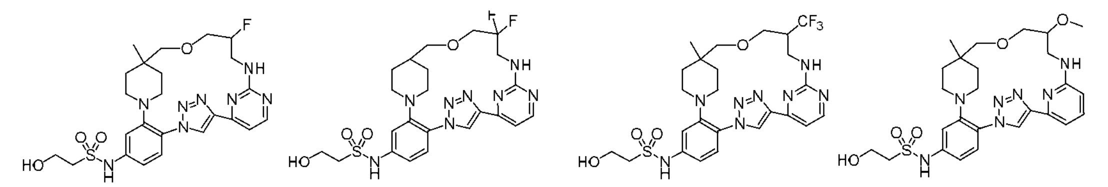

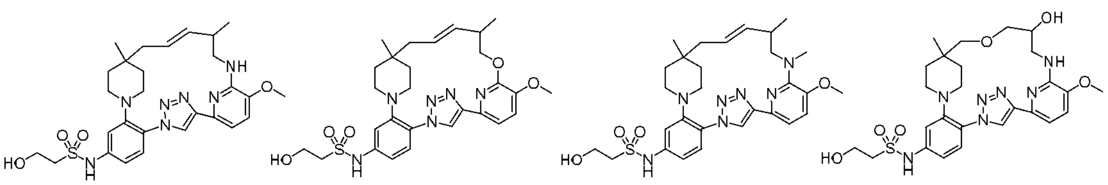

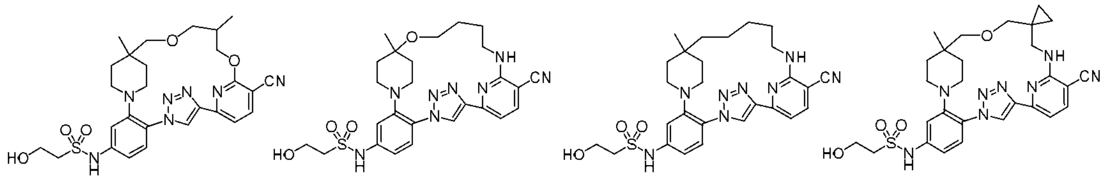

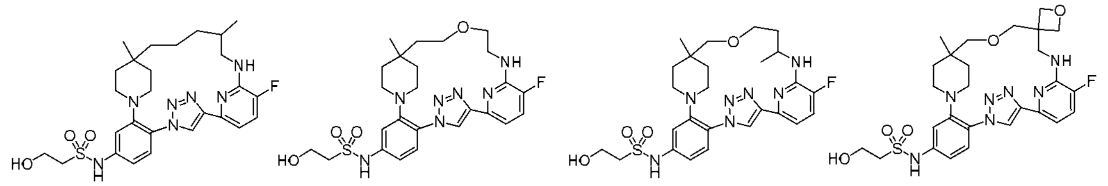

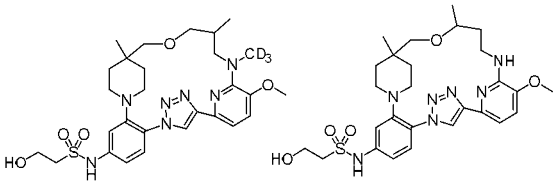
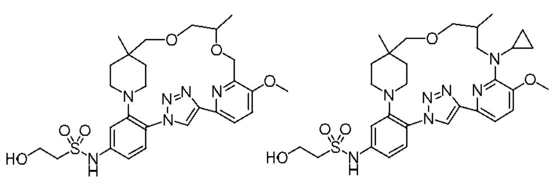

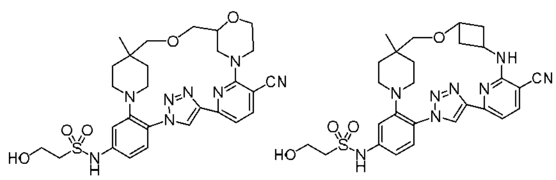
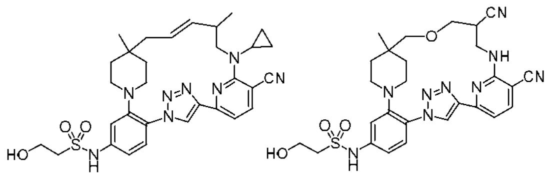

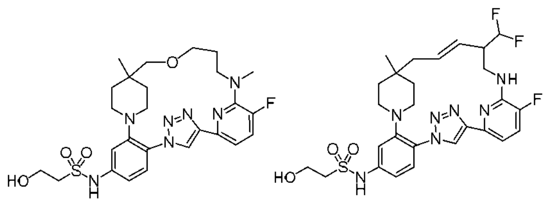
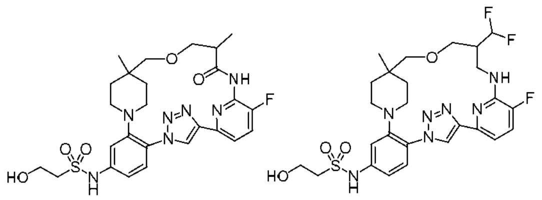

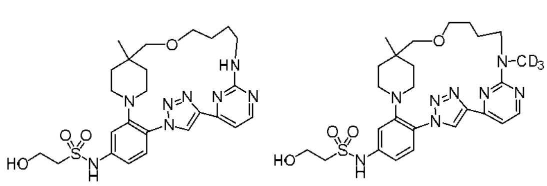
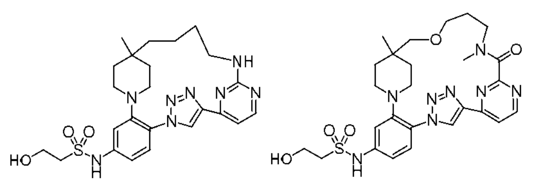

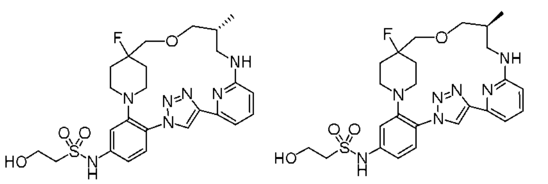
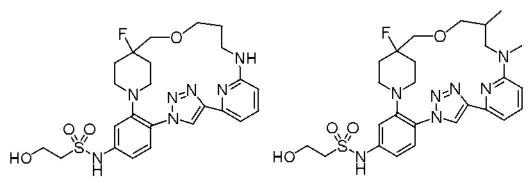

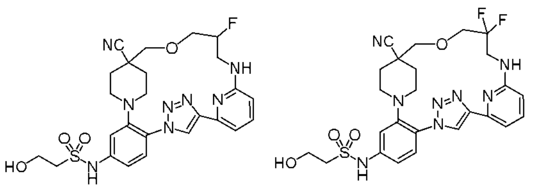
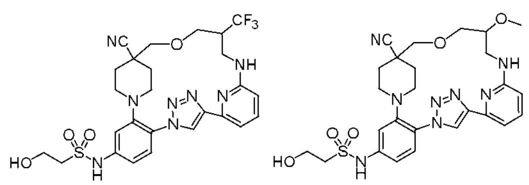

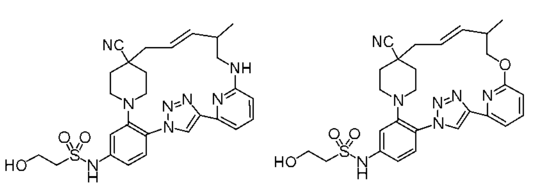
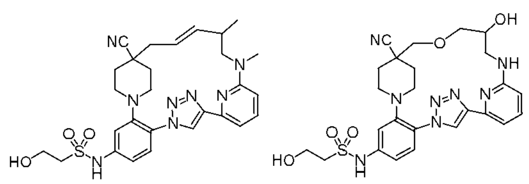

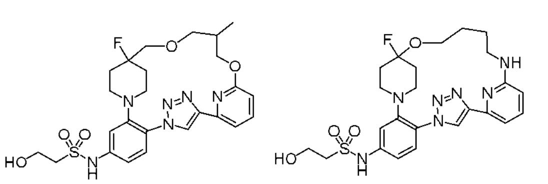
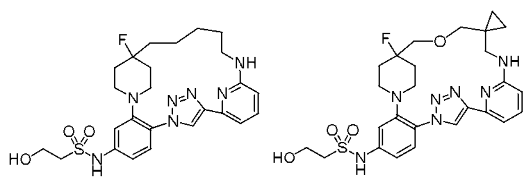

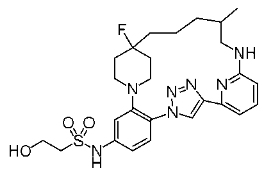
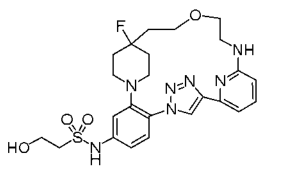
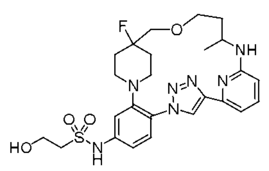
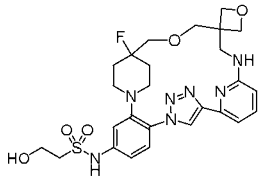
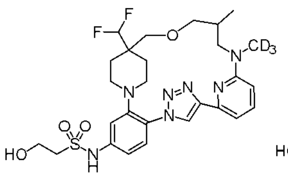
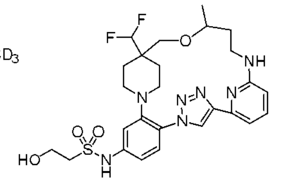
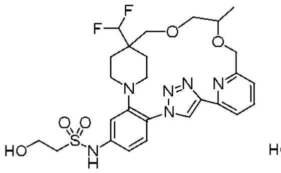
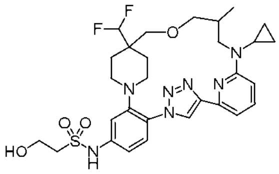
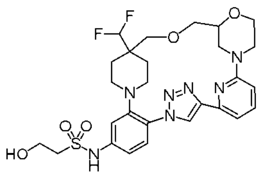
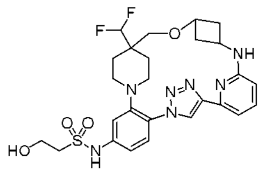
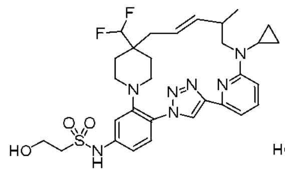
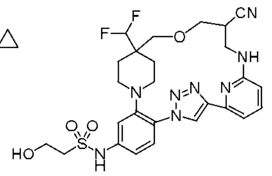
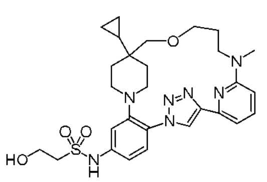
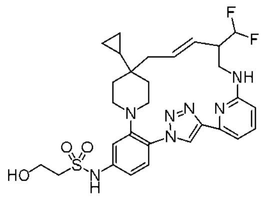
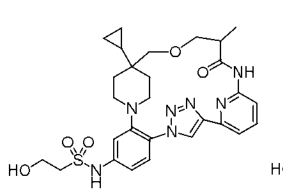
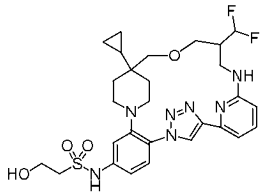
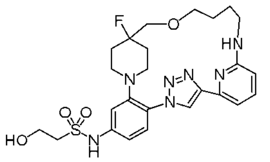
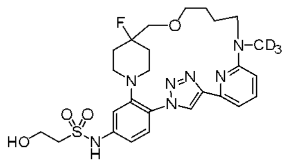
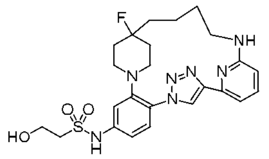
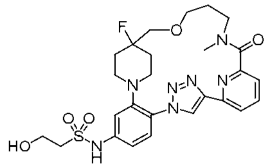
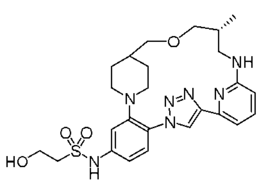
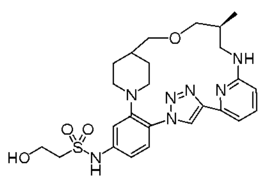
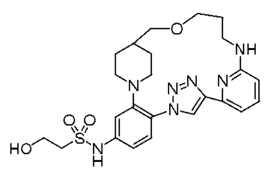
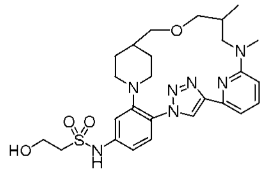
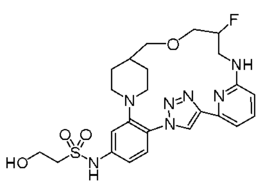
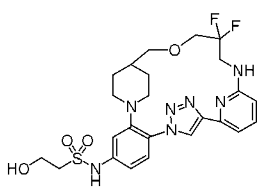
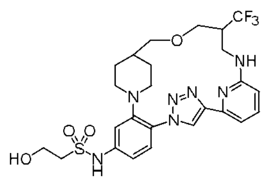
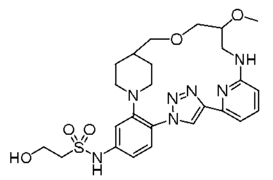
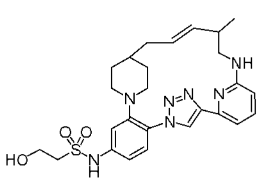
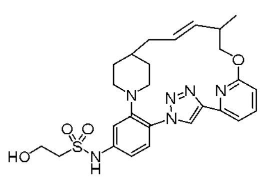
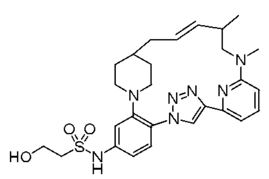
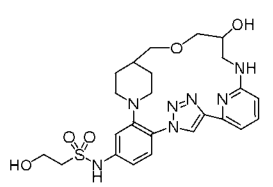

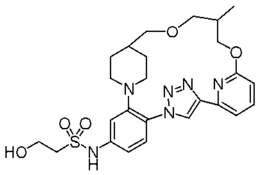
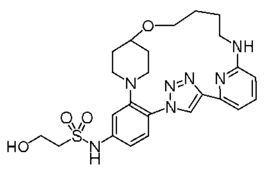
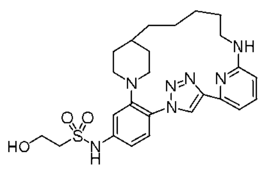
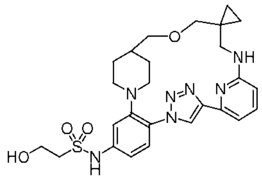

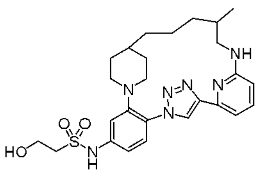
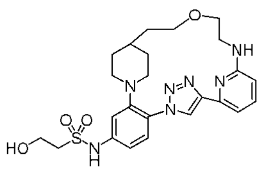
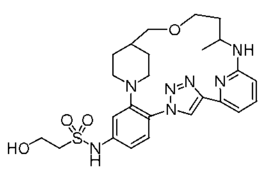
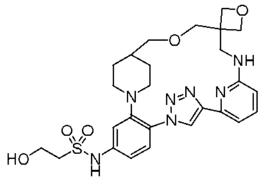

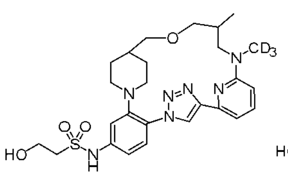
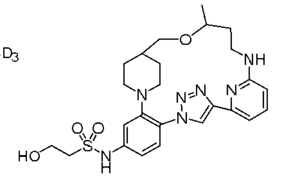
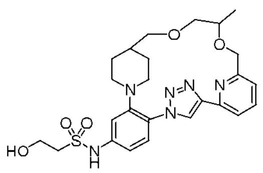
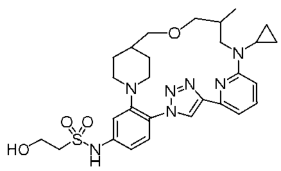

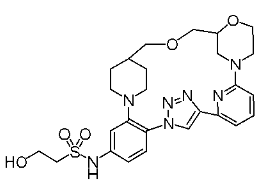
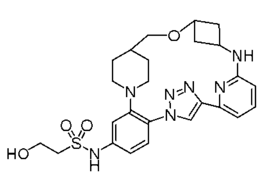
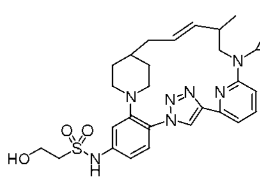
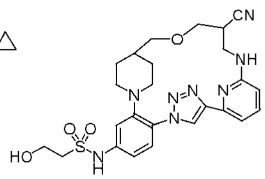

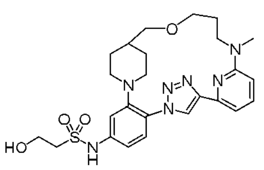
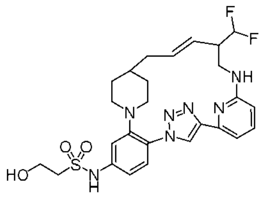
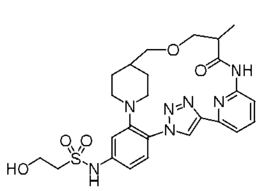
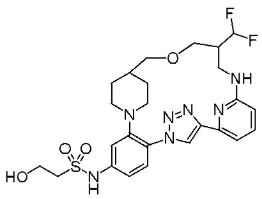

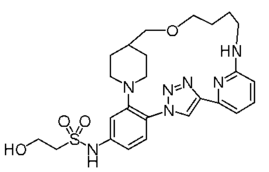
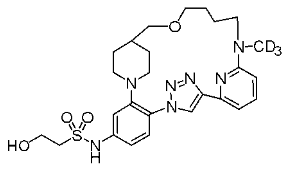
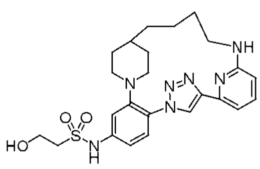
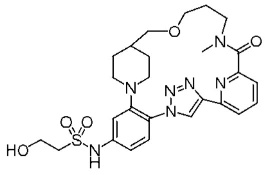

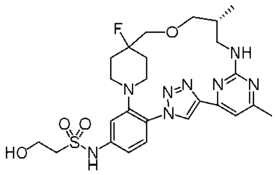
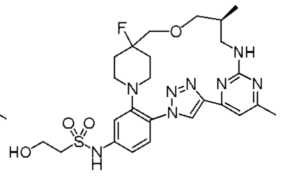
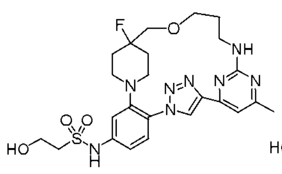
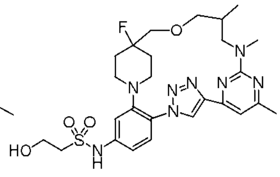

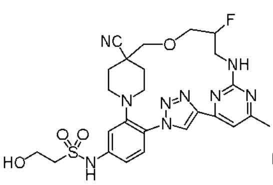
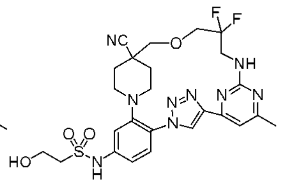
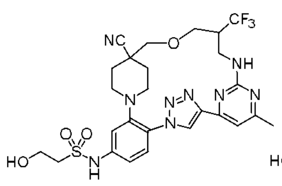
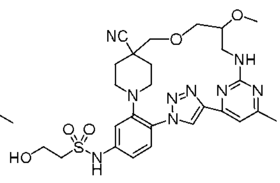

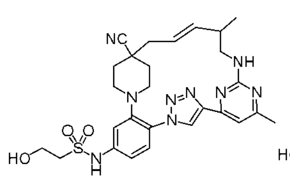
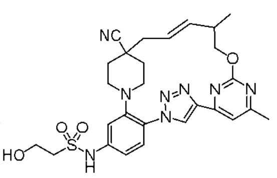
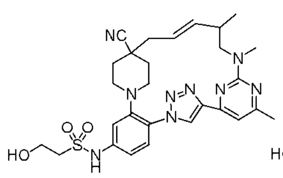
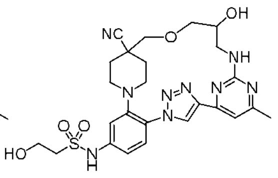

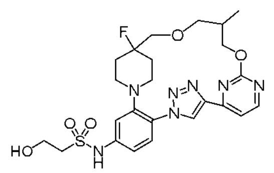
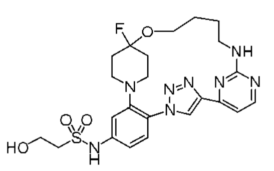
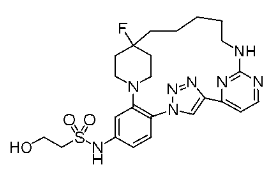
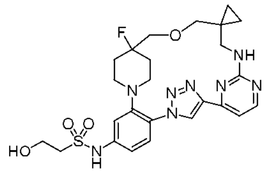

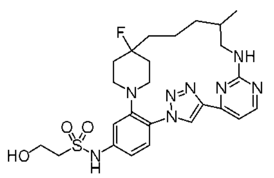
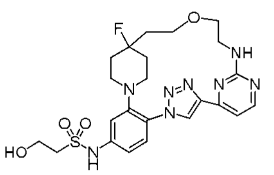
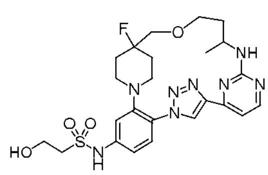
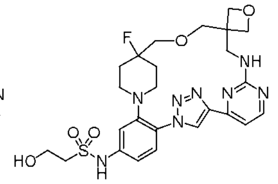

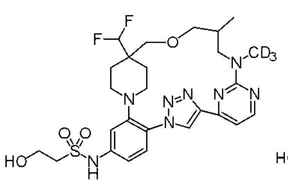
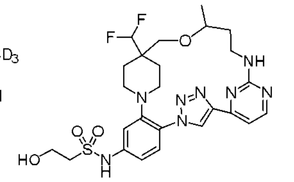
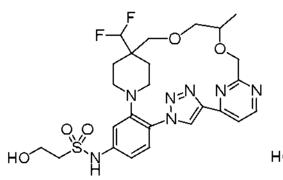
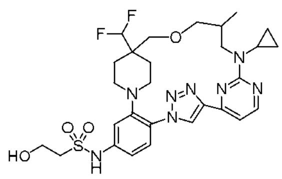

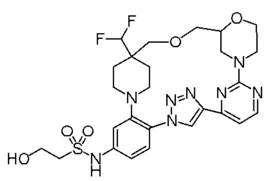
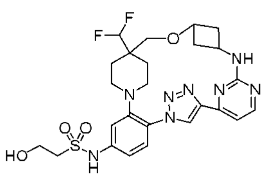
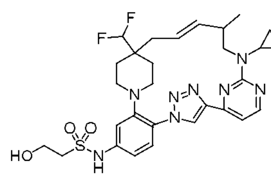
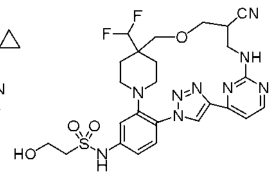

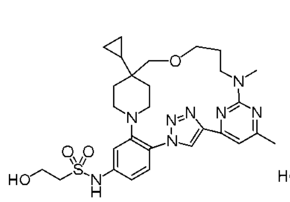
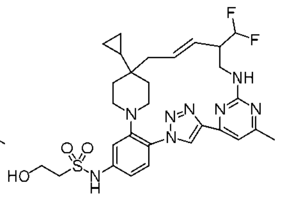
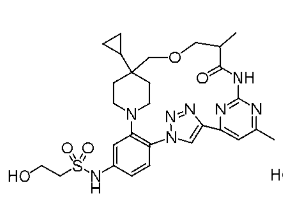
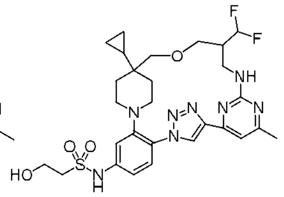

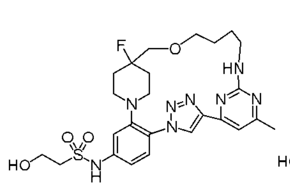
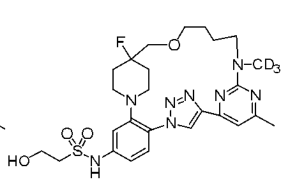
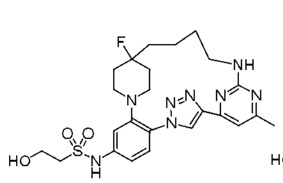
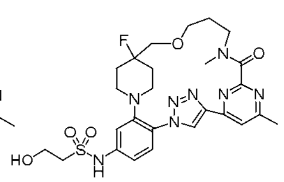

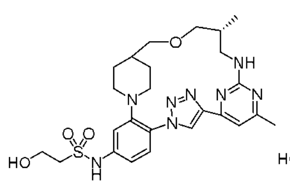
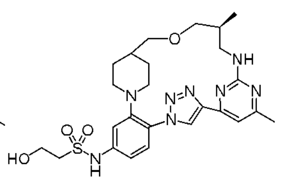
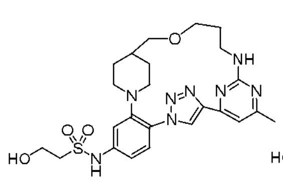
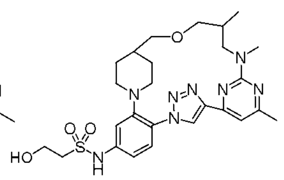

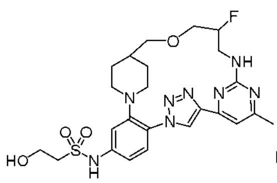
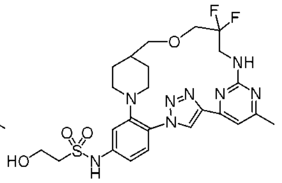
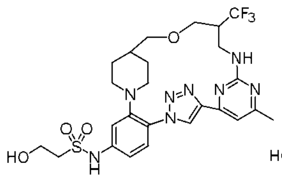
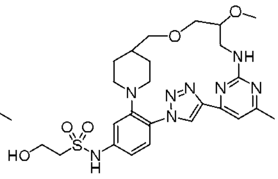

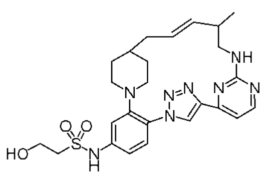
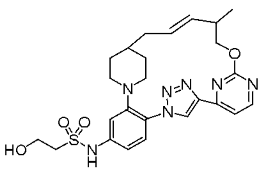
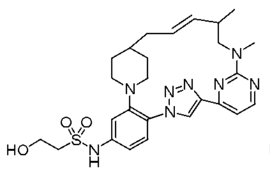
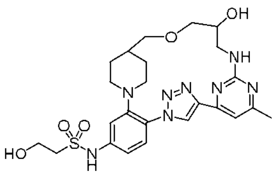

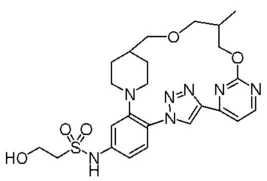
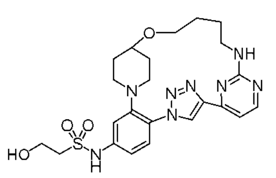
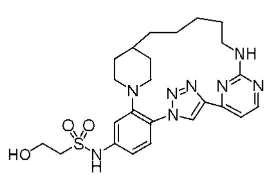
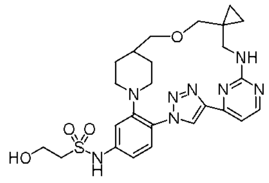

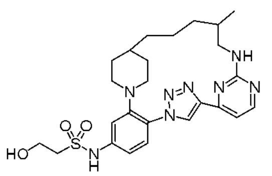
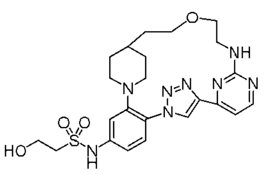
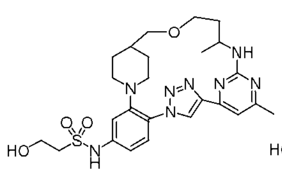
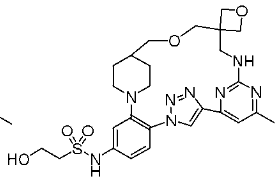

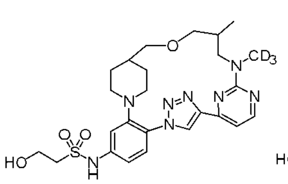
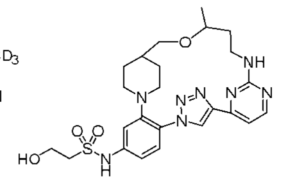
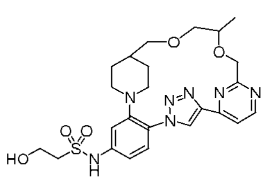
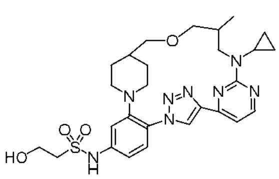

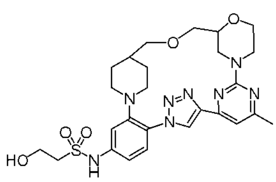
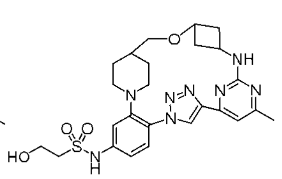
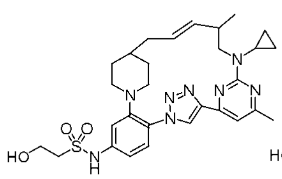
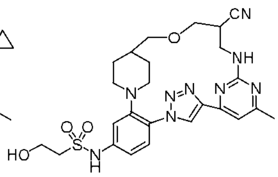

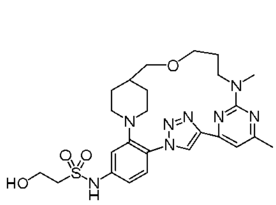
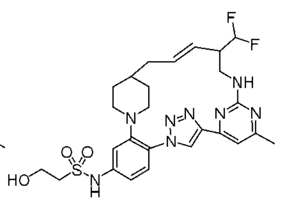
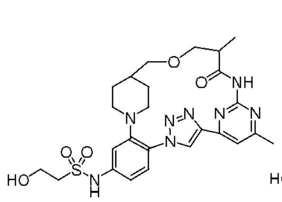
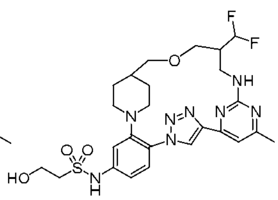

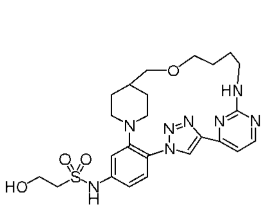
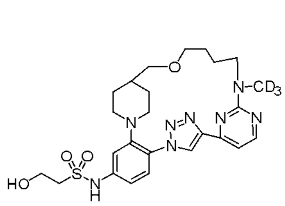
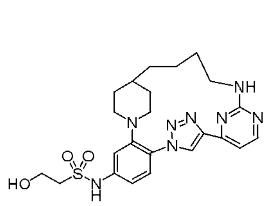
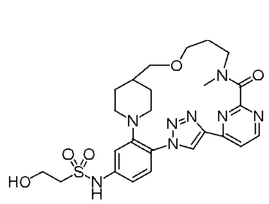

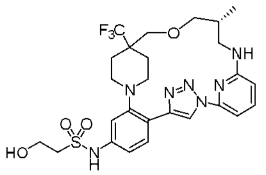
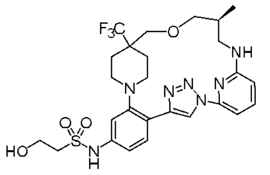
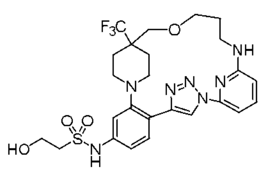
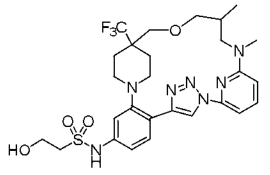
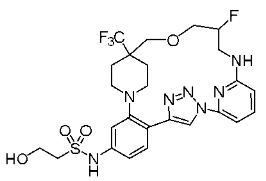
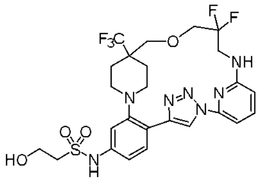
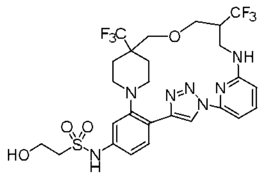
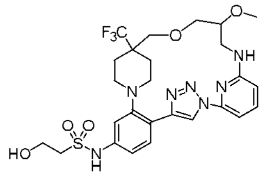
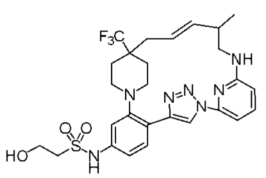
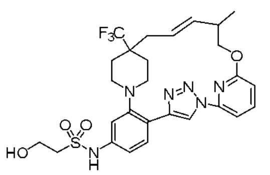
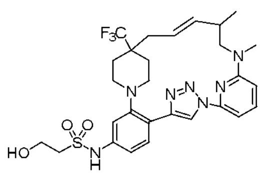
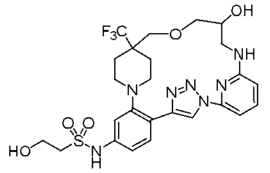
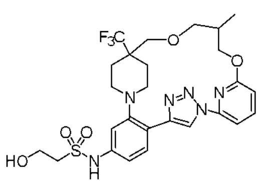
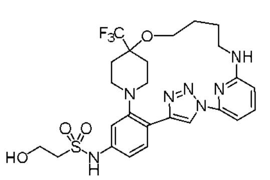
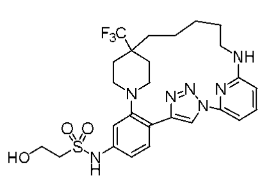
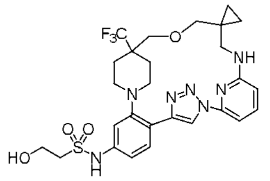
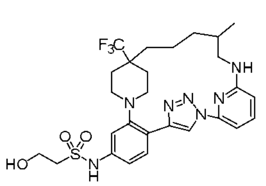
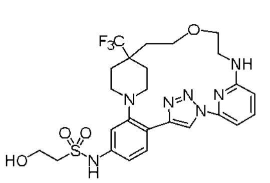
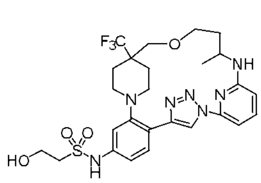
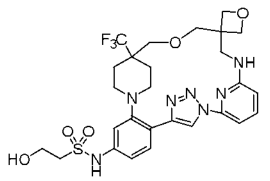
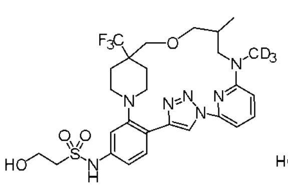
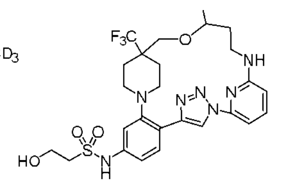
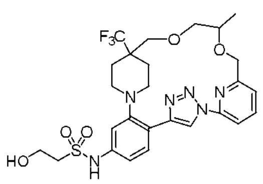
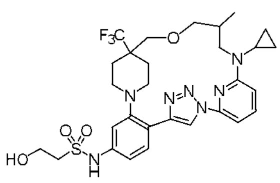
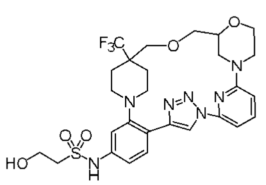
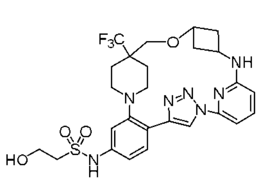
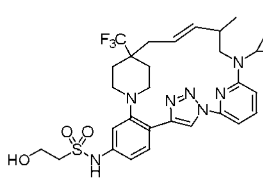
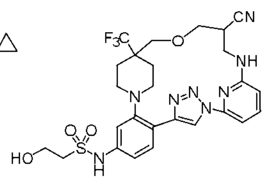
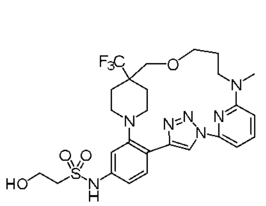
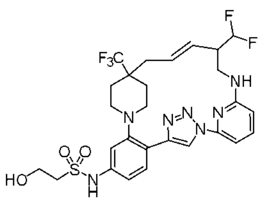
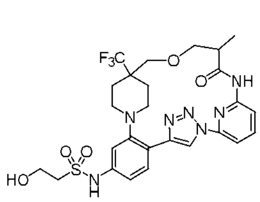
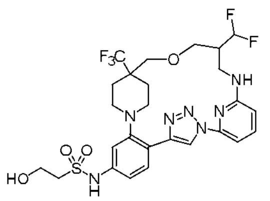

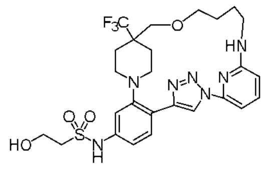 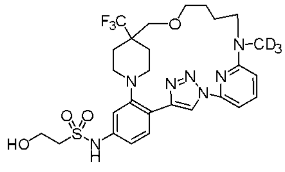 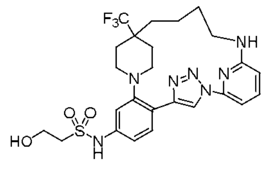 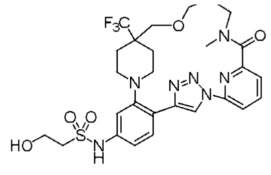

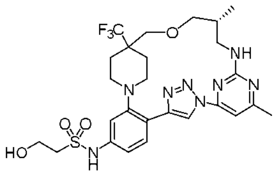 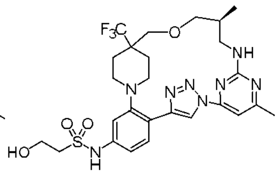 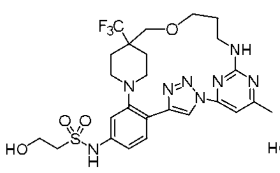 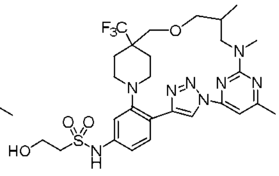

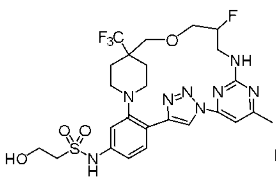 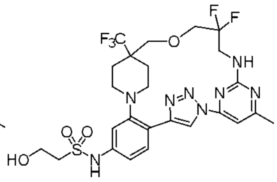 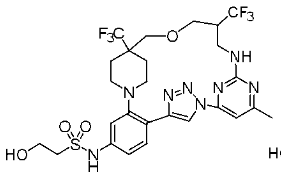 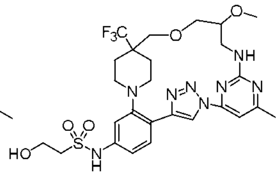

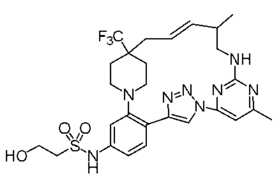 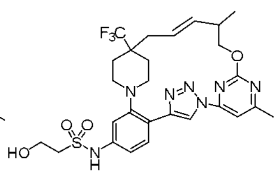 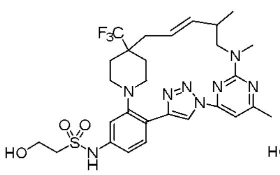 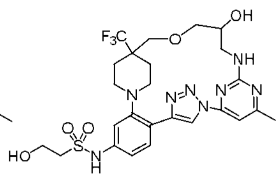

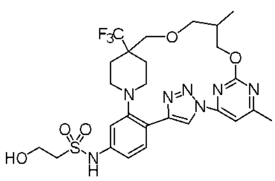 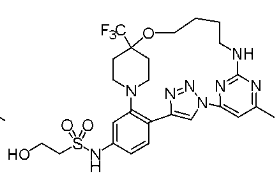 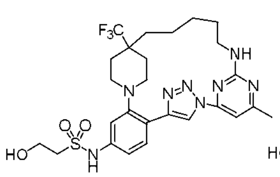 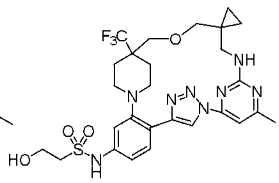

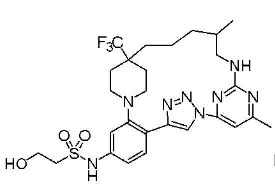 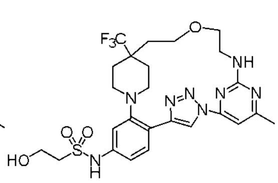 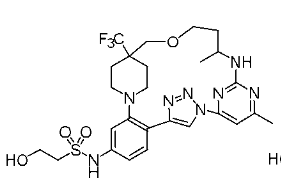 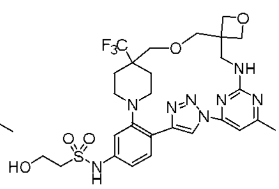

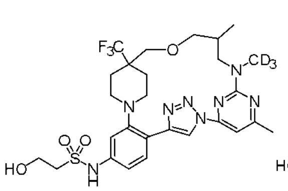 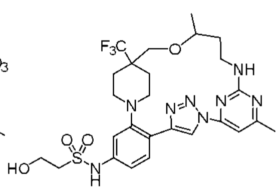 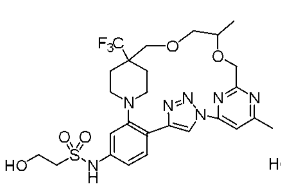 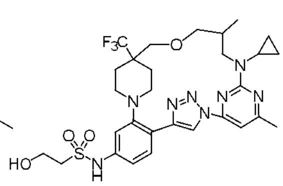

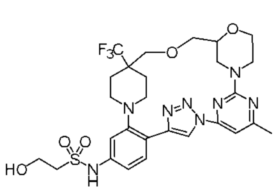 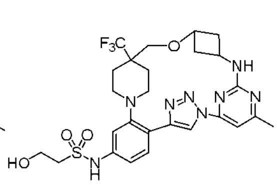 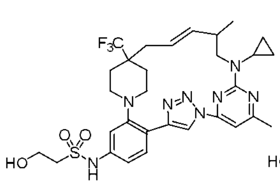 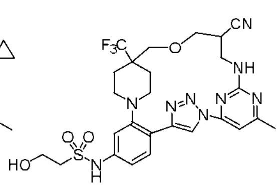

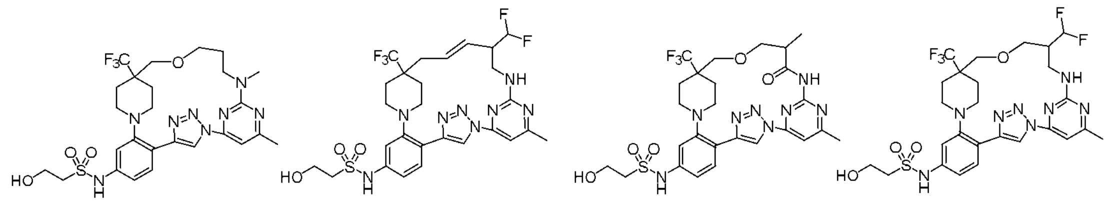

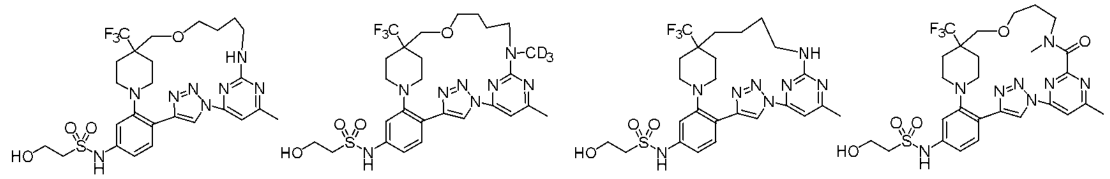

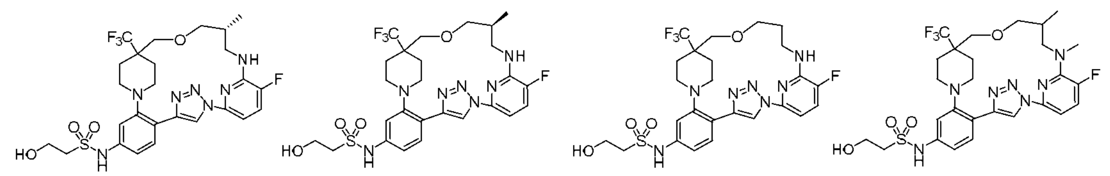

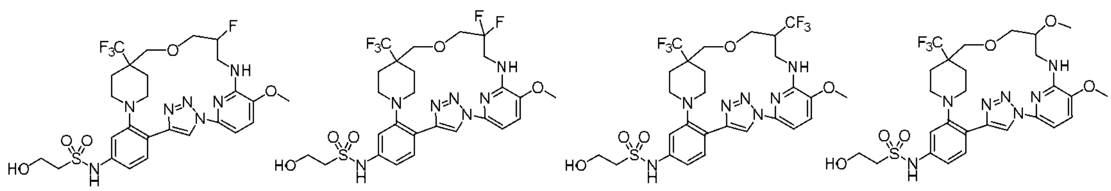

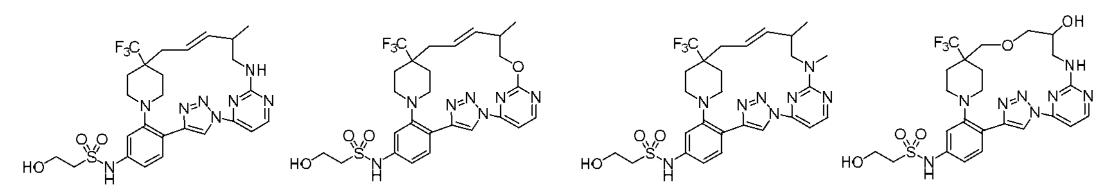

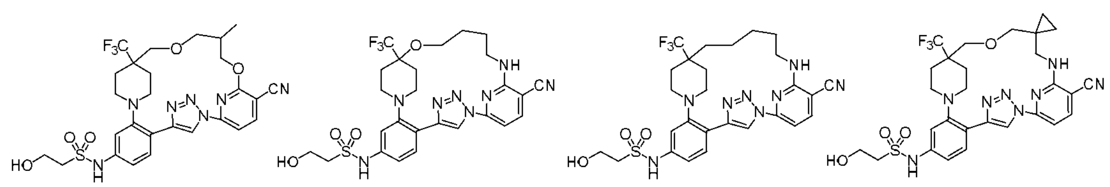

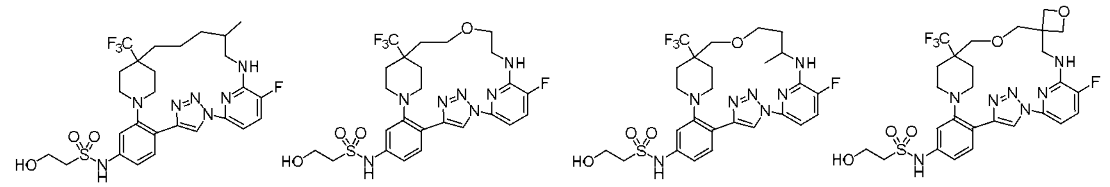

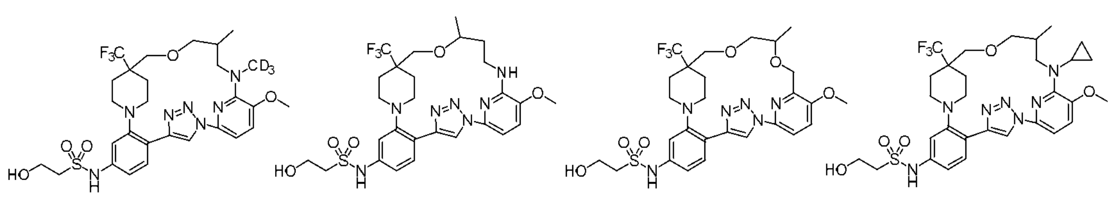

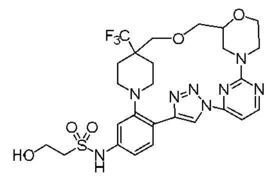
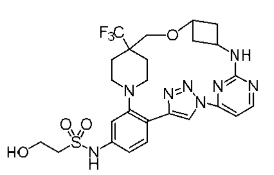
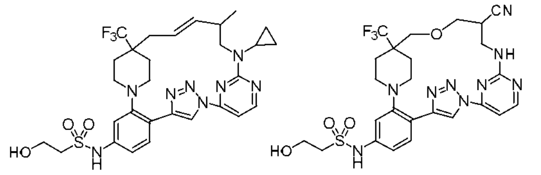

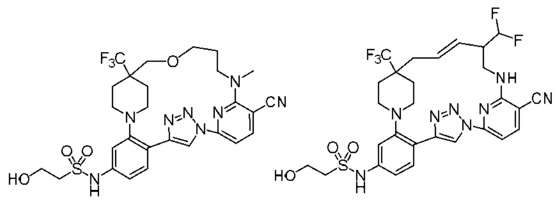
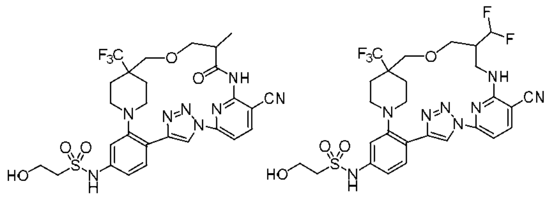

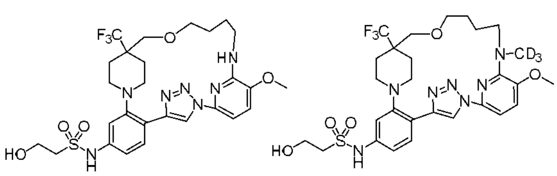
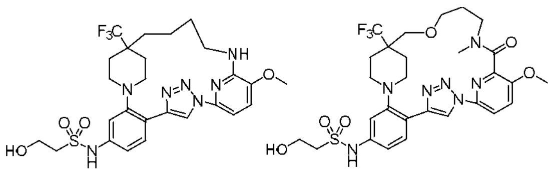

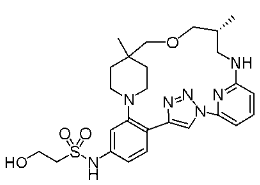
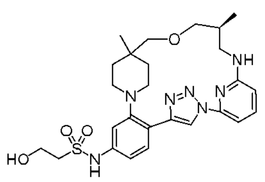
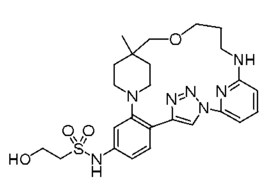
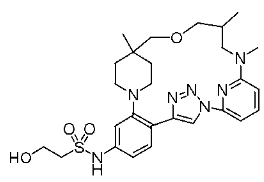

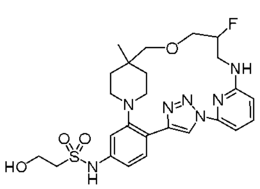
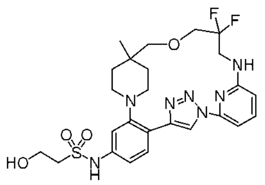
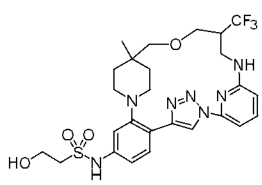
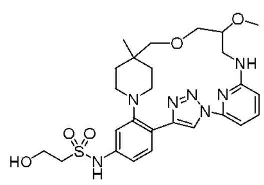

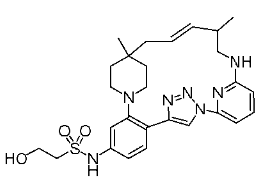
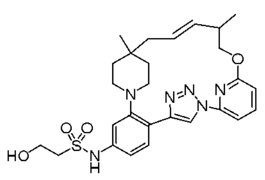
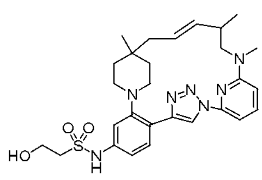
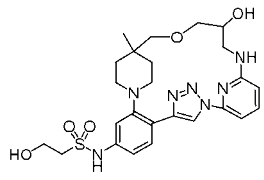

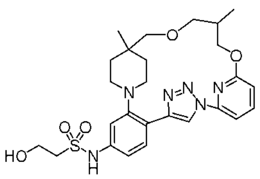
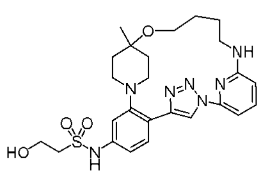
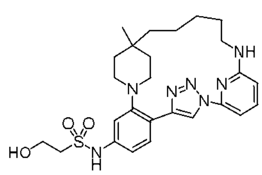
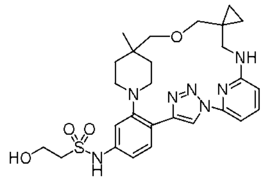

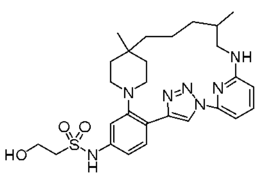
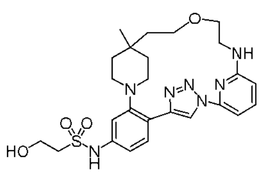
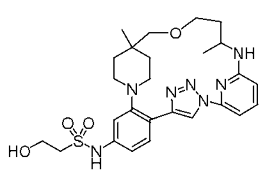
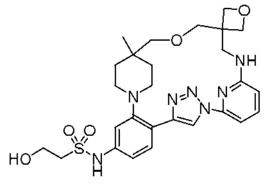

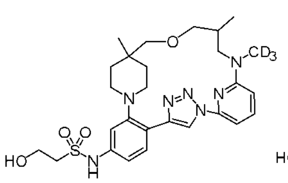
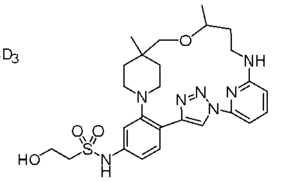
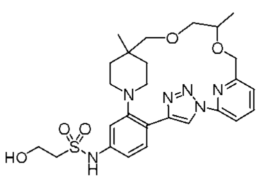
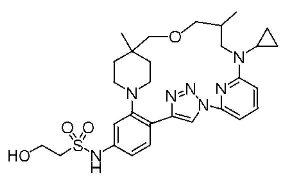

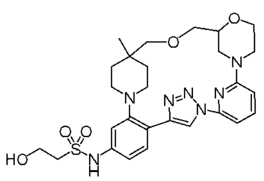
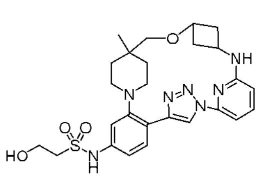
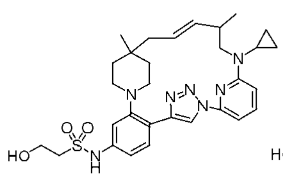
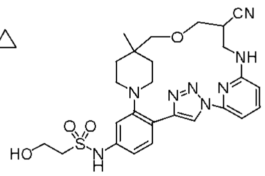

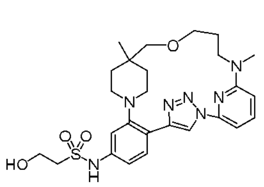
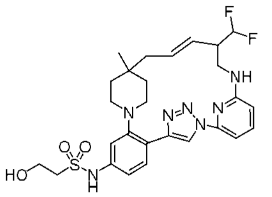
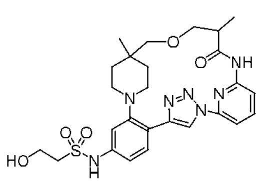
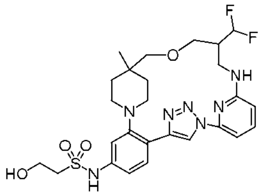

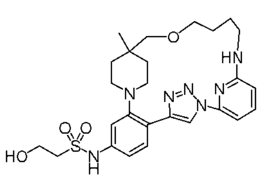
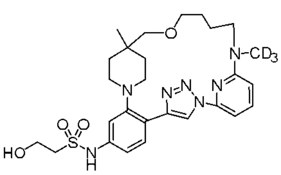
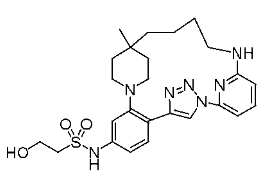
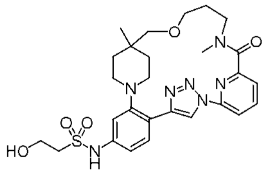

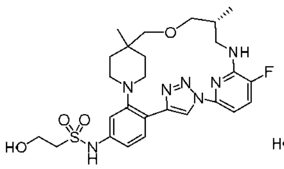
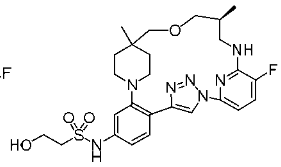
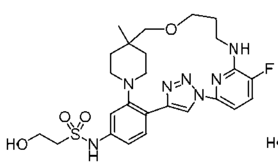
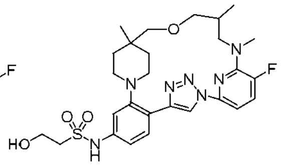

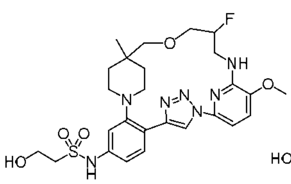
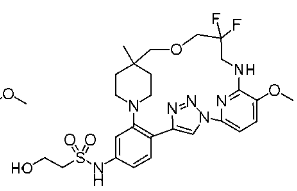
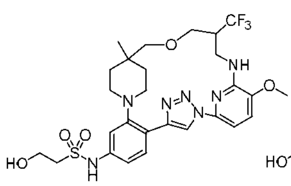
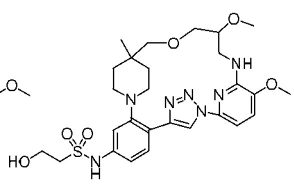

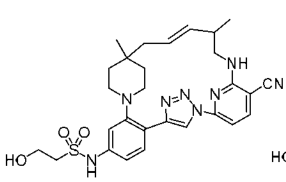
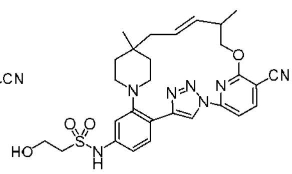
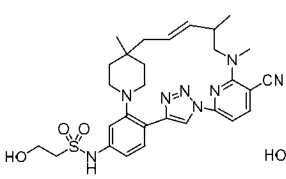
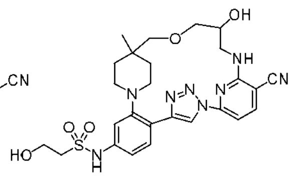

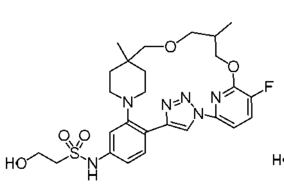
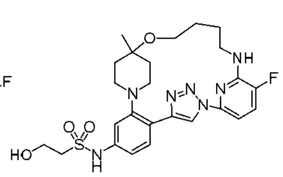
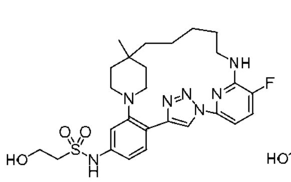
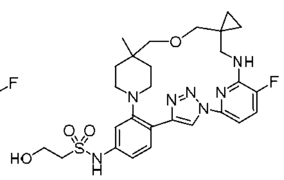

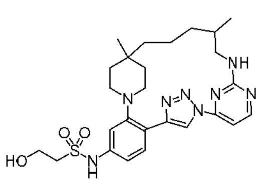
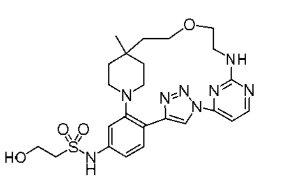
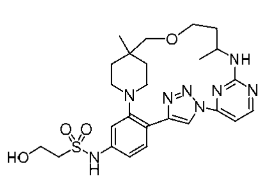
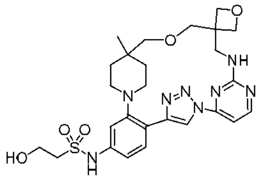

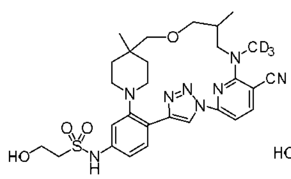
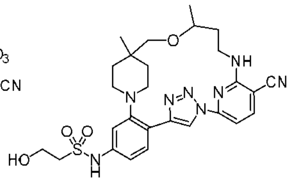
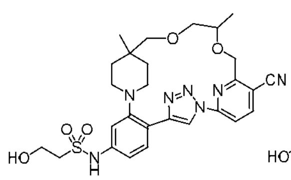
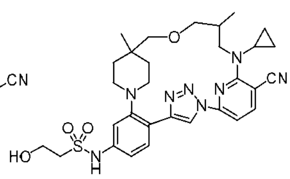

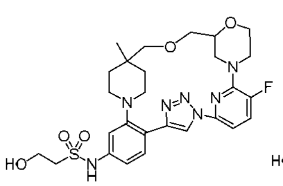
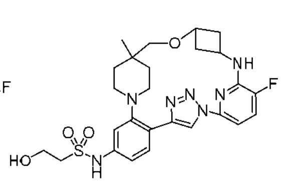
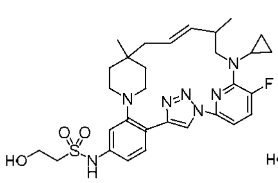
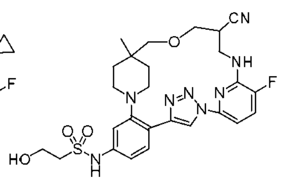

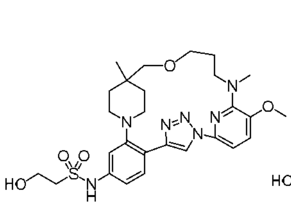
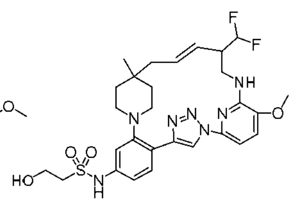
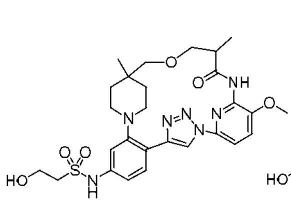
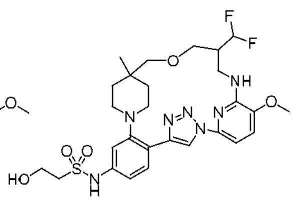

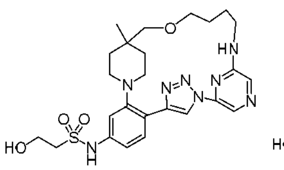
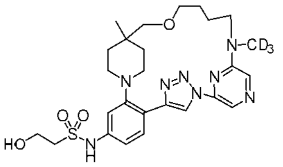
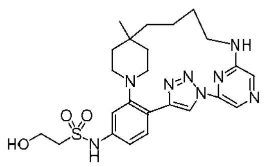
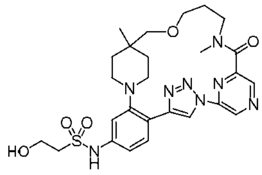

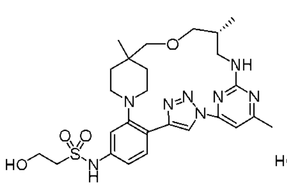
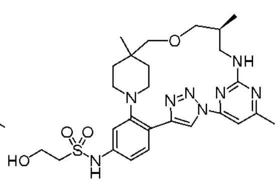
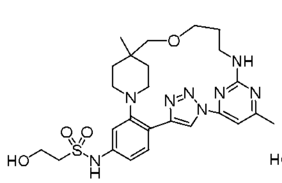
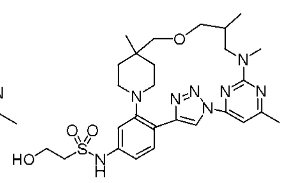

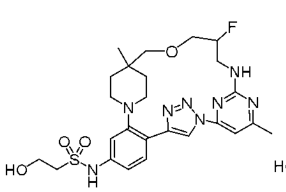
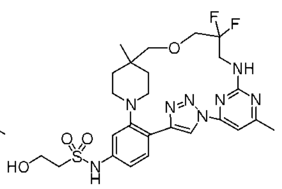
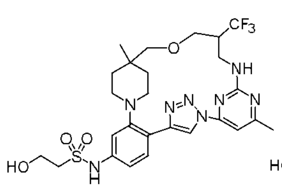
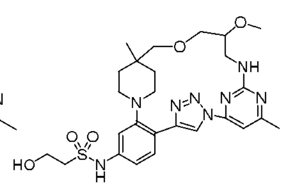

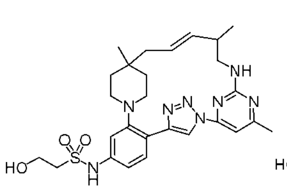
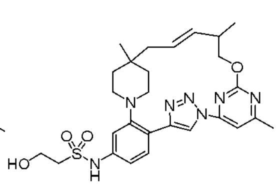
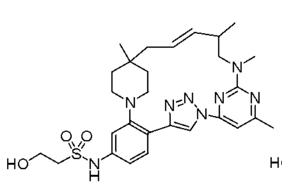
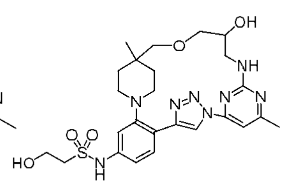

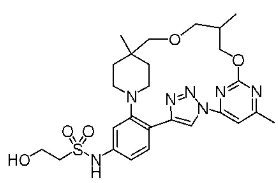 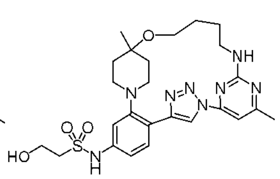 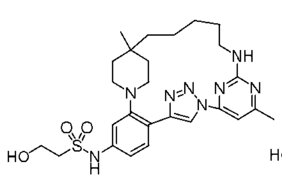 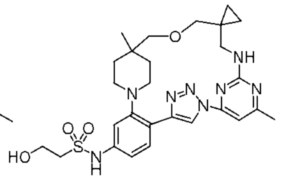

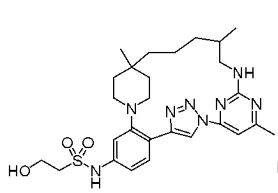 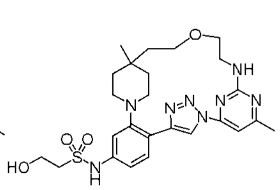 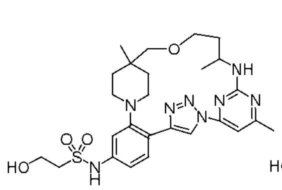 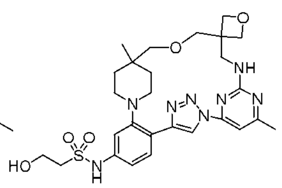

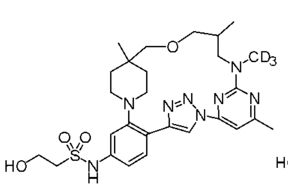 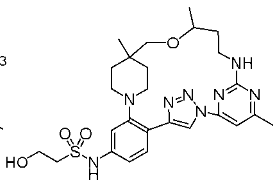 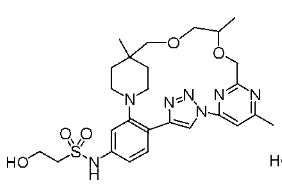 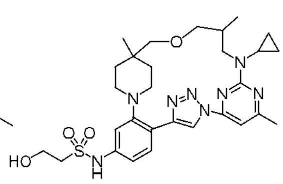

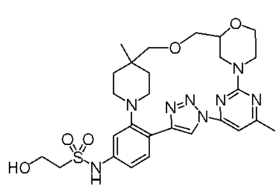 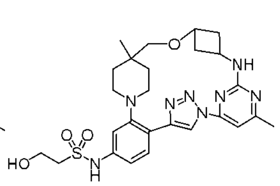 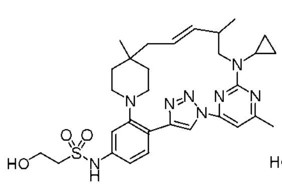 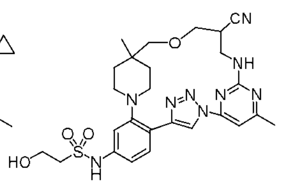

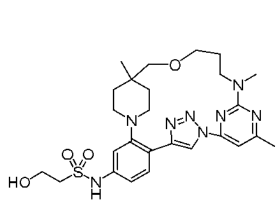 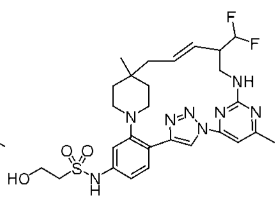 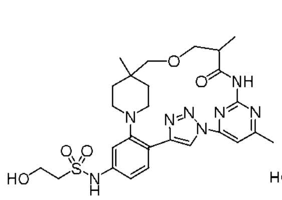 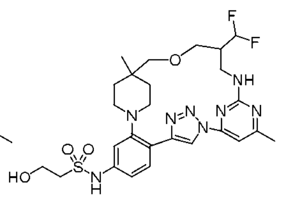

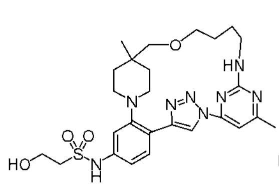 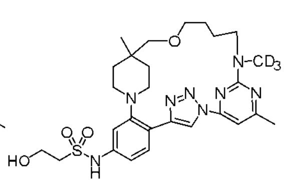 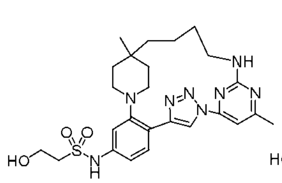 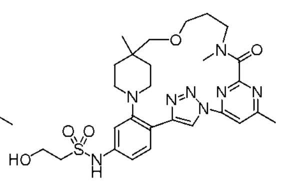

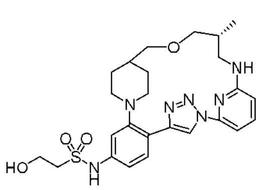 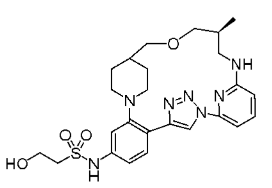 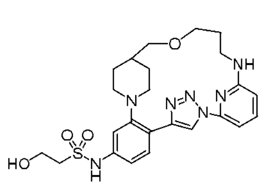 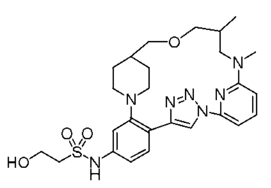

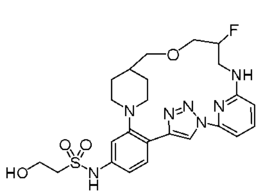 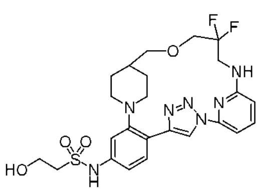 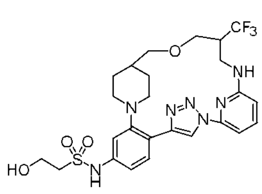 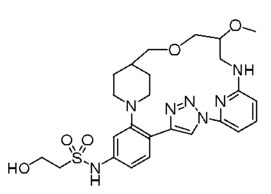

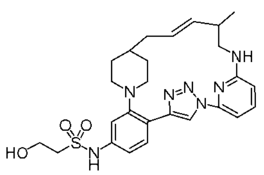 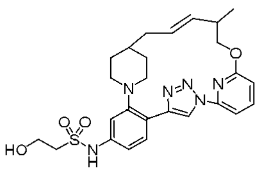 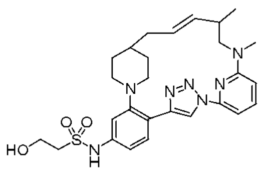 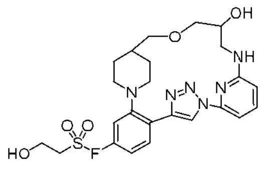

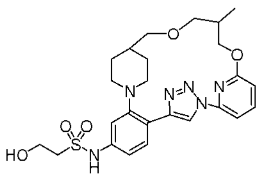 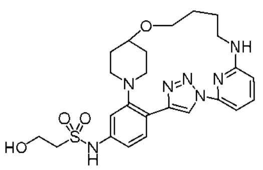 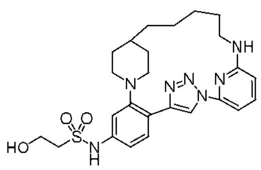 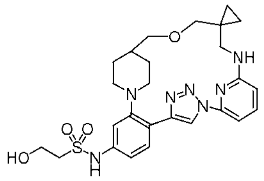

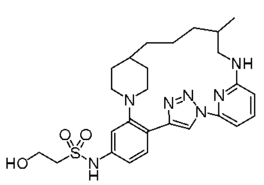 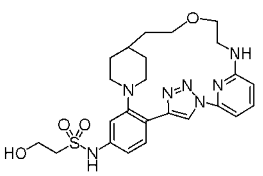 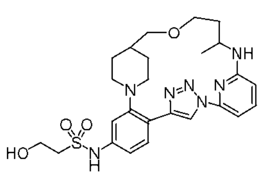 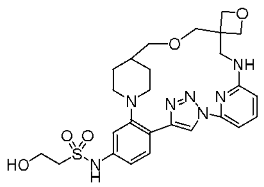

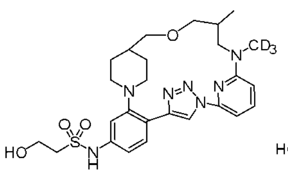 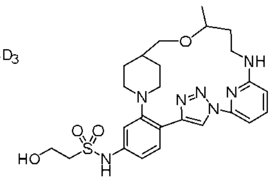 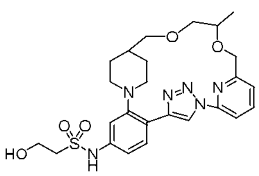 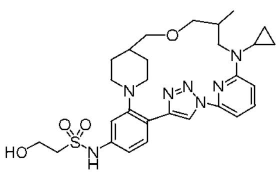

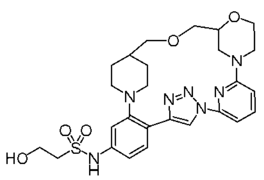 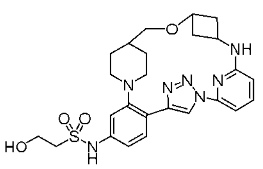 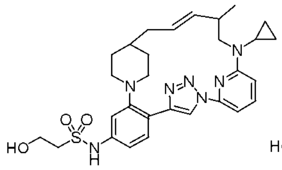 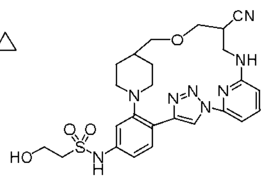

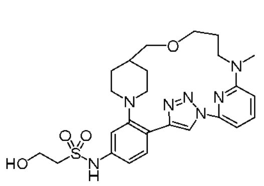 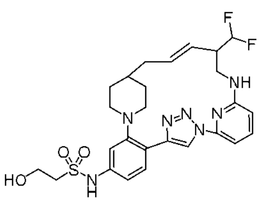 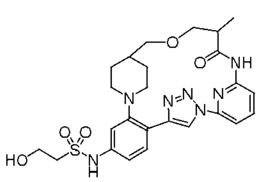 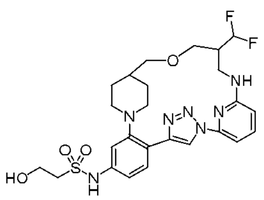

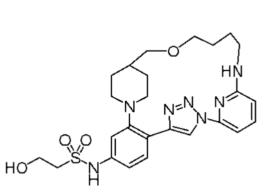 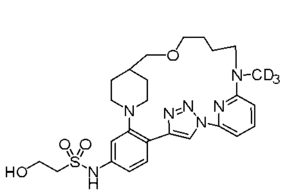 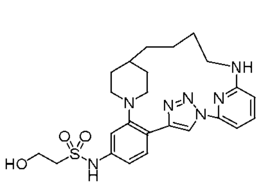 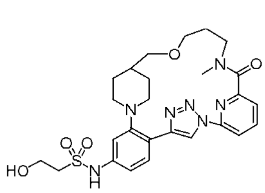

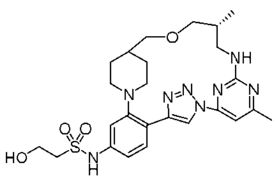 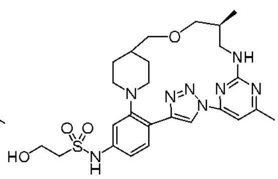 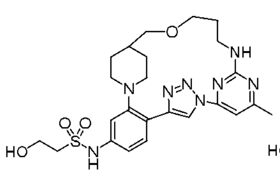 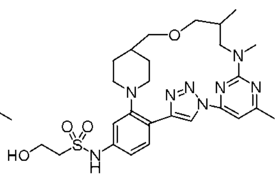

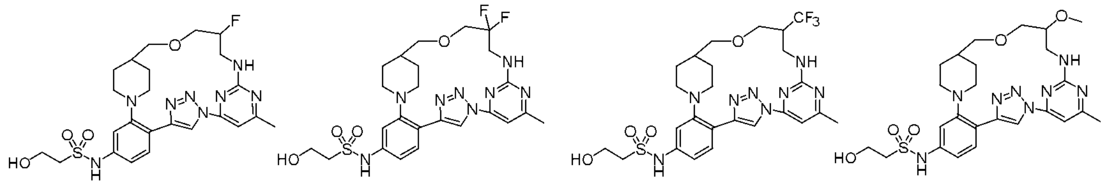

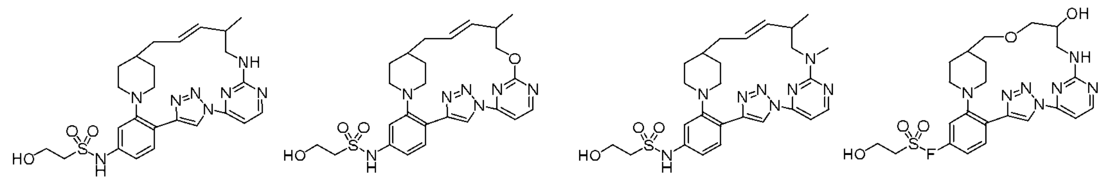

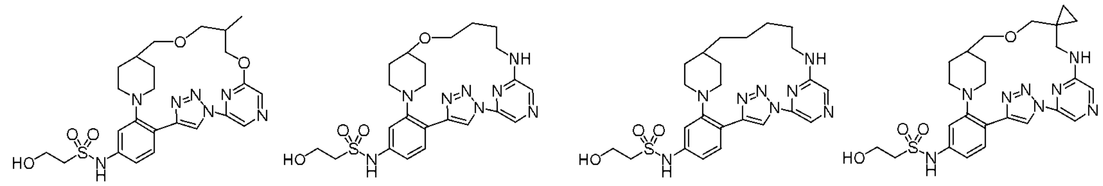

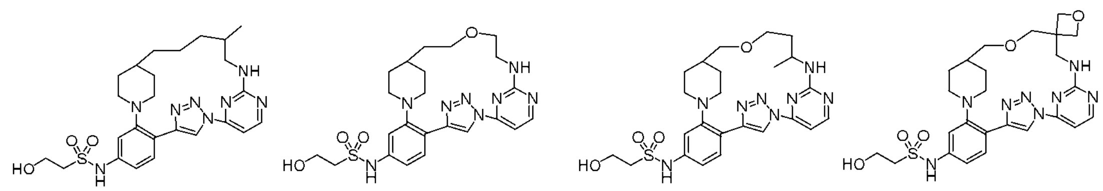

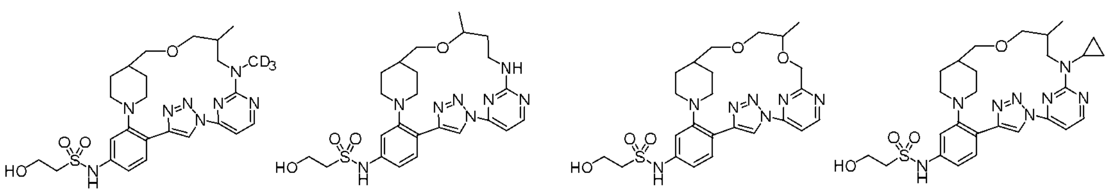

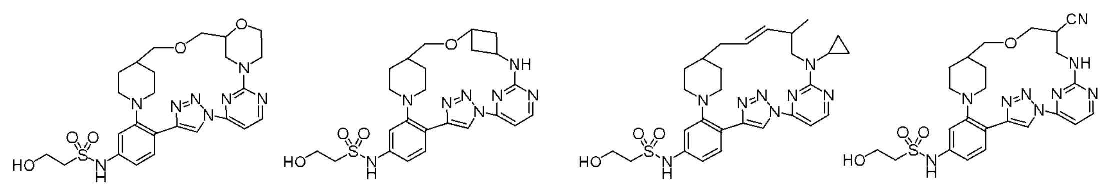

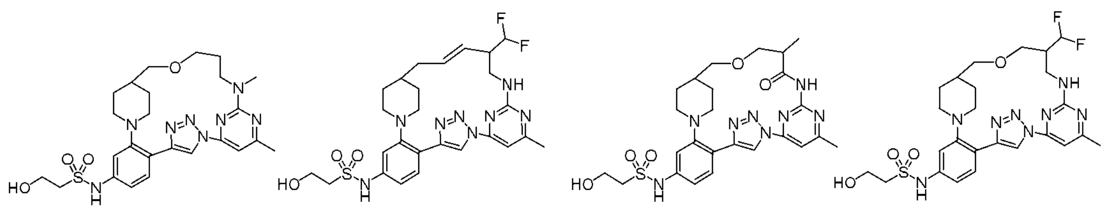

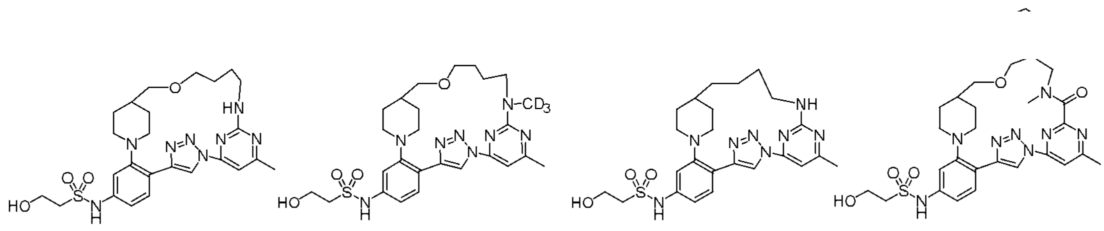

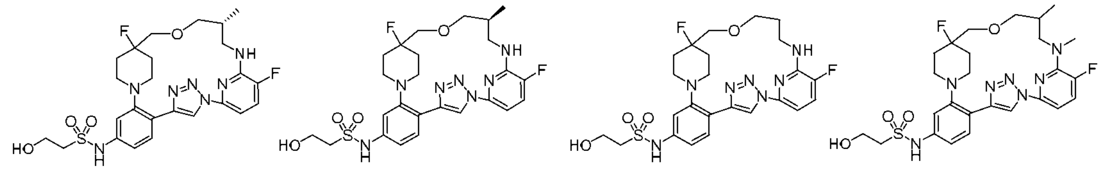

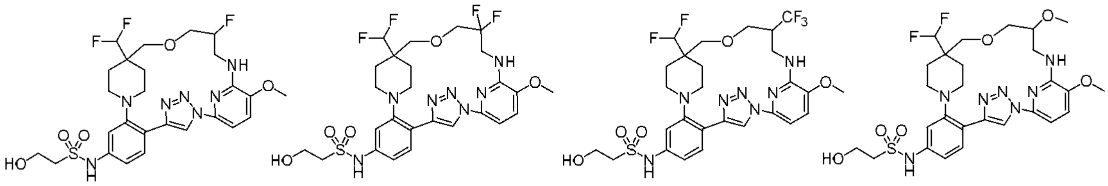

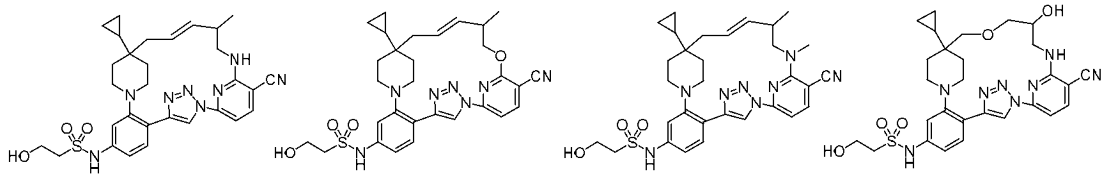

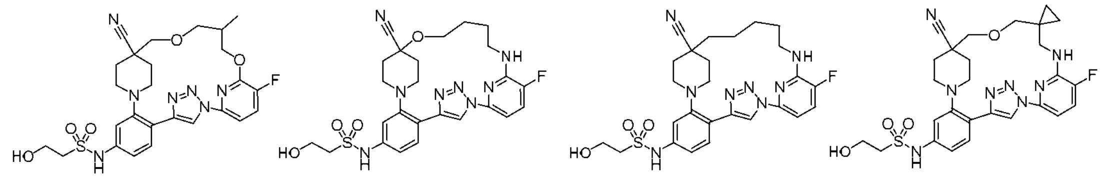

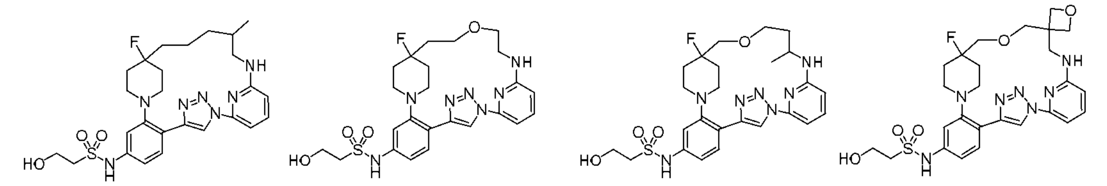

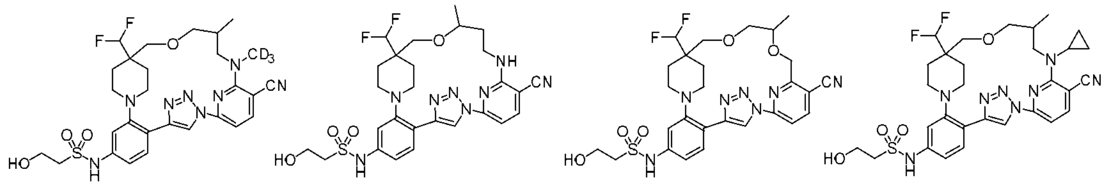

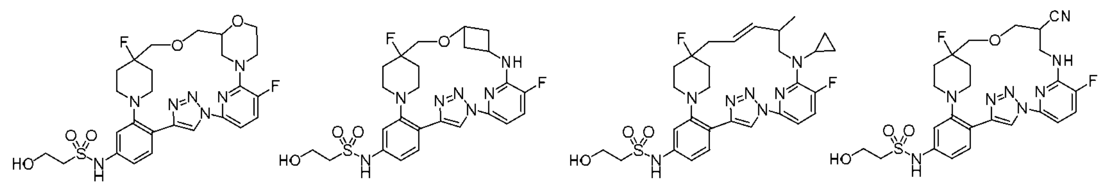

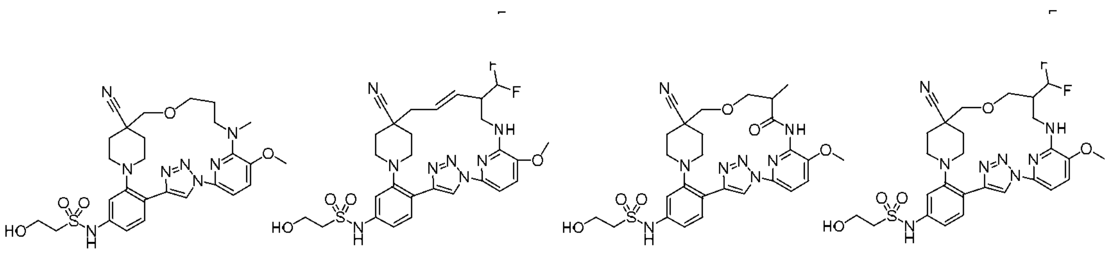

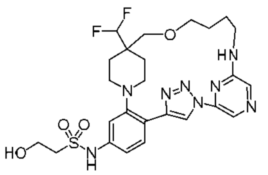
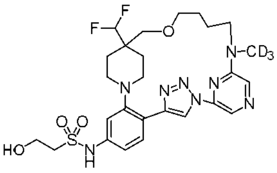
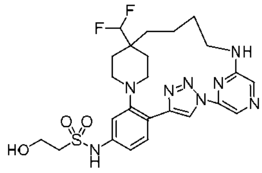
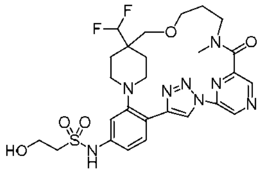
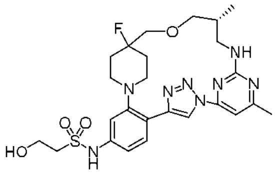
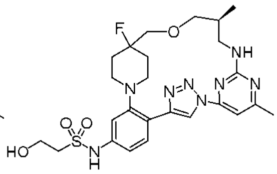
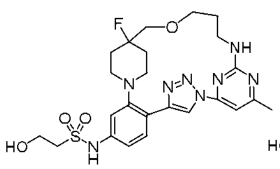
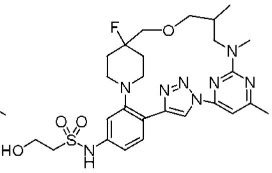
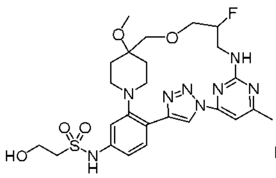
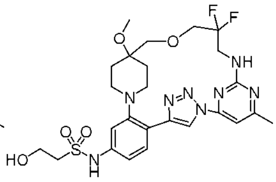
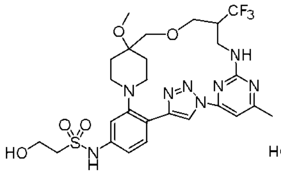
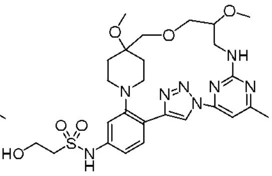
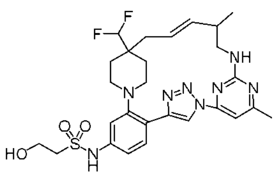
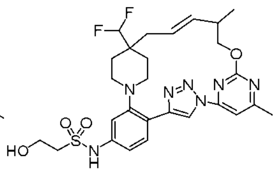
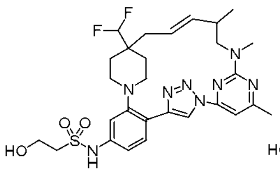
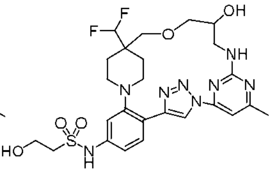
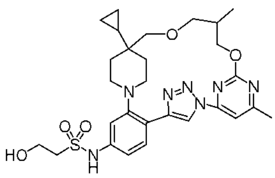
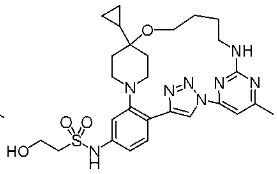
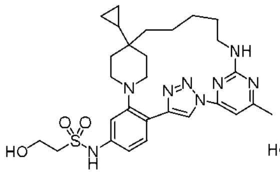
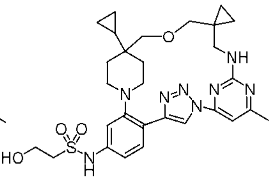
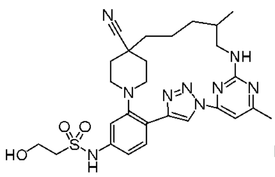
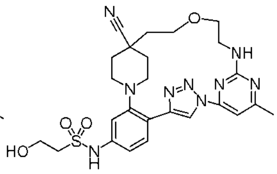
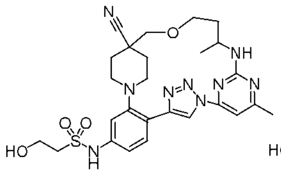
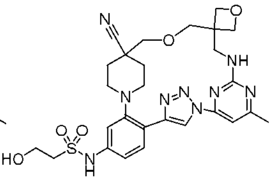
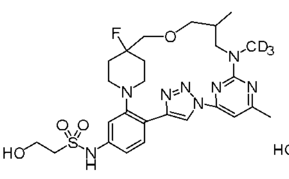
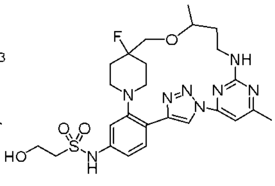
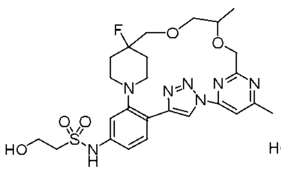
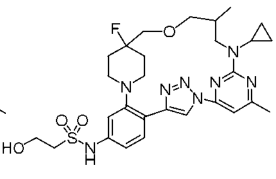
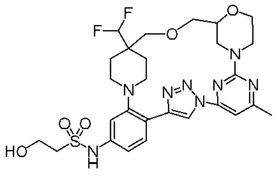
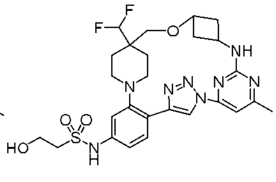
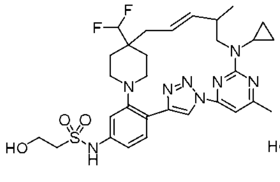
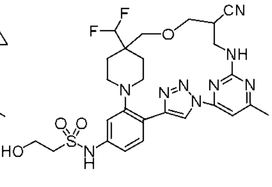

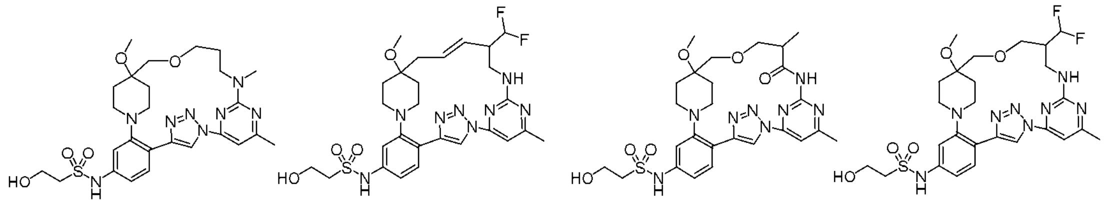

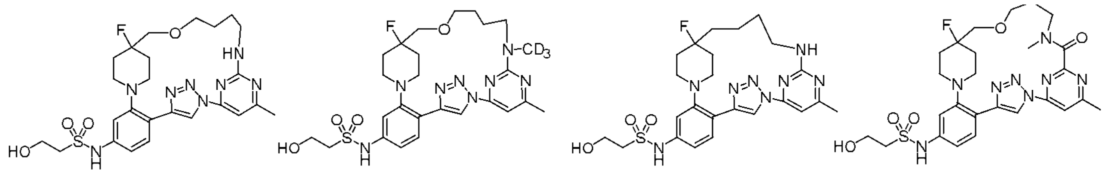

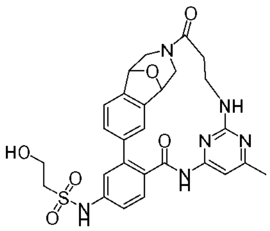
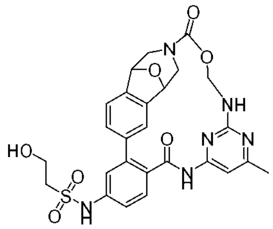
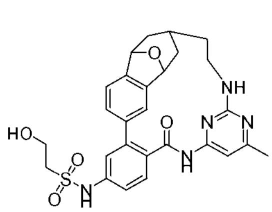
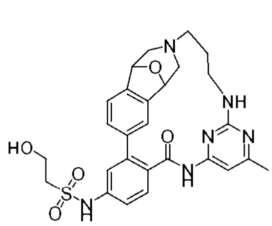

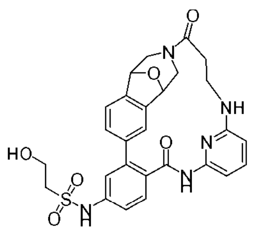
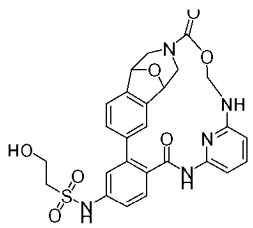
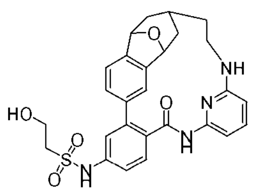
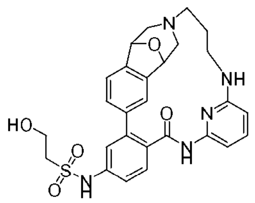

Furthermore, the present disclosure provides a pharmaceutical composition including any one of the above-mentioned compounds, or pharmaceutically acceptable salts, stereoisomers, isotope isomers or tautomers thereof.

In addition, the present disclosure provides a pharmaceutical composition that contains any one of the compounds in the Examples of the present disclosure or its pharmaceutically acceptable salts, stereoisomers, isotope isomers, prodrugs, hydrates or solvates and pharmaceutically acceptable carriers.

In addition, the present disclosure provides a method for treating tumors by inhibiting KIF18A, including administering any one of the compounds of the present disclosure or its pharmaceutically acceptable salts, stereoisomers, isotope isomers, prodrugs, hydrates or solvates to an individual in need.

## DEFINITION

Unless otherwise specified, the compounds of the present disclosure may encompass, in addition to their specific structures, pharmaceutically acceptable salts, stereoisomers, isotope isomers (e.g., deuterates), solvates, hydrates, prodrugs and metabolites thereof. Thus, the pharmaceutically acceptable salts, stereoisomers, isotope isomers, solvates, hydrates, prodrugs, and metabolites of these compounds are also considered within the scope of protection.

Unless otherwise specified, the terms used in this specification of patent and claims are defined as follows. Furthermore, many of the groups defined herein may be optionally substituted. A list of typical substituents is presented in Definition by way of example and is not intended to limit the substituents defined elsewhere in this specification of patent and claims.

Unless otherwise indicated, "R1", "$R^1$" and "$R_1$" have the same meaning and can be used interchangeably; similar definitions apply to other symbols such as R2.

The term "alkyl" refers to a linear or branched saturated aliphatic hydrocarbyl or linker, including 1-20 carbon atoms, preferably 1-12 carbon atoms, more preferably 1-8 carbon atoms, 1-6 carbon atoms or 1-4 carbon atoms. "Lower alkyl" specifically refers to an alkyl including 1-4 carbon atoms. Examples of alkyl include $-(CH_2)_3-$, methyl, trifluoromethyl, ethyl, propyl, isopropyl, n-butyl, isobutyl, tert-butyl and pentyl. Alkyl may be either substituted or unsubstituted. Typical

substituents include cycloalkyl, aryl, heteroaryl, heterocycloalkyl, hydroxyl, alkoxy, aryloxy, mercapto, alkylthio, arylthio, cyano, halo, carbonyl, thiocarbonyl, O-carbamoyl, N-carbamoyl, O-thiocarbamoyl, N-thiocarbamoyl, C-acylamino, N-amido, C-carboxyl, O-carboxyl, nitro, silyl, amino and $-NR^xR^y$; Wherein, $R^x$ and $R^y$ are independently selected from hydrogen, alkyl, cycloalkyl, aryl, carbonyl, acetyl, sulfonyl, trifluoromethanesulfonyl and a fused 5- or 6-membered heterocyclyl ring.

The term "alkenyl" refers to a linear or branched hydrocarbyl containing one or more double bonds and typically including 2-20 carbon atoms. For instance, "$C_2$-$C_6$ alkenyl" includes 2-6 carbon atoms. Examples of alkenyl include, but are not limited to, vinyl, propenyl, butenyl and 1-methyl-2-buten-1-yl.

The term "alkynyl" refers to a linear or branched hydrocarbyl containing one or more triple bonds and typically including 2-20 carbon atoms. For example, "$C_2$-$C_6$ alkynyl" includes 2-6 carbon atoms. Examples of alkynyl include, but are not limited to, ethynyl, 1-propynyl and 1-butynyl.

The term "alkoxy" or "alkyloxy" refers to -O-alkyl. "$C_1$-$C_6$ alkoxy" (or alkyloxy) is intended to include $C_1$, $C_2$, $C_3$, $C_4$, $C_5$ and $C_6$ alkoxy. Examples of alkoxy include, but are not limited to, methoxy, ethoxy, propoxy (e.g., n-propoxy and isopropoxy) and tert-butoxy. Similarly, "alkylthio" or "thioalkoxy" refers to sulfur-bridged alkyl including a specified number of carbon atoms as defined above; for example, methyl-S- and ethyl-S-.

The term "cycloalkyl" refers to a 3- to 8-membered all-carbon monocyclic or bicyclic ring, a 5-/6- or 6-/6-membered fused all-carbon bicyclic ring, or a fused polycyclic ring (in a "fused" ring system, each ring shares at least one adjacent carbon atom with other rings) group, in which one or more of the rings may contain one or more double bonds but none of such rings has an intact conjugated π-electron system, or two rings forms a spiro by sharing one carbon. Examples of cycloalkyl include, but are not limited to, cyclopropane, cyclobutane, cyclopentane, cyclopentene, cyclohexane, cyclohexadiene, adamantane, cycloheptane and cycloheptatriene. Bicyclic alkyl includes bridged, spiro or fused-ring cycloalkyl. Illustrative examples of cycloalkyl are derived from but not limited to the following:

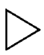
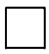
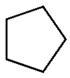
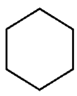
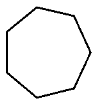
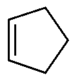
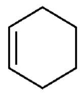
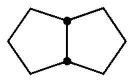

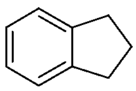
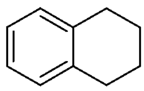
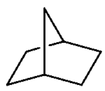
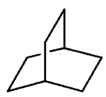
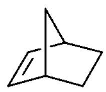
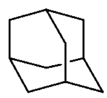

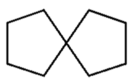
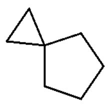
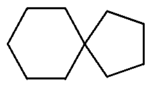
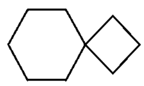

The term "aryl" refers to an all-carbon monocyclic or fused-ring polycyclic group including 6-12 carbon atoms, with an intact conjugated π-electron system. Examples of aryl include, but are not limited to, phenyl, naphthyl and anthracenyl. Aryl may be either substituted or unsubstituted. Typical substituents include halo, trihalomethyl, alkyl, hydroxy, alkoxy, aryloxy, mercapto, alkylthio, arylthio, cyano, nitro, carbonyl, thiocarbonyl, C-carboxyl, O-carbamoyl, N-carbamoyl, O-thiocarbamoyl, N-thiocarbamoyl, C-acylamino, N-amido, sulfinyl, sulfonyl, amino and $-NR^aR^b$, wherein $R^a$ and $R^b$ are

as defined above. An aryl-fused saturated or unsaturated cycloalkyl/saturated or unsaturated heterocycloalkyl can be regarded as a special substituent of aryl, with typical examples including but not limited to:

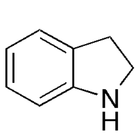
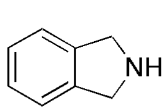
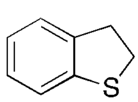
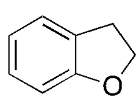
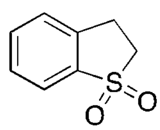

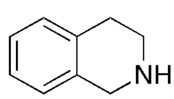
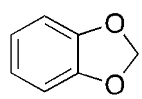

The term "heteroaryl" refers to a monocyclic or fused ring including 5-12 ring atoms, with one, two, three or four ring heteroatoms selected from N, O and S and the remaining ring atoms being C, having an intact conjugated π-electron system. Typical examples of heteroaryl include, but are not limited to, acridinyl, azetidinyl, azocinyl, benzimidazolyl,

benzofuryl, benzothiofuranyl, benzothienyl, benzoxazolyl, benzoxazolinyl, benzothiazolyl, benzotriazolyl, benzotetrazolyl, benzoisoxazolyl, benzisothiazolyl, benzimidazolinyl, carbazolyl, 4aH-carbazolyl, carbolinyl, chromanyl, chromenyl, cinnolinyl, decahydroquinolyl, 2H,6H-1,5,2-dithiazinyl, dihydrofuro[2,3-b]tetrahydrofuranyl, furanyl, furazanyl, imidazolidinyl, imidazolinyl, imidazolyl, 1H-indazolyl, imidazopyridinyl, indolenyl, dihydroindolyl, indolizinyl, indolyl, 3H-indolyl, isatinoyl, isobenzofuranyl, isochromanyl, isoindazolyl, isoindolinyl, isoindolyl, isoquinolinyl, isothiazolyl, isothiazolopyridyl, isoxazolyl, isoxazolopyridyl, methylenedioxyphenyl, morpholinyl, diazanaphthyl, octahydroisoquinolinyl, oxadiazolyl, 1,2,3-oxadiazolyl, 1,2,4-oxadiazolyl, 1,2,5-oxadiazolyl, 1,3,4-oxadiazolyl, oxazolidinyl, oxazolyl, oxazolopyridinyl, oxazolidinyl, perimidinyl, oxindolyl, pyrimidinyl, phenanthridinyl, phenanthrolinyl, phenazinyl, phenothiazinyl, phenoxathiinyl, phenoxazinyl, phthalazinyl, piperazinyl, piperidinyl, piperidonyl, 4-piperidonyl, piperonyl, pteridinyl, purinyl, pyranyl, pyrazinyl, pyrazolidinyl, pyrazolinyl, pyrazolopyridinyl, pyrazolyl, pyridazinyl, pyridoxazolyl, pyridinoimidazolyl, pyridothiazolyl, pyridyl, pyrimidinyl, pyrrolidinyl, pyrrolinyl, 2-pyrrolidonyl, 2H-pyrrolyl, pyrrolyl, quinazolinyl, quinolyl, 4H-quinolizinyl, quinoxalinyl, quinuclidinyl, tetrazolyl, tetrahydrofuryl, tetrahydroisoquinolyl, tetrahydroquinolyl, 6H-1,2,5-thiadiazinyl, 1,2,3-thiadiazolyl, 1,2,4-thiadiazolyl, 1,2,5-thiadiazolyl, 1,3,4-thiadiazolyl, thianthrenyl, thiazolyl, thienyl, thiazolopyridyl, thienothiazolyl, thienoxazolyl, thienoimidazolyl, thienyl, triazinyl, 1,2,3-triazolyl, 1,2,4-triazolyl, 1,2,5-triazolyl, 1,3,4-triazolyl and xanthyl, quinolyl, isoquinolinyl, phthalazinyl, quinazolinyl, indolyl, isoindolyl, dihydroindolyl, 1H-indazolyl, benzimidazolyl, 1,2,3,4-tetrahydroquinolyl, 1,2,3,4-tetrahydroisoquinolyl, 5,6,7,8-tetrahydro-quinolyl, 2,3-dihydro-benzofuranyl, chromanyl, 1,2,3,4-tetrahydro-quinoxalinyl and 1,2,3,4-tetrahydro-quinazolinyl. The term "heteroaryl" may also include biaryl structures formed by the above-defined "aryl" and monocyclic "heteroaryl", including but not limited to "-phenylbipyridyl-", "-phenylbipyrimidinyl", "-pyridylbiphenyl", "-pyridylbipyrimidinyl-" and "-pyrimidinylbiphenyl-"; wherein the present disclosure also includes fused ring and spiro ring compounds containing, for example, heterocyclic rings mentioned above.

Specifically, the term "5-6-membered heteroaryl" should be understood as an aromatic ring group having 5 or 6 ring atoms and 1, 2 or 3 heteroatoms independently selected from O, N or S; specific examples include but are not limited to: thienyl, furyl, pyrrolyl, oxazolyl, thiazolyl, imidazolyl, pyrazolyl, isoxazolyl, isothiazolyl, oxadiazolyl, triazolyl, thiadiazolyl; or pyridyl, pyridazinyl, pyrimidinyl, pyrazinyl, triazinyl.

Pharmaceutically acceptable refers to being sufficiently stable to be formulated into a pharmaceutical composition including the compound of the present disclosure, and subsequently administered to a patient in need thereof.

Unless otherwise defined, the substituents in the present disclosure are defined as being independently assigned rather than interdependently related. For example, for a substituent $R^a$ (or $R^b$), its definition in different substituents is independently assigned. Specifically, when $R^a$ (or $R^b$) is selected as one definition in one substituent, this does not imply that $R^a$ (or $R^b$) in other substituents must share the same definition. More specifically, when $R^a$ (or $R^b$) is defined as hydrogen in $NR^aR^b$, for example (non-exhaustive), it does not imply that $R^a$ (or $R^b$) in -C(O)-$NR^aR^b$ must be hydrogen.

"Halo" or "halogen" includes fluorine, chlorine, bromine and iodine. "Haloalkyl" is intended to include branched and linear saturated aliphatic hydrocarbyl having a specified number of carbon atoms substituted with 1 or more halogens. Examples of haloalkyl include, but are not limited to, fluoromethyl, difluoromethyl, trifluoromethyl, trichloromethyl, pentafluoroethyl, pentachloroethyl, 2,2,2-trifluoroethyl, heptafluoropropyl and heptachloropropyl. Examples of haloalkyl also include "fluoroalkyl" which is intended to include branched and linear saturated aliphatic hydrocarbyl having a specified number of carbon atoms and substituted with 1 or more fluorine atoms.

"Haloalkoxy" or "haloalkyloxy" refers to oxygen-bridged haloalkyl having a specified number of carbon atoms as defined above. For example, "$C_1$-$C_6$ haloalkoxy" is intended to include $C_1$, $C_2$, $C_3$, $C_4$, $C_5$ and $C_6$ haloalkoxy. Examples of haloalkoxy include, but are not limited to, trifluoromethoxy, 2,2,2-trifluoroethoxy and pentafluoroethoxy. Similarly, "haloalkylthio" or "thiohaloalkoxy" refers to sulfur-bridged haloalkyl including a specified number of carbon atoms as defined above; for example, trifluoromethyl-S- and pentafluoroethyl-S-.

In the present disclosure, the expression Cx1-Cx2 is used when referring to some substituents, which means that the number of carbon atoms in the substituent may be x1 to x2. For example, $C_0$-$C_8$ means that the group contains 0, 1, 2, 3, 4, 5, 6, 7 or 8 carbon atoms, $C_1$-$C_8$ means that the group contains 1, 2, 3, 4, 5, 6, 7 or 8 carbon atoms, $C_2$-$C_8$ means that the group contains 2, 3, 4, 5, 6, 7 or 8 carbon atoms, $C_3$-$C_8$ means that the group contains 3, 4, 5, 6, 7 or 8 carbon atoms, $C_4$-$C_8$ means that the group contains 4, 5, 6, 7 or 8 carbon atoms, $C_0$-$C_6$ means that the group contains 0, 1, 2, 3, 4, 5 or 6 carbon atoms, $C_1$-$C_6$ means that the group contains 1, 2, 3, 4, 5 or 6 carbon atoms, $C_2$-$C_6$ means that the group contains 2, 3, 4, 5 or 6 carbon atoms, and $C_3$-$C_6$ means that the group contains 3, 4, 5 or 6 carbon atoms.

In the present disclosure, the expression "x1-x2-membered ring" is used when referring to cyclic groups (e.g., aryl, heteroaryl, cycloalkyl and heterocycloalkyl), which means that the number of ring atoms in the group may be x1 to x2. For example, 3-12-membered cyclic group may be a 3, 4, 5, 6, 7, 8, 9, 10, 11 or 12-membered ring including 3, 4, 5, 6, 7, 8, 9, 10, 11 or 12 ring atoms; 3-6-membered cyclic group may be a 3, 4, 5 or 6-membered ring including 3, 4, 5 or 6 ring atoms; 3-8-membered cyclic group may be a 3, 4, 5, 6, 7 or 8-membered ring including 3, 4, 5, 6, 7 or 8 ring atoms; 3-9-membered cyclic group may be a 3, 4, 5, 6, 7, 8 or 9-membered ring including 3, 4, 5, 6, 7, 8 or 9 ring atoms; 4-7-membered cyclic group may be a 4, 5, 6 or 7-membered ring including 4, 5, 6 or 7 ring atoms; 5-8-membered cyclic group may be a 5, 6, 7 or 8-membered ring including 5, 6, 7 or 8 ring atoms; 5-12-membered cyclic group may be a 5, 6, 7, 8, 9, 10, 11 or 12-membered ring including 5, 6, 7, 8, 9, 10, 11 or 12 ring atoms; 6-12-membered cyclic group may be a 6, 7, 8, 9, 10, 11 or 12-membered ring including 6, 7, 8, 9, 10, 11 or 12 ring atoms. The ring atoms may be carbon atoms or heteroatoms, such as heteroatoms selected from N, O and S. The heterocyclic rings may contain 1, 2, 3, 4, 5, 6, 7, 8, 9, 10 or more ring heteroatoms, such as heteroatoms selected from N, O and S.

In the present disclosure, one or more halogens may independently be selected from fluorine, chlorine, bromine, and iodine.

The term "substituted" as used herein means that at least one hydrogen atom is substituted by a non-hydrogen group, provided that normal valence is maintained and the substitution results in a stable compound. The term "cyclic double bond" as used herein refers to a double bond formed between two adjacent ring atoms (e.g. C=C, C=N or N=N).

Where nitrogen atoms (e.g. amines) are present on the compounds of the present disclosure, these nitrogen atoms may be converted to N-oxides by treatment with an oxidizing agent (e.g. mCPBA and/or hydrogen peroxide) to obtain other compounds of the present disclosure. Therefore, the nitrogen atoms shown and claimed are deemed to encompass the nitrogen atoms shown and their N-oxides to obtain derivatives of the present disclosure.

When any variable occurs more than once in any composition or formula of a compound, its definition in each occurrence is independent of its definition in every other occurrence. Therefore, for example, if a group is shown substituted with 0-3 Rs, the group may be optionally substituted with up to three R groups, with R defined independently in each occurrence. Furthermore, combinations of substituents and/or variables are permitted only if such combinations result in stable compounds.

The term "patient" as used herein refers to an organism to be treated by a method of the present disclosure. Such organisms preferably include, but are not limited to, mammals (e.g. rodents, apes/monkeys, horses, cattle, pigs, dogs and cats) and most preferably humans.

The term "effective amount" as used herein refers to an amount of a drug or agent (i.e., a compound of the present disclosure) that will elicit, for example, a biological or medical response in a tissue, a system, an animal or a human sought by a researcher or clinician. Furthermore, the term "therapeutically effective amount" refers to an amount that results in improved treatment, cure, prevention or alleviation of a disease, condition or side effect, or reduction in the progression rate of a disease or condition, as compared to a corresponding subject who does not receive such amount. An effective amount may be administered in one or more doses and is not intended to be limited to a particular formulation or route of administration. The term also includes an amount effective to enhance normal physiological functions within its range.

The term "treatment" as used herein includes its broad sense and encompasses both therapeutic and/or prophylactic treatment of an object. Specifically, "treatment" includes any treatment that results in the alleviation, inhibition, elimination and relief and/or prevention of a condition, disease and disorder, such as alleviation, reduction, regulation, relief, elimination, prophylaxis, prevention or remission of symptoms thereof. The therapeutic treatment includes alleviating, inhibiting or relieving symptoms or conditions of a disease; inhibiting the occurrence of complications; alleviating the underlying metabolic syndrome; inhibiting the occurrence of a disease or condition such as controlling the progression of the disease or condition; alleviating a disease or condition; reducing a disease or condition; alleviating complications caused by a disease or condition, or treating signs caused by a disease or condition. The prophylactic treatment includes a prior treatment to prevent, block or delay, slow the onset or progression of a disease or condition or reduce the severity of the disease or condition.

Similarly, the term "therapeutic agent" also includes agents or reagents used in the therapeutic and/or prophylactic treatment of an object.

The terms "pharmaceutical" or "pharmaceutically acceptable" as used herein refer to compounds, substances, compositions and/or dosage forms that, within the scope of sound medical judgment, are suitable for contact with human and animal tissues without excessive toxicity, irritation, allergic reactions and/or other issues or complications, and commensurate with a reasonable benefit/risk ratio.

Specific Pharmaceutical and Medical Terms

The term "cancer" as used herein refers to an abnormal growth of cells that is uncontrollable and, under certain conditions, capable of metastasis (spread). This type of cancer includes, but is not limited to, solid tumors [such as bladder, intestine, brain, chest, uterus, heart, kidney, lung, lymphoid tissue (lymphoma), ovary, pancreas or other endocrine organs (e.g., thyroid gland), prostate and skin (melanoma)] or blood tumors (e.g., aleukemic leukemia).

The term "combined administration" or similar terms thereof, as used herein, refers to the administration of several selected therapeutic agents to a patient in the same or different modes of administration and at the same or different times.

The term "enhancement" or "capable of enhancing" as used herein refers to an expected outcome of an increase or extension in either potency or duration. Thus, in the context of enhancing the therapeutic effect of a drug, the term "capable of enhancing" refers to the ability of the drug to increase or extend its potency or duration within the system. The

term "enhancement value" as used herein refers to the ability to maximize the efficacy of another therapeutic agent in an ideal system.

The term "immune disease" refers to a disease or condition resulting from an adverse or harmful response to endogenous or exogenous antigens, typically leading to cellular dysfunction, or functional damage and impairment, or damage to organs or tissues that may produce immune symptoms.

The term "kit" is synonymous with "product packaging."

The terms "object", "subject" or "patient" include both mammals and non-mammals. Mammals include, but are not limited to, mammals including humans and non-human primates (such as orangutans, apes and monkeys), agricultural animals (such as cattle, horses, goats, sheep and pigs), domestic animals (such as rabbits and dogs), and laboratory animals including rodents (such as rats, mice and guinea pigs). Non-mammals include, but are not limited to, birds and fish. In a preferred Embodiment, the selected mammal is human.

As used herein, a compound or pharmaceutical composition, after administered, can relieve a disease, symptom or condition, particularly to reduce severity, delay onset, slow down progression or shorten duration. This applies whether the administration is fixed or temporary, continuous or intermittent, and can be attributed to or associated with the administration.

Route of Administration

Suitable routes of administration include, but are not limited to, oral, intravenous, rectal, aerosol, parenteral, ocular, pulmonary, transdermal, vaginal, ear canal, nasal and topical. In addition, by way of example only, routes of parenteral administration include intramuscular, subcutaneous, intravenous, intramedullary, ventricular, intraperitoneal, intralymphatic and intranasal.

The mode of administration of the compounds of the present disclosure invention may be topical. In certain Examples, long-acting formulations are administered by (subcutaneous or intramuscular) implantation or intramuscular injection. In another Example, administration is achieved by a targeted drug delivery system. For example, liposomes coated with organ-specific antibodies. In this Example, the liposomes are selectively directed to and absorbed by specific organs.

Pharmaceutical Composition and Dosage

The term "pharmaceutical carrier" as used herein refers to a pharmaceutical substance, composition or vehicle, such as a liquid or solid filler, a diluent, an excipient, a manufacturing aid (e.g., lubricant, talc, magnesium stearate, calcium stearate or zinc stearate or stearic acid) or a solvent-encapsulated substance, used to carry or deliver the target compound from one organ or part of the body to another. Each carrier must be "acceptable" in the sense of being compatible with other ingredients of the formulation and harmless to patients.

The term "pharmaceutical composition" refers to a composition including a compound of the present disclosure together with other optional pharmaceutically acceptable carriers. The "pharmaceutical carrier" refers to a medium generally accepted in the art for delivering a biologically active agent to animals (particularly mammals), including (i.e.) adjuvants, excipients or vehicles such as diluents, preservatives, filters, flow regulators, disintegrants, wetting agents, emulsifiers, suspending agents, sweeteners, corrigents, perfuming agents, antibacterial agents, antifungal agents, lubricants and dispersants, depending on the mode of administration and nature of the dosage form.

The pharmaceutical composition of the present disclosure may include a therapeutically effective amount of one or more compounds described in the present disclosure formulated with optionally one or more pharmaceutical carriers (additives) and/or diluents, and optionally one or more other therapeutic agents. The compounds of the present disclosure may be administered by any suitable means for any of the above uses, for example orally, such as in the form of tablets, pills, powders, granules, elixirs, tinctures, suspensions (including nanosuspensions, microsuspensions and spray-dried dispersions), syrups and emulsions; sublingually; buccally; parenterally, such as by subcutaneous, intravenous, intramuscular or intrasternal injection or infusion techniques (e.g. in the form of sterile injectable aqueous or non-aqueous solutions or suspensions); nasally, including administering to the nasal membrane, such as by inhalation spray; topically, such as in the form of creams or ointments; rectally, such as in the form of suppositories; or intratumorally. They may be administered alone; however, they are typically administered via a drug carrier selected based on the chosen route of administration and standard pharmaceutical practice.

Pharmaceutical carriers are formulated based on various factors known to those skilled in the art. These factors include, but are not limited to: the type and nature of the active agent formulated; the subject to whom the composition containing the active agent is to be administered; the intended route of administration of the composition; and the targeted therapeutic indication. Pharmaceutical carriers include aqueous and non-aqueous liquid media and various solid and semisolid dosage forms.

The above-mentioned carriers may include many different ingredients and additives in addition to the active

agent. These ingredients are included in the formulation for various reasons known to those skilled in the art, such as stabilizing active agents and binding agents. Descriptions of suitable pharmaceutical carriers and factors involved in carrier selection can be obtained from several readily available sources, such as Allen L. V. Jr. et al. Remington: The Science and Practice of Pharmacy (2 Volumes), 22nd Edition (2012), Pharmaceutical Press.

Dosage regimens of the compounds of the present disclosure will, of course, vary depending on known factors such as the pharmacodynamic properties of the particular agent and its mode and route of administration; the species, age, sex, health status, medical condition and weight of the recipient; the nature and severity of symptoms; the type of concurrent treatment; the frequency of treatment; the route of administration, renal and hepatic function of the patient and expected effects. According to general guidance, the daily oral dose of each active ingredient when used for a given effect should range from about 0.001 mg/day to 10-5000 mg/day, preferably from about 0.01 mg/day to 1000 mg/day, and most preferably from about 0.1 mg/day to 250 mg/day. During constant-rate infusion, the most preferred intravenous dose should range from about 0.01 mg/kg/min to 10 mg/kg/min. The compounds of the present disclosure may be administered in a single daily dose, or divided into two, three or four doses daily.

The compounds are typically administered in the form of mixtures with suitable pharmaceutical diluents, excipients or carriers (collectively referred to herein as pharmaceutical carriers) appropriately selected according to the intended form of administration (e.g., oral tablets, capsules, elixirs and syrups) and conventional pharmaceutical practice.

A dosage form (pharmaceutical composition) suitable for administration may contain about 1 mg-2000 mg of active ingredient per dosage unit. In any of these pharmaceutical compositions, the active ingredient will generally be present in an amount of about 0.1-95% by weight based on the total weight of the composition.

The scope of the present disclosure includes pharmaceutical compositions including a therapeutically effective amount of at least one compound of the invention as an active ingredient, alone or in combination with a pharmaceutical carrier. Optionally, the compounds of the present disclosure may be used alone, in combination with other compounds of the present disclosure or in combination with one or more other therapeutic agents (e.g. anticancer agents or other pharmaceutically active substances).

The compounds of the present disclosure (which may be used in a suitable hydrated form) and/or the pharmaceutical compositions of the present disclosure are formulated into pharmaceutical dosage forms by conventional methods known to those skilled in the art, regardless of the selected route of administration.

The actual dose level of the active ingredient in a pharmaceutical composition of the present disclosure may be adjusted to reach an amount effective for achieving the desired therapeutic response, composition and mode of administration and ensuring nontoxicity for a particular patient.

The dosage level will be selected depending on a variety of factors, including the activity of the particular compound of the present disclosure or its ester, salt or amide; route of administration; duration of administration; the excretion rate of the particular compound; the rate and extent of absorption; duration of treatment; other medications, compounds and/or substances used in combination with the particular compound; factors well known in the medical art such as age, sex, weight, condition, general health and previous medical history of the patient to be treated.

A physician or veterinarian of ordinary skill in the art can readily determine and prescribe an effective amount of a desired pharmaceutical composition. For example, to achieve a desired therapeutic effect, a physician or veterinarian may start with a dose level lower than the desired level for any compound of the present disclosure incorporated in a pharmaceutical composition, and then gradually increase the dose level until the desired effect is achieved. Typically, a suitable daily dose for a compound of the present disclosure will be the lowest dose of the compound effective to produce a therapeutic effect. This effective dose usually depends on the factors mentioned above. Typically, oral, intravenous, intracerebroventricular and subcutaneous doses for compounds of the present disclosure range from about 0.01 to 50 mg/kg body weight per day for use in patients. If desired, an effective daily dose of the active compound may be administered in two, three, four, five, six or more sub-doses at a suitable interval throughout the day, optionally in unit dosage form. In certain Examples of the present disclosure, administration is once daily.

Although the compounds of the present disclosure may be administered alone, they are preferably administered in the form of pharmaceutical formulations (compositions).

## Kit/Product Packaging

For use in the treatment of the above indications, kit/product packaging is also described herein. A kit may consist of a delivery device, a drug pack, or a container box which may be divided into several compartments to accommodate one or more containers such as vials, tubes and the like, each containing a separate ingredient involved in a method described herein. Suitable containers include bottles, vials, syringes and test tubes. These containers are made of acceptable materials such as glass or plastic.

For example, a container may contain one or more compounds described herein, either as a pharmaceutical ingredient or in a mixture with other ingredients described herein. The container may have a sterile outlet (e.g. the container

may be an intravenous infusion bag or bottle, and its stopper can be pierced by a hypodermic needle). A kit may contain a compound and a description, label or instructions for use for a method described herein.

A typical kit may include one or more containers, each containing one or more materials (e.g. reagents, concentrated stock solutions and/or devices) to accommodate commercial promotion and user needs for the use of the compound. These materials include, but are not limited to, buffers, diluents, filters, needles, syringes, delivery devices, bags, containers, bottles and/or test tubes, accompanied by a list of contents and/or instructions for use, and a description of built-in packaging (if any). The entire set of instructions should be included in the kit.

A label may appear on or closely associated with a container. The presence of a label on a container means that letters, numbers or other features are pasted, molded and engraved onto the container; the label may also appear inside a container box or transport box containing multiple containers, such as in a product insert. A label may be used to indicate a specific therapeutic use of the contents. The label may also carry instructions for the use of the contents, such as described in the methods above.

All features described in the specification (including any stated claim, abstract and figure) and/or all steps involved in any method or process may exist in any combination unless some features or steps are mutually exclusive in the same combination.

The above-mentioned features in the present disclosure or the features mentioned in Examples may be combined arbitrarily. All features disclosed in the specification may be used in conjunction with any composition form. Each feature disclosed in the specification may be replaced by any alternative feature that provides the same, equivalent or similar purpose. Therefore, unless otherwise specified, the features disclosed are only general examples of equivalent or similar features.

The present disclosure will be further described below in conjunction with specific Examples. It should be noted that these Examples are not intended to define the scope of the present disclosure but merely to describe the present disclosure. The experimental methods with no specific conditions indicated in the following Examples usually follow conventional conditions or the conditions recommended by manufacturers. Unless otherwise stated, all percentages, ratios, proportions or parts are measured by weight.

The unit of weight-to-volume percentage in the present disclosure is well known to those skilled in the art, for example, it refers to the weight of solute in 100 mL of solution. Unless otherwise defined, all technical and scientific terms used herein have the same meanings as those familiar to those skilled in the art. In addition, any methods and materials similar or equivalent to those described herein may be used in the methods of the present disclosure. The preferred methods and materials described herein are for exemplary purposes only.

## SPECIFIC IMPLEMENTATIONS

The present disclosure is further described through the following specific Examples, which are not intended to limit the scope of the present disclosure to these Examples. Experimental methods with no specific conditions indicated in the following Examples should be selected according to conventional methods and conditions, or commercial specifications. NMR was measured using Bruker AVANCE-400 NMR spectrometer. Solvents used for measurement were indicated in the spectrum interpretation.

MS measurement was conducted using Agilent 1200-G1956A/1200-6110A/1200-6140A/1260-6125B/Prime-6125B/1260-6120 liquid chromatograph-mass spectrometer (LC/MS), SHIMADZU 20A-2010/20A-2020 LC/MS and Waters ACQ-QDA LC/MS.

HPLC analysis was performed using a SHIMADZU 20A high performance liquid chromatograph.

SFC analysis and determination were conducted using Waters UPCC with PDA Detector and QDa Detector ultra-high performance convergence chromatograph, Waters UPC2 with PDA detector ultra-high performance convergence chromatograph, Agilent 1260 with DAD detector high-performance liquid chromatograph, Shimadzu LC-20AB with PDA detector high-performance liquid chromatograph and Shimadzu LC-20AD with PDA detector high-performance liquid chromatograph.

Preparative HPLC separation was conducted using Shimadzu LC-20AP pump, Shimadzu LH-40 Liquid Handler, Shimadzu SPD-20A Detector, Gilson GX-281 Liquid Handler, Gilson 322 pump and Gilson 156 UV Detector preparative chromatographs.

SFC separations were conducted using The Berger MG II, MG III, Sepiatec's Prep SFC 100 System, Waters Prep 80Q SFC SYSTEM, Prep 150 AP SFC SYSTEM, Prep 200 SFC SYSTEM, and Prep 350 SFC SYSTEM.

Flash column chromatography separation was conducted using Biotage IsoleraOne flash-preparative chromatograph.

TLC plates used were GF254 acrylic adhesive silica gel plates provided by Anhui Liangchen Silica Source Materials Co., Ltd. The TLC silica gel plates used for analysis were 0.25 mm in thickness, while those used for product purification were 0.5 mm in thickness.

Pressurized hydrogenation reaction is conducted using hydrogenation bottles and hydrogen gas cylinders.

Microwave reactions were performed using a Biotage Initiator+ microwave synthesizer.

A custom glove box from DELLIX was used.

The present disclosure is further described through the following Examples, which are not intended to impose any limitations on the present disclosure. The compounds of the present disclosure can be prepared by a variety of synthetic methods known to those skilled in the art, including the specific Examples listed below, Examples formed by their combination with other chemical synthesis methods and equivalent alternatives well known to those skilled in the art. Preferred Examples include, but are not limited to, the Examples of the present disclosure. It will be apparent to those skilled in the art that various changes and improvements can be made to specific Examples of the present disclosure without departing from the spirit and scope of the present disclosure.

Preparation of **Intermediate A**

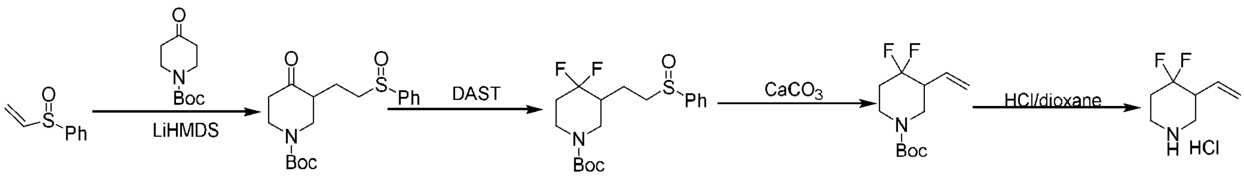


**Step 1:** At 25°C, N-tert-butoxycarbonyl-4-oxopiperidine (17.02 g, 85.41 mmol) was dissolved in tetrahydrofuran (50.00 mL). At -78°C under nitrogen atmosphere, the solution was slowly added to a solution of 1.0 M lithium bis(trimethylsilyl)amide (85.41 mL, 85.41 mmol) in tetrahydrofuran (150.00 mL). The reaction solution was stirred at -78°C for 1 hour and (vinylsulfinyl)benzene (10.0 g, 65.7 mmol) was added. The temperature was raised to 25°C, the mixture was stirred for 1 hour, then aqueous sodium hydroxide solution was added, and the mixture was stirred at 25°C for 1 hour. The mixture was extracted with ethyl acetate (300 mL × 2). The organic layer was washed with water (200 mL), dried over sodium sulfate and filtered. The filtrate was concentrated under reduced pressure to give a residue which was purified by flash column chromatography (silica gel, 0-30% gradient of ethyl acetate/petroleum ether) to give tert-butyl 4-oxo-3-(2-(phenylsulfinyl)ethyl)piperidine-1-carboxylate (16.00 g, 45.58 mmol, yield: 69.4%) as a yellow oil. LCMS (ESI): $[M+H]^+$ = 352.20.

**Step 2:** At -78°C and nitrogen atmosphere, to a solution of tert-butyl 4-oxo-3-(2-(phenylsulfinyl)ethyl)piperidine-1-carboxylate (16.00 g, 45.58 mmol) in dichloromethane (200.00 mL) was added diethylaminosulfur trifluoride (15.45 g, 91.05 mmol). The yellow mixture was stirred at 25°C for 16 h. The reaction was quenched with saturated sodium bicarbonate (200 mL) and the mixture was extracted with dichloromethane (200 mL × 3). The organic layer was washed with brine (200 mL), dried over anhydrous sodium sulfate and filtered. The filtrate was concentrated to give a crude product, which was purified by flash column chromatography (silica gel, 0-30% gradient of ethyl acetate/petroleum ether) to give tert-butyl 4,4-difluoro-3-(2-(phenylsulfinyl)ethyl)piperidine-1-carboxylate (6.90 g, 18.5 mmol, yield: 40.6%) as an orange oil. LCMS (ESI): $[M-99]^+$ = 274.00.

**Step 3:** A suspension of calcium carbonate (40.20 g, 401.65 mmol) and tert-butyl 4,4-difluoro-3-(2-(phenylsulfinyl)ethyl)piperidine-1-carboxylate (15.00 g, 40.16 mmol) in xylene (300 mL) was stirred at 140°C for 16 hours. The suspension was filtered and the filtrate was concentrated in a vacuum to give a crude product, which was purified by flash column chromatography (silica gel, 0-10% gradient of tetrahydrofuran/petroleum ether) to give tert-butyl 4,4-difluoro-3-vinylpiperidine-1-carboxylate (8.04 g, 32.51 mmol, yield: 81.0%) as a yellow oil. LCMS (ESI): $[M-55]^+$ = 192.10.

**Step 4:** At 20°C, to a 2 M solution of hydrogen chloride in 1,4-dioxane (200.00 mL) was added tert-butyl 4,4-difluoro-3-vinylpiperidine-1-carboxylate (10.73 g, 43.39 mmol). The reaction mixture was stirred at 25°C for 16 hours. Thin-layer chromatography (petroleum ether/ethyl acetate, 5:1 v/v) indicated complete reaction. The reaction solution was concentrated under reduced pressure to give 4,4-difluoro-3-vinylpiperidine hydrochloride (11.00 g, 42.57 mmol, yield: 98.1%, crude) as a light yellow solid. LCMS (ESI): $[M+H]^+$ = 148.10. $^1H$ NMR (400 MHz, DMSO-$d_6$) δ ppm 9.60 (br s, 2H), 5.90 - 5.65 (m, 1H), 5.46 - 5.24 (m, 2H), 3.47 - 3.36 (m, 2H), 3.23 - 3.07 (m, 1H), 3.05 - 2.91 (m, 2H), 2.41 - 2.23 (m, 2H)

Preparation of **Intermediate B**

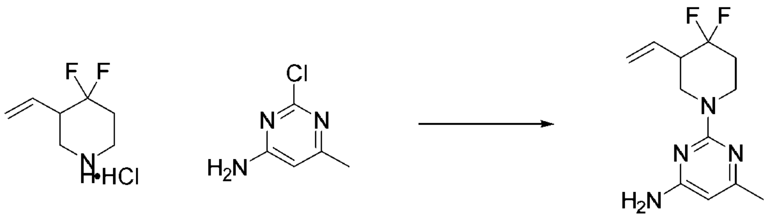

At 20°C, 4,4-difluoro-3-vinylpiperidine hydrochloride (1.00 g, 5.44 mmol), 2-chloro-6-methylpyrimidin-4-amine (782 mg, 5.44 mmol) and N,N-diisopropylethylamine (7.17 mL, 39.56 mmol) were dissolved in dry dimethyl sulfoxide (10.00 mL). The reaction mixture was stirred at 120°C for 24 hours. After the reaction was complete, the mixture was cooled naturally, and the reaction was quenched with cold water (100 mL). The mixture was extracted with ethyl acetate (200 mL × 3). The combined organic layer was washed with a sodium bicarbonate solution (100 mL), then dried, filtered and concentrated to give a crude product, which was purified by flash column chromatography (silica, 0-50% ethyl acetate/petroleum ether) to give 2-(4,4-difluoro-3-vinylpiperidin-1-yl)-6-methylpyrimidin-4-amine (1.30 g, 5.10 mmol, yield: 93.9%) as a colorless liquid, LCMS (ESI): $[M+H]^+$ =255.10. $^1H$ NMR (400 MHz, DMSO-$d_6$) δ ppm 6.50 - 6.17 (m, 2H), 5.91 - 5.71 (m, 1H), 5.65 (s, 1H), 5.38 - 5.25 (m, 2H), 4.61 - 4.43 (m, 2H), 3.19 - 3.01 (m, 2H), 2.74 - 2.56 (m, 1H), 2.15 - 1.97 (m, 4H), 1.93 - 1.72 (m, 1H)

Preparation of **Intermediate C**

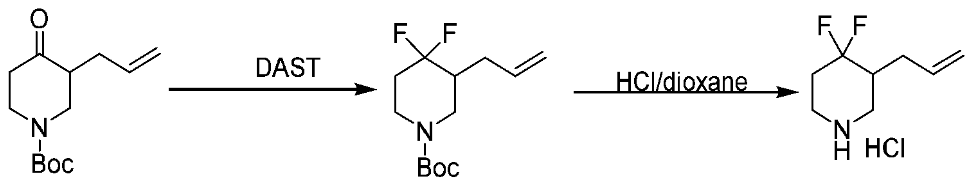


**Intermediate C** was given by a method similar to that for **intermediate A,** $^1H$ NMR (400 MHz, DMSO-$d_6$) δ ppm 9.67 (br s, 2H), 5.97-5.64 (m, 1H), 5.32-4.97 (m, 2H), 3.53-3.22 (m, 3H), 3.06-2.90 (m, 1H), 2.86-2.69 (m, 1H), 2.49-2.21 (m, 3H), 2.13-1.97 (m, 1H)

Preparation of **Intermediate D**

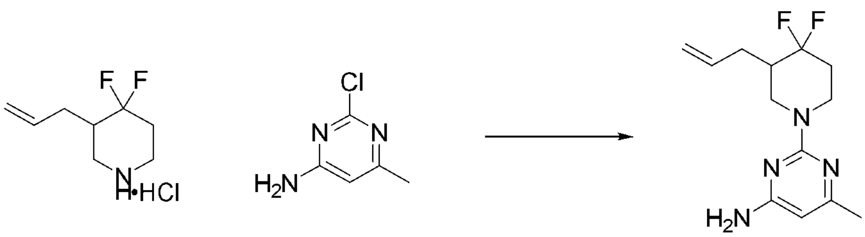

**Intermediate D** was given by a method similar to that for **intermediate B:** $^1H$ NMR (400 MHz, DMSO-$d_6$) δ ppm 6.29 (br s, 2H), 5.85 (tdd, J = 6.8, 10.0, 17.2 Hz, 1H), 5.64 (s, 1H), 5.25-5.02 (m, 2H), 4.54-4.35 (m, 2H), 3.23-3.11 (m, 1H), 2.90 (br dd, *J* = 9.2, 12.8 Hz, 1H), 2.44-2.31 (m, 1H), 2.06 (s, 3H), 2.05-1.69 (m, 4H)

Synthesis of **Intermediate E**

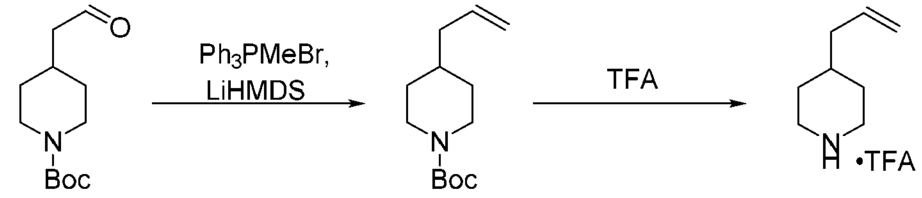


**Step 1:** Under nitrogen atmosphere at 20°C, methyltriphenylphosphonium bromide (24.06 g, 66.00 mmol) was dissolved in dry tetrahydrofuran (350.00 mL), the mixture was cooled to 0°C, and then a 1.0 M lithium bis(trimethylsilyl) amide solution in tetrahydrofuran (66.00 mL, 66.00 mmol) was slowly added and stirred for 1 hour. To the reaction system was added a solution of N-Boc-4-piperidineacetaldehyde (10.00 g, 44.00 mmol) in tetrahydrofuran (20.00 mL). The temperature was raised to 20°C and the mixture was stirred for 16 hours. After the reaction was complete, the reaction was quenched a saturated sodium bicarbonate solution (100 mL) and the mixture was extracted with ethyl acetate (200 mL × 3). The combined organic layer was dried, filtered and concentrated to give a crude product, which was purified by flash column chromatography (silica, 0-10% ethyl acetate/petroleum ether) to give tert-butyl 4-allylpiperidine-1-carboxylate (10.00 g, 66.00 mmol, yield: 100.0%) as a colorless liquid.

**Step 2:** At 20°C, tert-butyl 4-allylpiperidine-1-carboxylate (10.00 g, 66.00 mmol) was dissolved in dichloromethane (200.00 mL), then trifluoroacetic acid (10.02 mL, 131.81 mmol) was added and the mixture was stirred at 20°C for 16 hours. After the reaction was complete, the solvent and excess trifluoroacetic acid were removed to give 4-allylpiperidine trifluoroacetate (about 15.50 g, crude). $^1$H NMR (400 MHz, DMSO-$d_6$) $\delta$ = 9.36 - 8.94 (m, 2H), 5.75 (tdd, *J* = 6.8, 10.0, 16.8 Hz, 1H), 5.13 - 4.93 (m, 2H), 3.18 (br d, *J* = 12.4 Hz, 2H), 2.84 - 2.72 (m, 2H), 1.97 (t, *J* = 6.8 Hz, 2H), 1.73 (br d, *J* = 13.2 Hz, 2H), 1.58 (dtd, *J* = 4.0, 7.0, 14.8 Hz, 1H), 1.41 - 1.29 (m, 2H)

Synthesis of **Intermediate F**

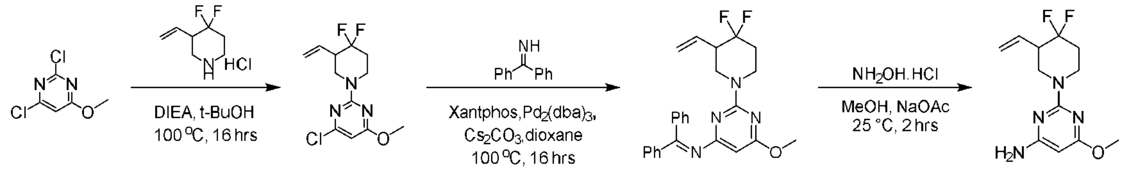


**Step 1:** 2,4-dichloro-6-methoxypyrimidine (20.0 g, 111 mmol) was dissolved in tert-butanol (400 mL), N,N-diisopropylethylamine (57.7 g, 446.93 mmol) and 4,4-difluoro-3-vinylpiperidine hydrochloride (26.6 g, 145 mmol) were added and the mixture was stirred at 100°C for 16 hours. The reaction mixture was diluted with water (500 mL), extracted with ethyl acetate (400 mL × 2), and the organic phase was dried, filtered and concentrated. The residue was purified by flash column chromatography (silica gel, 0-3% gradient of tetrahydrofuran/petroleum ether) to give 4-chloro-2-(4,4-difluoro-3-vinylpiperidin-1-yl)-6-methoxypyrimidine (27.8 g, 47.7 mmol, yield: 43.0%) as a yellow oil. LCMS (ESI): $[M+H]^+$ = 289.8. $^1$H NMR (400 MHz, CHLOROFORM-d) $\delta$ ppm 6.09 - 6.02 (m, 1H), 5.93 - 5.79 (m, 1H), 5.39 - 5.32 (m, 2H), 4.60 - 4.47 (m, 2H), 3.95 - 3.89 (m, 3H), 3.49 - 3.32 (m, 2H), 2.74 - 2.57 (m, 1H), 2.25 - 1.81 (m, 2H)

**Step 2:** 4-chloro-2-(4,4-difluoro-3-vinylpiperidin-1-yl)-6-methoxypyrimidine (10.0 g, 34.5 mmol) and diphenylmethanimine (9.57 g, 51.7 mmol) were dissolved in 1,4-dioxane (100 mL). Cesium carbonate (33.7 g, 103 mmol) and 4,5-bis(diphenylphosphine)-9,9-dimethylxanthene (3.99 g, 6.90 mmol) and tris(dibenzylideneacetone)dipalladium (3.16 g, 3.45 mmol) were added. The mixture was stirred at 100°C for 16 hours under nitrogen atmosphere. The reaction solution was diluted with water (200 mL) and extracted with ethyl acetate (200 mL × 3). The organic layer was dried, filtered and concentrated to give N-(2-(4,4-difluoro-3-vinylpiperidin-1-yl)-6-methoxypyrimidin-4-yl)-1,1-diphenylmethanimine (15.0 g, crude) as a brown oil, which was directly used in the next step. LCMS (ESI): $[M+H]^+$ = 435.1

**Step 3:** N-(2-(4,4-difluoro-3-vinylpiperidin-1-yl)-6-methoxypyrimidin-4-yl)-1,1-diphenylmethanimine (15.0 g, 34.5 mmol) was dissolved in methanol (300 mL), sodium acetate (8.50 g, 103 mmol) and hydroxylamine hydrochloride (4.80 g, 69.0 mmol) were added and the mixture was stirred at 25°C for 2 hours. To the reaction solution was added water (400 mL) and the mixture was extracted with ethyl acetate (400 mL × 2). The organic layer was dried, filtered and concentrated to give a residue, which was purified by flash column chromatography (silica gel, 0-10% gradient of tetrahydrofuran/petroleum ether) to give 2-(4,4-difluoro-3-vinylpiperidin-1-yl)-6-methoxypyrimidin-4-amine (7.90 g, 26.5 mmol, yield: 77.0%) as a yellow oil. LCMS (ESI): $[M+H]^+$ = 271.0. $^1$H NMR (400 MHz, CHLOROFORM-d) $\delta$ ppm 5.78 (ddd, *J* = 8.0, 10.4, 18.0 Hz, 1H), 5.27 - 5.18 (m, 2H), 5.11 (s, 1H), 4.50 - 4.35 (m, 4H), 3.75 (s, 3H), 3.26 - 3.07 (m, 2H), 2.63 - 2.43 (m, 1H), 2.06 - 1.89 (m, 1H), 1.87 - 1.65 (m, 1H)

Synthesis of **Intermediate G**

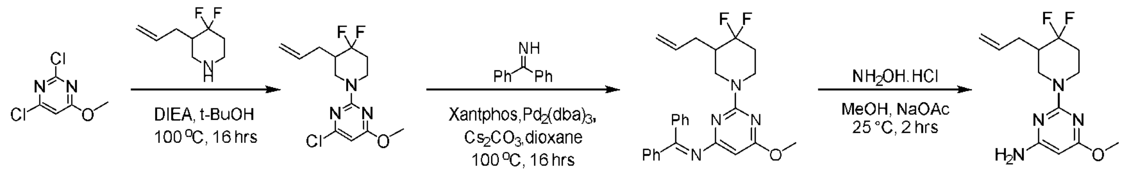


**Intermediate G** given by a method similar to that for **intermediate F,** $^1H$ NMR (400 MHz, CHLOROFORM-d) δ ppm 5.86-5.69 (m, 1H), 5.11 (s, 1H), 5.10-4.98 (m, 2H), 4.49-4.31 (m, 4H), 3.77-3.73 (m, 3H), 3.31-3.19 (m, 1H), 3.03-2.89 (m, 1H), 2.50-2.39 (m, 1H), 2.00-1.75 (m, 4H)

Synthesis of **Intermediate H**

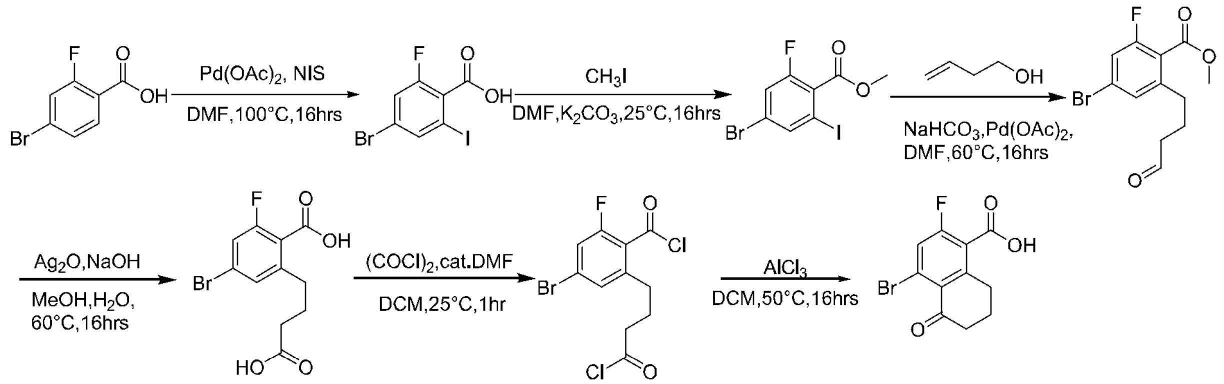


**Step 1:** 4-Bromo-2-fluorobenzoic acid (5.00 g, 22.8 mmol) and N-iodosuccinimide (5.13 g, 22.8 mmol) were dissolved in N,N-dimethylformamide (50 mL), palladium (II) acetate (0.52 g, 2.28 mmol) was added and the mixture was stirred at 100°C for 16 hours under nitrogen atmosphere. The reaction solution was diluted with water (50.0 mL) and the pH was adjusted to 3 with dilute hydrochloric acid. The mixture was extracted with ethyl acetate (50 mL × 3). The organic layer was dried, filtered and concentrated. The residue was purified by flash column chromatography (silica gel, 0-30% gradient of tetrahydrofuran/petroleum ether) to give 4-bromo-2-fluoro-6-iodobenzoic acid (6.00 g, 17.4 mmol, yield: 76.2%) as a yellow solid.

**Step 2:** 4-Bromo-2-fluoro-6-iodobenzoic acid (6.00 g, 17.4 mmol) was dissolved in N,N-dimethylformamide (60 mL), iodomethane (3.70 g, 26.1mmol) was added and the mixture was stirred at 25°C for 16 hours. The reaction mixture was diluted with water (50.0 mL), extracted with ethyl acetate (50 mL × 3), and the organic phase was dried, filtered and concentrated. The residue was purified by flash column chromatography (silica gel, 0-15% gradient of tetrahydrofuran/petroleum ether) to give methyl 4-bromo-2-fluoro-6-iodobenzoate (4.00 g, 11.1 mmol, yield: 64.0%) as a yellow solid. LCMS (ESI): No molecular ion was detected.

**Step 3:** Methyl 4-bromo-2-fluoro-6-iodobenzoate (500 mg, 1.39 mmol), but-3-en-1-ol (150 mg, 2.09 mmol), sodium bicarbonate (350 mg, 4.18 mmol) and tetra-n-butylammonium bromide (460 mg, 1.39 mmol) were dissolved in N,N-dimethylformamide (5.00 mL). To the reaction solution was added palladium (II) acetate (32.0 mg, 139 mmol). The temperature was raised to 60°C and the mixture was stirred for 16 hours under nitrogen atmosphere. To the reaction solution was added ammonium bicarbonate (10 mL). The mixture was extracted with ethyl acetate (50 mL × 3). The organic layer was dried, filtered and concentrated. The residue was purified by flash column chromatography (silica gel, 0-15% gradient of tetrahydrofuran/petroleum ether) to give methyl 4-bromo-2-fluoro-6-(4-oxobutyl)benzoate (150 mg, 0.49 mmol, yield: 35.5%) as a yellow oil. LCMS (ESI): $[M+H]^+$ = 302.9. $^1H$ NMR (400 MHz, DMSO-$d_6$) δ ppm 9.64 (t, $J$ = 1.2 Hz, 1H), 7.57 (dd, $J$ = 1.6, 9.2 Hz, 1H), 7.50 - 7.43 (m, 1H), 3.88 (s, 3H), 2.62 (dd, $J$ = 6.8, 8.8 Hz, 2H), 2.46 - 2.42 (m, 2H), 1.80 - 1.72 (m, 2H)

**Step 4:** Methyl 4-bromo-2-fluoro-6-(4-oxobutyl)benzoate (750 mg, 2.47 mmol) and sodium hydroxide (299 mg, 7.42 mmol) were dissolved in methanol (5.00 mL) and water (5.00 mL). To the reaction solution was added silver oxide (1.17 g, 4.94 mmol). The temperature was raised to 60°C and the mixture was stirred for 16 hours under nitrogen

atmosphere. The reaction solution was adjusted to pH 3 with dilute hydrochloric acid. The mixture was extracted with ethyl acetate (10 mL × 3). The organic layer was dried, filtered and concentrated to give 4-bromo-2-(3-carboxypropyl)-6-fluorobenzoic acid (750 mg, 2.45 mmol, yield: 99.3%) as a yellow oil. LCMS (ESI): $[M+H]^+$ = 326.9.

**Step 5:** 4-Bromo-2-(3-carboxypropyl)-6-fluorobenzoic acid (750 mg, 2.46 mmol) was dissolved in dichloromethane (15 mL). To the reaction solution were added oxalyl chloride (0.96 g, 7.37 mmol) and one drop of N,N-dimethylformamide . The solution was stirred at 25°C for 1 h. The reaction solution was concentrated under reduced pressure to give 4-bromo-2-(4-chloro-4-oxobutyl)-6-fluorobenzoyl chloride (0.80 g, 2.33 mmol, yield: 95.1%, crude) as a yellow oil, which was directly used in the next step. LCMS (ESI): $[M+H]^+$ = 335.0 (sample submitted in methanol)

**Step 6:** Aluminum chloride (0.88 g, 6.57 mmol) was dissolved in dichloromethane (15 mL). To the reaction solution was added a solution of methyl 4-bromo-2-(4-chloro-4-oxobutyl)-6-fluorobenzoyl chloride (0.75 g, 2.19 mmol) in dichloromethane (2 mL). The temperature was raised to 50°C and the mixture was stirred for 16 hours. The reaction mixture was diluted with water (20 mL), extracted with ethyl acetate (20 mL × 3), and the organic phase was dried, filtered and concentrated. The residue was purified by flash column chromatography (silica gel, 0-25% gradient of tetrahydrofuran/petroleum ether) to give 4-bromo-2-fluoro-5-oxo-5,6,7,8-tetrahydronaphthalene-1-carboxylic acid (600 mg, 2.08 mmol, yield: 95.3%) as a yellow solid. LCMS (ESI): $[M+H]^+$ = 287.0.

Synthesis of **Intermediate I**

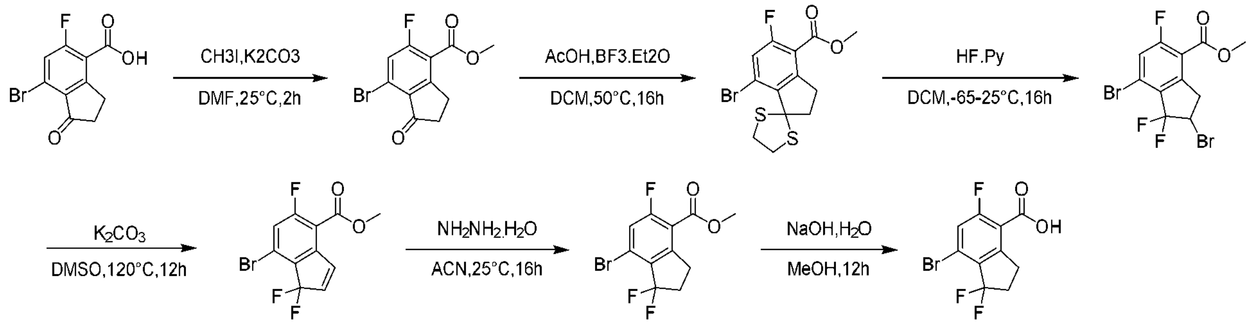


**Step 1:** 7-Bromo-5-fluoro-1-oxo-2,3-dihydro-1H-indene-4-carboxylic acid (3.00 g, 11.0 mmol) was dissolved in dimethylformamide (30 mL) and cooled to 0°C, potassium carbonate (3.80 g, 27.5 mmol) and iodomethane (2.34 g, 16.5 mmol) were added, and the reaction solution was stirred at 25°C for 2 hours. The reaction was quenched with water (100 mL) and the reaction solution was extracted with ethyl acetate (100 mL × 2). The combined organic layer was dried over anhydrous magnesium sulfate, then filtered and concentrated to give a crude product. The product was purified by flash column chromatography (silica gel, 0-10% gradient of tetrahydrofuran/petroleum ether) to give methyl 7-bromo-5-fluoro-1-oxo-2,3-dihydro-1H-indene-4-carboxylate (2.50 g, 8.69 mmol, yield: 79%) as a white solid. LCMS (ESI): $[M+H]^+$ =288.7

**Step 2:** At 25°C, to dichloromethane (20 mL) were added methyl 7-bromo-5-fluoro-1-oxo-2,3-dihydro-1H-indene-4-carboxylate (1.00 g, 3.48 mmol), then boron trifluoride diethyl etherate (2.97 g, 20.9 mmol), ethanedithiol (0.98 g, 10.5 mmol) and acetic acid (0.50 mL). The reaction solution was stirred at 50°C for 16 hours. The reaction was quenched with a saturated ammonium chloride solution (30 mL) and the reaction solution was extracted with dichloromethane (30 mL × 3). The organic phases were combined, dried over anhydrous magnesium sulfate, and filtered. The filtrate was dried under reduced pressure to give a residue. The residue was purified by flash column chromatography (silica gel, 0-30% gradient of ethyl acetate/petroleum ether) to give methyl 7'-bromo-5'-fluoro-2',3'-dihydrospiro[1,3-dithiolane-2,1'-indene]-4'-carboxylate (0.8 g, 2.20 mmol, yield: 63.2%) as a white solid. $^1$H NMR (400 MHz, CHLOROFORM-d) δ ppm 7.30 (d, $J$ = 10.0 Hz, 1H), 3.92 (s, 3H), 3.64 - 3.55 (m, 2H), 3.47 - 3.39 (m, 2H), 3.19 (t, $J$ = 6.8 Hz, 2H), 2.73 (t, $J$ = 6.8 Hz, 2H)

**Step 3:** To dichloromethane (80 mL) was added dibromohydantoin (4.10 g, 14.0 mmol) under nitrogen atmosphere. The reaction solution was cooled to -65°C, a solution of hydrogen fluoride in pyridine (8 mL) was added dropwise, then a solution of methyl 7'-bromo-5'-fluoro-2',3'-dihydrospiro[1,3-dithiolane-2,1'-indene]-4'-carboxylate (1.70 g, 4.68 mmol) in dichloromethane (10 mL) was added dropwise. The mixture was slowly heated to 25°C from -65°C and stirred for 16 hours. The reaction was quenched with 2N sodium hydroxide solution (50 mL) and sodium thiosulfate solution (5 mL). The reaction solution was extracted with dichloromethane (50 mL × 3). The organic layer was dried over anhydrous magnesium sulfate and filtered. The filtrate was concentrated under reduced pressure to give a residue. The residue was purified by flash column chromatography (silica gel, 0-10% gradient of tetrahydrofuran/petroleum ether) to give methyl 2,7-dibromo-1,1,5-trifluoro-2,3-dihydro-1H-indene-4-carboxylate (1.30 g, 3.37 mmol, yield: 72%) as a yellow oil. $^1$H NMR (400 MHz, DMSO-$d_6$) δ ppm 7.89 (d, *J* = 10.4 Hz, 1H), 5.20 - 4.99 (m, 1H), 3.96 - 3.88 (m, 1H), 3.87 (s, 3H), 3.33 - 3.28 (m, 1H)

**Step 4:** At 25°C, to a solution of methyl 2,7-dibromo-1,1,5-trifluoro-2,3-dihydro-1H-indene-4-carboxylate (1.30 g, 3.37 mmol) in dimethyl sulfoxide (20.0 mL) was added potassium carbonate (1.16 g, 8.38 mmol), and the reaction mixture was stirred at 120°C for 12 hours. The reaction system was diluted with water (30 mL) and extracted with ethyl acetate (30 mL × 3). The organic layer was dried over anhydrous magnesium sulfate and filtered. The filtrate was concentrated under reduced pressure to give a residue. The residue was purified by flash column chromatography (silica gel, 0-8% gradient of tetrahydrofuran/petroleum ether) to give methyl 7-bromo-1,1,5-trifluoro-1H-indene-4-carboxylate (0.22 g, 0.66 mmol, yield: 21%) as a yellow solid. $^1$H NMR (400 MHz, DMSO-*d6*) δ ppm 7.63 (d, *J* = 10.4 Hz, 1H), 7.29 (d, *J* = 6.0 Hz, 1H), 6.76 (d, *J* = 6.0 Hz, 1H), 3.88 (s, 3H)

**Step 5:** Methyl 7-bromo-1,1,5-trifluoro-1H-indene-4-carboxylate (200 mg, 0.65 mmol) and 2-nitrobenzene-1-sulfonyl chloride (289 mg, 1.30 mmol) were dissolved in acetonitrile (5 mL), the solution was cooled to 0°C, hydrazine hydrate (163 mg, 2.61 mmol) was added, and the mixture was stirred at 25°C for 16 hours. The reaction mixture was diluted with water (10 mL), extracted with ethyl acetate (10 mL × 3), and the organic phase was dried, filtered and concentrated. The residue was purified by flash column chromatography (silica gel, 0-8% gradient of tetrahydrofuran/petroleum ether) to give methyl 7-bromo-1,1,5-trifluoro-2,3-dihydro-1H-indene-4-carboxylate (0.1 g, 0.32 mmol, yield: 50%) as a white solid. $^1$H NMR (400 MHz, DMSO-d6) δ ppm 7.79 (d, *J* = 10.4 Hz, 1H), 3.86 (s, 3H), 3.26 - 3.20 (m, 2H), 2.73 - 2.61 (m, 2H)

**Step 6:** At 25°C, to a solution of methyl 7-bromo-1,1,5-trifluoro-2,3-dihydro-1H-indene-4-carboxylate (0.10 g, 0.32 mmol) in methanol (1.00 mL) and water (1.00 mL) was added sodium hydroxide (104 mg, 2.58 mmol), and the reaction mixture was stirred at 25°C for 12 hours. The reaction solution was diluted with water (5 mL), adjusted to pH 2-3 with a 1N dilute hydrochloric acid solution, and extracted with ethyl acetate (10 mL × 2). The organic layer was dried over magnesium sulfate, filtered and concentrated under reduced pressure to give 7-bromo-1,1,5-trifluoro-2,3-dihydro-1H-indene-4-carboxylic acid (95 mg, 0.29 mmol, yield: 92%) as a yellow solid.

Synthesis of **Intermediate J**

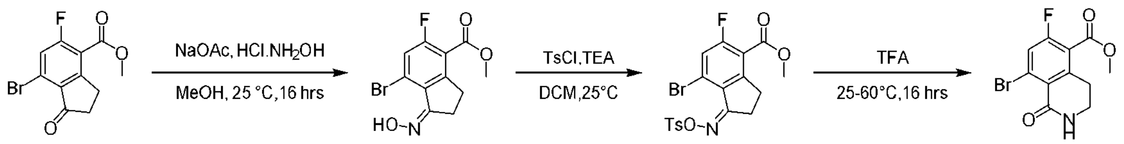


**Step 1:** Methyl 7-bromo-5-fluoro-1-oxo-2,3-dihydro-1H-indene-4-carboxylate (3.00 g, 10.4 mmol) was dissolved in methanol (20.0 mL), sodium acetate (2.05 g, 20.9 mmol) and hydroxylamine hydrochloride (1.45 g, 20.9 mmol) were added and the mixture was stirred at 25°C for 16 hours. The reaction solution was filtered and concentrated. Methyl (Z)-7-bromo-5-fluoro-1-(hydroxyimino)-2,3-dihydro-1H-indene-4-carboxylate (1.50 g, crude) was given as a yellow solid and used directly in the next step. LCMS (ESI): [M+H]$^+$ = 301.8

**Step 2:** Methyl (Z)-7-bromo-5-fluoro-1-(hydroxyimino)-2,3-dihydro-1H-indene-4-carboxylate (150 mg, 0.50 mmol) was dissolved in dichloromethane (4.00 mL), then triethylamine (753 mg, 7.45 mmol) and 4-dimethylaminopyridine (6.07 mg, 0.05 mmol) were added, p-toluenesulfonyl chloride (189 mg, 0.99 mmol) was added at 0°C, the temperature was raised to 25°C and the mixture was stirred for 3 hours. The reaction solution was diluted with water (50.0 mL) and extracted with ethyl acetate (100 mL × 2). The organic layer was dried, filtered and concentrated to give a residue, which was purified by flash column chromatography (silica gel, 0-9% gradient of tetrahydrofuran/petroleum ether) to give methyl (Z)-7-bromo-5-fluoro-1-((tosyloxy)imino)-2,3-dihydro-1H-indene-4-carboxylate (140 mg, 0.31 mmol, yield: 62.0%) as a white solid. LCMS (ESI): [M+H]$^+$ = 455.9

**Step 3:** Methyl (Z)-7-bromo-5-fluoro-1-((tosyloxy)imino)-2,3-dihydro-1H-indene-4-carboxylate (20.0 mg, 0.04 mmol) was dissolved in trifluoroacetic acid (0.50 mL), and the mixture was stirred at 25°C for 16 hours and at 60°C for 5 hours. The reaction solution was monitored by LC-MS. LCMS (ESI): [M+H]$^+$ = 303.7

Synthesis of **Intermediate K**

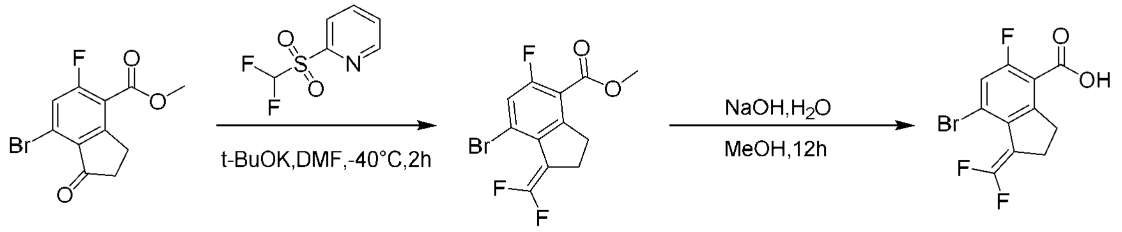


**Step 1:** At -40°C, to a solution of methyl 7-bromo-5-fluoro-1-oxo-2,3-dihydro-1H-indene-4-carboxylate (2.50 g, 8.7 mmol) and difluoro(pyridin-2-ylsulfonyl)methane (3.36 g, 17 mmol) in N,N-dimethylformamide (30.00 mL) was added dropwise a solution of potassium tert-butoxide (2.96 g, 26 mmol) in N,N-dimethylformamide (100.00 mL). The yellow mixture was stirred at -40°C for 0.5 h. To the reaction solution was added saturated ammonium chloride (12 mL) and 3M hydrochloric acid (30 mL). The reaction mixture was slowly heated to room temperature. TLC (PE:EA=10:1, Rf=0.7) indicated complete reaction. The reaction mixture was diluted with water (50 mL) and extracted with ethyl acetate (50 mL × 2). The combined organic layer was dried over anhydrous magnesium sulfate, filtered and concentrated under reduced pressure to give a residue. The residue was purified by flash silica gel chromatography (column: ISCO SepaFlash® silica gel flash column 80 g; gradient eluent: 0-8% tetrahydrofuran/petroleum ether; flow rate: 60 mL/min). Methyl 7-bromo-1-(difluoromethylene)-5-fluoro-2,3-dihydro-1H-indene-4-carboxylate (1.70 g, 60%) was given. LCMS (ESI): $[M+H]^+$ =321.0

**Step 2:** At 25°C, to a solution of methyl 7-bromo-1-(difluoromethylene)-5-fluoro-2,3-dihydro-1H-indene-4-carboxylate (0.85 g, 2.66 mmol) in methanol (5.00 mL) and water (5.00 mL) was added sodium hydroxide (312 mg, 7.74 mmol), and the reaction mixture was stirred at 25°C for 12 hours. The reaction mixture was diluted with water (5 mL), adjusted to pH 2-3 with a 1N dilute hydrochloric acid solution, and extracted with ethyl acetate (10 mL × 2). The organic layer was dried over magnesium sulfate, filtered and concentrated under reduced pressure to give 7-bromo-1-(difluoromethylene)-5-fluoro-2,3-dihydro-1H-indene-4-carboxylic acid (0.77 g, 2.52 mmol, yield: 93%) as a yellow solid. LCMS (ESI): $[M+H]^+$ =307.0

Synthesis of **Intermediate** L

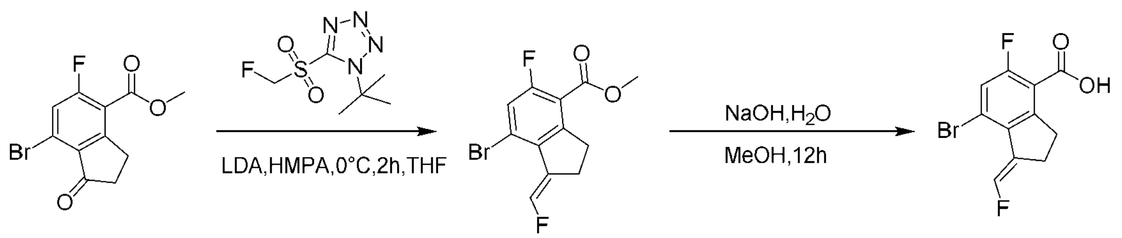


**Step 1:** At 0°C, to a solution of methyl 7-bromo-5-fluoro-1-oxo-2,3-dihydro-1H-indene-4-carboxylate (2.50 g, 8.7 mmol) and 1-(tert-butyl)-5-((fluoromethyl)sulfonyl)-1H-tetrazole (3.77 g, 17 mmol) in tetrahydrofuran (30.00 mL) were added dropwise hexamethylphosphoramide (1 mL) and lithium diisopropylamide (26 mL, 26 mmol). The yellow mixture was stirred at 0°C for 2 hours. To the reaction solution was added saturated ammonium chloride (12 mL) and 3M hydrochloric acid (30 mL). The reaction mixture was slowly heated to room temperature. TLC (PE:EA=10:1, Rf=0.7) indicated complete reaction. The reaction mixture was diluted with water (50 mL) and extracted with ethyl acetate (50 mL × 2). The combined organic layer was dried over magnesium sulfate, filtered and concentrated under reduced pressure to give a residue. The residue was purified by flash silica gel chromatography (column: ISCO SepaFlash® silica gel flash column 40 g, gradient eluent: 0-10% tetrahydrofuran/petroleum ether, flow rate: 60 mL/min). 7-bromo-1-(fluoromethylene)-5-fluoro-2,3-dihydro-1H-indene-4-carboxylate (1.70 g, 60%) was given. LCMS (ESI): $[M+H]^+$ =303.0

**Step 2:** At 25°C, to a solution of methyl 7-bromo-1-(fluoromethylene)-5-fluoro-2,3-dihydro-1H-indene-4-carboxylate (0.85 g, 2.66 mmol) in methanol (5.00 mL) and water (5.00 mL) was added sodium hydroxide (312 mg, 7.74 mmol), and the reaction mixture was stirred at 25°C for 12 hours. The reaction mixture was diluted with water (5 mL), adjusted to pH 2-3 with a 1N dilute hydrochloric acid solution, and extracted with ethyl acetate (10 mL × 2). The organic layer was dried over magnesium sulfate, filtered and concentrated under reduced pressure to give 7-bromo-1-(fluoromethylene)-5-fluoro-2,3-dihydro-1H-indene-4-carboxylic acid (0.70 g, 2.22 mmol, yield: 89%) as a yellow solid.

LCMS (ESI): $[M+H]^+$ =289.0

Synthesis of **Intermediate M**

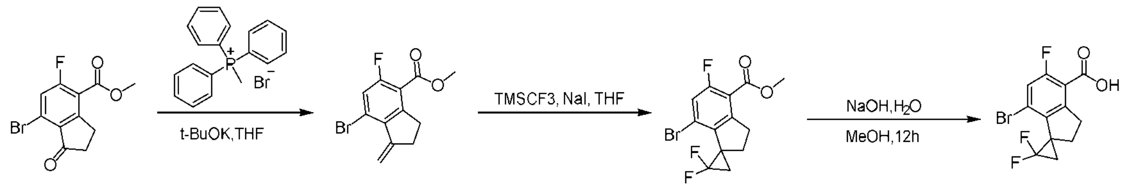


**Step 1:** At 0°C, to a solution of methyl 7-bromo-5-fluoro-1-oxo-2,3-dihydro-1H-indene-4-carboxylate (4.97 g, 17.4 mmol) and methyltriphenylphosphonium bromide (12.39 g, 34.8 mmol) in tetrahydrofuran (100.00 mL) was added potassium tert-butoxide (2.91 g, 26 mmol). The mixture was stirred at 0°C for 1 h. The reaction mixture was then slowly heated to 60°C and stirred for 10 hours. TLC (PE:EA=10:1, Rf=0.7) indicated complete reaction. The reaction mixture was diluted with water (50 mL) and extracted with ethyl acetate (50 mL × 2). The combined organic layer was dried over magnesium sulfate, filtered and concentrated under reduced pressure to give a residue. The residue was purified by flash silica gel chromatography (column: ISCO SepaFlash® silica gel flash column 100 g, gradient eluent: 0-10% tetrahydrofuran/petroleum ether, flow rate: 60 mL/min). Methyl 7-bromo-5-fluoro-1-methylene-2,3-dihydro-1H-indene-4-carboxylate (2.96 g, yield: 60%) was given. LCMS (ESI): $[M+H]^+$ =285.0

**Step 2:** At 25°C, to a solution of methyl 7-bromo-5-fluoro-1-methylene-2,3-dihydro-1H-indene-4-carboxylate (1.48 g, 5.22 mmol) in tetrahydrofuran (5.00 mL) were added (trifluoromethyl)trimethylsilane (7.41 g, 52.2 mmol) and sodium iodide (77 mg, 0.522 mmol), and the reaction mixture was stirred at 65°C for 12 hours. The reaction solution was diluted with water (5 mL) and extracted with ethyl acetate (10 mL × 2). The organic layer was dried over magnesium sulfate, filtered and concentrated under reduced pressure to give a residue which was purified by flash silica gel chromatography (column: ISCO SepaFlash® silica gel flash column 40 g; gradient eluent: 0-10% tetrahydrofuran/petroleum ether; flow rate: 60 mL/min). Methyl 7'-bromo-2,2,5'-trifluoro-2',3'-dihydrospiro[cyclopropane-1,1'-indene]-4'-carboxylate (871.8 mg, yield: 50%) was obtained. LCMS (ESI): $[M+H]^+$ = 335.0

Step 3: Methyl 7'-bromo-2,2,5'-trifluoro-2',3'-dihydrospiro[cyclopropane-1,1'-indene]-4'-carboxylate was obtained by a method similar to that for intermediate I. LCMS (ESI): $[M+H]^+$ =321.0

Synthesis of **Intermediate N**

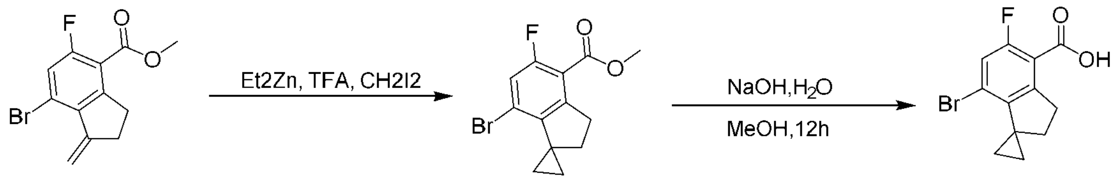


Step 1: At 0°C, to dichloromethane (5.00 mL) were added diethylzinc (28.7 mL, 28.7 mmol) and trifluoroacetic acid (2.1 mL), and then diiodomethane (7.70 g, 28.7mmol). The reaction mixture was stirred at 0°C for 0.5 hours. Then, a solution of methyl 7-bromo-5-fluoro-1-methylene-2,3-dihydro-1H-indene-4-carboxylate (4.09 g, 14.3 mmol) in dichloromethane (50 mL) was added. The temperature was raised to room temperature and the mixture was stirred for 4 hours. The reaction solution was diluted with water (50 mL) and extracted with ethyl acetate (50 mL × 2). The organic layer was dried over magnesium sulfate, filtered and concentrated under reduced pressure to give a residue which was purified by flash silica gel chromatography (column: ISCO SepaFlash® silica gel flash column 80 g; gradient eluent: 0-10% tetrahydrofuran/petroleum ether; flow rate: 60 mL/min). 7'-bromo-5'-fluoro-2',3'-dihydrospiro[cyclopropane-1,1'-indene]-4'-carboxylate (2.13 g, yield: 50%) was given. LCMS (ESI): $[M+H]^+$ = 299.0

Step 2: 7'-bromo-5'-fluoro-2',3'-dihydrospiro[cyclopropane-1,1'-indene]-4'-carboxylate was given by a method similar to that for intermediate I. LCMS (ESI): $[M+H]^+$ =285.0

**Examples 1A to 1D: Four isomers of N-($1^4$,$1^4$-difluoro-$2^6$-methyl-4-oxo-3-aza-2(2,4)-pyrimidin-1(1,3),6(1,4)-dipiperidin-5(1,2)-naphthacyclonon-8-en-$5^4$-yl) -2-hydroxyethane-1-sulfonamide**

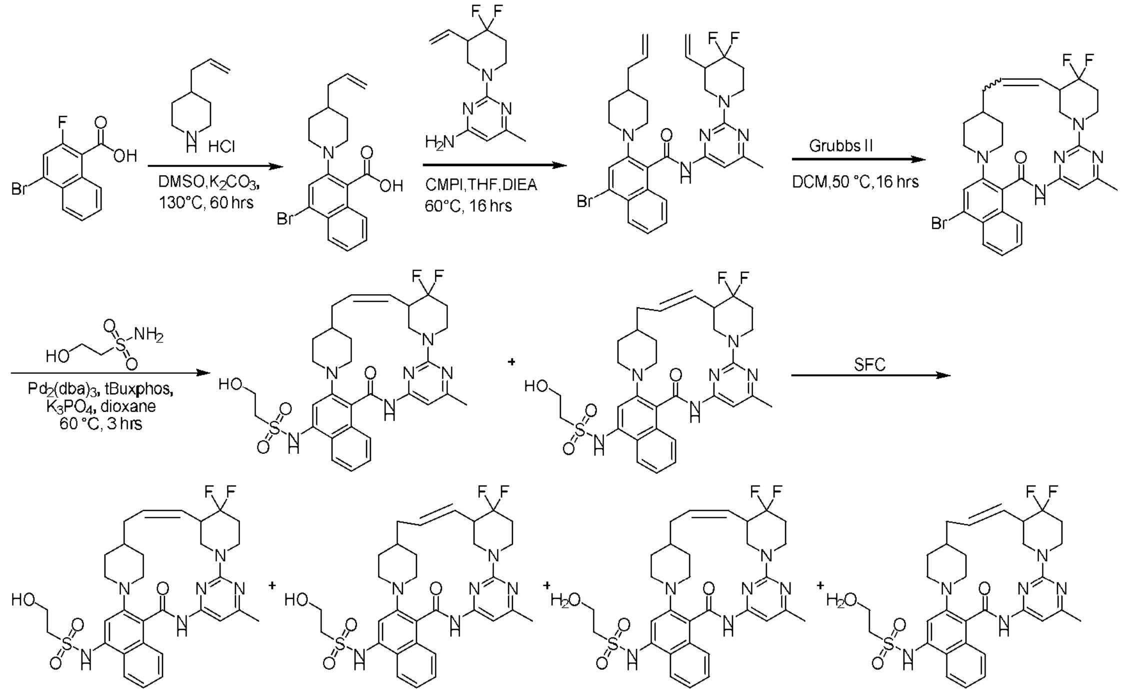


Step **1:** 4-bromo-2-fluoronaphthalene-1-carboxylic acid (5.00 g, 18.6 mmol) was dissolved in dimethyl sulfoxide (100 mL), potassium carbonate (7.86 g, 55.7 mmol) and 4-(prop-2-en-1-yl)piperidine hydrochloride (3.90 g, 1.30 mmol) were added and the mixture was stirred at 130°C for 60 hours. The reaction mixture was diluted with water (50.0 mL), extracted with ethyl acetate (100 mL × 2), and the organic phase was dried, filtered and concentrated. The residue was purified by flash column chromatography (silica gel, 0-30% gradient of tetrahydrofuran/petroleum ether) to give 4-bromo-2-[4-(prop-2-en-1-yl)piperidin-1-yl]naphthalene-1-carboxylic acid (3.20 g, 8.55 mmol, yield: 46.0%) as a yellow oil. LCMS (ESI): $[M+H]^+$ =374.0.

**Step 2:** 2-(3-Vinyl-4,4-difluoropiperidin-1-yl)-6-methylpyrimidin-4-amine (680 mg, 2.67 mmol) was dissolved in tetrahydrofuran (20.0 mL), N,N-diisopropylethylamine (1.04 g, 8.02 mmol), 4-bromo-2-[4-(prop-2-en-1-yl)piperidin-1-yl]naphthalene-1-carboxylic acid (1.00 g, 2.67 mmol) and 2-chloro-1-methylpyridinium iodide (1.04 g, 4.01 mmol) were added and the mixture was stirred at 60°C for 16 hours. The reaction mixture was diluted with water (50.0 mL), extracted with ethyl acetate (100 mL × 2), and the organic phase was dried, filtered and concentrated. The residue was purified by flash column chromatography (silica gel, 0-11% gradient of tetrahydrofuran/petroleum ether) to give 2-(4-allylpiperidin-1-yl)-4-bromo-N-(2-(4,4-difluoro-3-vinylpiperidin-1-acyl)-6-methylpyrimidin-4-yl)-1-naphthamide (0.50 g, 0.82 mmol, yield: 31.0%) as a yellow oil. LCMS (ESI): $[M+H]^+$ = 584.10. $^1$H NMR (400 MHz, DMSO-$d_6$) δ ppm 10.68 (br s, 1H), 8.22 - 8.05 (m, 1H), 7.95 - 7.68 (m, 2H), 7.68 - 7.54 (m, 2H), 7.49 (br s, 1H), 5.96 - 5.63 (m, 2H), 5.36 - 5.08 (m, 2H), 4.96 (br d, *J* = 12.4 Hz, 2H), 4.69 - 4.26 (m, 2H), 3.30 (br d, *J* = 2.0 Hz, 1H), 3.26 - 3.07 (m, 2H), 2.92 - 2.56 (m, 4H), 2.42 - 2.28 (m, 3H), 2.20 - 1.78 (m, 4H), 1.64 (br d, *J* = 11.2 Hz, 2H), 1.48 - 1.31 (m, 1H), 1.28 - 1.00 (m, 2H)

**Step 3:** 2-(4-Allylpiperidin-1-yl)-4-bromo-N-(2-(4,4-difluoro-3-vinylpiperidin-1-acyl)-6-methylpyrimidin-4-yl)-1-naphthamide (500 mg, 0.82 mmol) was dissolved in dichloromethane (200 mL), Grubbs catalyst 2$^{nd}$ generation (151 mg, 0.17 mmol) was added and the mixture was stirred at 50°C for 16 hours under nitrogen atmosphere. The reaction mixture was concentrated under reduced pressure. The residue was purified by flash column chromatography (silica gel, 0-5% gradient of tetrahydrofuran/petroleum ether) to give $5^4$-bromo-$1^4$,$1^4$-difluoro-$2^6$-methyl-3-aza-2(2,4)-pyrimidin-1(1,3),6(1,4)-bispiperidin-5(1,2)-naphthacyclonon-8-en-4-one (400 mg, 0.69 mmol, yield: 84.0%) as a yellow solid. LCMS (ESI): $[M+H]^+$ = 584.1

**Step 4:** $5^4$-Bromo-$1^4$,$1^4$-difluoro-$2^6$-methyl-3-aza-2(2,4)-pyrimidin-1(1,3),6(1,4)-bispiperidin-5(1,2)-naphthacyclonon-8-en-4-one (300 mg, 0.52 mmol) and 2-hydroxyethanesulfonamide (83.8 mg, 0.67 mmol) were dissolved in 1,4-dioxane (10.0 mL), and potassium phosphate (437 mg, 2.06 mmol), 2-di-tert-butylphosphino-2',4',6'-triisopropylbiphenyl (43.7 mg, 0.10 mmol) and tris(dibenzylideneacetone)dipalladium (47.2 mg, 0.05 mmol) were added. The solution was stirred at 60°C for 3 h under nitrogen atmosphere. The reaction solution was filtered, diluted and purified by preparative HPLC (C18, 25-56% gradient of water (hydrochloric acid)/acetonitrile) to give a cis product and a trans product.

Cis product: White solid ((cis)-N-($1^4$,$1^4$-difluoro-$2^6$-methyl-4-oxo-3-aza-2(2,4)-pyrimidin-1(1,3),6(1,4)-dipiperidin-5(1,2)-naphthacyclonon-8-en-$5^4$-yl)-2-hydroxyethane-1-sulfonamide (86 mg). LCMS (ESI): $[M+H]^+$ = 627.2 $^1H$ NMR (400 MHz, DMSO-$d_6$) δ ppm 11.02 - 10.34 (m, 1H), 8.37 - 8.23 (m, 2H), 7.59 - 7.37 (m, 3H), 7.25 (s, 1H), 5.84 - 5.65 (m, 1H), 5.46 (t, *J* = 10.4 Hz, 1H), 5.19 - 4.84 (m, 1H), 4.82 - 4.65 (m, 1H), 4.63 - 4.48 (m, 1H), 3.79 (t, *J* = 6.8 Hz, 2H), 3.50 - 3.40 (m, 1H), 3.24 (br t, *J* = 6.4 Hz, 2H), 3.14 (br t, *J* = 12.0 Hz, 1H), 3.06 - 2.71 (m, 5H), 2.49 - 2.43 (m, 1H), 2.38 (s, 3H), 2.28 - 1.87 (m, 4H), 1.84 - 1.62 (m, 4H), 1.45 - 1.29 (m, 1H)

Trans product: White solid ((trans)-N-($1^4$,$1^4$-difluoro-$2^6$-methyl-4-oxo-3-aza-2(2,4)-pyrimidin-1(1,3),6(1,4)-dipiperidin-5(1,2)-naphthacyclonon-8-en-5 $^4$-yl)-2-hydroxyethane-1-sulfonamide (27.7 mg). LCMS (ESI): $[M+H]^+$ = 627.3 $^1H$ NMR (400 MHz, DMSO-$d_6$) δ ppm 10.20 (s, 1H), 10.09 - 9.49 (m, 1H), 8.34 - 8.03 (m, 2H), 7.59 - 7.45 (m, 2H), 7.43 - 7.33 (m, 2H), 5.92 - 5.68 (m, 1H), 5.35 (dd, *J* = 8.4, 15.6 Hz, 1H), 5.11 - 4.87 (m, 3H), 3.79 (t, *J* = 6.8 Hz, 2H), 3.52 - 3.39 (m, 1H), 3.30 - 3.24 (m, 2H), 3.19 - 3.08 (m, 1H), 3.00 - 2.80 (m, 3H), 2.77 - 2.63 (m, 1H), 2.35 (s, 2H), 2.45 - 2.33 (m, 1H), 2.33 - 2.10 (m, 2H), 2.09 - 1.84 (m, 3H), 1.74 - 1.42 (m, 4H), 1.20 - 1.09 (m, 1H)

The cis product was separated by SFC (column: DAICEL CHIRALCEL OX (250 mm×30 mm, 10 μm); mobile phase: $CO_2$ - EtOH (0.1% $NH_3$ • $H_2O$); gradient: isocratic 50%; flow rate: 80 mL/min; column temperature: 40°C) to give **Example 1A** and **Example 1B.**

**Example 1A:** White solid (12.6 mg), SFC analysis (column: Chiralpak AS-3 50 × 4.6 mm I.D., 3 μm; mobile phase: carbon dioxide for phase A and 0.05% diethylamine/ethanol for phase B; gradient: 40% for phase B; flow rate: 4 mL/min). The retention time on the chiral column was 1.537 min; LCMS (ESI): $[M+H]^+$ =627.2 $^1H$ NMR (400 MHz, DMSO-$d_6$) δ ppm 10.27 (s, 1H), 10.05 - 9.74 (m, 1H), 8.27 (d, *J* = 8.4 Hz, 1H), 8.11 (d, *J* = 8.4 Hz, 1H), 7.6 - 7.4 (m, 3H), 7.18 (s, 1H), 5.74 (dt, *J* = 5.0, 11.2 Hz, 1H), 5.44 (br t, *J* = 10.4 Hz, 1H), 5.19 - 4.90 (m, 1H), 4.84 - 4.66 (m, 1H), 4.59 (br d, *J* = 12.0 Hz, 1H), 3.81 (br t, *J* = 6.4 Hz, 2H), 3.51 (br d, *J* = 10.8 Hz, 1H), 3.31 (br s, 2H), 3.23 - 3.10 (m, 1H), 3.07 - 2.96 (m, 2H), 2.95 - 2.69 (m, 3H), 2.39 (s, 3H), 2.26 - 1.84 (m, 4H), 1.82 - 1.57 (m, 4H), 1.43 - 1.19 (m, 1H)

**Example 1B:** White solid (16.9 mg), SFC analysis (column: Chiralpak AS-3 50 × 4.6 mm I.D., 3 μm; mobile phase: carbon dioxide for phase A and 0.05% diethylamine/ethanol for phase B; gradient: 40% for phase B; flow rate: 4 mL/min). The retention time on the chiral column was 1.212 min; LCMS (ESI): $[M+H]^+$ =627.3 $^1H$ NMR (400 MHz, DMSO-$d_6$) δ ppm 10.30 (s, 1H), 9.98 (s, 1H), 8.33 (d, *J* = 8.4 Hz, 1H), 8.16 (d, *J* = 8.4 Hz, 1H), 7.75 - 7.53 (m, 3H), 7.23 (s, 1H), 5.80 (dt, *J* = 5.6, 11.2 Hz, 1H), 5.50 (br t, *J* = 10.4 Hz, 1H), 4.78 (br *d, J* = 12.4 Hz, 1H), 4.65 (br d, *J* = 12.4 Hz, 1H), 3.87 (t, *J* = 6.4 Hz, 2H), 3.57 (br d, *J* = 10.8 Hz, 1H), 3.20 (br t, *J* = 12.4 Hz, 1H), 3.13 - 3.02 (m, 2H), 3.00 - 2.74 (m, 3H), 2.45 (s, 3H), 2.30 - 1.98 (m, 4H), 1.91 - 1.60 (m, 5H), 1.48 - 1.16 (m, 3H)

The trans product was separated by SFC (column: DAICEL CHIRALPAK AS (250 mm×30 mm, 10 μm); mobile phase: $CO_2$ - EtOH (0.1% $NH_3$ •$H_2O$); gradient: isocratic 35%; flow rate: 80 mL/min; column temperature: 35°C) to give **Example 1C** and **Example 1D.**

**Example 1C:** White solid (7.11 mg), SFC analysis(column: Chiralpakl IG-3 50 × 4.6 mm I.D., 3 μm; mobile phase: carbon dioxide for phase A and 0.05% diethylamine/ethanol for phase B; gradient: from 5% to 40% of B in 1.5 min and followed by **a 1-min hold at** 40%, then 5% of B for 0.5min; flow rate: 4 mL/min). The retention time on the chiral column was 0.572 min; LCMS (ESI): $[M+H]^+$ =627.3 $^1H$ NMR (400 MHz, DMSO-$d_6$) δ ppm 10.20 (s, 1H), 8.22 (d, *J* = 8.4 Hz, 1H), 8.15 (d, *J* = 8.4 Hz, 1H), 7.55 (s, 1H), 7.50 (br t, *J* = 7.6 Hz, 1H), 7.43 - 7.32 (m, 2H), 5.89 - 5.69 (m, 1H), 5.36 (br dd, *J* = 8.4, 15.6 Hz, 1H), 5.02 (br t, *J* = 13.2 Hz, 2H), 3.79 (t, *J* = 6.8 Hz, 2H), 3.44 (br d, *J* = 9.6 Hz, 1H), 3.31 - 3.24 (m, 4H), 3.16 (br d, *J* = 12.4 Hz, 1H), 3.00 - 2.80 (m, 3H), 2.72 (br t, *J* = 11.6 Hz, 1H), 2.36 (s, 3H), 2.30 - 1.87 (m, 4H), 1.79 - 1.43 (m, 4H), 1.31 - 1.12 (m, 2H)

**Example 1D:** White solid (8.64 mg), SFC analysis(column: Chiralpakl IG-3 50 × 4.6 mm I.D., 3 μm; mobile phase: carbon dioxide for phase A and 0.05% diethylamine/ethanol for phase B; gradient: from 5% to 40% of B in 1.5 min and hold 40% of 1 min, then 5% of B for 0.5min; flow rate: 4 mL/min). The retention time on the chiral column was 1.134 min; LCMS (ESI): $[M+H]^+$ =627.4 $^1H$ NMR (400 MHz, DMSO-$d_6$) δ ppm 10.25 (s, 1H), 8.28 (dd, *J* = 8.4, 16.8 Hz, 2H), 7.62 (s, 1H), 7.51 (br t, *J* = 7.8 Hz, 1H), 7.43 - 7.33 (m, 2H), 5.87 (td, *J* = 7.8, 15.2 Hz, 1H), 5.43 (br dd, *J* = 8.4, 15.2 Hz, 1H), 5.17 - 4.97 (m, 2H), 3.83 (t,*J* = 6.8 Hz, 2H), 3.47 (br d, *J* = 10.0 Hz, 1H), 3.32 - 3.16 (m, 4H), 3.09 - 2.86 (m, 3H), 2.74 (br t, *J* = 11.6 Hz, 1H), 2.41 (s, 3H), 2.37 - 2.26 (m, 1H), 2.24 - 1.87 (m, 4H), 1.75 - 1.52 (m, 4H), 1.34 - 1.18 (m, 4H), 0.99 - 0.85 (m, 1H)

**Examples 2A to 2D: Four isomers of N-($1^4$,$1^4$-difluoro-$2^6$-methyl-$5^1$,4-dioxo-$5^2$,$5^3$-dihydro-$5^1$*H*-3-aza-2(2,4)-pyrimidin-1(1,3),6(1,4)-dipiperidin-5(4,5)-inde noheterocyclonon-8-en-$5^7$-yl)-2-hydroxyethane-1-sulfonamide**

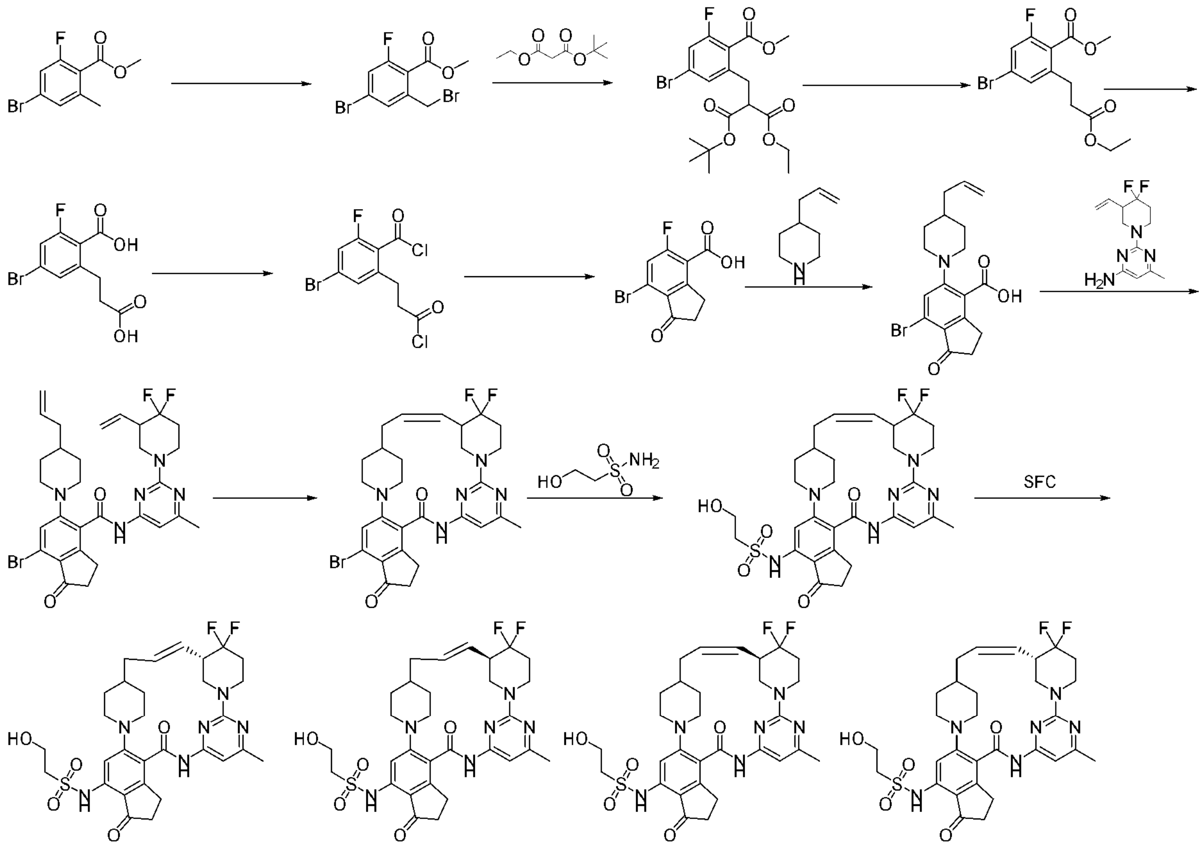


**Step 1:** A mixture containing methyl 4-bromo-2-fluoro-6-methylbenzoate (25.00 g, 101.19 mmol), N-bromosuccinimide (21.60 g, 121.43 mmol), azobisisobutyronitrile (8.50 g, 50.60 mmol) and acetonitrile (250.00 mL) was stirred at 70°C for 3 hours. The reaction solution was poured into water (50 mL) and extracted with ethyl acetate (50 mL × 3). The organic layer was dried over anhydrous sodium sulfate, filtered, concentrated and purified by column chromatography (silica gel, 0-10% gradient of ethyl acetate/petroleum ether) to give methyl 4-bromo-2-(bromomethyl)-6-fluorobenzoate (26.20 g, crude) as a colorless oil. LCMS (ESI): $[M+H]^+$ =326.9

**Step 2:** A mixture containing methyl 4-bromo-2-(bromomethyl)-6-fluorobenzoate (26 g, 79.76 mmol), tert-butyl propane-1,3-dioate (16.5 g, 87.74 mmol), cesium carbonate (53.0 g, 159.53 mmol) and N,N-dimethylformamide (200.00 mL) was stirred at 20°C for 2 hours. The reaction mixture was poured into water (300 mL) and extracted with ethyl acetate (100 mL × 3), and the organic phase was dried over anhydrous sodium sulfate, filtered, concentrated, and purified by column chromatography (silica gel, 0-10% gradient of ethyl acetate/petroleum ether) to give 1-(tert-butyl)-3-ethyl-2-(5-bromo-3-fluoro-2-(methoxycarbonyl)benzyl)malonate (21.00 g, crude) as a colorless oil compound. LCMS (ESI): $[M+Na]^+$ =455.0

**Step 3:** A solution containing 1-(tert-butyl)-3-ethyl-2-(5-bromo-3-fluoro-2-(methoxycarbonyl)benzyl)malonate (21.00 g, 48.47 mmol) and trifluoroacetic acid (60.00 mL) was stirred at 20°C for 1 hour. The reaction solution was concentrated to remove the trifluoroacetic acid, then toluene (60 mL) and triethylamine (50.0 g, 484.69 mmol) were added and the mixture was stirred at 120°C for 3 hours. The reaction mixture was concentrated, aqueous sodium bicarbonate solution was added to adjust the pH to be close to 8, and then the mixture was extracted with ethyl acetate (50 mL × 3). The organic phase was dried over anhydrous sodium sulfate, filtered, concentrated, and purified by column chromatography (silica gel, 0-10% gradient of ethyl acetate/petroleum ether) to give the compound methyl 4-bromo-2-(3-ethoxy-3-oxopropyl)-6-fluorobenzoate (11.20 g, crude). LCMS (ESI): $[M+H]^+$ = 333.0.

**Step 4:** A mixture containing methyl 4-bromo-2-(3-ethoxy-3-oxopropyl)-6-fluorobenzoate (11.20 g, 33.62 mmol), lithium hydroxide monohydrate (8.1 g, 336.18 mmol), tetrahydrofuran (40 mL), methanol (40 mL) and water (40 mL) was stirred at 20°C for 4 hours. The reaction mixture was concentrated to remove tetrahydrofuran and methanol, 4 M hydrochloric acid ethyl acetate solution (100 mL) was added to adjust the pH to 5, and then the mixture was extracted with ethyl acetate (100 mL × 3). The organic phase was dried over anhydrous sodium sulfate, filtered, and concentrated to give 4-bromo-2-(2-carboxyethyl)-6-fluorobenzoic acid (5.1 g, 17.52 mmol, yield: 52.1%) as a white solid compound. LCMS (ESI): $[M+Na]^+$ = 313.0.

**Step 5:** Oxalyl chloride (11.10 g, 85.89 mmol) was added to a mixed solution containing 4-bromo-2-(2-carboxyethyl)-6-fluorobenzoic acid (5.00 g, 17.18 mmol) and dichloromethane (50 mL), and the resulting mixture was stirred

at 20°C for 1 h. The reaction mixture was concentrated to give 4-bromo-2-(3-chloro-3-oxopropyl)-6-fluorobenzoyl chloride (5.7 g, crude) as a yellow oil compound. LCMS (ESI): $[M+H]^+$ = 319.0 (corresponding methyl ester).

**Step 6:** 4-Bromo-2-(3-chloro-3-oxypropyl)-6-fluorobenzoyl chloride (5.60 g, 17.08 mmol) was dissolved in dichloromethane (10.00 mL) and the mixture was added dropwise to a solution containing aluminum trichloride (9.10 g, 68.30 mmol) and dichloromethane (50.00 mL) at 0°C. The reaction solution was stirred at 40°C for 16 hours. The reaction mixture was poured into water (30 mL) and extracted with dichloromethane (30 mL × 3). The organic layer was dried over anhydrous sodium sulfate, filtered, concentrated and purified by column chromatography (silica gel, 0-100% gradient of ethyl acetate/petroleum ether) to give 7-bromo-5-fluoro-1-oxo-2,3-dihydro-1H-indene-4-carboxylic acid (3.40 g, crude) as a brown solid, LCMS (ESI): $[M+H]^+$ = 273.0.

**Step 7:** A mixture containing 7-bromo-5-fluoro-1-oxo-2,3-dihydro-1H-indene-4-carboxylic acid (3.40 g, 12.45 mmol), 4-allylpiperidine (1.90 g, 14.94 mmol), potassium carbonate (5.30 g, 37.35 mmol) and dimethyl sulfoxide (30 mL) was stirred at 100°C for 3 hours. The reaction solution was poured into water (30 mL), then hydrochloric acid/ethyl acetate solution (20 mL, 4 M) was added to adjust the pH to about 5, and the mixture was extracted with ethyl acetate (30 mL × 3). The organic layer was dried over anhydrous sodium sulfate, filtered, concentrated, and purified by column chromatography (silica gel, 0-50% gradient of ethyl acetate/petroleum ether) to give 5-(4-allylpiperidin-1-yl)-7-bromo-1-oxo-2,3-dihydro-1H-indene-4-carboxylic acid (1.90 g, 5.02 mmol, yield: 40.3%) as a yellow solid, LCMS (ESI): $[M+H]^+$ = 378.0.

**Step 8:** A mixture containing 5-(4-allylpiperidin-1-yl)-7-bromo-1-oxo-2,3-dihydro-1H-indene-4-carboxylic acid (1.40 g, 3.70 mmol), 2-(4,4-difluoro-3-vinylpiperidin)-6-methylpyrimidin-4-amine (1.10 g, 4.44 mmol), 2-chloro-1-methylpyridinium iodide (1.40 g, 5.55 mmol), N,N-diisopropylethylamine (1.50 g, 11.10 mmol) and dichloromethane (20.00 mL) was stirred at 20°C for 12 hours. The reaction solution was concentrated and purified by column chromatography (silica gel, 0-70% gradient of ethyl acetate/petroleum ether) to give 5-(4-allylpiperidin-1-yl)-7-bromo-N-(2-(4,4-difluoro-3-vinylpiperidin-1-acyl)-6-methylpyrimidin-4-yl)-1-oxo-2,3-dihydro-1 H-indene-4-carboxamide (1.00 g, 1.63 mmol, yield: 44.0%) as a yellow solid. LCMS (ESI): $[M+H]^+$ = 614.20.

**Step 9:** A solution containing 5-(4-allylpiperidin-1-yl)-7-bromo-N-(2-(4,4-difluoro-3-vinylpiperidin-1-acyl)-6-methylpyrimidin-4-yl)-1-oxo-2,3-dihydro-1 H-indene-4-carboxamide (1.00 g, 1.63 mmol), Grubbs catalyst $2^{nd}$ generation (141 mg, 0.16 mmol) and dichloromethane (200.00 mL) was stirred at 50°C for 2 hours. The reaction solution was concentrated and purified by column chromatography (silica gel, 0-20% gradient of ethyl acetate/petroleum ether) to give $5^7$-bromo-$1^4$,$1^4$-difluoro-$2^6$-methyl-$5^2$,$5^3$-dihydro-$5^1$H-3-aza-2(2,4)-pyrimidin-1(1,3),6(1,4)-dipiperidin-5(4,5)-indenoheteroc yclonon-8-en-$5^1$,4-dione (700 mg, crude) as a yellow solid. LCMS (ESI): $[M+H]^+$ = 586.20.

**Step 10:** Under nitrogen atmosphere, a mixture containing $5^7$-bromo-$1^4$,$1^4$-difluoro-$2^6$-methyl-$5^1$,$5^3$-dihydro-$5^1$H-3-aza-2(2,4)-pyrimidin-1(1,3),6(1,4)-dipiperidin-5(4,5)-indenoheteroc yclonon-8-en-$5^1$,4-dione (300 mg, 0.51 mmol), 2-hydroxyethanesulfonamide (131 mg, 1.02 mmol), potassium phosphate (332 mg, 1.53 mmol), 2-di-tert-butylphosphino-2',4',6'-triisopropylbiphenyl (22 mg, 0.051 mmol), tris(dibenzylideneacetone)dipalladium (48 mg, 0.051 mmol) and 1,4-dioxane (5 mL) was stirred at 100°C for 2 hours. The reaction solution was poured into water (30 mL) and extracted with ethyl acetate (30 mL × 3). The organic layer was dried over anhydrous sodium sulfate, filtered, concentrated, and purified by column chromatography (silica gel, 0-100% gradient of ethyl acetate/petroleum ether; C18, 0-100% gradient of acetonitrile/water) to give a mixture of the four isomers of **Example 2** (38 mg, 0.060 mmol, yield: 11.8%) as a white solid. LCMS (ESI): $[M+H]^+$ = 631.30. $^1$H NMR (400 MHz, DMSO) $\delta$ ppm 11.24 - 9.85 (m, 2H), 7.47 - 7.27 (m, 1H), 7.20 - 6.98 (m, 1H), 5.84 - 5.69 (m, 1H), 5.51 - 5.29 (m, 1H), 5.11 - 4.86 (m, 2H), 4.71 - 4.38 (m, 1H), 3.84 - 3.73 (m, 2H), 3.59 - 3.51 (m, 2H), 3.50 - 3.34 (m, 2H), 3.29 - 3.02 (m, 3H), 2.99 - 2.60 (m, 6H), 2.36 - 2.30 (m, 3H), 2.29 - 2.21 (m, 1H), 2.18 - 1.96 (m, 2H), 1.93 - 1.38 (m, 6H).

The mixture of the four isomers of **Example 2** was purified by preparative HPLC (C18, 26-66% gradient of water (hydrochloric acid)/acetonitrile) to give a mixture of trans isomers (10.7 mg) and a mixture of cis isomers (7.2 mg) as white solids.

The mixture of trans isomers (10.7 mg) was separated by SFC (column: REGIS (s,s) WHELK-O1 (250 mm×30 mm,5 μm); mobile phase: $CO_2$ - MeOH (0.1% $NH_3 \cdot H_2O$); gradient: isocratic 45%; flow rate: 80 mL/min; column temperature: 40°C) to give **Example 2A** (1.0 mg) and **Example 2B** (1.0 mg).

**Example 2A:** White solid (1.00 mg), SFC analysis(column: Chiralpak AD-3 50 × 4.6 mm I.D., 3 μm; mobile phase: carbon dioxide for phase A and 0.05% diethylamine/methanol for phase B; gradient: from 20% to 40% of B in 1.5 min and hold 40% of 1 min, then 20% of B for 0.5 min; flow rate: 4 mL/min). The retention time on the chiral column was 0.824 min; LCMS (ESI): $[M+H]^+$ =631.2 $^1$H NMR (400 MHz, DMSO-$d_6$) $\delta$ ppm 10.04 (br s, 1H), 9.91 (br s, 1H), 7.45 (s, 1H), 7.01 (br s, 1H), 5.89 - 5.67 (m, 1H), 5.46 - 5.29 (m, 1H), 5.16 - 4.79 (m, 3H), 3.77 (br d, $J$ = 4.4 Hz, 2H), 3.61 - 3.37 (m, 4H), 3.28 - 3.13 (m, 3H), 2.99 - 2.77 (m, 5H), 2.66 - 2.60 (m, 1H), 2.32 (s, 3H), 2.29 - 2.23 (m, 1H), 2.09 - 1.96 (m, 2H), 1.94 - 1.83 (m, 1H), 1.76 - 1.55 (m, 4H), 1.21 - 1.13 (m, 1H)

**Example 2B:** White solid (1.00 mg), SFC analysis(column: Chiralpak AD-3 50 × 4.6 mm I.D., 3 μm; mobile phase: carbon dioxide for phase A and 0.05% diethylamine/methanol for phase B; gradient: from 20% to 40% of B in 1.5 min and hold 40% of 1 min, then 20% of B for 0.5 min; flow rate: 4 mL/min). The retention time on the chiral column was

0.953 min; LCMS (ESI): $[M+H]^+$ =631.2 $^1H$ NMR (400 MHz, DMSO-$d_6$) δ ppm 10.09 (s, 1H), 9.97 (s, 1H), 7.51 (s, 1H), 7.07 (s, 1H), 5.91 - 5.76 (m, 1H), 5.41 (dd, *J* = 8.8, 15.6 Hz, 1H), 5.15 - 4.86 (m, 3H), 3.89 - 3.81 (m, 2H), 3.60 (br t, *J* = 5.2 Hz, 2H), 3.56 - 3.42 (m, 2H), 3.30 - 3.18 (m, 2H), 3.06 - 2.79 (m, 5H), 2.74 - 2.64 (m, 2H), 2.41 - 2.37 (m, 3H), 2.36 - 2.27 (m, 1H), 2.23 - 2.02 (m, 2H), 1.99 - 1.88 (m, 1H), 1.83 - 1.52 (m, 4H), 1.27 - 1.19 (m, 1H)

The mixture of cis isomers (7.2 mg) was separated by SFC (column: DAICEL CHIRALPAK AD (250 mm*30 mm, 10 μm); mobile phase: $CO_2$ - MeOH (0.1% $NH_3H_2O$); gradient: isocratic 50%; flow rate: 80 mL/min; column temperature: 40°C) to give **Example 2C** and **Example 2D.**

**Example 2C:** White solid (1.00 mg), SFC analysis(column: Chiralpak AD-3 50 × 4.6 mm I.D., 3 μm; mobile phase: carbon dioxide for phase A and 0.05% diethylamine/methanol for phase B; gradient: from 20% to 40% of B in 1.5 min and hold 40% of 1 min, then 20% of B for 0.5 min; flow rate: 4 mL/min). The retention time on the chiral column was 1.026 min; LCMS (ESI): $[M+H]^+$ =631.2 $^1H$ NMR (400 MHz, DMSO-$d_6$) δ ppm 11.38 - 10.98 (m, 1H), 10.21 - 9.72 (m, 1H), 7.30 (s, 1H), 7.23 - 7.04 (m, 1H), 5.89 - 5.67 (m, 1H), 5.47 (br t, *J*= 10.0 Hz, 1H), 5.21 - 4.92 (m, 1H), 4.75 - 4.58 (m, 1H), 4.53 - 4.36 (m, 1H), 3.89 - 3.71 (m, 2H), 3.66 - 3.39 (m, 4H), 3.24 - 3.04 (m, 4H), 3.02 - 2.75 (m, 3H), 2.66 - 2.61 (m, 1H), 2.38 - 2.26 (m, 5H), 2.17 - 2.02 (m, 2H), 1.98 - 1.68 (m, 5H), 1.63 - 1.50 (m, 1H)

**Example 2D:** White solid (1.00 mg), SFC analysis(column: Chiralpak AD-3 50 × 4.6 mm I.D., 3 μm; mobile phase: carbon dioxide for phase A and 0.05% diethylamine/methanol for phase B; gradient: from 20% to 40% of B in 1.5 min and hold 40% of 1 min, then 20% of B for 0.5 min; flow rate: 4 mL/min). The retention time on the chiral column was 1.497 min; LCMS (ESI): $[M+H]^+$ =631.2 $^1H$ NMR (400 MHz, DMSO-$d_6$) δ ppm 11.58 - 11.08 (m, 1H), 10.46 - 9.78 (m, 1H), 7.36 (s, 1H), 7.27 - 7.10 (m, 1H), 5.98 - 5.77 (m, 1H), 5.63 - 5.47 (m, 1H), 5.27 - 5.02 (m, 1H), 4.81 - 4.60 (m, 1H), 4.58 - 4.44 (m, 1H), 3.96 - 3.77 (m, 2H), 3.73 - 3.44 (m, 4H), 3.27 - 3.08 (m, 4H), 3.05 - 2.82 (m, 3H), 2.71 - 2.66 (m, 1H), 2.43 - 2.30 (m, 5H), 2.19 - 2.08 (m, 2H), 1.98 - 1.75 (m, 5H), 1.70 - 1.59 (m, 1H)

**Example 3: N-($1^4$-fluoro-$2^6$-methyl-$5^1$,4-dioxo-11,$1^2$,$1^3$,$1^6$,$5^2$,$5^3$-hexahydro-$5^1$H-3-aza-$^2$(2,4)-pyrimidin-1(1,5)-pyridin-6(1,4)-piperid in-5(4,5)-indenoheterononane-8-en-$5^7$-yl)-2-hydroxyethane-1-sulfonamide**

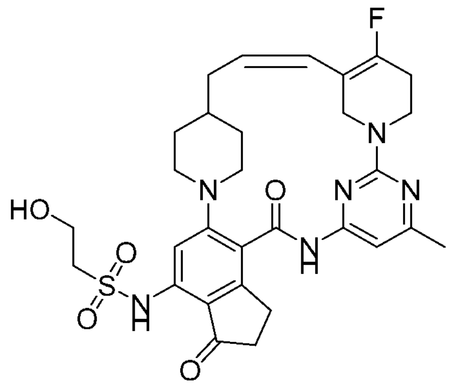

**Example** 3 Prepared using a similar synthetic route as described in Example 2: LCMS (ESI): $[M+H]^+$ = 611.30.

**Example 4: N-($1^4$,$1^4$-difluoro-$2^6$-methyl-4-oxo-$5^2$,$5^3$-dihydro-$5^1$*H*-3-aza-2(2,4)-pyrimidin-1(1,3),6(1,4)-dipiperidin-5(4,5)-indenohet erocyclonon-8-en-$5^7$-yl)-2-hydroxyethane-1-sulfonamide**

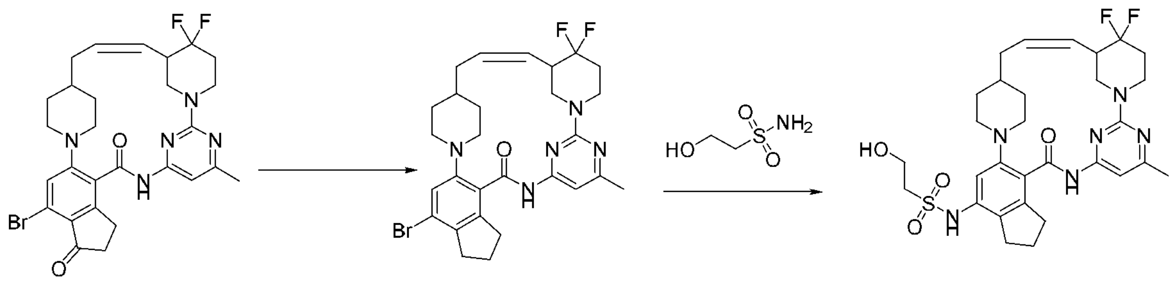

**Step 1:** A solution containing $5^7$-bromo-$1^4$,$1^4$-difluoro-$2^6$-methyl-$5^2$,$5^3$-dihydro-$5^1$*H*-3-aza-2(2,4)-pyrimidin-1(1,3),6(1,4)-dipiperidin-5(4,5)-indenoheteroc yclonon-8-en-$5^1$,4-dione (400 mg, 0.68 mmol), triethylsilane (801 mg, 6.82 mmol) and trifluoroacetic acid (5.00 mL) was stirred at 80°C for 12 hours. The reaction solution was concentrated, then aqueous sodium bicarbonate solution (500 mL) was added to adjust the pH to about 8, and the mixture was extracted with ethyl acetate. The organic layer was dried over anhydrous sodium sulfate, filtered, concentrated and purified by column chromatography (silica gel, 0-30% gradient of ethyl acetate/petroleum ether) to give $5^7$-bromo-$1^4$,$1^4$-difluoro-$2^6$-methyl-$5^2$,$5^3$-dihydro-$5^1$*H*-3-aza-2(2,4)-pyrimidin-1(1,3),6(1,4)-dipiperidin-5(4,5)-indenoheteroc yclonon-8-en-4-one (120 mg, 0.21 mmol, yield: 30.7%) as a yellow solid. LCMS (ESI): $[M+H]^+$ = 572.20.

**Step 2:** Under nitrogen atmosphere, a mixture containing $5^7$-bromo-$1^4$,$1^4$-difluoro-$2^1$-methyl-$5^2$,$5^3$-dihydro-$5^1$*H*-3-aza-2(2,4)-pyrimidin-1(1,3),6(1,4)-dipiperidin-5(4,5)-indenoheteroc yclonon-8-en-4-one (120 mg, 0.21 mmol), 2-hydroxyethanesulfonamide (54 mg, 0.42 mmol), potassium phosphate (136 mg, 0.63 mmol), 2-di-tert-butylphosphino-2',4',6'-triisopropylbiphenyl (9 mg, 0.021 mmol), tris(dibenzylideneacetone)dipalladium (20 mg, 0.021 mmol) and 1,4-dioxane (5.00 mL) was stirred at 100°C for 2 hours. The reaction solution was poured into water (30 mL) and extracted with ethyl acetate (30 mL × 3). The organic layer was dried over anhydrous sodium sulfate, filtered, concentrated, and purified by column chromatography (silica gel, 0-100% gradient of ethyl acetate/petroleum ether) to give N-($1^4$,$1^4$-difluoro-$2^6$-methyl-4-oxo-$5^2$,$5^3$-dihydro-$5^1$*H*-3-aza-2(2,4)-pyrimidin-1(1,3),6(1,4)-dipiperidin-5(4,5)-indenoheteroc yclonon-8-en-$5^7$-yl)-2-hydroxyethane-1-sulfonamide (51 mg, 0.08 mmol, yield: 39.5%) as a yellow solid.

LCMS (ESI): $[M+H]^+$ = 617.30. $^1$H NMR (400 MHz, DMSO) δ 12.75-12.65(m, 1H), 10.47-10.39 (m, 1H), 9.34 (s, 1H), 7.50-7.28 (m, 1H), 7.26-7.01 (m, 1H), 5.87-5.70 (m, 1H), 5.55 - 5.36 (m, 1H), 5.07-4.78 (m, 2H), 4.75-4.41 (m, 1H), 3.85-3.70 (m, 2H), 3.31 - 2.67 (m, 10H), 2.34-2.27 (m, 3H), 2.23 - 1.43 (m, 11H), 1.31 - 1.10 (m, 3H).

**Example 5: N-($1^4$,$1^4$-difluoro-$2^6$-methoxy-$5^1$,4-dioxo-$5^2$,$5^3$-dihydro-$5^1$*H*-3-aza-2(2,4)-pyrimidin-1(1,3),6(1,4)-dipiperidin-5(4,5)-ind enoheterocyclononane-8-en-$5^7$-yl)-2-hydroxyethane-1-sulfonamide**

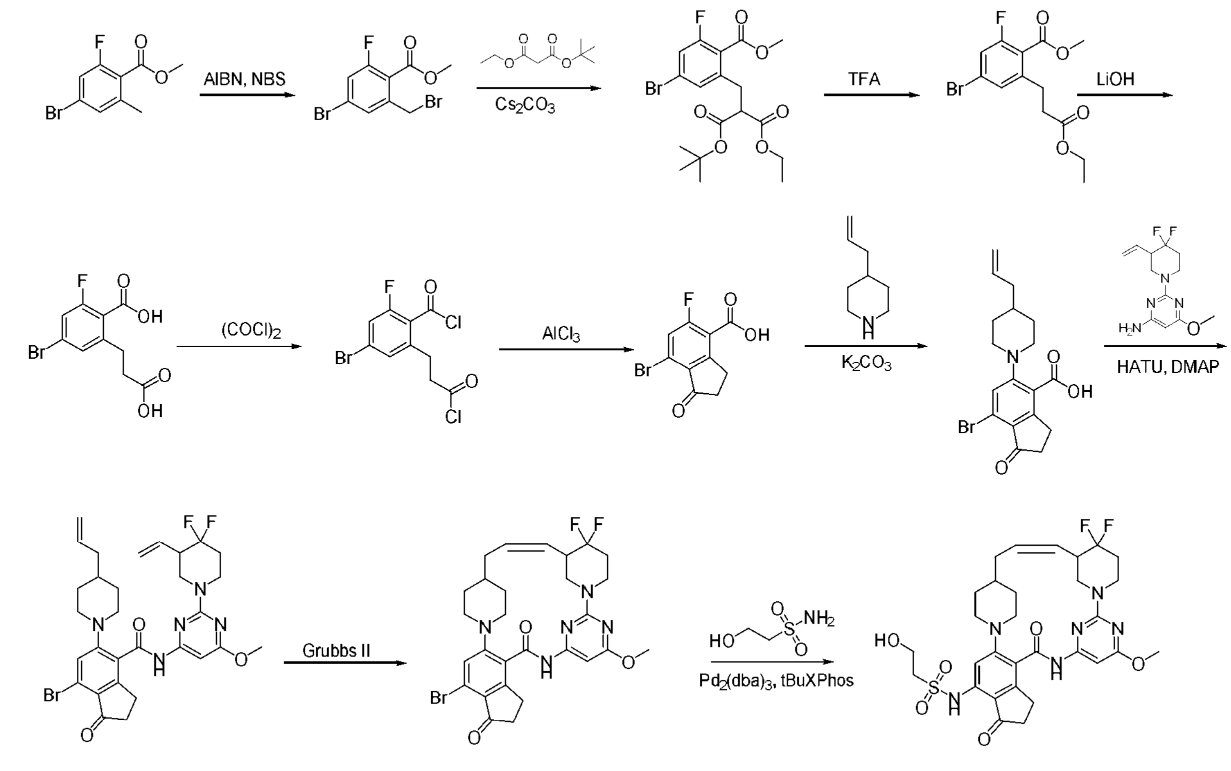


**Step 1:** A mixture containing methyl 4-bromo-2-fluoro-6-methylbenzoate (25.00 g, 101.19 mmol), N-bromosuccinimide (21.60 g, 121.43 mmol), azobisisobutyronitrile (8.50 g, 50.60 mmol) and acetonitrile (250.00 mL) was stirred at 70°C for 3 hours. The reaction mixture was poured into water (50 mL) and extracted with ethyl acetate (50 mL × 3). The organic layer was dried over anhydrous sodium sulfate, filtered, concentrated and purified by column chromatography (silica gel, 0-10% gradient of ethyl acetate/petroleum ether) to give methyl 4-bromo-2-(bromomethyl)-6-fluorobenzoate (26.20 g, crude) as a colorless oil. LCMS (ESI): $[M+H]^+$ =326.90.

**Step 2:** A mixture containing methyl 4-bromo-2-(bromomethyl)-6-fluorobenzoate (26.00 g, 79.76 mmol), tert-butyl propane-1,3-dioate (16.50 g, 87.74 mmol), cesium carbonate (53.00 g, 159.53 mmol) and N,N-dimethylformamide (200.00 mL) was stirred at 20°C for 2 hours. The reaction mixture was poured into water (300 mL) and extracted with ethyl acetate (100 mL × 3), and the organic phase was dried over anhydrous sodium sulfate, filtered, concentrated, and purified by column chromatography (silica gel, 0-10% gradient of ethyl acetate/petroleum ether) to give 1-(tert-butyl)-3-ethyl-2-(5-bromo-3-fluoro-2-(methoxycarbonyl)benzyl)malonate (21.00 g, crude) as a colorless oil compound. LCMS (ESI): $[M+Na]^+$ = 455.00.

**Step 3:** A solution containing 1-(tert-butyl)-3-ethyl-2-(5-bromo-3-fluoro-2-(methoxycarbonyl)benzyl)malonate (21.00 g, 48.47 mmol) and trifluoroacetic acid (60.00 mL) was stirred at 20°C for 1 hour. The reaction solution was concentrated to remove the trifluoroacetic acid, then toluene (60.00 mL) and triethylamine (50.00 g, 484.69 mmol) were added and the mixture was stirred at 120°C for 3 hours. The reaction mixture was concentrated, aqueous sodium bicarbonate solution was added to adjust the pH to be close to 8, and then the mixture was extracted with ethyl acetate (50 mL × 3). The organic phase was dried over anhydrous sodium sulfate, filtered, concentrated, and purified by column chromatography (silica gel, 0-10% gradient of ethyl acetate/petroleum ether) to give the compound methyl 4-bromo-2-(3-ethoxy-3-oxopropyl)-6-fluorobenzoate (11.20 g, crude). LCMS (ESI): $[M+H]^+$ = 333.00.

**Step 4:** A mixture containing methyl 4-bromo-2-(3-ethoxy-3-oxopropyl)-6-fluorobenzoate (11.20 g, 33.62 mmol), lithium hydroxide monohydrate (8.10 g, 336.18 mmol), tetrahydrofuran (40 mL), methanol (40 mL) and water (40 mL) was stirred at 20°C for 4 hours. The reaction mixture was concentrated to remove tetrahydrofuran and methanol, 4 M hydrochloric acid ethyl acetate solution (100 mL) was added to adjust the pH to 5, and then the mixture was extracted with ethyl acetate (100 mL × 3). The organic phase was dried over anhydrous sodium sulfate, filtered, and concentrated to give 4-bromo-2-(2-carboxyethyl)-6-fluorobenzoic acid (5.10 g, 17.52 mmol, yield: 52.1%) as a white solid compound. LCMS (ESI): $[M+Na]^+$ = 313.00.

**Step 5:** Oxalyl chloride (11.10 g, 85.89 mmol) was added to a mixed solution containing 4-bromo-2-(2-carboxyethyl)-6-fluorobenzoic acid (5.00 g, 17.18 mmol) and dichloromethane (50 mL), and the resulting mixture was stirred at 20°C for 1 h. The reaction mixture was concentrated to give 4-bromo-2-(3-chloro-3-oxopropyl)-6-fluorobenzoyl chloride (5.70 g, crude) as a yellow oil compound. LCMS (ESI): $[M+H]^+$ = 319.00 (corresponding methyl ester).

**Step 6:** 4-Bromo-2-(3-chloro-3-oxypropyl)-6-fluorobenzoyl chloride (5.60 g, 17.08 mmol) was dissolved in dichloromethane (10.00 mL) and the mixture was added dropwise to a solution containing aluminum trichloride (9.10 g, 68.30 mmol) and dichloromethane (50.00 mL) at 0°C. The reaction solution was stirred at 40°C for 16 hours. The reaction mixture was poured into water (30 mL) and extracted with dichloromethane (30 mL × 3). The organic layer was dried over anhydrous sodium sulfate, filtered, concentrated and purified by column chromatography (silica gel, 0-100% gradient of ethyl acetate/petroleum ether) to give 7-bromo-5-fluoro-1-oxo-2,3-dihydro-1H-indene-4-carboxylic acid (3.40 g, crude) as a brown solid, LCMS (ESI): $[M+H]^+$ = 273.00.

**Step 7:** A dimethyl sulfoxide solution (10.00 mL) containing 7-bromo-5-fluoro-1-oxo-2,3-dihydro-1*H*-indene-4-carboxylic acid (1.00 g, 3.66 mmol), 4-allylpiperidine (550 mg, 4.39 mmol), and potassium carbonate (2.60 g, 18.31 mmol) was stirred at 100°C for 3 hours. The reaction mixture was poured into water (30 mL), then hydrochloric acid/ethyl acetate solution (6 mL, 4 M) was added to adjust the pH to about 5, and the mixture was extracted with ethyl acetate (30 mL × 3). The organic layer was dried over anhydrous sodium sulfate, filtered, concentrated, and purified by column chromatography (silica gel, 0-100% gradient of ethyl acetate/petroleum ether) to give 5-(4-allylpiperidin-1-yl)-7-bromo-1-oxo-2,3-dihydro-1*H*-indene-4-carboxylic acid (1.00 g, 2.64 mmol, yield: 72.2%) as a yellow solid, LCMS (ESI): $[M+H]^+$ = 378.10.

**Step 8:** A solution of 5-(4-allylpiperidin-1-yl)-7-bromo-1-oxo-2,3-dihydro-1H-indene-4-carboxylic acid (1.00 g, 2.64 mmol), 2-(4,4-difluoro-3-vinylpiperidin)-6-methoxypyrimidin-4-amine (857 mg, 3.17 mmol), 2-(7-azobenzotriazol)-N,N,N',N'-tetramethyluronium hexafluorophosphate (3.10 g, 7.93 mmol) and 4-dimethylaminopyridine (1.60 g, 13.22 mmol) in dichloroethane (20.00 mL) was stirred at 80°C for 12 hours. The reaction solution was concentrated and purified by column chromatography (silica gel, 0-30% gradient of ethyl acetate/petroleum ether) to give 5-(4-allylpiperidin-1-yl)-7-bromo-*N*-(2-(4,4-difluoro-3-vinylpiperidin-1-yl)-6-methoxypyrimidin-4-yl)-1-oxo-2,3-dihydro-1 H-indene-4-carboxamide (1.00 g, crude) as a yellow oil. LCMS (ESI): $[M+H]^+$ = 630.20.

**Step 9:** A solution of 5-(4-allylpiperidin-1-yl)-7-bromo-*N*-(2-(4,4-difluoro-3-vinylpiperidin-1-yl)-6-methoxypyrimidin-4-yl)-1-oxo-2,3-dihydro-1 H-indene-4-carboxamide (400 mg, 0.63 mmol) and Grubbs catalyst $2^{nd}$ generation (55 mg, 0.063 mmol) in dichloromethane (100.00 mL) was stirred at 50°C for 2 hours. The reaction solution was concentrated and purified by column chromatography (silica gel, 0-30% gradient of ethyl acetate/petroleum ether) to give $5^7$-bromo-$1^4$,$1^4$-difluoro-$2^6$-methoxy-$5^2$,$5^3$-dihydro-$5^1$*H*-3-aza-2(2,4)-pyrimidin-1(1,3),6(1,4)-dipiperidin-5(4,5)-indenoheter ocyclonon-8-en-$5^1$,4-dione (200 mg, 0.33 mmol, yield: 52.4%) as a gray solid. LCMS (ESI): $[M+H]^+$ = 602.20.

**Step 10:** A solution of $5^7$-bromo-$1^4$,$1^4$-difluoro-$2^6$-methoxy-$5^2$,$5^3$-dihydro-$5^1$*H*-3-aza-2(2,4)-pyrimidin-1(1,3),6(1,4)-dipiperidin-5(4,5)-indenoheter ocyclonon-8-en-$5^1$,4-dione (200 mg, 0.33 mmol), 2-hydroxyethanesulfonamide (84 mg, 0.66 mmol), potassium phosphate (216 mg, 1.00 mmol), cuprous iodide (65 mg, 0.337 mmol) and trans-N,N-dimethylcyclohexane-1,2-diamine (48 mg, 0.33 mmol) in 1,4-dioxane (5.00 mL) was stirred at 100°C for 2 hours under nitrogen atmosphere. The reaction mixture was poured into water (30 mL) and extracted with ethyl acetate (30mL × 3). The organic layer was dried over anhydrous sodium sulfate, filtered, concentrated and purified by column chromatography (silica gel, 0-100% gradient of ethyl acetate/petroleum ether) to give *N*-($1^4$,$1^4$-difluoro-$2^6$-methoxy-$5^1$,4-dioxo-$5^2$,$5^3$-dihydro-$5^1$*H*-3-aza-2(2,4)-pyrimidin-1(1,3),6(1,4)-dipiperidin-5(4,5)-indenoh eterocyclononane-8-en-$5^7$-yl)-2-hydroxyethane-1-sulfonamide (60 mg, 0.093 mmol, yield: 27.9%) as a gray solid. LCMS (ESI): $[M+H]^+$ = 647.30.

**Example 6: N-(($1^3$S)-$1^4$,$1^4$-difluoro-$2^6$-methyl-$5^1$,4-dioxo-$5^2$,$5^3$-dihydro-$5^1$*H*-3-aza-2(2,4)-pyrimidin-1(1,3),6(1,4)-dipiperidin-5(4,5) -indenoheterocyclononane-$5^7$-yl)-2-hydroxyethane-1-sulfonamide**

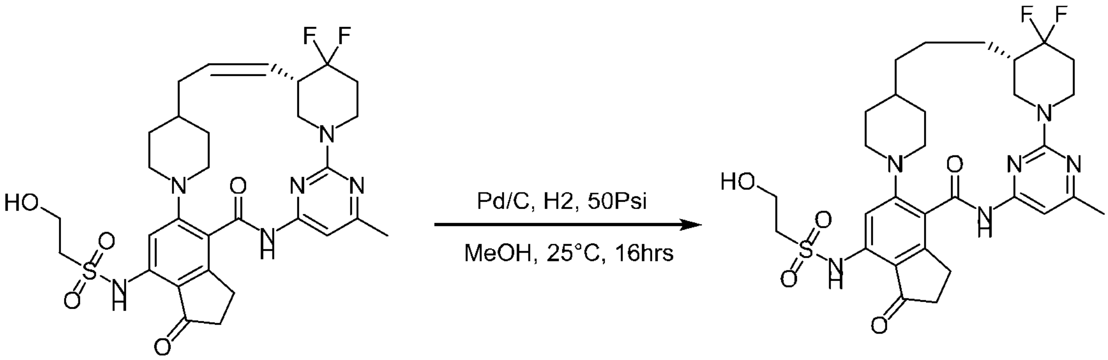


**Step 1:** N-(($1^3$S,Z)-$1^4$,$1^4$-difluoro-$2^6$-methyl-$5^1$,4-dioxo-$5^2$,$5^3$-dihydro-$5^1$*H*-3-aza-2(2,4)-pyrimidin-1(1,3),6(1,4)-dipiperidin-5(4,5)-i ndenoheterocyclonon-8-en-$5^7$-yl)-2-hydroxyethane-1-sulfonamide (40.0 mg, 0.06 mmol) was dissolved in methanol (10.00 mL), and 10% Pd/C (wet) (20.0 mg, 0.01 mmol) was added. The reaction mixture was purged with argon three times and then hydrogen three times, and stirred at 25°C for 16 hours under hydrogen (50 Psi) atmosphere. The reaction mixture was filtered under reduced pressure to remove palladium/carbon and then concentrated in vacuum. The residue was purified by preparative HPLC (C18, 46%-86% gradient of water (formic acid)/acetonitrile) to give N-(($1^3$S)-$1^4$,$1^4$-difluoro-$2^6$-methyl-$5^1$,4-dioxo-$5^2$,$5^3$-dihydro-$5^1$H-3-aza-2(2,4)-pyrimidin-1(1,3),6(1,4)-dipiperidin-5(4,5)-ind enoheterocyclononane-$5^7$-yl)-2-hydroxyethane-1-sulfonamide (15.0 mg, 0.02 mmol, yield: 37.4%) as a white solid. LCMS (ESI): $[M+H]^+$ =633.2. $^1$H NMR (400 MHz, DMSO-*d*$_6$) δ ppm 10.44 - 10.23 (m, 1H), 10.07 - 9.53 (m, 1H), 7.56 - 7.41 (m, 1H), 6.94 (br s, 1H), 5.33 - 4.88 (m, 1H), 4.80 - 4.52 (m, 2H), 3.77 (br d, *J* = 5.2 Hz, 2H), 3.52 (br s, 3H), 3.20 - 3.02 (m, 2H), 2.96 - 2.78 (m, 3H), 2.66 - 2.58 (m, 2H), 2.36 - 2.22 (m, 3H), 2.12 - 1.47 (m, 10H), 1.39 - 0.95 (m, 6H)

**Example 7: N-(($1^3$R,Z)-$1^4$,$1^4$-difluoro-$5^1$-hydroxy-$2^6$-methyl-4-oxo-$5^2$,$5^3$-dihydro-$5^1$H-3-aza-2(2,4)-pyrimidin-1(1,3),6(1,4)-dipiperi din-5(4,5)-indenoheterocyclonon-8-en-$5^7$-yl)-2-hydroxyethane-1-sulfonamide**

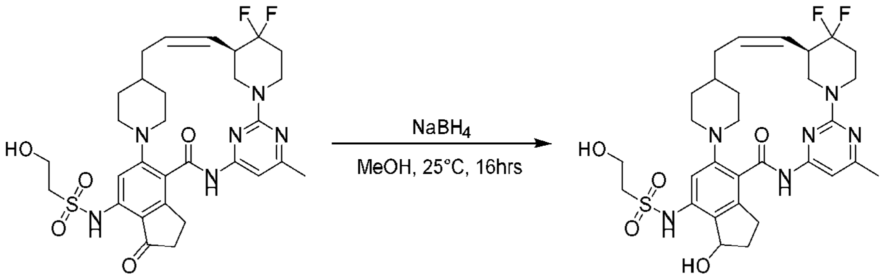


**Step 1:**

N-(($1^3$R,Z)-$1^4$,$1^4$-difluoro-$2^6$-methyl-$5^1$,4-dioxo-$5^2$,$5^3$-dihydro-$5^1$H-3-aza-2(2,4)-pyrimidin-1(1,3),6(1,4)-dipiperidin-5(4,5)-i ndenoheterocyclonon-8-en-$5^7$-yl)-2-hydroxyethane-1-sulfonamide (40.0 mg, 0.06 mmol) was dissolved in methanol (10.0 mL), sodium borohydride (7.32 mg, 0.19 mmol) was added and the reaction mixture was stirred at 25°C for 16 hours. The reaction solution was poured into water (5.00 mL) and extracted with ethyl acetate (5 mL×2). The organic layer was concentrated to give a crude product. The residue was purified by preparative HPLC (C18, 34%-74% gradient of water (formic acid)/acetonitrile) to give N-(($1^3$R,Z)-$1^4$,$1^4$-difluoro-$5^1$-hydroxy-$2^6$-methyl-4-oxo-$5^2$,$5^3$-dihydro-$5^1$H-3-aza-2(2,4)-pyrimidin-1(1,3),6(1,4)-dipiperidin-5(4,5)-indenoheterocyclonon-8-en-$5^7$-yl)-2-hydroxyethane-1-sulfonamide (14.0 mg, 0.02 mmol, yield: 34.8%) as a white solid. LCMS (ESI): $[M+H]^+$ = 591.2. $^1$H NMR (400 MHz, DMSO-$d_6$) δ ppm 12.60 (br d, *J* = 10.4 Hz, 1H), 9.37 - 8.66 (m, 1H), 7.30 (s, 2H), 5.87 - 5.71 (m, 1H), 5.50 (br t, *J* = 9.6 Hz, 2H), 5.39 - 5.20 (m, 1H), 5.15 - 4.95 (m, 1H), 4.71 (br d, *J* = 12.4 Hz, 1H), 4.57 - 4.39 (m, 1H), 3.78 (br s, 2H), 3.41 (br s, 2H), 3.17 (br s, 3H), 3.08 - 2.66 (m, 6H), 2.41 - 2.20 (m, 5H), 2.17 - 2.03 (m, 2H), 2.00 - 1.66 (m, 6H), 1.53 (br d, *J* = 13.6 Hz, 1H)

**Example 8: *N*-($1^4$,$1^4$-difluoro-$2^6$-methyl-$5^1$,4-dioxo-$5^1$,$5^2$,$5^3$,$5^4$-tetrahydro-3-aza-2(2,4)-pyrimidin-1(1,3),6(1,4)-dipiperidin-5(5,6)-in denoheterocyclonon-8-en-$5^8$-yl)-2-hydroxyethane-1-sulfonamide**

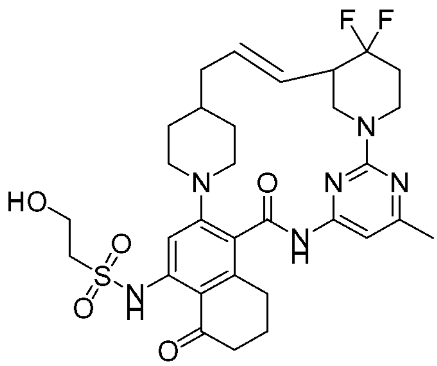

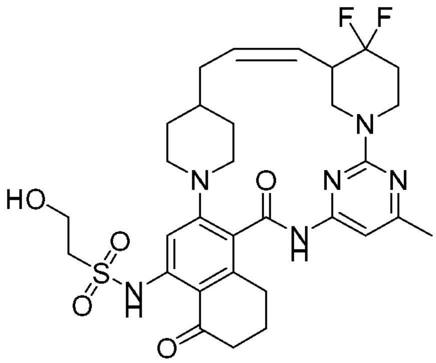

The preparations in **Example 8 followed** the synthetic route of **Example 2.**

**Example 8A (trans isomer)** LCMS (ESI): $[M+H]^+$ = 645.8. $^1$H NMR (400 MHz, DMSO-$d_6$) δ ppm 12.25 (s, 1H), 10.36 (s, 1H), 7.40 (s, 1H), 6.98 (s, 1H), 5.83 - 5.63 (m, 1H), 5.29 (dd, *J* = 8.4, 15.6 Hz, 1H), 4.99 (br d, *J* = 12.0 Hz, 2H), 3.74 (t, *J* = 6.0 Hz, 2H), 3.48 (br s, 2H), 3.12 - 3.06 (m, 1H), 3.03 - 2.79 (m, 4H), 2.76 - 2.57 (m, 4H), 2.34 (s, 3H), 2.29 - 1.94 (m, 5H), 1.93 - 1.71 (m, 3H), 1.67 - 1.52 (m, 3H), 1.44 - 1.28 (m, 1H), 1.18 - 1.00 (m, 1H).

**Example 8B (cis isomer)** LCMS (ESI): $[M+H]^+$ = 645.7. $^1$H NMR (400 MHz, DMSO-$d_6$) δ ppm 12.21 (br s, 1H), 10.32 (s, 1H), 7.02 (s, 1H), 6.82 (s, 1H), 5.71 (dt, *J* = 5.6, 11.2 Hz, 1H), 5.54 - 5.31 (m, 1H), 5.07 - 4.85 (m, 1H), 4.77 - 4.61 (m, 1H), 4.59 - 4.46 (m, 1H), 3.74 (br d, *J* = 2.4 Hz, 2H), 3.64 (br d, *J* = 10.8 Hz, 1H), 3.47 (t, *J* = 6.4 Hz, 2H), 3.31 - 3.27 (m, 1H), 3.24 - 3.12 (m, 1H), 3.11 - 2.92 (m, 2H), 2.91 - 2.72 (m, 4H), 2.63 - 2.57 (m, 2H), 2.34 (s, 3H), 2.20 (s, 2H), 2.02 - 1.77 (m, 4H), 1.75 - 1.59 (m, 4H), 1.43 - 1.27 (m, 1H).

**Example 9: *N*-(11,11-difluoro-6-methyl-1,5,22-trioxo-1,5,6,9,10,11,12,15,16,17,18,22,23,24-tetradecahydro-2H-3,7-(azanediyl)-16, 19-ethano-8,12-methyleneindeno[4,5-i] [1,3,7,11]tetraazacyclohenicosan-21-yl)-2-hydroxyethane-1-sulfonamide**

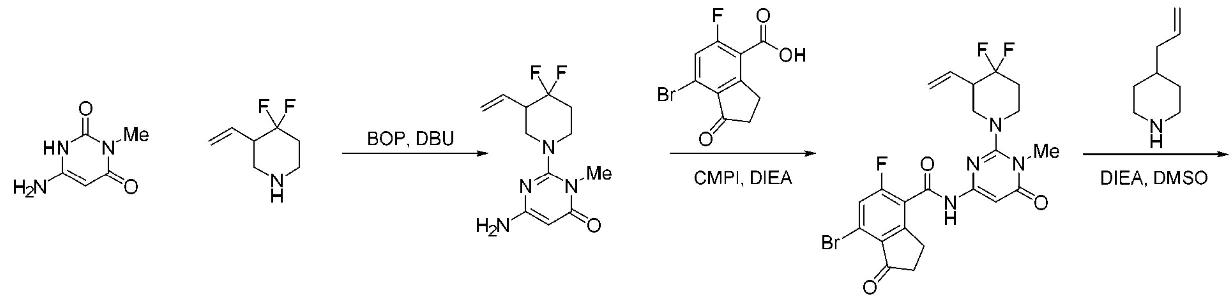


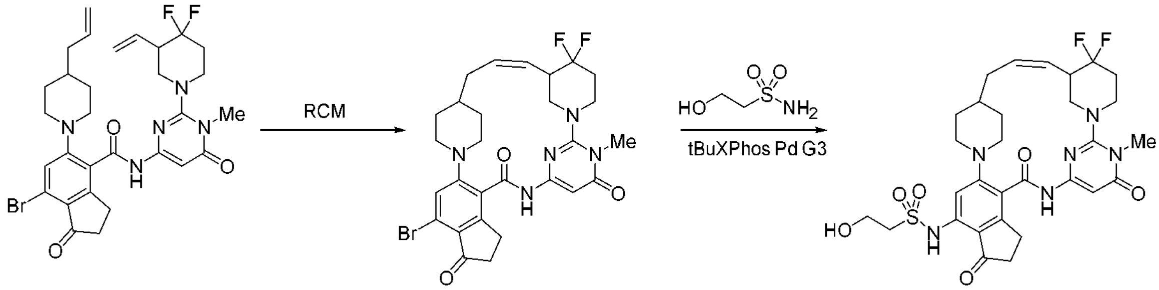


**Step 1:** Under nitrogen atmosphere, a solution of 6-amino-3-methylpyrimidine-2,4(1H,3H)-dione (1.00 g, 7.09 mmol), 1,8-diazabicyclo[5.4.0]undec-7-ene (5.45 g, 35.43 mmol) and benzotriazol-1-yloxytris(dimethylamino)phosphonium hexafluorophosphate (9.50 g, 21.26 mmol) *in N,N*-dimethylformamide (50.00 mL) was stirred for 15 minutes, then 4,4-difluoro-3-vinylpiperidine hydrochloride (1.56 g, 8.50 mmol) was added and the reaction mixture was stirred at 20°C for 2 hours. After the reaction was complete, the reaction was quenched with cold water (40 mL) and the mixture was extracted with ethyl acetate (50 mL × 3). The combined organic layer was washed with brine (100 mL), dried over magnesium sulfate, filtered and concentrated to give a crude product, which was purified by flash column chromatography (silica, 0-20% dichloromethane/methanol) to give 6-amino-2-(4,4-difluoro-3-vinylpiperidin-1-yl)-3-methylpyrimidin-4(3*H*)-one (1.60 g, 5.92 mmol, yield: 83.5%) as a yellow liquid. LCMS (ESI): $[M+H]^+$ = 271.10.

**Step 2:** A solution of 6-amino-2-(4,4-difluoro-3-vinylpiperidin-1-yl)-3-methylpyrimidin-4(3H)-one (1.60 g, 5.92 mmol), 7-bromo-5-fluoro-1-oxo-2,3-dihydro-1H-indene-4-carboxylic acid (1.62 g, 5.92 mmol), 2-chloro-1-methylpyridinium iodide (2.31 g, 8.88 mmol) and N,N-diisopropylethylamine (3.90 g, 29.60 mmol) in tetrahydrofuran (30.00 mL) was stirred in an oil bath at 50°C for 15 hours. After the reaction was complete, the reaction was quenched with a sodium bicarbonate solution (30 mL) and the mixture was extracted with ethyl acetate (50 mL × 3). The combined organic layer was washed with brine (100 mL), dried over sodium sulfate, filtered and concentrated to give a crude product, which was purified by flash column chromatography (silica, 50-100% ethyl acetate/petroleum ether) to give 7-bromo-*N*-(2-(4,4-difluoro-3-vinylpiperidin-1-yl)-1-methyl-6-oxo-1,6-dihydropyrimidin-4-yl)-5-fluoro-1-oxo-2,3-dihydro-1*H*-indene-4-carbonamide (370 mg, 0.70 mmol, yield: 11.9%) as a yellow solid. LCMS (ESI): $[M+H]^+$ = 525.10.

**Step 3:** A solution of 7-bromo-*N*-(2-(4,4-difluoro-3-vinylpiperidin-1-yl)-1-methyl-6-oxo-1,6-dihydropyrimidin-4-yl)-5-fluoro-1-oxo-2,3-dihydro-1H-indene-4-carbonamide (370 mg, 0.70 mmol), 4-allylpiperidine hydrochloride (228 mg, 1.41 mmol) and N,N-diisopropylethylamine (464 mg, 3.52 mmol) in dimethyl sulfoxide (20.00 mL) was stirred in an oil bath at 80°C for 8 hours. After the reaction was complete, the reaction was quenched with cold water (50 mL) and the mixture was extracted with ethyl acetate (50 mL × 3). The combined organic layer was dried, filtered and concentrated to give a crude product, which was purified by flash column chromatography (silica, 50-100% ethyl acetate/petroleum ether) to give 5-(4-allylpiperidin-1-yl)-7-bromo-*N*-(2-(4,4-difluoro-3-vinylpiperidin-1-yl)-1-methyl-6-oxo-1,6-dihydropyrimidin-4-yl)-1-o xo-2,3-dihydro-1*H*-indene-4-carbonamide (300 mg, 0.48 mmol, yield: 67.6%) as a yellow solid. LCMS (ESI): $[M+H]^+$ = 630.20.

**Step 4:** Under nitrogen atmosphere, 5-(4-allylpiperidin-1-yl)-7-bromo-*N*-(2-(4,4-difluoro-3-vinylpiperidin-1-yl)-1-methyl-6-oxo-1,6-dihydropyrimidin-4-yl)-1-o xo-2,3-dihydro-1*H*-indene-4-carbonamide (300 mg, 0.48 mmol) was dissolved in dry dichloroethane (50.00 mL), Grubbs catalyst 2$^{nd}$ generation (21 mg, 0.02 mmol) was added and the mixture was heated to 80°C for 16 hours. After the reaction was complete, the reaction solution was concentrated and purified by flash column chromatography (silica, 30-70% ethyl acetate/petroleum ether) to give 21-bromo-11,11-difluoro-6-methyl-9,10,11,12,15,16,17,18,23 ,24-decahydro-2*H*-3,7-(azanediyl)-16,19-ethano-8,12-methyle neindeno[*4,5-i*][1,3,7,11]tetraazacyclohenicosan-1,5,22(6*H*)-trione (60 mg, 0.10 mmol, yield: 20.9%) as a white solid. LCMS (ESI): $[M+H]^+$ = 602.20.

**Step 5:** Under nitrogen atmosphere, 21-bromo-11,11-difluoro-6-methyl-9,10,11,12,15,16,17,18,23,24-decahydro-2H-3,7-(azanediyl)-16,19-ethano-8,12-methyle neindeno[4,5-i][1,3,7,11]tetraazacyclohenicosan-1,5,22(6H)-trione (60 mg, 0.10 mmol), 2-hydroxyethylsulfonamide (38 mg, 0.30 mmol), potassium phosphate (49 mg, 0.50 mmol), methanesulfonic acid(2-di-tert-butylphosphino-2',4',6'-triisopropyl-1,1'-biphenyl)(2'-amino-1,1'-biphenyl-2-yl)palladium(II) (8 mg, 0.01 mmol) were dissolved in 1,4-dioxane (5.00 mL). The resulting reaction mixture was stirred at 100°C for 6 hours. After the reaction was complete, the reaction was quenched with cold water (20 mL) and the mixture was extracted with ethyl acetate (20 mL × 3). The combined organic layer was washed with brine (100 mL), dried over magnesium sulfate, filtered and concentrated to give a crude product, which was purified by flash column chromatography (silica, 60-90% ethyl acetate/petroleum ether) to give *N*-(11,11-difluoro-6-methyl-1,5,22-trioxo-1,5,6,9,10,11,12,15,16,17,18,22,23,24-tetradecahydro-2H-3,7-(azanediyl)-16,19-e thano-8,12-methyleneindeno[4,5-i][1,3,7,11]tetraazacyclohenicosan-21-yl)-2-hydroxyethane-1-sulfonamide (47 mg, 0.07 mmol, yield: 73.0%) as a white solid. LCMS (ESI): $[M+H]^+$ = 647.30.$^1H$ NMR (400 MHz, DMSO-$d_6$) δ 11.23 (s, 0.6H), 10.06 (s, 0.6H), 9.98 - 9.68 (m, 0.8H), 7.34 - 6.36 (m, 3H), 5.93 - 4.79 (m, 4H), 4.11 - 3.48 (m, 9H), 3.13 - 2.62 (m, 8H), 2.40 - 1.54 (m, 9H) ppm;

**Example 10: N-((1$^3$S)-1$^4$,1$^4$-difluoro-2$^6$-methyl-5$^1$,4-dioxo-5$^2$,5$^3$-dihydro-5$^1$H-3-aza-2(2,4)-pyrimidin-1(1,3),6(1,4)-dipiperidin-5(4,5) -indenobicyclononane-5$^7$-yl)-2-hydroxyethane-1-sulfonamide**

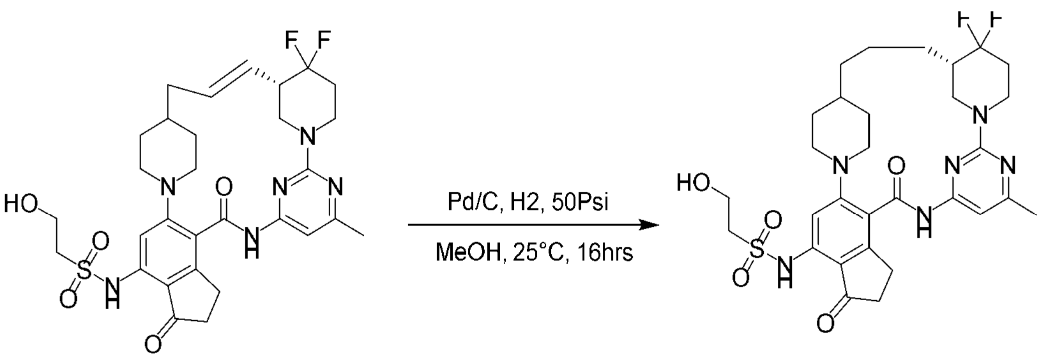


N-((1$^3$S,Z)-1$^4$,1$^4$-difluoro-2$^6$-methyl-5$^1$,4-dioxo-5$^2$,5$^3$-dihydro-5$^1$H-3-aza-2(2,4)-pyrimidin-1(1,3),6(1,4)-dipiperidin-5(4,5)-i ndenocyclononane-8-en-5$^1$-yl)-2-hydroxyethane-1-sulfonamide (100 mg, 0.16 mmol) was dissolved in methanol (10.00 mL), and 10% Pd/C (dry) (10.0 mg, 0.01 mmol) was added. The reaction mixture was purged with argon three times and then hydrogen three times, and stirred at 25°C for 16 hours under hydrogen (50 Psi) atmosphere. The reaction mixture was filtered under reduced pressure to remove palladium/carbon and then concentrated in vacuum. The residue was purified by preparative HPLC (C18, 58%88% gradient of water (formic acid)/acetonitrile) to give N-((1$^3$S)-1$^4$,1$^4$-difluoro-2$^6$-methyl-5$^1$,4-dioxo-5$^2$,5$^3$-dihydro-5$^1$H-3-aza-2(2,4)-pyrimidin-1(1,3),6(1,4)-dipiperidin-5(4,5)-ind enobicyclononane-5$^7$-yl)-2-hydroxyethane-1-sulfonamide (25.0 mg, 0.04 mmol, yield: 25%) as a white solid. LCMS (ESI): $[M+H]^+$ =633.2. $^1H$ NMR (400 MHz, DMSO-$d_6$) δ ppm 10.31 (s, 1H), 9.86 (s, 1H), 7.51 (s, 1H), 6.93 (s, 1H), 5.06 (br t, *J* = 4.4 Hz, 1H), 4.84 - 4.51 (m, 2H), 3.77 (q, *J* = 5.6 Hz, 2H), 3.60 - 3.48 (m, 3H), 3.25 - 3.01 (m, 3H), 2.93 - 2.81 (m, 3H), 2.62 (br d, *J* = 4.0 Hz, 2H), 2.31 (s, 3H), 2.09 - 1.56 (m, 9H), 1.52 (br d, *J* = 12.4 Hz, 1H), 1.39 - 1.30 (m, 1H), 1.23 - 1.01 (m, 4H)

**Example 11: (Z)-N-(1$^4$,1$^4$-difluoro-2$^6$-methyl-5$^1$,4-dioxo-5$^2$,5$^3$-dihydro-5$^1$hydro-3-aza-2(2,4)-pyrimidin-1(1,3),6(1,4)-dipiperidin-5(4, 5)-indenocyclonofin-8-en-5$^7$-yl)-1-hydroxypropane-2-sulfonamide**

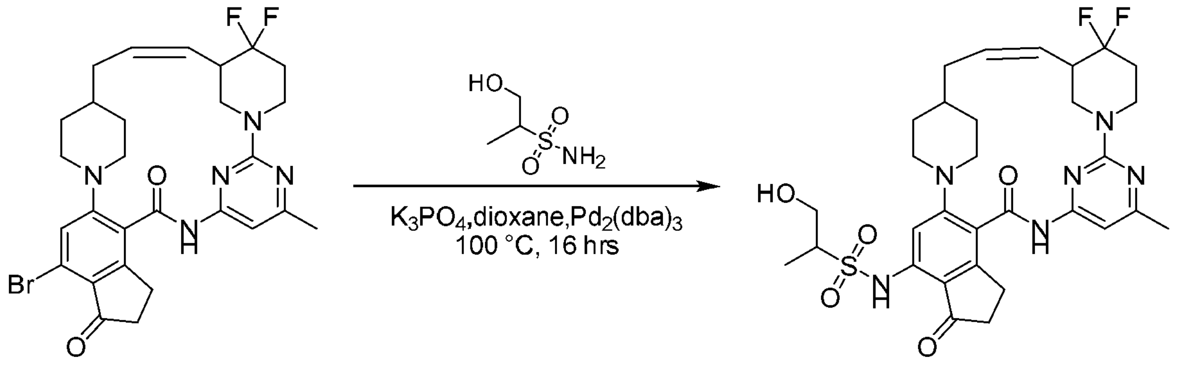


At 25°C, to a solution of (Z)-$5^7$-bromo-$1^4$,$1^4$-difluoro-$2^6$-methyl-$5^2$,9-dihydro-$5^1$hydro-3-aza-2(2,4)-pyrimidin-1(1,3),6(1,4)-dipiperidin-5(4,5)-inden ocyclononane-8-en-$5^1$,4-dione (300 mg, 0.51 mmol) in 1,4-dioxane (6.00 mL) were added 1-hydroxypropane-2-sulfonamide (142 mg, 1.02 mmol), potassium phosphate (332 mg, 1.53 mmol), di-tert-butyl-[2-(2,4,6-triisopropylphenyl)phenyl]phosphane (21.7 mg, 0.05 mmol) and tris(dibenzylideneacetone)dipalladium (47.8 mg, 0.05 mmol). The reaction mixture was heated to 100°C under nitrogen atmosphere and stirred for 16 hours. The reaction mixture was concentrated under reduced pressure, the solvent was removed, and the residue was diluted with N,N-dimethylformamide (10 mL). The diluted solution was purified by preparative HPLC (C18, 46-86% gradient of water (formic acid)/acetonitrile) to give (Z)-N-($1^4$,$1^4$-difluoro-$2^6$-methyl-$5^1$,4-dioxo-$5^2$,$5^3$-dihydro-$5^1$hydro-3-aza-2(2,4)-pyrimidin-1(1,3),6(1,4)-dipiperidin-5(4,5)-i ndenocyclonofin-8-en-$5^7$-yl)-1-hydroxypropane-2-sulfonamide (15 mg, 0.02 mmol, yield: 4.55%) as a white solid. LCMS (ESI): $[M+H]^+$ = 645.3. $^1H$ NMR (400 MHz, DMSO-*d6*) δ ppm 11.16 (br s, 1H), 10.02 (br s, 1H), 7.37 - 7.13 (m, 2H), 5.75 (dt, *J* = 5.6, 11.2 Hz, 1H), 5.45 (br t, *J* = 10.4 Hz, 1H), 5.20 (br s, 1H), 4.63 (br d, *J* = 12.8 Hz, 1H), 4.51 - 4.37 (m, 1H), 3.80 - 3.68 (m, 1H), 3.68 - 3.48 (m, 2H), 3.47 - 3.40 (m, 1H), 3.30 - 3.27 (m, 1H), 3.25 - 3.16 (m, 2H), 3.14 - 3.03 (m, 2H), 2.99 - 2.85 (m, 2H), 2.84 - 2.75 (m, 1H), 2.67 (br d, *J* = 2.0 Hz, 2H), 2.38 - 2.23 (m, 4H), 2.16 - 2.01 (m, 2H), 2.00 - 1.65 (m, 5H), 1.63 - 1.48 (m, 1H), 1.29 (br dd, *J* = 3.2, 6.8 Hz, 3H).

**Example 12: (Z)-N-($1^4$,$1^4$-difluoro-$2^6$-methyl-$5^1$,4-dioxo-$5^2$,$5^3$-dihydro-$5^1$hydro-3-aza-2(2,4)-pyrimidin-1(1,3),6(1,4)-dipiperidin-5(4, 5)-indenocyclonofin-8-en-$5^7$-yl)-1-hydroxy-2-methylpropane-2-sulfonamide**

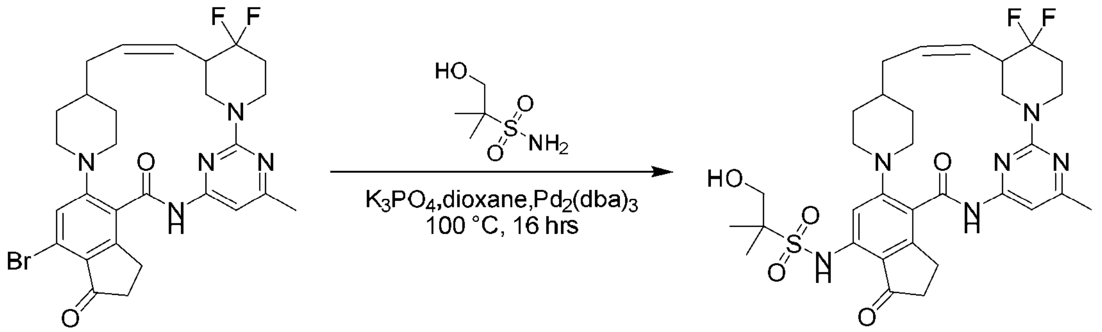


At 25°C, to a solution of (Z)-$5^7$-bromo-$1^4$,$1^4$-difluoro-$2^6$-methyl-$5^2$,$5^3$-dihydro-$5^1$hydro-3-aza-2(2,4)-pyrimidin-1(1,3),6(1,4)-dipiperidin-5(4,5)-inden ocyclononane-8-en-$5^1$,4-dione (300 mg, 0.51 mmol) in 1,4-dioxane (6.00 mL) were added 1-hydroxy-2-methylpropane-2-sulfonamide (157 mg, 1.02 mmol), potassium phosphate (332 mg, 1.53 mmol), di-tert-butyl-[2-(2,4,6-triisopropylphenyl)phenyl]phosphane (21.7 mg, 0.05 mmol) and tris(dibenzylideneacetone)dipalladium (47.8 mg, 0.05 mmol). The reaction mixture was heated to 100°C under nitrogen atmosphere and stirred for 16 hours. The reaction mixture was concentrated under reduced pressure, the solvent was removed, and the residue was diluted with N,N-dimethylformamide (10 mL). The diluted solution was purified by preparative HPLC (C18, 50-90% gradient of water (formic acid)/acetonitrile) to give (Z)-N-($1^4$,$1^4$-difluoro-$2^6$-methyl-$5^1$,4-dioxo-$5^1$,$5^1$-dihydro-$5^1$hydro-3-aza-2(2,4)-pyrimidin-1(1,3),6(1,4)-dipiperidin-5(4,5)-i ndenocyclonofin-8-en-$5^7$-yl)-1-hydroxy-2-methylpropane-2-sulfonamide (13 mg, 0.02 mmol, yield: 3.9%) as a white solid. LCMS (ESI): $[M+H]^+$ = 659.3. $^1H$ NMR (400 MHz, DMSO-$d_6$) δ ppm 11.36 (br s, 1H), 10.15 (br s, 1H), 7.43 (s, 1H), 7.36 (s, 1H), 5.86 - 5.78 (m, 1H), 5.59 - 5.37 (m, 2H), 4.70 (br d, *J* = 11.6 Hz, 1H), 4.52 (br d, *J* = 13.2 Hz, 1H), 3.66 (br s, 2H), 3.58 - 3.45 (m, 2H), 3.27 (br d, *J* = 14.0 Hz, 2H), 3.20 - 3.08 (m, 2H), 3.05 - 2.90 (m, 2H), 2.85 - 2.78 (m, 1H), 2.74 (br s, 2H), 2.42 - 2.32 (m, 4H), 2.21 - 2.07 (m, 2H), 2.01 - 1.85 (m, 2H), 1.84 - 1.74 (m, 3H), 1.70 - 1.60 (m, 1H), 1.36 (s, 6H).

**Example 13: (cis)-N-($1^4$,$1^4$-difluoro-$5^1$-hydroxy-$2^6$,$5^1$-dimethyl-4-oxo-$5^2$,$5^3$-dihydro-$5^1$H-3-aza-2(2,4)-pyrimidin-1(1,3),6(1,4)-dipipe ridin-5(4,5)-indenocyclononane-8-en-$5^7$-yl)-2-hydroxyethane-1-sulfonamide**

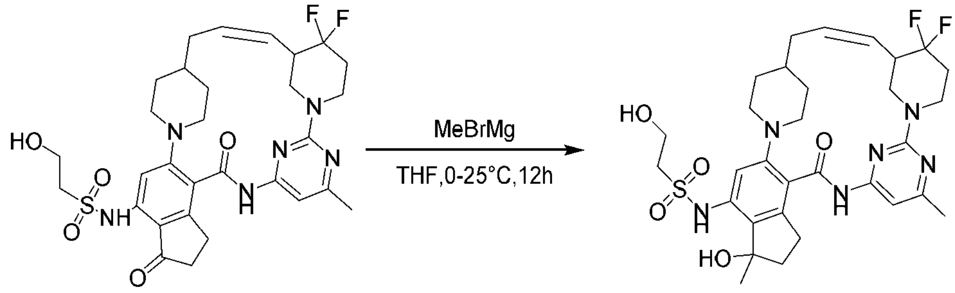


A solution of (cis)-N-($1^4$,$1^4$-difluoro-$2^6$-methyl-$5^1$,4-dioxo-$5^2$,$5^3$-dihydro-$5^1$H-3-aza-2(2,4)-pyrimidin-1(1,3),6(1,4)-dipiperidin-5(4,5)-inde nocyclonofin-8-en-$5^7$-yl)-2-hydroxyethane-1-sulfonamide (300 mg, 0.48 mmol) in tetrahydrofuran (12.0 mL) was cooled to 0°C, a mixture of methylmagnesium bromide (0.79 mL, 2.38 mmol) and tetrahydrofuran (4.00 mL) was slowly added dropwise, and the reaction mixture was naturally heated to 25°C under nitrogen atmosphere and stirred for 2 hours. The reaction was quenched with ice water (10 mL) and the mixture was extracted with ethyl acetate (10 × 3 mL). The combined organic layer was dried, filtered and concentrated under reduced pressure to give a residue which was diluted with N,N-dimethylformamide (10 mL). The diluted solution was purified by preparative HPLC (C18, 42-82% gradient of water (formic acid)/acetonitrile) to give (cis)-N-($1^4$,$1^4$-difluoro-$5^1$-hydroxy-$2^6$,$5^1$-dimethyl-4-oxo-$5^2$,$5^3$-dihydro-$5^1$hydro-3-aza-2(2,4)-pyrimidin-1(1,3),6(1,4)-dipiper idin-5(4,5)-indenocyclononane-8-en-$5^7$-yl)-2-hydroxyethane-1-sulfonamide (70 mg, 0.11 mmol, yield: 23%) as a white solid. LCMS (ESI): $[M+H]^+$ = 647.3. $^1$H NMR (400 MHz, DMSO-*d6*) δ ppm 12.89 - 12.33 (m, 1H), 8.95 - 8.80 (m, 1H), 7.36 - 7.19 (m, 2H), 6.23 - 6.08 (m, 1H), 5.76 (dt, *J* = 5.2, 11.2 Hz, 1H), 5.49 (br t, *J* = 10.0 Hz, 1H), 5.21 - 5.02 (m, 1H), 4.83 - 4.57 (m, 1H), 4.53 - 4.36 (m, 1H), 3.86 - 3.72 (m, 2H), 3.63 - 3.46 (m, 3H), 3.27 - 2.89 (m, 6H), 2.87 - 2.65 (m, 3H), 2.31 (d, *J* = 2.0 Hz, 4H), 2.17 - 2.02 (m, 4H), 1.98 - 1.64 (m, 5H), 1.60 - 1.48 (m, 1H), 1.47 - 1.25 (m, 3H)

**Example 14: N-($1^4$,$1^4$-difluoro-$2^6$-methyl-$5^3$,4-dioxo-$5^2$,5-dihydro-3-aza-2(2,4)-pyrimidin-1(1,3),6(1,4)-dipiperidin-5(7,6)-benzofura ncyclononane-8-en-$5^4$-yl)-2-hydroxyethane-1-sulfonamide**

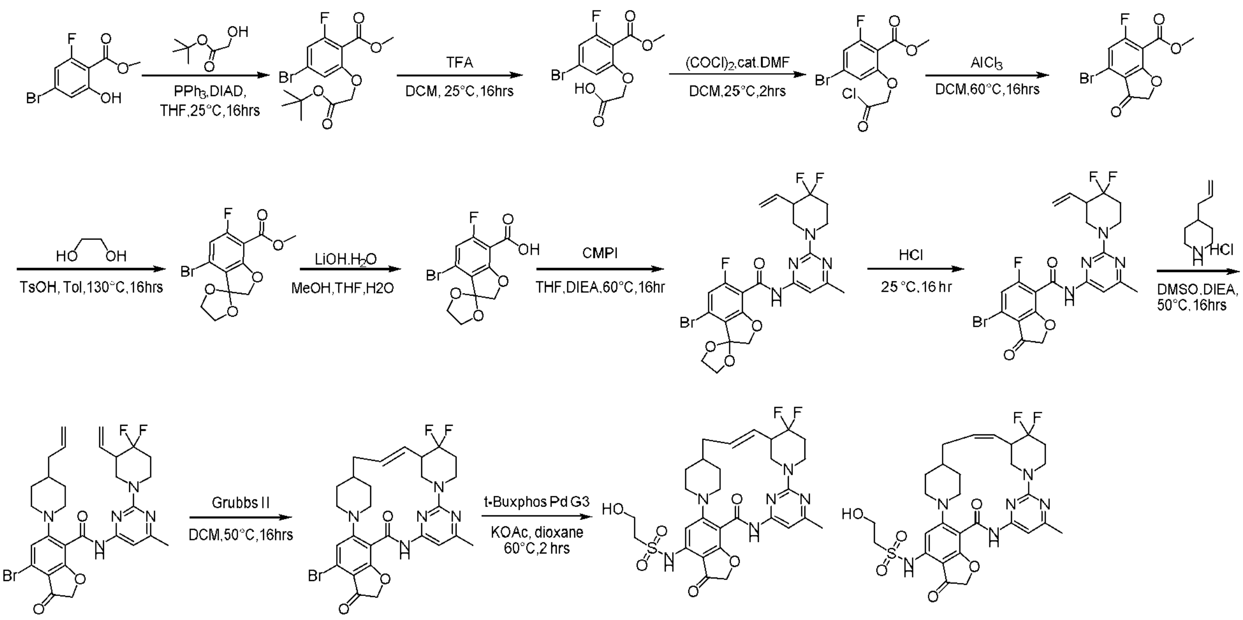


**Step 1:** Methyl 4-bromo-2-fluoro-6-hydroxybenzoate (9.50 g, 38.1 mmol) and tert-butyl 2-hydroxyacetate (6.04 g, 45.7 mmol) were dissolved in tetrahydrofuran (200 mL), then triphenylphosphine (20.0 g, 76.3 mmol) and diisopropyl

azodicarboxylate (11.6 g, 57.2 mmol) were added and the mixture was stirred at 25°C for 16 hours under nitrogen atmosphere. The reaction mixture was diluted with water (200.0 mL), extracted with ethyl acetate (200 mL × 3), and the organic phase was dried, filtered and concentrated. The residue was purified by flash column chromatography (silica gel, 0-9% gradient of tetrahydrofuran/petroleum ether) to give methyl 4-bromo-2-(2-(tert-butoxy)-2-oxoethoxy)-6-fluorobenzoate (13.0 g, 35.8 mmol, yield: 93.8%) as a yellow solid. $^1$H NMR (400 MHz, DMSO-$d_6$) δ ppm 7.32 (dd, *J* =1.2, 8.8 Hz, 1H), 7.14 (s, 1H), 4.85 (s, 2H), 3.84 (s, 3H), 1.42 (s, 9H)

**Step 2:** Methyl 4-bromo-2-(2-(tert-butoxy)-2-oxoethoxy)-6-fluorobenzoate (13.0 g, 38.1 mmol) was dissolved in dichloromethane (130 mL), then trifluoroacetic acid (30 mL) was added and the mixture was stirred at 25°C for 16 hours. The reaction solution was concentrated under reduced pressure to give 2-[5-bromo-3-fluoro-2-(methoxycarbonyl)phenoxy]acetic acid (10.9 g, 35.7 mmol, crude) as a yellow oil, which was directly used in the next step. LCMS (ESI): [M+H]$^+$ = 308.7

**Step 3:** 2-[5-bromo-3-fluoro-2-(methoxycarbonyl)phenoxy]acetic acid (10.9 g, 35.5 mmol) was dissolved in dichloromethane (130 mL). To the reaction solution were added oxalyl chloride (16.4 g, 127 mmol) and one drop of N,N-dimethylformamide. The solution was stirred at 25°C for 2 hours. The reaction solution was concentrated under reduced pressure to give methyl 4-bromo-2-(2-chloro-2-oxoethoxy)-6-fluorobenzoate (11.5 g, 35.5 mmol, crude) as a yellow oil, which was directly used in the next step. LCMS (ESI): [M+H]$^+$ = 322.8 (sample submitted in methanol)

**Step 4:** Aluminum chloride (16.4 g, 127 mmol) was dissolved in dichloromethane (110 mL). To the reaction mixture was added a solution of methyl 4-bromo-2-(2-chloro-2-oxoethoxy)-6-fluorobenzoate (16.4 g, 127 mmol) in dichloromethane (200 mL). The temperature was raised to 60°C and the mixture was stirred for 16 hours. The reaction mixture was diluted with water (200 mL), extracted with ethyl acetate (200 mL × 3), and the organic phase was dried, filtered and concentrated. The residue was purified by flash column chromatography (silica gel, 0-30% gradient of tetrahydrofuran/petroleum ether) to give methyl 4-bromo-6-fluoro-3-oxo-2,3-dihydro-1-benzofuran-7-carboxylate (5.50 g, 35.5 mmol, yield: 56.8%) as a yellow solid. LCMS (ESI): [M+H]$^+$ = 288.7

**Step 5:** To a solution of methyl 4-bromo-6-fluoro-3-oxo-2,3-dihydro-1-benzofuran-7-carboxylate (3.00 g, 10.4 mmol) and ethylene glycol (6.57 g, 104 mmol) in toluene (200 mL) was added p-toluenesulfonic acid (201 mg, 1.03 mmol). The temperature was raised to 130°C, and the mixture was stirred for 16 hours while water was removed using a water separator. The reaction was quenched with a saturated sodium bicarbonate solution (10 mL) and the mixture was extracted with ethyl acetate (200 mL × 3). The organic layer was dried, filtered and concentrated. The residue was purified by flash column chromatography (silica gel, 0-20% gradient of tetrahydrofuran/petroleum ether) to give methyl 4-bromo-6-fluoro-2H-spiro[1-benzofuro-3,2'-[1,3]dioxolane]-7-carboxylate (0.70 g, 2.10 mmol, yield: 20.2%) as a yellow solid. LCMS (ESI): [M+H]$^+$ = 334.8

**Step 6:** To a solution of methyl 4-bromo-6-fluoro-2H-spiro[1-benzofuro-3,2'-[1,3]dioxolane]-7-carboxylate (700 mg, 2.10 mmol) in methanol (7 mL) and water (7 mL) was added lithium hydroxide (133 mg, 3.15 mmol), and the mixture was stirred at 25°C for 16 hours. The reaction solution was adjusted to pH 3 with 1M hydrochloric acid, and extracted with ethyl acetate (10 mL × 3). The organic layer was dried, filtered and concentrated to give 4-bromo-6-fluoro-2H-spiro[1-benzofuro-3,2'-[1,3]dioxolane]-7-carboxylic acid (600 mg, 1.87 mmol, yield: 89.4%) as a yellow solid. LCMS (ESI): [M+H]$^+$ = 334.8. $^1$H NMR (400 MHz, DMSO-$d_6$) δ ppm 13.57 (br s, 1H), 7.26 (d, *J* = 10.0 Hz, 1H), 4.57 - 4.44 (m, 2H), 4.32 - 4.19 (m, 2H), 4.13 - 3.97 (m, 3H)

**Step 7:** To a solution of 4-bromo-6-fluoro-2H-spiro[1-benzofuro-3,2'-[1,3]dioxolane]-7-carboxylic acid (600 mg, 1.88 mmol) and 2-(4,4-difluoro-3-vinylpiperidin-1-yl)-6-methylpyrimidin-4-amine (478 mg, 1.88 mmol) in tetrahydrofuran (12 mL) were added N,N-diisopropylethylamine (1214 mg, 9.40 mmol) and 2-chloro-1-methylpyridinium iodide (1430 mg, 3.76 mmol). The temperature was raised to 60°C and the mixture was stirred for 16 hours. The reaction mixture was diluted with water (10.0 mL), extracted with ethyl acetate (10.0 mL × 2), and the organic phase was dried, filtered and concentrated. The residue was purified by flash column chromatography (silica gel, 0-15% gradient of tetrahydrofuran/petroleum ether) to give 4-bromo-N-(2-(4,4-difluoro-3-vinylpiperidin-1-yl)-6-methylpyrimidin-4-yl)-6-fluoro-2H-spiro[benzofuro-3,2'-[1,3]dioxolan e]-7-carboxamide (600 mg, 1.08 mmol, yield: 57.4%) as a yellow solid. LCMS (ESI): [M+H]$^+$ = 556.7

**Step 8:** A solution of 4-bromo-N-(2-(4,4-difluoro-3-vinylpiperidin-1-yl)-6-methylpyrimidin-4-yl)-6-fluoro-2H-spiro [benzofuro-3,2'-[1,3]dioxolan e]-7-carboxamide (500 mg, 1.88 mmol) in 2M hydrochloric acid (12 mL) was stirred at 25°C for 16 hours. The reaction solution was diluted with water (10.0 mL) and extracted with ethyl acetate (10.0 mL × 2). The organic layer was dried, filtered and concentrated to give 4-bromo-N-(2-(4,4-difluoro-3-vinylpiperidin-1-yl)-6-methylpyrimidin-4-yl)-6-fluoro-3-oxo-2,3-dihydrobenzofuran-7-carbo xamide (400 mg, 1.63 mmol, yield: 86.9%). LCMS (ESI): [M+H]$^+$ = 512.6

**Step 9:** To a solution of 4-bromo-N-(2-(4,4-difluoro-3-vinylpiperidin-1-yl)-6-methylpyrimidin-4-yl)-6-fluoro-3-oxo-2,3-dihydrobenzofuran-7-carbo xamide (400 mg, 0.78 mmol) and 4-allylpiperidine hydrochloride (478 mg, 1.88 mmol) in dimethyl sulfoxide (12 mL) was added N,N-diisopropylethylamine (1214 mg, 9.40 mmol). The temperature was raised to 50°C and the mixture was stirred for 16 hours. The reaction mixture was diluted with water (50.0 mL), extracted with ethyl acetate (50.0 mL × 2), and the organic phase was dried, filtered and concentrated. The residue was purified by

flash column chromatography (silica gel, 0-12% gradient of tetrahydrofuran/petroleum ether) to give 6-(4-allylpiperidin-1-yl)-4-bromo-N-(2-(4,4-difluoro-3-vinylpiperidin-1-yl)-6-methylpyrimidin-4-yl)-3-oxo-2,3-dihydroben zofuran-7-carboxamide (390 mg, crude) as a yellow solid. LCMS (ESI): $[M+H]^+$ = 618.1.

**Step 10:** 6-(4-allylpiperidin-1-yl)-4-bromo-N-(2-(4,4-difluoro-3-vinylpiperidin-1-yl)-6-methylpyrimidin-4-yl)-3-oxo-2,3-dihydroben zofuran-7-carboxamide (380 mg, 0.62 mmol) was dissolved in dichloromethane (190 mL), Grubbs catalyst 2nd generation (52.3 mg, 0.06 mmol) was added and the mixture was stirred at 50°C for 16 hours under nitrogen atmosphere. The reaction solution was concentrated under reduced pressure, and the residue was purified by flash column chromatography (silica gel, 0-8% gradient of tetrahydrofuran/petroleum ether) to give $5^4$-bromo-$1^4$,$1^4$-difluoro-$2^6$-methyl-$5^2$,5-dihydro-3-aza-2(2,4)-pyrimidin-1(1,3),6(1,4)-dipiperidin-5(7,6)-benzofurocyclonon ane-8-en-$5^3$,4-dione (300 mg, 0.51 mmol, yield: 83.0%) as a gray solid. LCMS (ESI): $[M+H]^+$ = 590.0

**Step 11:** $5^4$-bromo-$1^4$,$1^4$-difluoro-$2^6$-methyl-$5^2$5-dihydro-3-aza-2(2,4)-pyrimidin-1(1,3),6(1,4)-dipiperidin-5(7,6)-benzofurocyclonon ane-8-en-$5^3$,4-dione (290 mg, 0.49 mmol) and 2-hydroxyethane-1-sulfonamide (185 mg, 1.48 mmol) were dissolved in 1,4-dioxane (10.0 mL), potassium acetate (241 mg, 2.46 mmol) and t-BuXphos Pd G3 (117 mg, 0.15 mmol) were added and the mixture was stirred at 50°C for 2 hours under nitrogen atmosphere. The reaction mixture was diluted with water (50.0 mL), extracted with ethyl acetate (50.0 mL × 3), and the organic phase was dried, filtered and concentrated. The crude product was purified by preparative HPLC (C18, 24-64% gradient of water (formic acid)/acetonitrile) to give **Example 14A** (33.57 mg, yield: 11.0%) and **Example 14B** (15.19 mg, yield: 5.00%) as white solids

**Example 14A:** LCMS (ESI): $[M+H]^+$ = 633.7. $^1$H NMR (400 MHz, DMSO-$d_6$) δ ppm 10.24 (br s, 1H), 9.04 (s, 1H), 7.42 (s, 1H), 6.63 (s, 1H), 5.82 - 5.70 (m, 1H), 5.33 (dd, *J* = 8.4, 15.2 Hz, 1H), 4.96 (br d, *J* = 13.2 Hz, 2H), 4.90 - 4.78 (m, 2H), 3.82 - 3.78 (m, 2H), 3.60 - 3.55 (m, 2H), 3.53 - 3.49 (m, 1H), 3.31 (br d, *J* = 13.2 Hz, 1H), 3.19 (br d, *J* = 12.0 Hz, 1H), 3.00 (br t, *J* = 12.0 Hz, 1H), 2.94 - 2.80 (m, 3H), 2.38 - 2.32 (m, 3H), 2.28 - 2.20 (m, 1H), 2.18 - 1.98 (m, 2H), 1.89 (br dd, *J* = 9.2, 12.8 Hz, 1H), 1.72 - 1.55 (m, 3H), 1.51 - 1.37 (m, 1H), 1.13 (br d, *J* = 12.4 Hz, 1H)

**Example 14B:** LCMS (ESI): $[M+H]^+$ = 633.7. $^1$H NMR (400 MHz, DMSO-$d_6$) δ ppm 10.52 (br s, 1H), 9.09 (s, 1H), 7.19 (s, 1H), 6.69 (s, 1H), 5.73 (dt, *J* = 5.2, 11.2 Hz, 1H), 5.43 (t, *J* = 10.4 Hz, 1H), 4.93 - 4.80 (m, 2H), 4.64 - 4.54 (m, 1H), 4.45 (br d, *J* = 12.8 Hz, 1H), 3.80 (t, *J* = 6.0 Hz, 2H), 3.61 - 3.54 (m, 2H), 3.48 (br d, *J* = 11.2 Hz, 1H), 3.39 - 3.25 (m, 2H), 3.16 (br t, *J* = 12.0 Hz, 1H), 3.04 - 2.81 (m, 3H), 2.38 (s, 3H), 2.26 - 2.18 (m, 1H), 2.16 - 2.02 (m, 2H), 2.00 - 1.78 (m, 2H), 1.78 - 1.68 (m, 2H), 1.66 - 1.55 (m, 1H), 1.54 - 1.42 (m, 1H)

**Example 15: 2-Hydroxy-N-($1^4$,$1^4$,$5^1$,$5^1$-tetrafluoro-$2^1$-methyl4-dioxo-$5^2$,$5^1$-dihydro-$5^1$H-3-aza-2(2,4)-pyrimidin-1(1,3),6(1,4)-dipipe ridin-5(4,5)-benzofurancyclononane-8-en-$5^7$-yl)-ethane-1-sulfonamide**

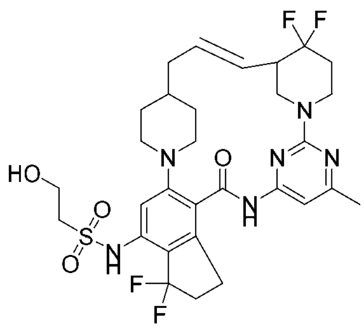

The preparations in **Example 15 followed** the synthetic route of **Example 2.** LCMS (ESI): $[M+H]^+$ =653.2

**Example 16: *N*-($6^4$,$6^4$-difluoro-$5^6$-methyl-$2^1$,3-dioxo-$2^2$,$2^3$-dihydro-$2^1$H-4-aza-2(5,4)-isoindole-5(4,2)-pyrimidin-1(1,4),6(1,3)-dipiper idineheterocyclonon-7-en-$2^7$-yl)-2-hydroxyethane-1-sulfonamide**

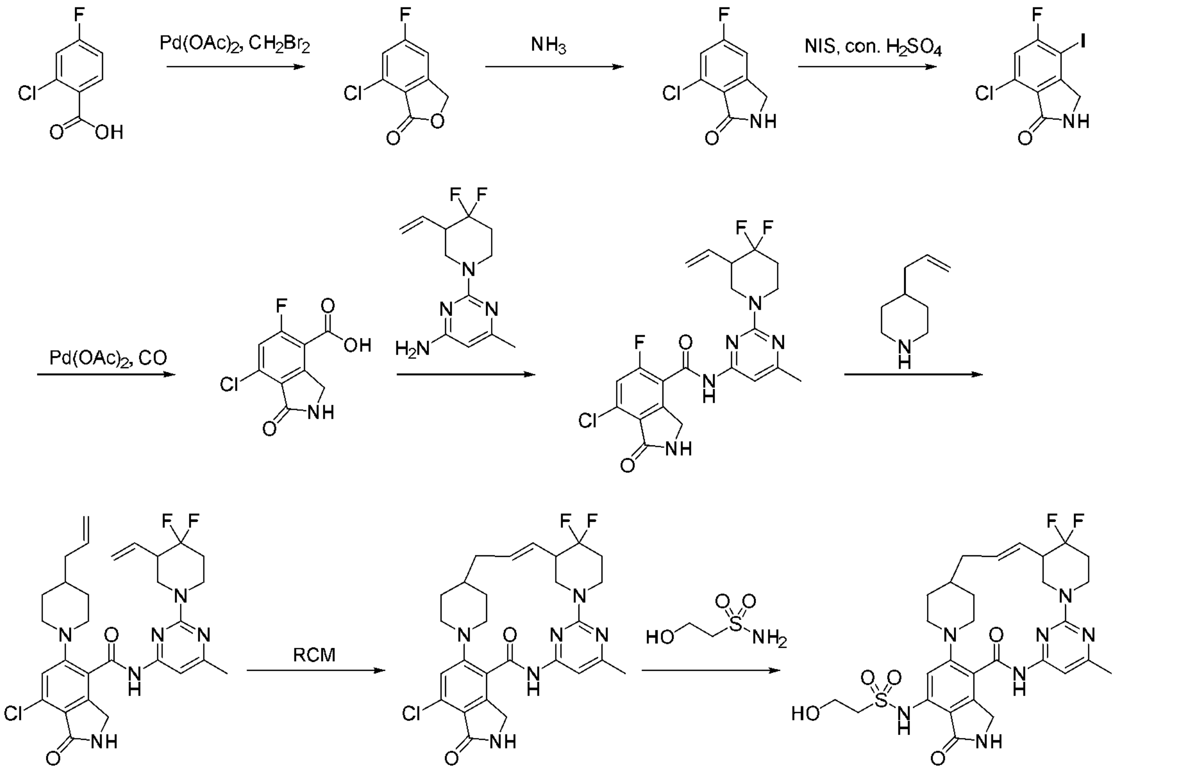


**Step 1:** Under nitrogen atmosphere, a mixture of 2-chloro-4-fluorobenzoic acid (25.00 g, 143.22 mmol), palladium (II) acetate (3.22 g, 14.32 mmol), potassium phosphate dibasic (58.47 g, 429.66 mmol) and dibromomethane (100.00 mL) was stirred at 140°C for 36 hours in a Teflon-sealed reaction tube and filtered through celite. The filtrate was concentrated and purified by column chromatography (silica gel, 0-30% gradient of ethyl acetate/petroleum ether) to give 4-chloro-5-fluoroisobenzofuran-1(3H)-one (20.10 g, 107.74 mmol, yield: 75.22%) as a yellow solid. LCMS (ESI): $[M+H]^+$ = 187.10.

**Step 2:** A solution of 4-chloro-5-fluoroisobenzofuran-1(3H)-one (20.10 g, 107.74 mmol) in methanol (500.00 mL) was purged with ammonia for 30 minutes, and the mixture was heated to 120°C in an autoclave to react for 7 hours. The reaction mixture was concentrated and purified by column chromatography (silica gel, 0-30% gradient of ethyl acetate/petroleum ether) to give 7-chloro-5-fluoroisoindolin-1-one (12.40 g, 107.20 mmol, yield: 62.3%) as a yellow solid. LCMS (ESI): $[M+H]^+$ = 186.00.

**Step 3:** At 0°C, 7-chloro-5-fluoroisoindolin-1-one (12.40 g, 107.20 mmol) was dissolved in concentrated sulfuric acid (100.00 mL), then *N*-iodosuccinimide (26.53 g, 117.92 mmol) was added portionwise and the reaction mixture was stirred at 0°C for 4 hours. The reaction solution was heated to room temperature and poured into ice water (1.2 L), and 1 N sodium thiosulfate (400 mL) was added. The mixture was extracted with ethyl acetate (100 mL × 3), washed with a saturated sodium chloride solution, dried over anhydrous sodium sulfate, filtered, and concentrated under reduced pressure to give a crude product. The product was purified by flash column chromatography (silica, 20-50% ethyl acetate/petroleum ether) to give 7-chloro-5-fluoro-4-iodoisoindolin-1-one (17.20 g, 55.22 mmol, yield: 82.6%) as a yellow solid. LCMS (ESI): $[M+H]^+$ = 312.00.

**Step 4:** At 20°C, 7-chloro-5-fluoro-4-iodoisoindolin-1-one (17.20 g, 55.22 mmol), palladium acetate (620 mg, 2.76 mmol) and potassium carbonate (22.88 g, 165.66 mmol) were dissolved in water (55.22 mL) and polyethylene glycol 400 (220.88 mL). The mixture was reacted for 2 hours under carbon monoxide atmosphere (15 psi). After the reaction was complete, the reaction was quenched with sodium chloride solution (1 L) and 3M hydrochloric acid was added to adjust the pH to 5. The mixture was extracted with ethyl acetate (200 mL × 3), washed with a saturated sodium chloride solution, dried over anhydrous sodium sulfate, filtered, and concentrated under reduced pressure to give a crude product. The product was purified by flash column chromatography (silica, 50-100% ethyl acetate/petroleum ether) to give 7-chloro-5-fluoro-1-oxoisoindoline-4-carboxylic acid (7.60 g, 33.10 mmol, yield: 59.9%) as a yellow solid. LCMS (ESI): $[M+H]^+$ = 230.00.

**Step 5:** At 20°C, 2-(4,4-difluoro-3-vinylpiperidin-1-yl)-6-methylpyrimidin-4-amine (2.22 g, 8.71 mmol), 7-chloro-5-fluoro-1-oxoisoindoline-4-carboxylic acid (2.00 g, 8.71 mmol), N,N,N',N'-tetramethyl-O-(7-azabenzotriazol-1-yl)uronium hexafluorophosphate (9.94 g, 26.13 mmol) and 4-dimethylaminopyridine (4.26 g, 34.84 mmol) were dissolved in dichloroethane (100.00 mL). The mixture was heated to 80°C and stirred for 24 hours. After the reaction was

complete, the reaction was quenched with a sodium bicarbonate solution (50 mL) and the mixture was extracted with dichloromethane (50 mL × 3). The combined organic layer was dried, filtered and concentrated to give a crude product, which was purified by flash column chromatography (silica, 0-20% ethyl acetate/petroleum ether) to give 7-chloro-*N*-(2-(4,4-difluoro-3-vinylpiperidin-1-yl)-6-methylpyrimidin-4-yl)-5-fluoro-1-oxoisoindoline-4-carboxamide (2.01 g, 4.31 mmol, yield: 49.5%) as a white solid. LCMS (ESI): $[M+H]^+$ = 466.20.

**Step 6:** A solution of 7-chloro-*N*-(2-(4,4-difluoro-3-vinylpiperidin-1-yl)-6-methylpyrimidin-4-yl)-5-fluoro-1-oxoisoindoline-4-carboxamide (2.01 g, 4.31 mmol), 4-allylpiperidine hydrochloride (756 mg, 6.04 mmol) and N,N-diisopropylethylamine (1.67 g, 12.93 mmol) in dimethyl sulfoxide (30.00 mL) was stirred at 60°C for 10 hours to complete the reaction. The mixture was cooled naturally, the reaction was quenched cold water (100 mL) and the mixture was extracted with ethyl acetate (200 mL × 3). The combined organic layer was washed with a saturated sodium chloride solution (100 mL), dried, filtered and concentrated to give 5-(4-allylpiperidin-1-yl)-7-chloro-*N*-(2-(4,4-difluoro-3-vinylpiperidin-1-yl)-6-methylpyrimidin-4-yl)-1-oxoisoindoline-4-car boxamide (2.10 g, 3.68 mmol, yield: 85.2%, crude) as a white solid. LCMS (ESI): $[M+H]^+$ = 571.40.

**Step 7:** Under nitrogen atmosphere, 5-(4-allylpiperidin-1-yl)-7-chloro-*N*-(2-(4,4-difluoro-3-vinylpiperidin-1-yl)-6-methylpyrimidin-4-yl)-1-oxoisoindoline-4-car boxamide (2.10 g, 3.68 mmol) was dissolved in dry dichloroethane (120.00 mL), Grubbs catalyst 2nd generation (156 mg, 0.18 mmol) was added and the reaction was heated to reflux for 16 hours. After the reaction was complete, the reaction solution was concentrated and purified by flash column chromatography (silica, 0-20% ethyl acetate/petroleum ether) to give $2^7$-chloro-$6^4$,$6^4$-difluoro-$5^6$-methyl-$2^2$,$2^3$-dihydro-$2^1$H-4-aza-2(5,4)-isoindole-5(4,2)-pyrimidin-1(1,4),6(1,3)-dipiperidinehete rocyclonon-7-en-$2^1$,3-dione (1.62 g, 2.98 mmol, yield: 81.1%) as a white solid. LCMS (ESI): $[M+H]^+$ = 543.20.

**Step 8:** Under nitrogen atmosphere, $2^7$-chloro-$6^4$,$6^4$-difluoro-$5^6$-methyl-$2^2$,$2^3$-dihydro-$2^1$H-4-aza-2(5,4)-isoindole-5(4,2)-pyrimidin-1(1,4),6(1,3)-dipiperidinehete rocyclonon-7-en-$2^1$,3-dione (1.62 g, 2.98 mmol), 2-hydroxyethylsulfonamide (1.13 g, 9.00 mmol), potassium acetate (1.18 g, 12.00 mmol) and methanesulfonic acid(2-di-tert-butylphosphino-2',4',6'-triisopropyl-1,1'-biphenyl)(2'-amino-1,1'-biphenyl-2-yl)palladium(II) (238 mg, 0.30 mmol) were dissolved in 1,4-dioxane (30.00 mL). The resulting reaction mixture was stirred at 50°C for 6 hours. After the reaction was complete, the reaction was quenched with a sodium carbonate solution (50 mL) and the mixture was extracted with ethyl acetate (50 mL × 3). The combined organic layer was washed with brine (100 mL), dried over magnesium sulfate, filtered and concentrated to give a crude product. The product was purified by flash column chromatography (silica, 70-90% ethyl acetate/petroleum ether) to give *N*-($6^4$,$6^4$-difluoro-$5^6$-methyl-$2^1$,3-dioxo-$2^2$,$2^3$-dihydro-$2^1$H-4-aza-2(5,4)-isoindole-5(4,2)-pyrimidin-1(1,4),6(1,3)-dipiperidin eheterocyclonon-7-en-$2^7$-yl)-2-hydroxyethane-1-sulfonamide (503 mg, 0.80 mmol, yield: 26.7%) as a white solid. LCMS (ESI): $[M+H]^+$ = 632.20.

**Example 17: N-($6^4$,$6^4$-difluoro-$5^6$-methyl-$2^1$,3-dioxo-$2^1$,$2^2$,$2^3$,$2^4$-tetrahydro-4-aza-2(6,5)-isoquinoline-5(4,2)-pyrimidin-1(1,4),6(1,3)-dipiperidinylcyclononane-7-en-$2^8$-yl)-2-hydroxyethane-1-sulfonamide**

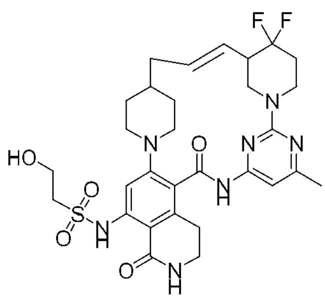

The preparations in **Example 17 followed** the synthetic route of **Example 16.** LCMS (ESI): $[M+H]^+$ = 646.3

**Example 18: N-($1^4$,$1^4$-difluoro-$2^6$-methyl-$5^1$,4-dioxo-$5^2$,$5^3$-dihydro-$5^1$*H*-3-aza-2(2,4)-pyrimidin-1(1,3),6(1,4)-dipiperidin-5(4,5)-inde noheterocyclonon-8-en-$5^7$-yl)-ethanesulfonamide**

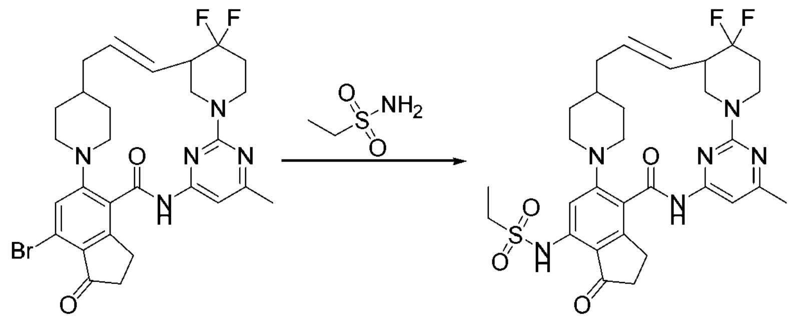

The preparations in **Example 18 followed** the synthetic route of **Example 2.**

**Example 18A (trans isomer)** LCMS (ESI): $[M+H]^+$ = 615.3. $^1H$ NMR (400 MHz, DMSO-$d_6$) δ ppm 10.05 (s, 1H), 9.90 (s, 1H), 7.45 (s, 1H), 6.96 (s, 1H), 5.90 - 5.66 (m, 1H), 5.34 (dd, $J$ = 8.8, 15.2 Hz, 1H), 5.13 - 4.82 (m, 2H), 3.52 - 3.44 (m, 2H), 3.25 - 3.12 (m, 2H), 3.02 - 2.76 (m, 5H), 2.72 - 2.64 (m, 2H), 2.33 (s, 3H), 2.29 - 2.21 (m, 1H), 2.19 - 1.97 (m, 2H), 1.93 - 1.84 (m, 1H), 1.75 - 1.46 (m, 4H), 1.30 - 1.11 (m, 6H).

**Example 18B (cis isomer)** LCMS (ESI): $[M+H]^+$ = 615.3. $^1H$ NMR (400 MHz, DMSO-$d_6$) δ ppm 11.19 (s, 1H), 9.99 (s, 1H), 7.31 (s, 1H), 7.12 (s, 1H), 5.76 (dt, $J$ = 5.6, 11.2 Hz, 1H), 5.46 (t, $J$ = 10.4 Hz, 1H), 4.69 - 4.56 (m, 1H), 4.49 - 4.39 (m, 1H), 3.51 - 3.46 (m, 2H), 3.28 - 3.18 (m, 2H), 3.16 - 3.05 (m, 2H), 3.01 - 2.80 (m, 3H), 2.76 - 2.64 (m, 2H), 2.41 - 2.35 (m, 3H), 2.34 - 2.25 (m, 1H), 2.18 - 2.03 (m, 2H), 2.00 - 1.65 (m, 5H), 1.62 - 1.51 (m, 1H), 1.29 - 1.12 (m, 5H).

**Example 19: *N*-($1^4$,$1^4$-difluoro-$2^6$-methyl-$5^1$,4-dioxo-$5^2$,$5^3$-dihydro-$5^1$*H*-3-aza-2(2,4)-pyrimidin-1(1,3),6(1,4)-dipiperidin-5(4,5)-inde noheterocyclonon-8-en-$5^7$-yl)-methanesulfonamide**

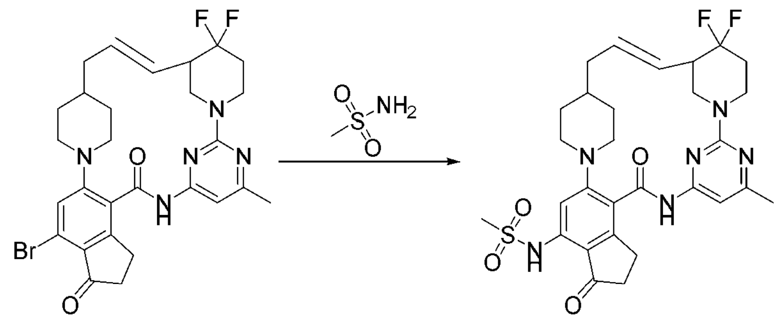

The preparations in **Example 19 followed** the synthetic route of **Example 2.** LCMS (ESI): $[M+H]^+$ = 601.3

Example 20: **N-($1^4$,$1^4$,$5^1$,$5^1$-tetrafluoro-$2^6$-methyl-4-dioxo-$5^2$,$5^3$-dihydro-$5^1$H-3-aza-2(2,4)-pyrimidin-1(1,3),6(1,4)-dipiperidin-5(4,5)-benzofurancyclononane-8-en-$5^7$-yl)-ethanesulfonamide**

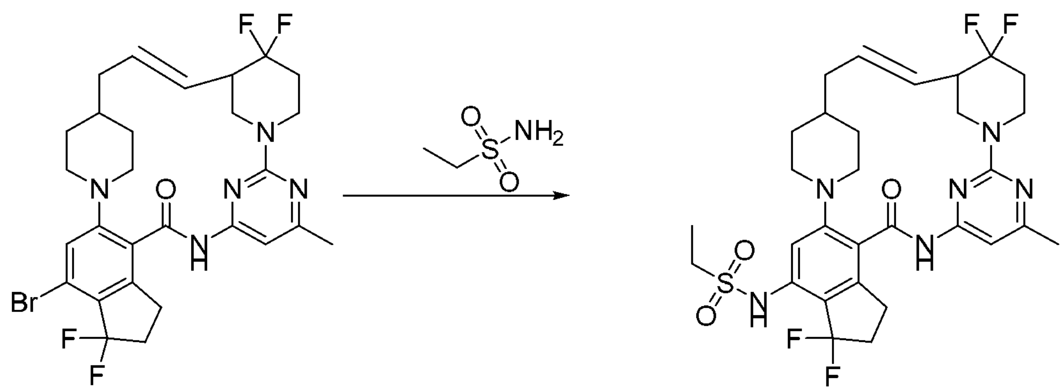

The preparations in **Example 20 followed** the synthetic route of **Example 2.** LCMS (ESI): $[M+H]^+$ = 637.3

**Example 21: 2-Hydroxy-N-($1^4$,$1^4$,$5^1$,$5^1$-tetrafluoro-$2^6$-methyl-4-dioxo-$5^2$,$5^3$-dihydro-$5^1$H-3-aza-2(2,4)-pyrimidin-1(1,3),6(1,4)-dipipe ridin-5(4,5)-benzofurancyclononane-8-en-$5^7$-yl)-methanesulfonamide**

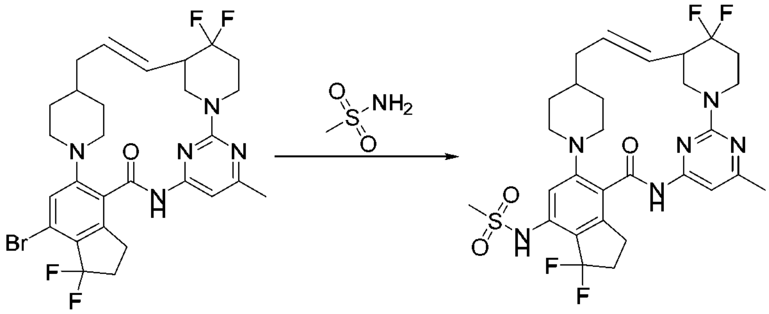

The preparations in **Example 21 followed** the synthetic route of **Example 2.** LCMS (ESI): $[M+H]^+$ = 623.3

**Example 22: N-($5^1$-(difluoromethylene)-$1^4$,$1^4$-difluoro-$2^6$-methyl-4-oxo-$5^2$,$5^3$-dihydro-$5^1$H-3-aza-2(2,4)-pyrimidin-1(1,3),6(1,4)-dipi peridin-5(4,5)-indenodiketonaphthalene-8-en-$5^7$-yl)-2-hydroxyethane-1-sulfonamide**

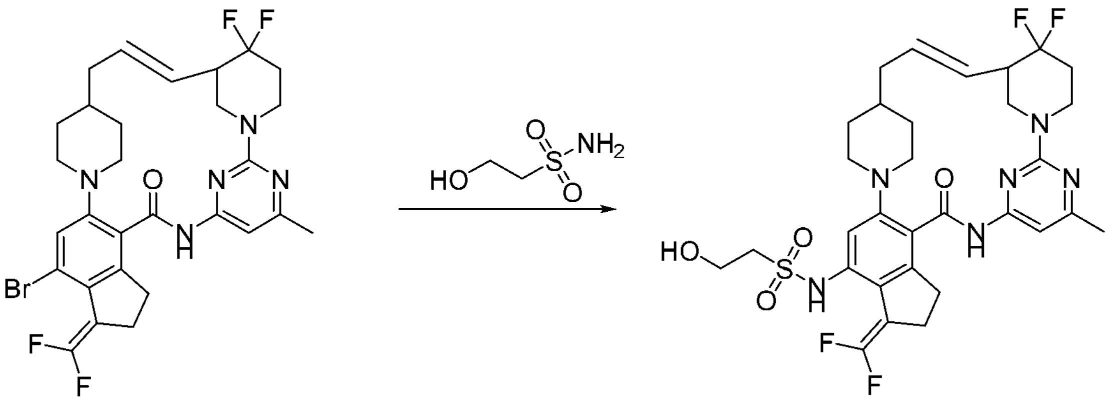

The preparations in **Example 22 followed** the synthetic route of **Example 2.** LCMS (ESI): $[M+H]^+$ = 665.3

**Example 23: N-(($5^1$,8)-$1^4$,$1^4$-difluoro-$5^1$-(fluoromethylene)-$2^6$-methyl-4-oxo-$5^2$,$5^3$-dihydro-$5^1$H-3-aza-2(2,4)-pyrimidin-1(1,3),6(1,4)-dipiperidin-5(4,5)-indenonaphthalene-8-en-$5^7$-yl)-2-hydroxyethane-1-sulfonamide**

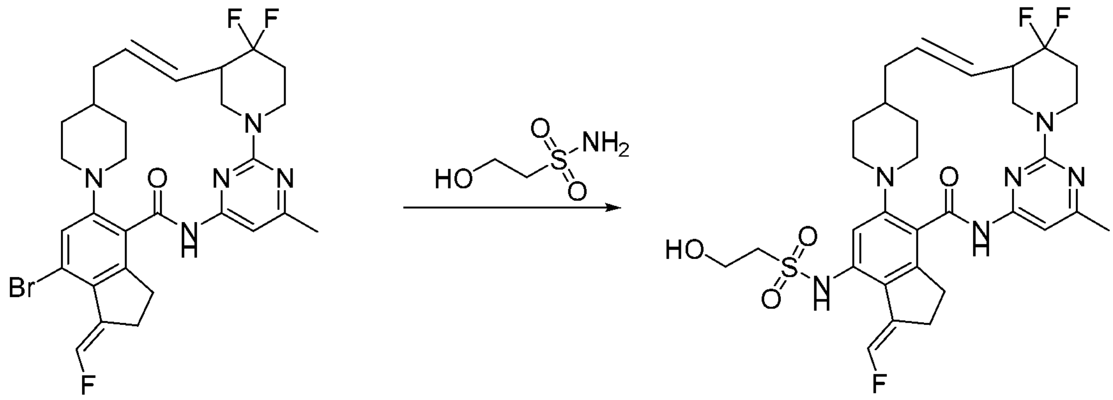

The preparations in **Example 23 followed** the synthetic route of **Example 2.** LCMS (ESI): $[M+H]^+$ = 647.3

**Example 24: N-($1^4$,$1^4$-difluoro-$2^6$-methyl-$5^1$,4-dione-$5^2$,$5^3$-dihydro-$5^1$H-3-aza-2(2,4)-pyrimidin-1(1,3),6(1,4)-dipiperidin-5(4,5)-inde nodiketone-8-en-$5^7$-yl)-1-hydroxypropane-2-sulfonamide**

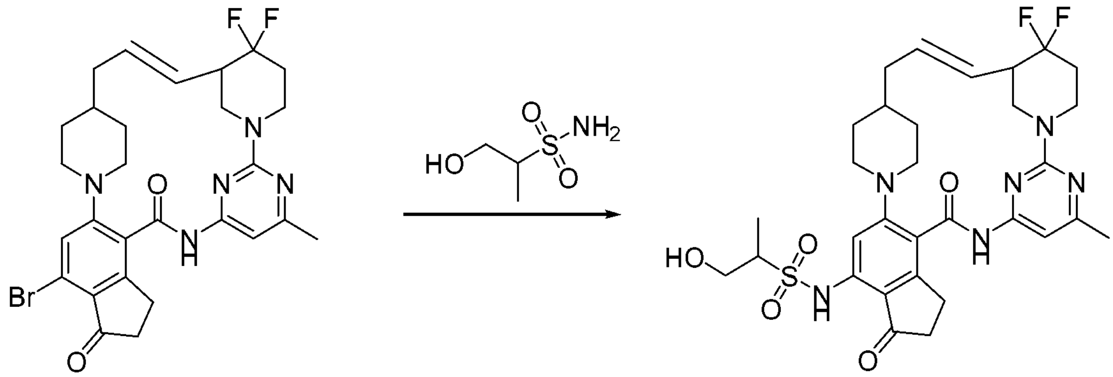

The preparations in **Example 24 followed** the synthetic route of **Example 2.** LCMS (ESI): $[M+H]^+$ = 645.3

**Example 25: *N*-($1^4$,$1^4$-difluoro-$2^6$-methyl-$5^1$,3-dioxo-$5^2$,$5^3$-dihydro-$5^1$*H*-4-aza-2(2,4)-pyrimidin-1(1,3),6(1,4)-dipiperidin-5(4,5)-inde noheterocyclonon-8-en-$5^7$-yl)-2-hydroxyethane-1-sulfonamide**

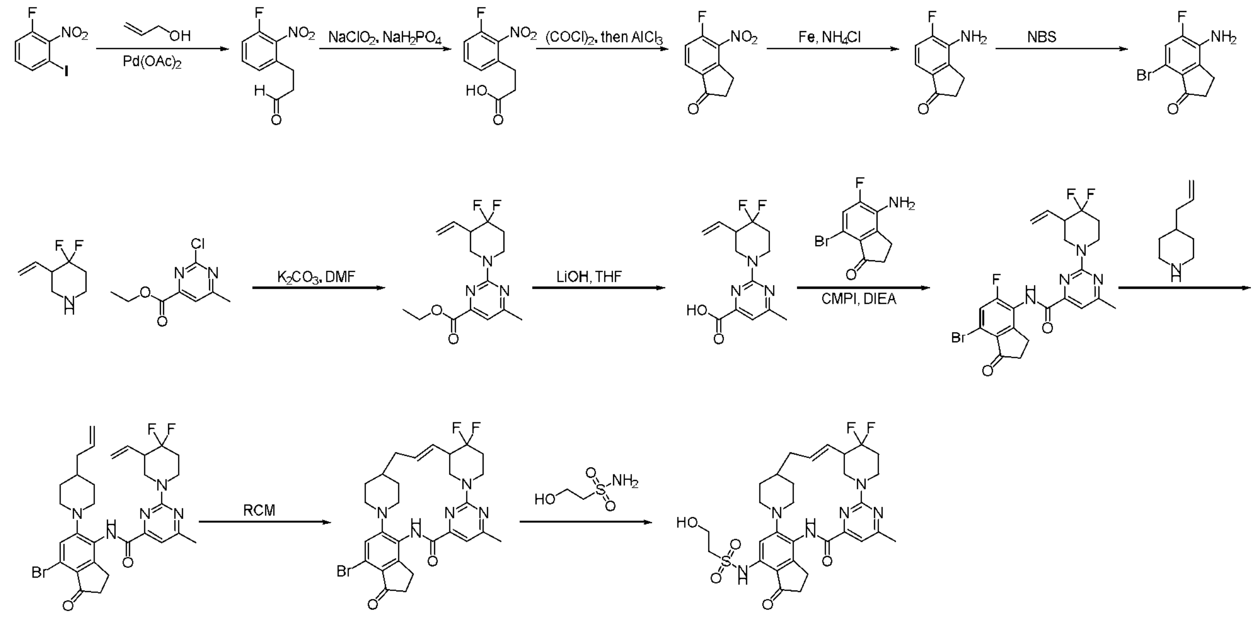


**Step 1:** Under nitrogen atmosphere, a solution of 1-fluoro-3-iodo-2-nitrobenzene (25.00 g, 93.63 mmol), prop-2-en-1-ol (8.16 g, 140.45 mmol), palladium acetate (1.05 g, 4.68 mmol), tetrabutyl ammonium chloride (26.02 g, 93.63 mmol) and sodium bicarbonate (39.32 g, 498.50 mmol) in N,N-dimethylformamide (200.00 mL) was stirred at 40°C for 8 hours. After the reaction was complete, the reaction was quenched with a sodium chloride solution (300 mL) and the mixture was extracted with ethyl acetate (250 mL × 3), washed with sodium chloride solution, dried over anhydrous sodium sulfate, filtered, concentrated, and purified by column chromatography (silica gel, 0-20% gradient of ethyl acetate/petroleum ether) to give 3-(3-fluoro-2-nitrophenyl)propanal (15.92 g, 80.74 mmol, yield: 86.2%) as a yellow solid. LCMS (ESI): $[M+H]^+$ = 198.30.

**Step 2:** At 0°C, to a solution of 3-(3-fluoro-2-nitrophenyl)propanal (15.92 g, 80.74 mmol) and 2-methyl-2-butene (100.00 mL) in tetrahydrofuran (300.00 mL) and tert-butanol (300.00 mL) was slowly added a solution of sodium chlorite (21.91 g, 242.22 mmol) and sodium phosphate monobasic (29.06 g, 242.22 mmol) in water (100.0 mL). After the reaction was complete, the reaction was quenched with a sodium sulfite solution (300 mL) and the mixture was extracted with ethyl acetate (250 mL × 3), washed with sodium chloride solution, dried over anhydrous sodium sulfate, filtered, concentrated, and purified by column chromatography (silica gel, 0-20% gradient of ethyl acetate/petroleum ether) to give 3-(3-fluoro-2-nitrophenyl)propionic acid (14.20 g, 66.62 mmol, yield: 82.5%) as a yellow solid. LCMS (ESI): $[M+H]^+$ = 214.30.

**Step 3:** To a solution of 3-(3-fluoro-2-nitrophenyl)propanoic acid (14.20 g, 66.62 mmol) in dichloromethane (120.00 mL) was added oxalyl chloride (34.04 g, 17.60 mmol). The mixture was stirred for 2 hours and concentrated under reduced pressure to remove the solvent and excess oxalyl chloride. The crude product was dissolved in dichloromethane (250.00 mL), aluminum chloride (17.77 g, 133.24 mmol) was added and the reaction mixture was stirred for 6 hours. The reaction solution was poured into ice water (500 mL) for quenching, extracted with dichloromethane (3 × 100 mL), then filtered and concentrated, and purified by column chromatography (silica gel, 0-20% gradient of ethyl acetate/petroleum ether) to give 5-fluoro-4-nitro-2,3-dihydro-1H-indene-1-one (6.20 g, 31.77 mmol, yield: 47.7%) as a yellow solid. LCMS (ESI): $[M+H]^+$ = 196.30.

**Step 4:** A mixture of 5-fluoro-4-nitro-2,3-dihydro-1H-indene-1-one (6.20 g, 31.77 mmol), ammonium chloride (17.00 g, 317.70 mmol) and iron powder (8.90 g, 158.85 mmol) in ethanol (160.00 mL) and water (40.00 mL) was stirred at 80°C for 4 hours. After the reaction was complete, the mixture was filtered and concentrated, extracted with ethyl acetate (100 mL × 3), dried over anhydrous sodium sulfate, then filtered and concentrated, and purified by column chromatography (silica gel, 0-50% gradient of ethyl acetate/petroleum ether) to give 4-amino-5-fluoro-2,3-dihydro-1H-indene-1-one (4.57 g, 27.67 mmol, yield: 87.1%) as a yellow solid. LCMS (ESI): $[M+H]^+$ = 166.30.

**Step 5:** To a solution of 4-amino-5-fluoro-2,3-dihydro-1H-indene-1-one (4.57 g, 27.67 mmol) in dichloromethane (180.00 mL) was added *N*-bromosuccinimide (9.61 g, 54.00 mmol), and the mixture was stirred for 3 hours to complete the reaction. The reaction was quenched with a sodium sulfite solution (200 mL), and the mixture was extracted with dichloromethane (150 mL × 3), dried over anhydrous sodium sulfate, filtered and concentrated, and purified by column chromatography (silica gel, 0-20% gradient of ethyl acetate/petroleum ether) to give 4-amino-7-bromo-5-fluoro-2,3-dihydro-1H-indene-1-one (4.30 g, 17.62 mmol, yield: 63.7%) as a yellow solid. LCMS (ESI): $[M+H]^+$ = 244.10.

**Step 6:** A solution of 4,4-difluoro-3-vinylpiperidine hydrochloride (4.00 g, 21.78 mmol), 2-chloro-6-methylpyrimidin-4-carboxylate (4.37 g, 21.78 mmol) and potassium carbonate (6.02 g, 43.56 mmol) in N,N-dimethylformamide

(100.00 mL) was stirred at 100°C for 16 h. After the reaction was complete, the mixture was quenched with ice water (100 mL), extracted with ethyl acetate (150 mL × 3), washed with brine, dried over anhydrous sodium sulfate, filtered, concentrated, and purified by column chromatography (silica gel, 30-50% gradient of ethyl acetate/petroleum ether) to give 2-(4,4-difluoro-3-vinylpiperidin-1-yl)-6-methylpyrimidin-4-carboxylate (4.25 g, 13.65 mmol, yield: 62.7%) as a yellow solid. LCMS (ESI): $[M+H]^+$ = 312.30.

**Step 7:** To a solution of 2-(4,4-difluoro-3-vinylpiperidin-1-yl)-6-methylpyrimidin-4-carboxylate (4.25 g, 13.65 mmol) in tetrahydrofuran (30.00 mL) and methanol (30.00 mL) was added 2 M sodium hydroxide solution (15.00 mL, 30.00 mmol) and was stirred at 50°C for 6 h. After the reaction was complete, 2 M hydrochloric acid (15 mL) was added to adjust the pH to 7. The mixture was extracted with ethyl acetate (150 mL × 3), washed with brine, dried over anhydrous sodium sulfate, filtered, concentrated, and purified by column chromatography (silica gel, 30-100% gradient of ethyl acetate/petroleum ether) to give 2-(4,4-difluoro-3-vinylpiperidin-1-yl)-6-methylpyrimidin-4-carboxylic acid (2.70 g, 9.53 mmol, yield: 69.8%) as a yellow solid. LCMS (ESI): $[M+H]^+$ = 384.30.

**Step 8:** A solution of 2-(4,4-difluoro-3-vinylpiperidin-1-yl)-6-methylpyrimidin-4-carboxylic acid (2.00 g, 7.06 mmol), 4-amino-7-bromo-5-fluoro-2,3-dihydro-1H-ind-1-one (1.72 g, 7.06 mmol), 2-chloro-1-methylpyridin iodide (3.82 g, 14.12 mmol) and N,N-diisopropylethylamine (4.83 g, 37.40 mmol) in tetrahydrofuran (100.00 mL) was stirred at 50°C for 16 h.

The mixture was quenched with sodium bicarbonate solution (100 mL), extracted with ethyl acetate (100 mL × 3), washed with sodium chloride solution, dried over anhydrous sodium sulfate, filtered, concentrated, and purified by column chromatography (silica gel, 0-30% gradient of ethyl acetate/petroleum ether) to give N-(7-bromo-5-fluoro-1-oxo-2,3-dihydro-1H-ind-4-yl)-2-(4,4-difluoro-3-vinylpiperidin-1-yl)-6-methylpyrimidin-4-carbona mide (2.53 g, 4.97 mmol, yield: 70.4%) as a yellow solid. LCMS (ESI): $[M+H]^+$ = 509.20.

**Step 9:** A solution of *N*-(7-bromo-5-fluoro-1-oxo-2,3-dihydro-1*H*-inden-4-yl)-2-(4,4-difluoro-3-vinylpiperidin-1-yl)-6-methylpyrimidin-4-carbon amide (2.53 g, 4.97 mmol), 4-allyl piperidine hydrochloride (1.21 g, 7.50 mmol) and potassium carbonate (13.83 g, 10.00 mmol) in dimethyl sulfoxide (30.00 mL) was stirred at 100°C for 16 h. The mixture was quenched with water (50 mL), extracted with ethyl acetate (100 mL × 3), washed with sodium chloride solution, dried over anhydrous sodium sulfate, filtered, concentrated, and purified by column chromatography (silica gel, 0-30% gradient of ethyl acetate/petroleum ether) to give *N*-(5-(4-allylpiperidin-1-yl)-7-bromo-1-oxo-2,3-dihydro-1*H*-ind-4-yl)-2-(4,4-difluoro-3-vinylpiperidin-1-yl)-6-methylpyrim idin-4-carbonamide (2.62 g, 4.26 mmol, yield: 85.8%) as a yellow solid. LCMS (ESI): $[M+H]^+$ = 614.30.

**Step 10:** A solution of *N*-(5-(4-allylpiperidin-1-yl)-7-bromo-1-oxo-2,3-dihydro-1*H*-inden-4-yl)-2-(4,4-difluoro-3-vinylpiperidin-1-yl)-6-methylpyri midin-4-carbonamide (2.62 g, 4.26 mmol) and Grubbs catalyst 2nd generation (366 mg, 0.43 mmol) in dichloromethane (200.00 mL) was stirred at 50°C for 16 h, and the reaction solution was concentrated and purified by column chromatography (silica gel, 0-30% gradient of ethyl acetate/petroleum ether) to give $5^7$-bromo-$1^4$,$1^4$-difluoro-$2^6$-methyl-$5^2$,$5^3$-dihydro-$5^1$*H*-4-aza-2(2,4)-pyrimidin-1(1,3),6(1,4)-dipiperidin-5(4,5)-indenoheteroc yclonon-8-en-51,3-dione (2.03 g, 3.46 mmol, yield: 81.2%) as a yellow solid. LCMS (ESI): $[M+H]^+$ = 586.30.

**Step 11:** Under nitrogen atmosphere, $5^7$-bromo-$1^4$,$1^4$-difluoro-$2^6$-methyl-$5^2$,$5^3$-dihydro-$5^1$*H*-4-aza-2(2,4)-pyrimidin-1(1,3),6(1,4)-dipiperidin-5(4,5)-indenoheteroc yclonon-8-en-$5^1$,3-dione (1.96 g, 3.33 mmol), 2-hydroxyethylsulfonamide (1.25 g, 10.00 mmol), potassium acetate (1.30 g, 13.33 mmol) and methanesulfonic acid(2-di-tert-butylphosphino-2',4',6'-triisopropyl-1,1'-biphenyl)(2'-amino-1,1'-biphenyl-2-yl)palladium(II) (265 mg, 0.33 mmol) were dissolved in 1,4-dioxane (15.00 mL). The resulting reaction mixture was stirred at 60°C for 8 hours. After the reaction was complete, the mixture was quenched with cold water (20 mL) and extracted with ethyl acetate (50 mL × 3), and the organic phases were combined, washed with sodium chloride solution (100 mL), dried over magnesium sulfate, filtered, and concentrated to give the crude product. The product was purified by flash column chromatography (silica, 60-90% ethyl acetate/petroleum ether) to give N-($1^4$,$1^4$-difluoro-$2^6$-methyl-$5^1$,3-dioxo-$5^2$,$5^3$-dihydro-$5^1$*H*-4-aza-2(2,4)-pyrimidin-1(1,3),6(1,4)-dipiperidin-5(4,5)-indenohe terocyclonon-8-en-$5^7$-yl)-2-hydroxyethane-1-sulfonamide (1.31 g, 2.08 mmol, yield: 62.2%) as a white solid. LCMS (ESI): $[M+H]^+$ = 631.30.

**Example 26: N-(($5^1$Z,8E)-$5^1$-(cyanofluoromethylidene)-$1^4$,$1^4$-difluoro-$2^6$-methyl-4-oxo-$5^2$,$5^3$-dihydro-$5^1$H-3-aza-2(2,4)-pyrimidin-1( 1,3),6(1,4)-dipiperidin-5(4,5)-indenophenanthren-8-en-$5^7$-yl)-2-hydroxyethane-1-sulfonamide**

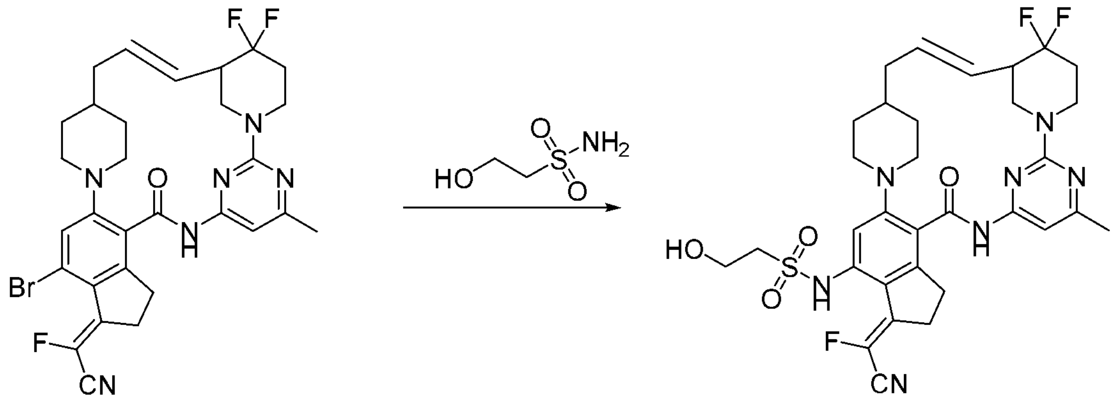

The preparations in **Example 26 followed** the synthetic route of **Example 22.** LCMS (ESI): $[M+H]^+$ = 672.3

**Example 27: N-(4,4-difluoro-6-methyl-4-oxospiro[3-aza-2(2,4)-pyrimidin-1(1,3),6(1,4)-dipiperidin-5(4,5)-indionedionenaphthalene -1,2'-[1,3]dioxolane]-8-en-7-yl)-2-hydroxyethane-1-sulfonamide**

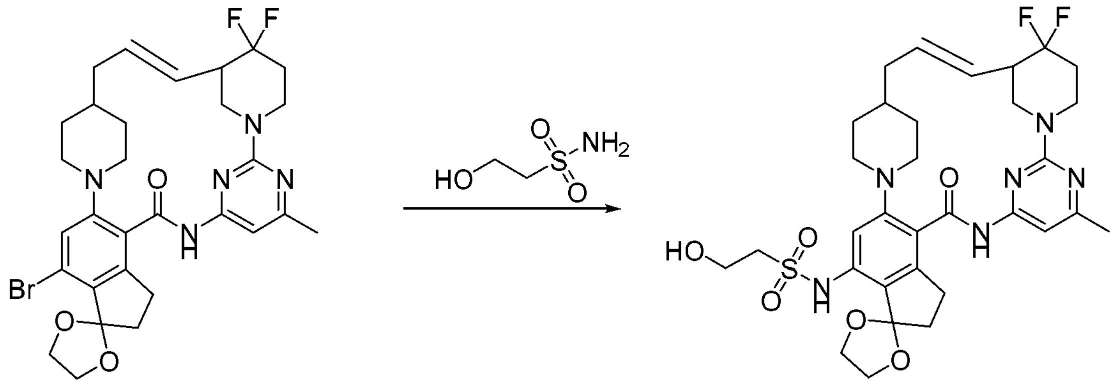

The preparations in **Example 27 followed** the synthetic route of **Example 15.** LCMS (ESI): $[M+H]^+$ = 675.3

**Example 28: N-(4,4-difluoro-6-methyl-4-oxospiro[3-aza-2(2,4)-pyrimidin-1(1,3),6(1,4)-dipiperidin-5(4,5)-indionedionenaphthalen-1,2'-[1,3]dithiolane]-8-en-7-yl)-2-hydroxyethane-1-sulfonamide**

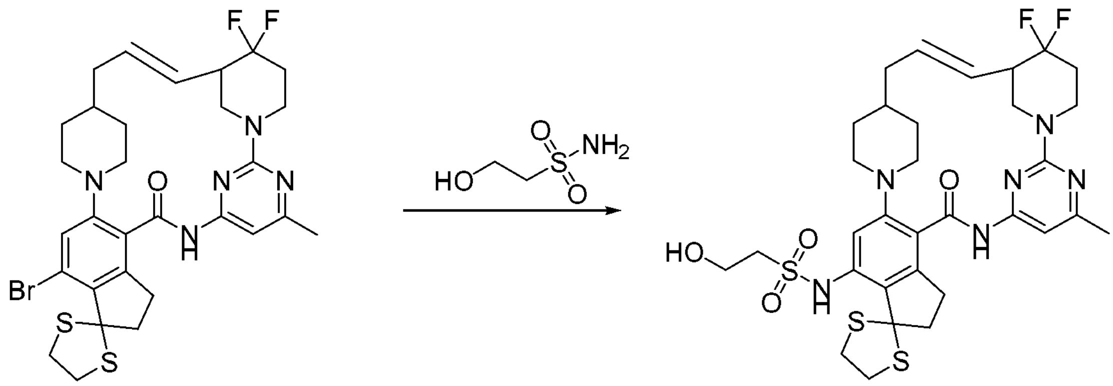

The preparations in **Example 28 followed** the synthetic route of **Example 15.** LCMS (ESI): $[M+H]^+$ = 707.3

**Example 29: (2-hydroxy-N-($1^4$,$1^4$,$5^1$,$5^1$-tetrafluoro-$2^6$-methyl-3-oxo-$5^2$,$5^3$-dihydro-$5^1$H-4-aza-2(2,4)-pyrimidin-1(1,3),6(1,4)-dipiperi din-5-(4,5)-indanedionenaphthalene-8-en-$5^7$-yl)ethane-1-sulfonamide**

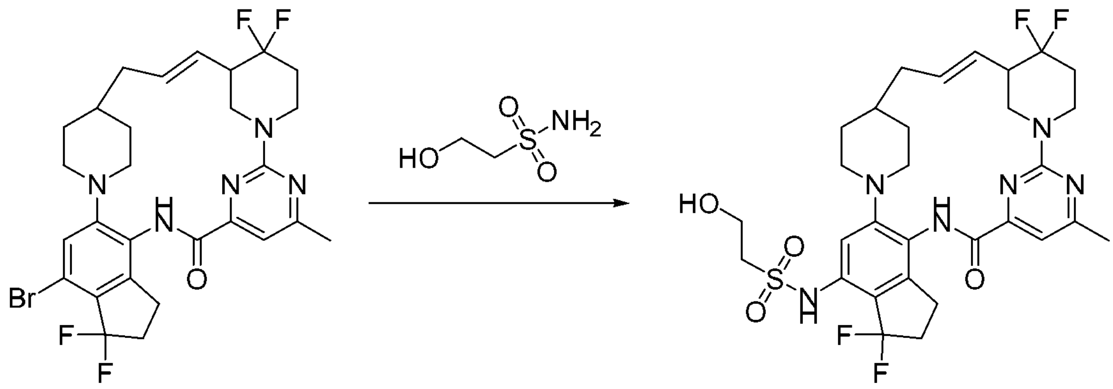

The preparations in **Example 29 followed** the synthetic route of **Example 25.** LCMS (ESI): $[M+H]^+$ = 653.2

**Example 30: 2-hydroxy-N-(2,2,4',4'-tetrafluoro-6'-methyl-4'-oxospiro[cyclopropane-1,1'-3-aza-2(2,4)-pyrimidin-1(1,3),6(1,4)-dipip eridin-5(4,5)-indophenanthronone]-8'-en-7'-yl)ethane-1-sulfonamide**

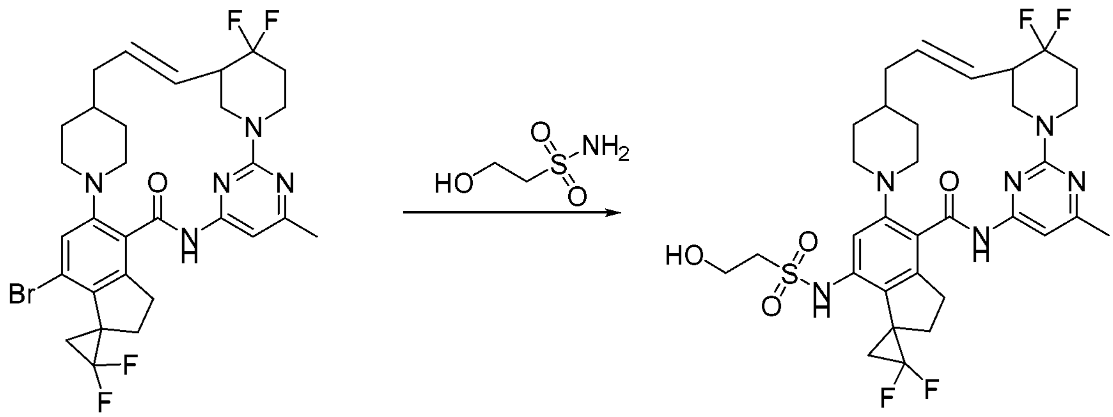

The preparations in **Example 30 followed** the synthetic route of **Example 2.** LCMS (ESI): $[M+H]^+$ = 679.3

**Example 31: N-(4',4'-difluoro-6'-methyl-4'-oxospiro[cyclopropane-1,1'-3-aza-2(2,4)-pyrimidin-1(1,3),6(1,4)-dipiperidin-5-(4,5)-ind onenaphthalene]-8'-en-7'-yl)-2-hydroxyethane-1-sulfonamide**

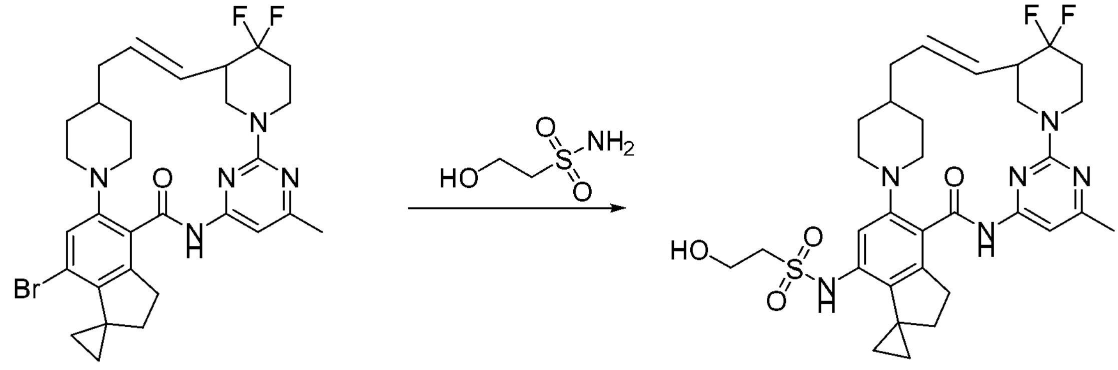

The preparations in **Example 31 followed** the synthetic route of **Example 2.** LCMS (ESI): $[M+H]^+$ = 643.3

**Example 32: N-($5^6$,8-dimethyl-$2^1$,3-dione-$2^2$,$2^3$-dihydro-$2^1$H-10-oxa-4,6-diaza-5(4,2)-pyrimidin-1(1,4)-piperidin-2(5,4)-indenocyclo undecane-$2^7$-yl)-2-hydroxyethane-1-sulfonamide**

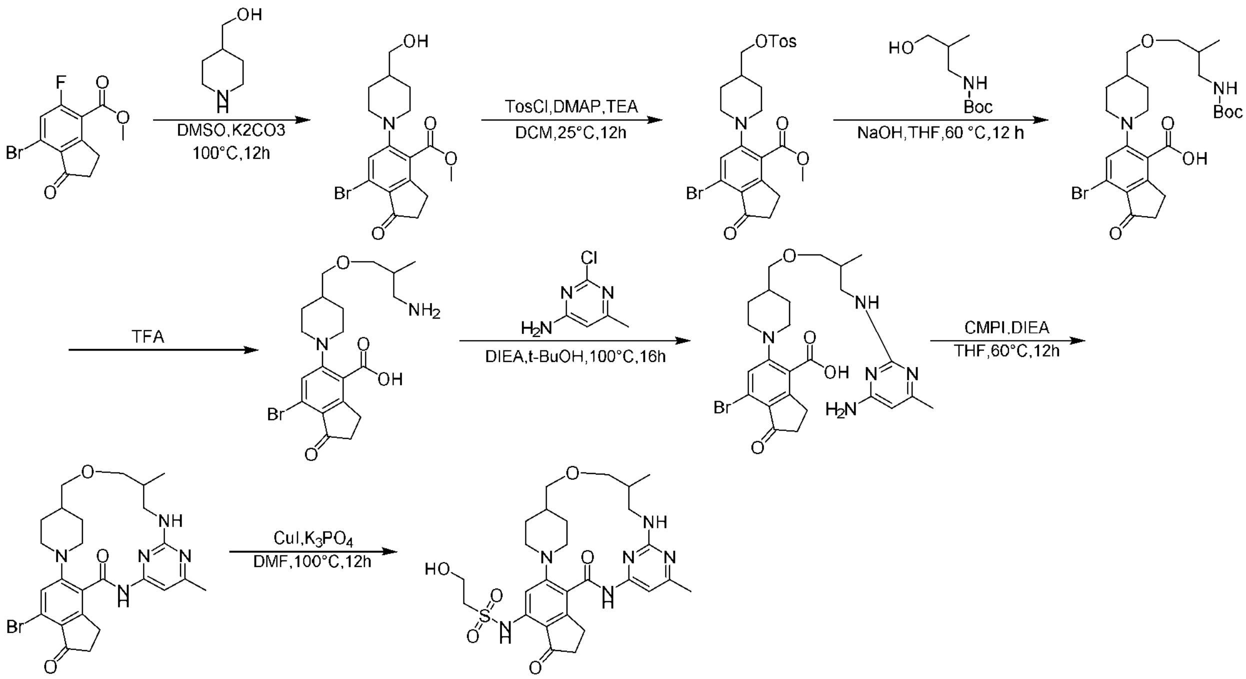


**Step 1:** To a solution of 7-bromo-5-fluoro-1-oxo-2,3-dihydro-1H-indene-4-carboxylate (14.07 g, 49.2 mmol) and potassium carbonate (17.80 g, 129.0 mmol) in dimethyl sulfoxide (200.00 mL) was added piperidin-4-ylmethanol (5.93 g, 51.5 mmol), and the mixture was stirred at 100°C for 12 h. The reaction mixture was poured into water (400 mL) and extracted with ethyl acetate (400 mL × 2). The combined organic layer was washed with brine (250 mL), dried over anhydrous magnesium sulfate, filtered, and concentrated under reduced pressure to give a residue. The residue was purified by flash column chromatography (silica gel, 0-16% gradient of tetrahydrofuran/petroleum ether) to give 7-bromo-5-(4-(hydroxymethyl)piperidin-1-yl)-1-oxo-2,3-dihydro-1H-indeno-4-carboxylate (15.89 g, 41.70 mmol, yield: 85%) as a yellow oil. LCMS (ESI): $[M+H]^+$ = 382.1

**Step 2:** At 25°C, to a solution of 7-bromo-5-(4-(hydroxymethyl)piperidin-1-yl)-1-oxo-2,3-dihydro-1H-indeno-4-carboxylate (8.14 g, 21.3 mmol) in dichloromethane (140.00 mL) were added triethylamine (4.32 g, 42.7 mmol), 4-dimethylaminopyridine (434 mg, 2.13 mmol) and 4-toluenesulfonyl chloride (4.47 g, 23.5 mmol). The reaction mixture was stirred at 25°C for 12 hours. The reaction mixture was poured into water (200 mL) and extracted with ethyl acetate (200 mL × 2). The combined organic layer was washed with brine (200 mL), dried over anhydrous magnesium sulfate, filtered, and concentrated under reduced pressure to give a residue. The residue was purified by flash column chromatography (silica gel, 0-8% gradient of tetrahydrofuran/petroleum ether) to give 7-bromo-1-oxo-5-(4-(toluenesulfonyloxy)methyl)piperidin-1-yl)-2,3-dihydro-1H-indeno-4-carboxylate (6.63 g, 12.4 mmol, yield: 58%) as a yellow oil. LCMS (ESI): $[M+H]^+$ = 536.1.

**Step 3:** To a solution of 7-bromo-1-oxo-5-(4-(toluenesulfonyloxy)methyl)piperidin-1-yl)-2,3-dihydro-1H-indeno-4-carboxylate (1.11 g, 2.07 mmol) and tert-butyl (3-hydroxy-2-methylpropyl)carbamate (0.59 g, 3.11 mmol) in tetrahydrofuran (20.0 mL) was added sodium hydroxide (0.21 g, 3.11 mmol), and the mixture was stirred at 60°C for 12 h. The reaction solution was diluted with water (80 mL), adjusted to pH 2-3 with saturated citric acid, and extracted with ethyl acetate (100 mL × 2), and the organic layer was dried over magnesium sulfate, filtered, and concentrated under reduced pressure to give the residue. The residue was purified by flash column chromatography (silica gel, 0-35% gradient of ethyl acetate/petroleum ether) to give 7-bromo-5-(4-((3-(tert-butyloxycarbonyl)amino)-2-methylpropoxy)methyl)piperidin-1-yl)-1-oxo-2,3-dihydro-1H-indene-4-carboxylic acid (0.46 g, 0.87 mmol, yield: 42%) as a yellow oil. LCMS (ESI): $[M+H]^+$ = 539.2

**Step 4:** A solution of 7-bromo-5-(4-((3-(tert-butyloxycarbonyl)amino)-2-methylpropoxy)methyl)piperidin-1-yl)-1-oxo-2,3-dihydro-1H-indene-4-carboxylic acid (420 mg, 0.78 mmol) in dichloromethane (20.00 mL) and trifluoroacetic acid (5.00 mL) was stirred at 25°C for 1 h. The reaction solution was concentrated under reduced pressure to give 5-(4-((3-amino-2-methylpropoxy)methyl)piperidin-1-yl)-7-bromo-1-oxo-2,3-dihydro-1H-indeno-4-carboxylic acid (500 mg, crude) as a yellow oil. LCMS (ESI): $[M+H]^+$ = 439.1

**Step 5:** To a solution of 5-(4-((3-amino-2-methylpropoxy)methyl)piperidin-1-yl)-7-bromo-1-oxo-2,3-dihydro-1H-indene-4-carboxylic acid (0.37 g, 0.85 mmol) in tert-butanol (7.00 mL) were added N,N-diisopropylethylamine (1.10 g, 8.51 mmol) and 2-chloro-6-methylpyrimidin-4-amine (147 mg, 1.02 mmol), and the mixture was stirred at 100°C for 16 h. The reaction solution was diluted with water (20 mL), adjusted to pH 2-3 with 1 N hydrochloric acid, and extracted with chloroform/isopropanol (3:1, 40 mL × 3). The organic layer was dried, filtered and concentrated. A brown oily residue was purified by flash column chromatography (silica gel, 0-10% gradient of dichloromethane/methanol) to give 5-(4-((3-(4-amino-6-methylpyrimidin-2-yl)amino)-2-methylpropoxy)methyl)piperidin-1-yl)-7-bromo-1-oxo-2,3-dihydro-1H -indeno-4-carboxylic acid (470 mg, crude) as a yellow oil. LCMS (ESI): $[M+H]^+$ = 546.3

**Step 6:** To a solution of N,N-diisopropylethylamine (763 mg, 5.78 mmol) and 2-chloro-1-methylpyridine iodide (444 mg, 1.74 mmol) in tetrahydrofuran (30.00 mL) was added 5-(4-((3-(4-amino-6-methylpyrimidin-2-yl)amino)-2-methylpropoxy)methyl)piperidin-1-yl)-7-bromo-1-oxo-2,3-dihydro-1H -indeno-4-carboxylic acid (316 mg, 0.58 mmol) in tetrahydrofuran (120.00 mL), and the mixture was stirred at 60°C for 12 h. The reaction mixture was diluted with water (100 mL), extracted with ethyl acetate (100 mL × 2), and the organic phase was dried, filtered and concentrated. The residue was purified by flash column chromatography (silica gel, 0-12% gradient of tetrahydrofuran/petroleum ether) to give $2^7$-bromo-$5^6$,8-dimethyl-$2^2$,$2^3$-dihydro-$2^1$H-10-oxa-4,6-diaza-5(4,2)-pyrimidin-1(1,4)-piperidin a-2(5,4)-indenocycloundecane-21,3-dione (70 mg, crude) as a colorless oil. LCMS (ESI): $[M+H]^+$ = 528.2

**Step 7:** To a solution of $2^7$-bromo-$5^6$,8-dimethyl-$2^2$,$2^3$-dihydro-$2^1$H-10-oxa-4,6-diaza-5(4,2)-pyrimidin-1(1,4)-piperidin a-2(5,4)-indenocycloundecane-21,3-dione (53 mg, 0.1 mmol) and 2-hydroxyethane-1-sulfonamide (15 mg, 0.12 mmol) in N,N-dimethylformamide (1.00 mL) were added potassium phosphate (63.6 mg, 0.3 mmol), (1R, 2R)-N1, N2-dimethylcyclohexane-1, 2-diamine (14.4 mg, 0.1 mmol) and cuprous iodide (19.1 mg, 0.1 mmol), and the mixture was stirred at 100°C for 12 h under nitrogen atmosphere. The reaction mixture was diluted with water (10.0 mL+0.5 mL liquor ammonia) and extracted with ethyl acetate (20 mL × 2), and the organic phase was dried, filtered, and concentrated. The residue was purified by preparative HPLC (C18, 6-46% gradient of water (formic acid)/acetonitrile) to give N-($5^6$,8-dimethyl-$2^1$,3-dione-$2^2$,$2^3$-dihydro-$2^1$H-10-oxa-4,6-diaza-5(4,2)-pyrimidin-1(1,4)-piperidin-2(5,4)-indenocyclounde cane-$2^7$-yl)-2-hydroxyethane-1-sulfonamide (6 mg, 0.01 mmol, yield: 14.7%) as a white solid. LCMS (ESI): $[M+H]^+$ = 573.2.

**Example 33: N-($5^6$,8-dimethyl-$2^1$,3-dione-$1^4$-(trifluoromethyl)-$2^2$,$2^3$-dihydro-$2^1$H-10-oxa-4,6-diaza-5(4,2)-pyrimidin-1(1,4)-piperidi n a-2(5,4)-indenocycloundecane-$2^7$-yl)-2-hydroxyethane-1-sulfonamide**

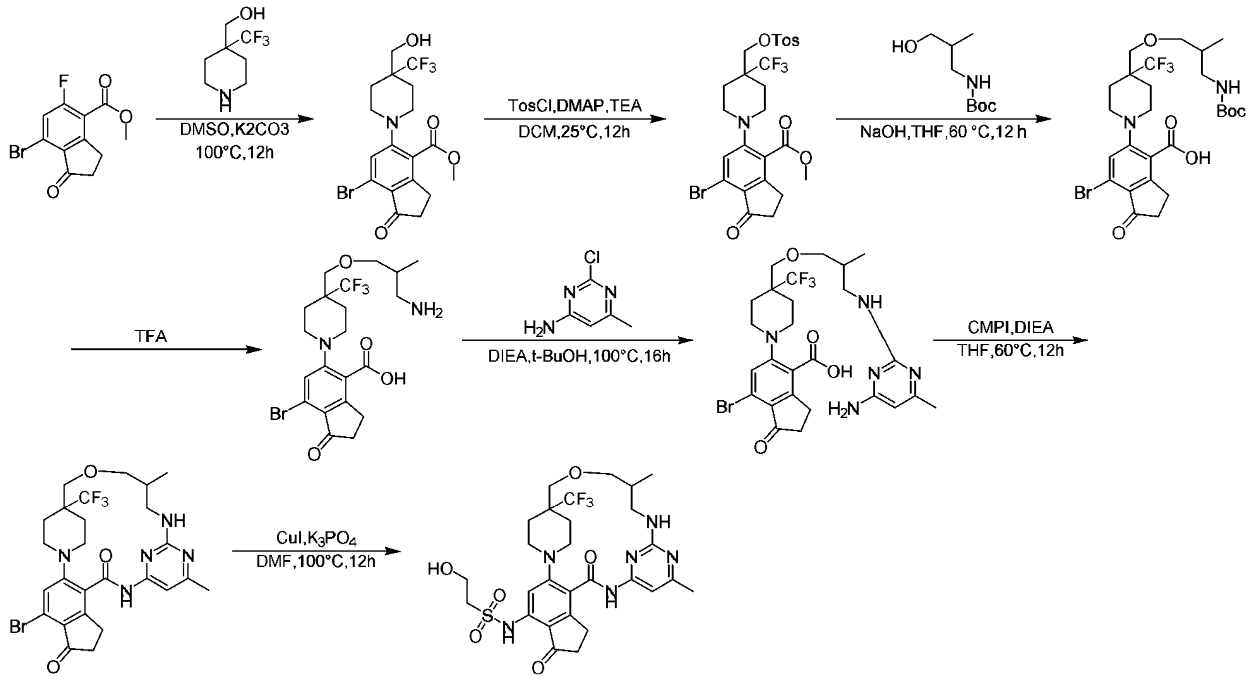


The preparations in **Example 33 followed** the synthetic route of **Example 32.** LCMS (ESI): $[M+H]^+$ = 641.2

**Example 34: N-((3Z,4E)-3-ethylene-8-methyl-1,18-dioxo-12-(trifluoromethyl)-1,2,3,6,7,8,9,11,12,13,14,18,20-tetradecahydro-12,15-ethylidene[4,5-k][1]oxa[5,7,9,13]tetraazacycloheptane-17-yl)-2-hydroxyethane-1-sulfonamide**

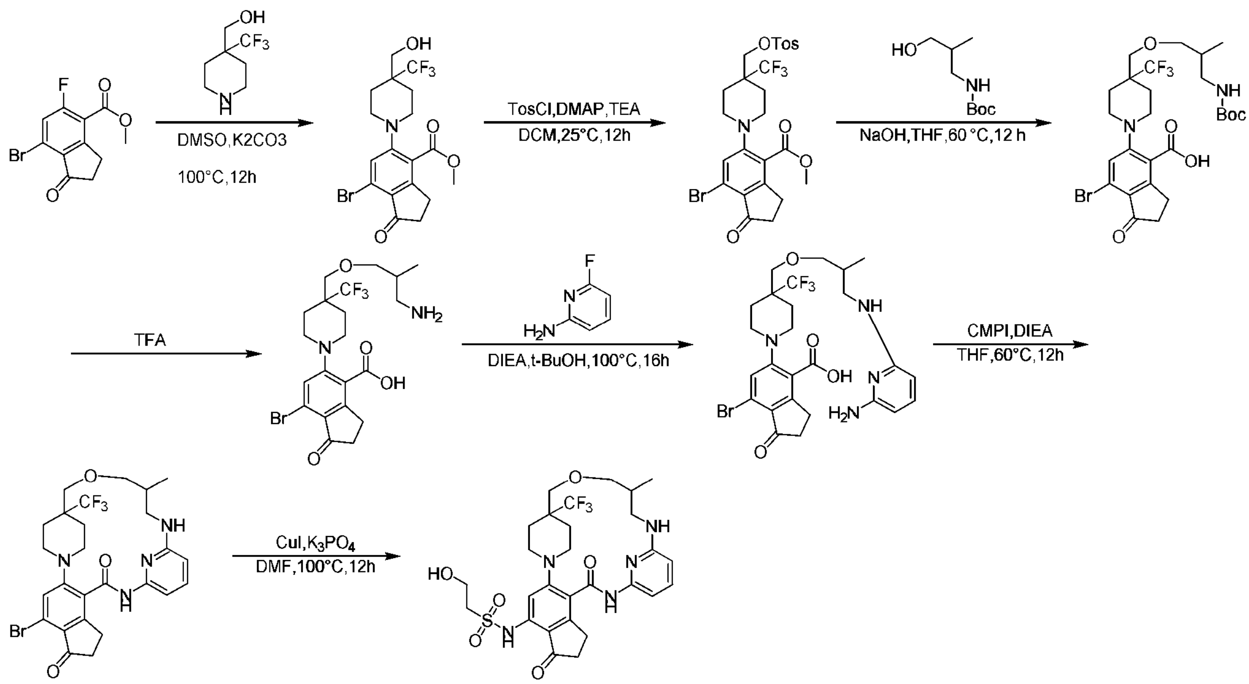


The preparations in **Example 34 followed** the synthetic route of **Example 32.** LCMS (ESI): $[M+H]^+$ = 616.2

**Example 35: N-((3Z,4E)-3-ethylene-8-methyl-1,18-dioxo-1,2,3,6,8,9,11,12,13,14,18,19,20-tetradecahydro-12,15-ethylidene[4,5-k][1] oxa[5,7,9,13]tetraazacycloheptane-17-yl)-2-hydroxyethane-1-sulfonamide**

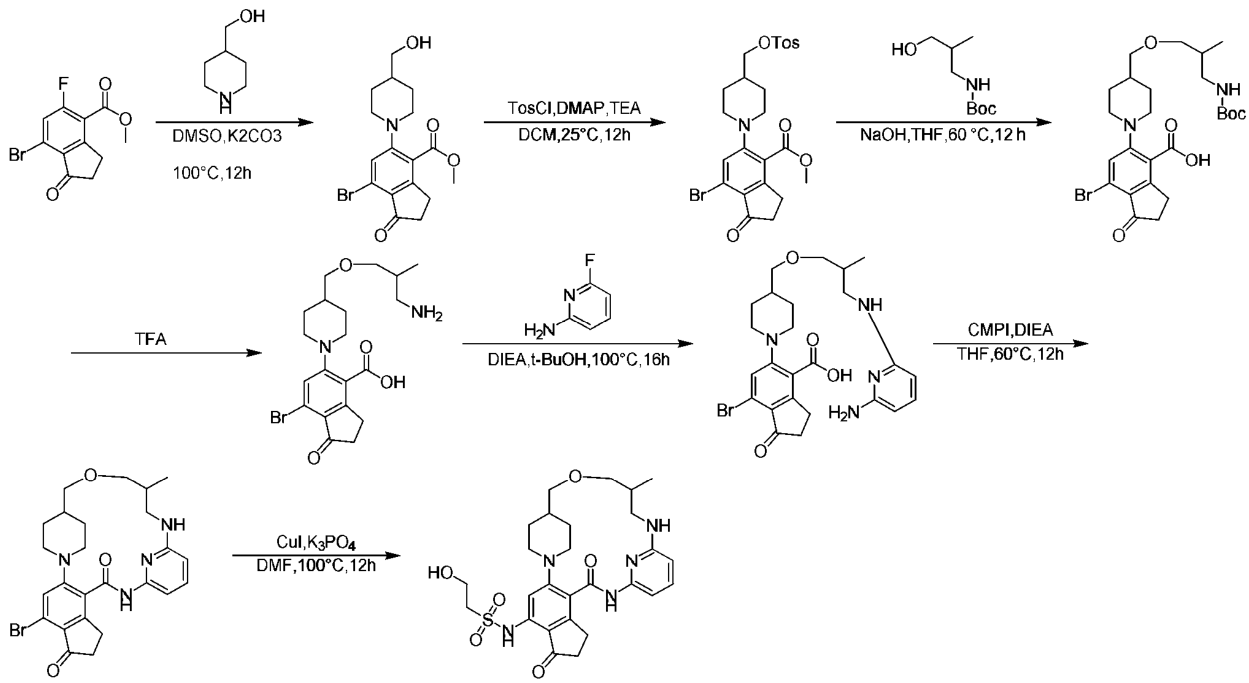


The preparations in **Example 35 followed** the synthetic route of **Example 32.** LCMS (ESI): $[M+H]^+$ = 548.2

**Example 36: 2-hydroxy-N-(5^6-methyl-2^1,3-dione-2^2,2^3-dihydro-2^1H-4,6-diaza-5(4,2)-pyrimidin-1(1,4)-piperidin-2(5,4)-indenocyclo undecane-9-en-2^7-yl)ethane-1-sulfonamide**

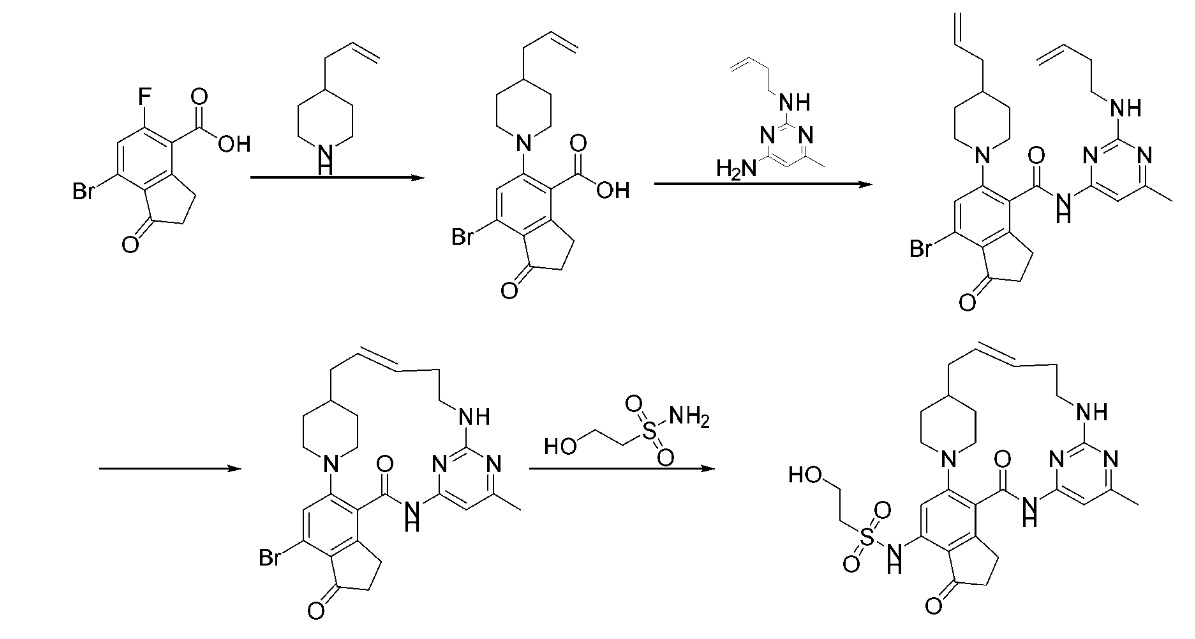

The preparations in **Example 36 followed** the synthetic route of **Example 2.** LCMS (ESI): $[M+H]^+$ = 555.2

**Example 37: N-($5^6$,8-dimethyl-$2^1$,3-dione-$2^2$,$2^3$-dihydro-$2^1$H-4,6-diaza-5(4,2)-pyrimidin-1(1,4)-piperidin a-2(5,4)-indenoundecane-9-en-$2^7$-yl)-2-hydroxyethane-1-sulfonamide**

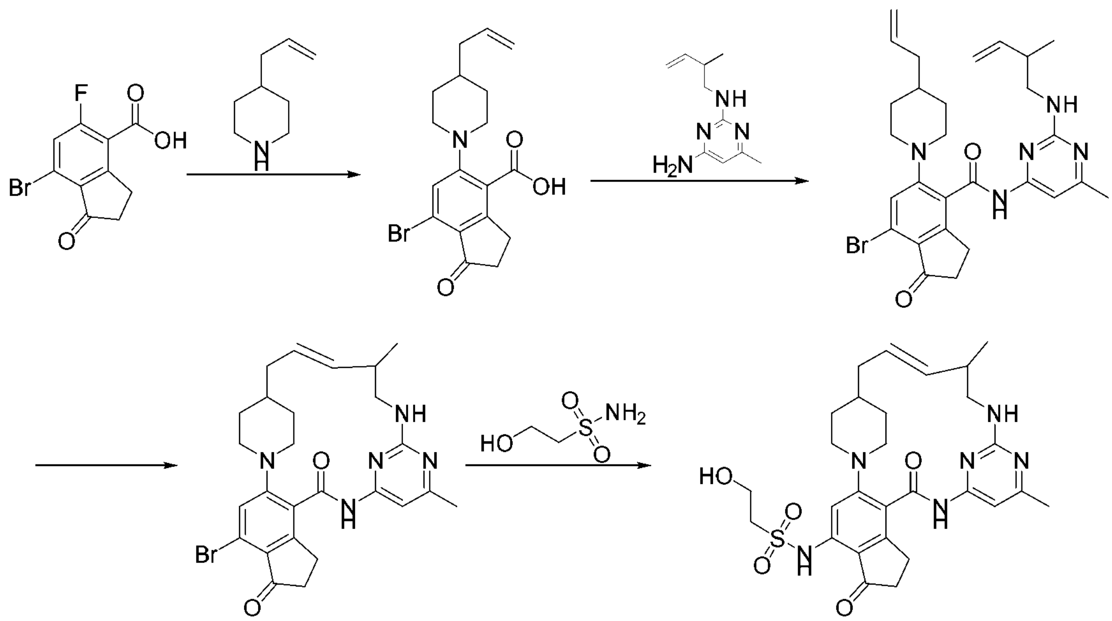

The preparations in **Example 37 followed** the synthetic route of **Example 2.** LCMS (ESI): $[M+H]^+$ = 569.2

**Example 38: 2-Hydroxy-N-(8-methyl-$2^1$,3-dione-$2^2$,$2^3$-dihydro-$2^1$H-4,6-diaza-5(2,6)-pyridin-1(1,4)-piperidin-2(5,4)-indenoundecan e-9-en-$2^7$-yl)ethane-1-sulfonamide**

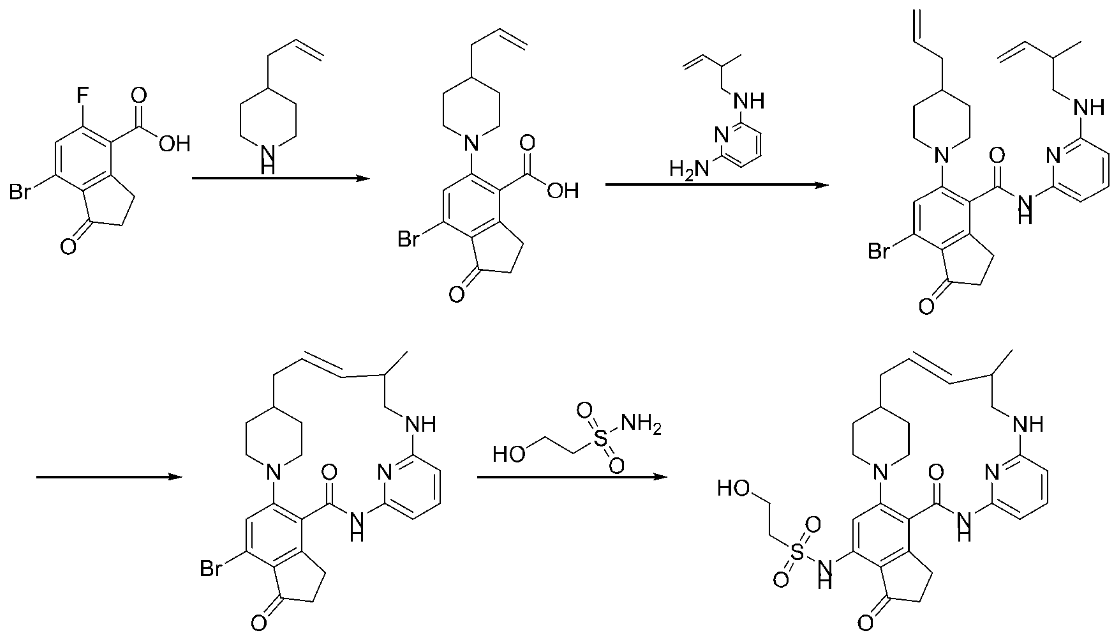

The preparations in **Example 38 followed** the synthetic route of **Example 2.** LCMS (ESI): $[M+H]^+$ = 554.2

**Example 39: N-($5^6$,6-dimethyl-$2^1$,3-dione-$2^2$,$2^3$-dihydro-$2^1$H-4,6-diaza-5(4,2)-pyrimidin-1(1,4)-piperidin a-2(5,4)-indenoundecane-9-en-$2^7$-yl)-2-hydroxyethane-1-sulfonamide**

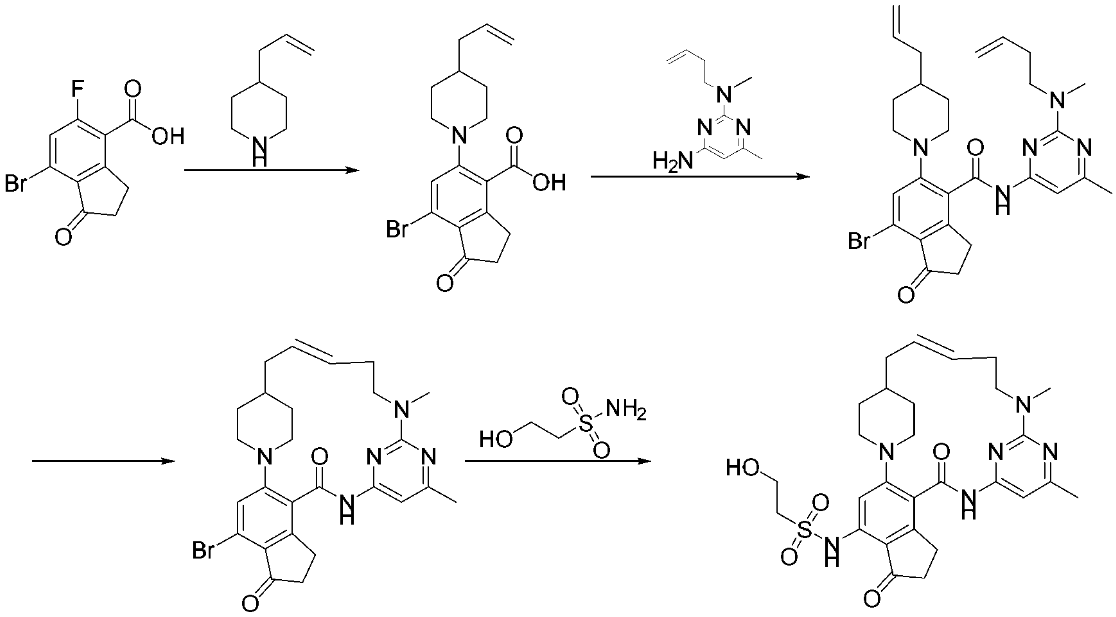

The preparations in **Example 39 followed the** synthetic route of **Example 2.** LCMS (ESI): $[M+H]^+$ = 569.2

**Example 40: 2-Hydroxy-N-($5^6$,6,8-trimethyl-$2^1$,3-dione-$2^2$,$2^3$-dihydro-$2^1$H-4,6-diaza-5(4,2)-pyrimidin-1(1,4)-piperidin a-2(5,4)-indenocycloundecane-9-en-$2^7$-yl)ethane-1-sulfonamide**

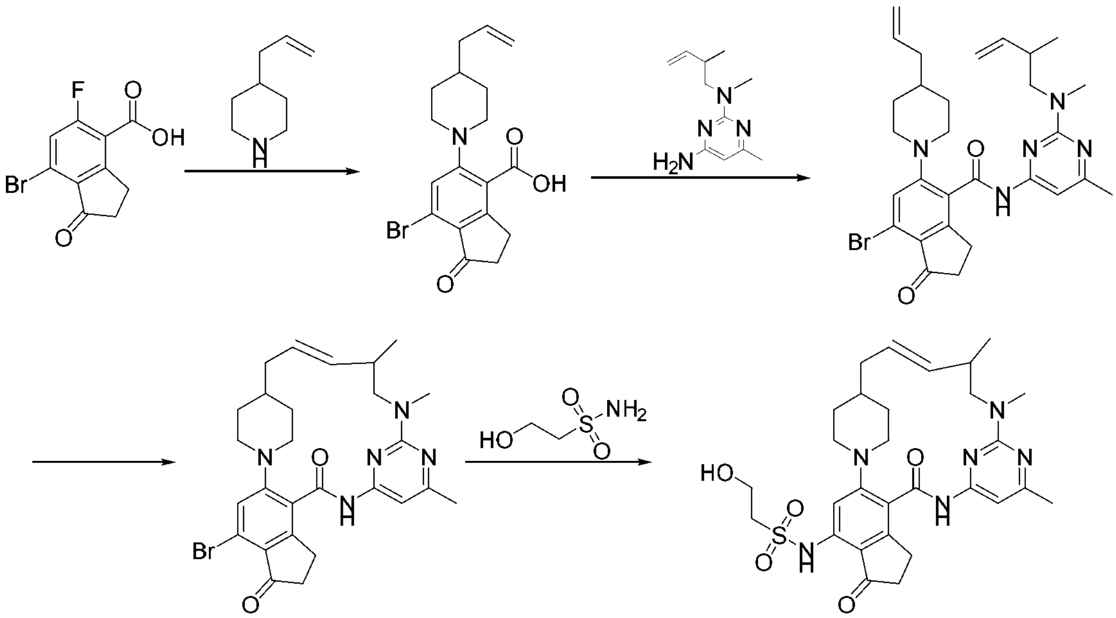

The preparations in **Example 40 followed** the synthetic route of **Example 2.** LCMS (ESI): $[M+H]^+$ = 583.2

**Example 41: N-(6,8-dimethyl-$2^1$,3-dione-$2^2$,$2^3$-dihydro-$2^1$H-4,6-diaza-5(2,6)-pyridin-1(1,4)-piperidin a-2(5,4)-indenoundecane-9-en-$2^7$-yl)-2-hydroxyethane-1-sulfonamide**

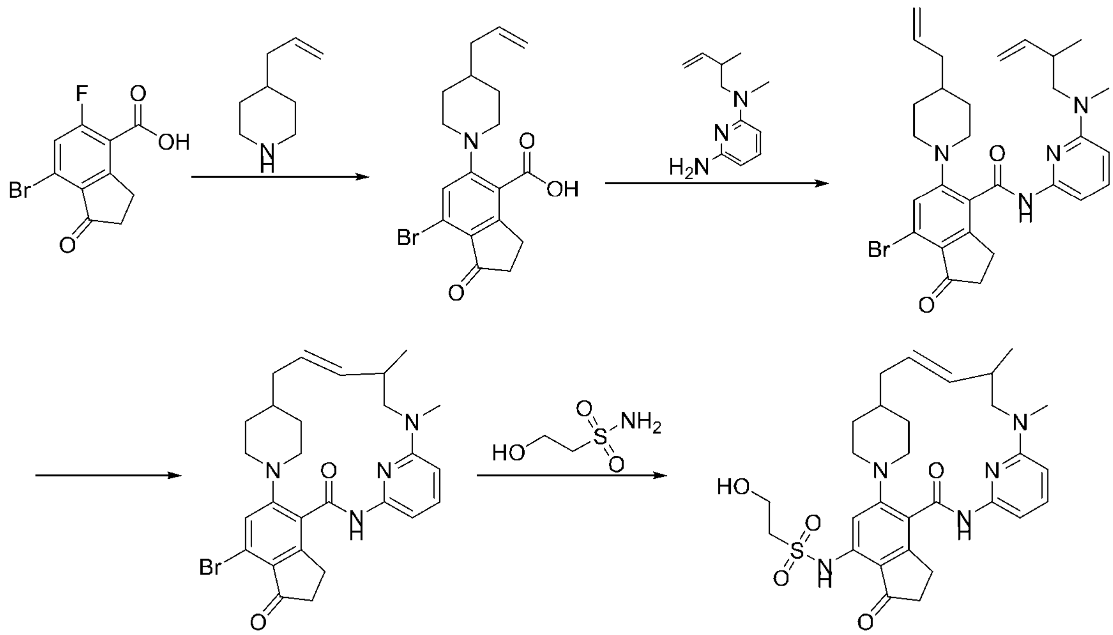

The preparations in **Example 41 followed** the synthetic route of **Example 2.** LCMS (ESI): $[M+H]^+$ = 568.3

Example **32,** Example **33,** Example 34 and Example **35** were separated by SFC to give Examples **32A and 32B,** Examples **33A and 33B, Examples 34A and 34B,** and Examples **35A and 35B,** respectively.

| Example | Structure | Name | LCMS/ $[M+H]^+$ |
|---|---|---|---|
| **32A** | 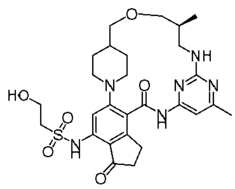 | (S)-N-($5^6$,8-dimethyl-$2^1$,3-dione-$2^2$,$2^3$-dihydro -$2^1$H-10-oxa-4,6-diaza-5(4,2)-pyrimidin-1(1,4 )-piperidin-2(5,4)-indenocycloundecane-$2^7$-yl )-2-hydroxyethane-1-sulfonamide | 573.2 |
| **32B** | 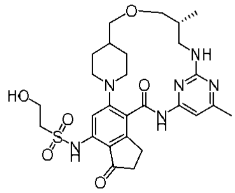 | (R)-N-($5^6$,8-dimethyl-$2^1$,3-dione-$2^2$,$2^3$-dihydr o-$2^1$H-10-oxa-4,6-diaza-5(4,2)-pyrimidin-1(1, 4)-piperidin-2(5,4)-indenocycloundecane-$2^7$-yl )-2-hydroxyethane-1-sulfonamide | 573.2 |
| **33A** | 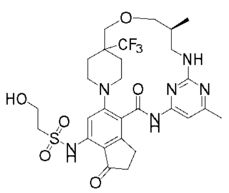 | (S)-N-($5^6$,8-dimethyl-$2^1$,3-dione-$1^4$-(trifluoro methyl)-$2^2$,$2^3$-dihydro-$2^1$H-10-oxa-4,6-diaza-5(4,2)-pyrimidin-1(1,4)-piperidin-2(5,4)-inde nocycloundecane-$2^7$-yl)-2-hydroxyethane-1-sulfonamide | 641.2 |

(continued)

| Example | Structure | Name | LCMS/ [M+H]+ |
|---|---|---|---|
| **33B** | 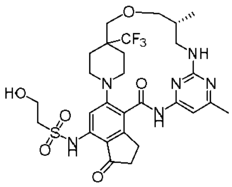 | (R)-N-($5^6$,8-dimethyl-$2^1$,3-dione-$1^4$-(trifluoro methyl)-$2^2$,$2^3$-dihydro-$2^1$H-10-oxa-4,6-diaza-5(4,2)-pyrimidin-1(1,4)-piperidin-2(5,4)-inde nocycloundecane-$2^7$-yl)-2-hydroxyethane-1-sulfonamide | 641.2 |
| **34A** | 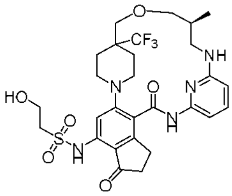 | (S)-2-hydroxy-N-(8-methyl-$2^1$,3-dione-$1^4$-(tri fluoromethyl)-$2^2$,$2^3$-dihydro-$2^1$H-10-oxa-4,6-diaza-5(2,6)-pyridin-1(1,4)-piperidin a-2(5,4)-indenocycloundecane-$2^7$-yl)ethane-1 -sulfonamide | 626.2 |
| **34B** | 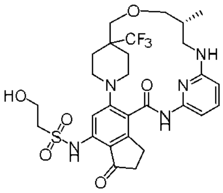 | (R)-2-hydroxy-N-(8-methyl-$2^1$,3-dione-$1^4$-(tri fluoromethyl)-$2^2$,$2^3$-dihydro-$2^1$H-10-oxa-4,6-diaza-5(2,6)-pyridin-1(1,4)-piperidin a-2(5,4)-indenocycloundecane-$2^7$-yl)ethane-1 -sulfonamide | 626.2 |
| **35A** | 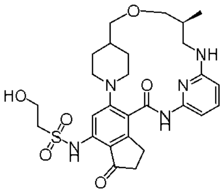 | (S)-2-hydroxy-N-(8-methyl-$2^1$,3-dione-$2^2$,$2^3$-dihydro-$2^1$H-10-oxa-4,6-diaza-5(2,6)-pyridin-1(1,4)-piperidin-2(5,4)-indenocycloundecane-$2^7$-yl)ethane-1-sulfonamide | 558.2 |
| **35B** | 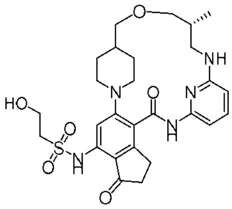 | (R)-2-hydroxy-N-(8-methyl-$2^1$,3-dione-$2^1$,$2^1$-dihydro-$2^1$H-10-oxa-4,6-diaza-5(2,6)-pyridin-1(1,4)-piperidin-2(5,4)-indenocycloundecane-$2^7$-yl)ethane-1-sulfonamide | 558.2 |

**Example 42:** ***N-($1^4$,$1^4$-difluoro-$2^6$-methyl-4-oxo-3-aza-2(2,4)-pyrimidin-1(1,3)-piperidin-5(1,2),6(1,4)-diphenylheterocyclonon-8-en-***$5^4$**-yl)-2-hydroxyethane-1-sulfonamide**

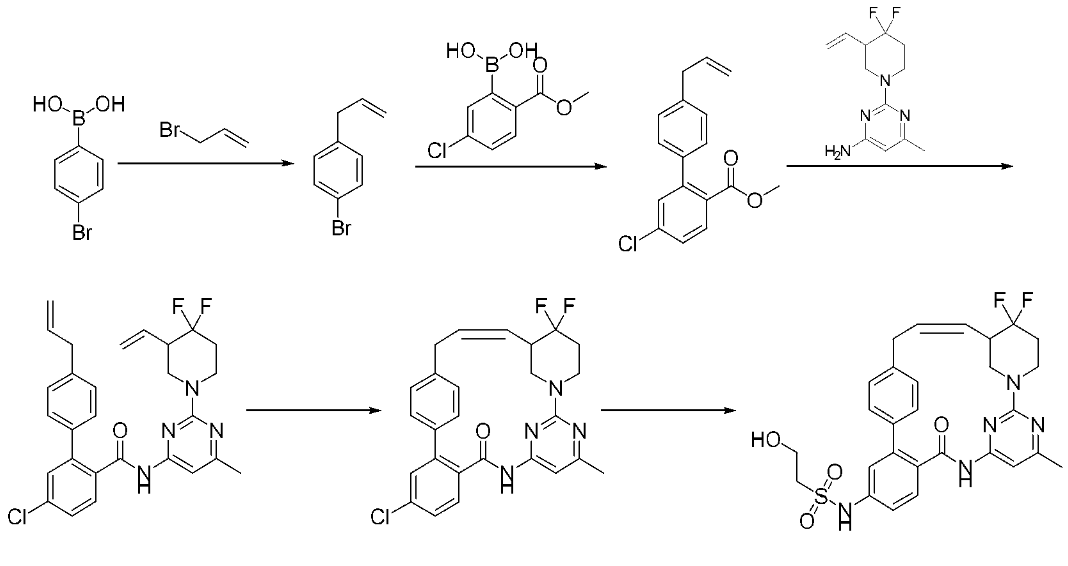

**Step 1:** A solution of (4-bromophenyl)boric acid (5.00 g, 24.90 mmol), allyl bromide (6.0 g, 49.79 mmol), potassium carbonate (10.5 g, 74.69 mmol) and palladium acetate (570 mg, 2.49 mmol) in toluene (50.00 mL) was stirred at 80°C for 12 h under nitrogen atmosphere, diluted with water (50 mL) and extracted with ethyl acetate (50 mL × 3), and the organic phase was dried over anhydrous sodium sulfate, filtered, concentrated, and purified by column chromatography (silica gel, 0-100% gradient of ethyl acetate/petroleum ether) to give 1-allyl-4-bromobenzene (3.50 g, crude) as a colorless oil. $^1$H NMR (400 MHz, DMSO) δ 7.48 (dd, *J* = 8.4, 2.4 Hz, 2H), 7.18 - 7.14 (m, 2H), 6.01 - 5.87 (m, 1H), 5.11 - 5.06 (m, 1H), 5.06 - 5.03 (m, 1H), 3.39 - 3.28 (m, 2H) ppm.

**Step 2:** A solution of 1-allyl-4-bromobenzene (3.50 g, 17.76 mmol), (5-chloro-2-(methoxycarbonyl)phenyl)boric acid (5.70 g, 26.64 mmol), potassium carbonate (7.50 g, 53.28 mmol) and 1,1-bis(diphenylphosphine)disulfonium palladium dichloride (II) (1.30 g, 1.78 mmol) in 1,4-dioxane (40.00 mL) and water (10.00 mL) was stirred at 80°C for 12 h under nitrogen atmosphere, diluted with water (50 mL) and extracted with ethyl acetate (50 mL × 3), and the organic phase was dried over anhydrous sodium sulfate, filtered, concentrated, and purified by column chromatography (silica gel, 0-100% gradient of ethyl acetate/petroleum ether) to give 4'-allyl-5-chloro-[1,1'-biphenyl]-2-carboxylate (2.60 g, crude) as a yellow oil. LCMS (ESI): $[M+H]^+$ = 287.10.

**Step 3:** To a solution of 2-(4,4-difluoro-3-vinylpiperidinyl)-6-methylpyrimidin-4-amine (2.10 g, 8.37 mmol) and methyl 4-chloro-2-(4-propan-2-phenyl)benzoate (2 g, 6.97mmol) in tetrahydrofuran (20 mL) was added lithium bis(trimethylsilyl)amide (10.50 mL, 1 M). The resulting mixture was stirred at 20°C for 1 h, diluted with water (50 mL) and extracted with ethyl acetate (50 mL × 3), and the organic phase was dried over anhydrous sodium sulfate, filtered, concentrated, and purified by column chromatography (silica gel, 0-100% gradient of ethyl acetate/petroleum ether) to give 4'-allyl-5-chloro-N-(2-(4,4-difluoro-3-vinylpiperidin-1-yl)-6-methylpyrimidin-4-yl)-[1,1'-biphenyl]-2-carboxamide (1.00 g, crude) as a yellow solid. LCMS (ESI): $[M+H]^+$ = 509.20. $^1$H NMR (400 MHz, DMSO) δ ppm 10.72 - 10.63 (m, 1H), 7.58 - 7.45 (m, 3H), 7.43 - 7.29 (m, 4H), 7.23 - 7.17 (m, 1H), 6.46 - 6.26 (m, 1H), 6.04 - 5.88(m, 1H), 5.85 - 5.68 (m, 1H), 5.40 - 5.22 (m, 2H), 5.18 - 4.99 (m, 1H), 4.46 (brs, 2H), 3.36 (d, *J* = 6.8 Hz, 1H), 3.13 (brs, 2H), 2.73 - 2.55 (m, 1H), 2.24 (s, 3H), 2.09 - 2.00 (m, 1H), 1.94 - 1.77 (m, 2H).

**Step 4:** A solution of 4'-allyl-5-chloro-N-(2-(4,4-difluoro-3-vinylpiperidin-1-yl)-6-methylpyrimidin-4-yl)-[1,1'-biphenyl]-2-carboxamide (1.00 g, 1.96 mmol) and Grubbs catalyst 2nd generation (170 mg, 0.20 mmol) in dichloromethane (200 mL) was stirred at 50°C for 2 h. The reaction solution was concentrated and purified by column chromatography (silica gel, 0-20% gradient of ethyl acetate/petroleum ether) to give $5^4$-chloro-$1^4$,$1^4$-difluoro-$2^6$-methyl-3-aza-2(2,4)-pyrimidin-1(1,3)-piperidin-5(1,2),6(1,4)-diphenylheterocyclonon-8-en-4-on e (460 mg, crude) as a yellow solid. LCMS (ESI): $[M+H]^+$ = 481.10.

**Step 5:** The reaction solution of $5^4$-chloro-$1^4$,$1^4$-difluoro-$2^6$-methyl-3-aza-2(2,4)-pyrimidin-1(1,3)-piperidin-5(1,2),6(1,4)-diphenylcyclonon-8-en-4-one (460 mg, 0.96 mmol), 2-hydroxyethanesulfonamide (244 mg, 1.91 mmol), potassium phosphate (622 mg, 2.87 mmol), 2-di-tert-butylphosphino-2',4',6'-triisopropylbiphenyl (41 mg, 0.096 mmol) and tris(dibenzylideneacetone)dipalladium (89 mg, 0.096 mmol) in dioxane (5 mL) was stirred at 100°C for 2 h under nitrogen atmosphere, diluted with water (30 mL), and extracted with ethyl acetate (30 mL × 3). The organic phase was dried over anhydrous sodium sulfate, filtered, concentrated, and purified by column chromatography (silica gel, 0-100% gradient of ethyl acetate/petroleum ether) to give N-($1^4$,$1^4$-difluoro-$2^6$-methyl-4-oxo-3-aza-2(2,4)-pyrimidin-1(1,3)-piperidin-5(1,2),6(1,4)-diphenylheterocyclonon-8-en-54-y 1)-2-hydroxyethane-1-sulfonamide (370 mg, crude) as a white solid. LCMS (ESI): $[M+H]^+$ = 570.20.

The above mixture was purified by preparative HPLC (C18, 24-64% gradient of water (hydrochloric acid)/acetonitrile) to give **Example** 1 N-($1^4$,$1^4$-difluoro-$6^4$-methoxy-$2^6$-methyl-4-oxo-3-aaa-2(2,4)-pyrimidin-1(1,3),6(1,4)-dipiperidin-5(1,2)-phenylheterocyclon on-8-en-$5^4$-yl)-2-hydroxyethane-1-sulfonamide (104 mg) as a white solid. LCMS (ESI): $[M+H]^+$ = 570.2 $^1H$ NMR (400 MHz, DMSO-$d_6$) δ ppm 10.30 (s, 1H), 8.15 (br s, 1H), 7.78 (d, *J* = 8.4 Hz, 1H), 7.50 (br d, *J* = 7.2 Hz, 1H), 7.41 - 7.30 (m, 2H), 7.21 (br d, *J* = 8.4 Hz, 3H), 6.98 (br d, *J* = 7.2 Hz, 1H), 6.50 - 6.33 (m, 1H), 5.58 (br t, J= 10.0 Hz, 1H), 4.66 (br d, J= 13.2 Hz, 1H), 4.00 (br dd, *J* = 3.6, 6.0 Hz, 1H), 3.77 (t, *J* = 6.4 Hz, 2H), 3.51 - 3.31 (m, 4H), 3.04 (br t, *J* = 13.2 Hz, 1H), 2.86 - 2.65 (m, 2H), 2.30 (s, 3H), 2.17 - 2.04 (m, 1H), 1.93 - 1.71 (m, 1H)

**Example 43: *N*-($2^4$,$2^4$-difluoro-$1^6$-methyl-7-oxo-8-aza-1(2,4)-pyrimidin-2(1,3)-piperidin-5(1,4),6(1,2)-diphenylcyclooct-3-en-$6^5$-yl)-2 -hydroxyethane-1-sulfonamide**

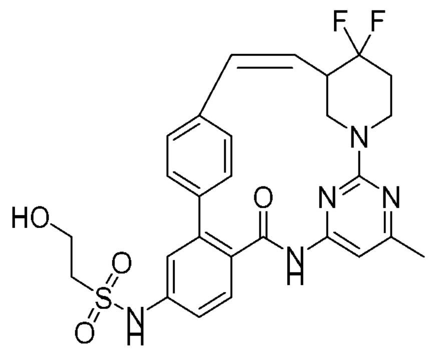

The preparations in **Example 43** followed the synthetic route of **Example 42.** LCMS (ESI): $[M+H]^+$ = 556.2 $^1H$ NMR (400 MHz, DMSO-$d_6$) δ ppm 10.33 (s, 1H), 7.88 (d, *J* = 8.4 Hz, 1H), 7.81 (s, 1H), 7.58 (br d, *J* = 8.0 Hz, 1H), 7.47 - 7.36 (m, 2H), 7.33 (br d, *J* = 8.0 Hz, 1H), 7.26 (s, 1H), 7.20 - 7.09 (m, 2H), 6.91 (br d, *J* = 11.6 Hz, 1H), 5.95 (dd, *J* = 8.0, 11.6 Hz, 1H), 4.97 (br t, *J*= 5.2 Hz, 1H), 4.63 (br d, *J* = 12.0 Hz, 1H), 4.12 - 3.91 (m, 1H), 3.78 (q, *J* = 6.0 Hz, 2H), 3.39 (br t, *J*= 6.4 Hz, 2H), 3.30 - 3.15 (m, 1H), 2.98 (br t, *J* = 12.8 Hz, 1H), 2.68 (br t, J= 12.4 Hz, 1H), 2.30 (s, 3H), 2.16 - 2.01 (m, 1H), 1.92 - 1.62 (m, 1H)

**Example 44: N-(($1^3$S,Z)-$1^4$,$1^4$-difluoro-$2^6$-methyl-$3^1$H-2(2,4)-pyrimidin-1(1,3),5(1,4)-dipiperidin-3(4,1)-triazole-4(1,2)-benzylcycloo ctyl-$4^4$-yl)-2-hydroxyethane-1-sulfonamide**

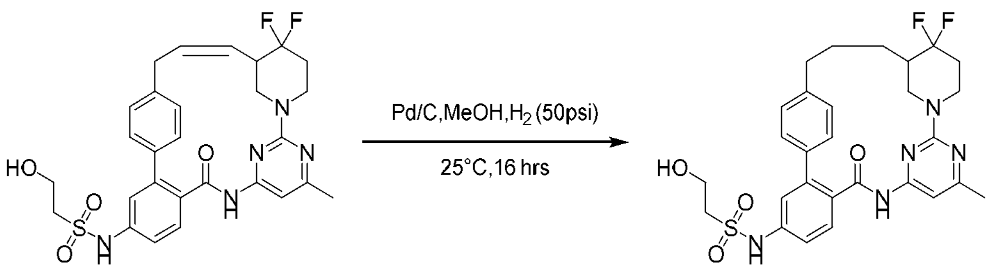


To a solution of N-($1^4$,$1^4$-difluoro-$2^6$-methyl-4-oxo-3-aza-2(2,4)-pyrimidin-1(1,3)-piperidin-5(1,2),6(1,4)-dibenzylcyclononane-8-en-$5^4$-yl)-2 -hydroxyethane-1-sulfonamide (100 mg, 0.17 mmol) in methanol (10.0 mL) was added 10% Pd/C (wet) (20.0 mg, 0.01 mmol), and the reaction mixture was purged with argon three times and then flushed with hydrogen three times and stirred at 25°C for 16 h under hydrogen atmosphere (50 Psi). The reaction mixture was filtered under reduced pressure to remove palladium/carbon and then concentrated in vacuum. The residue was purified by preparative HPLC (C18, 38-78% gradient of water (formic acid)/acetonitrile) to give N-($1^4$,$1^4$-difluoro-$2^6$-methyl-4-oxo-3-aza-2(2,4)-pyrimidin-1(1,3)-piperidin-5(1,2),6(1,4)-dibenzylcyclononane-$5^4$-yl)-2-hydr oxyethane-1-sulfonamide (49.8 mg, 0.08 mmol, yield: 49.6%) as a white solid. LCMS (ESI): $[M+H]^+$ =572.2 $^1H$ NMR (400 MHz, DMSO-$d_6$) δ ppm 10.88 - 9.44 (m, 1H), 8.63 - 8.38 (m, 1H), 7.69 (d, *J* = 8.4 Hz, 1H), 7.48 - 6.82 (m, 7H), 5.54 - 4.66 (m, 1H), 4.52 - 4.30 (m, 1H), 4.02 - 3.83 (m, 1H), 3.78 (t, *J*= 6.4 Hz, 2H), 3.41 - 3.34 (m, 2H), 3.24 - 3.15 (m, 1H), 3.12 - 2.99 (m, 1H), 2.90 - 2.76 (m, 1H), 2.45 - 2.37 (m, 1H), 2.29 - 2.16 (m, 3H), 2.09 - 1.48 (m, 5H), 1.30 - 0.99 (m, 2H)

**Example 45:** ***N-(2⁴,2⁴-difluoro-1⁶-methyl-7-oxo-8-aza-1(2,4)-pyrimidin-2(1,3)-piperidin-5(1,4),6(1,2)-dibenzylcycloophene-6⁵-yl)-2-*****hydroxyethane-1-sulfonamide**

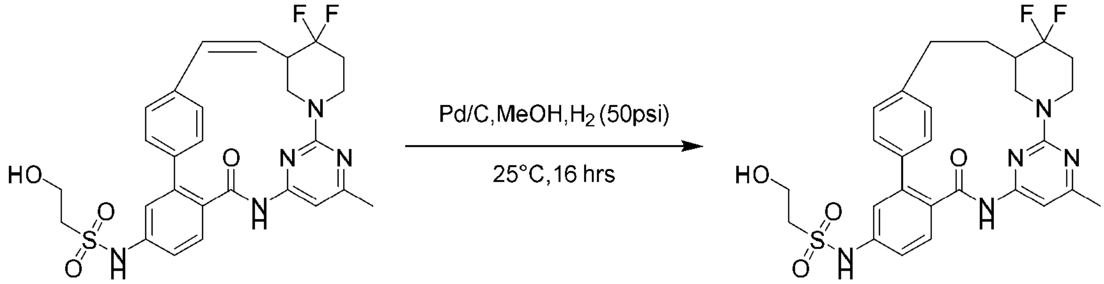


To a solution of N-($2^4$,$2^4$-difluoro-$1^6$-methyl-7-oxo-8-aza-1(2,4)-pyrimidin-2(1,3)-piperidin-5(1,4),6(1,2)-dibenzylcycloophene-3-en-$6^5$-yl)-2 -hydroxyethane-1-sulfonamide (40.0 mg, 0.07 mmol) in methanol (10.0 mL) was added 10% Pd/C (wet) (8.00 mg, 0.02 mmol), and the reaction mixture was purged with argon three times and then flushed with hydrogen three times and stirred at 25°C for 16 hours under hydrogen atmosphere (50 Psi). The reaction mixture was filtered under reduced pressure to remove palladium/carbon and then concentrated in vacuum. The residue was purified by preparative HPLC (C18, 38%-78% gradient of water (formic acid)/acetonitrile) to give N-($2^4$,$2^4$-difluoro-$1^6$-methyl-7-oxo-8-aza-1(2,4)-pyrimidin-2(1,3)-piperidin-5(1,4),6(1,2)-dibenzylcycloophene-$6^5$-yl)-2-hydr oxyethane-1-sulfonamide (8.67 mg, 0.01 mmol, yield: 61%) as a white solid. LCMS (ESI): $[M+H]^+$ =558.2 $^1$H NMR (400 MHz, DMSO-$d_6$) δ ppm 7.84 (s, 1H), 7.79 (d, *J* = 8.4 Hz, 1H), 7.53 (s, 2H), 7.27 (br d, *J* = 8.4 Hz, 1H), 7.19 - 7.07 (m, 3H), 7.01 (d, *J= 8.0* Hz, 1H), 4.60 - 4.48 (m, 1H), 3.96 - 3.69 (m, 3H), 3.29 - 3.26 (m, 2H), 3.05 - 2.95 (m, 2H), 2.72 - 2.64 (m, 1H), 2.46 - 2.35 (m, 2H), 2.31 - 2.15 (m, 4H), 2.11 - 1.94 (m, 1H), 1.79 - 1.59 (m, 2H), 1.43 - 1.27 (m, 1H)

**Example 46: Two isomers of N-(1⁴,1⁴-difluoro-2⁶-methyl-4-oxo-3-aza-2(2,4)-pyrimidin-1(1,3)-piperidin-5(1,2),6(1,4)-diphenylheterocyclononane-5⁴ -yl)-2-hydroxyethane-1-sulfonamide**

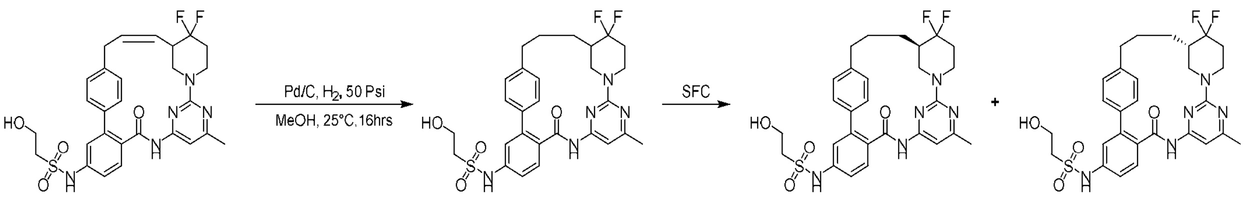


**Step 1:** To a solution of (cis)-N-($1^4$,$1^4$-difluoro-$2^6$-methyl-4-oxo-3-aza-2(2,4)-pyrimidin-1(1,3)-piperidin-5(1,2),6(1,4)-**diphenylheterocyclonon**-8-e n-$5^4$-yl)-2-hydroxyethane-1-sulfonamide (100 mg, 0.18 mmol) in methanol (10.00 mL) was added 10% Pd/C (wet) (20.0 mg, 0.01 mmol), and the reaction mixture was purged with argon three times and then flushed with hydrogen three timesand stirred at 25°C for 16 hours under hydrogen atmosphere (50 Psi). The reaction mixture was filtered under reduced pressure to remove palladium/carbon and then concentrated in vacuum. The residue was purified by preparative HPLC (C18, 38-78% gradient of water (formic acid)/acetonitrile) to give N-($1^4$,$1^4$-difluoro-$2^6$-methyl-4-oxo-3-aza-2(2,4)-pyrimidin-1(1,3)-piperidin-5(1,2),6(1,4)-diphenylheterocyclononane-$5^4$-yl)-2-hydroxyethane-1-sulfonamide (10-168-H2) (49.8 mg) as a white solid.

The mixture (49.8 mg) was separated by SFC (column: ChiralPak IH, 250×30 mm, 10 μm, mobile phase: $CO_2$-MeOH (0.1% $NH_3$ •$H_2O$), gradient: isocratic 35%, flow rate: 80 mL/min, column temperature: 40°C) to give **Example 46A** (16 mg) and **Example 46B** (18 mg).

**Example 46A:** White solid (16 mg), SFC analysis(column: Chiralpak IH-3 50 × 4.6 mm I.D., 3 μm; mobile phase: carbon dioxide for phase A and 0.05% diethylamine/methanol for phase B; gradient: from 5% to 40% of B in 1.5 min and hold 40% of 1 min, then 5% of B for 0.5min; flow rate: 4 mL/min). The retention time on the chiral column was 1.475 min; LCMS (ESI): $[M+H]^+$=572.2. $^1$H NMR (400 MHz, DMSO-$d_6$) δ ppm 10.62 - 9.62 (m, 1H), 8.59 - 8.37 (m, 1H), 7.68 (d, *J* = 8.4 Hz, 1H), 7.42 - 7.32 (m, 2H), 7.30 - 7.17 (m, 3H), 7.07 (br s, 2H), 5.37 - 4.72 (m, 1H), 4.49 - 4.35 (m, 1H), 4.00 - 3.87 (m, 1H), 3.77 (t, *J* = 6.4 Hz, 2H), 3.29 - 3.16 (m, 2H), 3.13 - 3.05 (m, 1H), 2.86 - 2.78 (m, 1H), 2.45 - 2.38 (m, 1H), 2.26 (s, 3H), 2.07 - 1.51 (m, 6H), 1.21 - 1.03 (m, 2H)

**Example 46B:** White solid (18 mg), SFC analysis(column: Chiralpak IH-3 50 × 4.6 mm I.D., 3 μm; mobile phase: carbon dioxide for phase A and 0.05% diethylamine/methanol for phase B; gradient: from 5% to 40% of B in 1.5 min and hold 40% of 1 min, then 5% of B for 0.5min; flow rate: 4 mL/min). The retention time on the chiral column was 1.561 min; LCMS (ESI): $[M+H]^+$=572.2. $^1H$ NMR (400 MHz, DMSO-$d_6$) δ ppm 10.62 - 9.62 (m, 1H), 8.59 - 8.37 (m, 1H), 7.68 (d, $J$= 8.4 Hz, 1H), 7.42 - 7.32 (m, 2H), 7.30 - 7.17 (m, 3H), 7.07 (br s, 2H), 5.37 - 4.72 (m, 1H), 4.49 - 4.35 (m, 1H), 4.00 - 3.87 (m, 1H), 3.77 (t, $J$= 6.4 Hz, 2H), 3.29 - 3.16 (m, 2H), 3.13 - 3.05 (m, 1H), 2.86 - 2.78 (m, 1H), 2.45 - 2.38 (m, 1H), 2.26 (s, 3H), 2.07 - 1.51 (m, 6H), 1.21 - 1.03 (m, 2H)

**Example 47: N-($6^4$,$6^4$-difluoro-$5^1$-methyl-3-oxo-4-aza-5(4,2)-pyrimidin-6(1,3)-piperidin-1(1,3)-azetidin-2(1,2)-benzene-10(1,3)-cycl obutyramide-7-en-$2^5$-yl)-2-hydroxyethane-1-sulfonamide**

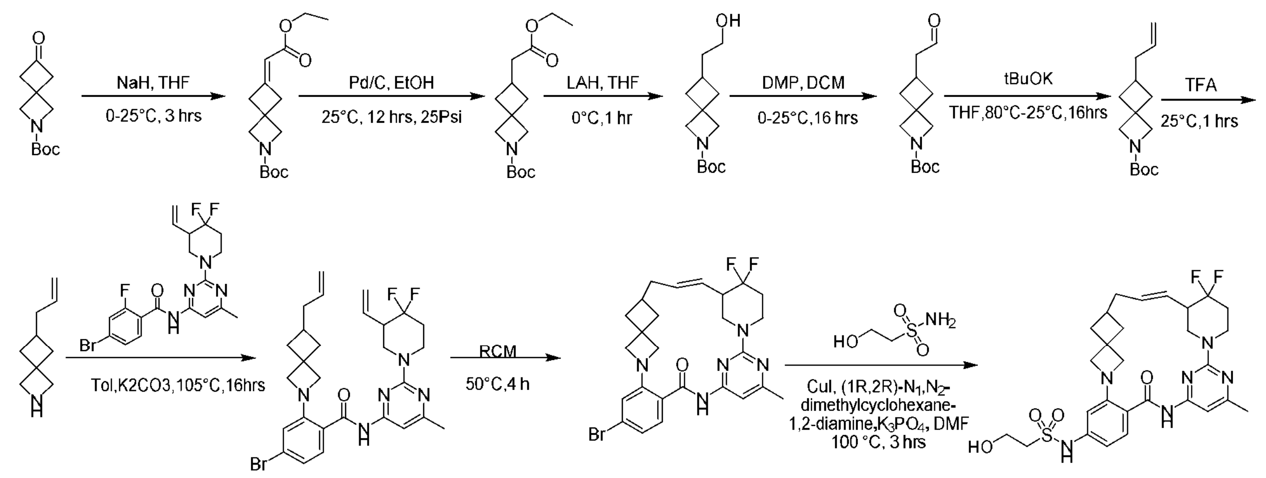


**Step 1:** To tetrahydrofuran (500 mL) were added triethyl phosphoacetate (5.47 g, 26.03 mmol) and sodium hydrogen (5.68 g, 142 mmol), and the mixture was stirred at 0°C for 1 h. To the reaction solution was added dropwise a solution of tert-butyl 6-oxo-2-azaspiro[3.3]heptane-2-carboxylate (25.0 g, 118 mmol) in tetrahydrofuran (20.0 mL), and the mixture was stirred at 25°C for 1 h under nitrogen atmosphere. The reaction mixture was diluted with water (500 mL), extracted with ethyl acetate (500 mL × 2), and the organic phase was dried, filtered and concentrated. The residue was purified by flash column chromatography (silica gel, 0-9% gradient of tetrahydrofuran/petroleum ether) to give tert-butyl 6-(2-ethoxy-2-oxoethylidene)-2-azaspiro[3.3]heptane-2-carboxylate (31.0 g, 110 mmol, yield: 93.0%) as a colorless oil. $^1H$ NMR (400 MHz, CHLOROFORM-d) δ ppm 4.25 - 4.01 (m, 3H), 3.99 - 3.90 (m, 2H), 3.85 - 3.75 (m, 1H), 3.33 - 3.19 (m, 1H), 3.09 - 2.97 (m, 1H), 2.53 - 2.41 (m, 1H), 2.38 - 2.27 (m, 1H), 1.45 (d, $J$ = 2.4 Hz, 9H), 1.36 - 1.21 (m, 3H)

**Step 2:** To a solution of tert-butyl 6-(2-ethoxy-2-oxoethylidene)-2-azaspiro[3.3]heptane-2-carboxylate in ethanol (300 mL) was added wet palladium on carbon (3.10 g), and the mixture was stirred at 25°C for 12 h under hydrogen atmosphere (25 Psi). The reaction solution was filtered, and the filtrate was collected. The filtrate was concentrated under reduced pressure to give tert-butyl 6-(2-ethoxy-2-oxoethyl)-2-azaspiro[3.3]heptane-2-carboxylate (23.0 g, 85.8 mmol, yield: 74%) as a colorless oil. $^1H$ NMR (400 MHz, CHLOROFORM-d) δ ppm 4.04 (q, $J$ = 7.0 Hz, 2H), 3.93 - 3.80 (m, 2H), 3.78 - 3.69 (m, 2H), 2.57 - 2.37 (m, 1H), 2.34 - 2.23 (m, 4H), 1.86 - 1.73 (m, 2H), 1.43 - 1.26 (m, 9H), 1.17 (t, $J$ = 7.0 Hz, 3H)

**Step 3:** To a solution of tert-butyl 6-(2-ethoxy-2-oxoethyl)-2-azaspiro[3.3]heptane-2-carboxylate (23.0 g, 85.8 mmol) in tetrahydrofuran (230 mL) was added dropwise 2.5 M lithium aluminum hydride solution in tetrahydrofuran (24.0 mL, 60.13 mmol). The mixture was stirred at 0°C for 1 h under nitrogen atmosphere. The reaction solution was quenched with water (24 mL), 15% aqueous sodium hydroxide (24 mL) and water (48 mL), dried, and filtered, and the filtrate was concentrated under reduced pressure to give tert-butyl 6-(2-hydroxyethyl)-2-azaspiro[3.3]heptane-2-carboxylate (20.0 g, crude product) as a colorless oil. $^1H$ NMR (400 MHz, CHLOROFORM-d) δ ppm 3.85 (s, 2H), 3.73 (s, 2H), 3.51 (t, $J$ = 6.4 Hz, 2H), 2.31 - 2.09 (m, 3H), 1.83 - 1.68 (m, 2H), 1.56 (q, $J$ = 6.8 Hz, 2H), 1.36 (s, 9H)

**Step 4:** To a solution of tert-butyl 6-(2-hydroxyethyl)-2-azaspiro[3.3]heptane-2-carboxylate (25.0 g, 104 mmol) in dichloromethane (500 mL) was added Dess-Martin periodinane (24.50 g, 57.1 mmol) at 0°C, and the mixture was stirred at 25°C for 16 h. The reaction mixture was diluted with water (100 mL), extracted with dichloromethane (500 mL × 2), and the organic phase was dried, filtered and concentrated. The residue was purified by flash column chromatography (silica gel, 0-9% gradient of tetrahydrofuran/petroleum ether) to give tert-butyl 6-(2-oxoethyl)-2-azaspiro[3.3]heptane-2-carboxylate (27.0 g,102 mmol, yield: 98.0%) as a colorless oil. $^1$H NMR (400 MHz, CHLOROFORM-d) δ ppm 9.72 (t, *J* = 1.6 Hz, 1H), 3.97 (s, 2H), 3.83 (s, 2H), 2.74 - 2.50 (m, 3H), 2.48 - 2.34 (m, 2H), 1.95 - 1.83 (m, 2H), 1.45 (s, 9H)

**Step 5:** A solution of methyl triphenylphosphine bromide (9.80 g, 40.9 mmol) and 1 M potassium tert-butoxide (81.90 mL, 81.9 mmol) in tetrahydrofuran (500 mL) was stirred at 80°C for 1 h, cooled to room temperature and added tert-butyl 6-(2-oxoethyl)-2-azaspiro[3.3]heptane-2-carboxylate (13.0 g, 54.3 mmol), and the mixture was stirred at 25°C for 16 h. The reaction mixture was diluted with water (100 mL), extracted with ethyl acetate (500 mL × 2), and the organic phase was dried, filtered and concentrated. The residue was purified by flash column chromatography (silica gel, 0-20% gradient of tetrahydrofuran/petroleum ether) to give tert-butyl 6-(prop-2-en-1-yl)-2-azaspiro[3.3]heptane-2-carboxylate (9.7 g, 40.0 mmol, yield: 74.0%) as a colorless oil. $^1$H NMR (400 MHz, DMSO-$d_6$) δ ppm 5.72 - 5.55 (m, 1H), 5.01 - 4.81 (m, 2H), 3.80 - 3.60 (m, 4H), 2.25 - 1.92 (m, 5H), 1.75 - 1.64 (m, 2H), 1.29 (s, 8H)

**Step 6:** To a mixed solution of trifluoroformic acid (8.00 mL) and dichloromethane (32.0 mL) was added 6-(prop-2-en-1-yl)-2-azaspiro[3.3]heptane-2-carboxylate (4.00 g, 16.8 mmol), and the mixture was stirred at 25°C for 1 h. The reaction solution was concentrated under reduced pressure to give 6-(prop-2-en-1-yl)-2-azaspiro[3.3]heptane trifluoroacetate (4.0 g, yield: 94%) as a yellow oil. $^1$H NMR (400 MHz, DMSO-$d_6$) δ ppm 8.65 (br d, *J* = 1.2 Hz, 2H), 5.16 - 4.84 (m, 2H), 4.13 - 3.77 (m, 4H), 2.42 - 2.24 (m, 2H), 2.22 - 2.01 (m, 3H), 1.94 - 1.74 (m, 2H)

**Step 7:** To a solution of 4-bromo-N-[2-(3-vinyl-4,4-difluoropiperidin-1-yl)-6-methylpyrimidin-4-yl]-2-fluorobenzamide (1.00 g, 2.20 mmol) in anhydrous toluene (20.0 mL) were added potassium carbonate (0.93 g, 6.59 mmol) and 6-(prop-2-en-1-yl)-2-azaspiro[3.3]heptanetrifluoroacetate (0.83 g, 3.29 mmol), and the mixture was stirred at 105°C for 16 h. The reaction mixture was diluted with water (10 mL), extracted with ethyl acetate (20 mL × 2), and the organic phase was dried, filtered and concentrated. The residue was purified by flash column chromatography (silica gel, 0-11% gradient of tetrahydrofuran/petroleum ether) to give 2-(6-allyl-2-azaspiro[3.3]heptane-2-yl)-4-bromo-N-(2-(4,4-difluoro-3-vinylpiperidin-1-yl)-6-methylpyrimidin-4-yl)benzami de (600.00 mg, 1.05 mmol, yield: 48.0%) as a yellow oil. LCMS (ESI): $[M+H]^+$ = 574.1

**Step 8:** To a solution of 2-(6-allyl-2-azaspiro[3.3]heptane-2-yl)-4-bromo-N-(2-(4,4-difluoro-3-vinylpiperidin-1-yl)-6-methylpyrimidin-4-yl)benzami de (600.00 mg, 1.05 mmol) in dichloromethane (240 mL) was added Grubbs catalyst 2nd generation (89.0 mg, 0.10 mmol), and the mixture was stirred at 50°C for 4 h under nitrogen atmosphere. The reaction mixture was concentrated under reduced pressure. The residue was purified by flash column chromatography (silica gel, 0-5% gradient of tetrahydrofuran/petroleum ether) to give $2^5$ bromo-$6^4$,$6^4$-difluoro-$5^6$-methyl-4-aza-5(4,2)-pyrimidin-6(1,3)-piperidin-1(1,3)-azetidin-2(1,2)-benzene-10(1,3)-cyclobutyra mide-7-en-3-one (200 mg, 0.38 mmol, yield: 35.0%) as a yellow solid. LCMS (ESI): $[M+H]^+$ = 546.1

**Step 9:** To a solution of $2^5$ bromo-$6^4$,$6^4$-difluoro-$5^6$-methyl-4-aza-5(4,2)-pyrimidin-6(1,3)-piperidin-1(1,3)-azetidin-2(1,2)-benzene-10(1,3)-cyclobutyra mide-7-en-3-one (62.1 mg, 0.50 mmol) in N,N-dimethylformamide (6.00 mL) were added potassium phosphate (211 mg, 0.99 mmol), (1R,2R)-N1,N2-dimethylcyclohexane-1,2-diamine (47.5 mg, 0.33 mmol) and cuprous iodide (63.0 mg, 0.33 mmol), and the mixture was stirred at 100°C for 3 h under nitrogen atmosphere. The reaction mixture was diluted with water (50.0 + 0.5 mL of aqueous ammonia) and extracted with ethyl acetate (100 mL × 3); the organic phase was dried, filtered, and concentrated. The residue was purified by preparative HPLC (C18, 34-74% gradient of water (aqueous ammonia)/acetonitrile) to give
(E)-N-(6',6'-difluoro-$5^6$-methyl-3-oxo-4-aza-5(4,2)-pyrimidin-6(1,3)-piperidin-1(1,3)-azetidin-2(1,2)-benzene-10(1,3)-cycl obutyramide-7-en-$2^5$-yl)-2-hydroxyethane-1-sulfonamide (29.0 mg, 0.05 mmol, yield: 15.0%) as a white solid. LCMS (ESI): $[M+H]^+$ = 589.3. $^1$H NMR (400 MHz, DMSO-$d_6$) δ ppm 10.13 (s, 1H), 10.04 - 9.66 (m, 1H), 7.32 (d, *J* = 8.4 Hz, 1H), 6.92 (s, 1H), 6.60 (br d, *J* = 8.8 Hz, 1H), 6.31 (s, 1H), 5.69 - 5.56 (m, 1H), 5.34 (br dd, J= 9.0, 14.4 Hz, 1H), 5.13 - 4.70 (m, 1H), 4.24 - 3.84 (m, 4H), 3.82 - 3.69 (m, 5H), 3.64 (br d, *J* = 7.6 Hz, 1H), 3.26 (br t, *J*= 6.8 Hz, 2H), 2.76 - 2.58 (m, 1H), 2.39 (br d, J= 9.0 Hz, 2H), 2.31 (s, 3H), 2.26 - 2.17 (m, 1H), 2.13 - 1.63 (m, 6H)

**Example 48: N-($1^4$,$1^4$-difluoro-$2^6$-methyl-4-oxo-3-aza-2(2,4)-pyrimidin-6(2,5)-pyridin-1(1,3)-piperidin-5(1,2)-phenylheterocyclono n-7-en-$5^4$-yl)-2-hydroxyethane-1-sulfonamide**

**Example 49:** ***N-($1^4$,$1^4$-difluoro-$2^6$-methyl-4-oxo-3-aza-2(2,4)-pyrimidin-6(2,5)-pyridin-1(1,3)-piperidin-5(1,2)-phenylheterocyclono*** **nane-$5^4$-yl)-2-hydroxyethane-1-sulfonamide**

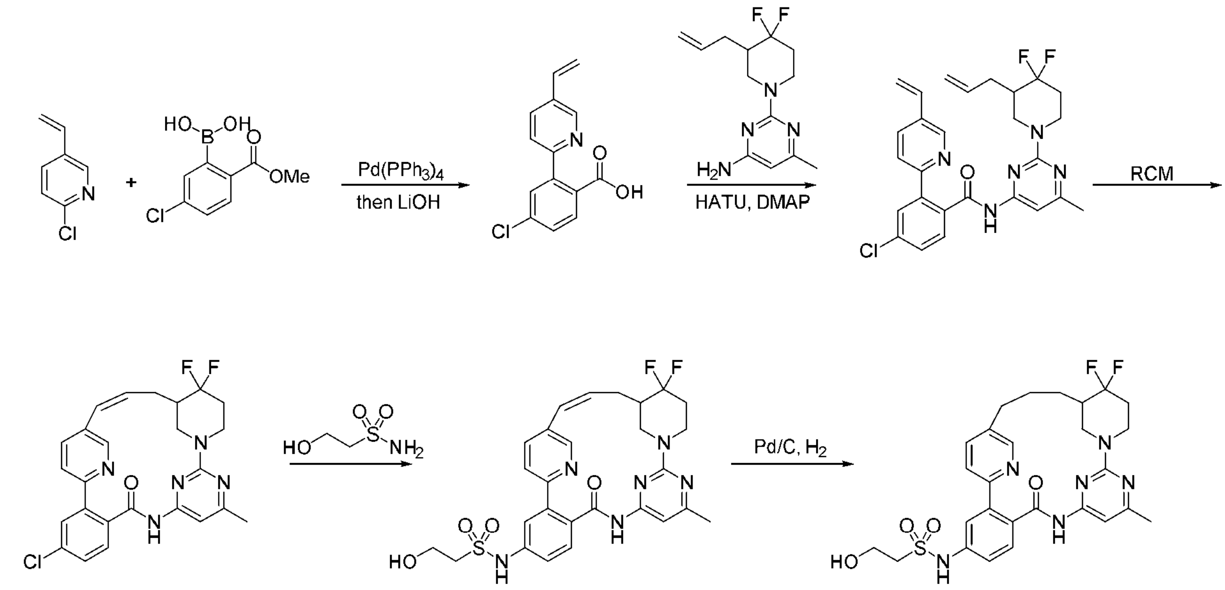


**Step 1:** A solution of 5-vinyl-2-chloropyridine (800 mg, 5.60 mmol), (5-chloro-2-(methoxycarbonyl)phenyl)boric acid (1.00 g, 4.66 mmol), tetrakis(triphenylphosphine)palladium (275 mg, 0.23 mmol) and potassium carbonate (1.32 g, 9.33 mmol) in 1,4-dioxane (20.00 mL) was stirred at 80°C for 3 h under nitrogen atmosphere, cooled to room temperature, and extracted with ethyl acetate (200 mL × 3). The organic phases were combined, dried, filtered, and concentrated to give a crude product. The crude product was purified by flash column chromatography (silica, 0-10% dichloromethane/methanol) to give 2-(5-vinylpyridin-2-yl)-4-chlorobenzoic acid (500 mg, 1.93 mmol, yield: 41.4%) as a yellow solid. LCMS (ESI): $[M+H]^+$ = 260.00.

**Step 2:** To dichloroethane (20.00 mL) were added 2-(4,4-difluoro-3-allylpiperidin-1-yl)-6-methylpyrimidin-4-amine (516 mg, 1.93 mmol), 2-(5-vinylpyridin-2-yl)-4-chlorobenzoic acid (500 mg, 1.93 mmol), N,N,N',N'-tetramethyl-O-(7-azabenzotriazol-1-yl)urea hexafluorophosphate (2.24 g, 5.78 mmol) and 4-dimethylaminopyridine (960 mg, 7.70 mmol) at 20°C, and the mixture was heated to 80°C and stirred for 24 h. After the reaction was complete, the mixture was quenched with sodium bicarbonate solution (50 mL) and extracted with dichloromethane (50 mL × 3), and the organic phases were combined, dried, filtered, and concentrated to give the crude product. The crude product was purified by flash column chromatography (silica, 0-20% gradient of ethyl acetate/petroleum ether) to give 2-(5-vinylpyridin-2-yl)-4-chloro-N-(2-(4,4-difluoro-3-allylpiperidin-1-yl)-6-methylpyrimidin-4-yl)benzamide (140 mg, 0.27 mmol, yield: 14.2%) as a colorless oil. LCMS (ESI): $[M+H]^+$ = 510.20.

**Step 3:** Under nitrogen atmosphere at 20°C, 2-(5-vinylpyridin-2-yl)-4-chloro-N-(2-(4,4-difluoro-3-allylpiperidin-1-yl)-6-methylpyrimidin-4-yl)benzamide (140 gm, 0.27 mmol) was dissolved in dry dichloroethane (120.00 mL), Grubbs catalyst $2^{nd}$ generation (25 mg, 0.03 mmol) was added and the reaction was heated to reflux for 16 hours. After the reaction was complete, the concentrated reaction solution was purified by flash column chromatography (silica gel, 0-20% gradient of ethyl acetate/petroleum ether) to give $5^4$-chloro-$1^4$,$1^4$-difluoro-$2^6$-methyl-3-aza-2(2,4)-pyrimidin-6(2,5)-pyridin-1(1,3)-piperidin-5(1,2)-phenylheterocyclonon-7-e n-4-one (50 mg, 0.10 mmol, yield: 38.4%) as a white solid. LCMS (ESI): $[M+H]^+$ = 482.10.

**Step 4:** Under nitrogen atmosphere at 20°C, $5^4$-chloro-$1^4$,$1^4$-difluoro-$2^6$-methyl-3-aza-2(2,4)-pyrimidin-6(2,5)-pyridin-1(1,3)-piperidin-5(1,2)-phenylheterocyclonon-7-e n-4-one (50 mg, 0.10 mmol), 2-hydroxyethylsulfonamide (39 mg, 0.31 mmol), potassium phosphate (87 mg, 0.41 mmol), cuprous iodide (29 mg, 0.15 mmol) and trans-N,N'-dimethyl-1,2-cyclohexanediamine (44 mg, 0.31 mmol) were dissolved in N,N-dimethylformamide (5.00 mL). The resulting reaction mixture was stirred at 100°C for 16 hours. After the reaction was complete, the mixture was quenched with cold water (20 mL) and extracted with ethyl acetate (20 mL × 3), and the organic phases were combined, washed with sodium chloride solution (100 mL), dried with magnesium sulfate, filtered, and concentrated to give the crude product. The crude product was purified by flash column chromatography (silica, 50-70% gradient of ethyl acetate/petroleum ether) to give N-($1^4$,$1^4$-difluoro-$2^6$-methyl-4-oxo-3-aza-2(2,4)-pyrimidin-6(2,5)-pyridin-1(1,3)-piperidin-5(1,2)-phenylheterocyclonon-7-e n-$5^4$-yl)-2-hydroxyethane-1-sulfonamide (25 mg, 0.044 mmol, yield: 42.2%) as a white solid. LCMS (ESI): $[M+H]^+$ = 571.20, $^1H$ NMR (400 MHz, DMSO-$d_6$) δ ppm 10.24 (s, 1H), 9.90 (s, 1H), 8.53 (s, 1H), 7.75 (d, $J$ = 8.6 Hz, 2H), 7.55 (d, $J$ = 7.7 Hz, 1H), 7.43 - 7.35 (m, 2H), 7.35 - 7.08 (m, 1H), 6.52 (d, $J$ = 11.3 Hz, 1H), 6.09 - 5.92 (m, 1H), 4.96 (t, $J$ = 5.6 Hz, 1H), 3.94 - 3.43 (m, 6H), 3.38 - 3.36 (m, 2H), 2.35 - 2.21 (m, 1H), 2.27 (s, 3H), 2.17 - 1.89 (m, 2H), 1.86 - 1.66 (m, 2H).

**Step 5:** To methanol (5.00 mL) were added *N-($1^4$,$1^4$-difluoro-$2^6$-methyl-4-oxo-3-aza-2(2,4)-pyrimidin-6(2,5)-pyridin-1(1,3)-piperidin-5(1,2)-phenylheterocyclonon-7-e* n-$5^4$-yl)-2-hydroxyethane-1-sulfonamide (20 mg,

0.035 mmol) and 10% wet palladium on carbon (20 mg) at 20°C. To the solution was added formic acid (0.16 g, 3.52 mmol), followed by three hydrogen replacements, and stirred at 20°C for 24 h. The reaction was complete. The solution was concentrated under reduced pressure and purified by flash column chromatography (silicon dioxide, 30-95% gradient of ethyl acetate/petroleum ether) to give *N-(1$^4$,1$^4$-difluoro-2$^6$-methyl-4-oxo-3-aza-2(2,4)-pyrimidin-6(2,5)-pyridin-1(1,3)-piperidin-5(1,2)-phenylheterocyclononane*-5$^4$-yl)-2-hydroxyethane-1-sulfonamide (10 mg, 0.017 mmol, yield: 49.9%) as a white solid. LCMS (ESI): $[M+H]^+$ = 573.20, $^1H$ NMR (400 MHz, DMSO-$d_6$) $\delta$ ppm 10.17 (s, 1H), 9.14 (s, 1H), 8.23 (s, 1H), 7.76 - 7.69 (m, 1H), 7.63 (d, *J* = 8.4 Hz, 1H), 7.55 - 7.45 (m, 2H), 7.35 (dd, *J* = 8.4, 2.2 Hz, 1H), 7.31 - 7.16 (m, 1H), 4.97 (s, 1H), 4.52 - 4.29 (m, 1H), 4.07 - 3.89 (m, 1H), 3.86 - 3.70 (m, 2H), 3.38 - 3.32 (m, 2H), 3.31 - 3.04 (m, 2H), 2.85 - 2.74 (m, 1H), 2.48 - 2.37 (m, 1H), 2.26 (s, 3H), 2.07 - 1.70 (m, 4H), 1.67 - 1.49 (m, 1H), 1.18 - 0.97 (m, 2H)

**Example 50: *N-(1$^4$,1$^4$-difluoro-2$^6$-methoxy-4-oxo-3-aza-2(2,4)-pyrimidin-1(1,3)-piperidin-5(1,2),6(1,4)-diphenyl-heterocyclonon-8-e* n-5$^4$-yl)-2-hydroxyethane-1-sulfonamide**

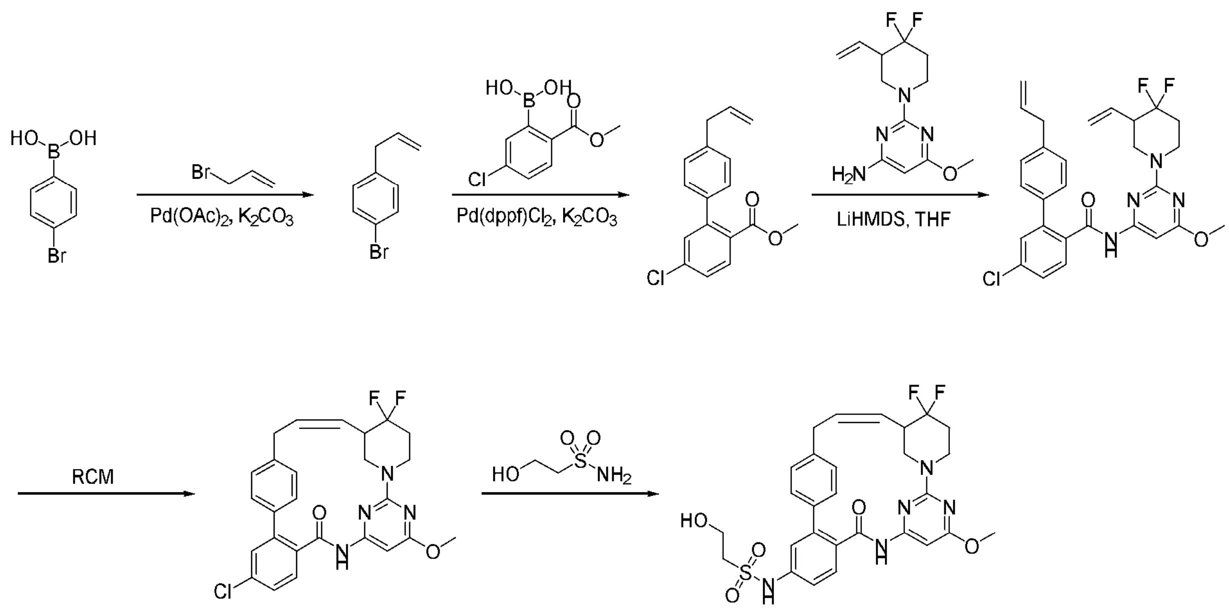


**Step** 1: A solution of (4-bromophenyl)boric acid (5.00 g, 24.90 mmol), allyl bromide (6.00 g, 49.79 mmol), potassium carbonate (10.50 g, 74.69 mmol), palladium acetate (570 mg, 2.49 mmol) in toluene (50.00 mL) was stirred at 80°C for 12 h under nitrogen atmosphere, diluted with water (50 mL), and extracted with ethyl acetate (50 mL $\times$ 3). The organic phase was dried over anhydrous sodium sulfate, filtered, concentrated, and purified by column chromatography (silica gel, 0-100% gradient of ethyl acetate/petroleum ether) to give 1-allyl-4-bromobenzene (2.60 g, 13.19 mmol, yield: 53.0%) as a colorless oil.

**Step 2:** A solution of 1-allyl-4-bromobenzene (2.60 g, 13.19 mmol), (5-chloro-2-(methoxycarbonyl)phenyl) phenyl)boric acid (4.20 g, 19.79 mmol), potassium carbonate (5.60 g, 39.58 mmol) and 1,1'-bis(diphenylphosphino) ferrocene palladium dichloride (II) (999 mg, 1.32 mmol) in 1,4-dioxane (40.00 mL) and water (10.00 mL) was stirred at 80 °C for 12 h under nitrogen atmosphere, diluted with water (50 mL), and extracted with ethyl acetate (50 mL $\times$ 3). The organic layer was dried over anhydrous sodium sulfate, filtered, concentrated, and purified by column chromatography (silica gel, 0-100% gradient of ethyl acetate/petroleum ether) to give 4'-allyl-5-chloro-[1,1'-biphenyl]-2-carboxylate (1.80 g, 6.28 mmol, yield: 47.6%) as a yellow oil. LCMS (ESI): $[M+H]^+$ = 287.00.

**Step 3:** To a solution of 2-(4,4-difluoro-3-vinylpiperidin-1-yl)-6-methoxypyrimidin-4-amine (1.90 g, 6.91 mmol) in tetrahydrofuran (20.00 mL) was added dropwise lithium bis(trimethylsilyl)amide (10 mL, 1.0 M), and the mixture was stirred at 0°C for 30 min under nitrogen atmosphere. To the reaction solution was added dropwise 4'-allyl-5-chloro-[1,1'-biphenyl]-2-carboxylate (1.80 g, 6.28 mmol). The reaction solution was stirred at 20°C for 1 h, quenched with saturated ammonium chloride (50 mL) and extracted with ethyl acetate (30 mL $\times$ 3), and the organic phase was dried over anhydrous sodium sulfate, filtered, concentrated, and purified by column chromatography (silica gel, 0-30% gradient of ethyl acetate/petroleum ether) to give 4'-allyl-5-chloro-N-(2-(4,4-difluoro-3-vinylpiperidin-1-yl)-6-methoxypyrimidin-4-yl)-[1,1'-biphenyl]-2-carboxamide (1.30 g, 2.48 mmol, yield: 39.4%) as a white solid. LCMS (ESI): $[M+H]^+$ = 525.20.

**Step 4:** A solution of 4'-allyl-5-chloro-N-(2-(4,4-difluoro-3-vinylpiperidin-1-yl)-6-methoxypyrimidin-4-yl)-[1,1'-biphenyl]-2-carboxamide (1.30 g, 2.48 mmol) and Grubbs catalyst 2nd generation (214 mg, 0.25 mmol) in dichloromethane (300.00 mL) was stirred at 50°C for 2 h. The reaction solution was concentrated and purified by column chromatography (silica gel, 0-30% gradient of ethyl acetate/petroleum ether) to give $5^4$-chloro-$1^4$,$1^4$-difluoro-$2^6$-methoxy-3-aza-2(2,4)-pyrimidin-1(1,3)-piperidin-5(1,2),6(1,4)-diphenylheterocyclonon-8-en-4-one (1.10 g, 2.21 mmol, yield: 89.4%) as a gray solid. LCMS (ESI): $[M+H]^+$ = 497.20.

**Step 5:** A solution of $5^4$-chloro-$1^4$,$1^4$-difluoro-$2^6$-methoxy-3-aza-2(2,4)-pyrimidin-1(1,3)-piperidin-5(1,2),6(1,4)-diphenylheterocyclonon-8-en-4-one (1.00 g, 2.01 mmol), 2-hydroxyethanesulfonamide (509 mg, 4.02 mmol), potassium phosphate (1.30 g, 6.04 mmol), 2-di-tert-butylphosphino-2',4',6'-triisopropylbiphenyl (85 mg, 0.207 mmol) and tris(dibenzylideneacetone)dipalladium (188 mg, 0.20 mmol) in dioxane (10.00 mL) was stirred at 100°C for 2 h under nitrogen atmosphere, diluted with water (30 mL), and extracted with ethyl acetate (30 mL × 3). The organic phase was dried over anhydrous sodium sulfate, filtered, concentrated, and purified by column chromatography (silica gel, 0-100% gradient of ethyl acetate/petroleum ether) to give N-($1^4$,$1^4$-difluoro-$2^6$-methoxy-4-oxo-3-aza-2(2,4)-pyrimidin-1(1,3)-piperidin-5(1,2),6(1,4)-diphenylheterocyclonon-8-en-$5^4$-yl)-2-hydroxyethane-1-sulfonamide (800 mg, 1.37 mmol, yield: 67.9%) as a gray solid. LCMS (ESI): $[M+H]^+$ = 586.20.

### Example 51A-Example 51B: Two isomers of N-($1^4$,$1^4$-difluoro-$2^6$-methoxy-4-oxo-3-aza-2(2,4)-pyrimidin-1(1,3)-piperidin-5(1,2),6(1,4)-dibenzylcyclononane-$5^4$-yl)-2-hydroxyethane-1-sulfonamide

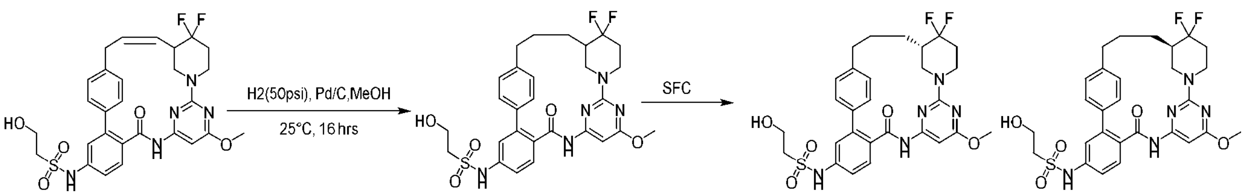


**Step 1:** To a solution of N-($1^4$,$1^4$-difluoro-$2^6$-methoxy-4-oxo-3-aza-2(2,4)-pyrimidin-1(1,3)-piperidine-5(1,2),6(1,4)-dibenzylcyclononane-8-en-$5^4$-yl) -2-hydroxyethane-1-sulfonamide (530 mg, 0.91 mmol) in methanol (30.0 mL) was added 10% Pd/C (wet) (300 mg, 0.01 mmol), and the reaction mixture was purged with argon three times, followed by three cycles of hydrogen.and stirred at 25°C for 16 h under hydrogen atmosphere (50 Psi). The reaction mixture was filtered under reduced pressure to remove palladium/carbon and then concentrated in vacuum. The residue was purified by preparative HPLC (C18, 36-76% gradient of water (aqueous ammonia + ammonium bicarbonate)/acetonitrile) to give N-($1^4$,$1^4$-difluoro-$2^6$-methoxy-4-oxo-3-aza-2(2,4)-pyrimidin-1(1,3)-piperidin-5(1,2),6(1,4)-dibenzylcyclononane-$5^4$-yl)-2-hy droxyethane-1-sulfonamide (76.86 mg, 0.13 mmol, yield: 14.0%) as a white solid. LCMS (ESI): $[M+H]^+$ = 588.3. $^1$H NMR (400 MHz, DMSO-$d_6$) δ ppm 10.19 (br s, 1H), 8.53 (br s, 1H), 7.69 (d, *J*= 8.4 Hz, 1H), 7.36 (br s, 2H), 7.31 (dd, *J*= 2.0, 8.4 Hz, 1H), 7.26 (d, *J* = 2.0 Hz, 1H), 7.07 (br s, 2H), 6.76 (br s, 1H), 5.27 - 4.70 (m, 1H), 4.38 (br d, *J* = 13.2 Hz, 1H), 3.94 (br d, *J* = 12.8 Hz, 1H), 3.82 (s, 3H), 3.78 (t, *J*= 6.8 Hz, 2H), 3.35 (t, *J* = 6.4 Hz, 2H), 3.21 (br t, *J* = 10.4 Hz, 1H), 3.08 (br d, *J* = 12.0 Hz, 1H), 2.89 - 2.78 (m, 1H), 2.47 - 2.38 (m, 1H), 2.06 - 1.85 (m, 3H), 1.82 - 1.56 (m, 2H), 1.23 - 1.02 (m, 2H)

The above products were separated by SFC (column: DAICEL CHIRALPAK AD (250 mm×30 mm, 10 μm), mobile phase: $CO_2$-EtOH (0.1% $NH_3 \cdot H_2O$), gradient: isocratic 40%, flow rate: 80 mL/min, column temperature: 40°C) to give two stereoisomers.

**Example 51A:** White solid (25.62 mg). SFC analysis (column: Chiralpak AD-3 50 × 4.6 mm I.D., 3 μm; mobile phase: carbon dioxide for phase A and ethanol (0.05% ethylene glycol) for phase B; gradient: 40% for phase B; flow rate: 4 mL/min). LCMS (ESI): $[M+H]^+$ = 588.3. $^1$H NMR (400 MHz, DMSO-$d_6$) δ ppm 10.19 (s, 1H), 8.54 (br s, 1H), 7.69 (d, *J*= 8.4 Hz, 1H), 7.37 (br d, *J*= 2.0 Hz, 2H), 7.31 (dd, *J*= 2.0, 8.4 Hz, 1H), 7.26 (d, *J* = 2.0 Hz, 1H), 7.07 (br s, 2H), 6.77 (br s, 1H), 5.22 - 4.73 (m, 1H), 4.38 (br d, *J* = 13.2 Hz, 1H), 3.94 (br d, *J* = 12.4 Hz, 1H), 3.82 (s, 3H), 3.78 (t, *J* = 6.8 Hz, 2H), 3.38 - 3.34 (m, 2H), 3.21 (br t, *J* = 10.4 Hz, 1H), 3.08 (br d, *J*= 12.0 Hz, 1H), 2.91 - 2.78 (m, 1H), 2.48 - 2.38 (m, 1H), 2.07 - 1.75 (m, 4H), 1.64 (br d, *J* = 2.8 Hz, 1H), 1.22 - 1.01 (m, 2H)

**Example 51B:** White solid (23.86 mg). SFC analysis (column: Chiralpak AD-3 50 x 4.6 mm I.D., 3 μm; mobile phase: carbon dioxide for phase A and ethanol (0.05% ethylene glycol) for phase B; gradient: 40% for phase B; flow rate: 4 mL/min). LCMS (ESI): $[M+H]^+$ = 588.3. $^1$H NMR (400 MHz, DMSO-$d_6$) δ ppm 8.49 (br s, 1H), 7.68 (d, *J*= 8.0 Hz, 1H), 7.37 (br s, 2H), 7.28 (dd, *J*= 2.0, 8.4 Hz, 1H), 7.23 (d, *J* = 1.6 Hz, 1H), 7.07 (br s, 2H), 6.76 (br s, 1H), 4.38 (br d, *J= 13.6* Hz, 1H), 3.94 (br d, *J* = 12.8 Hz, 1H), 3.82 (s, 3H), 3.77 (t, *J* = 6.8 Hz, 2H), 3.35 (br s, 2H), 3.21 (br t, *J* = 10.4 Hz, 1H), 3.08 (br d, *J*= 12.0 Hz, 1H), 2.89 - 2.79 (m, 1H), 2.46 - 2.40 (m, 1H), 2.03 - 1.76 (m, 4H), 1.64 (br d, *J* = 3.2 Hz, 1H), 1.20 - 1.04 (m, 2H)

**Example 52:** ***N*-(1⁴,1⁴-difluoro-2⁶-methyl-6²,4-dioxo-6¹,6²-dihydro-3-aza-2(2,4)-pyrimidin-6(1,4)-pyridin-1(1,3)-piperidin-5(1,2)-ph enylheterocyclononane-5⁴-yl)-2-hydroxyethane-1-sulfonamide**

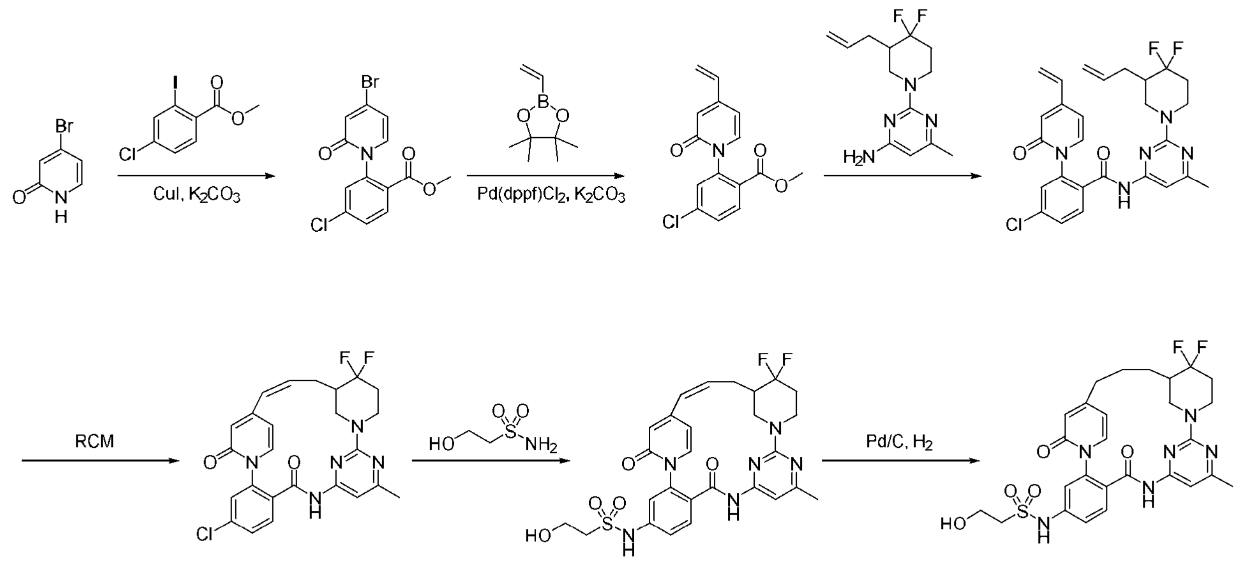


**Step 1:** A solution of 4-bromopyridin-2(1*H*)-one (5.00 g, 28.74 mmol), 4-chloro-2-iodo-benzoic acid methyl ester (12.80 g, 43.10 mmol), potassium carbonate (12.20 g, 86.21 mmol) and cuprous iodide (8.40 g, 43.10 mmol) in *N,N*-dimethylformamide (50.00 mL) was stirred at 100°C for 12 h under nitrogen atmosphere, diluted with water (100 mL), and extracted with ethyl acetate (50 mL × 3). The organic phase was dried over anhydrous sodium sulfate, filtered, concentrated, and purified by column chromatography (silica gel, 0-100% gradient of ethyl acetate/petroleum ether) to give 2-(4-bromo-2-oxopyridin-1(2*H*)-yl)-4-chlorobenzoate (900 mg, 2.63 mmol, yield: 9.1%) as colorless oil. LCMS (ESI): $[M+H]^+$ = 341.90.

**Step 2:** A solution of 2-(4-bromo-2-oxopyridin-1(2*H*)-yl)-4-chlorobenzoate (900 mg, 2.63 mmol), 4,4,5,5-tetramethyl-2-vinyl-1,3,2-dioxopentane (607 mg, 3.94 mmol), potassium carbonate (1.10 g, 7.88 mmol) and 1,1'-bis(diphenylphosphino)ferrocene palladium dichloride (II) (199 mg, 0.26 mmol) in dioxane (15.00 mL) and water (3.00 mL) was stirred at 80°C for 12 h under nitrogen atmosphere, diluted with water (50 mL) and extracted with ethyl acetate (50 mL × 3). The organic layer was dried over anhydrous sodium sulfate, filtered, concentrated, and purified by column chromatography (silica gel, 0-100% gradient of ethyl acetate/petroleum ether) to give 4-chloro-2-(2-oxo-4-vinylpyridin-1(2H)-yl) methyl benzoate (700 mg, 2.42 mmol, yield: 92.0%) as a white solid. LCMS (ESI): $[M+H]^+$ = 290.00.

**Step 3:** To a solution of 2-(4,4-difluoro-3-allylpiperidin-1-yl)-6-methylpyrimidin-4-amine (778 mg, 2.90 mmol) in tetrahydrofuran (10 mL) was added dropwise lithium bis(trimethylsilyl)amide (3.60 mL, 1.0 M), and the mixture was stirred at 0°C for 30 min under nitrogen atmosphere. To the reaction solution was added dropwise 4-chloro-2-(2-oxo-4-vinylpyridin-1(2H)-yl)methyl benzoate (700 mg, 2.42 mmol). The reaction solution was stirred at 20°C for 12 h, diluted with water (50 mL) and extracted with ethyl acetate (50 mL × 3), and the organic phase was dried over anhydrous sodium sulfate, filtered, concentrated, and purified by column chromatography (silica gel, 0-100% gradient of ethyl acetate/petroleum ether) to give *N*-(2-(3-allyl-4,4-difluoropiperidin-1-yl)-6-methylpyrimidin-4-yl)-4-chloro-2-(2-oxo-4-vinylpyridin-1(2H)-yl)benzamide (300 mg, 0.57 mmol, yield: 23.6%) as a yellow oil. LCMS (ESI): $[M+H]^+$ = 526.20.

**Step 4:** A solution of *N*-(2-(3-allyl-4,4-difluoropiperidin-1-yl)-6-methylpyrimidin-4-yl)-4-chloro-2-(2-oxo-4-vinylpyridin-1(2H)-yl)benzamide (300 mg, 0.57 mmol) and Grubbs catalyst 2nd generation (49 mg, 0.21 mmol) in dichloromethane (60.00 mL) was stirred at 50°C for 2 h, and the reaction solution was concentrated and purified by column chromatography (silica gel, 0-30% gradient of ethyl acetate/petroleum ether) to give 5⁴-chloro-1⁴,1⁴-difluoro-2⁶-methyl-6¹,6²-dihydro-3-aza-2(2,4)-pyrimidin-6(1,4)-pyridin-1(1,3)-piperidin-5(1,2)-phenylheter ocyclonon-7-en-6²,4-dione (200 mg, 0.40 mmol, yield: 70.4%) as a yellow oil. LCMS (ESI): $[M+H]^+$ = 498.20.

**Step 5:** A solution of 5⁴-chloro-1⁴,1⁴-difluoro-2⁶-methyl-6',6'-dihydro-3-aza-2(2,4)-pyrimidin-6(1,4)-pyridin-1(1,3)-piperidin-5(1,2)-benzenecycl on-7-en-6²,4-dione (200 mg, 0.40 mmol), 2-hydroxyethanesulfonamide (102 mg, 0.80 mmol), potassium phosphate (261 mg, 1.21 mmol), 2-di-tert-butylphosphino-2',4',6'-triisopropylbiphenyl (17 mg, 0.040 mmol) and tris(dibenzylideneacetone)dipalladium (38 mg, 0.040 mmol) in dioxane (5.00 mL) was stirred at 100°C for 12 h under nitrogen atmosphere, diluted with water (30 mL), and extracted with ethyl acetate (30mL × 3), and the organic phase was dried over anhydrous sodium sulfate, filtered, concentrated, and purified by column chromatography (silica gel,

0-100% gradient of ethyl acetate/petroleum ether) to give *N*-($1^4$,$1^4$-difluoro-$2^6$-methyl-$6^2$,4-dioxo-$6^1$,$6^2$-dihydro-3-aza-2(2,4)-pyrimidin-6(1,4)-pyridin-1(1,3)-piperidin-5(1,2)-phenylh eterocyclonon-7-en-$5^4$-yl)-2-hydroxyethane-1-sulfonamide (100 mg, 0.17 mmol, yield: 42.7%) as a yellow solid. LCMS (ESI): $[M+H]^+$ =587.20.

**Step 6:** A solution of *N*-($1^4$,$1^4$-difluoro-$2^6$-methyl-$6^1$,4-dioxo-$6^1$,$6^2$-dihydro-3-aza-2(2,4)-pyrimidin-6(l,4)-pyridin-1(1,3)-piperidin-5(1,2)-phenylh eterocyclonon-7-en-$5^4$-yl)-2-hydroxyethane-1-sulfonamide (100 mg, 0.17 mmol) and wet palladium on carbon (363 mg, 0.34 mmol) in tetrahydrofuran (10 mL) was stirred at 25°C for 12 h under hydrogen atmosphere (15 Psi), and the reaction solution was filtered, concentrated, and purified by column chromatography (C18, 0-100% gradient of acetonitrile/water) to give N-($1^4$,$1^4$-difluoro-$2^6$-methyl-$6^2$,4-dioxo-$6^1$,$6^2$-dihydro-3-aza-2(2,4)-pyrimidin-6(1,4)-pyridin-1(1,3)-piperidin-5(1,2)-phenylh eterocyclononane-$5^4$-yl)-2-hydroxyethane-1-sulfonamide (9.0 mg, 0.015 mmol, yield: 9.0%) as a white solid. $^1$H NMR (400 MHz, DMSO-$d_6$) $\delta$ 10.41 (s, 1H), 9.73 (s, 1H), 8.02 - 7.85(m, 2H), 7.39 - 7.16 (m, 4H), 7.03 - 6.90 (m, 1H), 4.98 (s, 1H), 4.47 - 4.12 (m, 2H), 3.83 - 3.71 (m, 2H), 3.49 - 3.36 (m, 4H), 2.88 - 2.72 (m, 1H), 2.69 - 2.56 (m, 1H), 2.24 (s, 3H), 2.14 - 1.83 (m, 4H), 1.81 - 1.67 (m, 1H), 1.45 - 1.30 (m, 1H), 1.20 - 1.04 (m, 1H) ppm; LCMS (ESI): $[M+H]^+$ =589.30

**Example 53: 2-Hydroxy-N-($1^4$,$1^4$,$5^5$-trifluoro-$2^6$-methyl-4-oxo-3-aza-2(2,4)-pyrimidin-1(1,3)-piperidin-5(1,2),6(1,4)-diphenylhetero cyclononane-$5^4$-yl)ethane-1-sulfonamide**

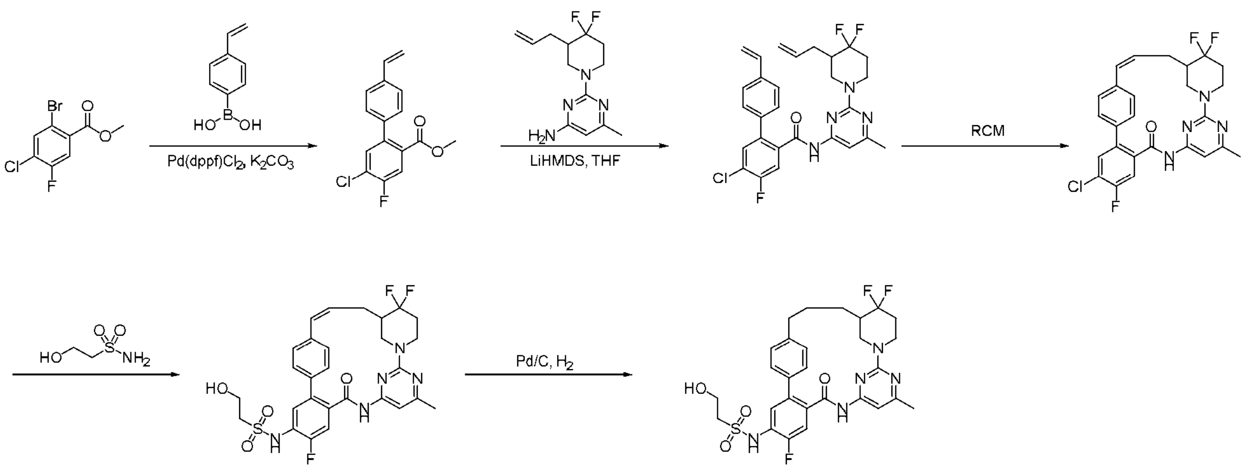


**Step 1:** A solution of methyl 2-bromo-4-chloro-5-fluorobenzoate (5.00 g, 18.69 mmol), (4-vinylphenyl)boric acid (3.30 g, 22.43 mmol), potassium carbonate (7.90 g, 56.08 mmol) and 1,1'-bis(diphenylphosphino)ferrocene palladium (II) dichloride (1.40 g, 1.87 mmol) in water (10.00 mL) and 1,4-dioxane (50.00 mL) was stirred at 80°C for 12 h under nitrogen atmosphere, diluted with water (100 mL), and extracted with ethyl acetate (50 mL $\times$ 3). The organic phase was dried over anhydrous sodium sulfate, filtered, concentrated, and purified by column chromatography (silica gel, 0-30% gradient of ethyl acetate/petroleum ether) to give 5-chloro-4-fluoro-4'-vinyl-[1,1'-biphenyl]-2-carboxylate (4.20 g, 14.45 mmol, yield: 77.3%) as a yellow oil. LCMS (ESI): $[M+H]^+$ = 291.00.

**Step 2:** To a solution of 2-(3-allyl-4,4-difluoropiperidin-1-yl)-6-methylpyrimidin-4-amine (1.00 g, 3.78 mmol) in tetrahydrofuran (10.00 mL) was added dropwise lithium bis(trimethylsilyl)amide (7.00 mL, 7 mmol, 1.0 M), and the mixture was stirred at 0°C for 30 min. To the reaction solution was added dropwise methyl 4-chloro-5-fluoro-2-(4-vinylphenyl) benzoate (1.00 g, 3.44 mmol). The reaction solution was stirred at 20°C for 12 h, diluted with water (50 mL) and extracted with ethyl acetate (50 mL $\times$ 3), and the organic phase was dried over anhydrous sodium sulfate, filtered, concentrated, and purified by column chromatography (silica gel, 0-100% gradient of ethyl acetate/petroleum ether) to give *N*-(2-(3-allyl-4,4-difluoropiperidin-1-yl)-6-methylpyrimidin-4-yl)-5-chloro-4-fluoro-4'-vinyl-[1,1'-biphenyl]-2-carboxamide (520 mg, 0.99 mmol, yield: 28.7%) as a yellow solid. LCMS (ESI): $[M+H]^+$ = 527.20.

**Step** 3: A solution of *N*-(2-(3-allyl-4,4-difluoropiperidin-1-yl)-6-methylpyrimidin-4-yl)-5-chloro-4-fluoro-4'-vinyl-[1,1'-biphenyl]-2-carboxamide (520 mg, 0.99 mmol) and Grubbs catalyst 2nd generation (82 mg, 0.095 mmol) in dichloromethane (100 mL) was stirred at 50°C for 2 h under nitrogen atmosphere, concentrated, and purified by column chromatography (silica gel, 0-30% gradient of ethyl acetate/petroleum ether) to give $5^4$-chloro-$1^4$,$1^4$,$5^5$-trifluoro-$2^6$-methyl-3-aza-2(2,4)-pyrimidin-1(1,3)-piperidin-5(1,2),6(1,4)-diphenylheterocyclonon-7-en-4 -one (300 mg, 0.60 mmol, yield: 63.4%) as a white solid. LCMS (ESI): $[M+H]^+$ = 499.10.

**Step 4:** A solution of $5^4$-chloro-$1^4$,$1^4$,$5^5$-trifluoro-$2^6$-methyl-3-aza-2(2,4)-pyrimidin-1(1,3)-piperidin-5(1,2),6(1,4)-diphenylcyclonon-7-en-4-one (300 mg, 0.60 mmol), 2-hydroxyethanesulfonamide (152 mg, 1.20 mmol),

potassium phosphate (391 mg, 1.80 mmol), 2-di-tert-butylphosphino-2',4',6'-triisopropylbiphenyl (26 mg, 0.060 mmol) and tris(dibenzylideneacetone)dipalladium (56 mg, 0.060 mmol) in dioxane (5.00 mL) was stirred at 100°C for 12 h under nitrogen atmosphere, diluted with water (30 mL) and extracted with ethyl acetate (30 mL × 3). The organic phase was dried over anhydrous sodium sulfate, filtered, concentrated, and purified by column chromatography (silica gel, 0-100% gradient of ethyl acetate/petroleum ether) to give *N*-($1^4$,$1^4$-difluoro-$2^6$-methoxy-4-oxo-3-aza-2(2,4)-pyrimidin-1(1,3)-piperidin-5(1,2),6(l,4)-diphenylheterocyclonon-8-en-$5^4$-yl)-2-hydroxyethane-1-sulfonamide (180 mg, 0.31 mmol, yield: 50.9%) as a light yellow solid. LCMS (ESI): $[M+H]^+$ =588.20.

**Step 5:** A solution of N-($1^4$,$1^4$-difluoro-$2^6$-methoxy-4-oxo-3-aza-2(2,4)-pyrimidin-1(1,3)-piperidin-5(1,2),6(1,4)-diphenylheterocyclonon-8-en-$5^4$-yl)-2-hydroxyethane-1-sulfonamide (180 mg, 0.31 mmol) and 10% Pd/C (wet) (33 mg, 0.03 mmol) in tetrahydrofuran (5.00 mL) was stirred at 25°C for 12 h under hydrogen atmosphere (15 Psi), filtered, concentrated, and purified by column chromatography (C18, 0-100% gradient of acetonitrile/water) to give 2-hydroxy-N-($1^4$,$1^4$5'-trifluoro-2'-methyl-4-oxo-3-aza-2(2,4)-pyrimidin-1(1,3)-piperidin-5(1,2),6(1,4)-diphenylheterocyclo nonane-$5^4$-yl)ethane-1-sulfonamide (104 mg, 0.18 mmol, yield: 57.6%) as a white solid. $^1$H NMR (400 MHz, DMSO-*$d_6$*) δ 10.10 - 9.99 (m, 1H), 8.93 (s, 1H), 7.69 - 7.55 (m, 1H), 7.54 - 7.46 (m, 1H), 7.45 - 7.17 (m, 3H), 7.13 - 6.95 (m, 2H), 5.01 (s, 1H), 4.56 - 4.30 (m, 1H), 4.00 - 3.87 (m, 1H), 3.86 - 3.76 (m, 2H), 3.43 - 3.35 (m, 2H), 3.25 - 3.13 (m, 1H), 3.11 - 3.01 (m, 1H), 2.87 - 2.77 (m, 1H), 2.45 - 2.35 (m, 1H), 2.30 - 2.22 (m, 3H), 2.04 - 1.70 (m, 4H), 1.68 - 1.51 (m, 1H), 1.21 - 0.96 (m, 2H) ppm; LCMS (ESI): $[M+H]^+$ =590.20.

**Example 54: *N-($1^4$,$1^4$-difluoro-$2^6$-methoxy-4-oxo-3-aza-2(2,4)-pyrimidin-1(1,3)-piperidin-5(1,2),6(1,3)-diphenyl-heterocyclonon-8-e* n-$5^4$-yl)-2-hydroxyethane-1-sulfonamide**

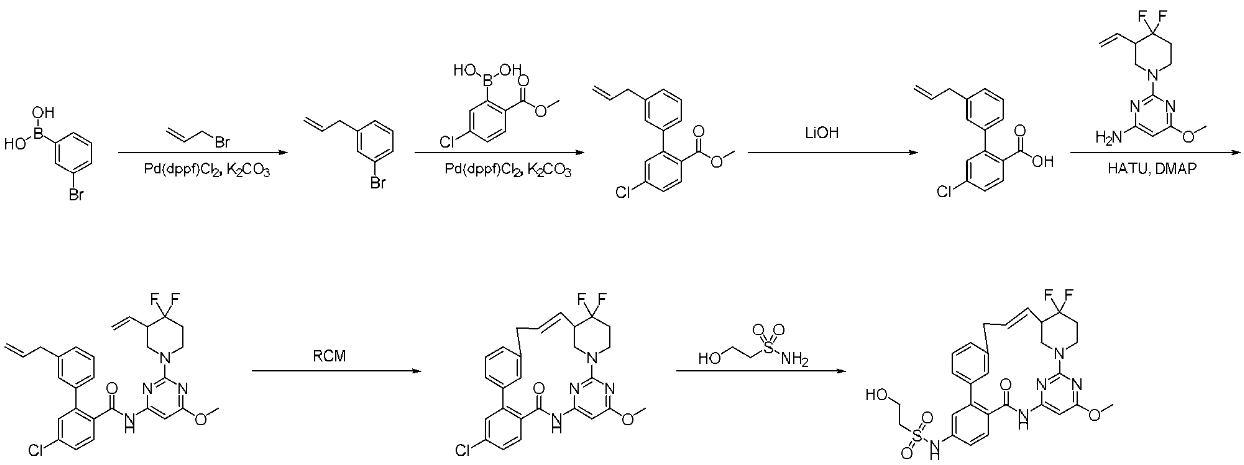


**Step 1:** A solution of (3-bromophenyl)boric acid (5.00 g, 24.90 mmol), allyl bromide (6.10 g, 49.79 mmol), potassium carbonate (10.5 g, 74.69 mmol) and 1,1'-bis(diphenylphosphino)ferrocene palladium dichloride (II) (1.90 g, 2.49 mmol) in 1,4-dioxane (50.00 mL) was stirred at 80°C for 12 h under nitrogen atmosphere, diluted with water (100 mL) and extracted with ethyl acetate (30 mL × 3). The organic phase was dried over anhydrous sodium sulfate, filtered, concentrated, and purified by column chromatography (silica gel, 0-30% gradient of ethyl acetate/petroleum ether) to give 1-allyl-3-bromobenzene (2.60 g, 13.19 mmol, yield: 53.0%) as a colorless oil. $^1$H NMR (400 MHz, DMSO-*$d_6$)* δ 7.47 - 7.37 (m, 2H), 7.31 - 7.17 (m, 2H), 6.02 - 5.86 (m, 1H), 5.14 - 5.01 (m, 2H), 3.42 - 3.31 (m, 2H) ppm.

**Step 2:** A solution of 1-allyl-3-bromobenzene (2.50 g, 12.69 mmol), (5-chloro-2-(methoxycarbonyl)phenyl)boric acid (4.10 g, 19.03 mmol), potassium carbonate (5.40 g, 38.06 mmol) and 1,1'-di(diphenylphosphino)ferrocene palladium dichloride (II) (960 mg, 1.27 mmol) in water (10.00 mL) and 1,4-dioxane (50.00 mL) was stirred at 80°C for 12 h under nitrogen atmosphere, diluted with water (100 mL) and extracted with ethyl acetate (30 mL × 3). The organic layer was dried over anhydrous sodium sulfate, filtered, concentrated, and purified by column chromatography (silica gel, 0-30% gradient of ethyl acetate/petroleum ether) to give 3'-allyl-5-chloro-[1,1'-biphenyl]-2-carboxylate (1.60 g, 5.58 mmol, yield: 44.0%) as a colorless oil. LCMS (ESI): $[M+H]^+$ = 287.00.

**Step 3:** A solution of methyl 3'-allyl-5-chloro-[1,1'-biphenyl]-2-carboxylate (1.00 g, 3.49 mmol) and lithium hydroxide (422 mg, 17.44 mmol) in water (5.00 mL), tetrahydrofuran (5.00 mL) and methanol (5.00 mL) was stirred at 20°C for 2 h, adjusted to pH 6 with 4 M hydrochloric acid and extracted with ethyl acetate (30 mL × 3). The organic phase was dried over anhydrous sodium sulfate, filtered, concentrated, and purified by column chromatography (silica gel, 0-100% gradient of ethyl acetate/petroleum ether) to give 3'-allyl-5-chloro-[1,1'-biphenyl]-2-carboxylic acid (600 mg, 2.20 mmol,

yield: 63.1%) as a yellow oil. LCMS (ESI): $[M+H]^+$ = 273.00.

**Step 4:** A solution of 3'-allyl-5-chloro-[1,1'-biphenyl]-2-carboxylic acid (600 mg, 1.89 mmol), 2-(4,4-difluoro-3-vinylpiperidin-1-yl)-6-methoxypyrimidin-4-amine (614 mg, 2.27 mmol), 2-(7-azobenzotriazole)-N,N,N',N'-tetramethylurea hexafluorophosphate (2.20 g, 5.68 mmol) and 4-dimethylaminopyridine (1.20 g, 9.46 mmol) in dichloroethane (20.00 mL) was stirred at 80°C for 12 h, diluted with water (50 mL) and extracted with ethyl acetate (30 mL × 3). The organic phase was dried over anhydrous sodium sulfate, filtered, concentrated, and purified by column chromatography (silica gel, 0-100% gradient of ethyl acetate/petroleum ether) to give 3'-allyl-5-chloro-N-(2-(4,4-difluoro-3-vinylpiperidin-1-yl)-6-methoxypyrimidin-4-yl)-[1,1'-biphenyl]-2-carboxamide (600 mg, 1.14 mmol, yield: 60.5%). LCMS (ESI): $[M+H]^+$ = 525.20.

**Step 5:** A solution of 3'-allyl-5-chloro-*N*-(2-(4,4-difluoro-3-vinylpiperidin-1-yl)-6-methoxypyrimidin-4-yl)-[1,1'-biphenyl]-2-carboxamide (600 mg, 1.14 mmol) and Grubbs catalyst 2nd generation (99 mg, 0.014 mmol) in toluene (120.00 mL) was stirred at 80°C for 2 h. The reaction mixture was concentrated and purified by column chromatography (silica gel, 0-30% gradient of ethyl acetate/petroleum ether) to give $5^4$-chloro-$1^4$,$1^4$-difluoro-$2^6$-methoxy-3-aza-2(2,4)-pyrimidin-1(1,3)-piperidin-5(1,2),6(1,3)-diphenylheterocyclonon-8-en-4-one (100 mg, 0.20 mmol, yield: 17.6%) as a white solid. LCMS (ESI): $[M+H]^+$ = 497.20.

**Step 5:** A solution of $5^4$-chloro-$1^4$,$1^4$-difluoro-$2^6$-methoxy-3-aza-2(2,4)-pyrimidin-1(1,3)-piperidin-5(1,2),6(1,3)-diphenylheterocyclonon-8-en-4-one (100 mg, 0.20 mmol), 2-hydroxyethanesulfonamide (51 mg, 0.40 mmol), potassium phosphate (131 mg, 0.60 mmol), 2-di-tert-butylphosphino-2',4',6'-triisopropylbiphenyl (9 mg, 0.020 mmol) and tris(dibenzylideneacetone)dipalladium (19 mg, 0.020 mmol) in 1,4-dioxane (3.00 mL) was stirred at 100°C for 12 h under nitrogen atmosphere, diluted with water (30 mL) and extracted with ethyl acetate (30 mL × 3). The organic phase was dried over anhydrous sodium sulfate, filtered, concentrated, and purified by column chromatography (silica gel, 0-100% gradient of ethyl acetate/petroleum ether) to give N-($1^4$,$1^4$-difluoro-$2^6$-methyl-$6^2$,4-dioxo-$6^1$,$6^2$-dihydro-3-aza-2(2,4)-pyrimidin-6(1,4)-pyridin-1(1,3)-piperidin-$5^4$(1,2)-phenyl heterocyclonon-7-en-4-yl)-2-hydroxyethane-1-sulfonamide (67 mg, 0.11 mmol, yield: 56.9%) as a white solid. $^1H$ NMR (400 MHz, $CDCl_3$) $\delta$ 8.33 (d, *J* = 8.8 Hz, 1H), 7.72 (s, 1H), 7.49 (t, *J* = 7.6 Hz, 1H), 7.40 - 7.29 (m, 4H), 7.25 - 7.19 (m, 2H), 6.77 (s, 1H), 6.27 - 6.16 (m, 1H), 6.05 - 5.94 (m, 1H), 4.69 - 4.60 (m, 1H), 4.17 - 4.01 (m, 4H), 3.86 (s, 3H), 3.78 - 3.65 (m, 1H), 3.52 - 3.42 (m, 1H), 3.41 - 3.32 (m, 2H), 3.20 - 3.01 (m, 1H), 2.90 - 2.76 (m, 2H), 2.70 (s, 1H), 2.08 (s, 1H) ppm; LCMS (ESI): $[M+H]^+$ =586.40.

**Example 55: *N*-($1^4$,$1^4$-difluoro-$2^6$-methyl-4-oxo-3-aza-2(2,4)-pyrimidin-1(1,3)-piperidin-5(1,2),6(1,4)-diphenylheterocyclonon-8-en-$5^4$-yl)-2-hydroxyethane-1-sulfonamide**

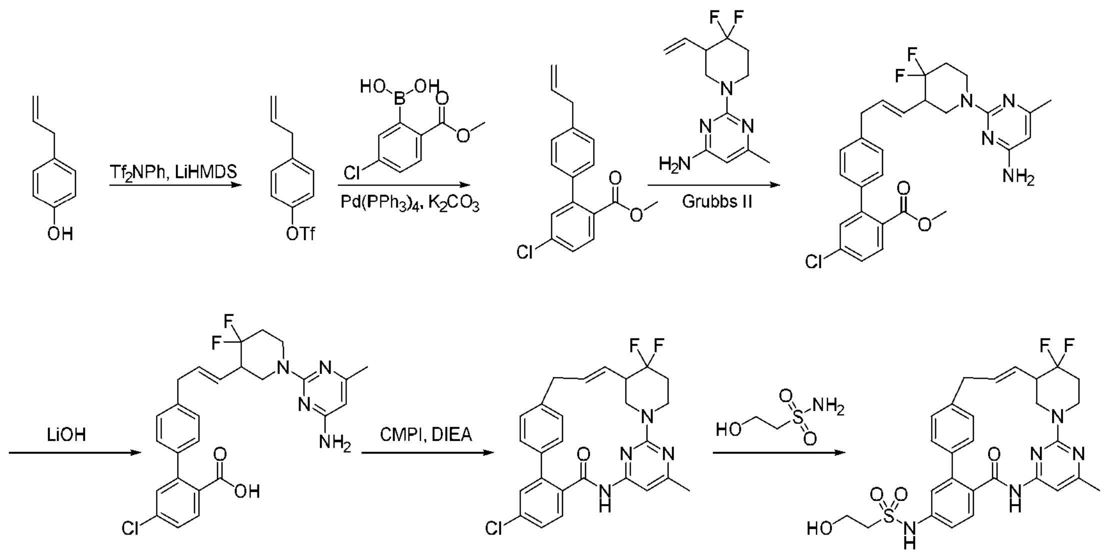


**Step 1:** To a solution of 4-allylphenol (2.00 g, 14.91 mmol) and N-phenyl-bis(trifluoromethanesulfonimide) (8.15 g, 22.36 mmol) in tetrahydrofuran (75.00 mL) was slowly added 1.0 M lithium bis(trimethylsilyl)amide (22.36 mL, 22.36 mmol) at -78°C under nitrogen atmosphere. Subsequently, the temperature was slowly increased by 20°C, and the mixture was stirred for 1 h. The reaction was complete. The reaction solution was quenched with sodium bicarbonate solution (50 mL) and extracted with ethyl acetate (50 mL × 3), and the organic phases were combined, washed with sodium chloride (100 mL), dried over sodium sulfate, filtered, and concentrated to give phenyl 4-allyl trifluoromethanesulfonate (crude).

**Step 2: A** solution of 4-allyl phenyl trifluoromethanesulfonate (crude, 14.91 mmol), tetrakis(triphenylphosphine)palladium (732 mg, 0.62 mmol), (5-chloro-2-(methoxycarbonyl)phenyl)boric acid (2.66 mg, 12.42 mmol) and potassium carbonate (3.50 g, 24.83 mmol) in 1,4-dioxane (50.00 mL) and water (10.00 mL) was stirred in an oil bath at 100°C for 10 h under nitrogen atmosphere. After the reaction was complete, the mixture was quenched with cold water (30 mL) and extracted with ethyl acetate (50 mL × 3), and the organic phases were combined, washed with sodium chloride solution (100 mL), dried over sodium sulfate, filtered, and concentrated to give the crude product. The crude product was purified by flash column chromatography (silicon dioxide, 0-30% gradient of ethyl acetate/petroleum ether) to give 4'-allyl-5-chloro-[1,1'-biphenyl]-2-carboxylate (1.40 g, 4.88 mmol, yield: 39.3%) as a colorless liquid. LCMS (ESI): $[M+H]^+$ = 287.10.

**Step 3:** A solution of 3-(4,4-difluoro-3-vinylpiperidin-1-yl)-6-methylpyrimidin-4-amine (400 mg, 1.57 mmol), 4'-allyl-5-chloro-[1,1'-biphenyl]-2-carboxylate (677 mg, 2.36 mmol) and Grubbs catalyst 2nd generation (68 mg, 0.08 mmol) in dichloromethane (10.00 mL) was stirred at 50°C for 16 h under nitrogen atmosphere. After the reaction was complete, the solution was concentrated to give the crude product. The crude product was purified by flash column chromatography (silicon dioxide, 10-30% gradient of ethyl acetate/petroleum ether) to give 4'-(3-(1-(4-amino-6-methylpyrimidin-2-yl)-4,4-difluoropiperidin-3-yl)allyl)-5-chloro-[1,1'-biphenyl]-2-carboxylate (100 mg, 0.19 mmol, yield: 12.4%) as a yellow solid. LCMS (ESI): $[M+H]^+$ = 513.20.

**Step 4:** To a solution of 4'-(3-(1-(4-amino-6-methylpyrimidin-2-yl)-4,4-difluoropiperidin-3-yl)allyl)-5-chloro-[1,1'-biphenyl]-2-carboxylate (100 mg, 0.19 mmol) in tetrahydrofuran (4.00 mL) was added 2N aqueous lithium hydroxide solution (2.00 mL), and the mixture was stirred at 50°C for 2 h. After the reaction was complete, 1 N hydrochloric acid (4.50 mL) was added to adjust the pH to 7. The solution was concentrated under reduced pressure to remove solvent and water and to give 4'-(3-(1-(4-amino-6-methylpyrimidin-2-yl)-4,4-difluoropiperidin-3-yl)allyl)-5-chloro-[1,1'-biphenyl]-2-carboxylic acid (crude).

**Step 5:** A solution of 4'-(3-(1-(4-amino-6-methylpyrimidin-2-yl)-4,4-difluoropiperidin-3-yl)allyl)-5-chloro-[1,1'-biphenyl]-2-carboxylic acid (crude, 0.19 mmol), 2-chloro-1-methylpyridinium iodide (152 mg, 0.58 mmol) and N,N-diisopropylethylamine (257 mg, 1.95 mmol) in tetrahydrofuran (50.00 mL) was stirred in an oil bath at 50°C for 24 h. After the reaction was complete, the solution was quenched with sodium bicarbonate (50 mL) and extracted with dichloromethane (50 mL × 3), and the organic phases were combined, dried, filtered, and concentrated to give the crude product. The crude product was purified by flash column chromatography (silicon dioxide, 0-20% gradient of ethyl acetate/petroleum ether) to give $5^4$-chloro-$1^4$,$1^4$-difluoro-$2^6$-methyl-3-aza-2(2,4)-pyrimidin-1(1,3)-piperidin-5(1,2),6(1,4)-diphenylheterocyclonon-8-en-4-on e (50 mg, 0.10 mmol, yield: 53.3%) as a white solid. LCMS (ESI): $[M+H]^+$ =481.20.

**Step 6:** A mixture of $5^4$-chloro-$1^4$,$1^4$-difluoro-$2^6$-methyl-3-aza-2(2,4)-pyrimidin-1(1,3)-piperidin-5(1,2),6(1,4)-diphenylheterocyclonon-8-en-4-on e (50 mg, 0.10 mmol), 2-hydroxyethylsulfonamide (39 mg, 0.31 mmol), potassium phosphate (90 mg, 0.42 mmol), 2-di-tert-butylphosphino-2',4',6'-triisopropylbiphenyl (4 mg, 0.001 mmol) and tris(dibenzylideneacetone)dipalladium (10 mg, 0.001 mmol) was dissolved in 1,4-dioxane (5.00 mL) at 20°C under nitrogen atmosphere, and the resulting reaction mixture was stirred at 100°C for 6 h. After the reaction was complete, the mixture was quenched with cold water (20 mL) and extracted with ethyl acetate (20 mL × 3), and the organic phases were combined, washed with sodium chloride solution (100 mL), dried with magnesium sulfate, filtered, and concentrated to give the crude product. The crude product was purified by flash column chromatography (silica, 60-90% gradient of ethyl acetate/petroleum ether) to give N-($1^4$,$1^4$-difluoro-$2^6$-methyl-4-oxo-3-aza-2(2,4)-pyrimidin-1(1,3)-piperidin-5(1,2),6(1,4)-diphenylheterocyclonon-8-en-$5^4$-yl) -2-hydroxyethane-1-sulfonamide (10 mg, 0.017 mmol, yield: 16.8%) as a white solid. $^1$H NMR (400 MHz, DMSO-$d_6$) δ 10.18 (s, 1H), 8.61 (br s, 1H), 7.63 (d, $J$ = 8.3 Hz, 1H), 7.43 - 7.19 (m, 5H), 6.91 (s, 2H), 6.16 - 5.96 (m, 1H), 5.13 - 4.82 (m, 3H), 4.36 (d, $J$ = 13.9 Hz, 1H), 3.77 (t, $J$ = 6.6 Hz, 2H), 3.41 - 3.28 (m, 3H), 3.10 (dd, $J$= 14.5, 6.6 Hz, 1H), 2.97 (d, $J$ = 13.5 Hz, 1H), 2.88 - 2.69 (m, 2H), 2.24 (s, 3H), 1.99 - 1.79 (m, 2H) ppm; LCMS (ESI): $[M+H]^+$ = 570.30.

**Example 56A and Example 56B: Two isomers** of **N-($1^4$,$1^4$-difluoro-$2^6$-methoxy-4-oxo-3-aza-2(2,4)-pyrimidin-5(3,4)-pyridin-1(1,3)-piperidin-6(1,4)-benzoazacyclonona ne-$5^6$-yl)-2-hydroxyethane-1-sulfonamide**

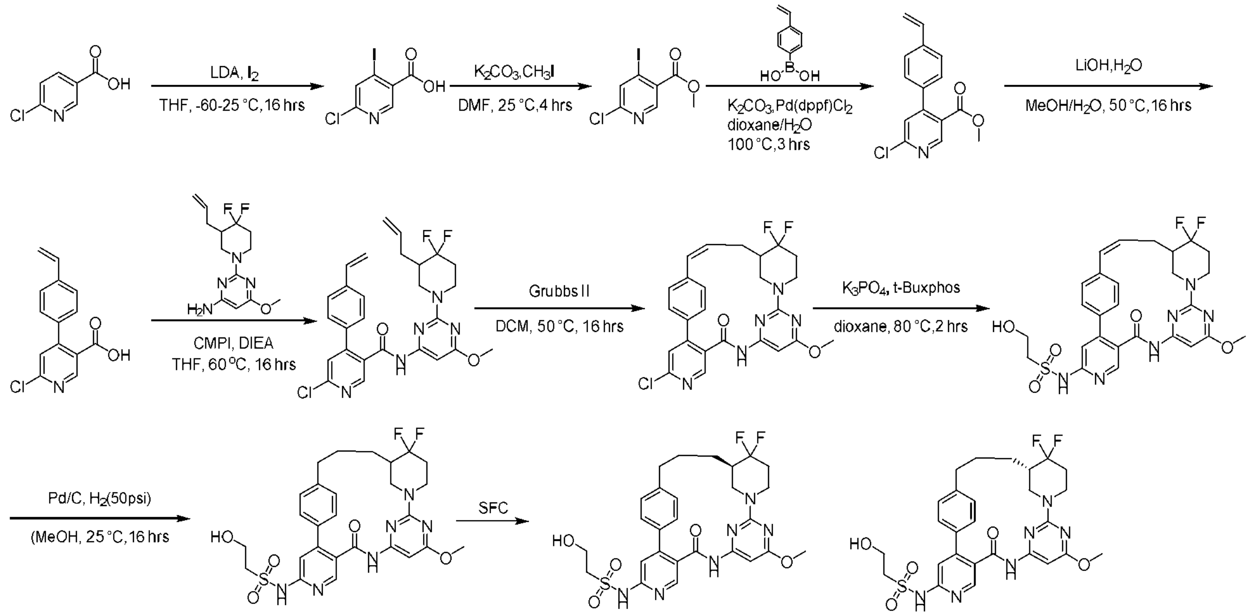


**Step 1:** To a solution of 2,4-dichloro-6-methoxypyrimidine (2.00 g, 11.1 mmol) and 3-allyl-4,4-difluoropiperidine trifluoroacetate (5.21 g, 20.1 mmol) in tert-butanol (40.0 mL) was added N,N-diisopropylethylamine (7.22 g, 55.8 mmol), and the mixture was stirred at 100°C for 3 h. The reaction mixture was diluted with water (100 mL), extracted with ethyl acetate (200 mL × 2), and the organic phase was dried, filtered and concentrated. The residue was purified by flash column chromatography (silica gel, 0-3% gradient of tetrahydrofuran/petroleum ether) to give 2-(3-allyl-4,4-difluoropiperidin-1-yl)-4-chloro-6-methoxypyrimidine (2.60 g, 7.55 mmol, yield: 68.0%) as a yellow oil. LCMS (ESI): $[M+H]^+$ = 303.9. $^1$H NMR (400 MHz, CHLOROFORM-d) δ ppm 6.00 - 5.88 (m, 1H), 5.83 - 5.68 (m, 1H), 5.12 - 5.00 (m, 2H), 4.43 - 4.30 (m, 2H), 3.83 - 3.79 (m, 3H), 3.38 (ddd, *J* = 3.6, 10.8, 14.0 Hz, 1H), 3.09 (br dd, *J* = 9.6, 13.6 Hz, 1H), 2.50 - 2.42 (m, 1H)

**Step 2:** To a solution of 2-(3-allyl-4,4-difluoropiperidin-1-yl)-4-chloro-6-methoxypyrimidine (2.60 g, 8.56 mmol) and benzophenone imine (2.37 g, 12.8 mmol) in dioxane (50.0 mL) were added cesium carbonate (8.37 g, 25.6 mmol), 4,5-bis(diphenylphosphine)-9,9-dimethylxanthracene (0.99 g, 1.71 mmol) and tris(dibenzylideneacetone)dipalladium (0.78 g, 0.86 mmol), and the mixture was stirred at 100°C for 16 h under nitrogen atmosphere. The reaction mixture was diluted with water (100 mL), extracted with ethyl acetate (200 mL × 2), and the organic phase was dried, filtered and concentrated. to give N-(2-(3-allyl-4,4-difluoropiperidin-1-yl)-6-methoxypyrimidin-4-yl)-1,1-diphenylmethanimine (3.80 g, crude) as a brown oil. The product was used directly in the next step. LCMS (ESI): $[M+H]^+$ = 449.2

**Step 3:** To a solution of N-(2-(3-allyl-4,4-difluoropiperidin-1-yl)-6-methoxypyrimidin-4-yl)-1,1-diphenylmethanimine (3.80 g, 8.47 mmol) in methanol (80.0 mL) were added sodium acetate (2.08 g, 25.4 mmol) and hydroxylamine hydrochloride (1.18 g, 16.9 mmol), and the mixture was stirred at 25°C for 2 h. The reaction mixture was diluted with water (100 mL), extracted with ethyl acetate (100 mL × 2), and the organic phase was dried, filtered and concentrated. The residue was purified by flash column chromatography (silica gel, 0-10% gradient of tetrahydrofuran/petroleum ether) to give 2-(3-allyl-4,4-difluoropiperidin-1-yl)-6-methoxypyrimidin-4-amine (2.10 g, 5.76 mmol, yield: 68.0%) as a yellow oil. LCMS (ESI): $[M+H]^+$ = 285.0. $^1$H NMR (400 MHz, CHLOROFORM-d) δ ppm 5.86 - 5.69 (m, 1H), 5.11 (s, 1H), 5.10 - 4.98 (m, 2H), 4.49 - 4.31 (m, 4H), 3.77 - 3.73 (m, 3H), 3.31 - 3.19 (m, 1H), 3.03 - 2.89 (m, 1H), 2.50 - 2.39 (m, 1H), 2.00 - 1.75 (m, 4H)

**Step 4:** To a solution of 2,2,6,6-tetramethylpiperidine (22.6 g, 158 mmol) in tetrahydrofuran (200 mL) was added n-butyllithium (63.4 mL, 158 mmol) dropwise at -60°C under nitrogen atmosphere, and the mixture was heated to 0°C, stirred for 1.5 h, and cooled to -60°C. To a solution of 6-chloronicotinic acid (10.0 g, 63.4 mmol) in tetrahydrofuran (100 mL) was added elemental iodine (19.3 g, 76.1 mmol) in tetrahydrofuran (50.0 mL) dropwise, and the mixture was heated to 25°C and stirred for 16 h. The reaction solution was quenched with dilute hydrochloric acid (1 M) and extracted with ethyl acetate (400 mL × 2), and the organic phase was dried, filtered, and concentrated. The residue was purified by slurrying with methyl tert-butyl ether (80.0 ml) to give 6-chloro-4-iodonicotinic acid (11.0 g, 35.5 mmol, yield: 56.0%) as a brown solid. LCMS (ESI): $[M+H]^+$ = 283.7. $^1$H NMR (400 MHz, DMSO-$d_6$) δ ppm 15.60 - 11.18 (m, 1H), 8.65 (s, 1H), 8.23 (s, 1H)

**Step 5:** To a solution of 6-chloro-4-iodonicotinic acid (5.00 g, 17.6 mmol) in N,N-dimethylformamide (100 mL) was added potassium carbonate (4.88 g, 35.2 mmol), and the mixture was stirred at 25°C for 10 min. To the reaction solution was added methyl iodide (5.01 g, 35.2 mmol) dropwise, and the mixture was stirred at 25°C for 4 h. The

reaction mixture was diluted with water (200 mL), extracted with ethyl acetate (300 mL × 2), and the organic phase was dried, filtered and concentrated. The residue was purified by flash column chromatography (silica gel, 0-6% gradient of tetrahydrofuran/petroleum ether) to give methyl 6-chloro-4-iodonicotinyl ester (4.80 g, 14.4 mmol, yield: 82.0%) as a white solid. LCMS (ESI): $[M+H]^+$ = 297.7. $^1H$ NMR (400 MHz, CHLOROFORM-d) δ ppm 8.69 (s, 1H), 7.94 (s, 1H), 3.90 (s, 3H)

**Step 6:** To a solution of methyl 6-chloro-4-iodonicotinyl ester (4.80 g, 16.1 mmol) and 4-vinylphenylboronic acid (2.39 g, 16.1 mmol) in dioxane (20.0 mL) and water (10.0 mL) were added potassium carbonate (6.69 g, 48.4 mmol) and 1,1'-bis(diphenylphosphino)ferrocene]palladium dichloride (1.18 g, 1.61 mmol), and the mixture was stirred at 100°C for 3 h under nitrogen atmosphere. The reaction mixture was diluted with water (100 mL), extracted with ethyl acetate (200 mL × 2), and the organic phase was dried, filtered and concentrated. The residue was purified by flash column chromatography (silica gel, 0-6% gradient of tetrahydrofuran/petroleum ether) to give methyl 6-chloro-4-(4-vinylphenyl) nicotinyl ester (3.40 g, 10.3 mmol, yield: 64.0%) as a yellow solid. LCMS (ESI): $[M+H]^+$ = 273.9. $^1H$ NMR (400 MHz, CHLOROFORM-d) δ ppm 8.74 (s, 1H), 7.41 (d, *J* = 8.4 Hz, 2H), 7.27 (s, 1H), 7.20 (d, *J* = 8.4 Hz, 2H), 6.68 (dd, *J* = 10.8, 17.6 Hz, 1H), 5.76 (d, *J* = 17.6 Hz, 1H), 5.26 (*d, J* = 10.8 Hz, 1H), 3.66 (s, 3H)

**Step 7:** To a solution of methyl 6-chloro-4-(4-vinylphenyl) nicotinyl ester (1.00 g, 3.65 mmol) in methanol (20.0 mL) was added lithium hydroxide monohydrate (10.0 mL) in water (10.0 mL), and the mixture was stirred at 50°C for 16 h. The reaction mixture was diluted with water (50.0 mL) and the pH was adjusted to 4 with dilute hydrochloric acid (1M). The mixture was extracted with ethyl acetate (100 mL × 2). The organic layer was dried, filtered and concentrated. to give 6-chloro-4-(4-vinylphenyl) nicotinic acid (0.90 g, crude) as a yellow solid. The product was used directly in the next step. LCMS (ESI): $[M+H]^+$ = 259.9

**Step 8:** To a solution of 6-chloro-4-(4-vinylphenyl)nicotinic acid (0.80 g, 3.08 mmol) and 2-(3-allyl-4,4-difluoropiperidin-1-yl)-6-methoxypyrimidin-4-amine (0.96 g, 3.39 mmol) in tetrahydrofuran (16.0 mL) were added N,N-diisopropylethylamine (1.99 g, 15.4 mmol) and 2-chloro-1-methylpyridine (salt) iodide (2.34 g, 6.16 mmol), and the mixture was stirred at 60°C for 16 h. The reaction mixture was diluted with water (100 mL), extracted with ethyl acetate (100 mL × 3), and the organic phase was dried, filtered and concentrated. The residue was purified by flash column chromatography (silica gel, 0-8% gradient of tetrahydrofuran/petroleum ether) to give N-(2-(3-allyl-4,4-difluoropiperidin-1-yl)-6-methoxypyrimidin-4-yl)-6-chloro-4-(4-vinylphenyl)niacinamide (1.30 g, 2.06 mmol, yield: 67.0%) as a yellow solid. LCMS (ESI): $[M+H]^+$ = 527.9

**Step 9:** To a solution of N-(2-(3-allyl-4,4-difluoropiperidin-1-yl)-6-methoxypyrimidin-4-yl)-6-chloro-4-(4-vinylphenyl) nicotinamide (980 mg, 1.86 mmol) in dichloromethane (1.00 L) was added Grubbs II (316 mg, 0.37 mmol), and the mixture was stirred at 50°C for 16 h under nitrogen atmosphere. The reaction solution was concentrated under reduced pressure, and the residue was purified by flash column chromatography (silica gel, 0-8% gradient of tetrahydrofuran/petroleum ether) to give $5^6$-chloro-$1^4$,$1^4$-difluoro-$2^6$-methoxy-3-aza-2(2,4)-pyrimidin-5(3,4)-pyridin-1(1,3)-piperidin-6(1,4)-benzocyclononan-7-en-4 -one (500 mg, 0.41 mmol, yield: 22.0%) as a yellow solid. LCMS (ESI): $[M+H]^+$ = 498.6

**Step 10:** To a solution of $5^6$-chloro-$1^4$,$1^4$-difluoro-$2^6$-methoxy-3-aza-2(2,4)-pyrimidin-5(3,4)-pyridin-1(1,3)-piperidin-6(1,4)-benzocyclononan-7-en-4 -one (250 mg, 0.50 mmol) and 2-hydroxyethane-1-sulfonamide (81.6 mg, 0.65 mmol) in dioxane (5.00 mL) were added potassium phosphate (319 mg, 1.51 mmol), 2-di-tert-butylphosphino-2',4',6'-triisopropyl diphenyl (42.6 mg, 0.10 mmol) and tris(dibenzylideneacetone)dipalladium (45.9 mg, 0.05 mmol), and the mixture was stirred at 80°C for 2 h under nitrogen atmosphere. The reaction mixture was diluted with water (50.0 mL), extracted with ethyl acetate (100 mL × 2), and the organic phase was dried, filtered and concentrated. The residue was purified by flash column chromatography (silica gel, 0-38% gradient of tetrahydrofuran/petroleum ether) to give N-($1^4$,$1^4$-difluoro-$2^6$-methoxy-4-oxo-3-aaa-2(2,4)-pyrimidin-5(3,4)-pyridin-1(1,3)-piperidin-6(1,4)-benzylcyclononane-7-en -$5^6$-yl)-2-hydroxyethane-1-sulfonamide (190 mg, 0.25 mmol, yield: 49.0%) as a brown oil. LCMS (ESI): $[M+H]^+$ = 587.2

**Step 11:** To a solution of N-($1^4$,$1^4$-difluoro-$2^6$-methoxy-4-oxo-3-aza-2(2,4)-pyrimidin-5(3,4)-pyridin-1(1,3)-piperidin-6(1,4)-benzylcyclononane-7-en -$5^6$-yl)-2-hydroxyethane-1-sulfonamide (190 mg, 0.34 mmol) in methanol (20.0 mL) was added 10% Pd/C (wet) (100 mg, 0.01 mmol), and the reaction mixture was purged with argon three times and then flushed with hydrogen three times. and stirred at 25°C for 16 h under hydrogen atmosphere (50 Psi). The reaction mixture was filtered under reduced pressure to remove palladium/carbon and then concentrated in vacuum. The residue was purified by preparative HPLC (C18, 34-74% gradient of water (formic acid)/acetonitrile) to give N-($1^4$,$1^4$-difluoro-$2^6$-methoxy-4-oxo-3-aza-2(2,4)-pyrimidin-5(3,4)-pyridin-1(1,3)-piperidin-6(1,4)-benzoazacyclononane-$5^6$ -yl)-2-hydroxyethane-1-sulfonamide (49.71 mg, 0.09 mmol, yield: 26.0%) as a white solid.

**Example 56:** The above products were separated by SFC (column: DAICEL CHIRALPAK AS (250 mm*30 mm, 10 μm), mobile phase: $CO_2$-EtOH (0.1% $NH_3H_2O$), gradient: isocratic 40%, flow rate: 80 mL/min, column temperature: 40°C) to give two stereoisomers.

**Example 56A:** White solid (21.17 mg). SFC analysis (column: Chiralpak AS-3 50 x 4.6 mm I.D., 3 μm; mobile phase: carbon dioxide for phase A and ethanol (0.05% ethylene glycol) for phase B; gradient: 40% for phase B; flow rate: 4

mL/min). SFC (method: AS_3_ETOH_DEA_5_40_4ML_2 miN, Rt = 1.481). LCMS (ESI): $[M+H]^+$ = 589.3. $^1H$ NMR (400 MHz, DMSO-$d_6$) δ ppm 8.91 (br s, 1H), 8.49 (s, 1H), 7.38 (br s, 2H), 7.07 (br s, 2H), 6.94 (s, 1H), 6.76 (br s, 1H), 4.47 - 4.28 (m, 1H), 3.94 (br d, *J* = 13.6 Hz, 1H), 3.82 (s, 3H), 3.80 (t, *J* = 7.2 Hz, 2H), 3.71 - 3.59 (m, 2H), 3.22 (br d, J = 13.2 Hz, 1H), 3.08 (br d, *J*= 10.8 Hz, 1H), 2.89 - 2.78 (m, 1H), 2.46 - 2.38 (m, 1H), 2.05 - 1.86 (m, 3H), 1.85 - 1.71 (m, 1H), 1.67 - 1.55 (m, 1H), 1.20 - 1.13 (m, 1H), 1.09 - 1.01 (m, 1H)

**Example 56B:** White solid (24.09 mg). SFC analysis (column: Chiralpak AS-3 50 x 4.6 mm I.D., 3 μm; mobile phase: carbon dioxide for phase A and ethanol (0.05% ethylene glycol) for phase B; gradient: 40% for phase B; flow rate: 4 mL/min). SFC (method: AS_3_ETOH_DEA_5_40_4ML_2 miN, Rt = 1.597). LCMS (ESI): $[M+H]^+$ = 589.3. $^1H$ NMR (400 MHz, DMSO- $d_6$) δ ppm 8.90 (br s, 1H), 8.54 (s, 1H), 7.44 (br s, 2H), 7.23 - 7.05 (m, 2H), 6.97 (s, 1H), 6.82 (br s, 1H), 4.53 - 4.35 (m, 1H), 4.00 (br d, *J* = 12.8 Hz, 1H), 3.89 (s, 3H), 3.86 (t, *J* = 6.8 Hz, 2H), 3.74 - 3.62 (m, 2H), 3.28 (br s, 1H), 3.17 - 3.12 (m, 1H), 2.94 - 2.86 (m, 1H), 2.52 - 2.45 (m, 1H), 2.10 - 1.92 (m, 3H), 1.89 - 1.82 (m, 1H), 1.73 - 1.61 (m, 1H), 1.25 - 1.18 (m, 1H), 1.16 - 1.08 (m, 1H).

**Example 57 and Example 57A: Two stereoisomers of N-($1^4$,$1^4$-difluoro-$2^6$-methyl-4-oxo-3-aza-2(2,4)-pyrimidin-1(1,3)-piperidin-6(2,5)-thiophene-5(1,2)-benzoazacyclon-8-e n-$5^4$-yl)-2-hydroxyethane-1-sulfonamide**

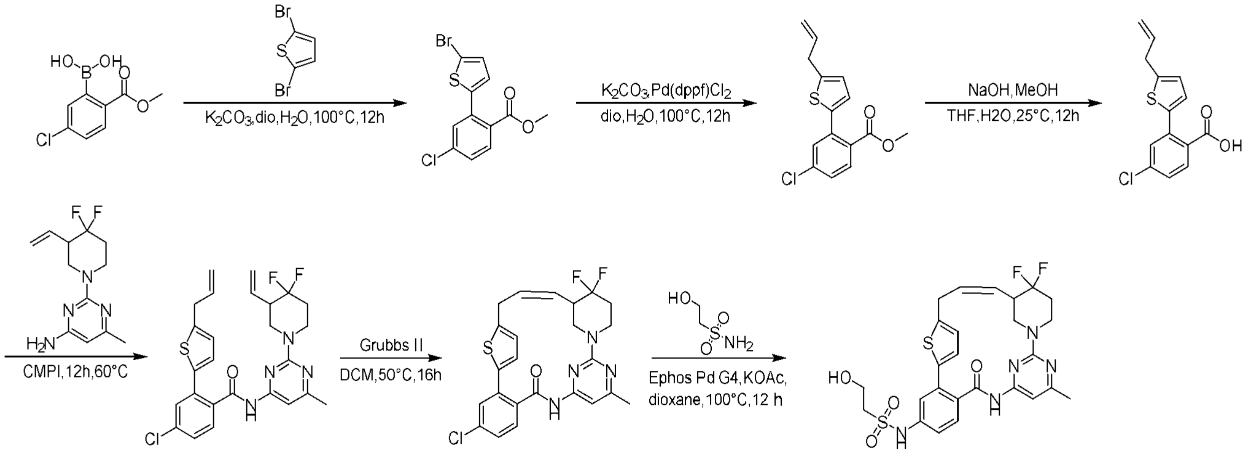


**Step 1:** To a solution of [5-chloro-2-(methyl ester)phenyl]boric acid (8.90 g, 41.5 mmol) and 2,5-dibromothiophene (15.1 g, 62.3 mmol) in dioxane (100 mL) and water (20.0 mL) were added potassium carbonate (17.6 g, 125 mmol) and 1,1-bis(diphenylphosphine)ferrocene palladium chloride (3.14 g, 4.15 mmol) under nitrogen atmosphere. The mixture was stirred at 100°C for 12 h. The reaction solution was diluted with water (150 mL) and extracted with ethyl acetate (150 mL x 2), and the combined organic phases were dried over anhydrous magnesium sulfate, filtered, and concentrated to give the crude product. The crude product was purified by flash column chromatography (silica gel, 0-3% gradient of tetrahydrofuran/petroleum ether) to give 2-(5-bromothiophene-2-yl)-4-chlorobenzoate (1.12 g, 3.32 mmol, yield: 8%) as a colorless liquid. $^1H$ NMR (400 MHz, CHLOROFORM-d) δ ppm 7.73 (d, *J*= 8.4 Hz, 1H), 7.45 - 7.37 (m, 2H), 7.03 (d, *J* = 3.6 Hz, 1H), 6.81 (*d, J* = 3.6 Hz, 1H), 3.78 (s, 3H)

**Step 2:** To a solution of methyl 2-(5-bromothiophene-2-yl)-4-chlorobenzoate (1.00 g, 3.03 mmol) and 2-allyl-4,4,5,5-tetramethyl-1,3,2-dioxaborolane (0.76 g, 4.52 mmol) in dioxane (20 mL) and water (4.00 mL) were added potassium carbonate (1.28 g, 9.05 mmol) and 1,1-bis(diphenylphosphine)ferrocene palladium chloride (0.23 g, 0.50 mmol) under nitrogen atmosphere. The mixture was stirred at 100°C for 12 h. The reaction solution was diluted with water (50 mL) and extracted with ethyl acetate (50 mL × 2). The combined organic phases were dried over anhydrous magnesium sulfate, filtered, and concentrated to give the crude product. The crude product was purified by flash column chromatography (silica gel, 0-8% gradient of tetrahydrofuran/petroleum ether) to give 4-chloro-2-[5-(prop-2-en-1-yl)thiophene-2-yl]benzoate (1.2 g, crude) as a colorless liquid. $^1H$ NMR (400 MHz, CHLOROFORM-d) δ ppm 7.57 (d, *J* = 8.4 Hz, 1H), 7.38 (d, *J* = 2.0 Hz, 1H), 7.29 - 7.24 (m, 1H), 6.81 (d, *J* = 3.6 Hz, 1H), 6.69 (d, *J* = 3.6 Hz, 1H), 5.99 - 5.90 (m, 1H), 5.14 - 5.03 (m, 2H), 3.68 (s, 3H), 3.51 (d, *J* = 6.8 Hz, 2H)

**Step 3:** To a mixed solution of methanol (10.0 mL), tetrahydrofuran (10.0 mL) and water (10.0 mL) were added methyl 4-chloro-2-[5-(prop-2-en-1-yl)thiophene-2-yl]benzoate (1.0 g, 3.42 mmol) and sodium hydroxide (1.09 g, 27.3 mmol), and the mixture was stirred at 25°C for 12 h. The reaction solution was concentrated under reduced pressure to remove methanol and tetrahydrofuran, and the residue was diluted with water (20.0 mL) and extracted once with ethyl acetate (20.0 mL) to collect the aqueous phase. The aqueous phase was adjusted to pH about 2 with dilute

hydrochloric acid (2 M) and extracted with ethyl acetate (20.0 mL × 3), and the organic phase was dried over anhydrous magnesium sulfate, filtered, and concentrated under reduced pressure to give 4-chloro-2-[5-(prop-2-en-1-yl)thiophene-2-yl]benzoic acid (0.50 g, crude) as a yellow oil.

**Step 4:** To a solution of N,N-diisopropylethylamine (1.34 g, 10.5 mmol) and 2-chloro-1-methylpyridinium iodide (1.98 g, 5.20 mmol) in tetrahydrofuran (15.0 mL) were added 2-(3-vinyl-4,4-difluoropiperidin-1-yl)-6-methylpyrimidin-4-amine (529 mg, 2.08 mmol) and 4-chloro-2-[5-(prop-2-en-1-yl)thiophene-2-yl]benzoic acid (580 mg, 2.08 mmol), and the mixture was stirred at 60°C for 12 h. The reaction mixture was diluted with water (20 mL), extracted with ethyl acetate (20 mL × 3), and the organic phase was dried, filtered and concentrated. The residue was purified by flash column chromatography (silica gel, 0-8% gradient of tetrahydrofuran/petroleum ether) to give 4-chloro-N-[2-(3-vinyl-4,4-difluoropiperidin-1-yl)-6-methylpyrimidin-4-yl]-2-[5-(prop-2-en-1-yl)thiophene-2-yl]benzamide (0.18 g, 0.35 mmol, yield: 17%) as a yellow solid. LCMS (ESI): $[M+H]^+$ =514.9

**Step 5:** To a solution of 4-chloro-N-[2-(3-vinyl-4,4-difluoropiperidin-1-yl)-6-methylpyrimidin-4-yl]-2-[5-(prop-2-en-1-yl)thiophene-2-yl]benzamide (160 mg, 0.31 mmol) in dry dichloromethane (50 mL) was added Grubbs catalyst 2nd generation (26.4 mg, 0.03 mmol) at 20°C under nitrogen atmosphere, and the mixture was heated to reflux at 50°C for 16 h. After the reaction was complete, the concentrated reaction solution was purified by flash column chromatography (silicon dioxide, 0-5% gradient of tetrahydrofuran/petroleum ether) to give $5^4$-chloro-$1^4$,$1^4$-difluoro-$2^6$-methyl-3-aza-2(2,4)-pyrimidin-1(1,3)-piperidin-6(2,5)-thiophene-5(1,2)-benzocyclononan-8-en-4-one (80 mg, 0.16 mmol, yield: 52.9%) as a yellow solid. LCMS (ESI): $[M+H]^+$ = 487.0

**Step 6:** To a solution of $5^4$-chloro-$1^4$,$1^4$-difluoro-$2^6$-methyl-3-aza-2(2,4)-pyrimidin-1(1,3)-piperidin-6(2,5)-thiophene-5(1,2)-benzocyclononan-8-en-4-one (80 mg, 0.16 mmol), 2-hydroxyethanesulfonamide (41.1 mg, 0.32 mmol) and potassium acetate (48.9 mg, 0.49 mmol) in dioxane (2.00 mL) was added [dicyclohexyl[3-(1-methylethoxy)-2',4",6'-tris(1-methylethyl)[1,1'-biphenyl]-2-yl]phosphine-κP(methanesulfonic acid-κO)[2'-(methylamino-κN)[1,1'-biphenyl]-2-yl-κC]palladium (15.1 mg, 0.016 mmol) under nitrogen atmosphere. The mixture was heated to 100°C and stirred for 12 h under nitrogen atmosphere. The reaction mixture was added with water (10 mL) and extracted with ethyl acetate (10 mL × 3). The organic phases were combined, dried over anhydrous sodium sulfate, and filtered, and the filtrate was concentrated to give the residue. The residue was purified by preparative HPLC (C18, 32-72% gradient of water (formic acid)/acetonitrile) to give a white solid compound.

**Example 57A** (3.6 mg, 0.01 mmol, yield: 3.8%). LCMS (ESI): $[M+H]^+$ = 576.2. $^1H$ NMR (400 MHz, DMSO-d6) δ ppm 9.59 (br s, 1H), 7.49 (d, *J* = 8.0 Hz, 1H), 7.29 - 7.21 (m, 2H), 7.13 - 6.94 (m, 1H), 6.77 - 6.62 (m, 2H), 6.12 - 5.91 (m, 1H), 5.21 (br dd, *J* = 8.8, 15.2 Hz, 1H), 4.93 (br d, J= 11.6 Hz, 1H), 4.75 - 4.64 (m, 1H), 3.77 (t, *J* = 6.4 Hz, 2H), 3.30 (br s, 2H), 3.09 (br d, *J* = 12.4 Hz, 2H), 2.99 - 2.78 (m, 4H), 2.26 (s, 3H), 2.07 - 1.89 (m, 2H), 1.89 - 1.72 (m, 1H)

And a mixture of white solid **Example 57** (15.0 mg, 0.03 mmol, yield: 15.8%). $^1H$ NMR (400 MHz, DMSO-d6) δ ppm 10.55 - 10.02 (m, 1H), 8.76 (s, 1H), 7.77 (d, *J* = 8.4 Hz, 1H), 7.34 (dd, *J* = 2.0, 8.4 Hz, 1H), 7.26 (d, *J* = 2.4 Hz, 1H), 7.07 (s, 1H), 6.99 (d, *J*= 3.6 Hz, 1H), 6.82 (d, *J* = 3.2 Hz, 1H), 6.28 - 6.17 (m, 1H), 5.67 - 5.58 (m, 1H), 5.10 - 4.87 (m, 1H), 4.64 - 4.58 (m, 1H), 4.32 - 4.22 (m, 1H), 3.77 (t, *J* = 6.4 Hz, 3H), 3.16 - 3.08 (m, 2H), 2.95 - 2.80 (m, 3H), 2.31 - 2.29 (m, 3H), 2.16 - 1.96 (m, 2H), 1.86 - 1.76 (m, 1H)

**Example 58: N-($1^4$,$1^4$-difluoro-$2^6$-methyl-4-oxo-3-aza-2(2,4)-pyrimidin-1(1,3)-piperidin-6(2,5)-thiophene-5(1,2)-benzoazacyclonona ne-$5^4$-yl)-2-hydroxyethane-1-sulfonamide**

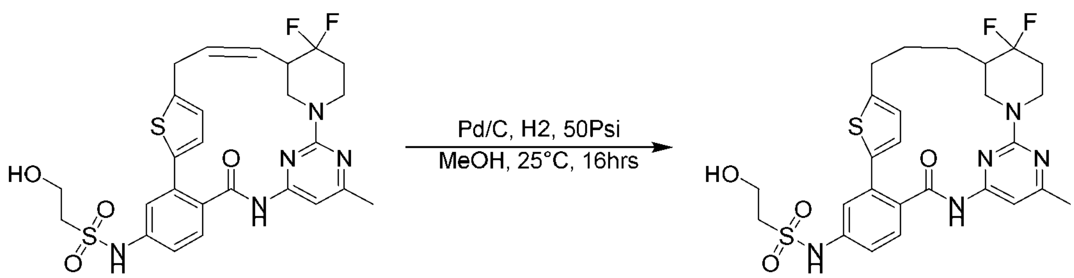


To a solution of N-($1^4$,$1^4$-difluoro-$2^6$-methyl-4-oxo-3-aza-2(2,4)-pyrimidin-1(1,3)-piperidin-6(2,5)-thiophene-5(1,2)-benzoaza-8-en-$5^4$-yl)-2-hydroxyethane-1-sulfonamide (20 mg, 0.03 mmol) in methanol (5.00 mL) was added 10% Pd/C (wet) (10.0 mg, 0.01 mmol), and the reaction mixture was **purged with argon three times** and then **hydrogen three times.** and stirred at 25°C for 16 h under hydrogen atmosphere (50 Psi). The reaction mixture was filtered under reduced pressure to remove palladium/carbon and then concentrated in vacuum. The residue was purified by preparative HPLC (C18, 42-72% gradient of water (formic acid)/acetonitrile) to give N-($1^4$,$1^4$-difluoro-$2^6$-methyl-4-oxo-3-aza-2(2,4)-pyrimidin-1(1,3)-piperidin-6(2,5)-thiophene-5(1,2)-benzoazacyclononane-5 4-yl)-2-hydroxyethane-1-sulfonamide (5.00 mg, 0.01 mmol, yield: 25%) as a white solid. LCMS (ESI): $[M+H]^+$ = 578.2. $^1H$ NMR (400 MHz, DMSO-$d_6$) δ ppm 10.93 - 9.53 (m, 1H), 9.18 - 8.96 (m, 1H), 7.59 (d, *J* = 8.4 Hz, 1H), 7.36 - 7.20 (m, 2H), 7.16 - 6.96 (m, 1H), 6.80 - 6.49 (m, 2H), 5.47 - 4.76 (m, 1H), 4.71 - 4.59 (m, 1H), 4.43 - 4.24 (m, 1H), 3.77 (t, *J* = 6.4 Hz, 2H), 3.30 (br s, 2H), 3.09 - 2.94 (m, 3H), 2.69 - 2.58 (m, 1H), 2.26 (s, 3H), 2.04 - 1.73 (m, 5H), 1.69 - 1.53 (m, 1H), 1.22 - 1.10 (m, 1H)

**Example 59: 2-hydroxy-N-($1^4$,$1^4$,$6^3$,$6^5$-tetrafluoro-$2^6$-methoxy-4-oxo-3-aza-2(2,4)-pyrimidin-1(1,3)-piperidin-5(1,2),6(1,4)-diphenyl heterocyclononane-54-yl)ethane-1-sulfonamide**

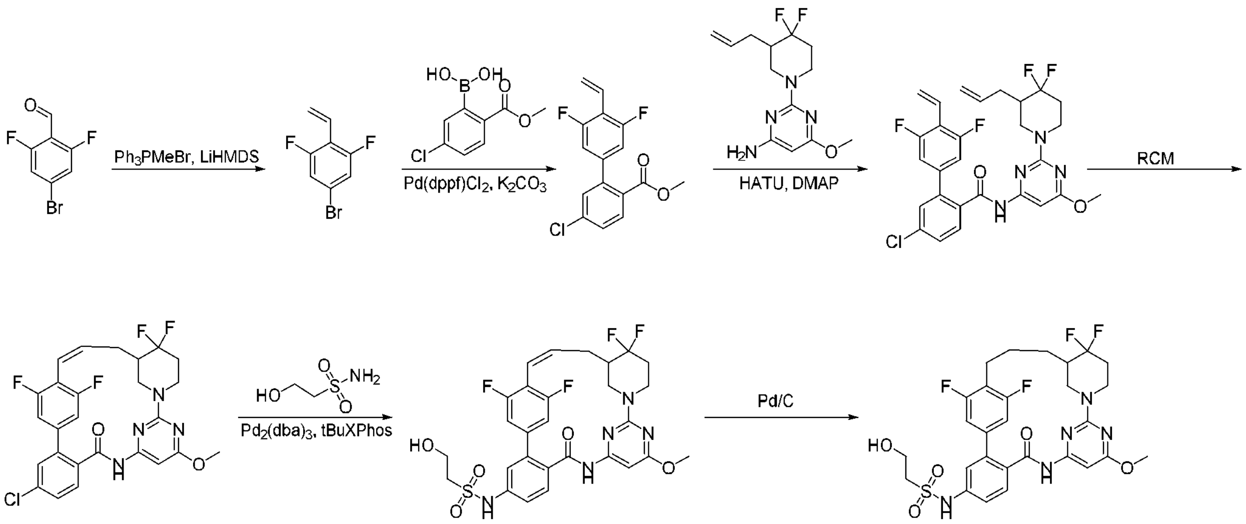


**Step 1:** To a solution of methyltriphenylphosphine bromide (24.70 g, 67.87 mmol) in tetrahydrofuran (100.00 mL) was added 1 M lithium bis(trimethylsilyl)amide (68.00 mL, 67.87 mol) at 0°C. After stirring for 1 h, 4-bromo-2,6-difluorobenzaldehyde (10.00 g, 45.25 mmol) was dissolved in tetrahydrofuran (20.00 mL) and slowly added dropwise to the reaction flask. The reaction solution was stirred at 25°C for 16 h, quenched with aqueous sodium bicarbonate solution (300 mL) and extracted with ethyl acetate (100 mL × 3). The organic phase was dried over anhydrous sodium sulfate, filtered, concentrated, and purified by column chromatography (silica gel, 0-100% gradient of ethyl acetate/petroleum ether) to give 5-bromo-1,3-difluoro-2-vinylbenzene (6.00 g, 27.39 mmol, yield: 60.5%) as a colorless oil. $^1H$ NMR (400 MHz, DMSO) δ 7.53 - 7.43 (m, 2H), 6.63 (dd, *J* = 18.0, 12.0 Hz, 1H), 6.04-5.92 (m, 1H), 5.76-5.65 (m, 1H) ppm.

**Step 2:** A solution of 5-bromo-1,3-difluoro-2-vinylbenzene (6.00 g, 27.39 mmol), (5-chloro-2-(methoxycarbonyl) phenyl)boric acid (8.80 g, 41.09 mmol), potassium carbonate (11.60 g, 82.18 mmol) and 1,1'-bis(diphenylphosphino) ferrocene palladium (II) dichloride (2.10 g, 2.74 mmol) in water (10.00 mL) and 1,4-dioxane (50.00 mL) was stirred at 80°C for 12 h under nitrogen atmosphere, diluted with water (30 mL) and extracted with ethyl acetate (30 mL × 3). The organic layer was dried over anhydrous sodium sulfate, filtered, concentrated, and purified by column chromatography (silica gel, 0-30% gradient of ethyl acetate/petroleum ether) to give 5-chloro-3',5'-difluoro-4'-vinyl-[1,1'-biphenyl]-2-carboxylate (3.0 g, 9.72 mmol, yield: 35.5%) as a white solid. LCMS (ESI): $[M+H]^+$ = 309.00.

**Step 3:** To a solution of 2-(3-allyl-4,4-difluoropiperidin-1-yl)-6-methoxypyrimidin-4-amine (3.00 g, 310.69 mmol) in tetrahydrofuran (30.00 mL) was slowly added dropwise 1 M lithium bis(trimethylsilyl)amide (19.00 mL, 19 mmol) at 0°C, and the mixture was stirred for 30 min. To the reaction solution was added 5-chloro-3',5'-difluoro-4'-vinyl-[1,1'-biphenyl]-2-carboxylate (3.0 g, 9.72 mmol), and the resulting mixture was stirred at 20°C for 12 h, diluted with water (50 mL) and extracted with ethyl acetate (50 mL × 3). The organic phase was dried over anhydrous sodium sulfate, filtered, concentrated, and purified by column chromatography (silica gel, 0-100% gradient of ethyl acetate/petroleum ether) to give N-(2-(3-allyl-4,4-difluoropiperidin-1-yl)-6-methoxypyrimidin-4-yl)-5-chloro-3',5'-difluoro-4'-vinyl-[1,1'-biphenyl]-2-carbox amide (2.10 g, 3.74 mmol, yield: 38.5%) as a yellow solid. LCMS (ESI): $[M+H]^+$ = 561.20.

**Step 4:** A solution of *N*-(2-(3-allyl-4,4-difluoropiperidin-1-yl)-6-methoxypyrimidin-4-yl)-5-chloro-3',5'-difluoro-4'-vinyl-[1,1'-biphenyl]-2-carbox amide (2.10 g, 3.74 mmol) and Grubbs catalyst 2nd generation (309 mg, 0.36 mmol) in dichloromethane (400 mL) was stirred at 50°C for 2 h, concentrated, and purified by column chromatography (silica gel, 0-30% gradient of ethyl acetate/petroleum ether) to give $5^4$-chloro-$1^4$,$1^4$,$6^3$,$6^5$-tetrafluoro-$2^6$-methoxy-3-aza-2(2,4)-pyrimidin-1(1,3)-piperidin-5(1,2),6(1,4)-dibenzocyclononan-7-en-4-one (1.00 g, 1.88 mmol, yield: 52.6%) as a white solid. LCMS (ESI): $[M+H]^+$ = 533.20.

**Step 5:** A solution of $5^4$-chloro-$1^4$,$1^4$,$6^3$,$6^5$-tetrafluoro-$2^6$-methoxy-3-aza-2(2,4)-pyrimidin-1(1,3)-piperidin-5(1,2),6(1,4)-diphenylheterocyclonona ne-7-en-4-one (1.00 g, 1.88 mmol), 2-hydroxyethanesulfonamide (474 mg, 3.75 mmol), potassium phosphate (1.20 g, 5.63 mmol), 2-di-tert-butylphosphino-2',4',6'-triisopropylbiphenyl (80 mg, 0.188 mmol) and tris(dibenzylideneacetone)dipalladium (175 mg, 0.188 mmol) in dioxane (20.00 mL) was stirred at 100°C for 12 h under nitrogen atmosphere, diluted with water (30 mL) and extracted with ethyl acetate (30 mL × 3). The organic phase was dried over anhydrous sodium sulfate, filtered, concentrated, and purified by column chromatography (silica gel, 0-100% gradient of ethyl acetate/petroleum ether) to give 2-hydroxy-N-($1^4$,$1^4$,$6^3$,$6^5$-tetrafluoro-$2^6$-methoxy-4-oxo-3-aza-2(2,4)-pyrimidin-1(1,3)-piperidin-5(1,2),6(1,4)-diphenylheter ocyclononane-7-en-$5^4$-yl)ethane-1-sulfonamide (760 mg, 1.22 mmol, yield: 65.2%) as a white solid. LCMS (ESI): $[M+H]^+$ =622.20.

**Step 6:** A solution of 2-hydroxy-N-($1^4$,$1^4$,$6^3$,$6^5$-tetrafluoro-$2^6$-methoxy-4-oxo-3-aza-2(2,4)-pyrimidin-1(1,3)-piperidin-5(1,2),6(1,4)-diphenylcyclo nonane-7-en-$5^4$-yl)ethane-1-sulfonamide (700 mg, 1.13 mmol) and wet palladium on carbon (120 mg, 0.113 mmol) in tetrahydrofuran (10.00 mL) was stirred for 12 h under hydrogen atmosphere (15 Psi), filtered, concentrated, and purified by column chromatography (C18, 0-100% gradient of acetonitrile/water) to give 2-hydroxy-N-($1^4$,$1^4$,$6^3$,$6^5$-tetrafluoro-$2^6$-methoxy-4-oxo-3-aza-2(2,4)-pyrimidin-1(1,3)-piperidin-5(1,2),6(1,4)-diphenylheter ocyclononane-$5^4$-yl)ethane-1-sulfonamide (93 mg, 0.15 mmol, yield: 13.2%) as a white solid. LCMS (ESI): $[M+H]^+$ =624.40; $^1$H NMR (400 MHz, DMSO) δ 10.20 (s, 1H), 9.39 (s, 1H), 7.66 (d, *J* = 8.4 Hz, 1H), 7.34 (dd, *J* = 8.4, 2.2 Hz, 1H), 7.29 (d, *J* = 2.4 Hz, 1H), 7.09 (d, *J* = 10.4 Hz, 1H), 6.74 (d, *J* = 10.2 Hz, 1H), 4.97 (s, 1H), 4.58 - 4.46 (m, 1H), 4.15 (d, *J* = 13.6 Hz, 1H), 3.88 - 3.73 (m, 6H), 3.39 - 3.34 (m, 2H), 3.19 - 3.05 (m , 2H), 2.82 - 2.74 (m , 1H), 2.62 - 2.54 (m, 1H), 2.03 - 1.78 (m, 4H), 1.67 - 1.55 (m, 1H), 1.30 - 1.16 (m, 1H), 1.12 - 1.01 (m, 1H) ppm.

**Example 60: *N*-($2^4$,$2^4$-difluoro-$1^1$-methoxy-7-oxo-8-aza-1(2,4)-pyrimidin-2(1,3)-piperidin-5(2,5)-thiophene-6(1,2)-benzenecycloocta ne-3-en-$6^5$-yl)-2-hydroxyethane-1-sulfonamide**

**Example 61: *N*-($2^4$,$2^4$-difluoro-$1^6$-methoxy-7-oxo-8-aza-1(2,4)-pyrimidin-2(1,3)-piperidin-5(2,5)-thiophene-6(1,2)-benzeneheterocyc looctan-$6^3$-yl)-2-hydroxyethane-1-sulfonamide**

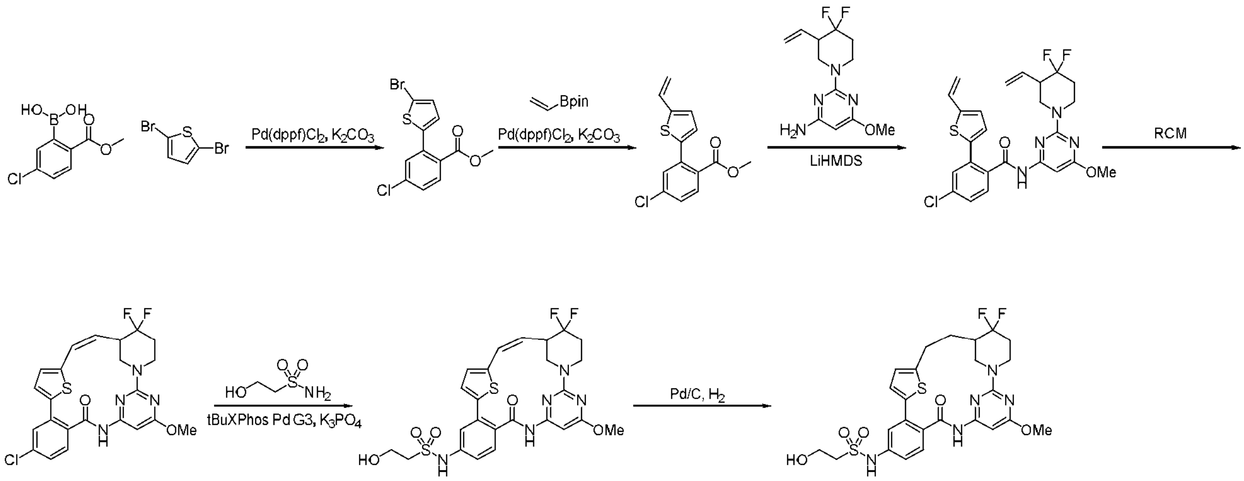


**Step 1:** A solution of (5-chloro-2-(methoxycarbonyl)phenyl)boric acid (5.00 g, 23.32 mmol), 2,5-dibromothiophene (8.46 g, 34.98 mmol), potassium carbonate (9.87 g, 69.96 mmol) and bis(diphenylphosphino)ferrocene palladium dichloride (1.76 g, 2.33 mmol) in 1,2-dioxane (100.00 mL) and water (20.00 mL) was stirred at 100°C for 16 h under nitrogen atmosphere. After the reaction was complete, the solution was quenched with water (100 mL) and extracted with ethyl acetate (100 mL × 3), and the organic phase was dried over anhydrous sodium sulfate, filtered, concentrated, and purified by column chromatography (silica gel, 0-10% gradient of ethyl acetate/petroleum ether) to give 2-(5-bromothiophene-2-yl)-4-chlorobenzoate (7.00 g, 21.11 mmol, yield: 90.5%) as a yellow oil. $^1$H NMR (400 MHz, Chloroform-d) δ 7.72 (d, *J* = 8.2 Hz, 1H), 7.42 (d, *J* = 2.1 Hz, 1H), 7.39 (dd, *J* = 8.3, 2.1 Hz, 1H), 7.02 (d, *J* = 3.8 Hz, 1H), 6.80 (d, *J* = 3.8 Hz, 1H), 3.77 (s, 3H) ppm.

**Step 2:** A solution of 2-(5-bromothiophene-2-yl)-4-chlorobenzoate (7.00 g, 21.11 mmol), pinacol vinylboronate (3.90 g, 23.22 mmol), potassium carbonate (8.93 g, 63.33 mmol) and tetrakis(triphenylphosphine)palladium (2.49 g, 2.11 mmol) in dioxane (100.00 mL) and water (20.00 mL) was stirred at 100°C for 16 h under nitrogen atmosphere. After the reaction was complete, the solution was quenched with water (100 mL) and extracted with ethyl acetate (100 mL x 3), and the organic layer was dried over anhydrous sodium sulfate, filtered, concentrated, and purified by column chromatography

(silica gel, 0-10% gradient of ethyl acetate/petroleum ether) to give 2-(5-vinylthiophene-2-yl)-4-chlorobenzoate (2.21 g, 7.93 mmol, yield: 37.6%) as a yellow oil.

**Step 3:** To a solution of 2-(4,4-difluoro-3-vinylpiperidin-1-yl)-6-methylpyrimidin-4-amine (2.14 g, 7.93 mmol) in tetrahydrofuran (50 mL) was added dropwise 1.0M bis(trimethylsilyl)amino lithium (7.93 mL, 7.93 mmol) at 0°C under nitrogen atmosphere, and the mixture was stirred for 30 min. To the solution was added dropwise methyl 2-(5-vinylthiophene-2-yl)-4-chlorobenzoate (2.21 g, 7.93 mmol). The reaction solution was stirred at 20°C for 3 h, diluted with water (50 mL) and extracted with ethyl acetate (50 mL × 3), and the organic phase was dried over anhydrous sodium sulfate, filtered, concentrated, and purified by column chromatography (silica gel, 0-10% gradient of ethyl acetate/petroleum ether) to give 4-chloro-N-(2-(4,4-difluoro-3-vinylpiperidin-1-yl)-6-methylpyrimidin-4-yl)-2-(5-vinylthiophene-2-yl) benzamide (2.50 g, 4.84 mmol, yield: 61.0%) as a yellow oil. LCMS (ESI): $[M+H]^+$ = 517.20.

**Step 4:** A solution of 4-chloro-*N*-(2-(4,4-difluoro-3-vinylpiperidin-1-yl)-6-methylpyrimidin-4-yl)-2-(5-vinylthiophene-2-yl)benzamide (2.50 g, 4.84 mmol) and Grubbs catalyst 2nd generation (409 mg, 0.48 mmol) in dichloromethane (200.00 mL) was stirred at 50°C for 2 h under nitrogen atmosphere, concentrated, and purified by column chromatography (silica gel, 0-30% gradient ethyl acetate/petroleum ether) to give $6^5$-chloro-$2^4$,$2^4$-difluoro-$1^6$-methoxy-8-aza-1(2,4)-pyrimidin-2(1,3)-piperidin-5(2,5)-thiophene-6(1,2)-benzenecyclooct-3-en -7-one (1.89 g, 3.87 mmol, yield: 79.9%) as a white solid. LCMS (ESI): $[M+H]^+$ = 489.20.

**Step 5:** A solution of $6^5$-chloro-$2^4$,$2^4$-difluoro-$1^6$-methoxy-8-aza-l(2,4)-pyrimidin-2(1,3)-piperidin-5(2,5)-thiophene-6(1,2)-benzenecyclooctane-3 -en-7-one (1.89 g, 3.87 mmol), 2-hydroxyethanesulfonamide (1.45 g, 11.61 mmol), potassium phosphate (3.29 g, 15.48 mmol), 2-di-tert-butylphosphino-2',4',6'-triisopropylbiphenyl (187 mg, 0.39 mmol) and tris(dibenzylideneacetone)dipalladium (357 mg, 0.39 mmol) in dioxane (25.00 mL) was stirred at 100°C for 12 h, diluted with water (30 mL) and extracted with ethyl acetate (50mL x 3) under nitrogen atmosphere. The organic phase was dried over anhydrous sodium sulfate, filtered, concentrated, and purified by column chromatography (silica gel, 0-100% gradient of ethyl acetate/petroleum ether) to give **Example 60** N-($2^4$,$2^4$-difluoro-16-methoxy-7-oxo-8-aza-1(2,4)-pyrimidin-2(1,3)-piperidin-5(2,5)-thiophene-6(1,2)-benzenecyclooctane-3 -en-$6^5$-yl)-2-hydroxyethane-1-sulfonamide (1.02 g, 1.77 mmol, yield: 45.7%) as a yellow solid. LCMS (ESI): $[M+H]^+$ =578.20.

**Step 6:** A solution of *N*-($2^4$,$2^4$-difluoro-$1^6$-methoxy-7-oxo-8-aza-1(2,4)-pyrimidin-2(1,3)-piperidin-5(2,5)-thiophene-6(1,2)-benzenecyclooctane-3 -en-$6^5$-yl)-2-hydroxyethane-1-sulfonamide (400 mg, 0.69 mmol) and wet palladium on carbon (726 mg, 0.68 mmol) in tetrahydrofuran (50 mL) was stirred at 25°C for 12 h under hydrogen atmosphere (15 psi), filtered, concentrated, and purified by column chromatography (C18, 0-100% gradient of acetonitrile/water) to give **Example 61** *N*-($2^4$,$2^4$-difluoro-$1^6$-methoxy-7-oxo-8-aza-1(2,4)-pyrimidin-2(1,3)-piperidin-5(2,5)-thiophene-6(1,2)-benzeneheterocyclooc tan-$6^3$-yl)-2-hydroxyethane-1-sulfonamide (159.0 mg, 0.27 mmol, yield: 37.8%) as a white solid. LCMS (ESI): $[M+H]^+$ =580.30

**Example 62: N-($2^4$,$2^4$-difluoro-$1^6$-methyl-7-oxo-8-aza-1(2,4)-pyrimidin-2(1,3)-piperidin-5(2,5)-thiophene-6(1,2)-benzeneheterocycl ooct-3-en-$6^5$-yl)-2-hydroxyethane-1-sulfonamide**

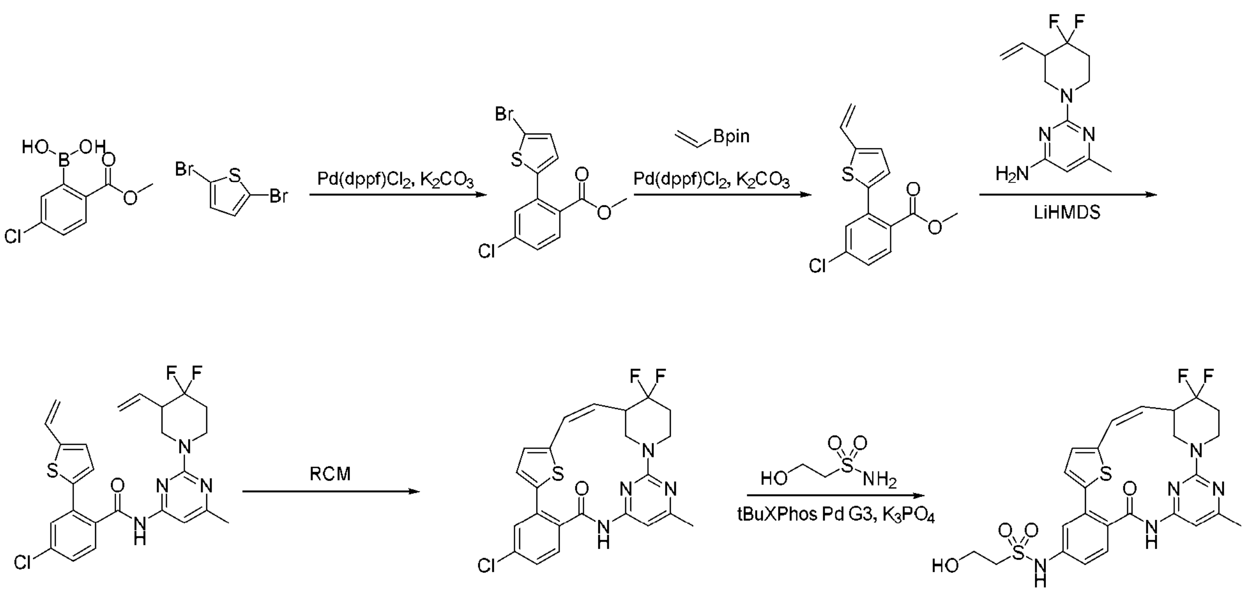

**Example 62** was prepared using the method for Example 60. LCMS (ESI): $[M+H]^+$ =562.10

**Example 63: *N-($2^4$,$2^4$-difluoro-$1^6$-methyl-7-oxo-8-aza-1(2,4)-pyrimidin-2(1,3)-piperidin-5(2,5)-thiophene-6(1,2)-benzeneheterocycl* ooctan-$6^5$-yl)-2-hydroxyethane-1-sulfonamide**

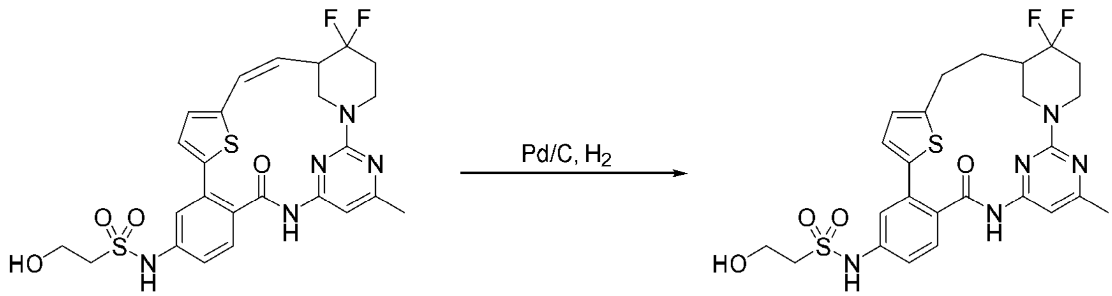


**Example 63** was prepared using the method for **Example 61.** LCMS (ESI): $[M+H]^+$ =564.10

**Example 64: N-($1^4$,$1^4$-difluoro-$2^6$-methoxy-4-oxo-3-aza-2(2,4)-pyrimidin-1(1,3)-piperidin-6(2,5)-thiophene-5(1,2)-benzoazacyclon-8 -en-$5^4$-yl)-2-hydroxyethane-1-sulfonamide**

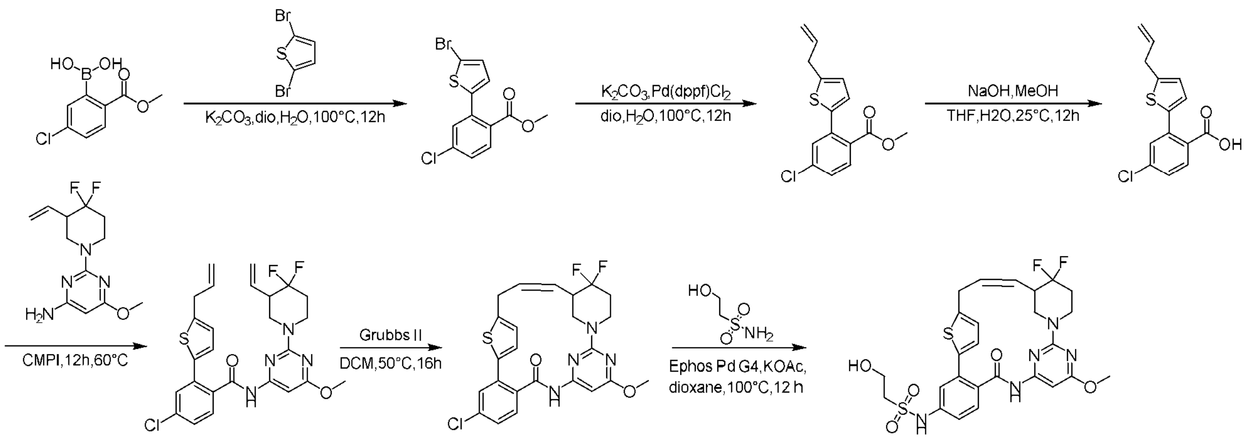


**Example 64** was prepared using the method for **Example 57.** LCMS (ESI): $[M+H]^+$ =592.10

**Example 65: N-($1^4$,$1^4$-difluoro-$2^6$-methoxy-4-oxo-3-aza-2(2,4)-pyrimidin-1(1,3)-piperidin-6(2,5)-thiophene-5(1,2)-benzoazacyclono nane-$5^4$-yl)-2-hydroxyethane-1-sulfonamide**

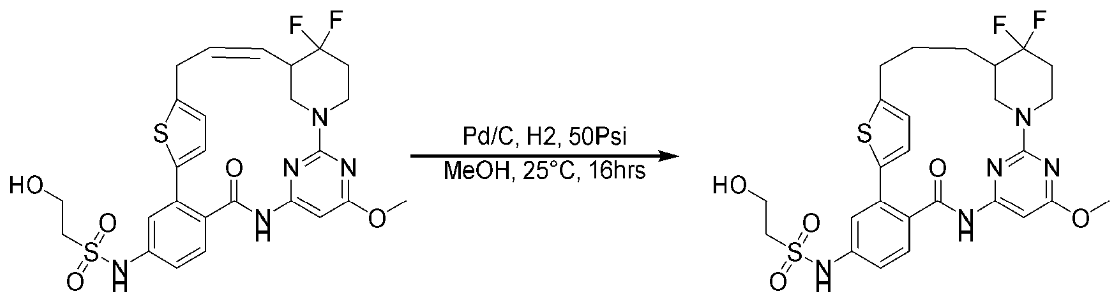

**Example 65** was prepared using the method for Example 61. LCMS (ESI): [M+H]+ =594.10

**Example 66: N-($1^4$,$1^4$-difluoro-$2^6$-methyl-4-oxo-3-aza-2(2,4)-pyrimidin-1(1,3)-piperidin-6(2,4)-thiophene-5(1,2)-benzoazacyclon-8-e n-$5^4$-yl)-2-hydroxyethane-1-sulfonamide**

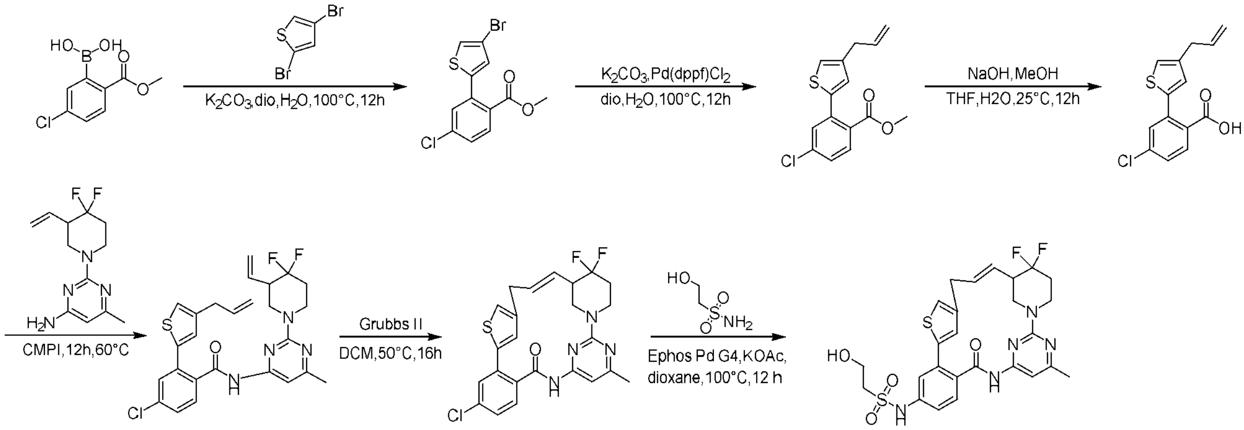


**Example** 66 was prepared using the method for Example 57. LCMS (ESI): [M+H]+ =576.10

**Example 67: *N-($2^4$,$2^4$-difluoro-$1^6$-methyl-7-oxo-8-aza-1(2,4)-pyrimidin-2(1,3)-piperidin-5(2,4)-thiophene-6(1,2)-benzeneheterocycl* ooct-3-en-$6^5$-yl)-2-hydroxyethane-1-sulfonamide**

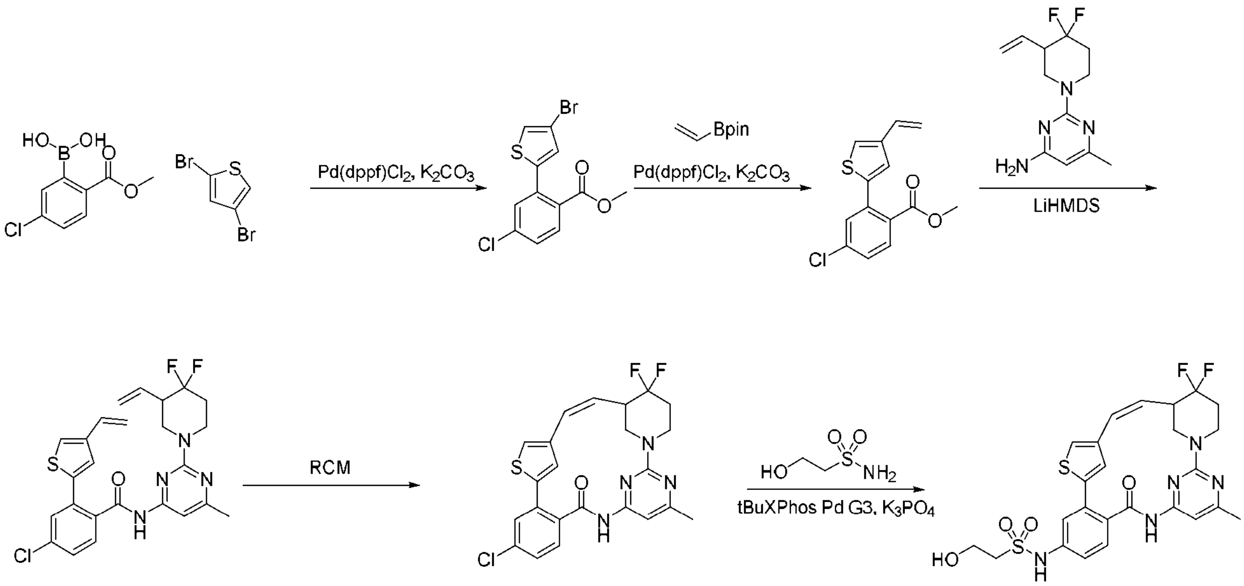


**Example 67 was** prepared using the method for **Example 62.** LCMS (ESI): [M+H]+ =562.10

**Example 68: N-($1^4$,$1^4$-difluoro-$2^6$-methyl-4-oxo-3-aza-2(2,4)-pyrimidin-1(1,3)-piperidin-6(2,4)-thiophene-5(1,2)-benzoazacyclonona ne-$5^4$-yl)-2-hydroxyethane-1-sulfonamide**

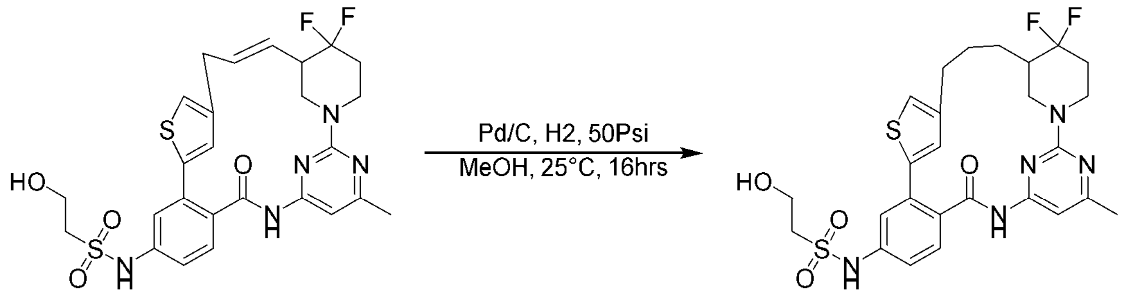


**Example 68** was prepared using the method for **Example 58.** LCMS (ESI): $[M+H]^+$ =578.20

**Example 69: N-($2^4$,$2^4$-difluoro-$1^6$-methyl-7-oxo-8-aza-1(2,4)-pyrimidin-2(1,3)-piperidin-5(2,4)-thiophene-6(1,2)-benzeneheterocycl ooctan-$6^5$-yl)-2-hydroxyethane-1-sulfonamide**

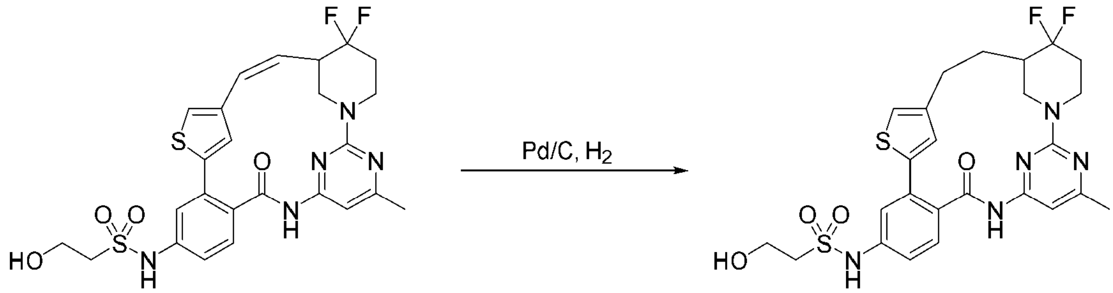


**Example 69** was prepared using the method for **Example 61.** LCMS (ESI): $[M+H]^+$ =564.10

Example 70: ***N*-($5^6$,8-dimethyl-3-oxo-4,6-diaza-5(4,2)-pyrimidin-1(2,5)-thiophene-2(1,2)-benzenecyclodec-9-en-25-yl)-2-hydroxyeth ane-1-sulfonamide**

**Example 71: N-($5^6$,8-dimethyl-3-oxo-4,6-diaza-5(4,2)-pyrimidin-1(2,5)-thiophene-2(1,2)-benzeneheterocyclodecan-25-yl)-2-hydrox yethane-1-sulfonamide**

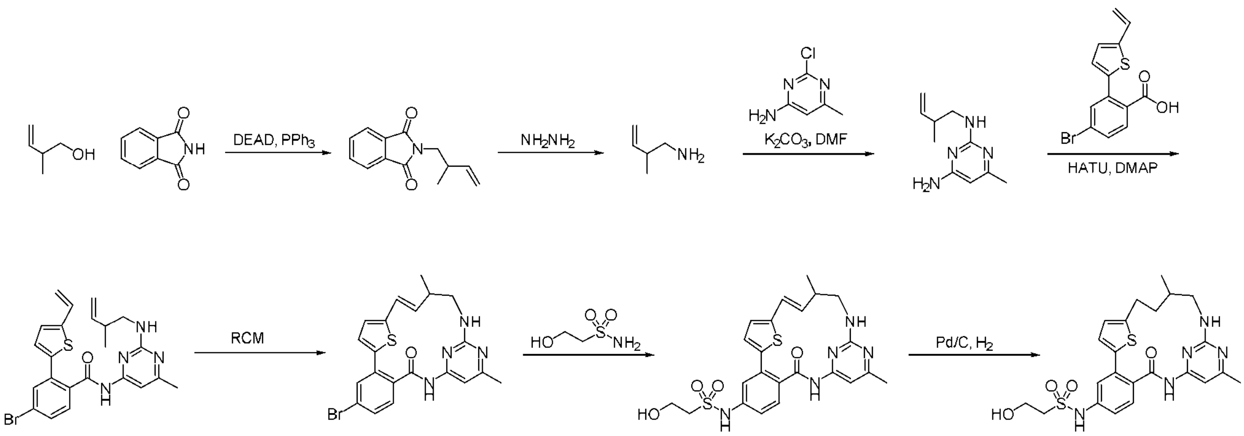


**Step 1:** To a solution of triphenylphosphine (32.79 g, 125.00 mmol), phthalimide (18.39 g, 125.00 mmol) and 2-methylbut-3-en-1-ol (10.77 g, 125.00 mmol) in tetrahydrofuran (150.00 mL) was added diethyl azodicarboxylate (21.75 g, 125.00 mmol) slowly at 0°C. The temperature was raised to 20°C and the mixture was stirred for 3 hours to complete the reaction. To the solution was added petroleum ether (300 mL), and the mixture was stirred for 30 min, filtered, and concentrated to give 2-(2-methylbut-3-en-1-yl)isoindolin-1,3-dione (37.0 g, crude). LCMS (ESI): $[M+H]^+$ = 216.30.

**Step 2:** To a solution of 2-(2-methylbut-3-en-1-yl)isoindoline-1,3-dione (37.0 g, crude) in ethanol (500.00 mL) was added hydrazine hydrate (8.82 g, 150.00 mmol), and the mixture was stirred at 50°C for 6 h. To the reaction solution was added 6M hydrochloric acid, and the mixture was filtered to remove solids, dried over anhydrous sodium sulfate, and concentrated to give 2-methylbut-3-en-1-amine hydrochloride (16.3 g, crude).

**Step 3:** A solution of 2-methylbut-3-en-1-amine hydrochloride (16.3 g, crude), 2-chloro-6-methylpyrimidin-4-amine (5.98 g, 41.67 mmol) and potassium carbonate (20.75 g, 150.00 mmol) in N,N-dimethylformamide (150.00 mL) was stirred at 100°C for 16 h. After the reaction was complete, the solution was quenched with sodium chloride solution (300 mL), extracted with ethyl acetate (150 mL × 3), washed with sodium chloride solution, dried over anhydrous sodium sulfate, filtered, concentrated, and purified by column chromatography (silica gel, 30-50% gradient of ethyl acetate/petroleum ether) to give 6-methyl-$N^2$-(2-methylbut-3-en-1-yl)pyrimidin-2,4-diamine (5.20 g, 27.05 mmol, yield: 64.9%) as a yellow solid. LCMS (ESI): $[M+H]^+$ = 193.30.

**Step 4:** A solution of 6-methyl-$N^2$-(2-methylbut-3-en-1-yl)pyrimidin-2,4-diamine (2.00 g, 10.40 mmol), 4-chloro-2-(5-vinylthiophene-2-yl)benzoic acid (2.75 g, 10.40 mmol), N,N,N',N'-tetramethyl-O-(7-azabenzotriazol-1-yl) urea hexafluorophosphate (11.86 g, 31.20 mmol) and 4-dimethylaminopyridine (5.08 g, 41.60 mmol) in dichloroethane (100.00 mL) was heated to 80°C and stirred for 24 h. After the reaction was complete, the solution was quenched with sodium bicarbonate (50 mL) and extracted with dichloromethane (50 mL x 3), and the organic phases were combined, dried, filtered, and concentrated to give the crude product. The crude product was purified by flash column chromatography (silicon dioxide, 0-20% ethyl acetate/petroleum ether) to give 4-chloro-N-(6-methyl-2-((2-methylbut-3-en-1-yl) amino)pyrimidin-4-yl)-2-(5-vinylthiophene-2-yl)benzamide (2.54 g, 5.79 mmol, yield: 55.7%) as a yellow solid. LCMS (ESI): $[M+H]^+$ = 483.50.

**Step 5: A** solution of 4-chloro-N-(6-methyl-2-((2-methylbut-3-en-1-yl)amino)pyrimidin-4-yl)-2-(5-vinylthiophene-2-yl)benzamide (2.54 g, 5.79 mmol) and Grubbs catalyst 2nd generation (495 mg, 0.58 mmol) in dichloromethane (300.00 mL) was stirred at 50°C for 16 h, concentrated, and purified by column chromatography (silica gel, 0-30% gradient of ethyl acetate/petroleum ether) to give $2^5$-chloro-$5^6$,8-dimethyl-4,6-diaza-5(4,2)-pyrimidin-1(2,5)-thiophene-2(1,2)-benzenecyclodec-9-en-3-one (1.52 g, 3.70 mmol, yield: 63.9%) as a yellow solid. LCMS (ESI): $[M+H]^+$ = 411.40.

**Step 6:** A solution of $2^5$-chloro-$5^6$,8-dimethyl-4,6-diaza-5(4,2)-pyrimidin-1(2,5)-thiophene-2(1,2)-benzenecyclodec-9-en-3-one (1.52 g, 3.70 mmol), 2-hydroxyethanesulfonamide (1.37 g, 11.10 mmol), potassium phosphate (3.12 g, 14.80 mmol), 2-di-tert-butylphosphino-2',4',6'-triisopropylbiphenyl (177 mg, 0.37 mmol) and tris(dibenzylideneacetone) dipalladium (338 mg, 0.37 mmol) in dioxane (25.00 mL) was stirred at 100°C for 12 h under nitrogen atmosphere, diluted with water (30 mL) and extracted with ethyl acetate (50 mL × 3). The organic phase was dried over anhydrous sodium sulfate, filtered, concentrated, and purified by column chromatography (silica gel, 0-100% gradient of ethyl acetate/petroleum ether) to give N-($5^6$,8-dimethyl-3-oxo-4,6-diaza-5(4,2)-pyrimidin-1(2,5)-thiophene-2(1,2)-benzenecyclodec-9-en-$2^5$-yl)-2-hydroxyethane-1-sulfonamide (1.40 g, 2.80 mmol, yield: 75.8%) as a light yellow solid. LCMS (ESI): $[M+H]^+$ =500.20.

**Step 7:** A solution of *N*-($5^6$,8-dimethyl-3-oxo-4,6-diaza-5(4,2)-pyrimidin-1(2,5)-thiophene-2(1,2)-benzenecyclodec-9-en-$2^5$-yl)-2-hydroxyethane-1-sulfonamide (500 mg, 1.00 mmol) and wet palladium on carbon (1.00 g) in tetrahydrofuran (50.00 mL) was stirred at 25°C for 12 h under hydrogen atmosphere (15 Psi), filtered, concentrated, and purified by column chromatography (C18, 0-100% gradient of acetonitrile/water) to give *N*-($5^6$,8-dimethyl-3-oxo-4,6-diaza-5(4,2)-pyrimidin-1(2,5)-thiophene-2(1,2)-benzeneheterocyclodecan-$2^5$-yl)-2-hydroxyetha ne-1-sulfonamide (259.0 mg, 0.52 mmol, yield: 51.6%) as a white solid. LCMS (ESI): $[M+H]^+$ =502.30

**Example 72: 2-hydroxy-N-($5^6$-methyl-3-oxo-9-oxa-4,6-diaza-5(4,2)-pyrimidin-1(2,5)-thiophene-2(1,2)-benzene-cyclodec-$2^5$-yl)ethan e-1-sulfonamide**

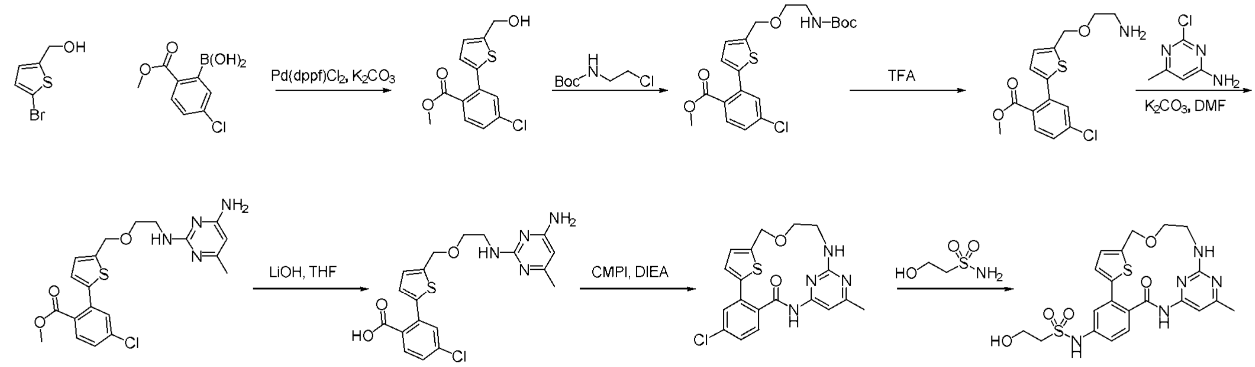

**Step 1:** A solution of (5-chloro-2-(methoxycarbonyl)phenyl)boric acid (5.00 g, 23.32 mmol), (5-bromothiophene-2-yl)methanol (4.50 g, 23.32 mmol), potassium carbonate (9.87 g, 69.96 mmol) and bis(diphenylphosphino) ferrocene palladium dichloride (1.76 g, 2.33 mmol) in 1,2-dioxane (100.00 mL) and water (20.00 mL) was stirred at 100°C for 16 h under nitrogen atmosphere. After the reaction was complete, the solution was quenched with water (100 mL) and extracted with ethyl acetate (100 mL × 3), and the organic phase was dried over anhydrous sodium sulfate, filtered, concentrated, and purified by column chromatography (silica gel, 0-30% gradient of ethyl acetate/petroleum ether) to give 2-(5-bromothiophene-2-yl)-4-chlorobenzoate (5.60 g, 19.81 mmol, yield: 84.9%) as a yellow solid. LCMS (ESI): $[M+H-H_2O]^+$=265.10.

**Step 2:** To a solution of methyl 2-(5-bromothiophene-2-yl)-4-chlorobenzoate (5.60 g, 19.81 mmol) in N,N-dimethylformamide (100.00 mL) was added 60% sodium hydride (4.00 g, 100.00 mmol) at 0°C, and the reaction solution was stirred for 30 min, followed by the addition of N-tert-butoxycarbonyl-2-chloroethylamine (10.78 g, 60.00 mmol). The temperature was raised to 60°C and the mixture was stirred for 6 hours to complete the reaction. The mixture was quenched with water (200 mL) and extracted with ethyl acetate (100 mL × 3), and the organic phase was dried over anhydrous sodium sulfate, filtered, concentrated, and purified by column chromatography (silica gel, 0-30% gradient of ethyl acetate/petroleum ether) to give 2-(5-((2-((tert-butoxycarbonyl)amino)ethoxy)methyl)thiophene-2-yl)-4-chlorobenzoate (4.69 g, 11.03 mmol, yield: 55.7%) as a yellow solid. LCMS (ESI): $[M-55]^+$ = 370.10.

**Step 3:** To a solution of methyl 2-(5-((2-(tert-butoxycarbonyl)amino)ethoxy)methyl)thiophene-2-yl)-4-chlorobenzoate (4.69 g, 11.03 mmol) in dichloromethane (100.00 mL) was added trifluoroacetic acid (20.00 mL), and the reaction solution was stirred for 2 h and concentrated to give methyl 2-(5-((2-aminoethoxy)methyl)thiophene-2-yl)-4-chlorobenzoate (7.30 g, crude) as a yellow liquid. LCMS (ESI): $[M+H]^+$ = 326.10.

**Step 4:** A solution of 2-(5-((2-aminoethoxy)methyl)thiophene-2-yl)-4-chlorobenzoat (7.30 g, crude), 2-chloro-6-methylpyrimidin-4-amine (2.41 g, 16.71 mmol) and potassium carbonate (4.58 g, 33.09 mmol) in N,N-dimethylformamide (50.00 mL) was stirred at 130°C for 16 h. After the reaction was complete, the solution was quenched with sodium chloride solution (300 mL), extracted with ethyl acetate (150 mL × 3), washed with sodium chloride solution, dried over anhydrous sodium sulfate, filtered, concentrated, and purified by column chromatography (silica gel, 30-80% gradient of ethyl acetate/petroleum ether) to give 2-(5-((2-((4-amino-6-methylpyrimidin-2-yl)amino)ethoxy)methyl)thiophene-2-yl)-4-chlorobenzoate (3.24 g, 7.48 mmol, yield: 67.2%) as a yellow solid. LCMS (ESI): $[M+H]^+$ = 433.20.

**Step 5:** To a solution of 2-(5-((2-((4-amino-6-methylpyrimidin-2-yl)amino)ethoxy)methyl)thiophene-2-yl)-4-chlorobenzoate (3.24 g, 7.48 mmol) in tetrahydrofuran (20.00 mL) and methanol (20.00 mL) was added 2M sodium hydroxide solution (10.00 mL, 20.00 mmol), and the mixture was stirred at 50°C for 6 h. The reaction solution was quenched with 2M hydrochloric acid (10 mL) to adjust the pH to 5. The solution was concentrated under reduced pressure to remove solvent and water and to give 2-(5-((2-((4-amino-6-methylpyrimidin-2-yl)amino)ethoxy)methyl)thiophene-2-yl)-4-chlorobenzoic acid (4.43 g, crude). LCMS (ESI): $[M+H]^+$ = 418.20.

Step 6: A solution of 2-(5-((2-((4-amino-6-methylpyrimidin-2-yl)amino)ethoxy)methyl)thiophene-2-yl)-4-chlorobenzoic acid (4.43 g, crude), 2-chloro-1-methylpyridinium iodide (3.82 g, 14.96 mmol) and N,N-diisopropylethylamine (4.83 g, 37.40 mmol) in tetrahydrofuran (750.00 mL) was stirred at 50°C for 16 h. The solution was quenched with sodium bicarbonate solution (300 mL), extracted with ethyl acetate (150 mL x 3), washed with sodium chloride solution, dried over anhydrous sodium sulfate, filtered, concentrated, and purified by column chromatography (silica gel, 0-30% gradient of ethyl acetate/petroleum ether) to give $2^5$-chloro-$5^6$-methyl-9-oxa-4,6-diaza-5(4,2)-pyrimidin-1(2,5)-thiophene-2(1,2)-benzenecyclodec-3-one (1.41 g, 3.49 mmol, yield: 46.67%) as a yellow solid. LCMS (ESI): $[M+H]^+$ = 401.20.

**Step 7:** A solution of $2^5$-chloro-$5^6$-methyl-9-oxa-4,6-diaza-5(4,2)-pyrimidin-1(2,5)-thiophene-2(1,2)-benzenecyclodec-3-one (1.41 g, 3.49 mmol), 2-hydroxyethanesulfonamide (1.29 g, 10.47 mmol), potassium phosphate (2.95 g, 13.96 mmol), 2-di-tert-butylphosphino-2',4',6'-triisopropylbiphenyl (167 mg, 0.35 mmol) and tris(dibenzylideneacetone) dipalladium (320 mg, 0.35 mmol) in dioxane (25.00 mL) was stirred at 100°C for 12 h under nitrogen atmosphere, diluted with water (30 mL) and extracted with ethyl acetate (50 mL × 3). The organic phase was dried over anhydrous sodium sulfate, filtered, concentrated, and purified by column chromatography (silica gel, 0-100% gradient of ethyl acetate/petroleum ether) to give 2-hydroxy-N-($5^6$-methyl-3-oxo-9-oxa-4,6-diaza-5(4,2)-pyrimidin-1(2,5)-thiophene-2(1,2)-benzenecyclodec-$2^5$-yl)ethane-1-s ulfonamide (923 mg, 1.89 mmol, yield: 54.0%) as a light yellow solid. LCMS (ESI): $[M+H]^+$ =490.20.

**Example 73: N-($5^6$,8-dimethyl-3-oxo-10-oxa-4,6-diaza-5(4,2)-pyrimidin-1(2,5)-thiophene-2(1,2)-benzocycloundecane-$2^5$-yl)-2-hydr oxyethane-1-sulfonamide**

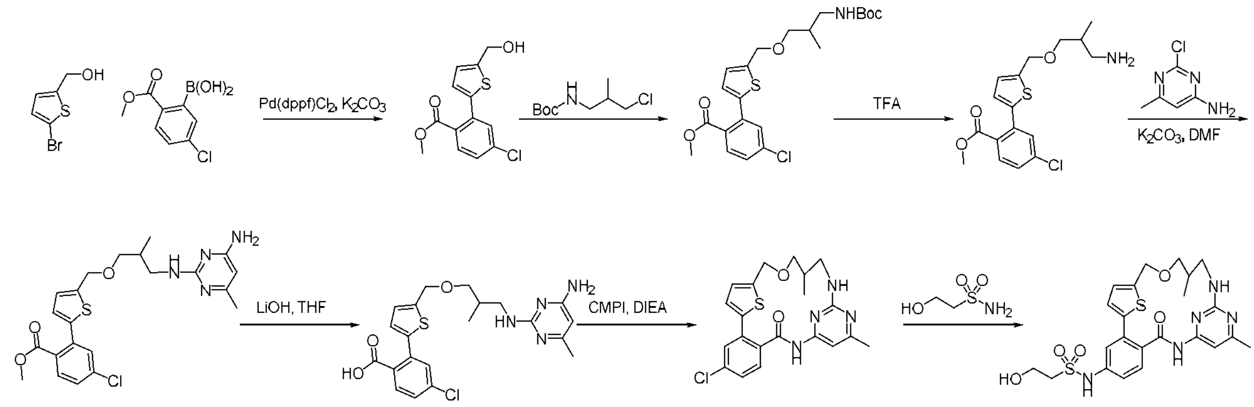


**Example 73** was prepared using the method for **Example 72.** LCMS (ESI): $[M+H]^+$ =518.10

**Example 74: N-(5$^6$,8-dimethyl-3-oxo-10-oxa-4,6-diaza-5(4,2)-pyrimidin-1(2,4)-thiophene-2(1,2)-benzocycloundecane-2$^5$-yl)-2-hydr oxyethane-1-sulfonamide**

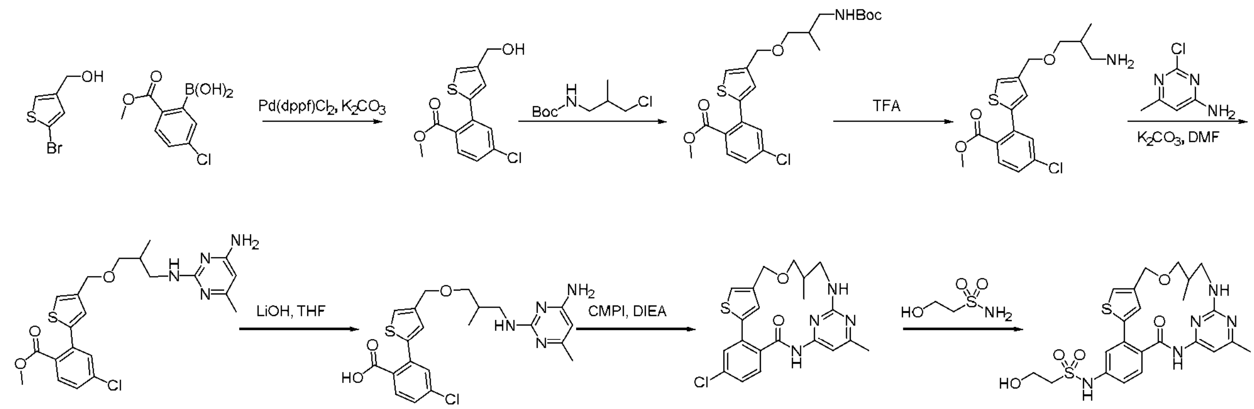


**Example 74 was prepared using the method for Example 72. LCMS (ESI): $[M+H]^+$ =518.10**

**Example 75: N-(6',8'-dimethyl-3'-oxospiro[cyclopropane-1,11'-10-oxa-4,6-diaza-5(4,2)-pyrimidin-1(2,5)-thiophene a-2(1,2)-benzocycloundecane]-5'-yl)-2-hydroxyethane-1-sulfonamide**

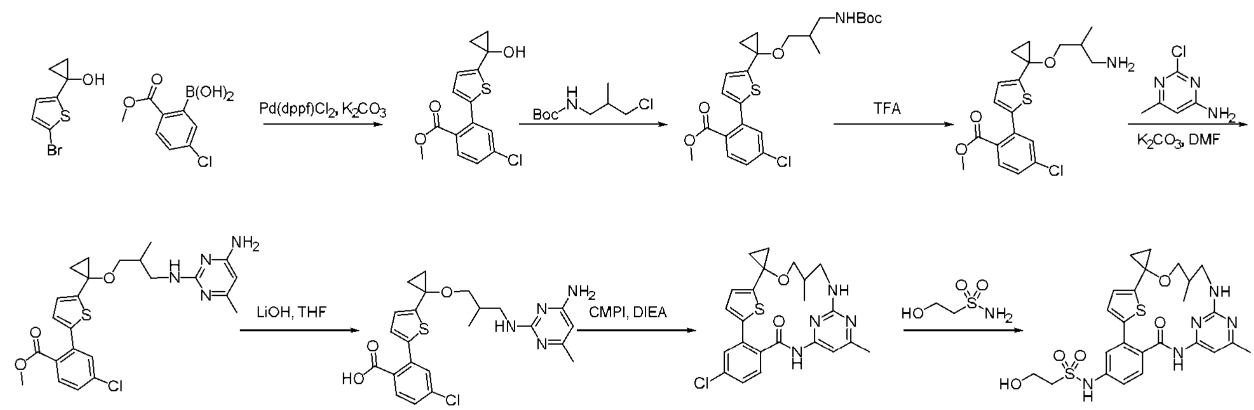


**Example 75 was prepared** using the method for **Example 72.** LCMS (ESI): $[M+H]^+$ =544.20

**Example 76: 2-Hydroxy-N-($5^6$,8,11,11-tetramethyl-3-oxo-10-oxa-4,6-diaza-5(4,2)-pyrimidin-1(2,4)-thiophene-2(1,2)-benzocyclound ecane-$2^5$-yl)ethane-1-sulfonamide**

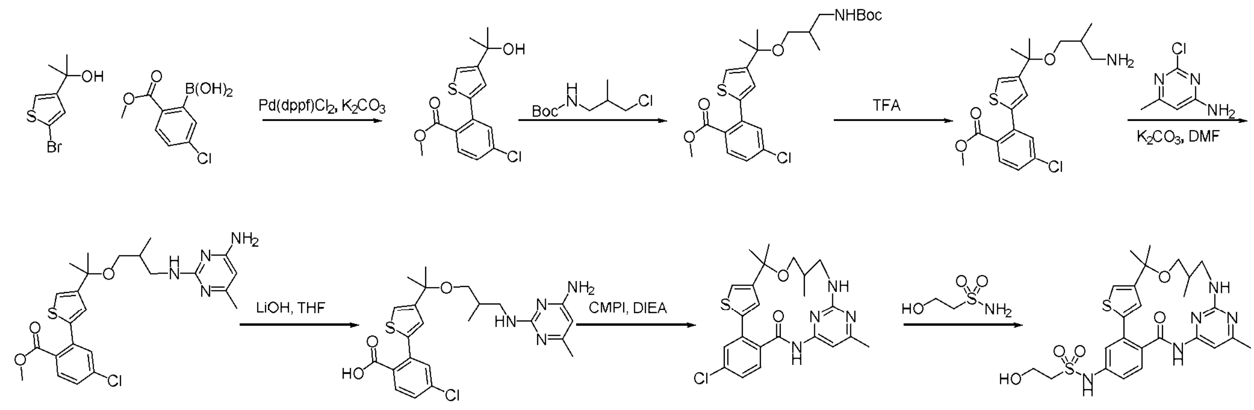


**Example 76** was prepared using the method **for Example 72.** LCMS (ESI): $[M+H]^+$ =546.20

**Example 77: 2-Hydroxy-N-(8-methyl-3-oxo-10-oxa-4,6-diaza-5(2,6)-pyridin-1(2,5)-thiophene-2(1,2)-benzocycloundecane-$2^5$-yl)eth ane-1-sulfonamide**

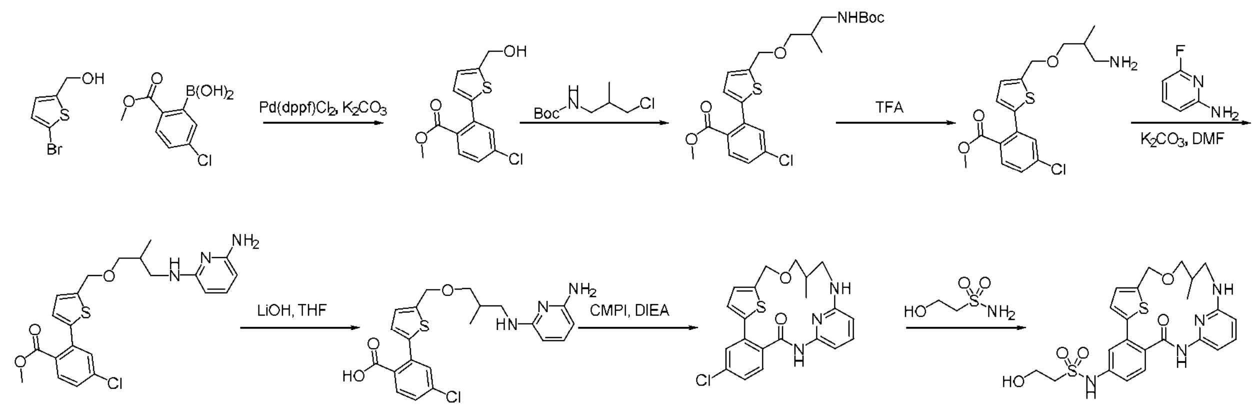


**Example** 77 was prepared using the method **for Example** 72. LCMS (ESI): $[M+H]^+$ =503.10

**Example 78:** 2-Hydroxy-N-(8-methyl-3-oxo-10-oxa-4,6-diaza-5(2,6)-pyridin-1(2,4)-thiophene-2(1,2)-benzocycloundecane-$2^5$-yl)eth ane-1-sulfonamide

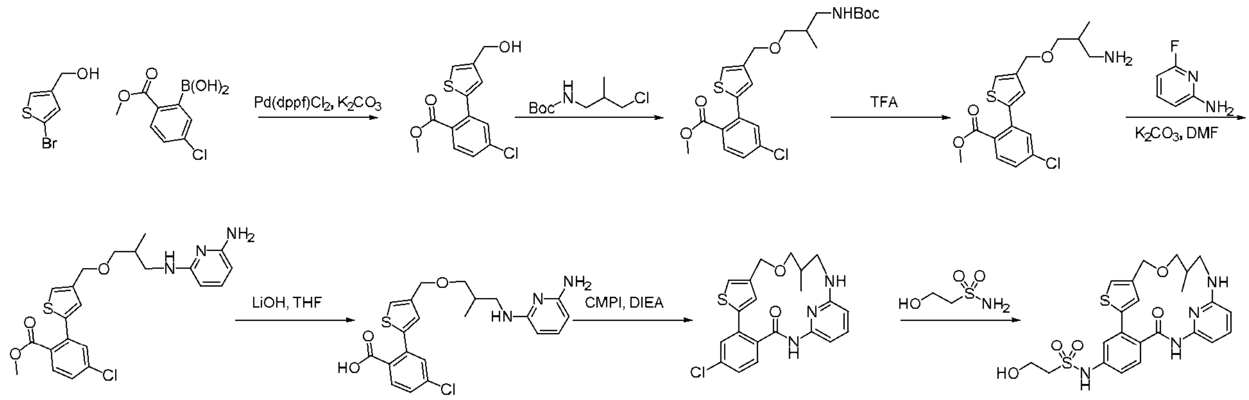


Example 78 was prepared using the method for Example 72. LCMS (ESI): $[M+H]^+$ =503.10

Example 79: 2-Hydroxy-N-(8'-methyl-3'-oxospiro[cyclopropane-1,11'-10-oxa-4,6-diaza-5(2,6)-pyridin-1(2,5)-thiophenea-2(1,2)-be nzocycloundecane]-5'-yl)ethane-1-sulfonamide

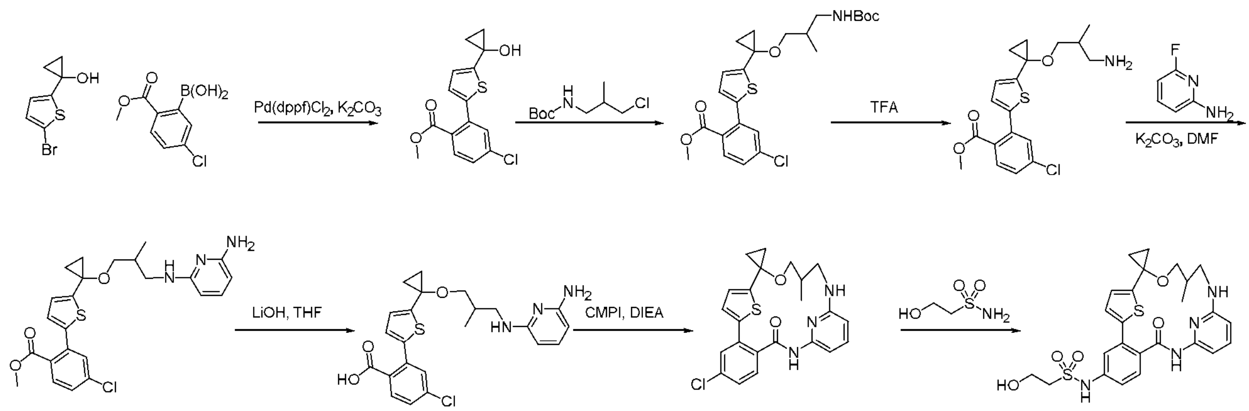


Example 79 was prepared using the method for Example 72. LCMS (ESI): $[M+H]^+$ =529.20

Example 80: 2-hydroxy-N-(8,11,11-trimethyl-3-oxo-10-oxa-4,6-diaza-5(2,6)-pyridin-1(2,4)-thiophene-2(1,2)-benzocycloundecane-2 5-yl)ethane-1-sulfonamide

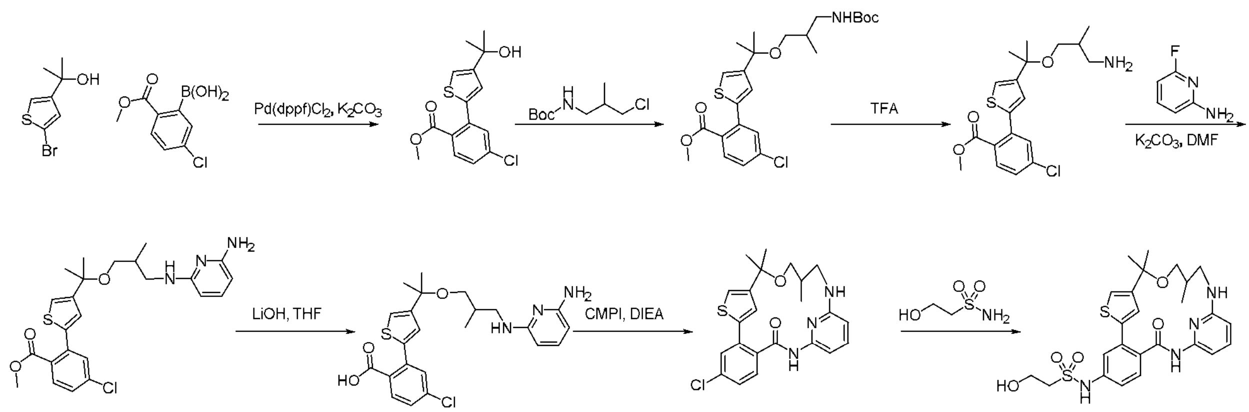


**Example 80** was prepared using the method for **Example 72.** LCMS (ESI): $[M+H]^+$ =531.20

**Example 81A-Example 81D: Four stereoisomers of N-(($3^4$,7)-$1^4$,$1^4$-difluoro-$2^6$-methyl-$3^1$H-2(2,4)-pyrimidin-1(1,3),5(1,4)-dipiperidin-3(4,1)-triazole-4(1,2)-benzocyclooct-7-en-44-yl)-2-hydroxyethane-1-sulfonamide**

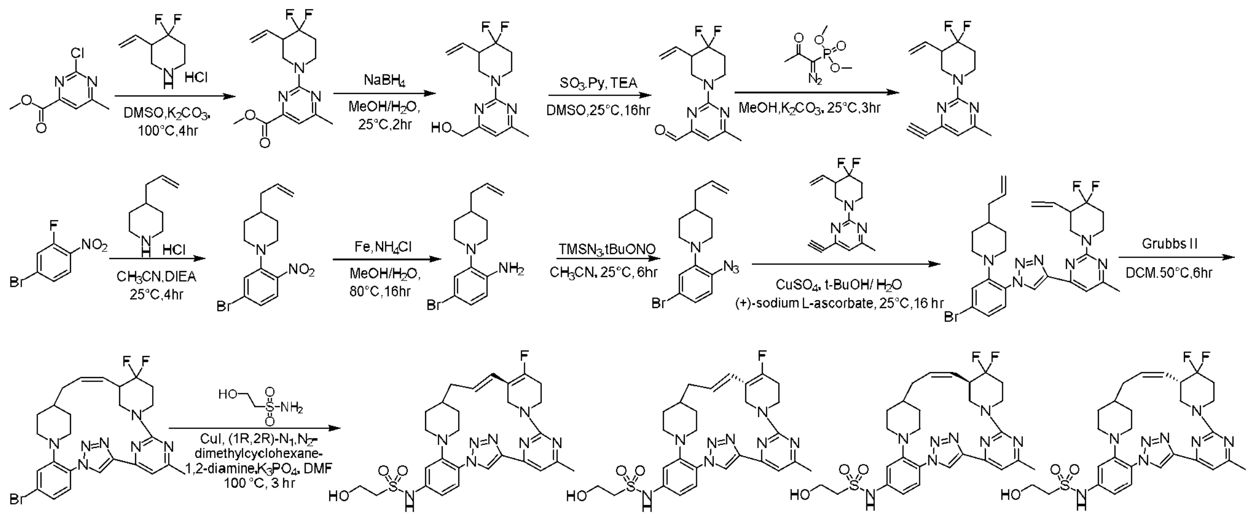


**Step 1:** To a solution of methyl 2-chloro-6-methylpyrimidin-4-carboxylate (3.00 g, 16.0 mmol) and 3-vinyl-4,4-difluoropiperidine hydrochloride (3.00 g, 16.0 mmol) in dimethyl sulfoxide (60 mL) was added potassium carbonate (3.00 g, 16.0 mmol). The mixture was slowly heated to 100°C and stirred for 4 h. The mixture was added to water (50 mL) and extracted with ethyl acetate (50 mL × 2). The organic phases were combined, dried over anhydrous sodium sulfate, and filtered, and the filtrate was concentrated to give the residue. The residue was purified by flash column chromatography (silica gel, 0-20% gradient of ethyl acetate/petroleum ether) to give methyl 2-(3-vinyl-4,4-difluoropiperidin-1-yl)-6-methylpyrimidin-4-carboxylate (3.50 g, 11.7 mmol, yield: 73.2%) as a yellow solid. LCMS (ESI): $[M+H]^+$ =298.4

**Step 2:** To a solution of methyl 2-(3-vinyl-4,4-difluoropiperidin-1-yl)-6-methylpyrimidin-4-carboxylate (3.50 g, 11.7 mmol) in methanol (70 mL) and water (7 mL) was added sodium borohydride (1.36 g, 35.3 mmol). The mixture was stirred at 25°C for 2 h. The reaction mixture was added with water (20 mL) and extracted with ethyl acetate (20 mL × 2). The organic phases were combined, dried over anhydrous sodium sulfate, filtered, and concentrated under reduced pressure to give [2-(3-vinyl-4,4-difluoropiperidin-1-yl)-6-methylpyrimidin-4-yl]methanol (3.20 g, 11.8 mmol, yield: 95.6%) as a yellow oil. LCMS (ESI): $[M+H]^+$ = 270.0.

**Step 3:** To a solution of [2-(3-vinyl-4,4-difluoropiperidin-1-yl)-6-methylpyrimidin-4-yl]methanol (3.20 g, 11.8 mmol) and triethylamine (6.01 g, 59.4 mmol) in dimethyl sulfoxide (64 mL) was added pyridine sulfur trioxide (5.67 g, 35.6 mmol). The mixture was stirred at 25°C for 16 h. The reaction mixture was added with water (50 mL) and extracted with ethyl acetate (50 mL × 2). The organic phases were combined, dried over anhydrous sodium sulfate, filtered, and concentrated under reduced pressure to give 2-(3-vinyl-4,4-difluoropiperidin-1-yl)-6-methylpyrimidin-4-carbaldehyde (3.00 g, 11.1 mmol, yield: 94.4%) as a yellow oil. LCMS (ESI): $[M+H]^+$ = 267.9.

**Step 4:** Dimethyl (1-diazo-2-oxopropylidene) phosphonate (2.58 g, 13.4 mmol) was added to a solution of 2-(3-vinyl-4,4-difluoropiperidin-1-yl)-6-methylpyrimidin-4-carbaldehyde (3.00 g, 11.1 mmol) and potassium carbonate (3.09 g, 22.4 mmol in methanol (20 mL). The mixture was stirred at 25°C for 3 h. The reaction mixture was added with water (40 mL) and extracted with ethyl acetate (40 mL × 2). The organic phases were combined, dried over anhydrous sodium sulfate, filtered, concentrated under reduced pressure, and purified by flash column chromatography (silica gel, 0-10% gradient of ethyl acetate/petroleum ether) to give 2-(3-vinyl-4,4-difluoropiperidin-1-yl)-4-ethynyl-6-methylpyrimidine (0.80 g, 3.00 mmol, yield: 27%) as a yellow oil. LCMS (ESI): $[M+H]^+$ = 264.1.

**Step 5:** To a solution of 2-fluoro-4-bromo-1-nitrobenzene (2.00 g, 9.09 mmol) and N,N-diisopropylethylamine (3.60 g, 27.2 mmol) in acetonitrile (20 mL) was added 4-(prop-2-en-1-yl)piperidine hydrochloride (1.91 g, 11.8 mmol). The mixture was stirred at 25°C for 4 h. Water (100 mL) was poured into the reaction mixture, which was extracted with ethyl acetate (100 mL × 3). The organic phases were combined, dried over anhydrous sodium sulfate, and filtered, and the filtrate was concentrated to give the residue. The residue was purified by flash column chromatography (silica gel, 0-10% gradient of ethyl acetate/petroleum ether) to give 1-(5-bromo-2-nitrophenyl)-4-(prop-2-en-1-yl)piperidine (2.90 g, 8.90 mmol, yield: 98.1%) as a yellow solid. LCMS (ESI): $[M+H]^+$ = 326.6.

**Step 6:** To a solution of 1-(5-bromo-2-nitrophenyl)-4-(prop-2-en-1-yl)piperidine (2.50 g, 7.69 mmol) in methanol (50 mL) and water (10 mL) were added ammonium chloride (1.25 g, 23.0 mmol) and iron powder (4.33 g, 76.8 mmol). The mixture was heated to 80°C and stirred for 16 h. The reaction mixture was filtered with diatomite and the filtrate was

extracted with ethyl acetate (100 mL × 3). The organic phases were combined, dried over anhydrous sodium sulfate, filtered, and concentrated under reduced pressure to give 4-bromo-2-[4-(prop-2-en-1-yl)piperidin-1-yl]aniline (2.20 g, 7.15 mmol, yield: 96.9%) as a yellow oil. LCMS (ESI): $[M+H]^+$ =297.0.

**Step 7:** Tert-butyl nitrite (1.07 g, 10.1 mmol) was added to a solution of 4-bromo-2-[4-(prop-2-en-1-yl)piperidin-1-yl]aniline (2.00 g, 6.77 mmol) in acetonitrile (20 mL) at 0°C. Then, azidotrimethylsilane (1.16 g, 10.1 mmol) was added to the reaction mixture and the mixture was stirred at 25°C for 6 h. The reaction mixture was poured into water (40 mL) and extracted with ethyl acetate (40 mL × 2). The organic phases were combined, dried over anhydrous sodium sulfate, and filtered, and the filtrate was concentrated to give the residue. The residue was purified by flash column chromatography (silica gel, 0-10% gradient of ethyl acetate/petroleum ether) to give 1-(2-azido-5-bromophenyl)-4-(prop-2-en-1-yl)piperidine (2.00 g, 6.22 mmol, yield: 91.9%) as a yellow solid. LCMS (ESI): $[M+H]^+$ =321.1.

**Step 8:** To a solution of 1-(2-azido-5-bromophenyl)-4-(prop-2-en-1-yl)piperidine (451 mg, 1.40 mmol) and 2-(3-vinyl-4,4-difluoropiperidin-1-yl)-4-ethynyl-6-methylpyrimidine (370 mg, 1.40 mmol) in tert-butanol (3 mL) and water (3 mL) were added copper sulfate (269 mg, 1.68 mmol) and sodium ascorbate (334 mg, 1.68 mmol). The mixture was stirred at 25°C for 4 h. The reaction mixture was added with water (20 mL) and extracted with ethyl acetate (20 mL × 3). The organic phases were combined, dried over anhydrous sodium sulfate, and filtered, and the filtrate was concentrated to give the residue. The residue was purified by flash column chromatography (silica gel, 0-5% gradient of ethyl acetate/petroleum ether) to give 4-(1-(2-(4-allylpiperidin-1-yl)-4-bromophenyl)-1H-1,2,3-triazol-4-yl)-2-(4,4-difluoro-3-vinylpiperidin-1-acyl)-6-methylpyri midine (689 mg, 1.17 mmol, yield: 83.8%) as a yellow solid. LCMS (ESI): $[M+H]^+$ =586.2.

**Step 9:** To a solution of 4-(1-(2-(4-allylpiperidin-1-yl)-4-bromophenyl)-1H-1,2,3-triazole-4-yl)-2-(4,4-difluoro-3-vinylpiperidin-1-acyl)-6-methylpy rimidine (689 mg, 1.17 mmol) in dichloromethane (280 mL) was added Grubbs catalyst 2nd generation (100g, 0.11mmol), and the mixture was stirred at 50°C for 6 h under nitrogen atmosphere. The reaction mixture was concentrated under reduced pressure. The residue was purified by flash column chromatography (silica gel, 0-5% gradient tetrahydrofuran/petroleum ether) to give ($3^4$Z,7Z)-$4^4$-bromo-$1^4$,$1^4$-difluoro-$2^6$-methyl-$3^1$H-2(2,4)-pyrimidin-1(1,3),5(1,4)-dipiperidin-3(4,1)-triazole-4(1,2)-benzocyc looct-7-ene (600 mg, 1.07 mmol, yield: 91.5%) as a yellow solid. LCMS (ESI): $[M+H]^+$ = 558.1

**Step 10:** To a solution of ($3^4$Z,7Z)-$4^4$-bromo-$1^4$,$1^4$-difluoro-$2^6$-methyl-$3^1$H-2(2,4)-pyrimidin-1(1,3),5(1,4)-dipiperidin-3(4,1)-triazole-4(1,2)-benzocyc looct-7-ene (600 mg, 1.07 mmol), 2-hydroxyethanesulfonamide (202 mg, 1.61 mmol), (1R,2R)-N1,N2-dimethylcyclohexane-1,2-diamine (155 mg, 1.08 mmol) and potassium phosphate (686 mg, 3.23 mmol) in N,N-dimethylformamide (12 mL) was added cuprous iodide (205 mg, 1.08 mmol). The mixture was heated to 100°C and stirred for 3 h under nitrogen atmosphere. Water (20 mL) and aqueous ammonia (0.5 mL) were added to the reaction mixture and extracted with ethyl acetate (10 mL × 2). The organic phases were combined, dried over anhydrous sodium sulfate, and filtered, and the filtrate was concentrated to give the residue. The residue was purified by preparative HPLC (C18, 44-84% gradient of water (formic acid)/acetonitrile) to give N-(($3^4$Z, 7Z)-$1^4$,$1^4$-difluoro-$2^6$-methyl-$3^1$H-2(2,4)-pyrimidin-1(1,3),5(1,4)-dipiperidin-3(4,1)-triazole-4(1,2)-benzocyclooct-7-en-$4^4$-y 1)-2-hydroxyethane-1-sulfonamide (200 mg) as a white solid and N-(($3^4$Z,7E)-$1^4$,$1^4$-difluoro-$2^6$-methyl-$3^1$H-2(2,4)-pyrimidin-1(1,3),5(1,4)-dipiperidin-3(4,1)-triazole-4(1,2)-benzocyclooct-7 -en-$4^4$-yl)-2-hydroxyethane-1-sulfonamide (22 mg) as a white solid.

The cis product was separated by SFC (column: DAICEL CHIRALCEL OX (250 mm*30 mm, 10 μm), mobile phase: $CO_2$-EtOH (0.1% $NH_3H_2O$), gradient: isocratic 55%, flow rate: 80 mL/min, column temperature: 40°C) to give Example **81A and Example 81B.**

**Example 81A:** White solid (64.5 mg), SFC analysis (column: Chiralcel OX-3 50 × 4.6 mm I.D., 3 μm; mobile phase: carbon dioxide for phase A and 0.05% diethylamine/ethanol for phase B; gradient: 40% for phase B; flow rate: 4 mL/min). The retention time on the chiral column was 0.821 min; LCMS (ESI): $[M+H]^+$ =601.2. $^1$H NMR (400 MHz, DMSO-$d_6$) δ ppm 10.19 - 9.69 (m, 1H), 9.11 (s, 1H), 7.63 (d, *J* = 9.2 Hz, 1H), 7.21 (s, 1H), 7.09 - 6.87 (m, 2H), 5.79 (ddd, *J* = 7.2, 8.8, 15.2 Hz, 1H), 5.38 (dd, *J* = 8.8, 15.2 Hz, 1H), 5.17 - 4.65 (m, 3H), 4.06 - 4.05 (m, 1H), 3.77 (t, *J* = 6.4 Hz, 2H), 3.48 - 3.40 (m, 1H), 3.31 (br s, 2H), 3.21 - 3.05 (m, 2H), 2.84 (br d, *J* = 8.0 Hz, 1H), 2.77 - 2.65 (m, 1H), 2.39 (s, 3H), 2.31 - 2.16 (m, 2H), 2.13 - 1.97 (m, 2H), 1.94 - 1.81 (m, 2H), 1.71 - 1.47 (m, 3H), 1.43 - 1.27 (m, 1H), 0.98 (br d, *J* = 12.8 Hz, 1H)

**Example 81B:** White solid (65.12 mg), SFC analysis (column: Chiralcel OX-3 50 × 4.6 mm I.D., 3 μm; mobile phase: carbon dioxide for phase A and 0.05% diethylamine/ethanol for phase B; gradient: 40% for phase B; flow rate: 4 mL/min). The retention time on the chiral column was 1.212 min; LCMS (ESI): $[M+H]^+$ =601.2. $^1$H NMR (400 MHz, DMSO-$d_6$) δ ppm 10.19 - 9.69 (m, 1H), 9.11 (s, 1H), 7.63 (d, *J* = 9.2 Hz, 1H), 7.21 (s, 1H), 7.09 - 6.87 (m, 2H), 5.79 (ddd, *J* = 7.2, 8.8, 15.2 Hz, 1H), 5.38 (dd, *J* = 8.8, 15.2 Hz, 1H), 5.17 - 4.65 (m, 3H), 4.06 - 4.05 (m, 1H), 3.77 (t, *J* = 6.4 Hz, 2H), 3.48 - 3.40 (m, 1H), 3.31 (br s, 2H), 3.21 - 3.05 (m, 2H), 2.84 (br d, *J* = 8.0 Hz, 1H), 2.77 - 2.65 (m, 1H), 2.39 (s, 3H), 2.31 - 2.16 (m, 2H), 2.13 - 1.97 (m, 2H), 1.94 - 1.81 (m, 2H), 1.71 - 1.47 (m, 3H), 1.43 - 1.27 (m, 1H), 0.98 (br d, *J* = 12.8 Hz, 1H)

The trans product was separated by SFC (column: DAICEL CHIRALPAK AS (250 mm*30 mm, 10 μm); mobile phase: $CO_2$ - EtOH (0.1% $NH_3H_2O$); gradient: isocratic 35%; flow rate: 80 mL/min; column temperature: 35°C) to give **Example 81C** and **Example 81D.**

**Example 81C:** White solid (6.31 mg), SFC analysis (column: Chiralpak AS-3 50 × 4.6 mm I.D., 3 μm; mobile phase: carbon dioxide for phase A and 0.05% diethylamine/ethanol for phase B; gradient: from 5% to 40% of B in 1.5 min and hold 40% for 1 min, then 5% of B for 0.5min; flow rate: 4 mL/min). The retention time on the chiral column was 1.446 min; LCMS (ESI): $[M+H]^+$ =601.2. $^1H$ NMR (400 MHz, DMSO-$d_6$) δ ppm 10.35 - 9.77 (m, 1H), 9.04 (s, 1H), 7.87 (d, *J* = 8.8 Hz, 1H), 7.30 - 7.02 (m, 3H), 5.67 (dt, *J* = 3.6, 11.6 Hz, 1H), 5.52 (br t, *J* = 10.0 Hz, 1H), 5.09 - 4.71 (m, 3H), 3.77 (br t, *J* = 6.4 Hz, 2H), 3.52 - 3.35 (m, 2H), 3.16 - 2.93 (m, 4H), 2.84 - 2.67 (m, 2H), 2.60 (br t, *J*= 11.2 Hz, 1H), 2.40 (s, 3H), 2.24 - 2.08 (m, 2H), 2.06 - 1.89 (m, 2H), 1.79 - 1.52 (m, 4H), 1.19 - 1.08 (m, 1H)

**Example 81D:** White solid (7.87 mg), SFC analysis (column: Chiralpak AS-3 50 × 4.6 mm I.D., 3 μm; mobile phase: carbon dioxide for phase A and 0.05% diethylamine/ethanol for phase B; gradient: from 5% to 40% of B in 1.5 min and hold 40% for 1 min, then 5% of B for 0.5min; flow rate: 4 mL/min). The retention time on the chiral column was 1.564 min; LCMS (ESI): $[M+H]^+$=601.2. $^1H$ NMR (400 MHz, DMSO-$d_6$) δ ppm 10.35 - 9.77 (m, 1H), 9.04 (s, 1H), 7.87 (d, *J* = 8.8 Hz, 1H), 7.30 - 7.02 (m, 3H), 5.67 (dt, *J* = 3.6, 11.6 Hz, 1H), 5.52 (br t, *J* = 10.0 Hz, 1H), 5.09 - 4.71 (m, 3H), 3.77 (br t, *J* = 6.4 Hz, 2H), 3.52 - 3.35 (m, 2H), 3.16 - 2.93 (m, 4H), 2.84 - 2.67 (m, 2H), 2.60 (br t, *J* = 11.2 Hz, 1H), 2.40 (s, 3H), 2.24 - 2.08 (m, 2H), 2.06 - 1.89 (m, 2H), 1.79 - 1.52 (m, 4H), 1.19 - 1.08 (m, 1H)

**Example 82: N-(($3^4$Z,7Z)-$1^4$-fluoro-$2^6$-inethyl-$1^1$,$1^2$,$1^3$,$1^6$-tetrahydro-$3^1$H-2(2,4)-pyrimidin-1(1,5)-pyridin-5(1,4)-piperidin-3(4,1)-tri azole-4(1,2)-benzylcyclooct-7-en-$4^4$-yl)-2-hydroxyethane-1-sulfonamide**

**Example 83: N-(($3^4$Z,7E)-$1^4$-fluoro-$2^6$-methyl-$1^1$,$1^2$,$1^3$,16-tetrahydro-$3^1$H-2(2,4)-pyrimidin-1(1,5)-pyridin-5(1,4)-piperidin-3(4,1)-tri azole-4(1,2)-benzylcyclooct-7-en-$4^4$-yl)-2-hydroxyethane-1-sulfonamide**

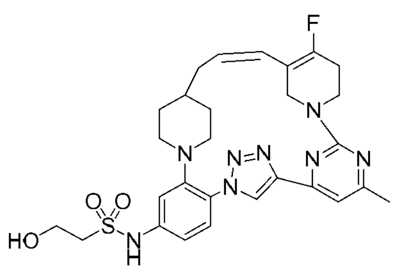
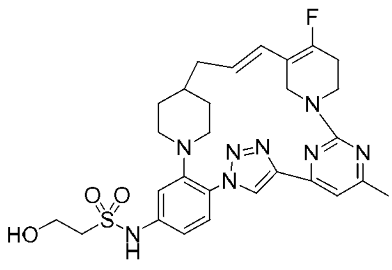

**Example 82** and **Example 83** were prepared using a similar method to **Example 81.**

**Example 82: White** solid (64.5 mg), SFC analysis (column: (S,S)-Whelk-0-1.8 50 × 6 mm I.D., 3 μm; mobile phase: carbon dioxide as phase A and 0.05% diethylamine/ethanol as phase B; gradient: 45% for phase B; flow rate: 2.2 mL/min). The retention time on the chiral column was 1.846 min; LCMS (ESI): $[M+H]^+$ =581.2. $^1H$ NMR (400 MHz, DMSO-$d_6$) δ ppm 10.06 (s, 1H), 9.49 - 9.10 (m, 1H), 7.91 - 7.62 (m, 1H), 7.28 (s, 1H), 7.23 - 7.13 (m, 1H), 7.11 - 7.01 (m, 1H), 6.35 (br d, *J*= 15.6 Hz, 1H), 5.90 - 5.70 (m, 1H), 4.63 (br s, 2H), 4.11 - 4.03 (m, 2H), 3.69 (br d, *J* = 2.0 Hz, 2H), 3.41 - 3.29 (m, 2H), 2.89 - 2.81 (m, 2H), 2.64 - 2.59 (m, 2H), 2.47 - 2.36 (m, 5H), 2.33 - 2.19 (m, 2H), 1.77 - 1.54 (m, 3H), 1.52 - 1.31 (m, 2H)

**Example 83:** White solid (1.01 mg), SFC analysis (column: Chiralpak IBN-3 50 × 4.6 mm I.D., 3 μm; mobile phase: carbon dioxide for phase A and 0.2% ammonia/methanol (7M)/ethanol for phase B; gradient: from 20% to 40% of B in 1.5 min and hold 40% of 1 min, then 20% of B for 0.5 min; flow rate: 4 mL/min). The retention time on the chiral column was 1.692 and 1.812 min, respectively; LCMS(ESI):$[M+H]^+$ =581.3. $^1H$ NMR (400 MHz, DMSO-$d_6$) δ ppm 10.07 (s, 1H), 9.07 (s, 1H), 7.91 (d, *J* = 8.8 Hz, 1H), 7.23 (d, *J* = 2.0 Hz, 1H), 7.18 - 7.12 (m, 2H), 5.69 (s, 1H), 5.52 (br d, *J* = 16.4 Hz, 1H), 5.44 - 5.32 (m, 1H), 4.68 - 4.58 (m, 1H), 4.56 - 4.49 (m, 1H), 4.13 - 4.01 (m, 1H), 3.83 - 3.77 (m, 2H), 3.55 (br d, J = 4.3 Hz, 2H), 3.34 (t, *J* = 6.5 Hz, 2H), 2.98 - 2.89 (m, 2H), 2.86 - 2.76 (m, 1H), 2.61 - 2.54 (m, 1H), 2.41 (s, 3H), 2.23 - 2.11 (m, 2H), 1.82 - 1.49 (m, 5H), 1.45 - 1.31 (m, 1H)

**Example 84A-Example 84D: Four stereoisomers of N-(($3^{4,}$7)-$1^4$,$1^4$-difluoro-$2^6$-methyl-3'H-2(2,4)-pyrimidin-1(1,3),5(1,4)-dipiperidin-3(1,4)-triazole-4(1,2)-benzocyclooct-7-en-$4^4$-yl)-2-hydroxyethane-1-sulfonamide**

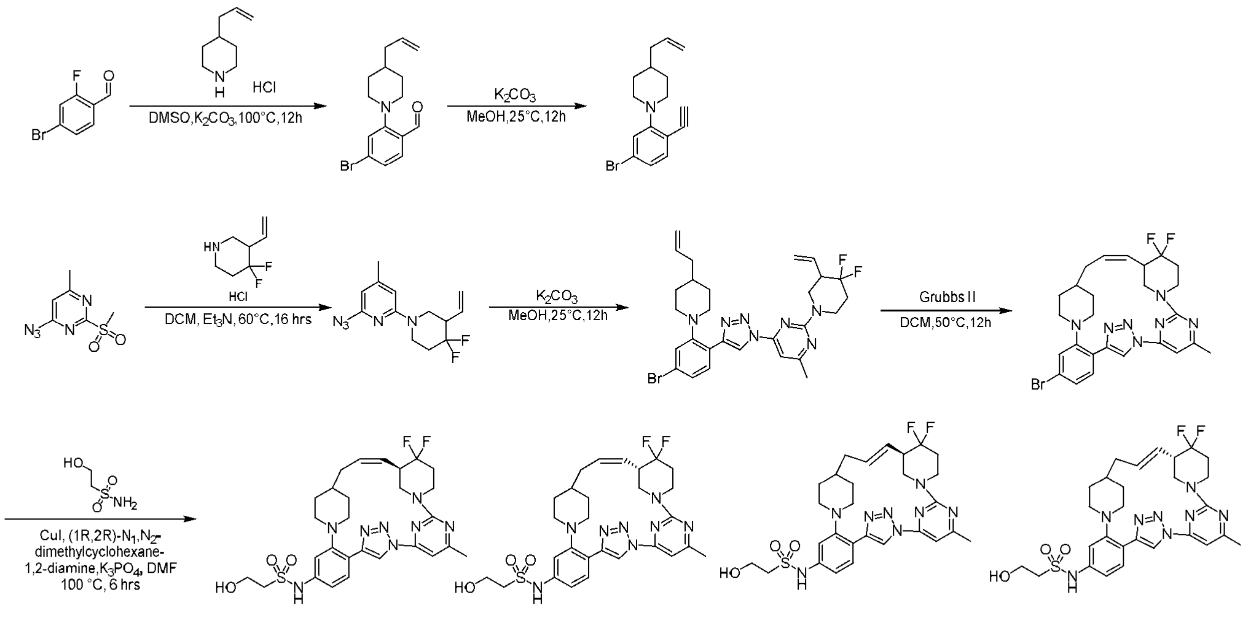


**Step 1:** To a solution of 4-bromo-2-fluorobenzene(formaldehyde) (2.00 g, 9.85 mmol) and 4-(prop-2-en-1-yl) piperidine hydrochloride (2.07 g, 12.8 mmol) in dimethyl sulfoxide (20.0 mL) was added potassium carbonate (4.08 g, 29.6 mmol) at 25°C, and the reaction mixture was stirred at 100°C for 12 h. The reaction system was diluted with water (100 mL) and extracted with ethyl acetate (100 mL × 3), and the organic phase was dried over anhydrous magnesium sulfate, filtered, and concentrated under reduced pressure to give 4-bromo-2-[4-(prop-2-en-1-yl)piperidin-1-yl]benzaldehyde (crude, 2.10 g, 0.46 mmol, yield: 69%) as a yellow oil. LCMS (ESI): $[M+H]^+$ =308.0.

**Step** 2: To a solution of 4-bromo-2-[4-(prop-2-en-1-yl)piperidin-1-yl]benzaldehyde (1.00 g, 3.24 mmol) and dimethyl(1-diazo-2-oxypropylene)phosphonate (748 mg, 3.89 mmol) in methanol (20.0 mL) was added potassium carbonate (890 mg, 6.49 mmol), and the mixture was stirred at 25°C for 12 h. The reaction mixture was concentrated and diluted with water (20 mL), extracted with ethyl acetate (20 mL × 2), and the organic phase was dried, filtered and concentrated. The residue was purified by flash column chromatography (silica gel, 0-2% gradient of tetrahydrofuran/petroleum ether) to give 1-(5-bromo-2-ethynylphenyl)-4-(prop-2-en-1-yl)piperidine (800 mg, 0.45 mmol, yield: 81%) as a yellow oil. LCMS (ESI): $[M+H]^+$ =306.1

**Step 3:** To a solution of 4-azido-2-methanesulfonyl-6-methoxypyrimidine (2.00 g, 9.38 mmol) in tetrahydrofuran (40.0 mL) were added triethylamine (4.74 g, 46.9 mmol) and 3-vinyl-4,4-difluoropiperidine hydrochloride (2.07 g, 11.3 mmol). The mixture was stirred at 60°C for 16 h. The reaction solution was diluted with water (80 mL) and extracted with ethyl acetate (100 mL × 2), and the organic phase was washed with saturated saline (100 mL). The organic phases were combined, dried over anhydrous sodium sulfate, and filtered. The filtrate was concentrated to give the residue. The residue was purified by flash column chromatography (silica gel, 0-5% gradient of tetrahydrofuran/petroleum ether) to give 2-azide-6-(3-vinyl-4,4-difluoropiperidin-1-yl)-4-methylpyridine (0.45 g, 1.59 mmol, yield: 17%) as a yellow oil. LCMS (ESI): $[M+H]^+$ = 281.1.

**Step 4:** To a solution of 2-azido-6-(3-vinyl-4,4-difluoropiperidin-1-yl)-4-methylpyridine (369 mg, 1.31 mmol) and 1-(5-bromo-2-ethynylphenyl)-4-(prop-2-en-1-yl)piperidine (400 mg, 1.31 mmol) in tert-butanol (3.00 mL), tetrahydrofuran (3.00 mL) and water (3.00 mL) were added copper sulfate (210 mg, 1.31 mmol) and sodium ascorbate (261 mg, 1.31 mmol). The mixture was stirred at 25°C for 12 h. The reaction mixture was added with water (20.0 mL) and extracted with ethyl acetate (20.0 mL × 3). The organic phases were combined, dried over anhydrous sodium sulfate, and filtered, and the filtrate was concentrated to give the residue. The residue was purified by flash column chromatography (silica gel, 0-3% gradient of tetrahydrofuran/petroleum ether) to give 4-(4-{4-bromo-2-[4-(prop-2-en-1-yl) piperidin-1-yl]phenyl}-1H-1,2,3-triazol-1-yl)-2-(3-vinyl-4,4-difluoropiperidin-1-yl)-6-methylpyrimidine (320 mg, 0.55 mmol, yield: 42%) as a yellow solid. LCMS (ESI): $[M+H]^+$ =586.1

**Step 5:** To a solution of 4-(4- f 4-bromo-2-[4-(prop-2-en-1-yl)piperidin-1-yl]phenyl}-1H-1,2,3-triazole-1-yl)-2-(3-vinyl-4,4-difluoropiperidin-1-yl)-6 -methylpyrimidine (300 mg, 0.51 mmol) in dry dichloromethane (120 mL) was added Grubbs catalyst 2nd generation (43.6 mg, 0.05 mmol) at 20°C under nitrogen atmosphere, and the mixture was heated to reflux at 50°C for 12 h. After the reaction was complete, the concentrated reaction solution was purified by flash column chromatography (silicon dioxide, 0-5% gradient of tetrahydrofuran/petroleum ether) to give ($3^4$Z,7Z)-$4^4$-bromo- $1^4$,$1^4$ -difluoro-$2^6$-methyl-$3^1$H-2(2,4)-pyrimidin-1(1,3),5(1,4)-dipiperidin-3(1,4)-triazole-4(1,2)-benzoeye lophene-7-ene (220 mg, 0.39 mmol, yield: 77%) as a yellow solid. LCMS (ESI): $[M+H]^+$ = 512.1

**Step 6:** To a solution of ($3^4$Z,7Z)-$4^4$-bromo-$1^4$,$1^4$-difluoro-$2^6$-methyl-$3^1$H-2(2,4)-pyrimidin-1(1,3),5(1,4)-dipiperidin-3(1,4)-triazole-4(1,2)-benzocyc lophene-7-ene (200 mg, 0.36 mmol) and 2-hydroxyethane-1-sulfonamide (67.5 mg, 0.54 mmol) in N,N dimethylformamide (4.00 mL) were added potassium phosphate (229 mg, 1.08 mmol), (1R, 2R)-N1, N2-dimethylcyclohexane-1, 2-diamine (51.6 mg, 0.36 mmol) and cuprous iodide (68.5 mg, 0.36 mmol), and the mixture was stirred at 100°C for 6 h. The reaction mixture was diluted with water (20.0 mL+20.0 mL aqueous ammonia) and extracted with ethyl acetate (40.0 mL × 2), and the organic phase was dried, filtered, and concentrated. The residue was purified by preparative HPLC (C18, 48-88% gradient of water (formic acid)/acetonitrile) to give a white solid.

Cis product: N-(($3^4$Z,7Z)-$1^4$,$1^4$-difluoro-$2^6$-methyl-$3^1$H-2(2,4)-pyrimidin-1(1,3),5(1,4)-dipiperidin-3(1,4)-triazole-4(1,2)-benzocyclooct-7 -en-$4^4$-yl)-2-hydroxyethane-1-sulfonamide (65 mg, 0.11 mmol, yield: 30%). LCMS (ESI): $[M+H]^+$ = 601.2. $^1$H NMR (400 MHz, DMSO-$d_6$) δ ppm 10.05 - 9.70 (m, 1H), 9.13 (s, 1H), 8.07 (d, *J* = 8.4 Hz, 1H), 7.29 (s, 1H), 7.13 (d, *J* = 2.0 Hz, 1H), 7.06 (dd, *J* = 2.0, 8.4 Hz, 1H), 5.85 (ddd, *J* = 6.0, 10.0, 15.2 Hz, 1H), 5.51 (dd, *J* = 8.8, 15.2 Hz, 1H), 4.94 (br d, *J* = 2.4 Hz, 1H), 4.19 - 3.93 (m, 3H), 3.91 - 3.69 (m, 3H), 3.32 - 3.27 (m, 2H), 3.06 (br d, *J* = 10.4 Hz, 1H), 2.88 (br d, *J* = 11.2 Hz, 2H), 2.79 - 2.64 (m, 1H), 2.47 (s, 3H), 2.44 - 2.36 (m, 1H), 2.34 - 2.25 (m, 1H), 2.24 - 2.12 (m, 1H), 2.10 - 1.98 (m, 2H), 1.78 - 1.50 (m, 4H), 1.37 (br d, *J* = 12.0 Hz, 1H)

The product was separated by SFC (column: DAICEL CHIRALPAK IC (250 mm×30 mm, 10 μm); mobile phase: $CO_2$-EtOH (0.1% $NH_3 \cdot H_2O$); gradient: isocratic 50%; flow rate: 80 mL/min; column temperature: 40°C) to give Example 84A and **Example 84B.**

**Example 84A:** White solid (20 mg), SFC analysis (column: Chiralpak IC-3 50×4.6 mm I.D, 3 μm; mobile phase: carbon dioxide for phase A and 0.05% diethylamine/ethanol for phase B; gradient: 40% for phase B; flow rate: 4 ml/min): The retention time on the chiral column was 1.259 min; LCMS (ESI): $[M+H]^+$ = 600.1. $^1$H NMR (400 MHz, DMSO-$d_6$) δ ppm 10.53 - 9.39 (m, 1H), 9.13 (s, 1H), 8.07 (d, *J* = 8.4 Hz, 1H), 7.29 (s, 1H), 7.12 (d, *J* = 1.6 Hz, 1H), 7.05 (dd, *J* = 2.0, 8.4 Hz, 1H), 5.85 (ddd, *J* = 6.0, 9.6, 15.2 Hz, 1H), 5.51 (br dd, *J* = 9.2, 15.2 Hz, 1H), 5.22 - 4.67 (m, 1H), 4.21 - 4.06 (m, 1H), 4.02 (br d, *J* = 4.0 Hz, 2H), 3.90 - 3.79 (m, 1H), 3.78 - 3.65 (m, 2H), 3.30 (br d, *J* = 6.8 Hz, 2H), 3.05 (br d, *J* = 11.2 Hz, 1H), 2.96 - 2.83 (m, 2H), 2.72 (br t, *J* = 10.0 Hz, 1H), 2.47 (s, 3H), 2.40 (br t, *J* = 11.2 Hz, 1H), 2.33 - 2.26 (m, 1H), 2.24 - 2.11 (m, 1H), 2.09 - 1.98 (m, 2H), 1.75 - 1.50 (m, 4H), 1.37 (br d, *J* = 12.0 Hz, 1H)

**Example 84B:** White solid (20 mg), SFC analysis (column: Chiralpak IC-3 50×4.6 mm I.D, 3 μm; mobile phase: carbon dioxide for phase A and 0.05% diethylamine/ethanol for phase B; gradient: 40% for phase B; flow rate: 4 ml/min): The retention time on the chiral column was 1.499 min; LCMS (ESI): $[M+H]^+$ = 600.1. $^1$H NMR (400 MHz, DMSO-$d_6$) δ ppm 10.18 - 9.44 (m, 1H), 9.14 (s, 1H), 8.07 (d, *J* = 8.4 Hz, 1H), 7.29 (s, 1H), 7.12 (d, *J* = 2.0 Hz, 1H), 7.05 (dd, *J* = 2.0, 8.4 Hz, 1H), 5.85 (ddd, *J* = 5.6, 9.6, 15.2 Hz, 1H), 5.52 (dd, *J* = 9.2, 14.8 Hz, 1H), 5.19 - 4.68 (m, 1H), 4.19 - 4.08 (m, 1H), 4.02 (br d, *J* = 4.0 Hz, 2H), 3.90 - 3.79 (m, 1H), 3.79 - 3.67 (m, 2H), 3.30 - 3.27 (m, 2H), 3.06 (br d, *J* = 9.2 Hz, 1H), 2.94 - 2.81 (m, 2H), 2.78 - 2.68 (m, 1H), 2.48 - 2.44 (m, 3H), 2.43 - 2.36 (m, 1H), 2.32 - 2.25 (m, 1H), 2.22 - 2.11 (m, 1H), 2.08 - 1.95 (m, 2H), 1.74 - 1.51 (m, 4H), 1.41 - 1.33 (m, 1H)

Trans product: N-(($3^4$Z,7E)-$1^4$,$1^4$-difluoro-$2^6$-methyl-$3^1$H-2(2,4)-pyrimidin-1(1,3),5(1,4)-dipiperidin-3(1,4)-triazole-4(1,2)-benzocyclooct-7 -en-$4^4$-yl)-2-hydroxyethane-1-sulfonamide (8 mg, 0.02 mmol, yield: 3.7%). LCMS (ESI): $[M+H]^+$ = 601.2. $^1$H NMR (400 MHz, DMSO-$d_6$) δ ppm 10.19 - 9.64 (m, 1H), 9.28 - 9.05 (m, 1H), 8.27 (d, *J* = 8.4 Hz, 1H), 7.20 (s, 1H), 7.16 (d, *J* = 2.0 Hz, 1H), 7.08 (dd, *J* = 2.0, 8.8 Hz, 1H), 5.90 (br d, *J* = 3.2 Hz, 1H), 5.60 - 5.35 (m, 1H), 5.08 - 4.62 (m, 3H), 3.75 (br t, *J* = 6.8 Hz, 2H), 3.29 - 3.27 (m, 2H), 3.18 - 2.98 (m, 3H), 2.97 - 2.85 (m, 2H), 2.48 - 2.46 (m, 3H), 2.28 - 2.08 (m, 3H), 2.06 - 1.85 (m, 2H), 1.83 - 1.35 (m, 5H).

The product was separated by SFC (column: DAICEL CHIRALPAK AS (250 mm × 30 mm, 10 μm), mobile phase: $CO_2$-EtOH (0.1% $NH_3 \cdot H_2O$), gradient: isocratic 35%, flow rate: 80 mL/min, column temperature: 35°C) to give Example 84C and Example 84D (a trans product).

Example 84C: N-(($3^4$Z,7E)-$1^4$,$1^4$-difluoro-$2^6$-methyl-$3^1$H-2(2,4)-pyrimidin-1(1,3),5(1,4)-dipiperidin-3(1,4)-triazole-4(1,2)-benzocyclooct-7 -en-$4^4$-yl)-2-hydroxyethane-1-sulfonamide (2.3 mg). The retention time on the chiral column was 1.549 min; LCMS (ESI): $[M+H]^+$ = 601.2. $^1$H NMR (400 MHz, DMSO-$d_6$) δ ppm 10.19 - 9.64 (m, 1H), 9.28 - 9.05 (m, 1H), 8.27 (d, *J* = 8.4 Hz, 1H), 7.20 (s, 1H), 7.16 (d, *J* = 2.0 Hz, 1H), 7.08 (dd, *J* = 2.0, 8.8 Hz, 1H), 5.90 (br d, *J* = 3.2 Hz, 1H), 5.60 - 5.35 (m, 1H), 5.08 - 4.62 (m, 3H), 3.75 (br t, *J* = 6.8 Hz, 2H), 3.29 - 3.27 (m, 2H), 3.18 - 2.98 (m, 3H), 2.97 - 2.85 (m, 2H), 2.48 - 2.46 (m, 3H), 2.28 - 2.08 (m, 3H), 2.06 - 1.85 (m, 2H), 1.83 - 1.35 (m, 5H)

**Example 84D:** N-(($3^4$Z,7E)-$1^4$,$1^4$-difluoro-$2^6$-methyl-$3^1$H-2(2,4)-pyrimidin-1(1,3),5(1,4)-dipiperidin-3(1,4)-triazole-4(1,2)-benzocyclooct-7 -en-$4^4$-yl)-2-hydroxyethane-1-sulfonamide (2.2 mg). The retention time on the chiral column was 1.758 min; LCMS (ESI): $[M+H]^+$ = 601.2.

$^1$H NMR (400 MHz, DMSO-$d_6$) δ ppm 10.19 - 9.64 (m, 1H), 9.28 - 9.05 (m, 1H), 8.27 (d, *J* = 8.4 Hz, 1H), 7.20 (s, 1H), 7.16 (d, *J*= 2.0 Hz, 1H), 7.08 (dd, *J* = 2.0, 8.8 Hz, 1H), 5.90 (br d, *J* = 3.2 Hz, 1H), 5.60 - 5.35 (m, 1H), 5.08 - 4.62 (m, 3H), 3.75 (br t, *J* = 6.8 Hz, 2H), 3.29 - 3.27 (m, 2H), 3.18 - 2.98 (m, 3H), 2.97 - 2.85 (m, 2H), 2.48 - 2.46 (m, 3H), 2.28 - 2.08 (m, 3H), 2.06 - 1.85 (m, 2H), 1.83 - 1.35 (m, 5H)

**Example 85: N-((1$^3$S,Z)-1$^4$,1$^4$-difluoro-2$^6$-methyl-3$^1$H-2(2,4)-pyrimidin-1(1,3),5(1,4)-dipiperidin-3(4,1)-triazole-4(1,2)-benzylcycloo ctyl-4$^4$-yl)-2-hydroxyethane-1-sulfonamide**

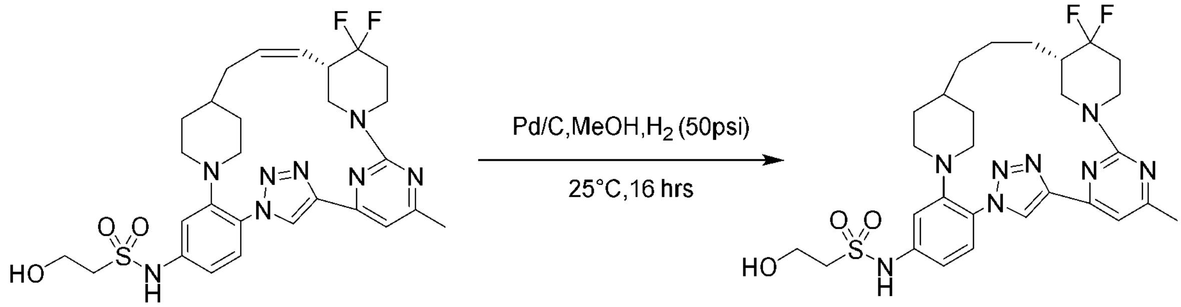


**Step 1:** To a solution of N-((1$^3$S,3$^4$Z,7Z)-1$^4$,1$^4$-difluoro-2$^6$-methyl-3$^1$H-2(2,4)-pyrimidin-1(1,3),5(1,4)-dipiperidin-3(4,1)-triazole-4(1,2)-benzocycloo ct-7-en-44-yl)-2-hydroxyethane-l-sulfonamide (56.0 mg, 0.09 mmol) in methanol (2.00 mL) was added 10% wet palladium on carbon (6.00 mg, 0.01 mmol), and the reaction mixture was purged with argon three times and then flushed with hydrogen three times. and stirred at 25°C for 16 h under hydrogen atmosphere (50 Psi). The reaction mixture was filtered under reduced pressure to remove palladium/carbon and then concentrated in vacuum. The residue was purified by preparative HPLC (C18, 38-78% gradient of water (formic acid)/acetonitrile) to give N-((1$^3$S,Z)-1$^4$,1$^4$-difluoro-2$^6$-methyl-3$^1$H-2(2,4)-pyrimidin-1(1,3),5(1,4)-dipiperidin-3(4,1)-triazole-4(1,2)-benzylcyclooctyl-4$^4$-yl)-2-hydroxyethane-1-sulfonamide (34.2 mg, 0.056 mmol, yield: 61%) as a white solid. LCMS (ESI): $[M+H]^+$ = 603.2. $^1$H NMR (400 MHz, DMSO-$d_6$) δ ppm 10.00 (s, 1H), 9.18 (s, 1H), 7.62 (d, $J$ = 9.2 Hz, 1H), 7.27 (s, 1H), 7.08 - 6.90 (m, 2H), 4.31 - 4.20 (m, 2H), 3.78 (br s, 2H), 3.70 - 3.56 (m, 2H), 3.33 (t, $J$ = 6.4 Hz, 2H), 3.02 - 2.90 (m, 1H), 2.71 - 2.60 (m, 1H), 2.46 - 2.32 (m, 5H), 2.18 - 1.88 (m, 3H), 1.80 - 1.40 (m, 7H), 1.38 - 1.09 (m, 4H)

**Example 86: N-((3$^4$,8)-1$^4$,1$^4$-difluoro-2$^6$-methyl-3$^1$H-2(2,4)-pyrimidin-1(1,3),5(1,4)-dipiperidin-3(4,1)-triazole-4(1,2)-benzoazacyclo nonane-8-en-44-yl)-2-hydroxyethane-1-sulfonamide**

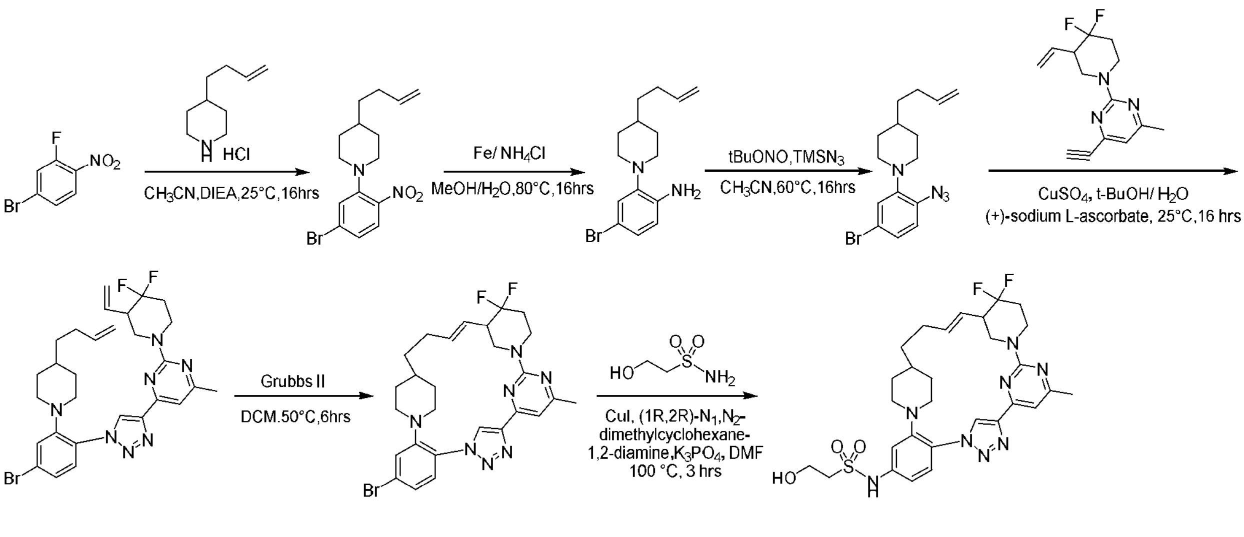


**Step 1:** To a solution of 2-fluoro-4-bromo-1-nitrobenzene (2.00 g, 9.09 mmol) and N,N-diisopropylethylamine (3.60 g, 27.2 mmol) in acetonitrile (20 mL) was added 4-(but-3-en-1-yl)piperidine hydrochloride (1.59 g, 9.09 mmol). The mixture was stirred at 25°C for 16 h. Water (100 mL) was poured into the reaction mixture, which was extracted with ethyl acetate (100 mL × 3). The organic phases were combined, dried over anhydrous sodium sulfate, and filtered, and the filtrate was concentrated to give the residue. The residue was purified by flash column chromatography (silica gel, 0-10% gradient of ethyl acetate/petroleum ether) to give 1-(5-bromo-2-nitrophenyl)-4-(but-3-en-1-yl)piperidine (3.00 g, 8.84 mmol, yield: 97.2%) as a yellow solid. LCMS (ESI): $[M+H]^+$ = 340.6.

**Step 2:** To a solution of 1-(5-bromo-2-nitrophenyl)-4-(but-3-en-1-yl)piperidine (3.00 g, 8.81 mmol) in methanol (60

mL) and water (15 mL) were added ammonium chloride (1.43 g, 26.5 mmol) and iron powder (4.98 g, 88.4 mmol). The mixture was heated to 80°C and stirred for 16 h. The reaction mixture was filtered with diatomite and the filtrate was extracted with ethyl acetate (100 mL × 3). The organic phases were combined, dried over anhydrous sodium sulfate, filtered, and concentrated under reduced pressure to give 4-bromo-2-[4-(but-3-en-1-yl)piperidin-1-yl]aniline (2.70 g, 8.72 mmol, yield: 98.7%) as a yellow oil. LCMS (ESI): $[M+H]^+$ =311.0.

**Step 3:** Tert-butyl nitrite (1.87 g, 17.8 mmol) was added to a solution of 4-bromo-2-[4-(but-3-en-1-yl)piperidin-1-yl]aniline (2.70 g, 8.73 mmol) in acetonitrile (40 mL) at 0°C. Then, azidotrimethylsilane (1.87 g, 17.8 mmol) was added to the reaction mixture and the mixture was stirred at 25°C for 16 h. The reaction mixture was poured into water (40 mL) and extracted with ethyl acetate (40 mL × 2). The organic phases were combined, dried over anhydrous sodium sulfate, and filtered, and the filtrate was concentrated to give the residue. The residue was purified by flash column chromatography (silica gel, 0-10% gradient of ethyl acetate/petroleum ether) to give 1-(2-azido-5-bromophenyl)-4-(but-3-en-1-yl)piperidine (2.40 g, 7.15 mmol, yield: 82%) as a yellow solid. LCMS (ESI): $[M+H]^+$ =336.6.

**Step 4:** To a solution of 1-(2-azido-5-bromophenyl)-4-(but-3-en-1-yl)piperidine (483 mg, 1.44 mmol) and 2-(3-vinyl-4,4-difluoropiperidin-1-yl)-4-ethynyl-6-methylpyrimidine (350 mg, 1.44 mmol) in tert-butanol (3 mL) and water (3 mL) were added copper sulfate (276 mg, 1.73 mmol) and sodium ascorbate (343 mg, 1.73 mmol). The mixture was stirred at 25°C for 16 h. The reaction mixture was added with water (20 mL) and extracted with ethyl acetate (20 mL × 3). The organic phases were combined, dried over anhydrous sodium sulfate, and filtered, and the filtrate was concentrated to give the residue. The residue was purified by flash column chromatography (silica gel, 0-5% gradient of ethyl acetate/petroleum ether) to give 4-(1-{4-bromo-2-[4-(but-3-en-1-yl)piperidin-1-yl]phenyl}-1H-1,2,3-triazol-4-yl)-2-(3-vinyl-4,4-difluoropiperidin-1-yl)-6-m ethylpyrimidine (500 mg, 0.83 mmol, yield: 57.8%) as a yellow solid. LCMS (ESI): $[M+H]^+$ =600.1.

**Step 5:** To a solution of 4-(1-{4-bromo-2-[4-(but-3-en-1-yl)piperidin-1-yl]phenyl}-1H-1,2,3-triazole-4-yl)-2-(3-vinyl-4,4-difluoropiperidin-1-yl)-6-methylpyrimidine (700 mg, 1.17 mmol) in dichloromethane (280 mL) was added Grubbs catalyst 2nd generation (99.3 mg, 0.11mmol), and the mixture was stirred at 50°C for 6 h under nitrogen atmosphere. The reaction mixture was concentrated under reduced pressure. The residue was purified by flash column chromatography (silica gel, 0-5% gradient tetrahydrofuran/petroleum ether) to give ($3^4$Z,8E)-$4^4$-bromo-$1^4$,$1^4$-difluoro-$2^6$-methyl-$3^1$H-2(2,4)-pyrimidin-1(1,3),5(1,4)-dipiperidin-3(4,1)-triazole-4(1,2)-benzoaza cyclononane-8-ene (500 mg, 0.87 mmol, yield: 91.5%) as a yellow solid. LCMS (ESI): $[M+H]^+$ = 572.2

**Step 6:** To a solution of ($3^4$Z,8E)-$4^4$-bromo-$1^4$,$1^4$-difluoro-$2^6$-methyl-$3^1$H-2(2,4)-pyrimidin-1(1,3),5(1,4)-dipiperidin-3(4,1)-triazole-4(1,2)-benzoaza cyclononane-8-ene (500 mg, 0.87 mmol), 2-hydroxyethanesulfonamide (219 mg, 1.75 mmol), (1R,2R)-N1,N2-dimethylcyclohexane-1,2-diamine (125 mg, 0.87 mmol) and potassium phosphate (558 mg, 2.62 mmol) in N,N-dimethylformamide (10 mL) was added cuprous iodide (167 mg, 0.87 mmol). The mixture was heated to 100°C and stirred for 3 h under nitrogen atmosphere. Water (20 mL) and aqueous ammonia (0.5 mL) were added to the reaction mixture and extracted with ethyl acetate (10 mL × 2). The organic phases were combined, dried over anhydrous sodium sulfate, and filtered, and the filtrate was concentrated to give the residue. The residue was purified by preparative HPLC (C18, 46-86% gradient water (formic acid)/acetonitrile) to give N-(($3^4$Z,8E)-$1^4$,$1^4$-difluoro-$2^6$-methyl-$3^1$H-2(2,4)-pyrimidin-1(1,3),5(1,4)-dipiperidin-3(4,1)-triazole-4(1,2)-benzoazacyclon onane-8-en-44-yl)-2-hydroxyethane-1-sulfonamide (135 mg, 0.21 mmol, yield: 25.0%) as a white solid. LCMS (ESI): $[M+H]^+$ =615.2 $^1$H NMR (400 MHz, DMSO-$d_6$) $\delta$ ppm 10.03 (s, 1H), 9.14 - 9.04 (m, 1H), 7.71 - 7.47 (m, 1H), 7.31 - 7.18 (m, 1H), 7.16 - 7.08 (m, 1H), 7.07 - 7.01 (m, 1H), 5.80 - 5.57 (m, 1H), 5.36 - 5.15 (m, 1H), 4.96 (br s, 1H), 4.80 - 4.63 (m, 1H), 4.59 - 4.41 (m, 1H), 3.77 (br t, $J$ = 6.0 Hz, 2H), 3.68 - 3.43 (m, 1H), 3.39 - 3.33 (m, 2H), 3.31 - 3.06 (m, 3H), 3.03 - 2.80 (m, 1H), 2.80 - 2.57 (m, 1H), 2.46 - 2.39 (m, 3H), 2.38 - 2.21 (m, 2H), 2.18 - 1.88 (m, 3H), 1.72 - 1.41 (m, 5H), 1.36 - 1.07 (m, 2H)

**Example 87: N-(($1^3$S,Z)-$1^4$,$1^4$-difluoro-$2^6$-methyl-$3^1$H-2(2,4)-pyrimidin-1(1,3),5(1,4)-dipiperidin-3(4,1)-triazole-4(1,2)-benzylcycloo etyl-$4^4$-yl)-2-hydroxyethane-1-sulfonamide**

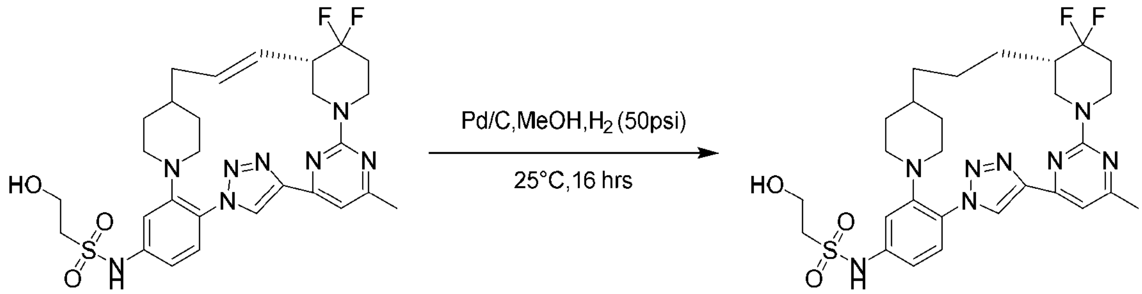


To a solution of N-(($1^3$S,$3^4$Z7E)-$1^4$,$1^4$-difluoro-$2^6$-methyl-$3^1$H-2(2,4)-pyrimidin-1(1,3),5(1,4)-dipiperidin-3(4,1)-triazole-4(1,2)-benzocycloo ct-7-en-$4^4$-yl)-2-hydroxyethane-1-sulfonamide (50.0 mg, 0.08 mmol) in methanol (2.00 mL) was added 10% Pd/C (wet) (6.00 mg, 0.01 mmol), and the reaction mixture was purged with argon three times and then flushed with hydrogen three times. and stirred at 25°C for 16 h under hydrogen atmosphere (50 Psi). The reaction mixture was filtered under reduced pressure to remove palladium/carbon and then concentrated in vacuum. The residue was purified by preparative HPLC (C18, 46-86% gradient of water (hydrochloric acid)/acetonitrile) to give N-(($1^3$S,Z)-$1^4$,$1^4$-difluoro-$2^6$-methyl-$3^1$H-2(2,4)-pyrimidin-1(1,3),5(1,4)-dipiperidin-3(4,1)-triazole-4(1,2)-benzylcyclooctyl-$4^4$-yl)-2-hydroxyethane-1-sulfonamide (11.0 mg, 0.017 mmol, yield: 21.9%) as a white solid LCMS (ESI): $[M+H]^+$ = 603.4. $^1$H NMR (400 MHz, DMSO-$d_6$) δ ppm 10.01 (s, 1H), 9.17 (s, 1H), 7.62 (d, *J* = 9.2 Hz, 1H), 7.27 (s, 1H), 7.10-6.91 (m, 2H), 4.24 (br d, *J* = 12.8 Hz, 1H), 3.84 (br s, 1H), 3.78 (br t, *J* = 6.4 Hz, 2H), 3.66 (br d, *J* = 12.8 Hz, 1H), 3.33-3.31 (m, 2H), 2.96 (br d, *J* = 11.2 Hz, 1H), 2.70-2.61 (m, 1H), 2.47-2.32 (m, 5H), 2.15-1.89 (m, 3H), 1.78-1.65 (m, 2H), 1.61-1.40 (m, 5H), 1.38-1.11 (m, 5H); SFC analysis (column: (S,S)-Whelk-0-1.8 50 × 4.6 mm I.D., 1.8 μm; mobile phase: carbon dioxide for phase A and 0.05% diethylamine/ethanol for phase B; gradient: 45% for phase B; flow rate: 2.2 mL/min).

**Example 88: N-(($3^4$Z,7E)-$4^5$-acetyl-$1^4$,$1^4$-difluoro-$2^6$-methyl-$3^1$H-2(2,4)-pyrimidin-1(1,3),5(1,4)-dipiperidin-3(4,1)-triazole-4(1,2)-be nzocyclooct-7-en-$4^4$-yl)-2-hydroxyethane-1-sulfonamide**

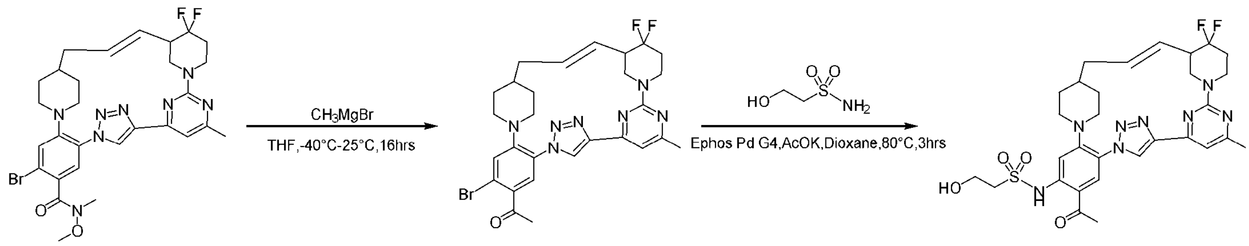


**Step 1:** To a solution of ($3^4$Z, 7E)-$4^4$-bromo-$1^4$,$1^4$-difluoro-N-methoxy-N, $2^6$-dimethyl-$3^1$H-2(2,4)-pyrimidin-1(1,3),5(1,4)-dipiperidin-3(4,1)-triazole-4(1,2)-benzocyclooct-7-en-$4^5$-carboxamide (700 mg, 1.08 mmol) in tetrahydrofuran (15 mL) was slowly added methylmagnesium bromide solution 3 M in diethyl ether (3.62 mL, 10.8 mmol) at -60°C, and the mixture was slowly heated to 25°C and stirred for 16 h. The reaction mixture was poured into water (20 mL) and extracted with ethyl acetate (100 mL × 3). The organic phases were combined, dried over anhydrous sodium sulfate, and filtered, and the filtrate was concentrated to give the residue. The residue was purified by flash column chromatography (silica gel, 0-20% gradient of ethyl acetate/petroleum ether) to give 1-(($3^4$Z, 7E)-$4^4$-bromo-$1^4$,$1^4$-difluoro-$2^6$-methyl-$3^1$H-2(2,4)-pyrimidin-1(1,3),5(1,4)-dipiperidin-3(4,1)-triazole-4(1,2)-benzocyclooct -7-en-45-yl)ethane-1-one (600 mg, 0.99 mmol, yield: 92.0%) as a yellow solid. LCMS (ESI): $[M+H]^+$ = 600.1.

**Step 2:** To a solution of 1-(($3^4$Z,7E)-$4^4$-bromo-$1^4$,$1^4$-difluoro-$2^6$-methyl-$3^1$H-2(2,4)-pyrimidin-1(1,3),5(1,4)-dipiperidin-3(4,1)-triazole-4(1,2)-benzoc yclooct-7-en-45-yl)ethane-1-one (300 mg, 0.50 mmol), 2-hydroxyethanesulfonamide (188 mg, 1.50 mmol) and potassium acetate (149 mg, 1.50 mmol) in dioxane (6 mL) was added [dicyclohexyl[3-(1-methylethoxy)-2',4',6'-tris(1-methylethyl)[1,1'-biphenyl]-2-yl]phosphine-κP](methanesulfonic acid-κO)[2'-(methylamino-κN)[1,1'-biphenyl]-2-yl-κC]palladium (39.8 mg, 0.05 mmol). The mixture was heated to 80°C and stirred for 3 h under nitrogen atmosphere. Water (20 mL) was added to the reaction mixture and extracted with ethyl acetate (10 mL × 2). The organic phases were combined, dried over anhydrous sodium sulfate, and filtered, and the filtrate was concentrated to give the residue. The residue was purified by preparative HPLC (C18, 50-90% gradient of water (formic acid)/acetonitrile) to give N-(($3^4$Z,7E)-$4^5$-acetyl-$1^4$,$1^4$-difluoro-$2^6$-methyl-$3^1$H-2(2,4)-pyrimidin-1(1,3),5(1,4)-dipiperidin-3(4,1)-triazole-4(1,2)-benzoc yclooct-7-en-$4^4$-yl)-2-hydroxyethane-1-sulfonamide (23.6 mg, 0.03 mmol, yield: 7.33%) as a white solid. LCMS (ESI): $[M+H]^+$ =643.3. $^1$H NMR (400 MHz, DMSO-$d_6$) δ ppm 11.91-11.28 (m, 1H), 9.26-8.96 (m, 1H), 8.35-8.02

(m, 1H), 7.37-7.06 (m, 2H), 5.97-5.61 (m, 1H), 5.51-5.29 (m, 1H), 5.18-4.62 (m, 3H), 3.76 (br d, $J$ = 4.4 Hz, 2H), 3.58-3.40 (m, 2H), 3.26-3.21 (m, 1H), 3.17-2.96 (m, 2H), 2.91-2.73 (m, 2H), 2.62 (s, 3H), 2.42-2.34 (m, 4H), 2.25-2.16 (m, 1H), 2.10-1.97 (m, 2H), 1.90-1.78 (m, 2H), 1.73-1.52 (m, 3H), 1.36-1.28 (m, 1H), 1.00-0.88 (m, 1H); SFC analysis (column: Chiralpak IG 50 × 4.6 mm I.D., 3 μm; mobile phase: carbon dioxide for phase A and 0.05% diethylamine/ethanol for phase B; gradient: 40% for phase B; flow rate: 4 mL/min).

**Example 89: N-((3$^4$Z,7E)-4$^5$-(cyclopropanecarbonyl)-1$^4$,1$^4$-difluoro-2$^6$-methyl-3$^1$H-2(2,4)-pyrimidin-1(1,3),5(1,4)-dipiperidin-3(4,1) -triazole-4(1,2)-benzocyclooct-7-en-4$^4$-yl)-2-hydroxyethane-1-sulfonamide**

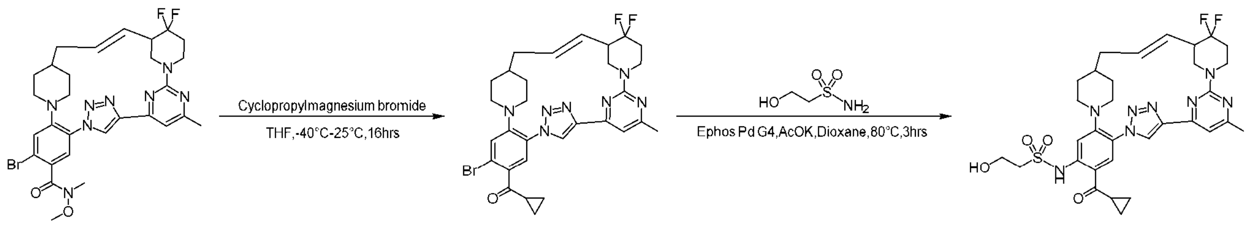


**Step 1:** To a solution of (3$^4$Z,7E)-4$^4$-bromo-1$^4$,1$^4$-difluoro-N-methoxy-N, 2$^6$-dimethyl-3$^1$H-2(2,4)-pyrimidin-1(1,3),5(1,4)-dipiperidin-3(4,1)-triazole-4(1,2)-benzocyclooct-7-en-4$^5$-carboxamide (300 mg, 0.46 mmol) in tetrahydrofuran (4 mL) was slowly added cyclopropylmagnesium bromide solution 0.5 M in tetrahydrofuran (4.66 mL, 2.33 mmol) at -40°C, and the mixture was slowly heated to 25°C and stirred for 16 h. The reaction mixture was poured into water (20 mL) and extracted with ethyl acetate (100 mL × 3). The organic phases were combined, dried over anhydrous sodium sulfate, and filtered, and the filtrate was concentrated to give the residue. The residue was purified by flash column chromatography (silica gel, 0-20% gradient of ethyl acetate/petroleum ether) to give ((3$^4$Z,7E)-4$^4$-bromo-1$^4$,1$^4$-difluoro-2$^6$-methyl-3$^1$H-2(2,4)-pyrimidin-1(1,3),5(1,4)-dipiperidin-3(4,1)-triazole-4(1,2)-benzocy clooct-7-en-4$^5$-yl)(cyclopropyl)methanone (200 mg, 0.31 mmol, yield: 69%) as a yellow solid. LCMS (ESI): $[M+H]^+$ = 626.0.

**Step 2:** To a solution of (3$^4$Z,7E)-4$^4$-bromo-1$^4$,1$^4$-difluoro-2$^6$-methyl-3$^1$H-2(2,4)-pyrimidin-1(1,3),5(1,4)-dipiperidin-3(4,1)-triazole-4(1,2)-benzocyc looct-7-en-4$^5$-yl) (cyclopropyl)methanone (200 mg, 0.32 mmol), 2-hydroxyethanesulfonamide (80.1 mg, 0.64 mmol) and potassium acetate (95.2 mg, 0.96 mmol) in dioxane (4 mL) was added [dicyclohexyl [3-(1-methylethoxy)-2',4',6'-tris(1-methylethyl)[1,1'-biphenyl]-2-yl]phosphine-κP](methanesulfonic acid-κO)[2'-(methylamino-κN)[1,1'-biphenyl]-2-yl-κC]palladium (29.4 mg, 0.03 mmol). The mixture was heated to 80°C and stirred for 3 h under nitrogen atmosphere. Water (20 mL) was added to the reaction mixture and extracted with ethyl acetate (10 mL × 2). The organic phases were combined, dried over anhydrous sodium sulfate, and filtered, and the filtrate was concentrated to give the residue. The residue was purified by preparative HPLC (C18, 50-90% gradient of water (formic acid)/acetonitrile) to give N-((3$^4$Z,7E)-4$^5$-(cyclopropanecarbonyl)-1$^4$,1$^4$-difluoro-2$^6$-methyl-3$^1$H-2(2,4)-pyrimidin-1(1,3),5(1,4)-dipiperidin-3(4,1)-tria zole-4(1,2)-benzocyclooct-7-en-4$^4$-yl)-2-hydroxyethane-1-sulfonamide (3.74 mg, 0.005 mmol, yield: 1.75%) as a white solid. LCMS (ESI): $[M+H]^+$ = 669.3. $^1$H NMR (400 MHz, DMSO-$d_6$) δ ppm 9.27-9.01 (m, 1H), 8.07-7.96 (m, 1H), 7.36 (s, 1H), 7.29-7.19 (m, 1H), 5.80 (td, $J$ = 7.6, 15.0 Hz, 1H), 5.50-5.30 (m, 1H), 5.24-5.05 (m, 2H), 5.03-4.82 (m, 2H), 3.96-3.55 (m, 5H), 3.29-3.20 (m, 2H), 3.10-2.79 (m, 3H), 2.46-2.32 (m, 4H), 2.27-2.17 (m, 1H), 2.15-1.95 (m, 2H), 1.94-1.80 (m, 2H), 1.74-1.51 (m, 3H), 1.41-1.22 (m, 4H), 0.98-0.84 (m, 1H); SFC analysis (column: (S,S)-Whelk-0-1.8 50 × 4.6 mm I.D., 1.8 μm; mobile phase: carbon dioxide for phase A and 0.05% diethylamine/ethanol for phase B; gradient: 45% for phase B; flow rate: 2.2 mL/min).

**Example 90: (3$^4$Z,7E)-1$^4$,1$^4$-difluoro-4$^4$-((2-hydroxyethyl)sulfonamido)-N-methoxy-N,2$^6$-dimethyl-3$^1$H-2(2,4)-pyrimidin-1(1,3),5(1,4) -dipiperidin-3(4,1)-triazole-4(1,2)-benzocyclooct-7-en-45-carboxamide**

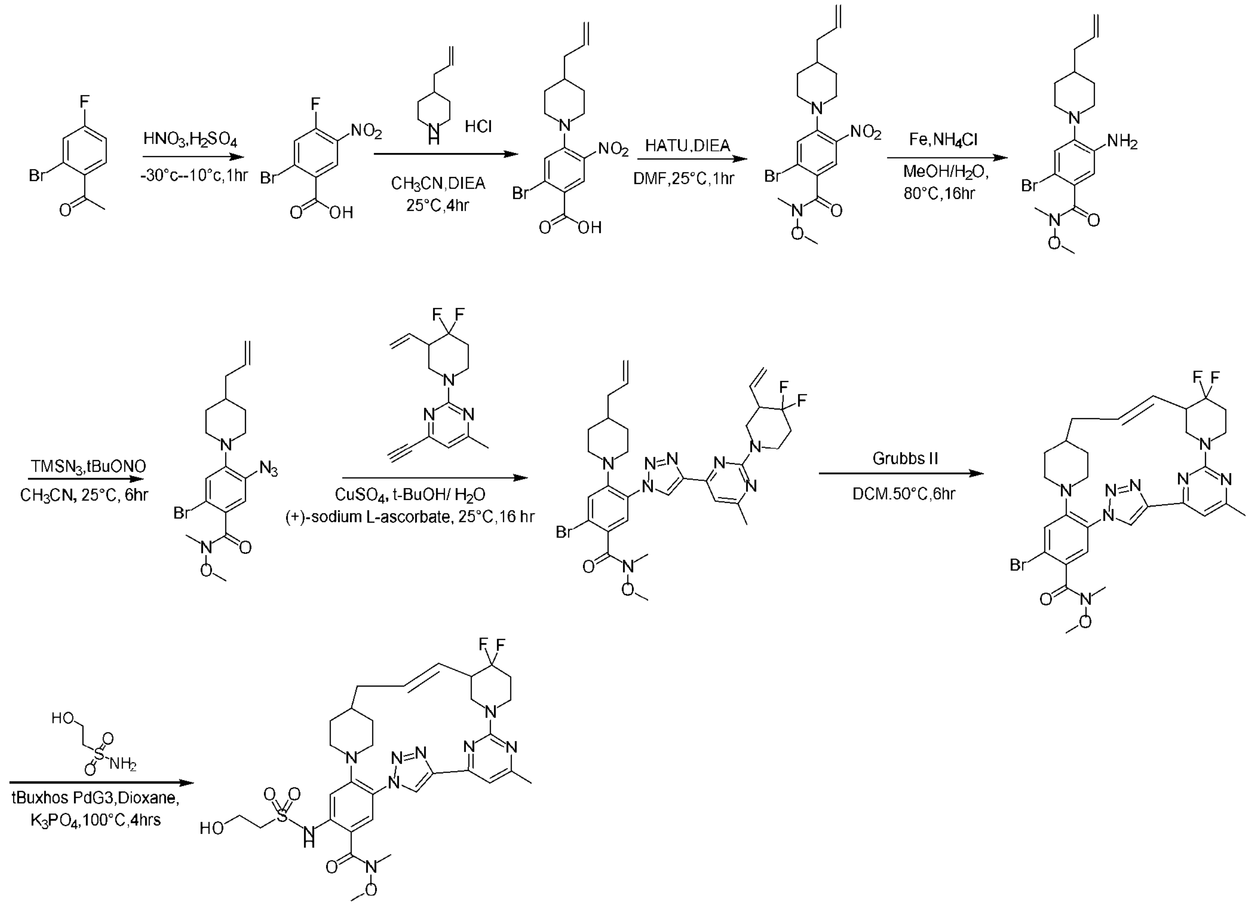


**Step 1:** To a solution of 1-(2-bromo-4-fluorophenyl)ethane-1-one (10.0 g, 46.0 mmol) in sulfuric acid (100 mL) was slowly added fuming nitric acid (22.3 g, 230 mmol) dropwise at -30°C, and the mixture was stirred at -10°C for 1 h. The reaction solution was diluted with water (500 mL) to give 2-bromo-4-fluoro-5-nitrobenzoic acid (7.50 g, 28.5 mmol, yield: 62.0%) as a white solid. $^1H$ NMR (400 MHz, DMSO-$d_6$) δ ppm 14.53 - 12.32 (m, 1H), 8.50 (d, $J$ = 8.0 Hz, 1H), 8.16 (d, $J$ = 10.8 Hz, 1H)

**Step 2:** To a solution of 2-bromo-4-fluoro-5-nitrobenzoic acid (7.50 g, 28.4 mmol) and N,N-diisopropylethylamine (12.7 g, 96.3 mmol) in acetonitrile (100 mL) was added 4-(prop-2-en-1-yl)piperidine hydrochloride (5.50 g, 34.0 mmol). The mixture was stirred at 25°C for 4 h. Water (100 mL) was poured into the reaction mixture, which was extracted with ethyl acetate (100 mL × 3). The organic phases were combined, dried over anhydrous sodium sulfate, and filtered, and the filtrate was concentrated to give the residue. The residue was purified by flash column chromatography (silica gel, 0-10% gradient of ethyl acetate/petroleum ether) to give 2-bromo-5-nitro-4-[4-(prop-2-en-1-yl)piperidin-1-yl]benzoic acid (10.0 g, 27.0 mmol, yield: 95.3%) as a yellow solid. LCMS (ESI): $[M+H]^+$ = 369.0.

**Step 3:** To a solution of 2-bromo-5-nitro-4-[4-(prop-2-en-1-yl)piperidin-1-yl]benzoic acid (9.50 g, 25.7 mmol), N,O-dimethylhydroxylamine hydrochloride (3.07 g, 30.8 mmol) and N,N-diisopropylethylamine (10.2 g, 77.2 mmol) in N,N-dimethylformamide (100 mL) was added O-(7-azabenzotriazole-1-yl)-N,N,N',N'-tetramethylurea hexafluorophosphate (11.9 g, 30.8 mmol). The mixture was stirred at 25°C for 1 h. Extracted with ethyl acetate (100 mL × 3). The organic phases were combined, dried over anhydrous sodium sulfate, filtered, and concentrated to give 2-bromo-N-methoxy-N-methyl-5-nitro-4-[4-(prop-2-en-1-yl)piperidin-1-yl]benzamide (10.0 g, 24.2 mmol, yield: 94.3%) as a yellow solid. LCMS (ESI): $[M+H]^+$ =413.7.

**Step 4:** To a solution of 2-bromo-N-methoxy-N-methyl-5-nitro-4-[4-(prop-2-en-1-yl)piperidin-1-yl]benzamide (10.0 g, 24.2 mmol) in methanol (100 mL) and water (20 mL) were added ammonium chloride (3.93 g, 72.7 mmol) and iron powder (13.7 g, 242 mmol). The mixture was heated to 80°C and stirred for 16 h. The reaction mixture was filtered with diatomite and the filtrate was extracted with ethyl acetate (100 mL × 3). The organic phases were combined, dried over anhydrous sodium sulfate, and filtered. The filtrate was spin-dried. The residue was purified by flash column chromatography (silica gel, 0-10% gradient of ethyl acetate/petroleum ether) to give 5-amino-2-bromo-N-methoxy-N-methyl-4-[4-(prop-2-en-1-yl)piperidin-1-yl]benzamide (8.00 g, 20.8 mmol, yield: 86.3%) as a yellow oil. LCMS (ESI): $[M+H]^+$ =382.0. $^1H$ NMR (400 MHz, DMSO-$d_6$) δ ppm 6.97 (s, 1H), 6.64 (s, 1H), 5.90 - 5.78 (m, 1H), 5.76 (s, 1H), 5.12 - 4.86 (m, 4H), 3.87 - 3.48 (m, 3H), 3.27 - 2.98 (m, 5H), 2.49 - 2.44 (m, 1H), 2.12 - 1.97 (m, 2H), 1.87 - 1.65 (m, 2H), 1.47 -

1.21 (m, 3H)

**Step 5:** To a solution of 5-amino-2-bromo-N-methoxy-N-methyl-4-[4-(prop-2-en-1-yl)piperidin-1-yl]benzamide (7.40 g, 19.3 mmol) in acetonitrile (150 mL) was added tert-butyl nitrite (3.05 g, 29.0 mmol) at 0°C. Then, azidotrimethylsilane (3.34 g, 29.0 mmol) was added to the reaction mixture and the mixture was stirred at 25°C for 6 h. The reaction mixture was poured into water (40 mL) and extracted with ethyl acetate (150 mL × 2). The organic phases were combined, dried over anhydrous sodium sulfate, and filtered, and the filtrate was concentrated to give the residue. The residue was purified by flash column chromatography (silica gel, 0-10% gradient of ethyl acetate/petroleum ether) to give 5-azido-2-bromo-N-methoxy-N-methyl-4-[4-(prop-2-en-1-yl)piperidin-1-yl]benzamide (7.60 g, 18.5 mmol, yield: 96.1%) as a yellow oil. LCMS (ESI): $[M+H]^+$ =409.8.

**Step 6:** To a solution of 5-azido-2-bromo-N-methoxy-N-methyl-4-[4-(prop-2-en-1-yl)piperidin-1-yl]benzamide (2.50 g, 6.12 mmol) and 2-(3-vinyl-4,4-difluoropiperidin-1-yl)-4-ethynyl-6-methylpyrimidine (1.61 g, 6.12 mmol) in tert-butanol (15 mL), tetrahydrofuran (15 mL) and water (15 mL) were added copper sulfate (1.17 g, 7.34 mmol) and sodium ascorbate (1.45 g, 7.34 mmol). The mixture was stirred at 25°C for 16 h. The reaction mixture was added with water (20 mL) and extracted with ethyl acetate (20 mL × 3). The organic phases were combined, dried over anhydrous sodium sulfate, and filtered, and the filtrate was concentrated to give the residue. The residue was purified by flash column chromatography (silica gel, 0-30% gradient of ethyl acetate/petroleum ether) to give 2-bromo-5-{4-[2-(3-vinyl-4,4-difluoropiperidin-1-yl)-6-methylpyrimidin-4-yl]-1H-1,2,3-triazol-1-yl}-N-methoxy-N-methyl -4-[4-(prop-2-en-1-yl)piperidin-1-yl]benzamide (3.20 g, 4.76 mmol, yield: 77.8%) as a yellow solid. LCMS (ESI): $[M+H]^+$ =673.6.

**Step 7:** To a solution of 2-bromo-5-{4-[2-(3-vinyl-4,4-difluoropiperidin-1-yl)-6-methylpyrimidin-4-yl]-1H-1,2,3-triazol-1-yl}-N-methoxy-N-methyl -4-[4-(prop-2-en-1-yl)piperidin-1-yl]benzamide (2.50 g, 1.17 mmol) in dichloromethane (700 mL) was added Grubbs catalyst 2nd generation (0.31 g, 0.37 mmol), and the mixture was stirred at 50°C for 6 h under nitrogen atmosphere. The reaction mixture was concentrated under reduced pressure. The residue was purified by flash column chromatography (silica gel, 0-5% gradient of tetrahydrofuran/petroleum ether) to give ($3^4$Z,7E)-$4^4$-bromo-$1^4$,$1^4$-difluoro-N-methoxy-N, $2^6$-dimethyl-$3^1$H-2(2,4)-pyrimidin-1(1,3),5(1,4)-dipiperidin-3(4,1)-triazole-4(1,2)-benzocyclooct-7-en-$4^5$-carboxamide (1.40 g, 0.32 mmol, yield: 28.0%) as a yellow solid. LCMS (ESI): $[M+H]^+$ = 645.0

**Step 8:** To a solution of ($3^4$Z,7E)-$4^4$-bromo-$1^4$,$1^4$-difluoro-N-methoxy-N, $2^6$-dimethyl-$3^1$H-2(2,4)-pyrimidin-1(1,3),5(1,4)-dipiperidin-3(4,1)-triazole-4(1,2)-benzocyclooct-7-en-$4^5$-carboxamide (300 mg, 0.46 mmol), 2-hydroxyethanesulfonamide (116 mg, 0.93 mmol) and potassium phosphate (296 mg, 1.39 mmol) in N,N-dimethylformamide (6 mL) was added methanesulfonic acid(2-di-tert-butylphosphino-2,4,6-triisopropyl-1,1-biphenyl)(2-amino-1,1-biphenyl-2-yl)palladium (37.0 mg, 0.04 mmol). The mixture was heated to 100°C and stirred for 4 h under nitrogen atmosphere. Water (20 mL) was added to the reaction mixture and extracted with ethyl acetate (10 mL × 2). The organic phases were combined, dried over anhydrous sodium sulfate, and filtered, and the filtrate was concentrated to give the residue. The residue was purified by preparative HPLC (C18, 44-84% gradient of water (trifluoroacetic acid)/acetonitrile) to give ($3^4$Z, 7E)-$1^4$,$1^4$-difluoro-$4^4$-((2-hydroxyethyl)sulfonamido)-N-methoxy-N, $2^6$-dimethyl-$3^1$H-2(2,4)-pyrimidin-1(1,3),5(1,4)-dipiperidin-3(4,1)-triazole-4(1,2)-benzocyclooct-7-en-45-carboxamide (3.00 mg, 2.82 mmol, yield: 0.94%) as a white solid. LCMS (ESI): $[M+H]^+$ =688.3. $^1$H NMR (400 MHz, DMSO-$d_6$) δ ppm 9.05 (s, 1H), 7.38-7.26 (m, 1H), 7.21 (s, 1H), 7.14 (s, 1H), 5.89-5.74 (m, 1H), 5.45-5.30 (m, 1H), 5.08-4.64 (m, 3H), 3.71 (br t, *J* = 6.4 Hz, 2H), 3.62 (s, 3H), 3.49-3.46 (m, 1H), 3.20-3.16 (m, 4H), 3.05-2.94 (m, 2H), 2.90-2.79 (m, 1H), 2.75-2.66 (m, 1H), 2.39 (s, 3H), 2.28-2.17 (m, 2H), 2.02-1.82 (m, 4H), 1.69-1.51 (m, 4H), 1.43-1.32 (m, 2H), 1.01 (br s, 1H); SFC analysis (column: (S,S)-Whelk-0-1.8 50 × 4.6 mm I.D., 1.8 μm; mobile phase: carbon dioxide for phase A and 0.05% diethylamine/ethanol for phase B; gradient: 45% for phase B; flow rate: 4 mL/min).

**Example 91: $1^4$,$1^4$-difluoro-$4^4$-((2-hydroxyethyl)sulfonamido)-$2^6$-methyl-$3^1$*H*-2(2,4)-pyrimidin-1(1,3),5(1,4)-dipiperidin-3(4,1)-triaz ole-4(1,2)-benzocyclooct-7-en-$4^5$ carboxylic acid**

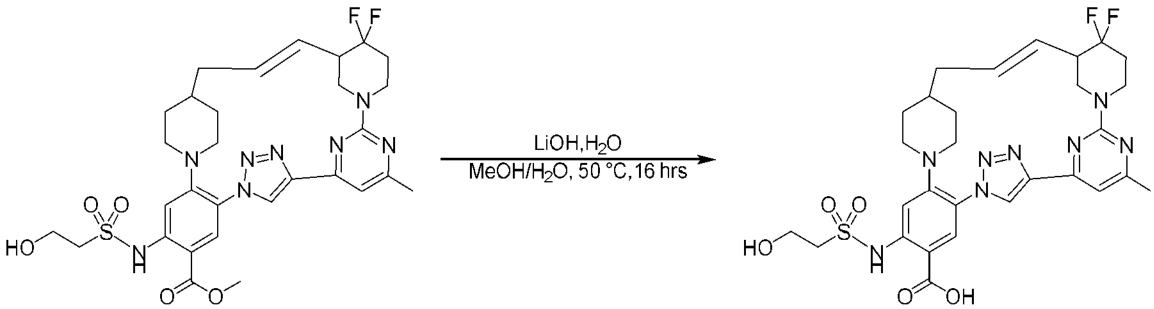

To a solution of methyl($1^3S,3^4Z,7E$)-$1^4,1^4$-difluoro-$4^4$-((2-hydroxyethyl)sulfonamido)-$2^6$-methyl $3^1H$-2(2,4)-pyrimidin-1(1,3),5(1,4)-dipiperidin-3(4,1)-triazole-4(1,2)-benzocyclooct-7-en-$4^5$ carboxylate (40.0 mg, 0.06 mmol) in methanol (1.00 mL) was added lithium hydroxide monohydrate (7.64 mg, 0.18 mmol) in water (0.50 mL), and the mixture was stirred at 50°C for 16 h. The reaction mixture was diluted with 10 mL of water and the pH was adjusted to 4 with dilute hydrochloric acid. The mixture was extracted with ethyl acetate (20 mL $\times$ 2). The organic layer was dried, filtered and concentrated. The residue was purified by preparative HPLC (C18, 20-60% gradient of water (ammonium bicarbonate)/acetonitrile) to give ($3^4Z,7E$)-$1^4,1^4$-difluoro-$4^4$-((2-hydroxyethyl)sulfonamido)-$2^6$-methyl-$3^1H$-2(2,4)-pyrimidin-1(1,3),5(1,4)-dipiperidin-3(4,1)-triazole-4(1,2)-benzocyclooct-7-en-$4^5$carboxylic acid (8.49 mg, 0.02 mmol, yield: 40.0%) as a white solid. LCMS (ESI): $[M+H]^+$ =645.2; SFC analysis (column: Chiralpak AD-3 50 $\times$ 4.6 mm I.D., 3 $\mu$m; mobile phase: carbon dioxide for phase A and 0.05% diethylamine/isopropanol for phase B; gradient: 40% of phase B; flow rate: 4 mL/min); $^1$H NMR (400 MHz, DMSO-$d_6$) $\delta$ ppm 9.11 - 9.03 (m, 1H), 8.41 - 8.10 (m, 1H), 7.43 -7.15 (m, 3H), 5.80 (ddd, $J$ = 6.8, 9.2, 15.2 Hz, 1H), 5.38 (dd, $J$ = 8.8, 15.2 Hz, 1H), 5.05 (br d, $J$ = 14.0 Hz, 1H), 4.91 (br d, $J$ = 12.4 Hz, 2H), 3.74 (br t, $J$ = 6.4 Hz, 2H), 3.39 (br s, 2H), 3.22 - 2.97 (m, 3H), 2.90 - 2.75 (m, 2H), 2.44 - 2.38 (m, 3H), 2.36 - 2.27 (m, 1H), 2.25 - 2.07 (m, 2H), 2.05 - 2.00 (m, 1H), 1.93 - 1.82 (m, 2H), 1.70 - 1.65 (m, 1H), 1.62 - 1.51 (m, 2H), 1.39 - 1.27 (m, 1H), 0.94 (br d, $J$ = 12.4 Hz, 1H).

**Example 92: Methyl-$1^4,1^4$-difluoro-$4^4$-((2-hydroxyethyl)sulfonamido)-$2^6$-methyl-$3^1H$-2(2,4)-pyrimidin-1(1,3),5(1,4)-dipiperidin-3(4, 1)-triazole-4(1,2)-benzocyclooct-7-en-$4^5$ carboxylate**

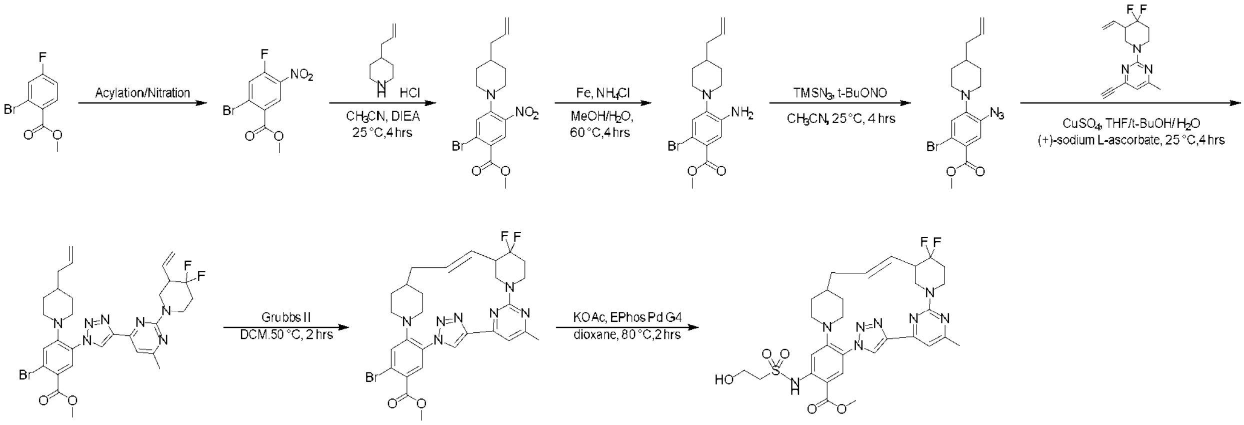


**Step 1:** A solution of methyl 2-bromo-4-fluorobenzoate (2.00 g, 8.58 mmol) in concentrated sulfuric acid (5 V) was transferred into pump 1 {S1, P1, 2 mL/min}, and a solution of nitric acid (3eq, 68% purity) in concentrated sulfuric acid (5 V) was transferred into pump 2 {S1, P1, 2 mL/min}. The reaction was carried out using the reaction device {FLR1, PFA, Coils reactor, 3.175(1/8") mm, 20 mL, 0°C, 20 min}. The mixture was quenched with ice water (100 mL), filtered, and concentrated under reduced pressure to give methyl 2-bromo-4-fluoro-5-nitrobenzoate (1.60 g, 5.74 mmol, yield: 67.0%) as a yellow solid. $^1$H NMR (400 MHz, CHLOROFORM-d) $\delta$ ppm 8.65 (d, $J$ = 7.6 Hz, 1H), 7.70 (d, $J$ = 10.0 Hz, 1H), 4.00 (s, 3H)

**Step 2**: To a solution of methyl 2-bromo-4-fluoro-5-nitrobenzoate (1.60 g, 5.75 mmol) and 4-allylpiperidine hydrochloride (1.12 g, 6.91 mmol) in acetonitrile (32.0 mL) was added N,N-diisopropylethylamine (2.28 g, 17.2 mmol), and the mixture was stirred at 25°C for 4 h. The reaction mixture was diluted with water (100 mL), extracted with ethyl acetate (200 mL $\times$ 2), and the organic phase was dried, filtered and concentrated. The residue was purified by flash column chromatography (silica gel, 0-7% gradient of tetrahydrofuran/petroleum ether) to give methyl 4-(4-allylpiperidin-1-yl)-2-bromo-5-nitrobenzoate (1.80 g, 4.48 mmol, yield: 78.0%) as a yellow oil. LCMS (ESI): $[M+H]^+$ = 384.9

**Step 3:** To a solution of methyl 4-(4-allylpiperidin-1-yl)-2-bromo-5-nitrobenzoate (1.00 g, 2.61 mmol) in methanol (20.0 mL) were added ammonium chloride (0.42 g, 7.83 mmol) in water (4.00 mL) and iron powder (1.47 g, 26.0 mmol) in batches at 50°C, and the mixture was stirred at 60°C for 4 h. The reaction solution was filtered and concentrated to remove the solvent. The residue was purified by flash column chromatography (silica gel, 0-15% gradient of tetrahydrofuran/petroleum ether) to give methyl 4-(4-allylpiperidin-1-yl)-5-amino-2-bromobenzoate (0.77 g, 1.95 mmol, yield: 75.0%) as a brown solid. LCMS (ESI): $[M+H]^+$ = 384.8. $^1$H NMR (400 MHz, CHLOROFORM-d) $\delta$ ppm 7.24 (s, 1H), 7.16 (s, 1H), 5.91 - 5.74 (m, 1H), 5.13 - 4.98 (m, 2H), 4.03 - 3.84 (m, 5H), 3.22 (br d, $J$ = 12.0 Hz, 2H), 2.65 - 2.54 (m, 2H), 2.15 - 2.05 (m, 2H), 1.84 (br d, $J$= 12.8 Hz, 2H), 1.55 - 1.47 (m, 1H), 1.43 - 1.30 (m, 2H)

**Step 4:** To a solution of methyl 4-(4-allylpiperidin-1-yl)-5-amino-2-bromobenzoate (770 mg, 2.18 mmol) in acetonitrile

(15.0 mL) were added azidotrimethylsilane (376 mg, 3.27 mmol) and tert-butyl nitrite (344 mg, 3.27 mmol), and the mixture was stirred at 25°C for 4 h. The reaction mixture was diluted with water (100 mL), extracted with ethyl acetate (200 mL × 2), and the organic phase was dried, filtered and concentrated. The residue was purified by flash column chromatography (silica gel, 0-5% gradient of tetrahydrofuran/petroleum ether) to give methyl 4-(4-allylpiperidin-1-yl)-5-azido-2-bromobenzoate (710 mg, 1.76 mmol, yield: 81.0%) as a yellow oil. LCMS (ESI): $[M+H]^+$ = 381.0.

**Step 5:** To a solution of methyl 4-(4-allylpiperidin-1-yl)-5-azido-2-bromobenzoate (0.71 g, 1.87 mmol) and 2-(4,4-difluoro-3-vinylpiperidin-1-yl)-4-ethynyl-6-methylpyrimidine (0.49 g, 1.87 mmol) in tetrahydrofuran (7.00 mL), tert-butanol (7.00 mL) and water (7.00 mL) were added sodium ascorbate (0.45 g, 2.25 mmol) and anhydrous copper sulfate (0.36 g, 2.25 mmol), and the mixture was stirred at 25°C for 4 h. The reaction mixture was diluted with water (100 mL), extracted with ethyl acetate (100 mL × 2), and the organic phase was dried, filtered and concentrated. The residue was purified by flash column chromatography (silica gel, 0-9% gradient of ethyl acetate/petroleum ether) to give methyl 4-(4-allylpiperidin-1-yl)-2-bromo-5-(4-(2-(4,4-difluoro-3-vinylpiperidin-1-yl)-6-methylpyrimidin-4-yl)-1H-1,2,3-triazole-1-yl)benzoate (950 mg, 1.23 mmol, yield: 66.2%) as a yellow solid. LCMS (ESI): $[M+H]^+$ = 643.4

**Step 6:** To a solution of methyl 4-(4-allylpiperidin-1-yl)-2-bromo-5-(4-(2-(4,4-difluoro-3-vinylpiperidin-1-yl)-6-methylpyrimidin-4-yl)-1H-1,2,3-triazol-1-y l)benzoate (900 mg, 1.40 mmol) in dichloromethane (360 mL) was added Grubbs catalyst 2nd generation (121 mg, 0.14 mmol), and the mixture was stirred at 50°C for 2 h under nitrogen atmosphere. The reaction mixture was concentrated under reduced pressure. The residue was purified by flash column chromatography (silica gel, 0-8% gradient tetrahydrofuran/petroleum ether) to give methyl-($3^4$Z,7E)-$4^4$-bromo-$1^4$,$1^4$-difluoro-$2^6$-methyl-$3^1$*H*-2(2,4)-pyrimidin-1(1,3),5(1,4)-dipiperidin-3(4,1)-triazole-4(1,2)-b enzocyclooct-7-en-$4^5$carboxylate (800 mg, 0.98 mmol, yield: 70.0%) as a yellow solid. LCMS (ESI): $[M+H]^+$ = 616.0

**Step 7:** To a solution of methyl($3^4$Z,7E)-$4^4$-bromo-$1^4$,$1^4$-difluoro-26-methyl-$3^1$*H*-2(2,4)-pyrimidin-1(1,3),5(1,4)-dipiperidin-3(4,1)-triazole-4(1,2)-be nzocyclooct-7-en-$4^5$carboxylate (400 mg, 0.65 mmol) and 2-hydroxyethane-1-sulfonamide (162 mg, 1.30 mmol) in dioxane (8.00 mL) were added potassium acetate (193 mg, 1.95 mmol) and Ephos Pd G4 (59.7 mg, 0.07 mmol), and the mixture was stirred at 80°C for 2 h under nitrogen atmosphere. The reaction mixture was diluted with water (50.0 mL), extracted with ethyl acetate (100 mL × 2), and the organic phase was dried, filtered and concentrated. The residue was purified by flash column chromatography (silica gel, 0-36% gradient of tetrahydrofuran/petroleum ether) to give methyl($1^3$S, $3^4$Z, 7E)-$1^4$,$1^4$-difluoro-$4^4$-((2-hydroxyethyl)sulfonamido)-$2^6$-methyl $3^1$*H*-2(2,4)-pyrimidin-1(1,3),5(1,4)-dipiperidin-3(4,1)-triazole-4(1,2)-benzocyclooct-7-en-$4^5$ carboxylate (420 mg, 0.42 mmol, yield: 65.0%) as a yellow solid. LCMS (ESI): $[M+H]^+$ = 659.2

**Step 8:** The crude product was purified by preparative HPLC (C18, 52-92% gradient of water (formic acid)/acetonitrile) to give methyl($3^4$Z,7E)-$1^4$,$1^4$-difluoro-$4^4$-((2-hydroxyethyl)sulfonamido)-$2^6$-methyl $3^1$*H*-2(2,4)-pyrimidin-1(1,3),5(1,4)-dipiperidin-3(4,1)-triazole-4(1,2)-benzocyclooct-7-en-$4^5$carboxylate (2.49 mg) as a white solid P1 **(Example 92A).** LCMS (ESI): $[M+H]^+$ =659.2; SFC analysis (column: Chiralpak IB N-3 50 × 4.6 mm I.D., 3 μm; mobile phase: carbon dioxide for phase A and 0.05% diethylamine/ethanol for phase B; gradient: 40% for phase B; flow rate: 4 mL/min). $^1$H NMR (400 MHz, DMSO-$d_6$) δ ppm 10.60 - 10.14 (m, 1H), 9.10 (s, 1H), 8.13 (s, 1H), 7.32 (s, 1H), 7.23 (s, 1H), 5.79 (ddd, *J* = 6.4, 8.4, 15.2 Hz, 1H), 5.38 (dd, *J*= 8.8, 14.8 Hz, 1H), 5.17 - 4.87 (m, 3H), 3.87 (s, 3H), 3.78 (br s, 2H), 3.57 - 3.46 (m, 2H), 3.43 - 3.38 (m, 1H), 3.29 - 3.25 (m, 1H), 3.08 - 3.00 (m, 1H), 2.92 - 2.81 (m, 2H), 2.41 (s, 3H), 2.38 - 2.32 (m, 1H), 2.25 - 2.17 (m, 1H), 2.13 - 1.99 (m, 2H), 1.91 - 1.81 (m, 2H), 1.71 - 1.54 (m, 3H), 1.38 - 1.27 (m, 1H), 0.93 (br d, *J*= 11.6 Hz, 1H).

And P2 **(Example 92B):** Methyl($3^4$Z,7Z)-$1^4$,$1^4$-difluoro-$4^4$-((2-hydroxyethyl)sulfonamido)-$2^6$-methyl $3^1$*H*-2(2,4)-pyrimidin-1(1,3),5(1,4)-dipiperidin-3(4,1)-triazole-4(1,2)-benzocyclooct-7-en-$4^5$ carboxylate (10.31 mg). LCMS (ESI): $[M+H]^+$ =659.2

SFC analysis (column: Chiralpak IB N-3 50 × 4.6 mm I.D., 3 μm; mobile phase: carbon dioxide for phase A and 0.05% diethylamine/ethanol for phase B; gradient: 40% for phase B; flow rate: 4 mL/min). $^1$H NMR (400 MHz, DMSO-$d_6$) δ ppm 10.40 (br s, 1H), 9.14 - 9.04 (m, 1H), 8.41 - 8.05 (m, 1H), 7.46 - 7.30 (m, 1H), 7.26 - 7.10 (m, 1H), 5.85 - 5.72 (m, 1H), 5.38 (dd, *J* = 8.8, 15.2 Hz, 1H), 5.14 - 4.91 (m, 3H), 3.90 - 3.86 (m, 3H), 3.79 (br d, *J* = 4.0 Hz, 2H), 3.52 (br t, *J* = 6.0 Hz, 2H), 3.40 (br s, 1H), 3.30 - 3.25 (m, 1H), 3.06 - 3.00 (m, 1H), 2.92 - 2.81 (m, 2H), 2.43 - 2.39 (m, 3H), 2.35 (br d, *J* = 12.8 Hz, 1H), 2.24 - 2.17 (m, 1H), 2.13 - 1.98 (m, 2H), 1.91 - 1.81 (m, 2H), 1.71 - 1.56 (m, 3H), 1.37 - 1.26 (m, 1H), 0.93 (br d, *J* = 12.8 Hz, 1H).

**Example 93:** ($3^4$*Z*,7*E*)-$1^4$,$1^4$-difluoro-$4^4$-((2-hydroxyethyl)sulfonamido)-$2^6$-methyl-$3^1$*H*-2(2,4)-pyrimidin-1(1,3),5(1,4)-dipiperidin-3(4,1)-triazole-4(1,2)-benzocyclooct-7-en-$4^5$carboxamide

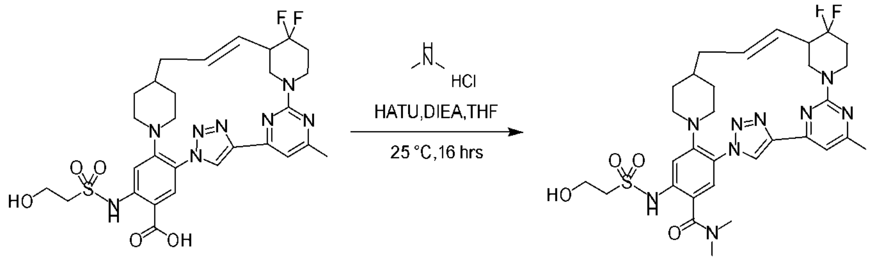


To a solution of $1^4$,$1^4$-difluoro-$4^4$-((2-hydroxyethyl)sulfonamido)-$2^6$-methyl-$3^1H$-2(2,4)-pyrimidin-1(1,3),5(1,4)-dipiperidin-3(4,1)-triazole-4 (1,2)-benzocyclooct-7-en-$4^5$carboxylic acid (50.0 mg, 0.08 mmol) in N,N-dimethylformamide (1.00 mL) were added dimethylamine hydrochloride (70.8 mg, 0.47 mmol), N,N-diisopropylethylamine (50.1 mg, 0.39 mmol) and O-(7-azabenzotriazol-1-yl)-N,N,N,N-tetramethyluronium hexafluorophosphate (88.4 mg, 0.23 mmol), and the mixture was stirred at 25°C for 16 h. The reaction mixture was diluted with water (10.0 mL), extracted with ethyl acetate (20.0 mL × 2), and the organic phase was dried, filtered and concentrated. The residue was purified by preparative HPLC (C18, 40-80% gradient of water (formic acid)/acetonitrile) to give ($3^4Z$,$7E$)-$1^4$,$1^4$-difluoro-$4^4$-((2-hydroxyethyl)sulfonamido)-$2^6$-methyl-$3^1H$-2(2,4)-pyrimidin-1(1,3),5(1,4)-dipiperidin-3(4,1)-triazole-4(1,2)-benzocyclooct-7-en-$4^5$carboxamide (5.37 mg, 0.01 mmol, yield: 10.0%) as a white solid. LCMS (ESI): $[M+H]^+$ = 672.3; SFC analysis (column: Chiralpak IG 50 x 4.6 mm I.D., 3 μm; mobile phase: carbon dioxide for phase A and 0.05% diethylamine/ethanol for phase B; gradient: 40% of phase B; flow rate: 4 mL/min); $^1H$ NMR (400 MHz, DMSO-$d_6$) δ ppm 9.33 (s, 1H), 9.16 (s, 1H), 7.60 (s, 1H), 7.26 (s, 1H), 7.23 (s, 1H), 5.86 - 5.74 (m, 1H), 5.39 (dd, $J$= 8.8, 15.2 Hz, 1H), 4.99 (br d, $J$= 12.8 Hz, 1H), 4.86 (br d, $J$= 13.2 Hz, 1H), 3.78 (t, $J$= 6.4 Hz, 2H), 3.41 - 3.38 (m, 2H), 3.27 (br d, $J$= 12.0 Hz, 2H), 3.14 - 3.07 (m, 1H), 2.96 (s, 6H), 2.90 - 2.77 (m, 2H), 2.40 (s, 3H), 2.31 (br t, $J$ = 12.0 Hz, 1H), 2.26 - 2.18 (m, 1H), 2.14- 2.08 (m, 1H), 2.05 - 2.00 (m, 1H), 1.93 - 1.83 (m, 2H), 1.70 - 1.54 (m, 3H), 1.41 - 1.29 (m, 1H), 0.98 (br d, $J$ = 12.0 Hz, 1H).

**Example 94: 2-hydroxy-N-(8-methyl-$2^1$H-6-oxa-10-aza-1(2,6)-pyridin-4-(1,4)-piperidin-2-(4,1)-triazole-3(1,2)-benzocyclodecane-3 4-yl)ethane-1-sulfonamide**

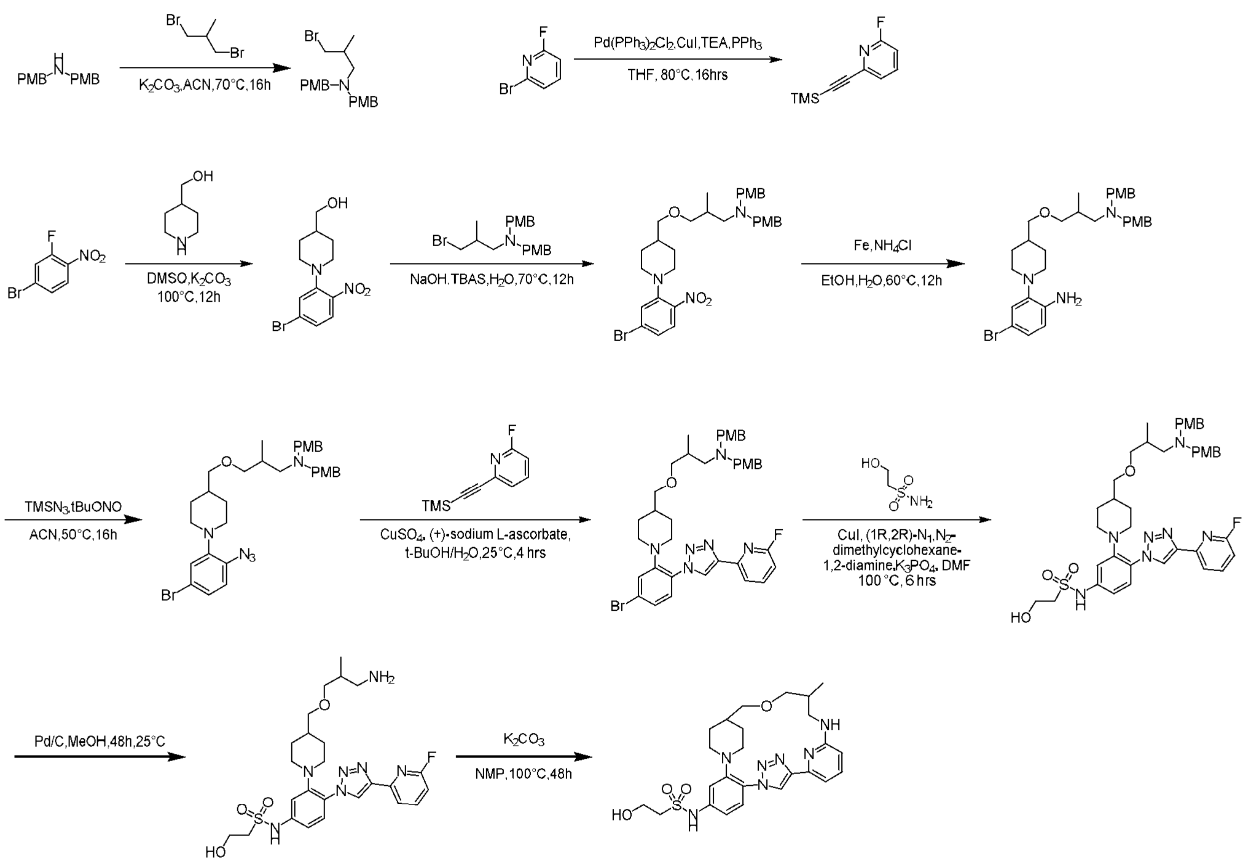

Step 1: To a solution of di[(4-methoxyphenyl)methyl]amine (12.0 g, 46.6 mmol) and 1,3-dibromo-2-methyl-propane (100 g, 466 mmol) in acetonitrile (240 mL) was added potassium carbonate (13.2 g, 93.3 mmol), and the mixture was stirred at 70°C for 16 h. The reaction mixture was poured into water (400 mL) and extracted with EA (400 mL × 2). The combined organic layer was washed with saturated saline (400 mL), dried over anhydrous magnesium sulfate, filtered, and concentrated under reduced pressure to give a residue. The residue was purified by flash column chromatography (silica gel, 0-3% gradient of tetrahydrofuran/petroleum ether) to give (3-bromo-2-methylpropyl)di[(4-methoxyphenyl)methyl]amine (10.5 g, 26.6 mmol, yield: 57%) as a yellow oil. $^{1}$H NMR (400 MHz, CHLOROFORM-d) δ ppm 7.26 (d, *J* = 8.4 Hz, 4H), 6.88 (d, *J*= 8.4 Hz, 4H), 3.83 (s, 6H), 3.60 - 3.53 (m, 3H), 3.44 (d, *J* = 8.8 Hz, 2H), 3.28 (dd, *J* = 6.8, 9.6 Hz, 1H), 2.43 (dd, *J*= 8.8, 12.8 Hz, 1H), 2.25 (dd, *J* = 6.0, 12.8 Hz, 1H), 2.11 - 1.99 (m, 1H), 0.97 (d, *J*= 6.8 Hz, 3H)

Step 2: To a solution of 2-bromo-6-fluoropyridine (10.0 g, 56.8 mmol) and trimethylsilylacetylene (11.2 g, 114 mmol) in tetrahydrofuran (200 mL) were added triethylamine (17.6 g, 170 mmol), bis(triphenylphosphine)palladium(II) chloride (4.03 g, 5.68 mmol) and cuprous iodide (5.52 g, 28.4 mmol) at 25°C, and the reaction mixture was stirred at 80°C for 16 h. The reaction mixture was poured into water (200 mL) and extracted with EA (200 mL × 2). The combined organic layer was washed with saturated saline (200 mL), dried over anhydrous magnesium sulfate, filtered, and concentrated under reduced pressure to give a residue. The residue was purified by flash column chromatography (silica gel, 0-3% gradient of tetrahydrofuran/petroleum ether) to give 2-fluoro-6-[2-(trimethylsilyl)ethynyl]pyridine (10.8 g, 55.7 mmol, yield: 98%) as a yellow oil. $^{1}$H NMR (400 MHz, CHLOROFORM-d) δ ppm 7.62 (q, *J* = 8.0 Hz, 1H), 7.22 (ddd, *J* = 0.8, 2.4, 7.6 Hz, 1H), 6.78 (ddd, *J* = 0.8, 2.8, 8.4 Hz, 1H), 0.19 - 0.12 (m, 9H)

Steps 3 to 9 were conducted with reference to the method for Example **81.**

Step 10: To a solution of N-(3-{4-[(3-amino-2-methylpropoxy)methyl]piperidin-1-yl}-4-[4-(6-fluoropyridin-2-yl)-1H-1,2,3-triazol-1-yl]phenyl)-2-hy droxyethane-1-sulfonamide (10 mg, 0.02 mmol) in 1-methylpyrrolidine-2-one (1.00 mL) was added potassium carbonate (5.05 mg, 0.04 mmol), and the mixture was stirred at 100°C for 48 h under nitrogen atmosphere. The reaction solution was filtered and purified by preparative HPLC (C18, 24-64% gradient of water (aqueous ammonia)/ammonium bicarbonate) to give 2-hydroxy-N-(8-methyl-$2^1$H-6-oxa-10-aza-1(2,6)-pyridin-4-(1,4)-piperidin-2-(4,1)-triazole-3(1,2)-benzocyclodecane-34-yl) ethane-1-sulfonamide (1 mg, 0.002 mmol, yield: 10%) as a white solid.

LCMS (ESI): $[M+H]^+$ = 528.2

$^{1}$H NMR (400 MHz, DMSO-*d6*) δ ppm 9.20 (s, 1H), 7.85 (d, *J* = 8.8 Hz, 1H), 7.59 - 7.47 (m, 2H), 7.26 - 7.18 (m, 2H), 7.13 (dd, *J* = 2.0, 8.8 Hz, 1H), 6.73 (br dd, *J* = 4.8, 8.0 Hz, 1H), 6.57 (d, *J* = 8.4 Hz, 1H), 5.21 - 4.86 (m, 1H), 4.42 (ddd, *J* = 4.4, 8.4, 13.2 Hz, 1H), 3.86 (br t, *J*= 6.4 Hz, 2H), 3.39 (br s, 2H), 3.21 (br t, *J* = 8.8 Hz, 2H), 3.10 - 3.03 (m, 1H), 3.02 - 2.96 (m, 1H), 2.77 (br d, *J* = 1.6 Hz, 2H), 2.19 - 2.02 (m, 3H), 2.01 - 1.74 (m, 2H), 1.72 - 1.53 (m, 3H), 1.47 - 1.38 (m, 1H), 0.99 (d, *J*= *6.8* Hz, 3H)

**Example 95**: N-($4^4$,8-dimethyl-$2^1$H-6-oxa-10-aza-1(2,6)-pyridin-4-(1,4)-piperidin-2(4,1)-triazole-3(1,2)-benzocyclodecane-$3^4$-yl)-2-hydr oxyethane-1-sulfonamide

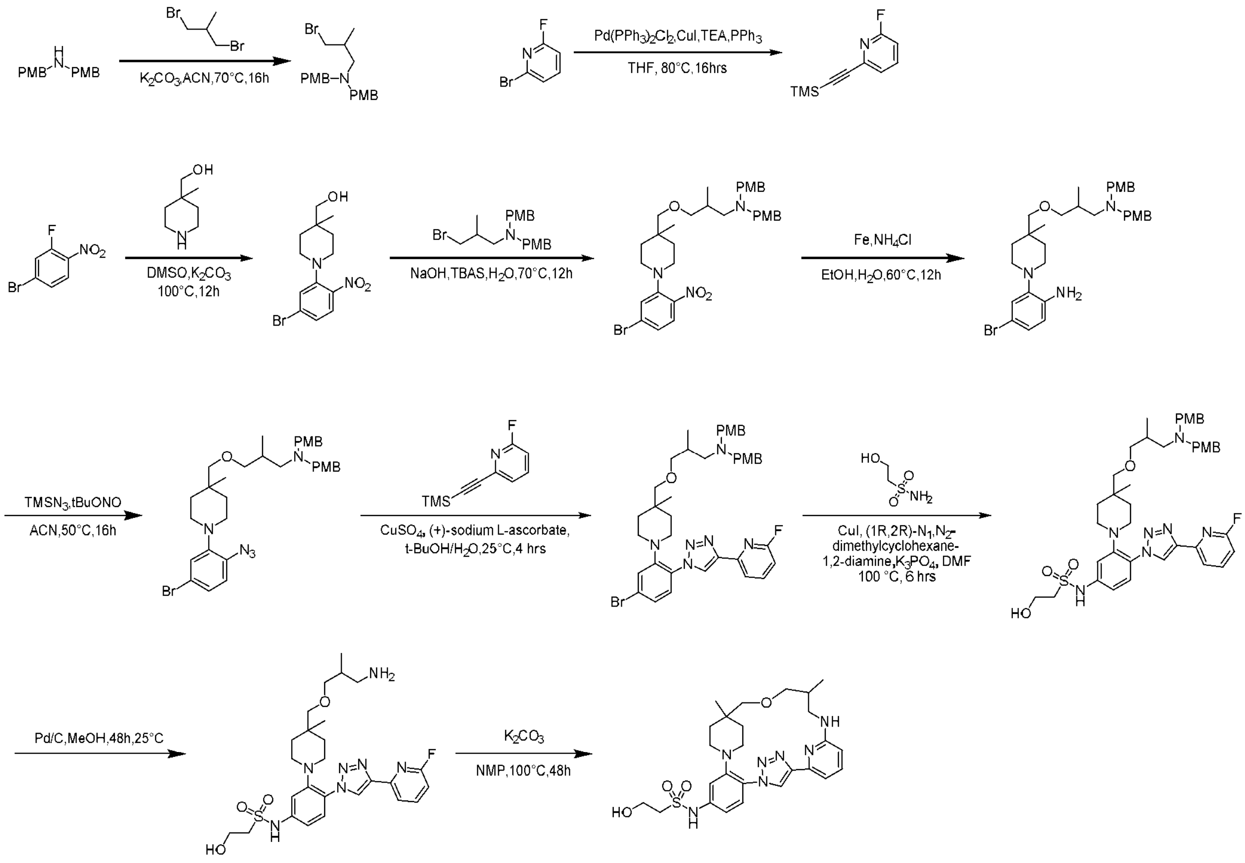


The preparations in **Example 95** followed the synthetic route of **Example 94.** LCMS (ESI): $[M+H]^+$ = 542.2

**Example 96**: **2-Hydroxy-N-(8-methyl-4⁴-(trifluoromethyl)-2¹H-6-oxa-10-aza-1(2,6)-pyridin-4-(1,4)-piperidin-2-(4,1)-triazole-3(1,2) -benzocyclodecane-3⁴-yl)ethane-1-sulfonamide**

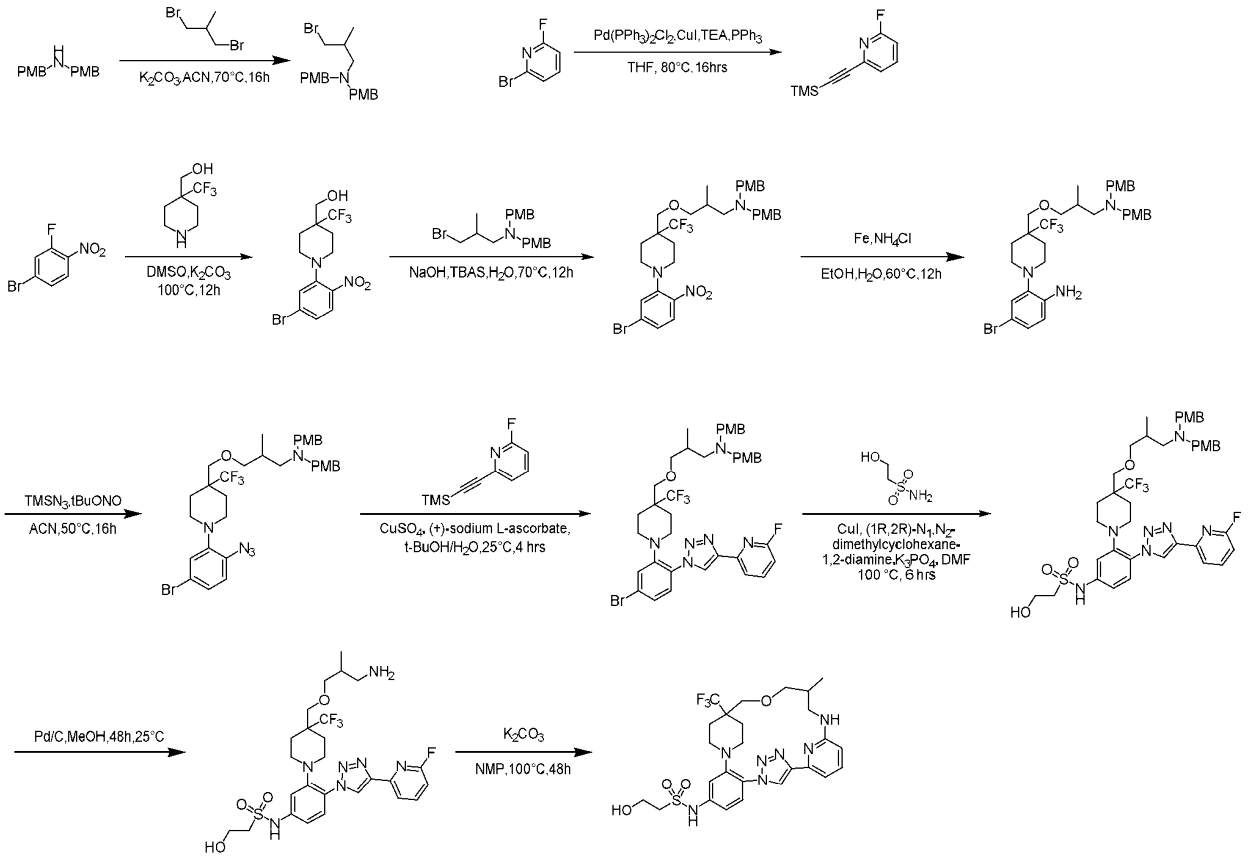


The preparations in **Example 96 followed** the synthetic route of Example 94. LCMS (ESI): $[M+H]^+$ = 596.2

Assay 1 : **Enzymatic Activity Test**

**Materials:** Human KIF18A (amino acid sequence 1-417) was purchased from Viva Biotech (Shanghai) Ltd.; the ADP-Glo™ protein kinase kit was acquired from Promega, USA; tubulin was sourced from Cytoskeleton, USA; and the 384-well assay plate and Envision multifunctional microplate reader were obtained from PerkinElmer, USA.

**Enzymatic Activity Test:** The compound powder was dissolved in DMSO to prepare a stock solution of 10 mM. Then gradient dilution was performed for the compounds in the microplate to achieve a final concentration of 0-10 $\mu$M. Then, 2.5 $\mu$L each of tubulin, compound, ATP, and KIF18A protein were added to the microplate in sequence for reaction at ambient temperature for 120 min. The final concentrations in the enzyme reaction were 60 $\mu$g/mL for microtubules, 25 $\mu$M for ATP, and 2.5 nM for KIF18A protein. After the enzyme reaction, 10 $\mu$L of ADP-GLO reaction reagent was added to each well and incubated at room temperature for 30 min. After that, 20 $\mu$L of detection reagent was added to each well and incubated at room temperature for 30 min in the dark. Finally, chemiluminescence detection was performed using the PerkinElmer Envision.

| Example | KIF18A $IC_{50}$ nM | Example | KIF18A $IC_{50}$ nM | Example | KIF18A $IC_{50}$ nM |
|---|---|---|---|---|---|
| Example 1A | >3000 | Example 42 | 167 | Example 66 | 43 |
| Example 1B | 220 | Example 43 | NT | Example 67 | 86 |
| Example 1C | 1062 | Example 44 | 83 | Example 68 | 155 |
| Example 1D | 211 | Example 45 | 114 | Example 69 | 45 |
| Example 2A | 35 | Example 46A | 117 | Example 70 | 251 |
| Example 2B | 3549 | Example 46B | 57 | Example 81A | 219 |
| Example 2C | 43 | Example 47 | 566 | Example 81B | 491 |
| Example 2D | 546 | Example 47 | 625 | Example 81C | 521 |

(continued)

| Example | KIF18A $IC_{50}$ nM | Example | KIF18A $IC_{50}$ nM | Example | KIF18A $IC_{50}$ nM |
|---|---|---|---|---|---|
| Example 3 | 566 | Example 49 | 187 | Example 81D | 132 |
| Example 4 | 404 | Example 50 | NT | Example 82 | 1023 |
| Example 5 | 162 | Example 51A | 107 | Example 38 | 113 |
| Example 6 | 73 | Example 51B | 144 | Example 84A | 309 |
| Example 7 | 51 | Example 52 | 555 | Example 84B | 258 |
| Example 8 | 48 | Example 53 | 49 | Example 84C | 186 |
| Example 9 | 264 | Example 54 | 1560 | Example 84D | 284 |
| Example 10 | 43 | Example 55 | 88 | Example 85 | 350 |
| Example 11 | 76 | Example 56A | 41 | Example 86 | 352 |
| Example 12 | 172 | Example 56B | 9 | Example 87 | 350 |
| Example 13 | 294 | Example 57A | 60 | Example 88 | >3000 |
| Example 14A | 44 | Example 57 | 52 | Example 89 | 1703 |
| Example 14B | 307 | Example 58 | 67 | Example 90 | 274 |
| Example 15 | 358 | Example 59 | 223 | Example 91 | 37 |
| Example 16 | 45 | Example 60 | 40 | Example 92A | 427 |
| Example 17 | 52 | Example 61 | 60 | Example 92B | 405 |
| Example 18 | 67 | Example 62 | 35 | Example 93 | 45 |
| Example 19 | 73 | Example 63 | 126 | | |
| Example 20 | 410 | Example 64 | 97 | | |
| Example 21 | 450 | Example 65 | 135 | | |

**Assay 2: Cell Proliferation Activity Test**

**Materials and Cells:** OVCAR3 cells were purchased from Nanjing Cobioer Biosciences Co., Ltd. RPMI-1640 medium, fetal bovine serum, and the CyQUANT Direct Cell Proliferation Assay kit were purchased from Thermo Fisher Scientific, USA; 96-well cell culture plates were sourced from Corning, USA.

**Cell Culture:** OVCAR3 cells were cultured with RPMI-1640 medium containing 10% fetal bovine serum and incubated in a 5% $CO_2$ incubator at 37°C. Only cells in the logarithmic growth phase could be used for the test.

**Cell Proliferation Activity Test:**

OVCAR3 cells were inoculated into a 96-well cell culture plate at 90 μL per well and incubated overnight in a 5% $CO_2$ incubator at 37°C. The compound powder was dissolved in DMSO to prepare a stock solution of 10 mM. Then gradient dilution was performed for the compounds in the microplate to achieve a final concentration of 0-10 μM. 10 μL of cell culture medium containing compounds was added to each well to make the final DMSO content 0.2%. The cell plates were incubated in a 5% $CO_2$ incubator at 37°C for 3 days. 100 μL of CyQUANT detection reagent was added to each well, reacted at 37°C for 60 min, and fluorescence detection was performed using the PerkinElmer Envision.

| Example | OVCAR3 $IC_{50}$ nM | Example | OVCAR3 $IC_{50}$ nM | Example | OVCAR3 $IC_{50}$ nM |
|---|---|---|---|---|---|
| Example 1A | 2790 | Example 42 | 136 | Example 84A | 26 |
| Example 1B | 22 | Example 43 | 2581 | Example 84B | 472 |
| Example 1C | 1020 | Example 44 | 27 | Example 84C | 38 |
| Example 1D | 137 | Example 45 | 77 | Example 84D | >3000 |
| Example 2A | 8 | Example 46A | 36 | Example 85 | 60 |

(continued)

| Example | OVCAR3 $IC_{50}$ nM | Example | OVCAR3 $IC_{50}$ nM | Example | OVCAR3 $IC_{50}$ nM |
|---|---|---|---|---|---|
| Example 2B | 639 | Example 47B | 26 | Example 86 | 63 |
| Example 2C | 25 | Example 67 | 481 | Example 87 | 60 |
| Example 2D | 679 | Example 48 | 1208 | Example 88 | >3000 |
| Example 3 | 1134 | Example 49 | 1082 | Example 89 | 171 |
| Example 4 | 49 | Example 50 | NT | Example 90 | 136 |
| Example 5 | 42 | Example 51A | 45 | Example 91 | >3000 |
| Example 6 | 41 | Example 51B | 72 | Example 92A | 141 |
| Example 7 | 41 | Example 52 | >3,000 | Example 92B | 173 |
| Example 8 | 37 | Example 53 | 46 | Example 93 | 91 |
| Example 9 | 1056 | Example 54 | 867 | | |
| Example 10 | 36 | Example 55 | 30 | | |
| Example 11 | 25 | Example 56A | 188 | | |
| Example 12 | 96 | Example 56B | 112 | | |
| Example 13 | 181 | Example 57A | 114 | | |
| Example 14A | 635 | Example 57 | 119 | | |
| Example 14B | >3000 | Example 58 | 116 | | |
| Example 15 | 24 | Example 81 | 34 | | |
| Example 16 | 89 | Example 81A | 22 | | |
| Example 17 | 96 | Example 81B | 178 | | |
| Example 18 | 38 | Example 81C | 500 | | |
| Example 19 | 52 | Example 81D | 31 | | |
| Example 20 | 57 | Example 82 | 1279 | | |
| Example 21 | 78 | Example 83 | 100 | | |

**Assay 3:** CyQuant **Cell Proliferation Activity Test**

**Materials and Cells:** HT29 cells were purchased from Nanjing Cobioer Biosciences Co., Ltd.; RPMI-1640 medium, fetal bovine serum, Trypsin-EDTA (0.25%), 96-well plates, and CyQuant reagents were purchased from ThermoFisher (USA); DMSO was purchased from SIGMA (USA).

**Cell Culture:** HT29 cells were cultured with RPMI-1640 containing 10% fetal bovine serum under the condition of 37°C and 5% $CO_2$. Only cells in the logarithmic growth phase could be used for the test.

**Cell Proliferation Activity Test:** CyQuant reagent was used to detect the proliferation inhibitory activity of compounds on HT29 cells. The cell density was adjusted, and 100 μL per well was inoculated into a 96-well plate (HT29 at 2000 cells per well), then incubated overnight at 37°C and 5% $CO_2$. Add compounds of various target concentrations (initial concentration of 3000 nM, 3-fold dilution, 9 concentration gradients), and make the DMSO content 0.2%. The cell plate was incubated at 37°C in 5% $CO_2$ for 3 days. CyQuant reagent was added and incubated for 1 h. The plates were read by Envision, and $IC_{50}$ was calculated using XLFIT.

| Example | HT-29 $IC_{50}$ nM | Example | HT-29 $IC_{50}$ nM | Example | HT-29 $IC_{50}$ nM |
|---|---|---|---|---|---|
| Example 1A | 2790 | Example 42 | 162 | Example 84A | NT |
| Example 1B | 22 | Example 43 | NT | Example 84B | NT |
| Example 1C | 1020 | Example 44 | 39 | Example 84C | NT |
| Example 1D | 137 | Example 45 | 100 | Example 84D | NT |

(continued)

| Example | HT-29 $IC_{50}$ nM | Example | HT-29 $IC_{50}$ nM | Example | HT-29 $IC_{50}$ nM |
|---|---|---|---|---|---|
| Example 2A | 9 | Example 46A | 58 | Example 85 | 76 |
| Example 2B | 639 | Example 46B | 36 | Example 86 | NT |
| Example 2C | 25 | Example 47 | NT | Example 87 | 76 |
| Example 2D | 679 | Example 48 | 1560 | Example 88 | >3000 |
| Example 3 | NT | Example 49 | 1310 | Example 89 | 173 |
| Example 4 | 55 | Example 50 | NT | Example 90 | 333 |
| Example 5 | 42 | Example 51A | 39 | Example 91 | >3000 |
| Example 6 | 41 | Example 51B | 74 | Example 92A | 154 |
| Example 7 | 26 | Example 52 | >3000 | Example 92B | 164 |
| Example 8 | 43 | Example 53 | NT | Example 93 | 194 |
| Example 9 | 1016 | Example 54 | >3,000 | | |
| Example 10 | 39 | Example 55 | 29 | | |
| Example 11 | 33 | Example 56A | 378 | | |
| Example 12 | 165 | Example 56B | 192 | | |
| Example 13 | 187 | Example 57A | 39 | | |
| Example 14A | 837 | Example 57 | 22 | | |
| Example 14B | >3000 | Example 58 | 42 | | |
| Example 15 | 46 | Example 81 | NT | | |
| Example 16 | 95 | Example 81A | 23 | | |
| Example 17 | 105 | Example 81B | 254 | | |
| Example 18 | 41 | Example 81C | 671 | | |
| Example 19 | 48 | Example 81D | 29 | | |
| Example 20 | 69 | Example 82 | NT | | |
| Example 21 | 85 | Example 83 | NT | | |

**Assay 4:** Potential Cytotoxicity Study of Compounds on Human Primary Hepatocytes

**Materials and Cells:** Primary hepatocyte donor: Caucasian (BiolVT); CellTiter-Glo Luminescent Cell Viability Assay System/Promega (Madison, WI); 96-well plate/Corning; centrifuge/Eppendorf; $CO_2$ incubator/Thermo Scientific; cell counter/Nexcelom Bioscience; oscillator/IKA; microplate reader/TECAN

**EXPERIMENTAL DESIGN**

1) The following media were heated to 37°C in a water bath:

The hepatocyte recovery medium, inoculation medium and incubation medium were prepared according to the table below.

Recovery Medium

| Reagent | Initial Concentration | Final Concentration | Volume |
|---|---|---|---|
| Williams' Medium E | - | - | 31.2 mL |

(continued)

| Reagent | Initial Concentration | Final Concentration | Volume |
| --- | --- | --- | --- |
| Isotonic Percoll | - | 30% | 13.5 mL |
| DPBS (10×) | - | - | 1.5 mL |
| GlutaMAX™-1(100×) | 200 mM/100× | 2 mM | 500 μL |
| HEPES | 1 M | 15 mM | 750 μL |
| FBS | - | 5% | 2.5 mL |
| Human recombinant insulin | 4 mg/mL | 4 μg/mL | 50 μL |
| Dexamethasone | 10 mM | 1 μM | 5 μL |

Inoculation Medium

| Reagent | Initial Concentration | Final Concentration | Volume |
| --- | --- | --- | --- |
| Williams' Medium E | - | - | 45.7 mL |
| FBS | - | 5% | 2.5 mL |
| Dexamethasone | 10 mM | 1 μM | 5 μL |
| Penicillin-streptomycin mixed solution | Penicillin: 10,000 u/mL<br>Streptomycin: 10,000 μg/mL | Penicillin: 100 u/mL<br>Streptomycin: 100 μg/mL | 500 μL |
| Human recombinant insulin | 4 mg/mL | 4 μg/mL | 50 μL |
| GlutaMAX™-1(100×) | 200 mM/100× | 2 mM | 500 μL |
| HEPES | 1 M | 15 mM | 750 μL |

Incubation Medium

| Reagent | Initial Concentration | Final Concentration | Volume |
| --- | --- | --- | --- |
| Williams' Medium E | - | - | 48 mL |
| Dexamethasone | 10mM | 0.1 μM | 0.5 μL |
| ITS (100×) | - | - | 500 μL |
| Penicillin-streptomycin mixed solution | Penicillin: 10,000 u/mL<br>Streptomycin: 10,000 μg/mL | Penicillin: 50 u/mL<br>Streptomycin: 50 μg/mL | 250 μL |
| GlutaMAX™-1(100×) | 200 mM/100× | 2 mM | 500 μL |
| HEPES | 1 M | 15 mM | 750 μL |

**2)** One tube of cryopreserved hepatocytes was taken, ensuring the hepatocytes were cryogenically frozen until recovery. The hepatocytes were quickly placed in a 37°C water bath and gently shaken until all ice crystals were completely dissolved. After spraying with 75% ethanol, the tube was transferred to a biosafety cabinet.

**3)** The contents of the hepatocyte tube were poured (1 mL, approximately $5 \times 10^6$ cells) into a 50 mL centrifuge tube containing 50 mL of recovery medium and centrifuged at 100 g for 10 min. After centrifugation, the recovery medium was aspirated, and sufficient inoculation medium was added to obtain a cell suspension with a cell density of approximately $1.0 \times 10^6$ cells/mL. The hepatocytes were counted and viable cell density was determined using a Cellometer® cell counter; the hepatocyte viability was required to be greater than 80%. The cell density was adjusted to $0.2 \times 10^6$ cells/mL with inoculation medium, and the cells were inoculated into collagen I-coated 96-well plates at 100 μL per well. The plates were incubated at 37°C for 4-6 h in a 5% $CO_2$ incubator with 95% relative humidity.

**4)** A 200X stock solution of the test compound was prepared in DMSO (the concentration of the stock solution of the test compound could be reduced based on solubility). The final DMSO concentration was 0.5%.

**5)** The working solutions were prepared on sterile 96-well plates by adding 2.5 μL of the test compound stock solution to 497.5 μL of hepatocyte culture medium.

**6)** The hepatocyte culture medium was removed from the cell plate, and 125 μL of the working solution was added to the corresponding wells in triplicate. The plate was incubated at 37°C and 5% $CO_2$ for 72 h. After every 24 h of treatment, replace the medium in cell plates with freshly diluted test and positive control compounds from hepatocyte culture media. The cell plate was put back into the incubator.

**1.1** A total of 50 μL of CellTiter-Glo reagent was added into each well of the 96-well plate. The plate was shaken on a plate shaker at room temperature for 10 min. After 10 min, 100 μL of the above incubation mixture was transferred to a new white flat-bottom opaque 96-well plate, and the fluorescence was recorded.

**Data Analysis:** All calculations were performed using Microsoft Excel.

The viability of the test compound could be calculated using the following formula:

$$\%\,\text{Vehicle} = \frac{\text{Read}_{\text{Compound}} - \text{Read}_{\text{Blank}}}{\text{Read}_{\text{Vehicle}} - \text{Read}_{\text{Blank}}} \times 100\%$$

The % Vehicle was fitted against the concentration of the test compound, and the data were fitted to a sigmoidal dose-response curve with a variable slope using GraphPad Prism 5.0. The $IC_{50}$ of this compound was calculated from the curve using the following formula:

$$Y=\text{Bottom} + (\text{Top-Bottom})/(1+10\verb|^|((\text{LogIC50}-X)*\text{HillSlope}))$$

## Claims

**1.** A compound having the structure of the following formula, or pharmaceutically acceptable salts, stereoisomers, isotope isomers or tautomers thereof:

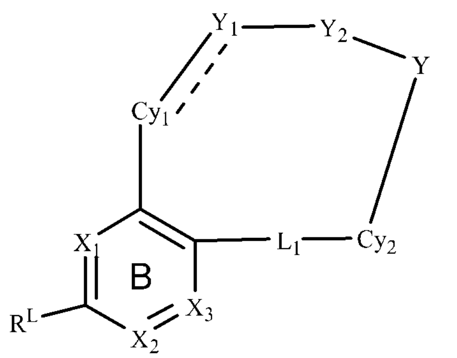

Formula (I)

wherein, $C_{y2}$ represents

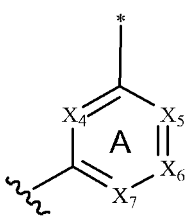 or 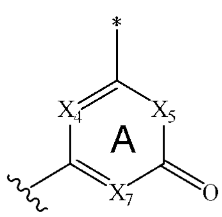 or 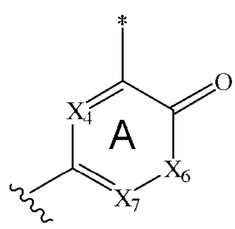 or 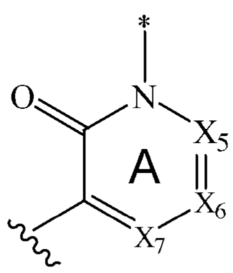

or

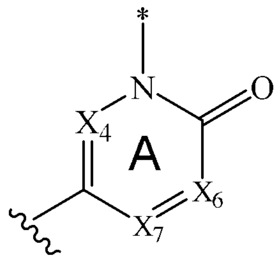

;

wherein, * represents the site of connection with Y, and the wavy line represents the site of connection with $L_1$;
wherein, - - - represents a single bond or double bond;
wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1-C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1, 2 or 3 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1-C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1, 2 or 3 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1-C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^aR^b)$, $-C_0$-$C_{10}$ alkyl $P(R^a R^b)$ or $-C_0$-$C_{10}$ alkyl $NR^aR^b$;
wherein, $L_1$ represents $-C(O)NR^a-$, $-NR^aC(O)-$ or a 5-6-membered heteroaromatic ring;
wherein, Y represents absence, $-CH_2-$, -O-, $-NR^a-$, $-C(O)NR^a$, $-NR^aC(O)-$ or the following groups:

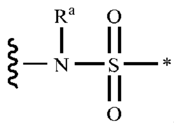 , 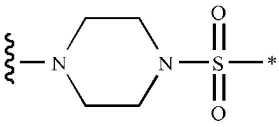

or $C_{y3}$;
wherein, * represents the site of connection with ring A; the wavy line represents the site of connection with $Y_2$;
wherein, $C_{y1}$ and $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein substituents are each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$ -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)$OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;
wherein, $Y_1$ represents -$(CR^aR^b)_m$-; $Y_2$ represents -$(CR^aR^b)_n$-;
wherein, optionally -$CR^aR^b$- may be substituted with -O-, -S-, -Se-, -$NR^a$-, -$NR^aSO_2$-, -$NR^a$C(=O)-, -C(=O)$NR^a$-, -OC(=O)-, -C(=O)O-, -$SO_2NR^a$-, -S(=O)($NR^a$)-, -P(O)$(OR^a)_2$-, PO($R^a$ $R^b$), -$NR^a$P(O)$(OR^a)_2$- or -$NR^a$P(O)$(R^a)_2$-, -$CR^a$=$CR^b$-,

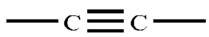 , 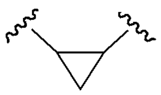 , 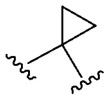 , 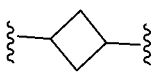 , 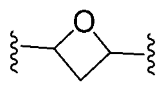 , 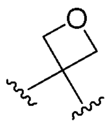 ,

5-6-membered heteroaryl,

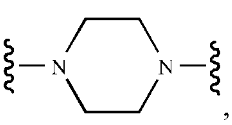 , 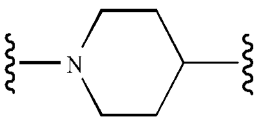 or 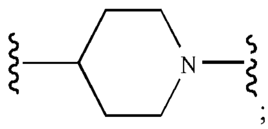 ;

wherein, $R^L$ represents $L_2$-$R^M$;
wherein, $L_2$ represents absence, -$C_1$-$C_6$ alkyl-, -$NR^a$-, -$NR^aSO_2$-, -$SO_2NR^a$-, -$NR^a$S(=O)(=NH)-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)_2$, -$NR^a$P(O) $(OR^a)_2$ or -$NR^a$P(O)$(R^a)_2$-;
wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;
alternatively, $L_2$-$R^M$ represents

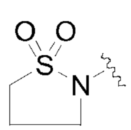 or 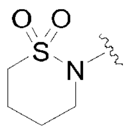 ;

wherein, m and n each independently represent an integer from 0 to 10;
wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$,

together with the atom attached thereto, form a 3-6-membered saturated or unsaturated ring, or a 3-6-membered saturated or unsaturated heterocyclic ring, which may optionally contain 0-2 heteroatoms selected from O, S, N and Se.

**2.** A compound having the structure of Formula (I-1), or pharmaceutically acceptable salts, stereoisomers, isotope isomers or tautomers thereof:

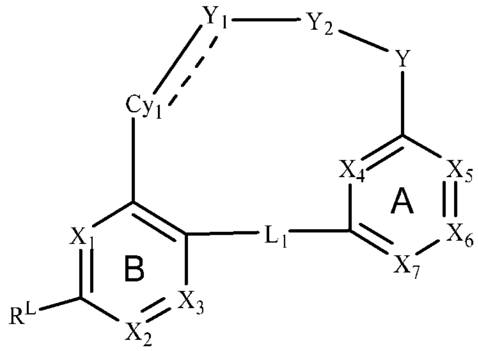

Formula (I-1)

wherein, - - - represents a single bond or double bond;
wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1, 2 or 3 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1, 2 or 3 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$,

$-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR^aC(O)OR^b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;
wherein, $L_1$ represents $-C(O)NR^a-$, $-NR^aC(O)-$ or a 5-6-membered heteroaromatic ring;
wherein, Y represents absence, $-CH_2-$, -O-, $-NR^a-$, $-C(O)NR^a$, $-NR^aC(O)-$ or the following groups:

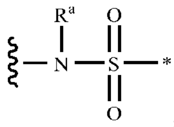, 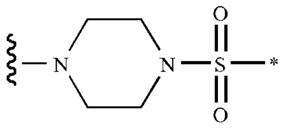

or $C_{y3}$;
wherein, * represents the site of connection with ring A; the wavy line represents the site of connection with $Y_2$;
wherein, $C_{y1}$ and $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein substituents are each independently selected from the following: hydrogen, deuterium, halogen, cyano, nitro, azido, hydroxy $(C_1-C_{10})$ alkyl, $C_1-C_{10}$ alkyl, $C_2-C_{10}$ alkenyl, $C_2-C_{10}$ alkynyl, $C_1-C_{10}$ deuterated alkyl, $C_3-C_{12}$ cycloalkyl, $C_1-C_{10}$ haloalkyl, $C_2-C_{10}$ haloalkenyl, $C_2-C_{10}$ haloalkynyl, $C_3-C_{12}$ halocycloalkyl, 3-12-membered heterocyclyl, $C_6-C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR^aC(O)OR^b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;
wherein, $Y_1$ represents $-(CR^aR^b)_m-$; $Y_2$ represents $-(CR^aR^b)_n-$;
wherein, optionally $-CR^aR^b-$ may be substituted with -O-, -S-, -Se-, $-NR^a-$, $-NR^aSO_2-$, $-NR^aC(=O)-$, $-C(=O)NR^a-$, -OC(=O)-, -C(=O)O-, $-SO_2NR^a-$, $-S(=O)(NR^a)-$, $-P(O)(OR^a)_2-$, $-NR^aP(O)(OR^a)_2-$ or $-NR^aP(O)(R^a)_2-$, $-CR^a=CR^b-$,

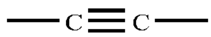,

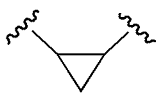, 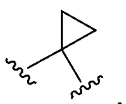, 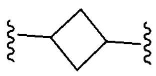, 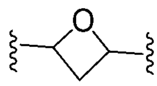, 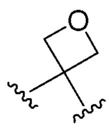,

5-6-membered heteroaryl,

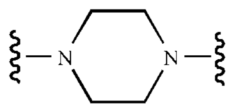,

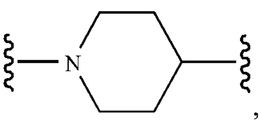, or 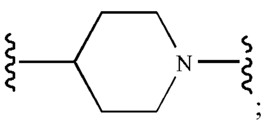;

wherein, $R^L$ represents $L_2-R^M$;
wherein, $L_2$ represents absence, $-C_1-C_6$ alkyl-, $-NR^a-$, $-NR^aSO_2-$, $-SO_2NR^a-$, $-NR^aS(=O)(=NH)-$, -S(=O) (=NH)-, -S-, -S(=O)-, $-SO_2-$, $-C_1-C_6$ alkyl-O-, -(C=O)-, $-(C=O)NR^a-$, -C=N(OH)-, $-NR^a$ (C=O), $-P(O)(OR^a)_2$, $-NR^aP(O)(OR^a)_2$ or $-NR^aP(O)(R^a)_2-$;

wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, $-OR^a$, $-NR^aR^b$, cyano and $-O-C_1$-$C_6$ haloalkyl;
alternatively, $L_2$-$R^M$ represents

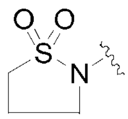 or 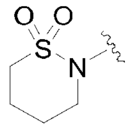 ;

wherein, m and n each independently represent an integer from 0 to 10;
wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, N and Se.

3. A compound according to any one of claim 1 or 2, its pharmaceutically acceptable salt, stereoisomer, isotopic isomer or tautomeric form thereof, wherein $X_1$ represents CH or N, preferably CH.

4. A compound according to any one of claim 1 or 2, its pharmaceutically acceptable salt, stereoisomer, isotopic isomer or tautomeric form thereof, wherein $X_2$ is CH or N, preferably CH.

5. A compound according to any one of claim 1 or 2, its pharmaceutically acceptable salt, stereoisomer, isotopic isomer or tautomeric form thereof, wherein $X_3$ is CH or N, preferably CH.

6. A compound according to any one of claim 1 or 2, its pharmaceutically acceptable salt, stereoisomer, isotopic isomer or tautomeric form thereof, wherein the chemical bond between $X_2$ and $X_3$ is fused with ring B to form a group in the following structure:

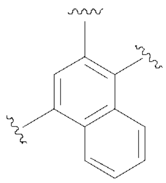 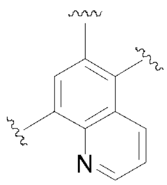 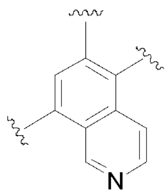 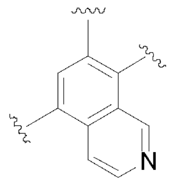 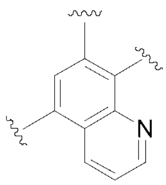 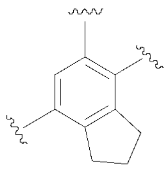

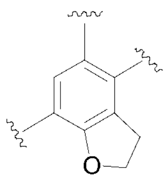 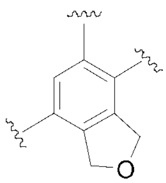 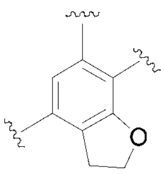 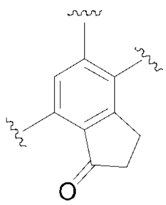 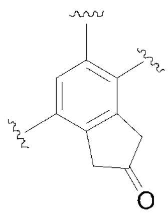 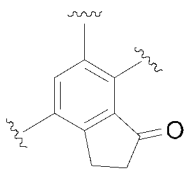

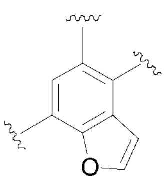 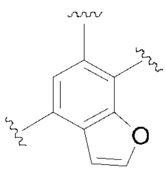 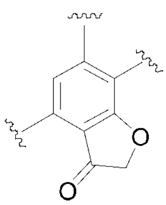 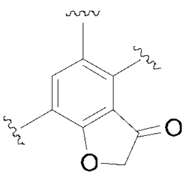

7. A compound according to any one of claim 1 or 2, its pharmaceutically acceptable salt, stereoisomer, isotopic isomer or tautomeric form thereof, wherein $X_4$ is CH or N, preferably N.

8. A compound according to any one of claim 1 or 2, its pharmaceutically acceptable salt, stereoisomer, isotopic isomer or tautomeric form thereof, wherein $X_5$ is $CR^{W5}$ or N, and $R^{W5}$ is hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

9. A compound according to any one of claim 1 or 2, its pharmaceutically acceptable salt, stereoisomer, isotopic isomer or tautomeric form thereof, wherein $X_6$ is $CR^{W6}$ or N, and $R^{W6}$ is hydrogen, halogen, $-CH_3O$, $-CF_3$, $-CHF_2$, $-CH_2F$, $-NH_2$, $-N(CH_3)_2$, CN, -OH, $-C(O)CH_3$, $-OC(O)CH_3$, $-C(O)OCH_3$ or $-C(O)NH_2$.

10. A compound according to any one of claim 1 or 2, its pharmaceutically acceptable salt, stereoisomer, isotopic isomer or tautomeric form thereof, wherein $X_7$ is CH or N, preferably N.

11. A compound according to any one of claim 1 or 2, its pharmaceutically acceptable salt, stereoisomer, isotopic isomer or tautomeric form thereof, wherein the chemical bond between $X_5$ and $X_6$ is fused with ring A to form a group in the following structure:

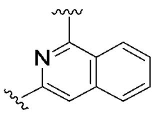 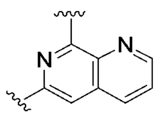 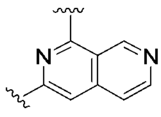 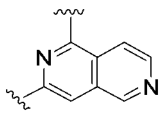 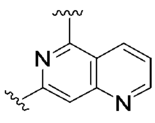 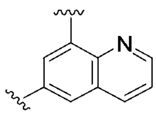

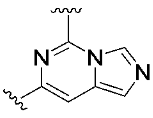 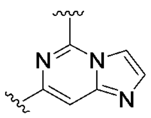 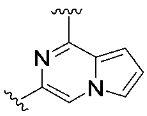 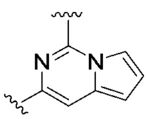 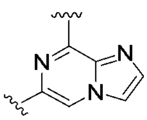 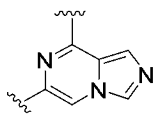 .

12. A compound according to any one of claim 1 or 2, its pharmaceutically acceptable salt, stereoisomer, isotopic isomer or tautomeric form thereof, wherein the chemical bond between $X_6$ and $X_7$ is fused with ring A to form a group in the following structure:

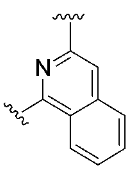 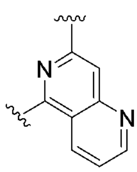 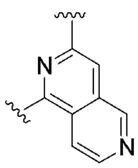 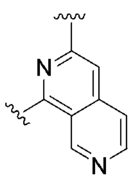 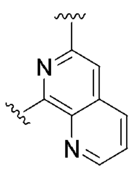 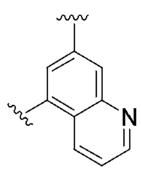

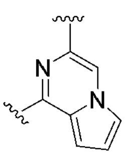 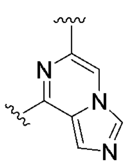 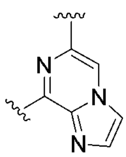 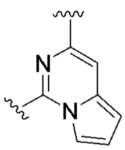 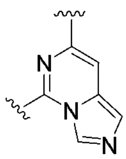 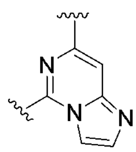 .

13. A compound according to any one of claim 1 or 2, its pharmaceutically acceptable salt, stereoisomer, isotopic isomer or tautomeric form thereof, wherein $C_{y1}$ is the following group:

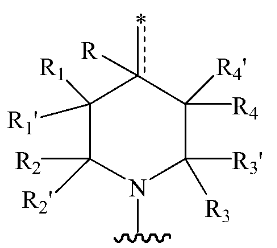 , 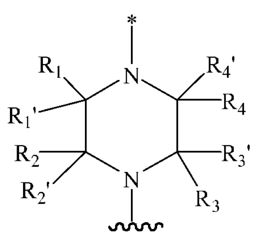 , 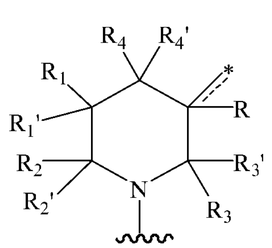 , 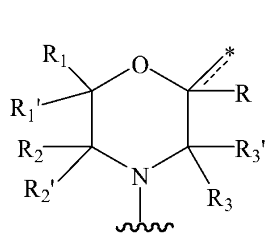 ,

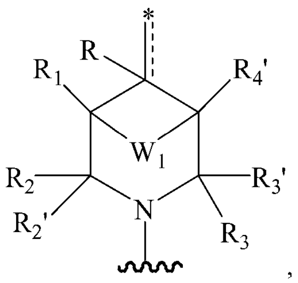 , 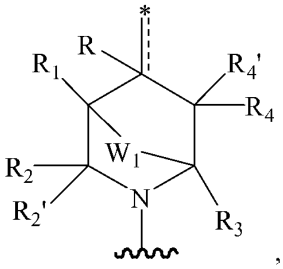 , 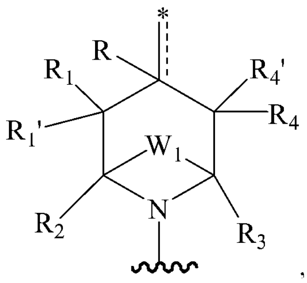 , 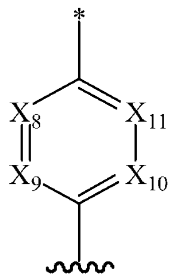 or 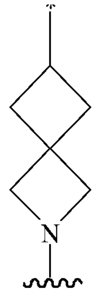

wherein, when $\overline{---}$ represents a double bond, R represents absence; wherein, when $\overline{---}$ represents a single bond, R represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;
wherein, $R_1$, $R_1$', $R_2$, $R_2$', $R_3$, $R_3$', $R_4$ and $R_4$' each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;
alternatively, pairs $R_1$ and $R_1$', pairs $R_2$ and $R_2$', pairs $R_3$ and $R_3$' and pairs $R_4$ and $R_4$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, -$OR^a$ or oxo;
wherein, $W_1$ represents -$(CR^sR^t)_o$-;
wherein, $R^s$ and $R^t$ each independently represent hydrogen or $C_1$-$C_6$ alkyl;
wherein, o represents 1 or 2;
wherein, $X_8$ represents $CR^{W8}$ or N;
wherein, $X_9$ represents $CR^{W9}$ or N;
wherein, $X_{10}$ represents $CR^{W10}$ or N;
wherein, $X_{11}$ represents $CR^{W11}$ or N;
wherein, $R^{W8}$, $R^{W9}$, $R^{W10}$ and $R^{W11}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^aC(O)R^b$ -C(O)$NR^aR^b$, -$NR^aC(O)OR^b$, -OC(O)$NR^aR^b$, -OC(O)$OR^a$, -$SF_5$, -PO($R^a$ $R^b$), -P($R^aR^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^aC(O)R^b$, -$C_0$-$C_{10}$ alkyl C(O)$NR^aR^b$, -$C_0$-$C_{10}$ alkyl $NR_aC(O)OR_b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$.

**14.** A compound according to claim 13, its pharmaceutically acceptable salt, stereoisomer, isotopic isomer or tautomeric form thereof, wherein $X_8$ is CH or N.

**15.** A compound according to claim 13, its pharmaceutically acceptable salt, stereoisomer, isotopic isomer or tautomeric form thereof, wherein $X_9$ is CH or N.

**16.** A compound according to claim 13, its pharmaceutically acceptable salt, stereoisomer, isotopic isomer or tautomeric form thereof, wherein $X_{10}$ is CH or N.

**17.** A compound according to claim 13, its pharmaceutically acceptable salt, stereoisomer, isotopic isomer or tautomeric form thereof, wherein $X_{11}$ is CH or N.

**18.** A compound according to claim 13, its pharmaceutically acceptable salt, stereoisomer, isotopic isomer or tautomeric form thereof, wherein $R_1$, $R_1$', R2, $R_2$', $R_3$, $R_3$', $R_4$ or $R_4$' is hydrogen, respectively.

**19.** A compound according to claim 13, its pharmaceutically acceptable salt, stereoisomer, isotopic isomer or tautomeric form thereof, wherein $W_1$ is -$CH_2$ or -$CH_2CH_2$.

**20.** A compound according to any one of claim 1 or 2, its pharmaceutically acceptable salt, stereoisomer, isotopic isomer or tautomeric form thereof, wherein Y represents the following groups: absent, -$CH_2$-, -O-, -Se-, -$NR^a$-, -C(O)$NR^a$,

-NRᵃC(O)-or the following groups:

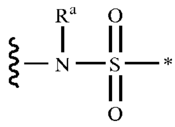, 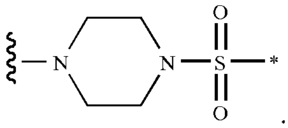.

**21.** A compound according to any one of claim 1 or 2, its pharmaceutically acceptable salt, stereoisomer, isotopic isomer or tautomeric form thereof, wherein Y is -O- or -NH-.

**22.** A compound according to any one of claim 1 or 2, its pharmaceutically acceptable salt, stereoisomer, isotopic isomer or tautomeric form thereof, wherein Y is

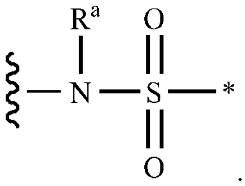.

**23.** A compound according to any one of claim 1 or 2, its pharmaceutically acceptable salt, stereoisomer, isotopic isomer or tautomeric form thereof, wherein Y is

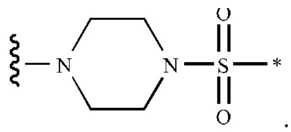.

**24.** A compound according to any one of claim 1 or 2, its pharmaceutically acceptable salt, stereoisomer, isotopic isomer or tautomeric form thereof, wherein Y is $C_{y3}$ and $C_{y3}$ represents the following group:

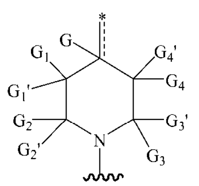, 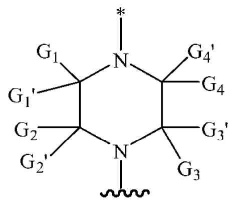, 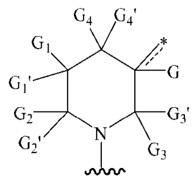, 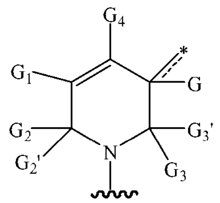, 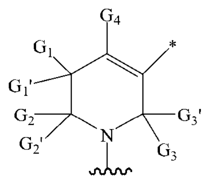,

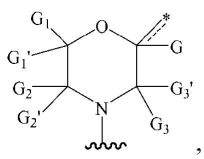, 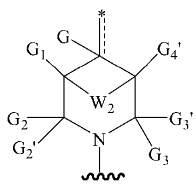, 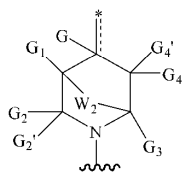, 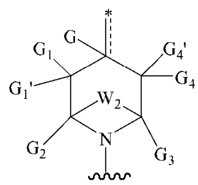, 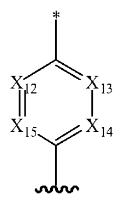, 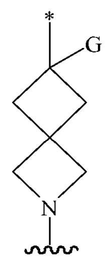, 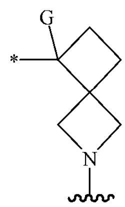,

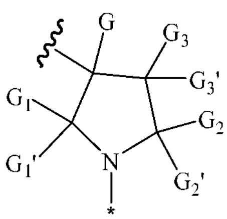, 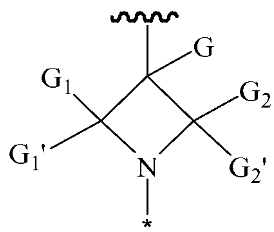;

wherein, when $\overline{---}$ represents a double bond, G represents absence; wherein, when $\overline{---}$ represents a single bond, G represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;
wherein, $G_1$, $G_1'$, $G_2$, $G_2'$, $G_3$, $G_3'$, $G_4$ and $G_4'$ each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;
alternatively, pairs $G_1$ and $G_1'$, pairs $G_2$ and $G_2'$, pairs $G_3$ and $G_3'$ and pairs $G_4$ and $G_4'$, together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $OR^a$ or oxo;
wherein, $W_2$ represents -$(CR^iR^j)_p$-;
wherein, $R^i$ and $R^j$ each independently represent hydrogen or $C_1$-$C_6$ alkyl;
wherein, p represents 1 or 2;
wherein, $X_{12}$ represents $CR^{W12}$ or N;
wherein, $X_{13}$ represents $CR^{W13}$ or N;
wherein, $X_{14}$ represents $CR^{W14}$ or N;
wherein, $X_{15}$ represents $CR^{W15}$ or N;
wherein, $R^{W12}$, $R^{W13}$, $R^{W14}$ and $R^{W15}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$ -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR_a$C(O)$OR_b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;

**25.** A compound according to claim 24, its pharmaceutically acceptable salt, stereoisomer, isotopic isomer or tautomeric form thereof, wherein $X_{12}$ is CH or N.

**26.** A compound according to claim 24, its pharmaceutically acceptable salt, stereoisomer, isotopic isomer or tautomeric form thereof, wherein $X_{13}$ is CH or N.

**27.** A compound according to claim 24, its pharmaceutically acceptable salt, stereoisomer, isotopic isomer or tautomeric form thereof, wherein $X_{14}$ is CH or N.

**28.** A compound according to claim 24, its pharmaceutically acceptable salt, stereoisomer, isotopic isomer or tautomeric form thereof, wherein $X_{15}$ is CH or N.

**29.** A compound according to claim 24, its pharmaceutically acceptable salt, stereoisomer, isotopic isomer or tautomeric form thereof, wherein $G_1$, $G_1'$, $G_2$, $G_2'$, $G_3$, $G_3'$, $G_4$ or $G_4'$ is hydrogen, respectively.

**30.** A compound according to claim 24, its pharmaceutically acceptable salt, stereoisomer, isotopic isomer or tautomeric form thereof, wherein $W_2$ is -$CH_2$ or -$CH_2CH_2$.

**31.** A compound according to any one of claim 1 or 2, its pharmaceutically acceptable salt, stereoisomer, isotopic isomer or tautomeric form thereof,
$L_1$ represents -C(O)$NR^a$-, -$NR^a$C(O)-,

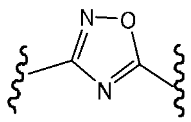, 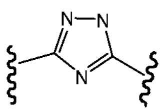, 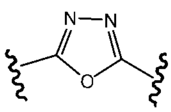, 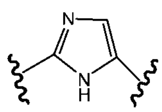, 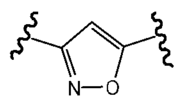,

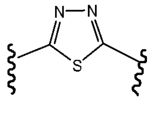, 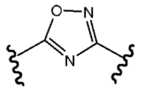, 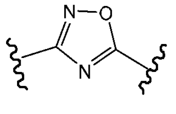, 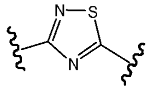, 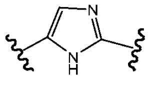, 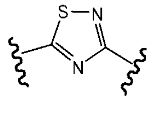, 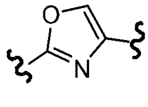,

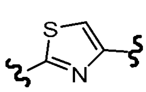, 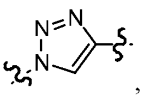, 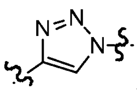, 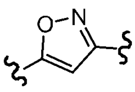, 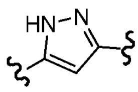, 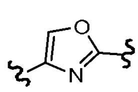, 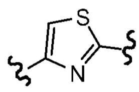, 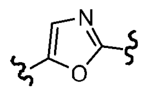,

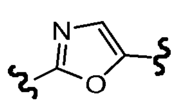, 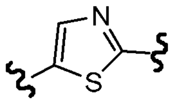 or 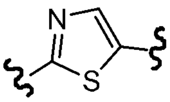.

**32.** A compound according to claim 31, its pharmaceutically acceptable salt, stereoisomer, isotopic isomer or tautomeric form thereof, wherein $L_1$ is -C(O)NH-.

**33.** A compound according to claim 31, its pharmaceutically acceptable salt, stereoisomer, isotopic isomer or tautomeric form thereof, wherein $L_1$ is

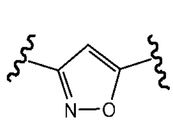, 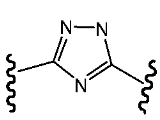, 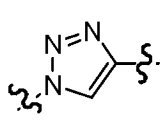, 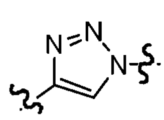, 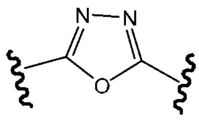

or

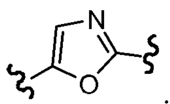.

**34.** A compound according to any one of claim 1 or 2, its pharmaceutically acceptable salt, stereoisomer, isotopic isomer or tautomeric form thereof, wherein $Y_1$ is $-(CR^aR^b)_m-$.

**35.** A compound according to any one of claim 1 or 2, its pharmaceutically acceptable salt, stereoisomer, isotopic isomer or tautomeric form thereof, wherein $Y_1$ is -NR-C(=O)-, -O-C(=O)- or -C(=O)O-.

**36.** A compound according to any one of claim 1 or 2, its pharmaceutically acceptable salt, stereoisomer, isotopic isomer or tautomeric form thereof, wherein $Y_1$ is $-O(CR^aR^b)_m-$ or $-(CR^aR^b)_mO-$.

**37.** A compound according to any one of claim 1 or 2, its pharmaceutically acceptable salt, stereoisomer, isotopic isomer or tautomeric form thereof, wherein $Y_1$ is $-CR^a=CR^a-$ or $-CR^aR^b-CR^a=CR^b-$.

**38.** A compound according to any one of claim 1 or 2, its pharmaceutically acceptable salt, stereoisomer, isotopic isomer or tautomeric form thereof, wherein $Y_2$ is $-(CR^aR^b)_n-$.

**39.** A compound according to any one of claim 1 or 2, its pharmaceutically acceptable salt, stereoisomer, isotopic isomer or tautomeric form thereof, wherein $Y_2$ is $-NR^a-C(=O)-$.

**40.** A compound according to any one of claim 1 or 2, its pharmaceutically acceptable salt, stereoisomer, isotopic isomer or tautomeric form thereof, wherein $Y_2$ is $-O(CR^aR^b)_n-$ or $-(CR^aR^b)_nO-$.

**41.** A compound according to any one of claim 1 or 2, its pharmaceutically acceptable salt, stereoisomer, isotopic isomer or tautomeric form thereof, wherein $Y_2$ is $-CR^a=CR^a-$, $-CR^a=CR^b-CR^aR^b-$ or $-CR^aR^b-CR^a=CR^b-$.

**42.** A compound according to any one of claim 1 or 2, its pharmaceutically acceptable salt, stereoisomer, isotopic isomer or tautomeric form thereof, wherein $Y_2$ is pyrazolyl, oxazolyl, oxadiazolyl, thiazolyl, thiadiazolyl or triazolyl.

**43.** A compound according to any one of claim 1 or 2, its pharmaceutically acceptable salt, stereoisomer, isotopic isomer or tautomeric form thereof, wherein $-Y_1-Y_2-$ is $-CR^a=CR^a-$ or $-CR^aR^bCR^a=CR^b-$.

**44.** A compound according to claim 1 or 2, its pharmaceutically acceptable salt, stereoisomer, isotopic isomer or

tautomeric form thereof, wherein $-Y_1-Y_2-$ is $-CR^aR^b-NR^aC(O)-$.

**45.** A compound according to any one of claim 1 or 2, its pharmaceutically acceptable salt, stereoisomer, isotopic isomer or tautomeric form thereof, wherein $-Y_1-Y_2-$ is

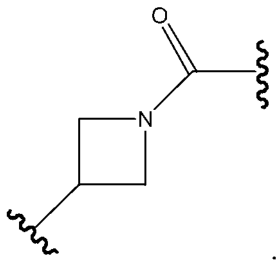

**46.** A compound according to any one of claim 1 or 2, its pharmaceutically acceptable salt, stereoisomer, isotopic isomer or tautomeric form thereof, wherein $R^L$ is $-NHS(O)_2CH_2CH_2OH$,

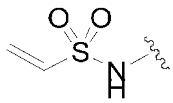 , 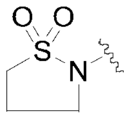 , 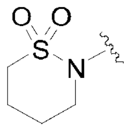 , 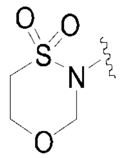

or $-NHS(O)_2CH_2CH_3$ or $-NHS(O)_2CH_3$.

**47.** A compound having the structure of Formula (II) or Formula (III), or pharmaceutically acceptable salts, stereoisomers, isotope isomers or tautomers thereof,

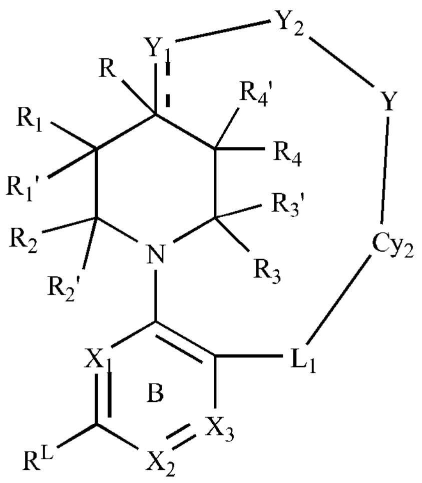

Formula (II)

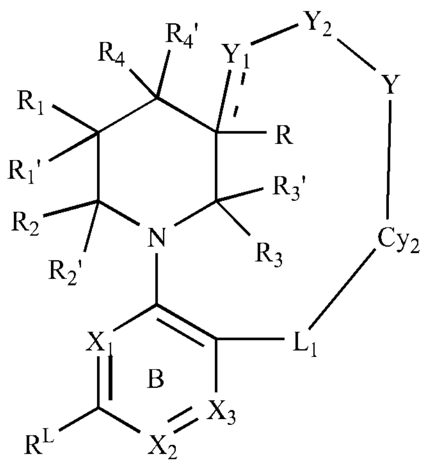

Formula (III)

**wherein,** $C_{y2}$ represents

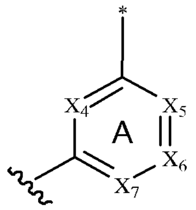 or 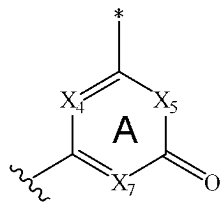 or 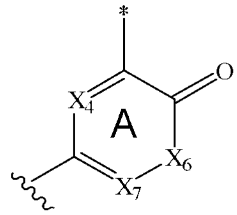 or 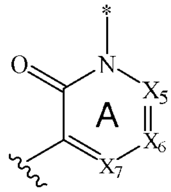

or

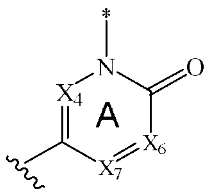

;

wherein, * represents the site of connection with Y, and the wavy line represents the site of connection with $L_1$;
wherein, - - - represents a single bond or double bond;
wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)O$R^a$, -C(O)$R^a$, -OC(O)$R^a$, -$NR^a$C(O)$R^b$ -C(O)$NR^aR^b$, -$NR^a$C(O)O$R^b$, -OC(O)$NR^aR^b$, -OC(O)O$R^a$, -$SF_5$, -PO($R^a$ $R^b$), -P($R^a$ $R^b$) or -$NR^aR^b$; -$C_0$-$C_{10}$ alkyl O$R^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl S$R^a$, -$C_0$-$C_{10}$ alkyl C(O)O$R^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR_a$C(O)$OR_b$, -$C_0$-$C_{10}$ alkyl OC(O)$NR^aR^b$, -$C_0$-$C_{10}$ alkyl OC(O)O$R^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)O$R^a$, -C(O)$R^a$, -OC(O)$R^a$, -$NR^a$C(O)$R^b$, -C(O)$NR^aR^b$, -$NR^a$C(O)O$R^b$, -OC(O)$NR^aR^b$, -OC(O)O$R^a$, -$SF_5$, -PO($R^a$ $R^b$), -P($R^a$ $R^b$) or -$NR^aR^b$; -$C_0$-$C_{10}$ alkyl O$R^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl S$R^a$, -$C_0$-$C_{10}$ alkyl C(O)O$R^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR_a$C(O)$OR_b$, -$C_0$-$C_{10}$ alkyl OC(O)$NR^aR^b$, -$C_0$-$C_{10}$ alkyl OC(O)O$R^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents selected from the following: deuterium, halogen, cyano, nitro, oxo, hydroxy ($C_1$-$C_6$) alkyl, $C_1$-$C_6$ alkyl, $C_2$-$C_6$ alkenyl, $C_2$-$C_6$ alkynyl, $C_3$-$C_6$ cycloalkyl, $C_1$-$C_6$ haloalkyl, $C_2$-$C_6$ haloalkenyl, $C_2$-$C_6$ haloalkynyl, $C_3$-$C_6$ halocycloalkyl, -$OR^a$, -$SO_3R^a$, -S(O)$R^a$, -O-$C_1$-$C_6$ haloalkyl, -$SR^a$, -C(=O)O$R^a$, -C(=O)$R^a$, -C(=O)$NR^aR^b$, -$SF_5$ or -$NR^aR^b$;
wherein, $L_1$ represents -C(O)$NR^a$-, -$NR^a$C(O)- or a 5-6-membered heteroaromatic ring;
wherein, Y represents absence, -$CH_2$-, -O-, -$NR^a$-, -C(O)$NR^a$, -$NR^a$C(O)- or the following groups:

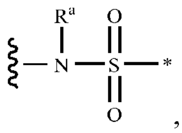 , 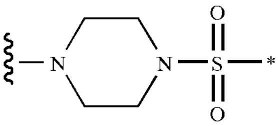 ,

or $C_{y3}$;

wherein, * represents the site of connection with ring A; the wavy line represents the site of connection with $Y_2$;

wherein, when $\overline{---}$ represents a double bond, R represents absence; wherein, when $\overline{---}$ represents a single bond, R represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;

wherein, $R_1$, $R_1'$, $R_2$, $R_2'$, $R_3$, $R_3'$, $R_4$ and $R_4'$ each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;

alternatively, pairs $R_1$ and $R_1'$, pairs $R_2$ and $R_2'$, pairs $R_3$ and $R_3'$ and pairs $R_4$ and $R_4'$, together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, -$OR^a$ or oxo;

wherein, $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein the substituents are each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$, -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR_a$C(O)$OR_b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;

wherein, $Y_1$ represents -$(CR^aR^b)_m$-; $Y_2$ represents -$(CR^aR^b)_n$-;

wherein, optionally -$CR^aR^b$- may be substituted with -O-, -S-, -Se-, -$NR^a$-, -$NR^aSO_2$-, -$NR^a$C(=O)-, -C(=O)$NR^a$-, -OC(=O)-, -C(=O)O-, -$SO_2NR^a$-, -S(=O)($NR^a$)-, -P(O)$(OR^a)_2$-, PO($R^a$ $R^b$), -$NR^a$P(O)$(OR^a)_2$- or -$NR^a$P(O)$(R^a)_2$-, -$CR^a$=$CR^b$-,

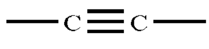 , 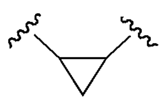 , 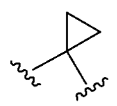 , 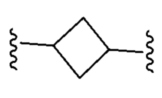 , 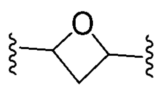 , 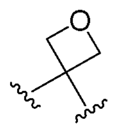 ,

5-6-membered heteroaryl,

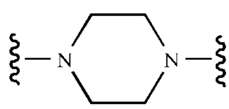 , 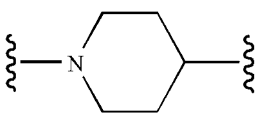 or 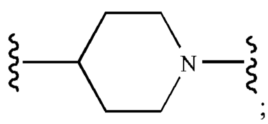 ;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^a$S(=O)(=NH)-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)$2, -$NR^a$P(O)$(OR^a)_2$ or -$NR^a$P(O)$(R^a)_2$-;

wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;

alternatively, $L_2$-$R^M$ represents

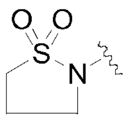 or 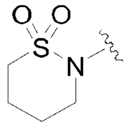 ;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, Se and N.

**48.** A compound having the structure of Formula (II-1) or Formula (III-1), or pharmaceutically acceptable salts, stereoisomers, isotope isomers or tautomers thereof:

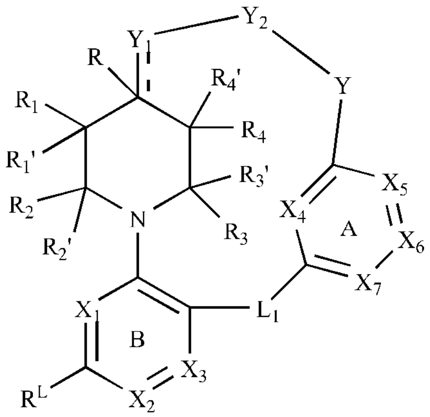

Formula (II-1)

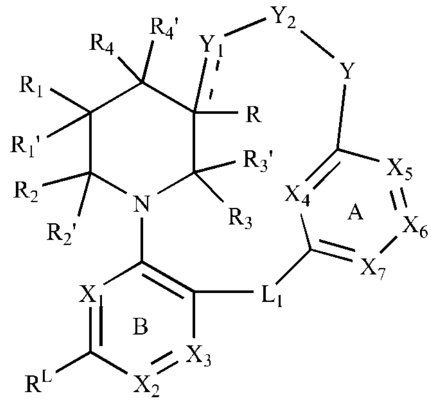

Formula (III-1)

wherein, $\overline{---}$ represents a single bond or double bond;

wherein, $X_1$ represents $CR^{W1}$ or N;

wherein, $X_2$ represents $CR^{W2}$ or N;

wherein, $X_3$ represents $CR^{W3}$ or N;

wherein, $X_4$ represents $CR^{W4}$ or N;

wherein, $X_5$ represents $CR^{W5}$ or N;

wherein, $X_6$ represents $CR^{W6}$ or N;

wherein, $X_7$ represents $CR^{W7}$ or N;

wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O$-$C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR_aC(O)OR_b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;

alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, oxo, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl,

$-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR_aC(O)OR_b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;

alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents selected from the following: deuterium, halogen, cyano, nitro, oxo, hydroxy ($C_1-C_6$) alkyl, $C_1-C_6$ alkyl, $C_2-C_6$ alkenyl, $C_2-C_6$ alkynyl, $C_3-C_6$ cycloalkyl, $C_1-C_6$ haloalkyl, $C_2-C_6$ haloalkenyl, $C_2-C_6$ haloalkynyl, $C_3-C_6$ halocycloalkyl, $-OR^a$, $-SO_3R^a$, $-S(O)R^a$, $-O-C_1-C_6$ haloalkyl, $-SR^a$, $-C(=O)OR^a$, $-C(=O)R^a$, $-C(=O)NR^aR^b$, $-SF_5$ or $-NR^aR^b$;

wherein, $L_1$ represents $-C(O)NR^a-$, $-NR^aC(O)-$ or a 5-6-membered heteroaromatic ring;

wherein, Y represents absence, $-CH_2-$, -O-, $-NR^a-$, $-C(O)NR^a$, $-NR^aC(O)-$ or the following groups:

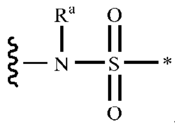 , 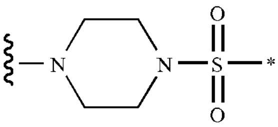

or $C_{y3}$;

wherein, * represents the site of connection with ring A; the wavy line represents the site of connection with $Y_2$; wherein, when --- represents a double bond, R represents absence; wherein, when --- represents a single bond, R represents hydrogen, deuterium, $C_1-C_6$ alkyl, $C_2-C_6$ alkenyl, $C_2-C_6$ alkynyl, halogen, cyano, $C_1-C_6$ haloalkyl, $C_1-C_6$ haloalkoxy, hydroxyl or $-C(O)CH_3$;

wherein, $R_1$, $R_1'$, $R_2$, $R_2'$, $R_3$, $R_3'$, $R_4$ and $R_4'$ each independently represent hydrogen, $C_1-C_6$ alkyl, halogen or $C_3-C_6$ cycloalkyl; hydroxy ($C_1-C_6$) alkyl or $C_1-C_6$ haloalkyl;

alternatively, pairs $R_1$ and $R_1'$, pairs $R_2$ and $R_2'$, pairs $R_3$ and $R_3'$ and pairs $R_4$ and $R_4'$, together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1-C_6$ alkyl, $C_1-C_6$ haloalkyl, $-OR^a$ or oxo;

wherein, $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein the substituents are each independently selected from the following: hydrogen, deuterium, halogen, cyano, nitro, azido, hydroxy ($C_1-C_{10}$) alkyl, $C_1-C_{10}$ alkyl, $C_2-C_{10}$ alkenyl, $C_2-C_{10}$ alkynyl, $C_1-C_{10}$ deuterated alkyl, $C_3-C_{12}$ cycloalkyl, $C_1-C_{10}$ haloalkyl, $C_2-C_{10}$ haloalkenyl, $C_2-C_{10}$ haloalkynyl, $C_3-C_{12}$ halocycloalkyl, 3-12-membered heterocyclyl, $C_6-C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR_aC(O)OR_b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;

wherein, $Y_1$ represents $-(CR^aR^b)_m-$; $Y_2$ represents $-(CR^aR^b)_n-$;

wherein, optionally $-CR^aR^b-$ may be substituted with -O-, -S-, -Se-, $-NR^a-$, $-NR^aSO_2-$, $-NR^aC(=O)-$, $-C(=O)NR^a-$, -OC(=O)-, -C(=O)O-, $-SO_2NR^a-$, $-S(=O)(NR^a)-$, $-P(O)(OR^a)_2-$, $PO(R^a R^b)$, $-NR^aP(O)(OR^a)_2-$ or $-NR^aP(O)(R^a)_2-$, $-CR^a=CR^b-$,

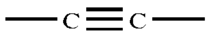 , 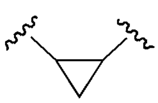 , 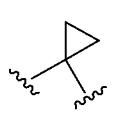 , 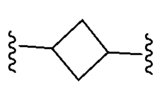 , 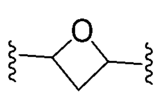 , 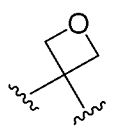 ,

5-6-membered heteroaryl,

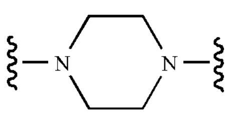, 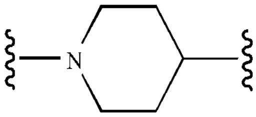 or 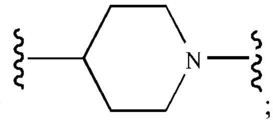;

wherein, $R^L$ represents $L_2$-$R^M$;
wherein, $L_2$ represents absence, -$C_1$-$C_6$ alkyl-, -$NR^a$-, -$NR^aSO_2$-, -$SO_2NR^a$-, -$NR^aS(=O)(=NH)$-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)_2$, -$NR^a$P(O)$(OR^a)_2$ or -$NR^a$P(O)$(R^a)_2$-;
wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;
alternatively, $L_2$-$R^M$ represents

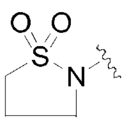 or 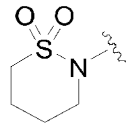;

wherein, m and n each independently represent an integer from 0 to 10;
wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, and N.

**49.** A compound according to any one of claim 47 or 48, its pharmaceutically acceptable salt, stereoisomer, isotopic isomer or tautomeric form thereof, wherein $X_1$ represents CH or N, preferably CH.

**50.** A compound according to any one of claim 47 or 48, its pharmaceutically acceptable salt, stereoisomer, isotopic isomer or tautomeric form thereof, wherein $X_2$ is CH or N, preferably CH.

**51.** A compound according to any one of claim 47 or 48, its pharmaceutically acceptable salt, stereoisomer, isotopic isomer or tautomeric form thereof, wherein $X_3$ is CH or N, preferably CH.

**52.** A compound according to any one of claim 47 or 48, its pharmaceutically acceptable salt, stereoisomer, isotopic isomer or tautomeric form thereof, wherein the chemical bond between $X_2$ and $X_3$ is fused with ring B to form a group in the following structure:

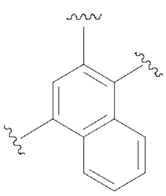 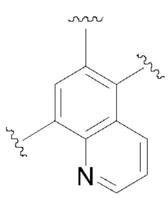 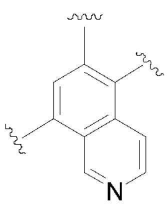 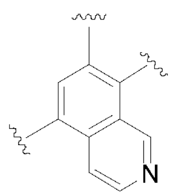 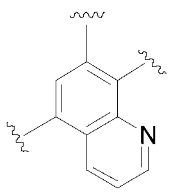 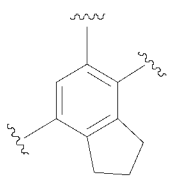

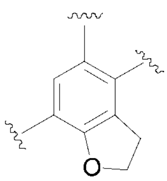 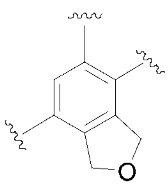 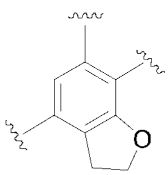 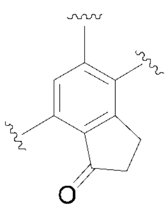 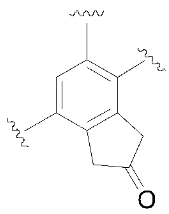 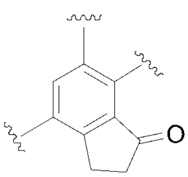

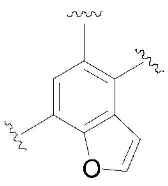 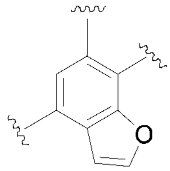 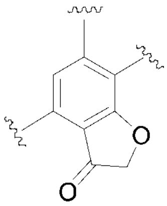 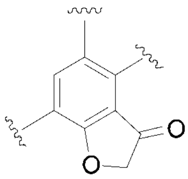

**53.** A compound according to any one of claim 47 or 48, its pharmaceutically acceptable salt, stereoisomer, isotopic isomer or tautomeric form thereof, wherein $X_4$ is CH or N, preferably N.

**54.** A compound according to any one of claim 47 or 48, its pharmaceutically acceptable salt, stereoisomer, isotopic isomer or tautomeric form thereof, wherein $X_5$ is $CR^{W5}$ or N and $R^{W5}$ is hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

**55.** A compound according to any one of claim 47 or 48, its pharmaceutically acceptable salt, stereoisomer, isotopic isomer or tautomeric form thereof, wherein $X_6$ is $CR^{W6}$ or N, and $R^{W6}$ is hydrogen, halogen, $-CH_3O$, $-CF_3$, $-CHF_2$, $-CH_2F$, $-NH_2$, $-N(CH_3)_2$, -CN, -OH, $-C(O)CH_3$, $-OC(O)CH_3$, $-C(O)OCH_3$ or $-C(O)NH_2$.

**56.** A compound according to any one of claim 47 or 48, its pharmaceutically acceptable salt, stereoisomer, isotopic isomer or tautomeric form thereof, wherein $X_7$ is CH or N, preferably N.

**57.** A compound according to any one of claim 47 or 48, its pharmaceutically acceptable salt, stereoisomer, isotopic isomer or tautomeric form thereof, wherein the chemical bond between $X_5$ and $X_6$ is fused with ring A to form a group in the following structure:

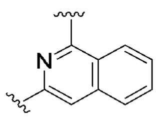 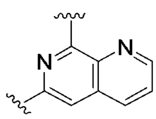 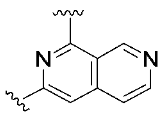 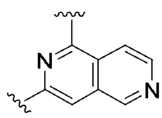 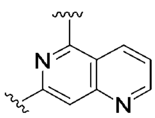 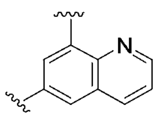

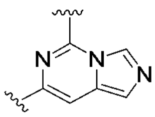 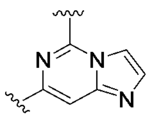 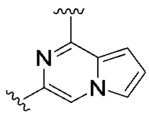 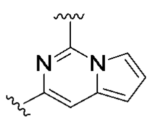 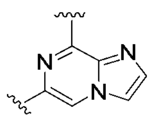 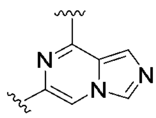
.

**58.** A compound according to any one of claim 47 or 48, its pharmaceutically acceptable salt, stereoisomer, isotopic isomer or tautomeric form thereof, wherein the chemical bond between $X_6$ and $X_7$ is fused with ring A to form a group in the following structure:

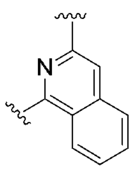 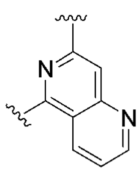 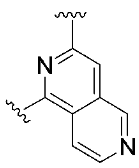 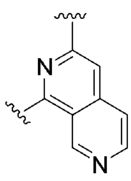 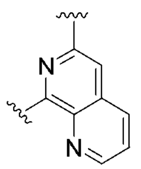 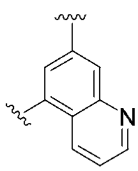

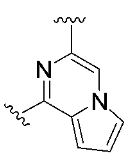 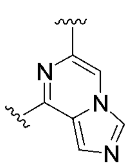 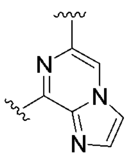 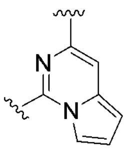 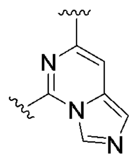 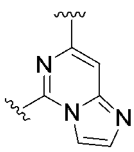
.

**59.** A compound according to any one of claim 47 or 48, its pharmaceutically acceptable salt, stereoisomer, isotopic isomer or tautomeric form thereof, wherein Y represents the following groups: absent, $-CH_2-$, -O-, -Se-, $-NR^a-$, $-C(O)NR^a$, $-NR^aC(O)-$ or the following groups:

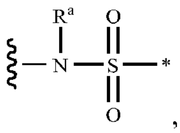, 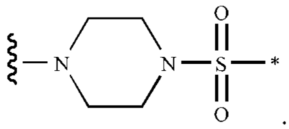.

**60.** A compound according to any one of claim 47 or 48, its pharmaceutically acceptable salt, stereoisomer, isotopic isomer or tautomeric form thereof, wherein Y is -O-, -Se- or -NH-.

**61.** A compound according to any one of claim 47 or 48, its pharmaceutically acceptable salt, stereoisomer, isotopic isomer or tautomeric form thereof, wherein Y is

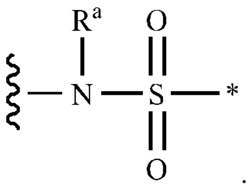.

**62.** A compound according to any one of claim 47 or 48, its pharmaceutically acceptable salt, stereoisomer, isotopic isomer or tautomeric form thereof, wherein Y is

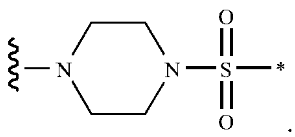.

**63.** A compound according to any one of claim 47 or 48, its pharmaceutically acceptable salt, stereoisomer, isotopic isomer or tautomeric form thereof, wherein Y is $C_{y3}$ and $C_{y3}$ represents the following group:

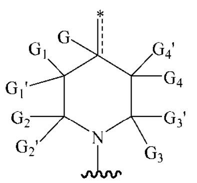, 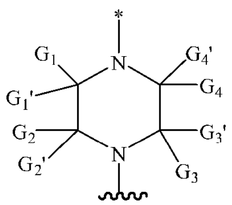, 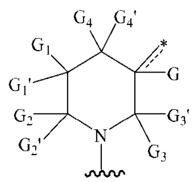, 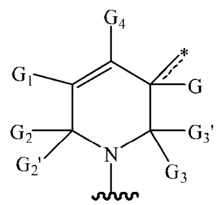, 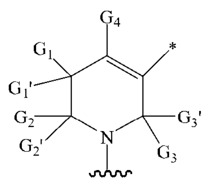,

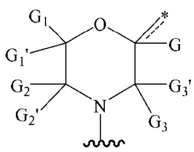, 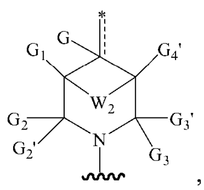, 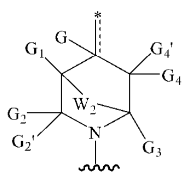, 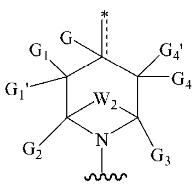, 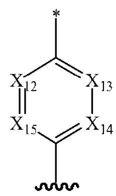, 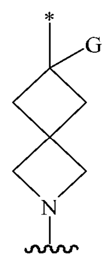, 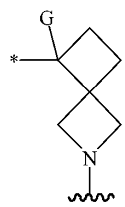,

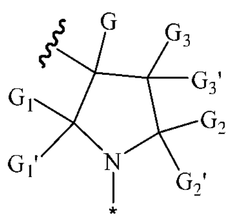, 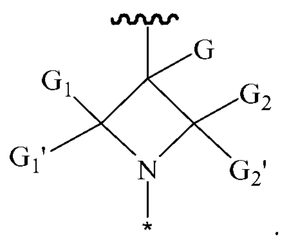;

wherein, when --- represents a double bond, G represents absence; wherein, when --- represents a single bond,

G represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;
wherein, $G_1$, $G_1$', $G_2$, $G_2$', $G_3$, $G_3$, $G_4$ and $G_4$' each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;
alternatively, pairs $G_1$ and $G_1$', pairs $G_2$ and $G_2$', pairs $G_3$ and $G_3$' and pairs $G_4$ and $G_4$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $OR^a$ or oxo;
wherein, $W_2$ represents $-(CR^iR^j)_p-$;
wherein, $R^i$ and $R^j$ each independently represent hydrogen or $C_1$-$C_6$ alkyl;
wherein, p represents 1 or 2;
wherein, $X_{12}$ represents $CR^{W12}$ or N;
wherein, $X_{13}$ represents $CR^{W13}$ or N;
wherein, $X_{14}$ represents $CR^{W14}$ or N;
wherein, $X_{15}$ represents $CR^{W15}$ or N;
wherein, $R^{W12}$, $R^{W13}$, $R^{W14}$ and $R^{W15}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR_aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;

**64.** A compound according to any one of claim 47 or 48, its pharmaceutically acceptable salt, stereoisomer, isotopic isomer or tautomeric form thereof, wherein $X_{12}$ is CH or N.

**65.** A compound according to any one of claim 47 or 48, its pharmaceutically acceptable salt, stereoisomer, isotopic isomer or tautomeric form thereof, wherein $X_{13}$ is CH or N.

**66.** A compound according to any one of claim 47 or 48, its pharmaceutically acceptable salt, stereoisomer, isotopic isomer or tautomeric form thereof, wherein $X_{14}$ is CH or N.

**67.** A compound according to any one of claim 47 or 48, its pharmaceutically acceptable salt, stereoisomer, isotopic isomer or tautomeric form thereof, wherein $X_{15}$ is CH or N.

**68.** A compound according to any one of claim 47 or 48, its pharmaceutically acceptable salt, stereoisomer, isotopic isomer or tautomeric form thereof, wherein $G_1$, $G_1$', $G_2$, $G_2$', $G_3$, $G_3$', $G_4$ or $G_4$' is hydrogen, respectively.

**69.** A compound according to any one of claim 47 or 48, its pharmaceutically acceptable salt, stereoisomer, isotopic isomer or tautomeric form thereof, wherein $W_2$ is $-CH_2$ or $-CH_2CH_2$.

**70.** A compound according to any one of claim 47 or 48, its pharmaceutically acceptable salt, stereoisomer, isotopic isomer or tautomeric form thereof,
$L_1$ represents $-C(O)NR^a-$, $-NR^aC(O)-$,

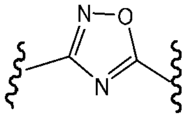, 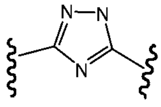, 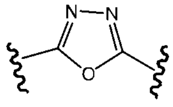, 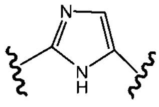, 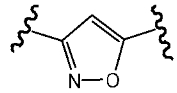,

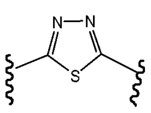, 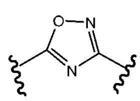, 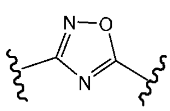, 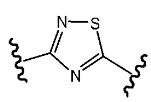, 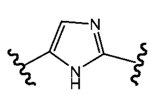, 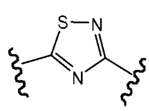, 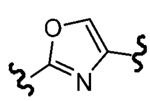,

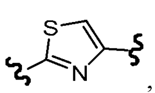, 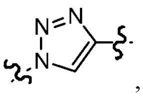, 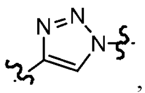, 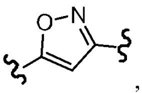, 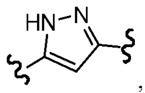, 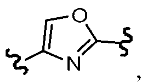, 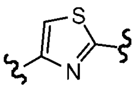, 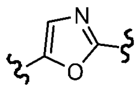,

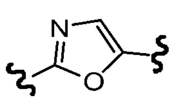, 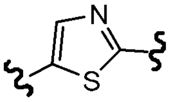 or 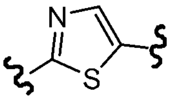.

**71.** A compound according to any one of claim 47 or 48, its pharmaceutically acceptable salt, stereoisomer, isotopic isomer or tautomeric form thereof, wherein $L_1$ is -C(O)NH-.

**72.** A compound according to any one of claim 47 or 48, its pharmaceutically acceptable salt, stereoisomer, isotopic isomer or tautomeric form thereof, wherein $L_1$ is

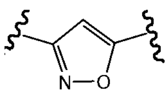, 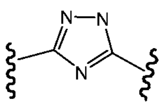, 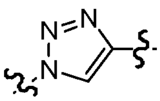, 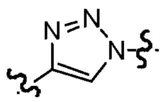, 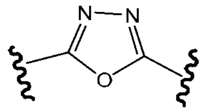

or

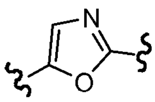.

**73.** A compound according to any one of claim 47 or 48, its pharmaceutically acceptable salt, stereoisomer, isotopic isomer or tautomeric form thereof, wherein $Y_1$ is $-(CR^aR^b)_m-$.

**74.** A compound according to any one of claim 47 or 48, its pharmaceutically acceptable salt, stereoisomer, isotopic isomer or tautomeric form thereof, wherein $Y_1$ is -NR-C(=O)-, -O-C(=O)- or -C(=O)O-.

**75.** A compound according to any one of claim 47 or 48, its pharmaceutically acceptable salt, stereoisomer, isotopic isomer or tautomeric form thereof, wherein $Y_1$ is $-O(CR^aR^b)_m-$ or $-(CR^aR^b)_mO-$.

**76.** A compound according to any one of claim 47 or 48, its pharmaceutically acceptable salt, stereoisomer, isotopic isomer or tautomeric form thereof, wherein $Y_1$ is $-CR^a=CR^a-$ or $-CR^aR^b-CR^a=CR^b-$.

**77.** A compound according to any one of claim 47 or 48, its pharmaceutically acceptable salt, stereoisomer, isotopic isomer or tautomeric form thereof, wherein $Y_2$ is $-(CR^aR^b)_n-$.

**78.** A compound according to any one of claim 47 or 48, its pharmaceutically acceptable salt, stereoisomer, isotopic isomer or tautomeric form thereof, wherein $Y_2$ is $-NR^a-C(=O)-$.

**79.** A compound according to any one of claim 47 or 48, its pharmaceutically acceptable salt, stereoisomer, isotopic isomer or tautomeric form thereof, wherein $Y_2$ is $-O(CR^aR^b)_n-$ or $-(CR^aR^b)_nO-$.

**80.** A compound according to any one of claim 47 or 48, its pharmaceutically acceptable salt, stereoisomer, isotopic isomer or tautomeric form thereof, wherein $Y_2$ is $-CR^a=CR^a-$, $-CR^a=CR^b-CR^aR^b-$ or $-CR^aR^b-CR^a=CR^b-$.

**81.** A compound according to any one of claim 47 or 48, its pharmaceutically acceptable salt, stereoisomer, isotopic isomer or tautomeric form thereof, wherein $Y_2$ is pyrazolyl, oxazolyl, oxadiazolyl, thiazolyl, thiadiazolyl or triazolyl.

**82.** A compound according to any one of claim 47 or 48, its pharmaceutically acceptable salt, stereoisomer, isotopic isomer or tautomeric form thereof, wherein $-Y_1-Y_2-$ is $-CR^a=CR^a-$ or $-CR^aR^bCR^a=CR^b-$.

**83.** A compound according to claim 46, its pharmaceutically acceptable salt, stereoisomer, isotopic isomer or tautomeric

form thereof, wherein $-Y_1-Y_2-$ is $-CR^aR^b-NR^aC(O)-$.

**84.** A compound according to any one of claim 47 or 48, its pharmaceutically acceptable salt, stereoisomer, isotopic isomer or tautomeric form thereof, wherein $-Y_1-Y_2-$ is

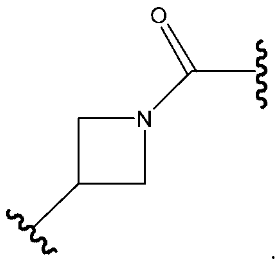

.

**85.** A compound according to any one of claim 47 or 48, its pharmaceutically acceptable salt, stereoisomer, isotopic isomer or tautomeric form thereof, wherein $R^L$ is $-NHS(O)_2CH_2CH_2OH$,

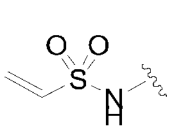 , 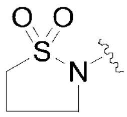 , 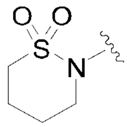 , 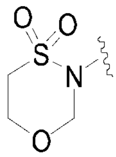 ,

$-NHS(O)_2CH_2CH_3$ or $-NHS(O)_2CH_3$.

**86.** A compound having the structure of Formula (I-3), or pharmaceutically acceptable salts, stereoisomers, isotope isomers or tautomers thereof,

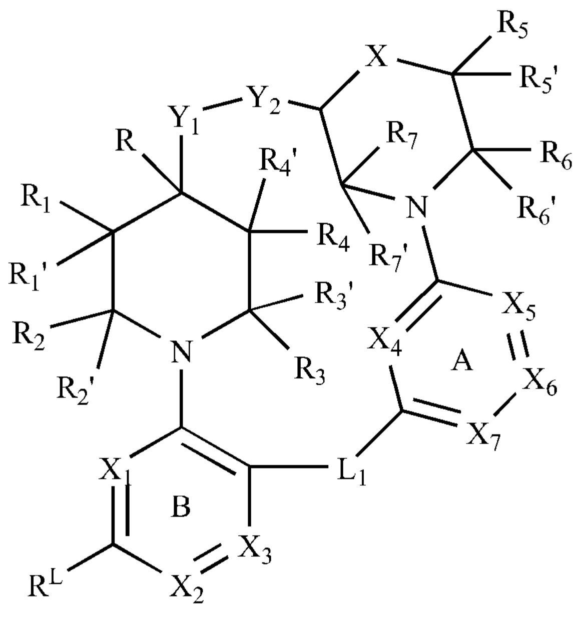

Formula(I-3)

wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;

wherein, $X_6$ represents $CR^{W6}$ or N;
wherein, $X_7$ represents $CR^{W7}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1$-$C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR_aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR_aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1$-$C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR_aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
wherein, $L_1$ represents $-C(O)NR^a-$, $-NR^aC(O)-$ or a 5-6-membered heteroaromatic ring;
wherein, R represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or $-C(O)CH_3$;
wherein, X represents $CR_9R_9'$, O or $NR^a$;
wherein, $R_1$, $R_1'$, $R_2$, $R_2'$, $R_3$, $R_3'$, $R_4$, $R_4'$, $R_5$, $R_5'$, $R_6$, $R_6'$, $R_7$, $R_7'$, $R_9$ and $R_9'$ each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;
alternatively, pairs $R_1$ and $R_1'$, pairs $R_2$ and $R_2'$, pairs $R_3$ and $R_3'$, pairs $R_4$ and $R_4'$, pairs $R_5$ and $R_5'$, pairs $R_6$ and $R_6'$, pairs $R_7$ and $R_7'$ and pairs $R_9$ and $R_9'$, together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $-OR^a$ or oxo;
wherein, $Y_1$ represents $-(CR^aR^b)_m-$; $Y_2$ represents $-(CR^aR^b)_n-$;
wherein, optionally $-CR^aR^b-$ may be substituted with -O-, -S-, -Se-, $-NR^a-$, $-NR^aSO_2-$, $-NR^aC(=O)-$, $-C(=O)NR^a-$, -OC(=O)-, -C(=O)O-, $-SO_2NR^a-$, $-S(=O)(NR^a)-$, $-P(O)(OR^a)_2-$, $PO(R^a R^b)$, $-NR^aP(O)(OR^a)_2-$ or $-NR^aP(O)(R^a)_2-$, $-CR^a=CR^b-$,

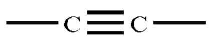 , 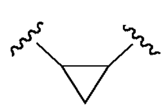 , 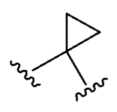 , 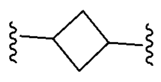 , 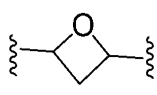 , 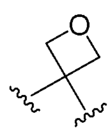 ,

5-6-membered heteroaryl,

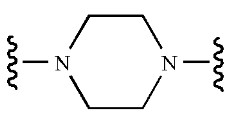 , 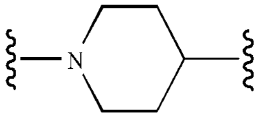 or 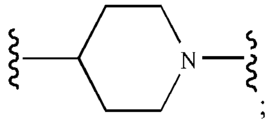 ;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^aS(=O)(=NH)$-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)_2$, -$NR^a$P(O)$(OR^a)_2$ or -$NR^a$P(O)$(R^a)_2$-;

wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;

alternatively, $L_2$-$R^M$ represents

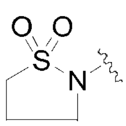 or 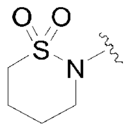 ;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, Se and N.

**87.** A compound according to claim 86, its pharmaceutically acceptable salt, stereoisomer, isotopic isomer or tautomeric form thereof, wherein $X_1$ represents CH or N, preferably CH.

**88.** A compound according to claim 86, its pharmaceutically acceptable salt, stereoisomer, isotopic isomer or tautomeric form thereof, wherein $X_2$ is CH or N, preferably CH.

**89.** A compound according to claim 86, its pharmaceutically acceptable salt, stereoisomer, isotopic isomer or tautomeric form thereof, wherein $X_3$ is CH or N, preferably CH.

**90.** A compound according to claim 86, its pharmaceutically acceptable salt, stereoisomer, isotopic isomer or tautomeric form thereof, wherein the chemical bond between $X_2$ and $X_3$ is fused with ring B to form a group in the following structure:

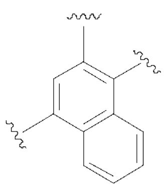 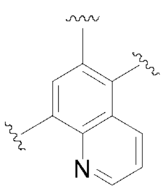 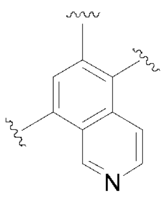 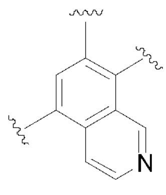 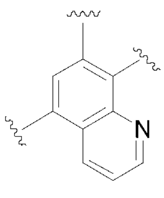 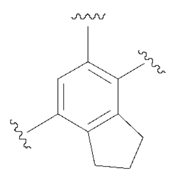

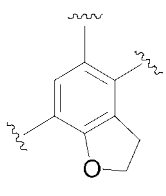 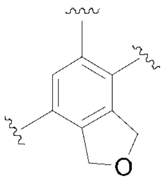 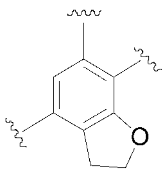 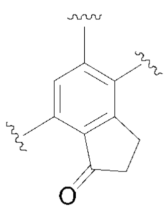 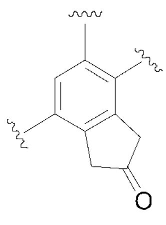 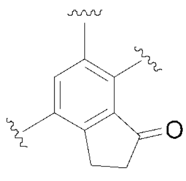

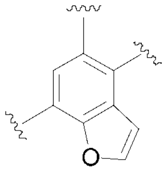 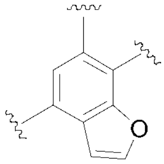 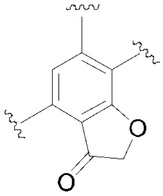 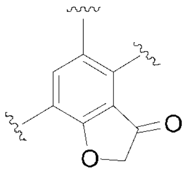

**91.** A compound according to claim 86, its pharmaceutically acceptable salt, stereoisomer, isotopic isomer or tautomeric form thereof, wherein $X_4$ is CH or N, preferably N.

**92.** A compound according to claim 86, its pharmaceutically acceptable salt, stereoisomer, isotopic isomer or tautomeric form thereof, wherein $X_5$ is $CR^{W5}$ or N, and $R^{W5}$ is hydrogen, halogen, $-CH_3O$, $-CF_3$, $-CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

**93.** A compound according to claim 86, its pharmaceutically acceptable salt, stereoisomer, isotopic isomer or tautomeric form thereof, wherein $X_6$ is $CR^{W6}$ or N, and $R^{W6}$ is hydrogen, halogen, $-CH_3O$, $-CF_3$, $-CHF_2$, $-CH_2F$, $-NH_2$, $-N(CH_3)_2$, -CN, -OH, $-C(O)CH_3$, $-OC(O)CH_3$, $-C(O)OCH_3$ or $-C(O)NH_2$.

**94.** A compound according to claim 86, its pharmaceutically acceptable salt, stereoisomer, isotopic isomer or tautomeric form thereof, wherein $X_7$ is CH or N, preferably N.

**95.** A compound according to claim 86, its pharmaceutically acceptable salt, stereoisomer, isotopic isomer or tautomeric form thereof, wherein the chemical bond between $X_5$ and $X_6$ is fused with ring A to form a group in the following structure:

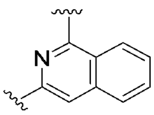 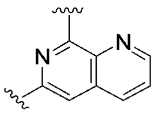 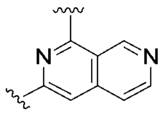 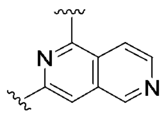 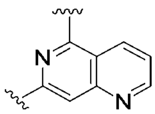 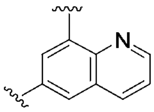

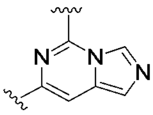 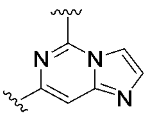 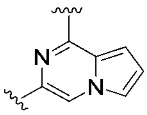 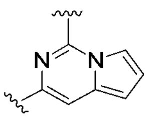 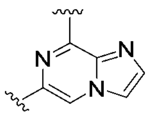 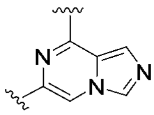 .

**96.** A compound according to claim 86, its pharmaceutically acceptable salt, stereoisomer, isotopic isomer or tautomeric form thereof, wherein the chemical bond between $X_6$ and $X_7$ is fused with ring A to form a group in the following structure:

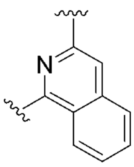 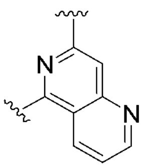 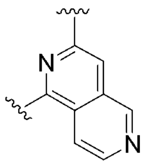 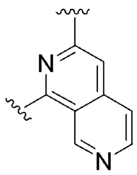 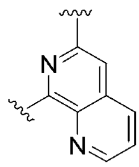 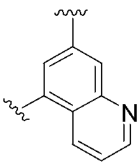

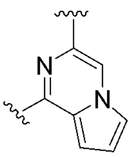 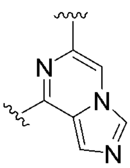 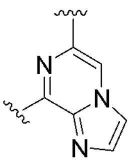 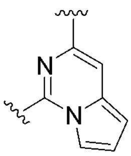 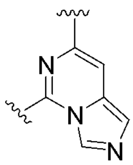 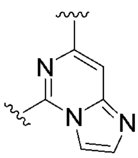 .

**97.** A compound according to claim 86, its pharmaceutically acceptable salt, stereoisomer, isotopic isomer or tautomeric form thereof,
$L_1$ represents $-C(O)NR^a-$, $-NR^aC(O)-$,

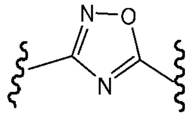, 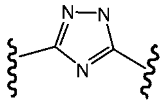, 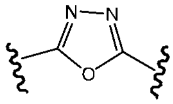, 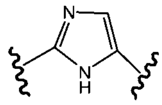, 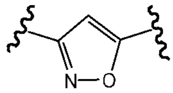,

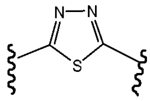, 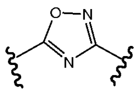, 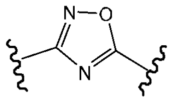, 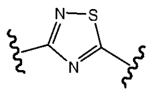, 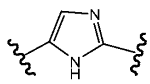, 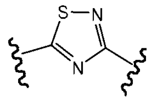, 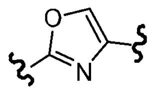,

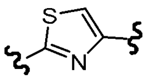, 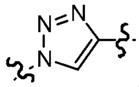, 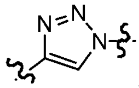, 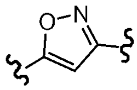, 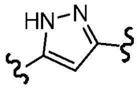, 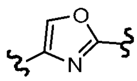, 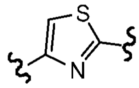, 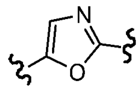,

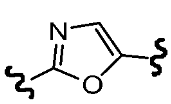, 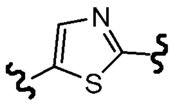 or 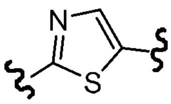.

**98.** A compound according to claim 86, its pharmaceutically acceptable salt, stereoisomer, isotopic isomer or tautomeric form thereof, wherein $L_1$ is -C(O)NH-.

**99.** A compound according to claim 86, its pharmaceutically acceptable salt, stereoisomer, isotopic isomer or tautomeric form thereof, wherein $L_1$ is

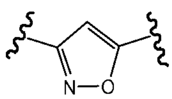, 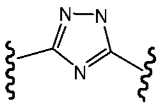, 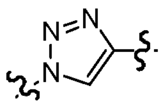, 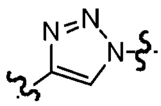, 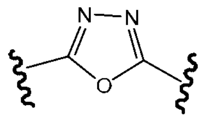

or

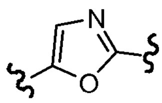.

**100.**
A compound according to claim 86, its pharmaceutically acceptable salt, stereoisomer, isotopic isomer or tautomeric form thereof, wherein $Y_1$ is $-(CR^aR^b)_m-$.

**101.**
A compound according to claim 86, its pharmaceutically acceptable salt, stereoisomer, isotopic isomer or tautomeric form thereof, wherein $Y_1$ is -NR-C(=O)-, -O-C(=O)- or -C(=O)O-.

**102.**
A compound according to claim 86, its pharmaceutically acceptable salt, stereoisomer, isotopic isomer or tautomeric form thereof, wherein $Y_1$ is $-O(CR^aR^b)_m-$ or $-(CR^aR^b)_mO-$.

**103.**
A compound according to claim 86, its pharmaceutically acceptable salt, stereoisomer, isotopic isomer or tautomeric form thereof, wherein $Y_1$ is $-CR^a=CR^a-$ or $-CR^aR^b-CR^a=CR^b-$.

**104.**
A compound according to claim 86, its pharmaceutically acceptable salt, stereoisomer, isotopic isomer or tautomeric form thereof, wherein $Y_2$ is $-(CR^aR^b)_n-$.

**105.**
A compound according to claim 86, its pharmaceutically acceptable salt, stereoisomer, isotopic isomer or tautomeric form thereof, wherein $Y_2$ is $-NR^a-C(=O)-$.

**106.**
A compound according to claim 86, its pharmaceutically acceptable salt, stereoisomer, isotopic isomer or tautomeric form thereof, wherein $Y_2$ is $-O(CR^aR^b)_n-$ or $-(CR^aR^b)_nO-$.

**107.**
A compound according to claim 86, its pharmaceutically acceptable salt, stereoisomer, isotopic isomer or tautomeric form thereof, wherein $Y_2$ is $-CR^a=CR^a-$, $-CR^a=CR^b-CR^aR^b-$ or $-CR^aR^b-CR^a=CR^b-$.

**108.**
A compound according to claim 86, its pharmaceutically acceptable salt, stereoisomer, isotopic isomer or tautomeric form thereof, wherein $Y_2$ is pyrazolyl, oxazolyl, oxadiazolyl, thiazolyl, thiadiazolyl or triazolyl.

**109.**
A compound according to claim 86, its pharmaceutically acceptable salt, stereoisomer, isotopic isomer or tautomeric form thereof, wherein $-Y_1-Y_2-$ is $-CR^a=CR^a-$ or $-CR^aR^bCR^a=CR^b-$.

**110.**
A compound according to claim 86, its pharmaceutically acceptable salt, stereoisomer, isotopic isomer or tautomeric form thereof, wherein $-Y_1-Y_2-$ is $-CR^aR^b-NR^aC(O)-$.

**111.** A compound according to claim 86, its pharmaceutically acceptable salt, stereoisomer, isotopic isomer or tautomeric form thereof, wherein $-Y_1-Y_2-$ is

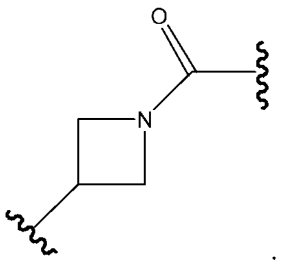

.

**112.**
A compound according to claim 86, its pharmaceutically acceptable salt, stereoisomer, isotopic isomer or tautomeric form thereof, wherein $R^L$ is $-NHS(O)_2CH_2CH_2OH$,

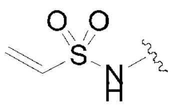 , 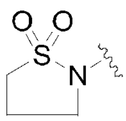 , 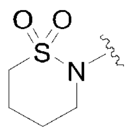 , 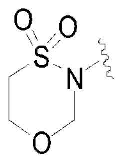 ,

$-NHS(O)_2CH_2CH_3$ or $-NHS(O)_2CH_3$.

**113.**
A compound having the structure of Formula (I-1-1) or Formula (I-1-2), or pharmaceutically acceptable salts, stereoisomers, isotope isomers or tautomers thereof,

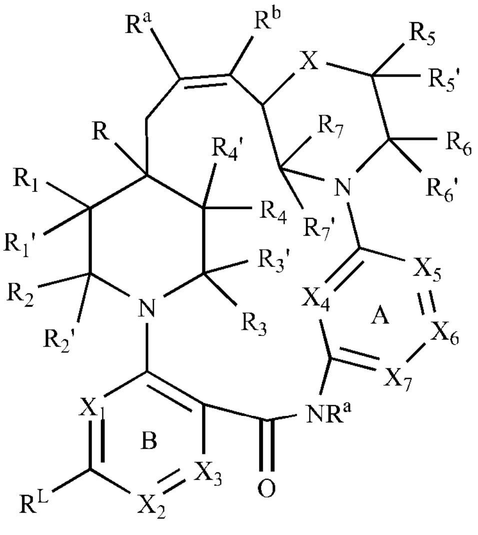

Formula (I-1-1)

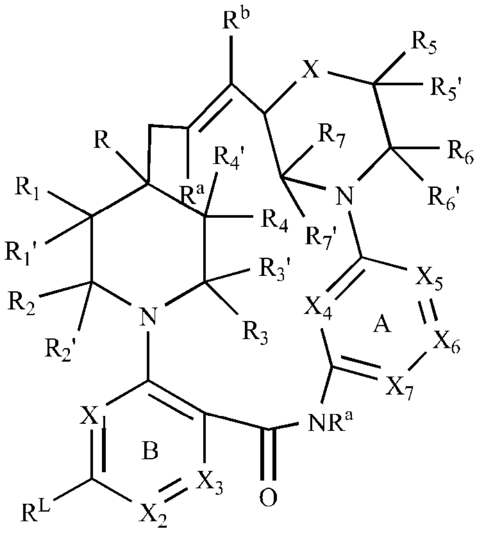

Formula(I-1-2)

wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1-C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR_aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl $O-C_1-C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR_aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^aR^b)$ or $-NR^aR^b$;

$-C_0-C_{10}$ alkyl $OR^a$, $-C_0-C_{10}$ alkyl $SO_3R^a$, $-C_0-C_{10}$ alkyl $SO_2R^a$, $-C_0-C_{10}$ alkyl $S(O)R^a$, $-C_0-C_{10}$ alkyl $O-C_1-C_{10}$ haloalkyl, $-C_0-C_{10}$ alkyl $SR^a$, $-C_0-C_{10}$ alkyl $C(O)OR^a$, $-C_0-C_{10}$ alkyl $C(O)R^a$, $-C_0-C_{10}$ alkyl $OC(O)R^a$, $C_0-C_{10}$ alkyl-$NR^aC(O)R^b$, $-C_0-C_{10}$ alkyl $C(O)NR^aR^b$, $-C_0-C_{10}$ alkyl $NR_aC(O)OR^b$, $-C_0-C_{10}$ alkyl $OC(O)NR^aR^b$, $-C_0-C_{10}$ alkyl $OC(O)OR^a$, $-C_0-C_{10}$ alkyl $SF_5$, $-C_0-C_{10}$ alkyl $PO(R^a R^b)$, $-C_0-C_{10}$ alkyl $P(R^a R^b)$ or $-C_0-C_{10}$ alkyl $NR^aR^b$;
wherein, R represents hydrogen, deuterium, $C_1-C_6$ alkyl, $C_2-C_6$ alkenyl, $C_2-C_6$ alkynyl, halogen, cyano, $C_1-C_6$ haloalkyl, $C_1-C_6$ haloalkoxy, hydroxyl or $-C(O)CH_3$;
wherein, X represents $CR_9R_9$', O or $NR^a$;
wherein, $R_1$, $R_1$', $R_2$, $R_2$', $R_3$, $R_3$', $R_4$, $R_4$', $R_5$, $R_5$', $R_6$, $R_6$', $R_7$, $R_7$', $R_9$ and $R_9$' each independently represent hydrogen, $C_1-C_6$ alkyl, halogen or $C_3-C_6$ cycloalkyl; hydroxy ($C_1-C_6$) alkyl or $C_1-C_6$ haloalkyl;
alternatively, pairs $R_1$ and $R_1$', pairs $R_2$ and $R_2$', pairs $R_3$ and $R_3$', pairs $R_4$ and $R_4$', pairs $R_5$ and $R_5$', pairs $R_6$ and $R_6$', pairs $R_7$ and $R_7$' and pairs $R_9$ and $R_9$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1-C_6$ alkyl, $C_1-C_6$ haloalkyl, $OR^a$ or oxo;
wherein, $R^L$ represents $L_2-R^M$;
wherein, $L_2$ represents absence, $-C_1-C_6$ alkyl-, $-NR^a-$, $-NR^aSO_2-$, $-SO_2NR^a-$, $-NR^aS(=O)(=NH)-$, -S(=O) (=NH)-, -S-, -S(=O)-, $-SO_2-$, $-C_1-C_6$ alkyl-O-, -(C=O)-, $-(C=O)NR^a-$, -C=N(OH)-, $-NR^a$ (C=O), $-P(O)(OR^a)_2$, $-NR^aP(O)(OR^a)_2$ or $-NR^aP(O)(R^a)_2-$;
wherein, RM represents $C_1-C_6$ alkyl or $C_3-C_6$ (hetero) cycloalkyl, wherein the $C_1-C_6$ alkyl or $C_3-C_6$ (hetero) cycloalkyl can be optionally independently substituted with 0-3 substituents selected from halogen, $C_1-C_6$ alkyl, $-OR^a$, $-NR^aR^b$, cyano and $-O-C_1-C_6$ haloalkyl;
alternatively, $L_2-R^M$ represents

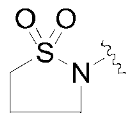 or 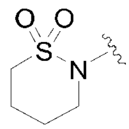 ;

wherein, m and n each independently represent an integer from 0 to 10;
wherein, $R^a$ and $R^b$ each independently represent hydrogen, halogen, $C_1-C_6$ alkyl, $C_3-C_6$ cycloalkyl, $C_1-C_6$ haloalkyl, hydroxy ($C_1-C_6$) alkyl, $C_1-C_6$ alkyl substituted with saturated or unsaturated 5-6-membered cycloalkyl, or $C_1-C_6$ alkyl substituted with saturated or unsaturated 5-6-membered heterocycloalkyl; alternatively, $R^a$ and $R^b$, together with the atom attached thereto, form a 3-6-membered saturated or unsaturated (heterocyclic) ring which may optionally contain 0-2 heteroatoms selected from O, S, Se and N.

**114.**
A compound according to claim 113, its pharmaceutically acceptable salt, stereoisomer, isotopic isomer or tautomeric form thereof, wherein $X_1$ represents CH or N, preferably CH.

**115.**
A compound according to claim 113, its pharmaceutically acceptable salt, stereoisomer, isotopic isomer or tautomeric form thereof, wherein $X_2$ is CH or N, preferably CH.

**116.**
A compound according to claim 113, its pharmaceutically acceptable salt, stereoisomer, isotopic isomer or tautomeric form thereof, wherein $X_3$ is CH or N, preferably CH.

**117.**
A compound according to claim 113, its pharmaceutically acceptable salt, stereoisomer, isotopic isomer or tautomeric form thereof, wherein the chemical bond between $X_2$ and $X_3$ is fused with ring B to form a group in the following structure:

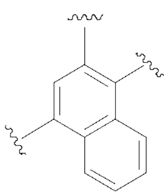 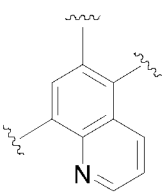 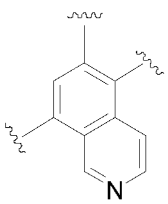 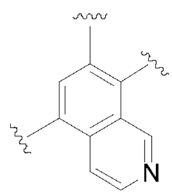 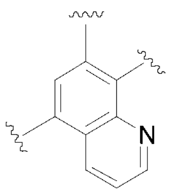 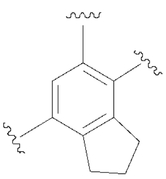

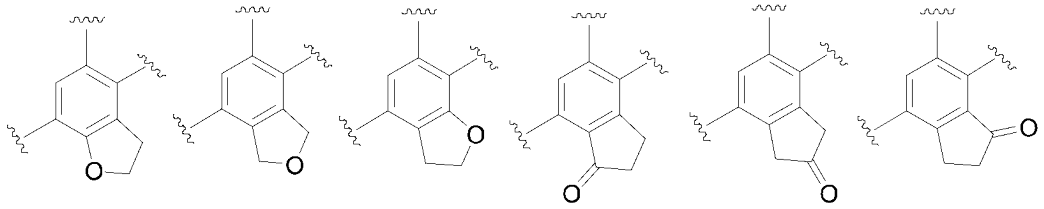

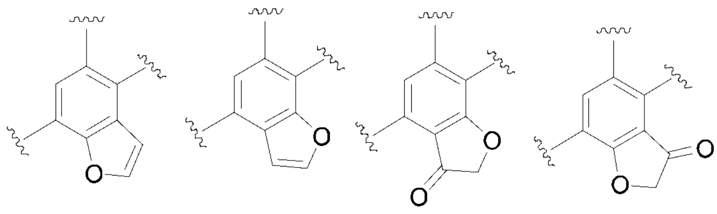

**118.**
A compound according to claim 113, its pharmaceutically acceptable salt, stereoisomer, isotopic isomer or tautomeric form thereof, wherein $X_4$ is CH or N, preferably N.

**119.**
A compound according to claim 113, its pharmaceutically acceptable salt, stereoisomer, isotopic isomer or tautomeric form thereof, wherein $X_5$ is $CR^{W5}$ or N, and $R^{W5}$ is hydrogen, halogen, $-CH_3O$, $-CF_3$, $-CHF_2$, $-CH_2F$, $-NH_2$, $-N(CH_3)_2$, -CN, -OH, $-C(O)CH_3$, $-OC(O)CH_3$, $-C(O)OCH_3$ or $-C(O)NH_2$.

**120.**
A compound according to claim 113, its pharmaceutically acceptable salt, stereoisomer, isotopic isomer or tautomeric form thereof, wherein $X_6$ is $CR^{W6}$ or N, and $R^{W6}$ is hydrogen, halogen, $-CH_3O$, $-CF_3$, $-CHF_2$, $-CH_2F$, $-NH_2$, $-N(CH_3)_2$, -CN, -OH, $-C(O)CH_3$, $-OC(O)CH_3$, $-C(O)OCH_3$ or $-C(O)NH_2$.

**121.**
A compound according to claim 113, its pharmaceutically acceptable salt, stereoisomer, isotopic isomer or tautomeric form thereof, wherein $X_7$ is CH or N, preferably N.

**122.**
A compound according to claim 113, its pharmaceutically acceptable salt, stereoisomer, isotopic isomer or tautomeric form thereof, wherein the chemical bond between $X_5$ and $X_6$ is fused with ring A to form a group in the following structure:

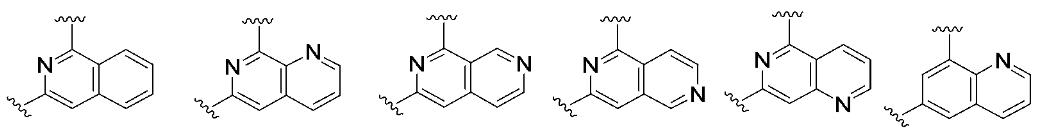

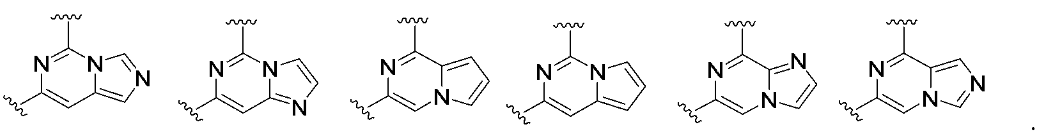

.

**123.**
A compound according to claim 113, its pharmaceutically acceptable salt, stereoisomer, isotopic isomer or tautomeric form thereof, wherein the chemical bond between $X_6$ and $X_7$ is fused with ring A to form a group in the following structure:

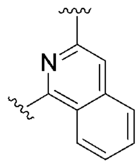 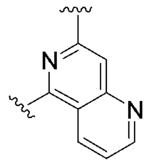 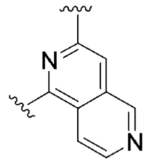 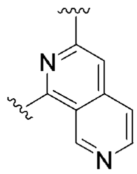 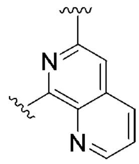 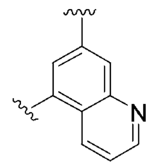

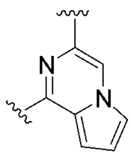 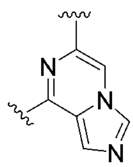 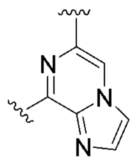 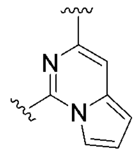 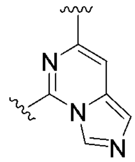 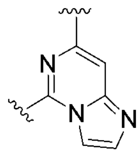 .

**124.**

A compound according to claim 113, its pharmaceutically acceptable salt, stereoisomer, isotopic isomer or tautomeric form thereof,

$L_1$ represents -C(O)NR$^a$-, -NR$^a$C(O)-,

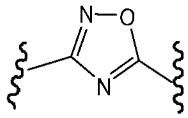 , 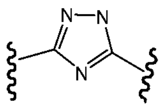 , 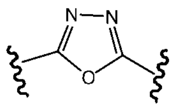 , 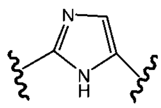 , 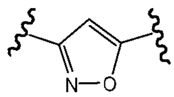 ,

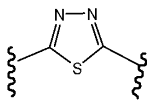 , 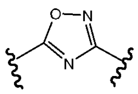 , 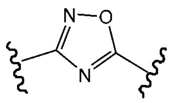 , 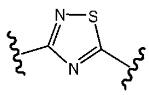 , 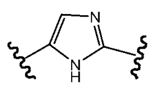 , 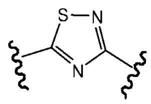 , 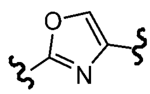 ,

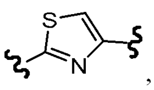 , 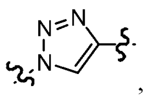 , 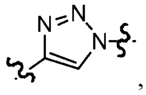 , 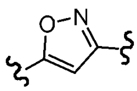 , 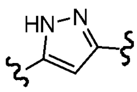 , 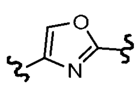 , 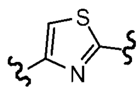 , 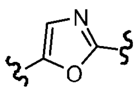 ,

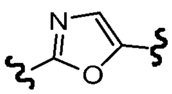 , 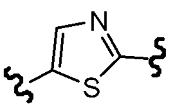 or 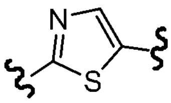 .

**125.**

A compound according to claim 113, its pharmaceutically acceptable salt, stereoisomer, isotopic isomer or tautomeric form thereof, wherein R$^L$ is $-NHS(O)_2CH_2CH_2OH$,

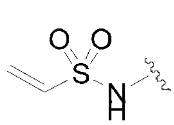 , 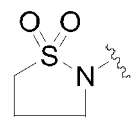 , 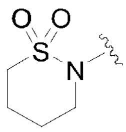 , 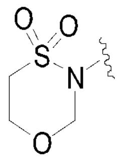 ,

$-NHS(O)_2CH_2CH_3$ or $-NHS(O)_2CH_3$.

**126.**

A compound having the structure of Formula (I-1-3), or pharmaceutically acceptable salts, stereoisomers, isotope isomers or tautomers thereof,

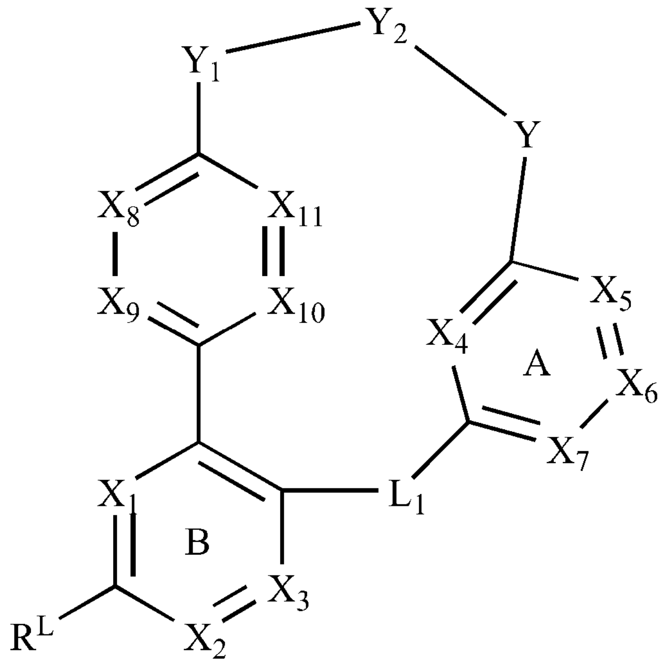

Formula (I-1-3)

wherein, $X_1$ represents $CR^{W1}$ or N;
wherein, $X_2$ represents $CR^{W2}$ or N;
wherein, $X_3$ represents $CR^{W3}$ or N;
wherein, $X_4$ represents $CR^{W4}$ or N;
wherein, $X_5$ represents $CR^{W5}$ or N;
wherein, $X_6$ represents $CR^{W6}$ or N;
wherein, $X_7$ represents $CR^{W7}$ or N;
wherein, $X_8$ represents $CR^{W8}$ or N;
wherein, $X_9$ represents $CR^{W9}$ or N;
wherein, $X_{10}$ represents $CR^{W10}$ or N;
wherein, $X_{11}$ represents $CR^{W11}$ or N;
wherein, $R^{W1}$, $R^{W2}$, $R^{W3}$, $R^{W4}$, $R^{W5}$, $R^{W6}$ and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -$S(O)R^a$, -O-$C_1$-$C_{10}$ haloalkyl, -$SR^a$, -$C(O)OR^a$, -$C(O)R^a$, -$OC(O)R^a$, -$NR^aC(O)R^b$ -$C(O)NR^aR^b$, -$NR^aC(O)OR^b$, -$OC(O)NR^aR^b$, -$OC(O)OR^a$, -$SF_5$, -$PO(R^a R^b)$, -$P(R^a R^b)$ or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{19}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{19}$ alkyl $P(R^a R^b)$ or -$C_0$-$C_{10}$ alkyl $NR^aR^b$;
wherein, $R^{W8}$, $R^{W9}$, $R^{W10}$ and $R^{W11}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -$S(O)R^a$, -O-$C_1$-$C_{10}$ haloalkyl, -$SR^a$, -$C(O)OR^a$, -$C(O)R^a$, -$OC(O)R^a$, -$NR^aC(O)R^b$, -$C(O)NR^aR^b$, -$NR^aC(O)OR^b$, -$OC(O)NR^aR^b$, -$OC(O)OR^a$, -$SF_5$, -$PO(R^a R^b)$, -$P(R^a R^b)$ or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;

alternatively, the chemical bond between $X_2$ and $X_3$ may be fused with ring B to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$, -C(O)$NR^aR^b$, -$NR^a$C(O)$OR^b$, -OC(O)$NR^aR^b$, -OC(O)$OR^a$, -$SF_5$, -PO($R^a$ $R^b$), -p($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)$OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^aR^b$), -$C_0$-$C_{10}$ alkyl P($R^a$ $R^b$) or -$C_0$-$C_{10}$ alkyl $NR^aR^b$;

alternatively, the chemical bond between $X_5$ and $X_6$ or between $X_6$ and $X_7$ may be fused with ring A to form a 4-8-membered saturated or unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$, -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)$OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;

wherein, $L_1$ represents -C(O)$NR^a$-, -$NR^a$C(O)- or a 5-6-membered heteroaromatic ring;

wherein, Y represents absence, -$CH_2$-, -O-, -$NR^a$-, -C(O)$NR^a$, -$NR^a$C(O)- or the following groups:

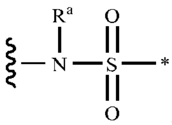
,
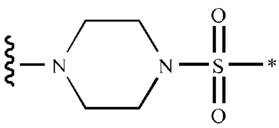

or $C_{y3}$;

wherein, * represents the site of connection with ring A; the wavy line represents the site of connection with $Y_2$;

wherein, $C_{y3}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein the substituents are each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^a$C(O)$R^b$, -C(O)$NR^aR^b$, -$NR^a$C(O)$OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{19}$ alkyl $NR^a$C(O)$OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^aR^b$) or -$C_0$-$C_{10}$ alkyl $NR^aR^b$;

wherein, $Y_1$ represents -$(CR^aR^b)_m$-; $Y_2$ represents -$(CR^aR^b)_n$-;

wherein, optionally -$CR^aR^b$- may be substituted with -O-, -S-, -Se-, -$NR^a$-, -$NR^aSO_2$-, -$NR^a$C(=O)-, -C(=O)$NR^a$-, -OC(=O)-, -C(=O)O-, -$SO_2NR^a$-, -S(=O)($NR^a$)-, -P(O)$(OR^a)_2$-, PO($R^a$ $R^b$), -$NR^a$P(O)$(OR^a)_2$- or -$NR^a$P(O)$(R^a)_2$-, -$CR^a$=$CR^b$-,

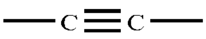, 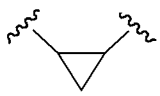, 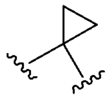, 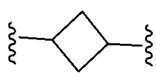, 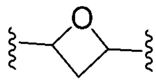, 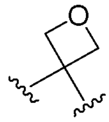,

5-6-membered heteroaryl,

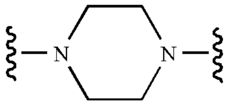, 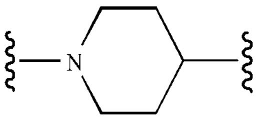 or 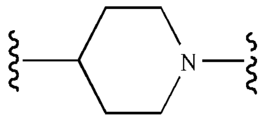;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^a$S(=O)(=NH)-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)_2$, -$NR^a$P(O)$(OR^a)_2$ or -$NR^a$P(O)$(R^a)_2$-;

wherein, $R^M$ represents $C_1$-$C_6$ alkyl or $C_3$-$C_6$ cycloalkyl, wherein the $C_1$-$C_6$ alkyl or $C_3$-$C_6$ cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;

alternatively, $L_2$-$R^M$ represents

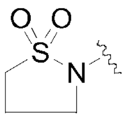 or 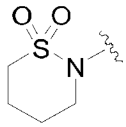;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, and N.

**127.**

A compound according to claim 126, its pharmaceutically acceptable salt, stereoisomer, isotopic isomer or tautomeric form thereof, wherein $X_1$ represents CH or N, preferably CH.

**128.**

A compound according to claim 126, its pharmaceutically acceptable salt, stereoisomer, isotopic isomer or tautomeric form thereof, wherein $X_2$ is CH or N, preferably CH.

**129.**

A compound according to claim 126, its pharmaceutically acceptable salt, stereoisomer, isotopic isomer or tautomeric form thereof, wherein $X_3$ is CH or N, preferably CH.

**130.**

A compound according to claim 126, its pharmaceutically acceptable salt, stereoisomer, isotopic isomer or tautomeric form thereof, wherein the chemical bond between $X_2$ and $X_3$ is fused with ring B to form a group in the following structure:

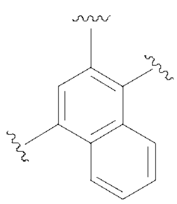 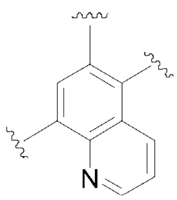 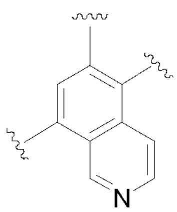 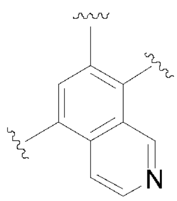 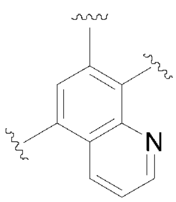 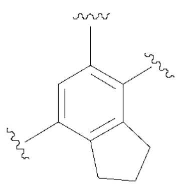

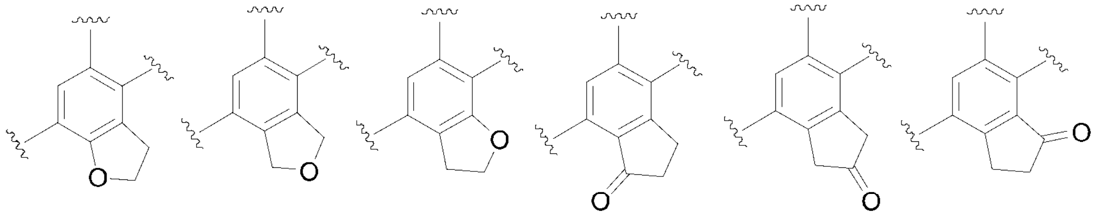

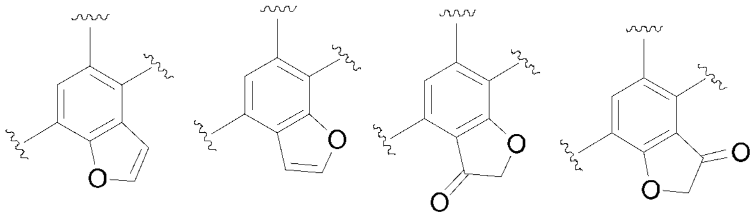

**131.**
A compound according to claim 126, its pharmaceutically acceptable salt, stereoisomer, isotopic isomer or tautomeric form thereof, wherein $X_4$ is CH or N, preferably N.

**132.**
A compound according to claim 126, its pharmaceutically acceptable salt, stereoisomer, isotopic isomer or tautomeric form thereof, wherein $X_5$ is $CR^{W5}$ or N, and $R^{W5}$ is hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

**133.**
A compound according to claim 126, its pharmaceutically acceptable salt, stereoisomer, isotopic isomer or tautomeric form thereof, wherein $X_6$ is $CR^{W6}$ or N, and $R^{W6}$ is hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

**134.**
A compound according to claim 126, its pharmaceutically acceptable salt, stereoisomer, isotopic isomer or tautomeric form thereof, wherein $X_7$ is CH or N, preferably N.

**135.**
A compound according to claim 126, its pharmaceutically acceptable salt, stereoisomer, isotopic isomer or tautomeric form thereof, wherein the chemical bond between $X_5$ and $X_6$ is fused with ring A to form a group in the following structure:

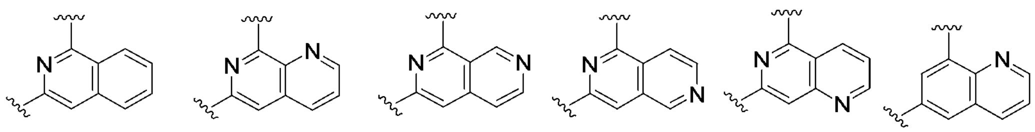

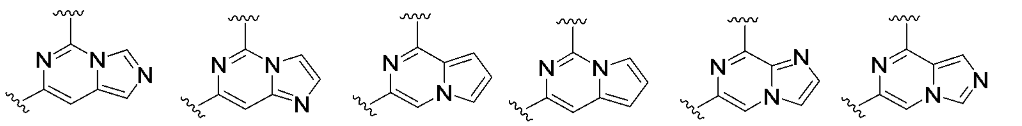

.

**136.**
A compound according to claim 126, its pharmaceutically acceptable salt, stereoisomer, isotopic isomer or tautomeric form thereof, wherein the chemical bond between $X_6$ and $X_7$ is fused with ring A to form a group in the following structure:

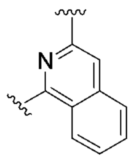 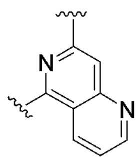 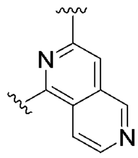 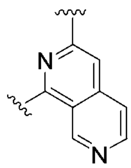 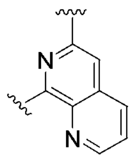 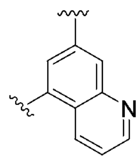

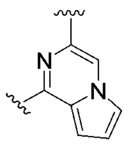 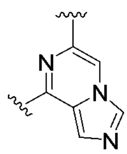 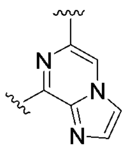 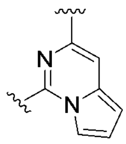 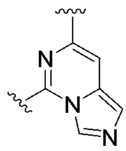 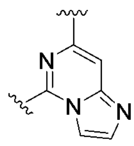 .

**137.**

A compound according to claim 126, its pharmaceutically acceptable salt, stereoisomer, isotopic isomer or tautomeric form thereof, wherein Y represents the following groups: absent, $-CH_2-$, -O-, -Se-, $-NR^a-$, $-C(O)NR^a$, $-NR^aC(O)-$ or the following groups:

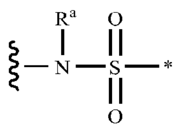 , 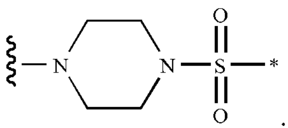 .

**138.**

A compound according to claim 126, its pharmaceutically acceptable salt, stereoisomer, isotopic isomer or tautomeric form thereof, wherein Y is -O- or -NH-.

**139.**

A compound according to claim 126, its pharmaceutically acceptable salt, stereoisomer, isotopic isomer or tautomeric form thereof, wherein Y is

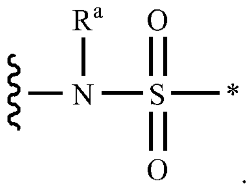 .

**140.**

A compound according to claim 126, its pharmaceutically acceptable salt, stereoisomer, isotopic isomer or tautomeric form thereof, wherein Y is

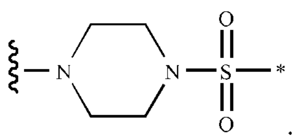 .

**141.**

A compound according to claim 126, its pharmaceutically acceptable salt, stereoisomer, isotopic isomer or tautomeric form thereof, wherein Y is $C_{y3}$ and $C_{y3}$ represents the following group:

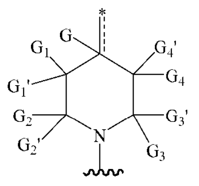, 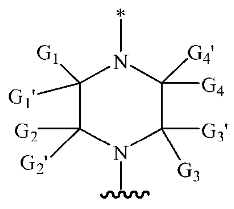, 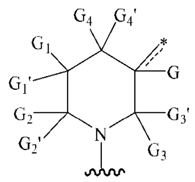, 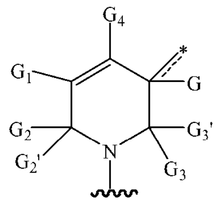, 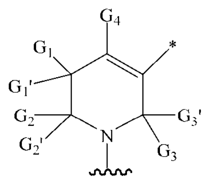,

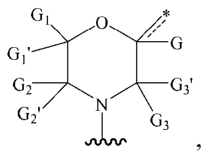, 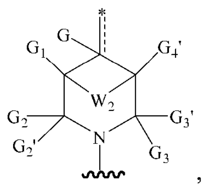, 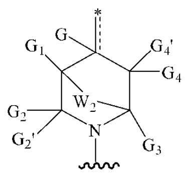, 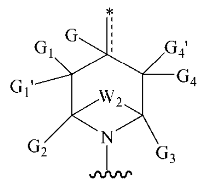, 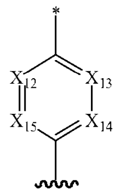, 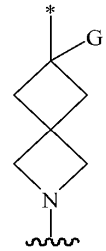, 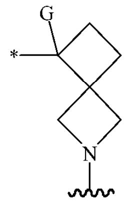,

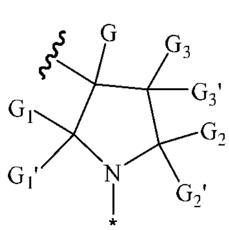, 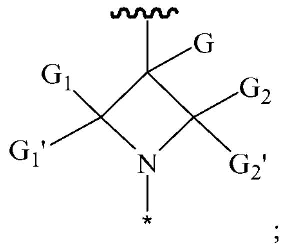;

wherein, when --- represents a double bond, G represents absence; wherein, when --- represents a single bond, G represents hydrogen, deuterium, $C_1$-$C_6$ alkyl, $C_2$-$C_6$ alkenyl, $C_2$-$C_6$ alkynyl, halogen, cyano, $C_1$-$C_6$ haloalkyl, $C_1$-$C_6$ haloalkoxy, hydroxyl or -C(O)$CH_3$;

wherein, $G_1$, $G_1$', $G_2$, $G_2$', $G_3$, $G_3$', $G_4$ and $G_4$' each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;

alternatively, pairs $G_1$ and $G_1$', pairs $G_2$ and $G_2$', pairs $G_3$ and $G_3$' and pairs $G_4$ and $G_4$', together with the carbon atom connected thereto, form a 3-6-membered (heterocyclic) ring, wherein the ring may optionally contain 0, 1 or 2 heteroatoms selected from O, S, N and Se; furthermore, the ring may be substituted with 0, 1, 2 or 3 substituents selected from halogen, $C_1$-$C_6$ alkyl, $C_1$-$C_6$ haloalkyl, $OR^a$ or oxo;

wherein, $W_2$ represents $-(CR^iR^j)_p-$;

wherein, $R^i$ and $R^j$ each independently represent hydrogen or $C_1$-$C_6$ alkyl;

wherein, p represents 1 or 2;

wherein, $X_{12}$ represents $CR^{W12}$ or N;

wherein, $X_{13}$ represents $CR^{W13}$ or N;

wherein, $X_{14}$ represents $CR^{W14}$ or N;

wherein, $X_{15}$ represents $CR^{W15}$ or N;

wherein, $R^{W12}$, $R^{W13}$, $R^{W14}$ and $R^{W15}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, -O-$C_1$-$C_{10}$ haloalkyl, $-SR^a$, $-C(O)OR^a$, $-C(O)R^a$, $-OC(O)R^a$, $-NR^aC(O)R^b$ $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl $S(O)R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl $C(O)OR^a$, -$C_0$-$C_{10}$ alkyl $C(O)R^a$, -$C_0$-$C_{10}$ alkyl $OC(O)R^a$, $C_0$-$C_{10}$ alkyl-$NR^aC(O)R^b$, -$C_0$-$C_{10}$ alkyl $C(O)NR^aR^b$, -$C_0$-$C_{10}$ alkyl $NR^aC(O)OR^b$, -$C_0$-$C_{10}$ alkyl $OC(O)NR^aR^b$, -$C_0$-$C_{10}$ alkyl $OC(O)OR^a$, -$C_0$-$C_{10}$ alkyl $SF_5$, -$C_0$-$C_{10}$ alkyl $PO(R^a R^b)$, -$C_0$-$C_{10}$ alkyl $P(R^a R^b)$ or -$C_0$-$C_{10}$ alkyl $NR^aR^b$;

**142.**

A compound according to claim 141, its pharmaceutically acceptable salt, stereoisomer, isotopic isomer or tautomeric form thereof, wherein $X_{12}$ is CH or N.

**143.**

A compound according to claim 141, its pharmaceutically acceptable salt, stereoisomer, isotopic isomer or tautomeric form thereof, wherein $X_{13}$ is CH or N.

**144.**

A compound according to claim 141, its pharmaceutically acceptable salt, stereoisomer, isotopic isomer or tautomeric form thereof, wherein $X_{14}$ is CH or N.

**145.**

A compound according to claim 141, its pharmaceutically acceptable salt, stereoisomer, isotopic isomer or tautomeric form thereof, wherein $X_{15}$ is CH or N.

**146.**

A compound according to claim 141, its pharmaceutically acceptable salt, stereoisomer, isotopic isomer or tautomeric form thereof, wherein $G_1$, $G_1'$, $G_2$, $G_2'$, $G_3$, $G_3'$, $G_4$ or $G_4'$ is hydrogen, respectively.

**147.**

A compound according to claim 141, its pharmaceutically acceptable salt, stereoisomer, isotopic isomer or tautomeric form thereof, wherein $W_2$ is $-CH_2$ or $-CH_2CH_2$.

**148.**

A compound according to claim 126, its pharmaceutically acceptable salt, stereoisomer, isotopic isomer or tautomeric form thereof,

$L_1$ represents $-C(O)NR^a-$, $-NR^aC(O)-$,

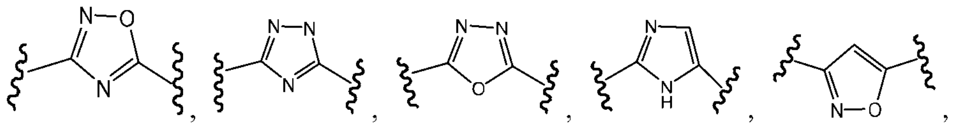

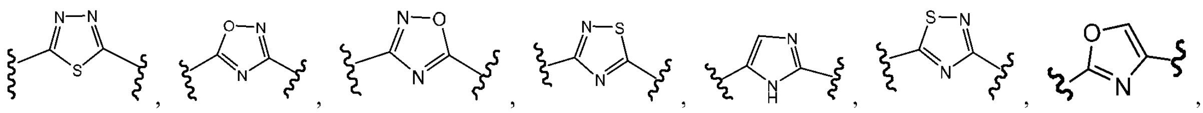

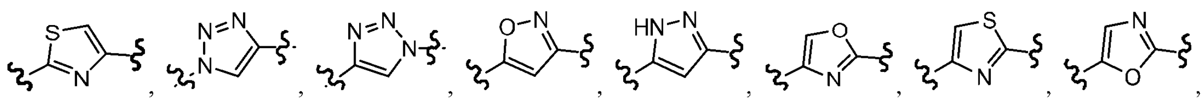

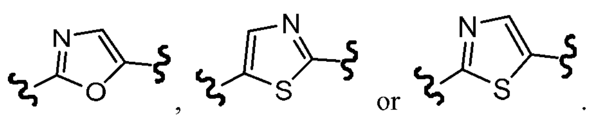

or

.

**149.**

A compound according to claim 126, its pharmaceutically acceptable salt, stereoisomer, isotopic isomer or tautomeric form thereof, wherein $L_1$ is -C(O)NH-.

**150.**

A compound according to claim 126, its pharmaceutically acceptable salt, stereoisomer, isotopic isomer or tautomeric form thereof, wherein $L_1$ is

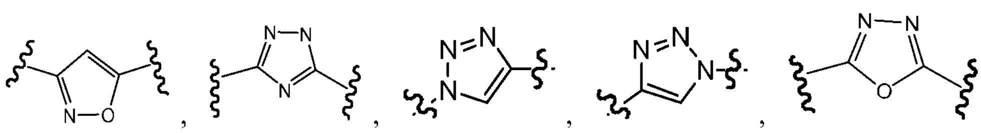

or

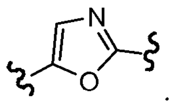

**151.**
A compound according to claim 126, its pharmaceutically acceptable salt, stereoisomer, isotopic isomer or tautomeric form thereof, wherein $Y_1$ is $-(CR^aR^b)_m-$.

**152.**
A compound according to claim 126, its pharmaceutically acceptable salt, stereoisomer, isotopic isomer or tautomeric form thereof, wherein $Y_1$ is -NR-C(=O)-, -O-C(=O)- or -C(=O)O-.

**153.**
A compound according to claim 126, its pharmaceutically acceptable salt, stereoisomer, isotopic isomer or tautomeric form thereof, wherein $Y_1$ is $-O(CR^aR^b)_m-$ or $-(CR^aR^b)$ mO-.

**154.**
A compound according to claim 126, its pharmaceutically acceptable salt, stereoisomer, isotopic isomer or tautomeric form thereof, wherein $Y_1$ is $-CR^a=CR^a-$ or $-CR^aR^b-CR^a=CR^b-$.

**155.**
A compound according to claim 126, its pharmaceutically acceptable salt, stereoisomer, isotopic isomer or tautomeric form thereof, wherein $Y_2$ is $-(CR^aR^b)_n-$.

**156.**
A compound according to claim 126, its pharmaceutically acceptable salt, stereoisomer, isotopic isomer or tautomeric form thereof, wherein $Y_2$ is $-NR^a-C(=O)-$.

**157.**
A compound according to claim 126, its pharmaceutically acceptable salt, stereoisomer, isotopic isomer or tautomeric form thereof, wherein $Y_2$ is $-O(CR^aR^b)_n-$ or $-(CR^aR^b)_nO-$.

**158.**
A compound according to claim 126, its pharmaceutically acceptable salt, stereoisomer, isotopic isomer or tautomeric form thereof, wherein $Y_2$ is $-CR^a=CR^a-$, $-CR^a=CR^b-CR^aR^b-$ or $-CR^aR^b-CR^a=CR^b-$.

**159.**
A compound according to claim 126, its pharmaceutically acceptable salt, stereoisomer, isotopic isomer or tautomeric form thereof, wherein $Y_2$ is pyrazolyl, oxazolyl, oxadiazolyl, thiazolyl, thiadiazolyl or triazolyl.

**160.**
A compound according to claim 126, its pharmaceutically acceptable salt, stereoisomer, isotopic isomer or tautomeric form thereof, wherein $-Y_1-Y_2-$ is $-CR^a=CR^a-$ or $-CR^aR^bCR^a=CR^b-$.

**161.**
A compound according to claim 126, its pharmaceutically acceptable salt, stereoisomer, isotopic isomer or tautomeric form thereof, wherein $-Y_1-Y_2-$ is $-CR^aR^b-NR^aC(O)-$.

**162.**
A compound according to claim 126, its pharmaceutically acceptable salt, stereoisomer, isotopic isomer or tautomeric form thereof, wherein $-Y_1-Y_2-$ is

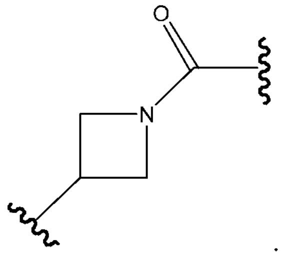

**163.**

A compound according to claim 126, its pharmaceutically acceptable salt, stereoisomer, isotopic isomer or tautomeric form thereof, wherein $R^L$ is $-NHS(O)_2CH_2CH_2OH$,

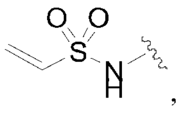 , 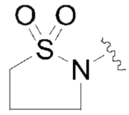 , 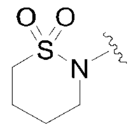 , 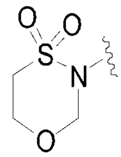 ,

$-NHS(O)_2CH_2CH_3$ or $-NHS(O)_2CH_3$.

**164.**

A compound having the structure of Formula (I-1-4), or pharmaceutically acceptable salts, stereoisomers, isotope isomers or tautomers thereof,

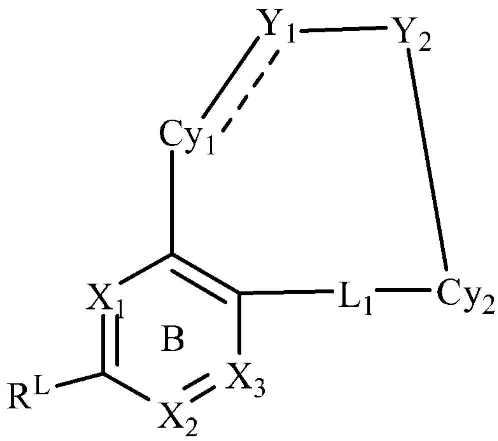

Formula (I-1-4)

wherein, $X_1$ represents $CR^{W1}$ or N;
wherein, $X_2$ represents $CR^{W2}$ or N;
wherein, $X_3$ represents $CR^{W3}$ or N;
wherein, $R^{W1}$, $R^{W2}$, and $R^{W3}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, $-OR^a$, $-SO_3R^a$, $-SO_2R^a$, $-S(O)R^a$, $-O-C_1-C_{10}$ haloalkyl, $-SR^a$, $-C(O)OR^a$, $-C(O)R^a$, $-OC(O)R^a$, $-NR^aC(O)R^b$, $-C(O)NR^aR^b$, $-NR^aC(O)OR^b$, $-OC(O)NR^aR^b$, $-OC(O)OR^a$, $-SF_5$, $-PO(R^a R^b)$, $-P(R^a R^b)$ or $-NR^aR^b$; $-C_0$-$C_{10}$ alkyl $OR^a$, $-C_0$-$C_{10}$ alkyl $SO_3R^a$, $-C_0$-$C_{10}$ alkyl $SO_2R^a$, $-C_0$-$C_{10}$ alkyl $S(O)R^a$, $-C_0$-$C_{10}$ alkyl O-$C_1$-$C_{10}$ haloalkyl, $-C_0$-$C_{10}$ alkyl $SR^a$, $-C_0$-$C_{10}$ alkyl $C(O)OR^a$, $-C_0$-$C_{10}$ alkyl $C(O)R^a$, $-C_0$-$C_{10}$ alkyl $OC(O)R^a$, $C_0$-$C_{10}$ alkyl-$NR^aC(O)R^b$, $-C_0$-$C_{10}$ alkyl $C(O)NR^aR^b$, $-C_0$-$C_{10}$ alkyl $NR^aC(O)OR^b$, $-C_0$-$C_{10}$ alkyl $OC(O)NR^aR^b$, $-C_0$-$C_{10}$ alkyl $OC(O)OR^a$, $-C_0$-$C_{10}$ alkyl $SF_5$, $-C_0$-$C_{10}$ alkyl $PO(R^a R^b)$, $-C_0$-$C_{10}$ alkyl $P(R^a R^b)$ or $-C_0$-$C_{10}$ alkyl $NR^aR^b$;
alternatively, the chemical bond between $X_2$ and $X_3$ may be fused with ring B to form a 4-8-membered saturated or

unsaturated (heterocyclic) ring, which may optionally contain 0, 1 or 2 heteroatoms selected from O, S, N and Se; the ring may be optionally substituted with 0-3 substituents each independently selected from the following: hydrogen, deuterium, halogen, cyano, oxo, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^aC(O)R^b$, -C(O)$NR^aR^b$, -$NR^aC(O)OR^b$, -OC(O)$NR^aR^b$, -OC(O)$OR^a$, -$SF_5$, -PO($R^a$ $R^b$), -P($R^a$ $R^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^aC(O)R^b$, -$C_0$-$C_{10}$ alkyl C(O)$NR^aR^b$, -$C_0$-$C_{10}$ alkyl $NR^aC(O)OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;

wherein, $C_{y1}$ each independently represent a substituted or unsubstituted 3-10-membered saturated or unsaturated cycloalkyl, a 6-10-membered saturated or unsaturated spirocycloalkyl, a 6-10-membered saturated or unsaturated bridged cycloalkyl, a 6-10-membered saturated or unsaturated fused cycloalkyl, a 3-10-membered saturated or unsaturated heterocycloalkyl, a 6-10-membered saturated or unsaturated spiroheterocycloalkyl, a 6-10-membered saturated or unsaturated bridged heterocycloalkyl, a 6-10-membered fused heterocycloalkyl, a 6-10-membered aryl, or a 5-10-membered heteroaryl, wherein substituents are each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)$OR^a$, -C(O)$R^a$, -OC(O)$R^a$, -$NR^aC(O)R^b$, -C(O)$NR^aR^b$, -$NR^aC(O)OR^b$, -OC(O)$NR^aR^b$, -OC(O)$OR^a$, -$SF_5$, -PO($R^a$ $R^b$), -P($R^aR^b$) or -$NR^aR^b$; -$C_0$-$C_{10}$ alkyl $OR^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl $SR^a$, -$C_0$-$C_{10}$ alkyl C(O)$OR^a$, -$C_0$-$C_{10}$ alkyl C(O)$R^a$, -$C_0$-$C_{10}$ alkyl OC(O)$R^a$, $C_0$-$C_{10}$ alkyl-$NR^aC(O)R^b$, -$C_0$-$C_{10}$ alkyl C(O)$NR^aR^b$, -$C_0$-$C_{10}$ alkyl $NR^aC(O)OR^b$, -$C_0$-$C_{10}$ alkyl OC(O)$NR^aR^b$, -$C_0$-$C_{10}$ alkyl OC(O)$OR^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;

wherein, $L_1$ represents -C(O)$NR^a$-, -$NR^aC(O)$- or a 5-6-membered heteroaromatic ring;

wherein, $Y_1$ represents -$(CR^aR^b)_m$-; $Y_2$ represents -$(CR^aR^b)_n$-;

wherein, optionally -$CR^aR^b$- may be substituted with -O-, -S-, -Se-, -$NR^a$-, -$NR^aSO_2$-, -$NR^aC(=O)$-, -C(=O)$NR^a$-, -OC(=O)-, -C(=O)O-, -$SO_2NR^a$-, -S(=O)($NR^a$)-, -P(O)$(OR^a)_2$-, PO($R^a$ $R^b$), -$NR^aP(O)(OR^a)_2$- or -$NR^aP(O)(R^a)_2$-, -$CR^a$=$CR^b$-,

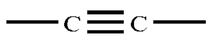, 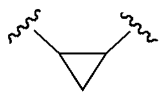, 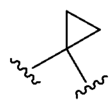, 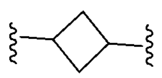, 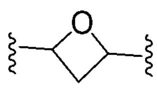, 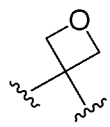,

5-6-membered heteroaryl,

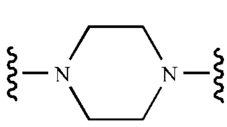, 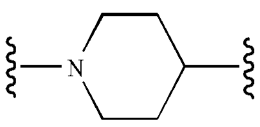 or 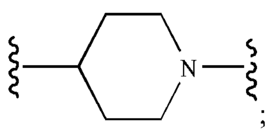;

wherein, $R^L$ represents $L_2$-$R^M$;

wherein, $L_2$ represents absence, -$C_1$-$C_6$ alkyl-, -$NR^a$-, -$NR^aSO_2$-, -$SO_2NR^a$-, -$NR^aS(=O)(=NH)$-, -S(=O) (=NH)-, -S-, -S(=O)-, -$SO_2$-, -$C_1$-$C_6$ alkyl-O-, -(C=O)-, -(C=O)$NR^a$-, -C=N(OH)-, -$NR^a$ (C=O), -P(O)$(OR^a)_2$, -$NR^aP(O)(OR^a)_2$ or -$NR^aP(O)(R^a)_2$-;

wherein, $C_{y2}$ represents

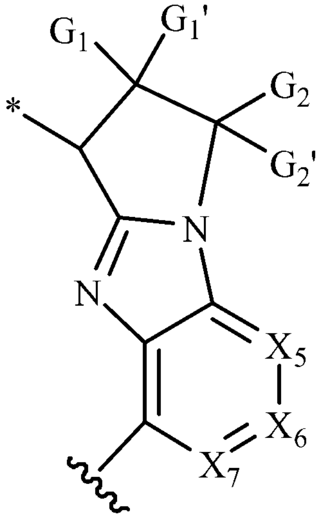
or
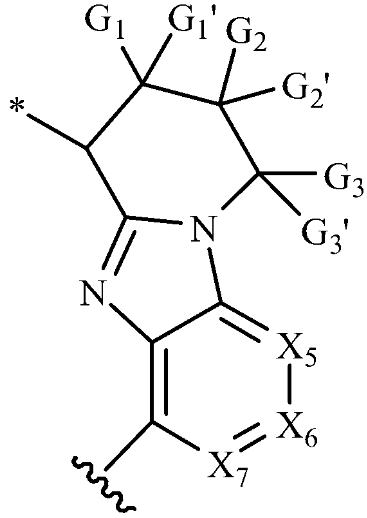
;

wherein, * represents the site of connection with $Y_2$, and the wavy line represents the site of connection with $L_1$;
wherein, $X_5$ represents $CR^{W5}$ or N;
wherein, $X_6$ represents $CR^{W6}$ or N;
wherein, $X_7$ represents $CR^{W7}$ or N;
wherein, $R^{W5}$, $R^{W6}$, and $R^{W7}$ each independently represent hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)O$R^a$, -C(O)$R^a$, -OC(O)$R^a$, -$NR^a$C(O)$R^b$, -C(O)$NR^aR^b$, -$NR^a$C(O)O$R^b$, -OC(O)$NR^aR^b$, -OC(O)O$R^a$, -$SF_5$, -PO($R^a$ $R^b$), -P($R^a$ $R^b$) or -$NR^aR^b$; -$C_0$-$C_{10}$ alkyl O$R^a$, -$C_0$-$C_{19}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl S$R^a$, -$C_0$-$C_{10}$ alkyl C(O)O$R^a$, -$C_0$-$C_{10}$ alkyl C(O)$R^a$, -$C_0$-$C_{10}$ alkyl OC(O) $R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)O$R^b$, -$C_0$-$C_{10}$ alkyl OC(O)$NR^aR^b$, -$C_0$-$C_{10}$ alkyl OC(O)O$R^a$, -$C_0$-$C_{10}$ alkyl $SF_5$, -$C_0$-$C_{10}$ alkyl PO($R^a$ $R^b$), -$C_0$-$C_{10}$ alkyl P($R^a$ $R^b$) or -$C_0$-$C_{10}$ alkyl $NR^aR^b$;
alternatively, the chemical bond between $X_5$ and $X_6$ or between $X_6$ and $X_7$ may be fused with ring A to form a 4-8-membered saturated or unsaturated ring, which may optionally contain 0, 1 or 2 heteroatoms selected from O, S, N and Se; the ring may be optionally substituted with 0-3 substituents each independently selected from the following: hydrogen, deuterium, halogen, cyano, nitro, azido, hydroxy ($C_1$-$C_{10}$) alkyl, $C_1$-$C_{10}$ alkyl, $C_2$-$C_{10}$ alkenyl, $C_2$-$C_{10}$ alkynyl, $C_1$-$C_{10}$ deuterated alkyl, $C_3$-$C_{12}$ cycloalkyl, $C_1$-$C_{10}$ haloalkyl, $C_2$-$C_{10}$ haloalkenyl, $C_2$-$C_{10}$ haloalkynyl, $C_3$-$C_{12}$ halocycloalkyl, 3-12-membered heterocyclyl, $C_6$-$C_{10}$ aryl, 5-10-membered heteroaryl, -$OR^a$, -$SO_3R^a$, -$SO_2R^a$, -S(O)$R^a$, -O-$C_1$-$C_{10}$ haloalkyl, -$SR^a$, -C(O)O$R^a$, -C(O)$R^a$, -OC(O)$R^a$, -$NR^a$C(O)$R^b$, -C(O)$NR^aR^b$, -$NR^a$C(O)O$R^b$, -OC(O)$NR^aR^b$, -OC(O)O$R^a$, -$SF_5$, -PO($R^a$ $R^b$), -P($R^a$ $R^b$) or -$NR^aR^b$; -$C_0$-$C_{10}$ alkyl O$R^a$, -$C_0$-$C_{10}$ alkyl $SO_3R^a$, -$C_0$-$C_{10}$ alkyl $SO_2R^a$, -$C_0$-$C_{10}$ alkyl S(O)$R^a$, -$C_0$-$C_{10}$ alkyl O-$C_1$-$C_{10}$ haloalkyl, -$C_0$-$C_{10}$ alkyl S$R^a$, -$C_0$-$C_{10}$ alkyl C(O)O$R^a$, -$C_0$-$C_{10}$ alkyl C(O)$R^a$, -$C_0$-$C_{10}$ alkyl OC(O)$R^a$, $C_0$-$C_{10}$ alkyl-$NR^a$C(O)$R^b$, -$C_0$-$C_{10}$ alkyl C(O)$NR^aR^b$, -$C_0$-$C_{10}$ alkyl $NR^a$C(O)O$R^b$, -$C_0$-$C_{10}$ alkyl OC(O)$NR^aR^b$, -$C_0$-$C_{10}$ alkyl OC(O)O$R^a$, -$C_0$-$C_{10}$ alkyl $SF_5$, -$C_0$-$C_{10}$ alkyl PO($R^a$ $R^b$), -$C_0$-$C_{19}$ alkyl P($R^a$ $R^b$) or -$C_0$-$C_{10}$ alkyl $NR^aR^b$;
wherein, $G_1$, $G_1'$, $G_2$, $G_2'$, $G_3$ and $G_3'$ each independently represent hydrogen, $C_1$-$C_6$ alkyl, halogen or $C_3$-$C_6$ cycloalkyl; hydroxy ($C_1$-$C_6$) alkyl or $C_1$-$C_6$ haloalkyl;
alternatively, pairs $G_1$ and $G_1'$, pairs $G_2$ and $G_2'$ and pairs $G_3$ and $G_3'$, together with the carbon atom connected thereto, form a 3-6-membered ring or a 3-6-membered heterocyclic ring, wherein the ring may optionally contain 0, 1 or 2 heteroatoms selected from O, S, N and Se; furthermore, the ring may be substituted with 0, **1, 2** or 3 substituents selected from halogen, $C_1$-$C_6$ alkyl, $C_1$-$C_6$ haloalkyl, $OR^a$ or oxo;
wherein, RM represents $C_1$-$C_6$ alkyl or $C_3$-$C_6$ (hetero) cycloalkyl, wherein the $C_1$-$C_6$ alkyl or $C_3$-$C_6$ (hetero) cycloalkyl can be optionally independently substituted with 0-3 substituents selected from halogen, $C_1$-$C_6$ alkyl, -$OR^a$, -$NR^aR^b$, cyano and -O-$C_1$-$C_6$ haloalkyl;
alternatively, $L_2$-$R^M$ represents

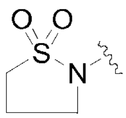 or 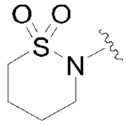 ;

wherein, m and n each independently represent an integer from 0 to 10;

wherein, $R^a$ and $R^b$ each independently represent hydrogen, halogen, $C_1$-$C_6$ alkyl, $C_3$-$C_6$ cycloalkyl, $C_1$-$C_6$ haloalkyl, hydroxy ($C_1$-$C_6$) alkyl, $C_1$-$C_6$ alkyl substituted with saturated or unsaturated 5-6-membered cycloalkyl, or $C_1$-$C_6$ alkyl substituted with saturated or unsaturated 5-6-membered heterocycloalkyl; alternatively, $R^a$ and $R^b$, together with the atom attached thereto, form a 3-6-membered saturated or unsaturated ring, or a 3-6-membered saturated or unsaturated heterocyclic ring, which may optionally contain 0-2 heteroatoms selected from O, S, Se and N.

**165.**
A compound according to claim 164, its pharmaceutically acceptable salt, stereoisomer, isotopic isomer or tautomeric form thereof, wherein $X_1$ represents CH or N, preferably CH.

**166.**
A compound according to claim 164, its pharmaceutically acceptable salt, stereoisomer, isotopic isomer or tautomeric form thereof, wherein $X_2$ is CH or N, preferably CH.

**167.**
A compound according to claim 164, its pharmaceutically acceptable salt, stereoisomer, isotopic isomer or tautomeric form thereof, wherein $X_3$ is CH or N, preferably CH.

**168.**
A compound according to claim 164, its pharmaceutically acceptable salt, stereoisomer, isotopic isomer or tautomeric form thereof, wherein the chemical bond between $X_2$ and $X_3$ is fused with ring B to form a group in the following structure:

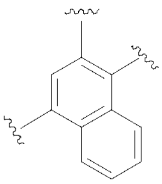 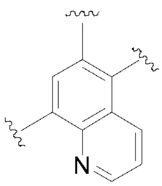 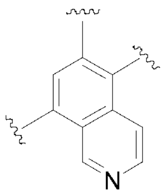 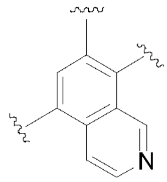 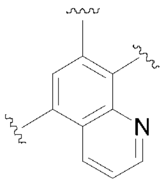 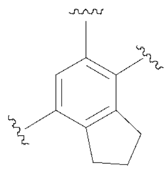

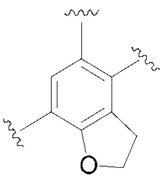 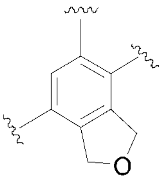 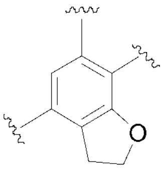 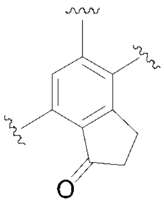 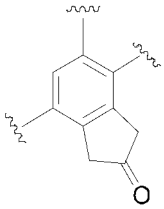 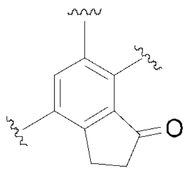

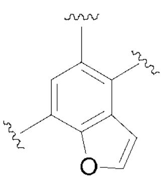 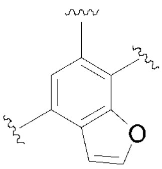 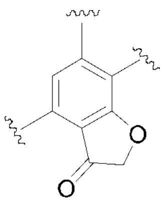 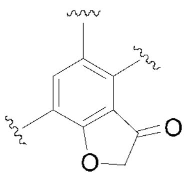

**169.**
A compound according to claim 164, its pharmaceutically acceptable salt, stereoisomer, isotopic isomer or tautomeric form thereof, wherein $X_5$ is $CR^{W5}$ or N, and $R^{W5}$ is hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

**170.**

A compound according to claim 164, its pharmaceutically acceptable salt, stereoisomer, isotopic isomer or tautomeric form thereof, wherein $X_6$ is $CR^{W6}$ or N, and $R^{W6}$ is hydrogen, halogen, $CH_3O$, $CF_3$, $CHF_2$, $CH_2F$, $NH_2$, $-N(CH_3)_2$, CN, -OH, $-C(O)CH_3$, $-OC(O)CH_3$, $-C(O)OCH_3$ or $-C(O)NH_2$.

**171.**

A compound according to claim 164, its pharmaceutically acceptable salt, stereoisomer, isotopic isomer or tautomeric form thereof, wherein $X_7$ is CH or N, preferably N.

**172.**

A compound according to claim 164, its pharmaceutically acceptable salt, stereoisomer, isotopic isomer or tautomeric form thereof, wherein the chemical bond between $X_5$ and $X_6$ is fused with ring A to form a group in the following structure:

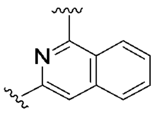 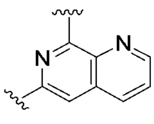 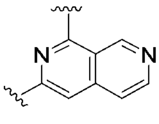 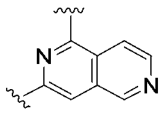 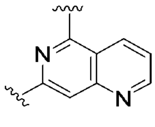 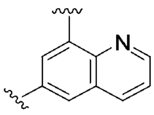

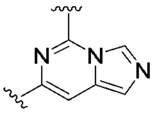 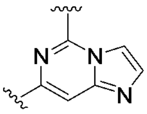 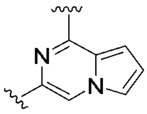 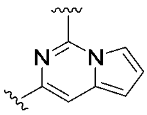 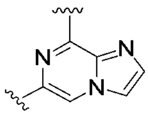 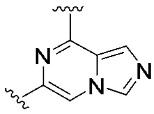 .

**173.**

A compound according to claim 164, its pharmaceutically acceptable salt, stereoisomer, isotopic isomer or tautomeric form thereof, wherein the chemical bond between $X_6$ and $X_7$ is fused with ring A to form a group in the following structure:

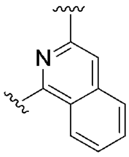 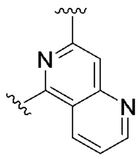 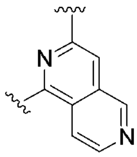 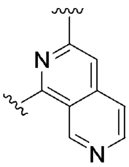 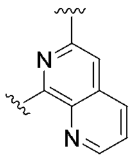 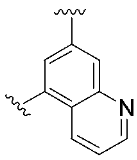

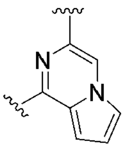 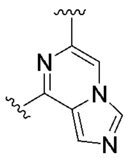 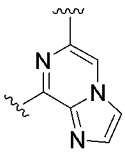 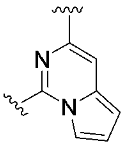 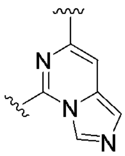 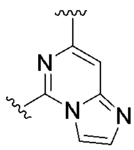 .

**174.**

A compound according to claim 164, its pharmaceutically acceptable salt, stereoisomer, isotopic isomer or tautomeric form thereof,

$L_1$ represents $-C(O)NR^a-$, $-NR^aC(O)-$,

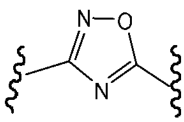, 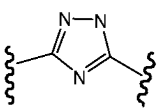, 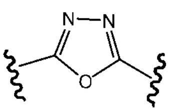, 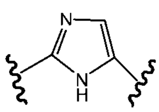, 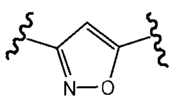,

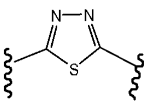, 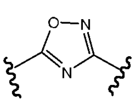, 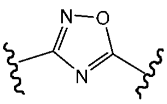, 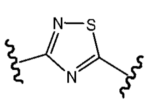, 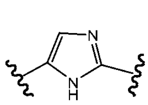, 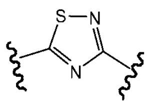, 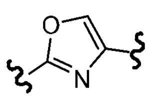,

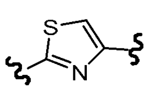, 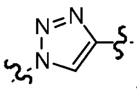, 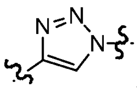, 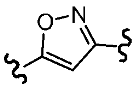, 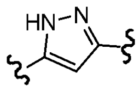, 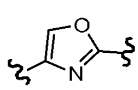, 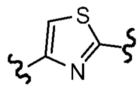, 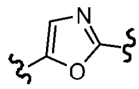,

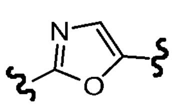, 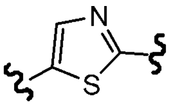 or 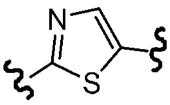.

**175.**
A compound according to claim 164, its pharmaceutically acceptable salt, stereoisomer, isotopic isomer or tautomeric form thereof, wherein $L_1$ is -C(O)NH-.

**176.**
A compound according to claim 164, its pharmaceutically acceptable salt, stereoisomer, isotopic isomer or tautomeric form thereof, wherein $L_1$ is

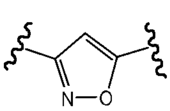, 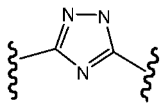, 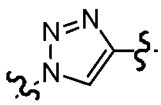, 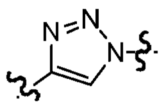, 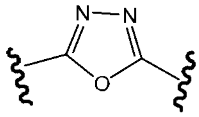

or

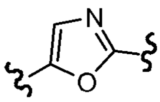.

**177.**
A compound according to claim 164, its pharmaceutically acceptable salt, stereoisomer, isotopic isomer or tautomeric form thereof, wherein $Y_1$ is $-(CR^aR^b)_m-$.

**178.**
A compound according to claim 164, its pharmaceutically acceptable salt, stereoisomer, isotopic isomer or tautomeric form thereof, wherein $Y_1$ is -NR-C(=O)-, -O-C(=O)- or -C(=O)O-.

**179.**
A compound according to claim 164, its pharmaceutically acceptable salt, stereoisomer, isotopic isomer or tautomeric form thereof, wherein $Y_1$ is $-O(CR^aR^b)_m-$ or $-(CR^aR^b)_mO-$.

**180.**
A compound according to claim 164, its pharmaceutically acceptable salt, stereoisomer, isotopic isomer or tautomeric form thereof, wherein $Y_1$ is $-CR^a=CR^a-$ or $-CR^aR^b-CR^a=CR^b-$.

**181.**
A compound according to claim 164, its pharmaceutically acceptable salt, stereoisomer, isotopic isomer or tautomeric form thereof, wherein $Y_2$ is $-(CR^aR^b)_n-$.

**182.**
A compound according to claim 164, its pharmaceutically acceptable salt, stereoisomer, isotopic isomer or tautomeric form thereof, wherein $Y_2$ is $-NR^a-C(=O)-$.

**183.**
A compound according to claim 164, its pharmaceutically acceptable salt, stereoisomer, isotopic isomer or tautomeric form thereof, wherein $Y_2$ is $-O(CR^aR^b)_n-$ or $-(CR^aR^b)_nO-$.

**184.**

A compound according to claim 164, its pharmaceutically acceptable salt, stereoisomer, isotopic isomer or tautomeric form thereof, wherein $Y_2$ is $-CR^a=CR^a-$, $-CR^a=CR^b-CR^aR^b-$ or $-CR^aR^b-CR^a=CR^b-$.

**185.**
A compound according to claim 164, its pharmaceutically acceptable salt, stereoisomer, isotopic isomer or tautomeric form thereof, wherein $Y_2$ is pyrazolyl, oxazolyl, oxadiazolyl, thiazolyl, thiadiazolyl or triazolyl.

**186.**
A compound according to claim 164, its pharmaceutically acceptable salt, stereoisomer, isotopic isomer or tautomeric form thereof, wherein $-Y_1-Y_2-$ is $-CR^a=CR^a-$ or $-CR^aR^bCR^a=CR^b-$.

**187.**
A compound according to claim 164, its pharmaceutically acceptable salt, stereoisomer, isotopic isomer or tautomeric form thereof, wherein $-Y_1-Y_2-$ is $-CR^aR^b-NR^aC(O)-$.

**188.**
A compound according to claim 164, its pharmaceutically acceptable salt, stereoisomer, isotopic isomer or tautomeric form thereof, wherein $-Y_1-Y_2-$ is

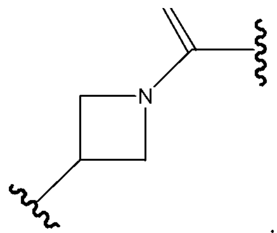

.

**189.**
A compound according to claim 164, its pharmaceutically acceptable salt, stereoisomer, isotopic isomer or tautomeric form thereof, wherein $R^L$ is $-NHS(O)_2CH_2CH_2OH$,

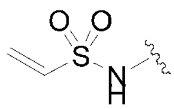 , 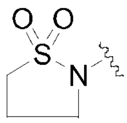 , 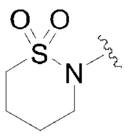 , 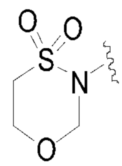 ,

$-NHS(O)_2CH_2CH_3$ or $-NHS(O)_2CH_3$.

**190.**
Compounds with the following structures:

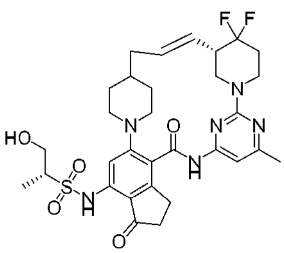 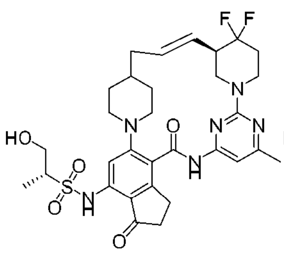 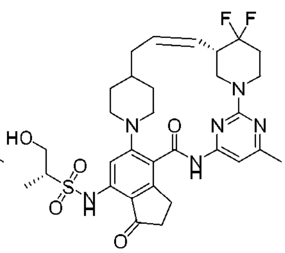 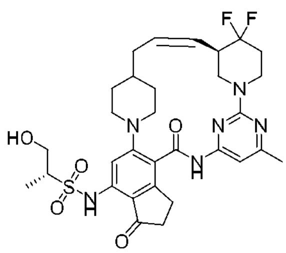

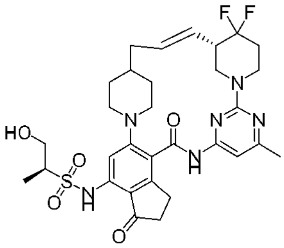
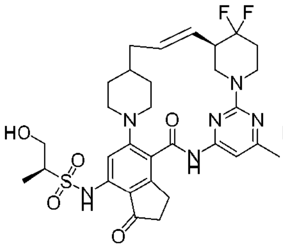
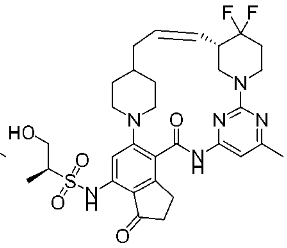
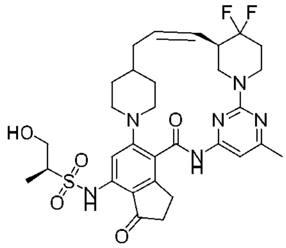
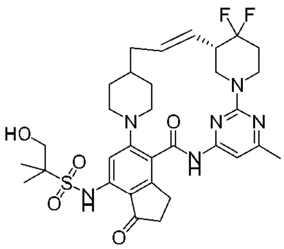
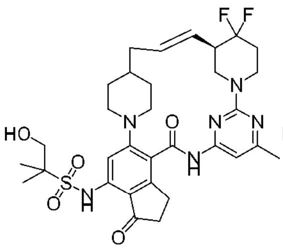
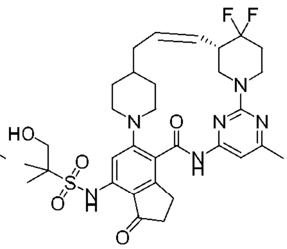
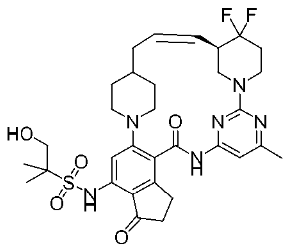
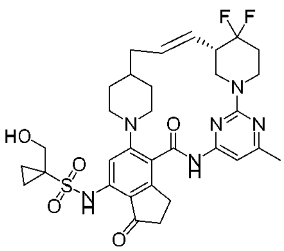
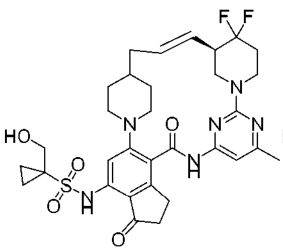
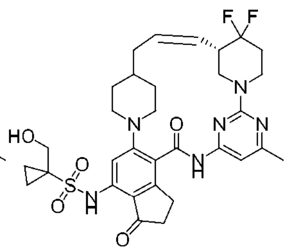
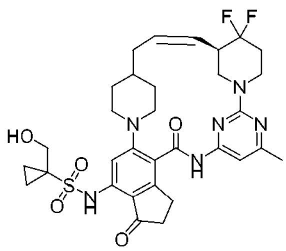
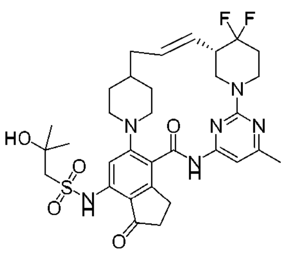
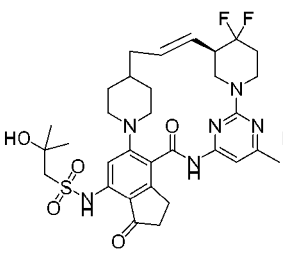
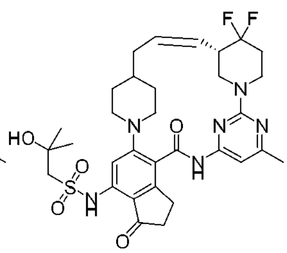
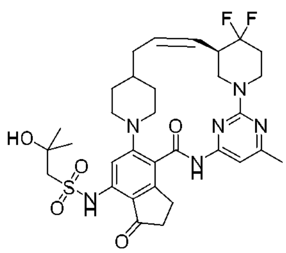
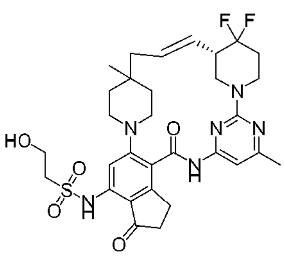
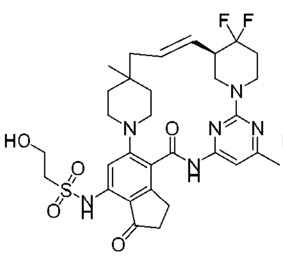
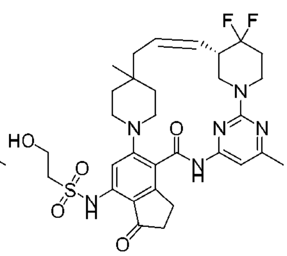
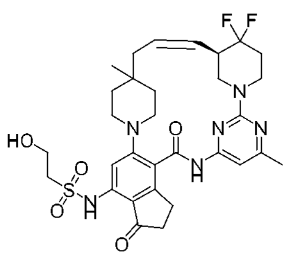
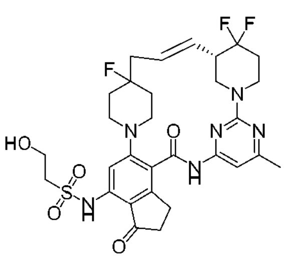
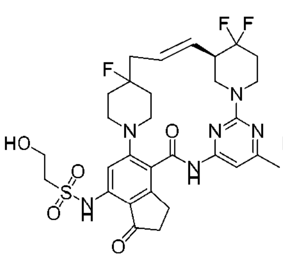
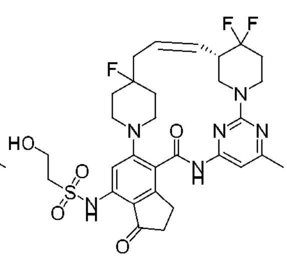
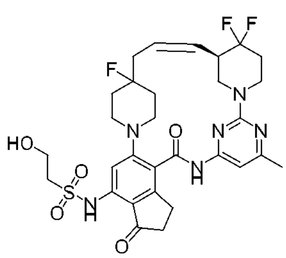

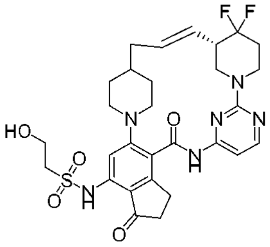
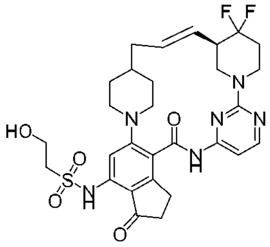
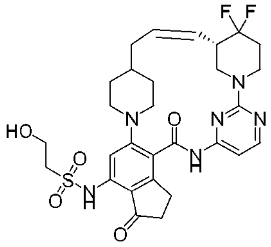
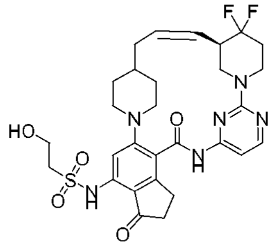

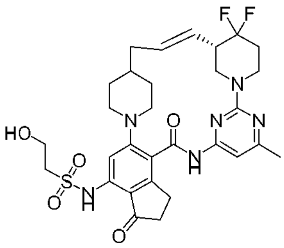
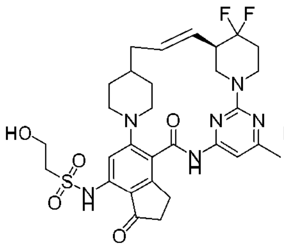
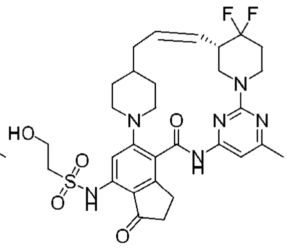
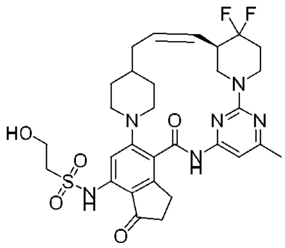

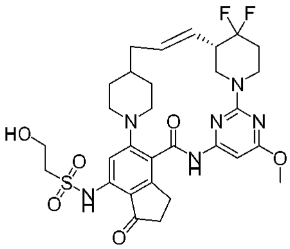
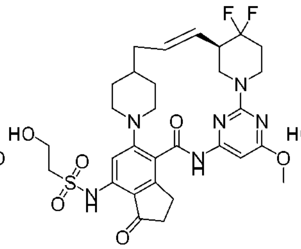
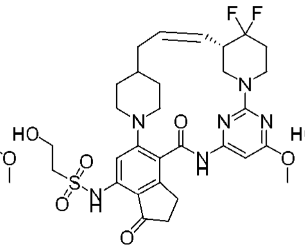
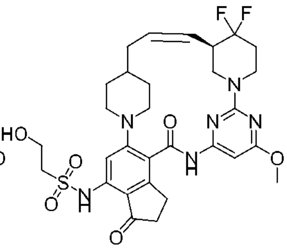

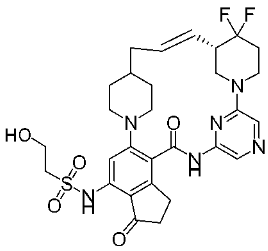
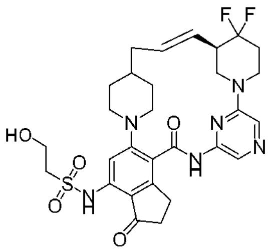
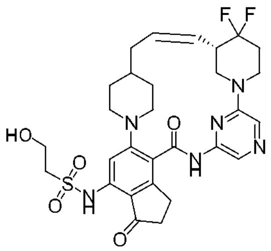
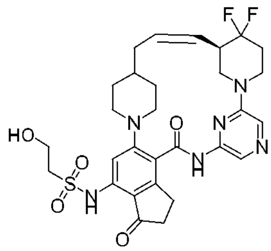

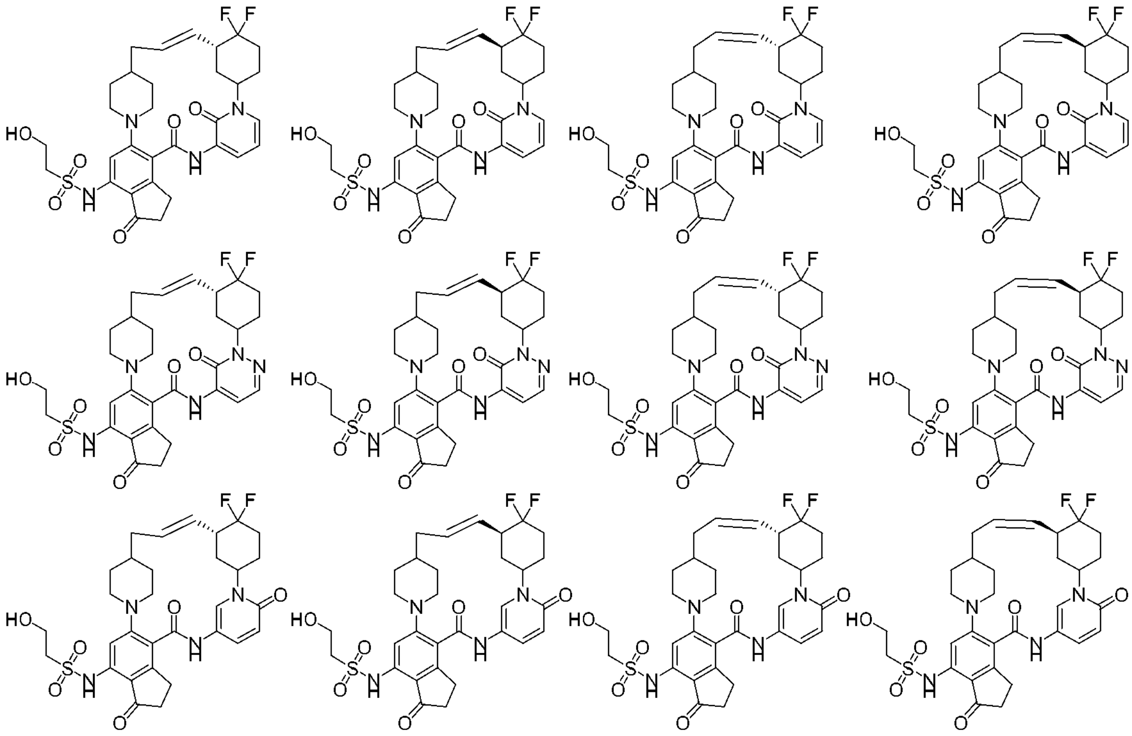

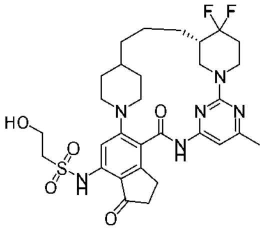
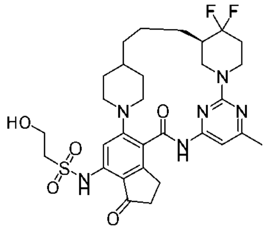
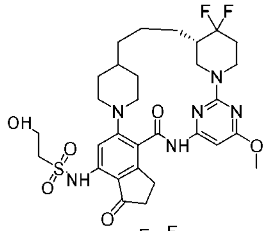
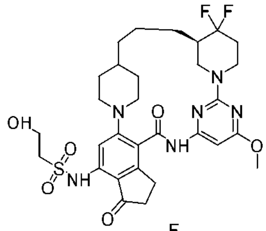
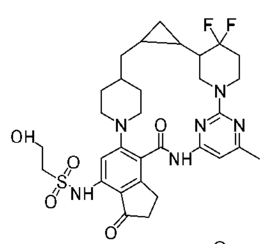
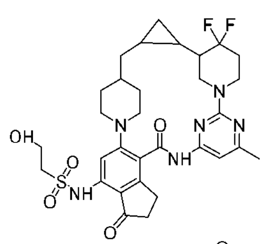
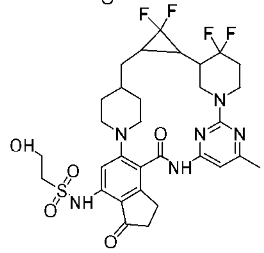
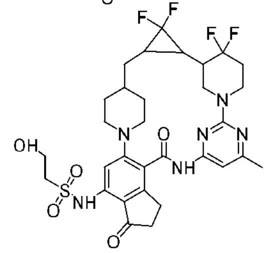
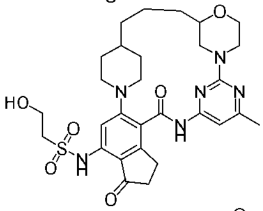
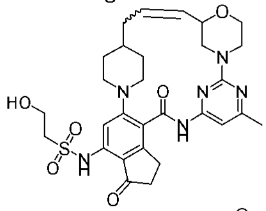
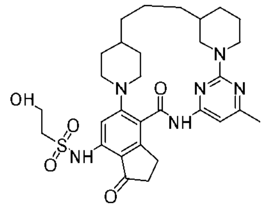
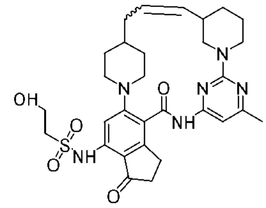
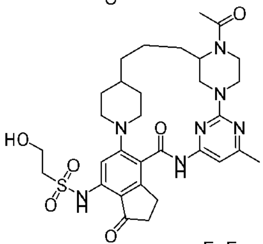
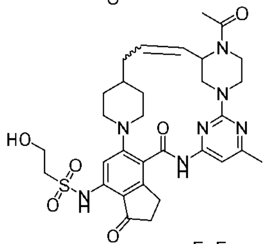
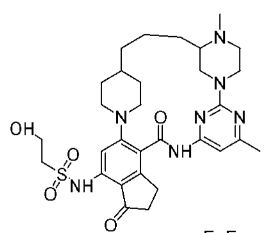
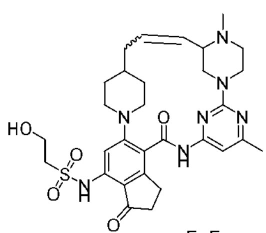
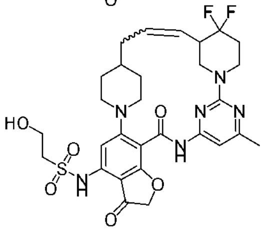
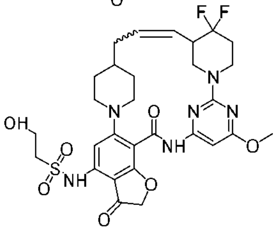
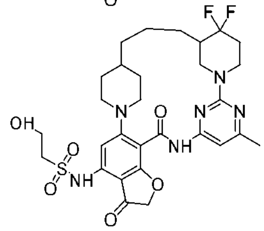
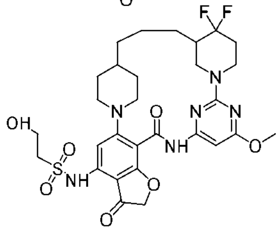
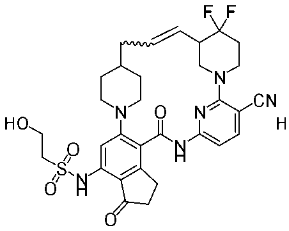
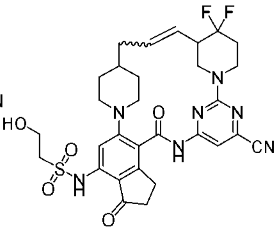
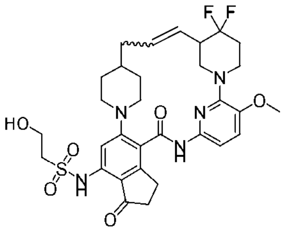
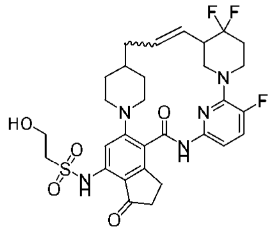

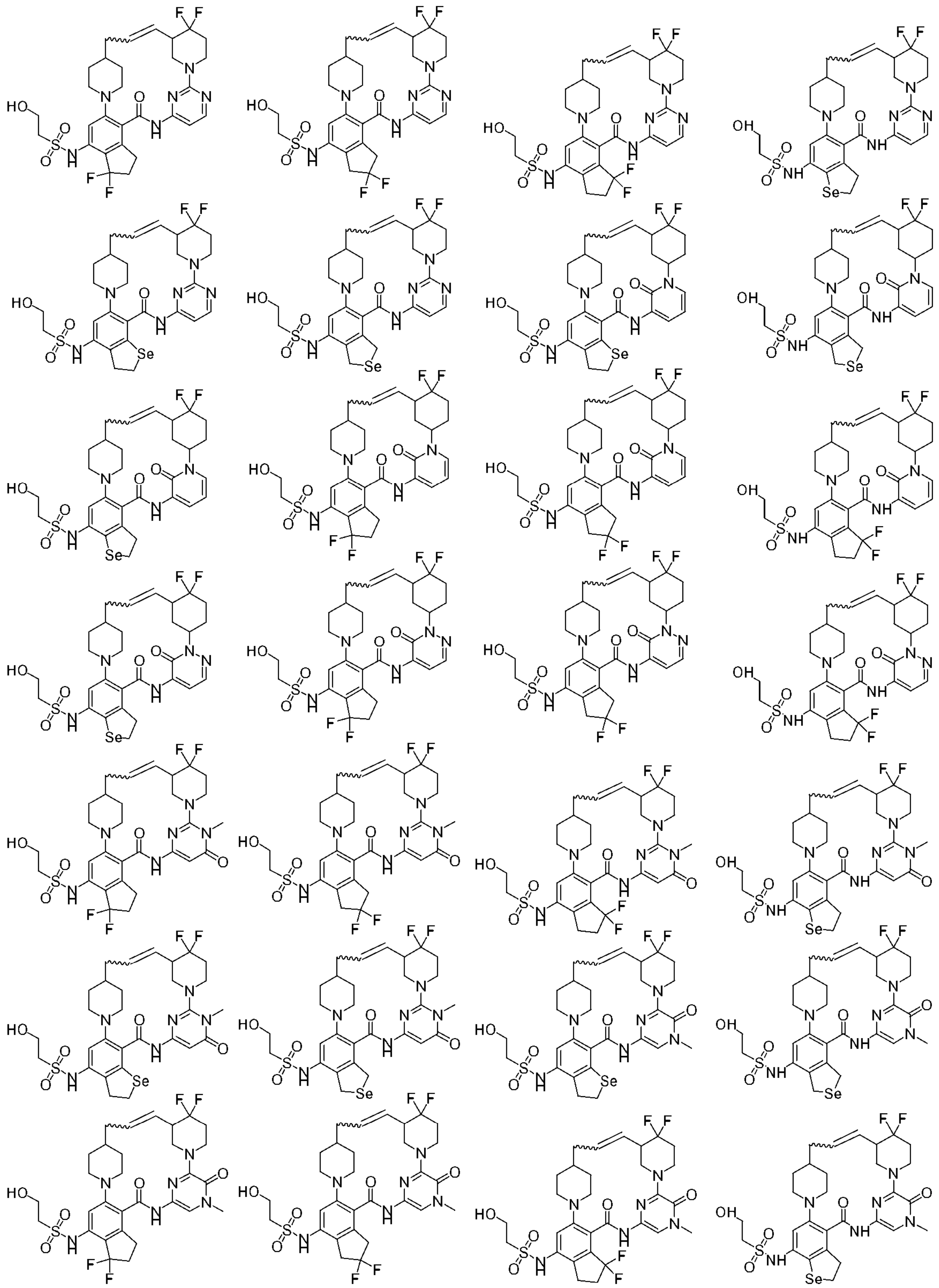

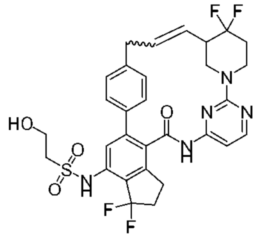
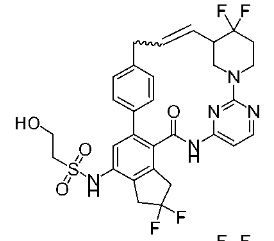
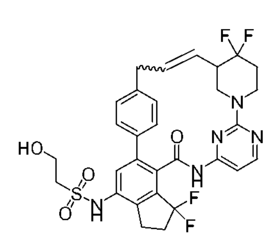
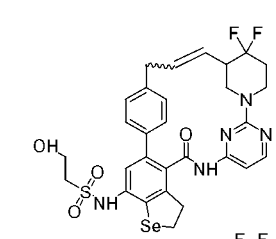
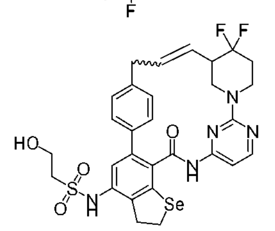
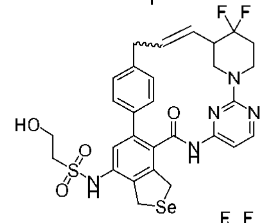
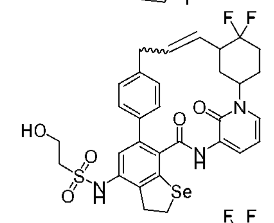
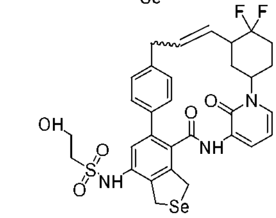
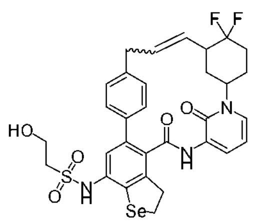
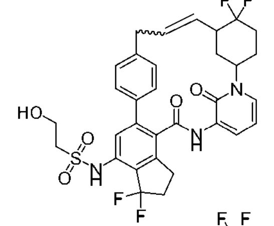
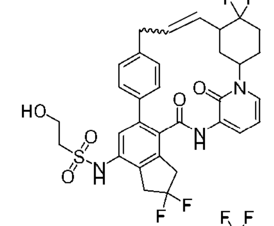
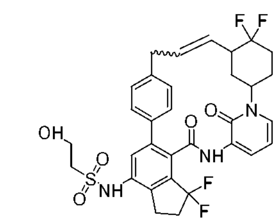
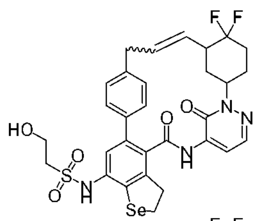
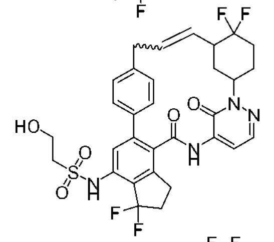
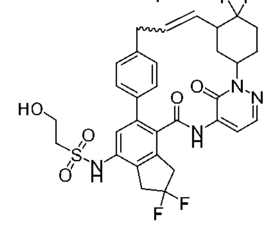
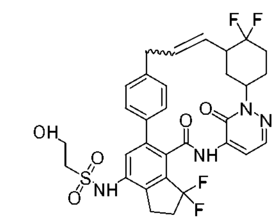
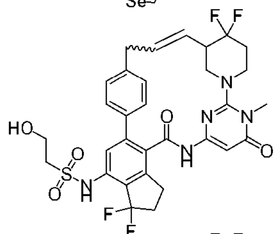
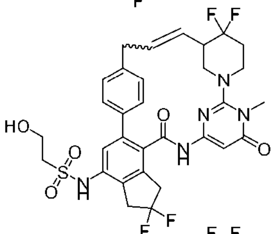
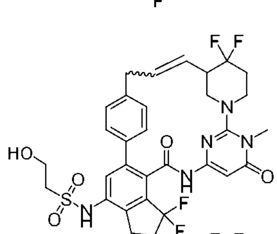
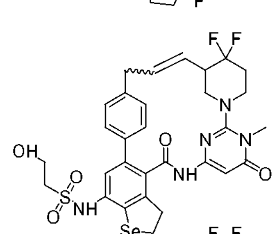
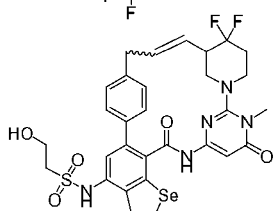
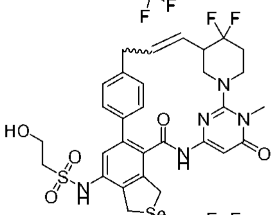
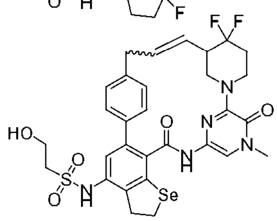
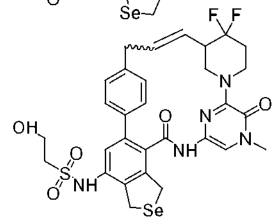
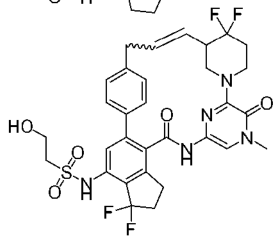
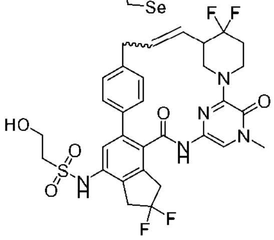
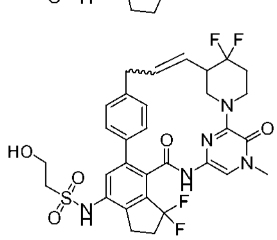
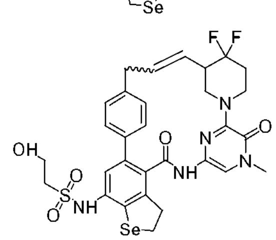
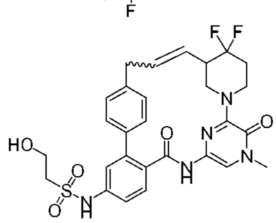
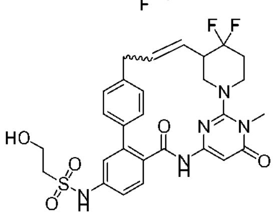
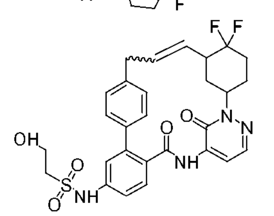
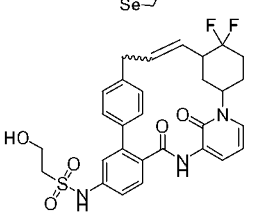

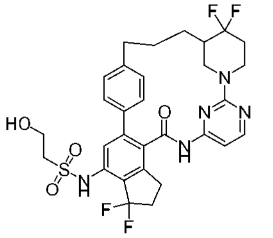
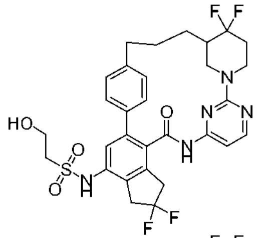
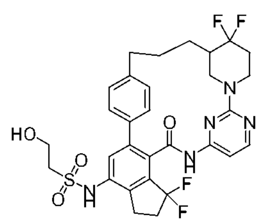
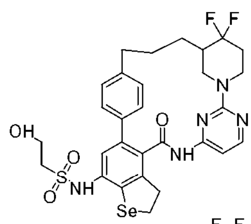

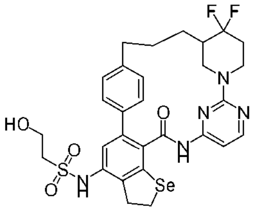
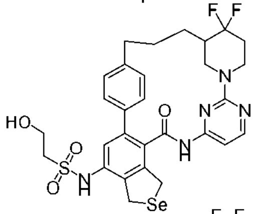
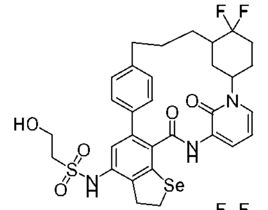
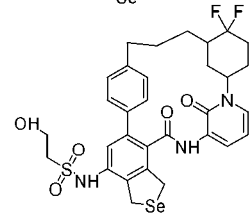

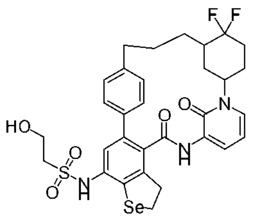
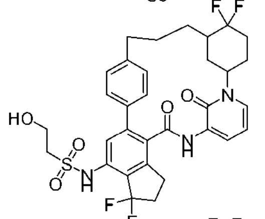
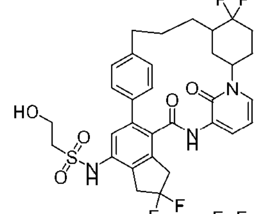
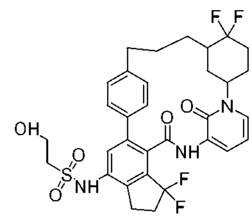

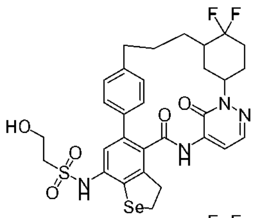
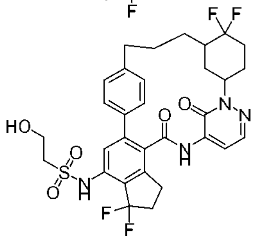
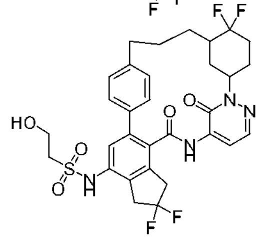
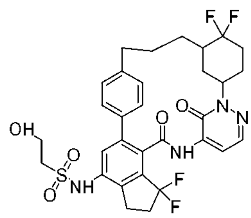

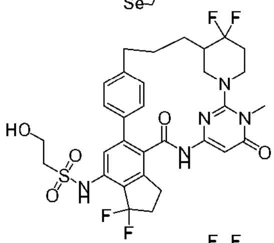
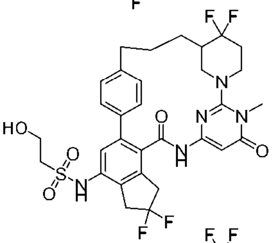
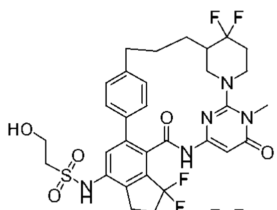
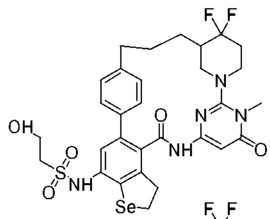

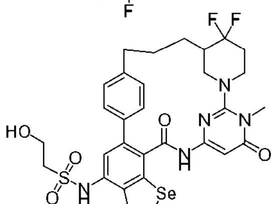
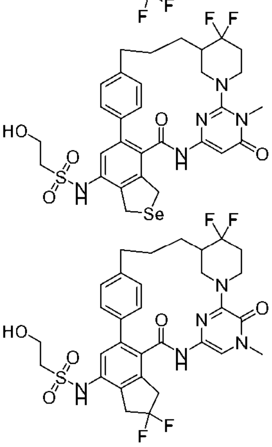
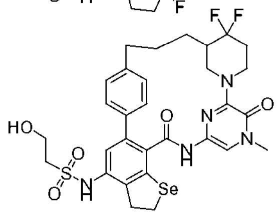
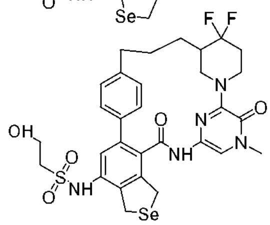

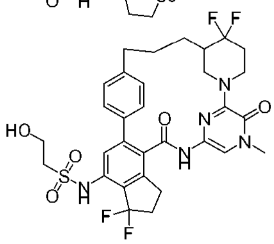
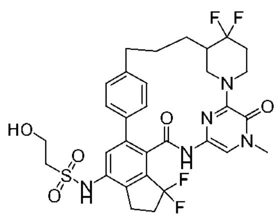
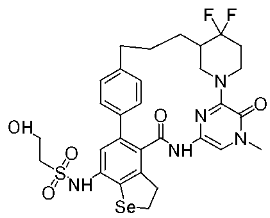

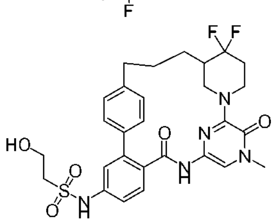
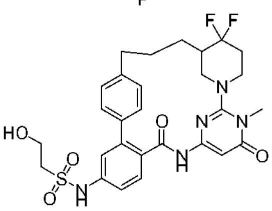
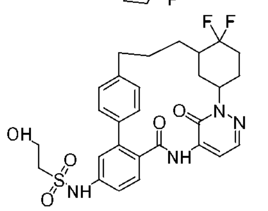
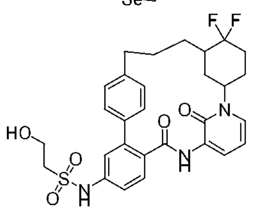

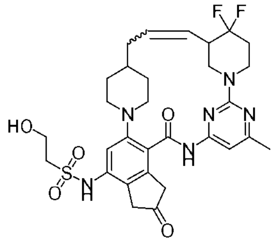
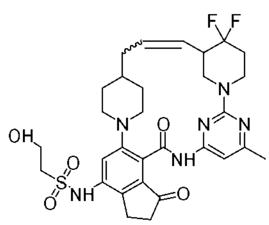
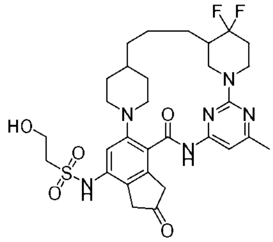
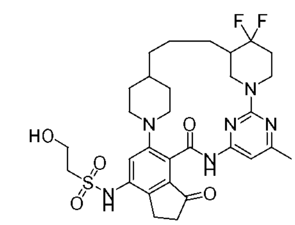
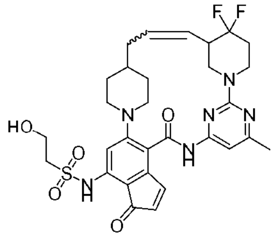
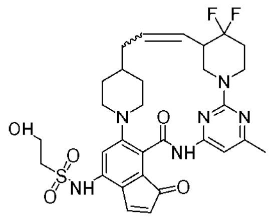
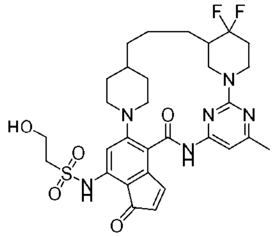
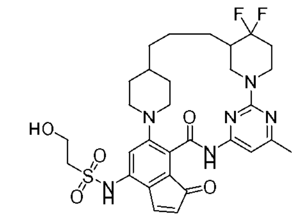
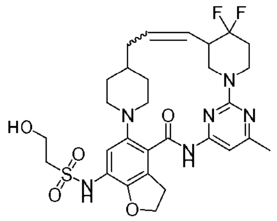
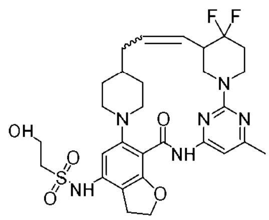
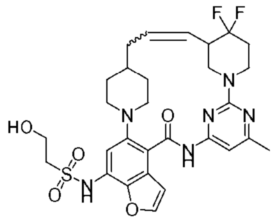
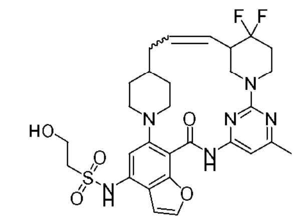
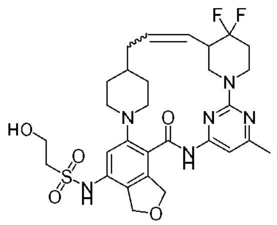
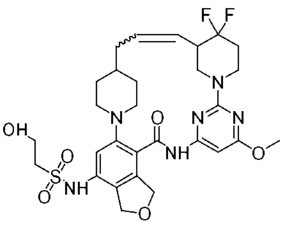
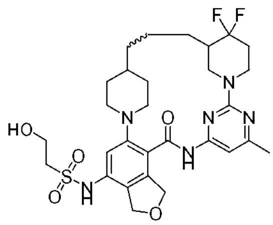
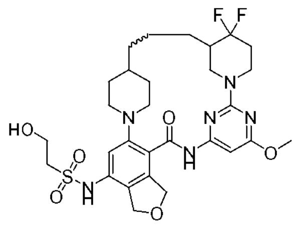
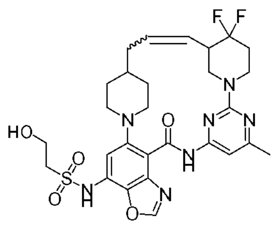
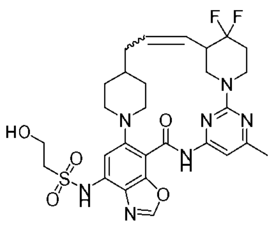
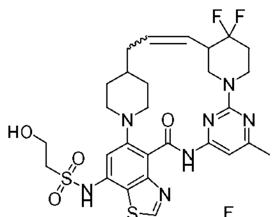
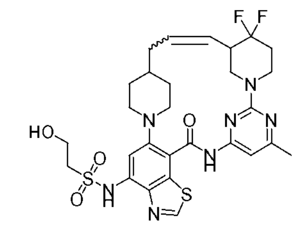
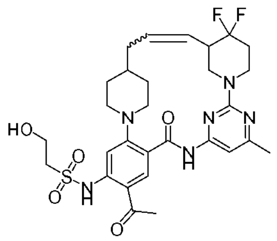
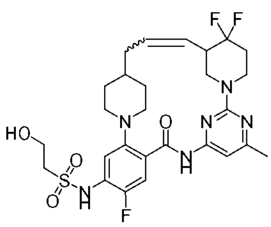
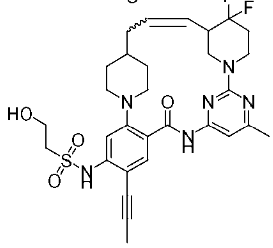
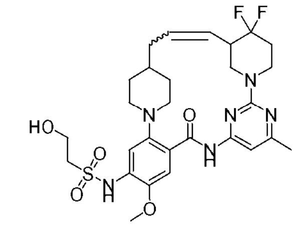
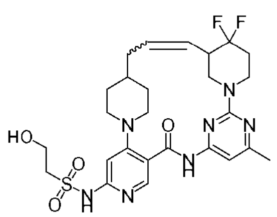
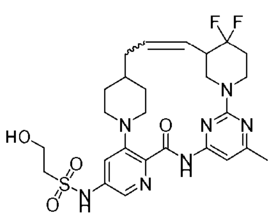
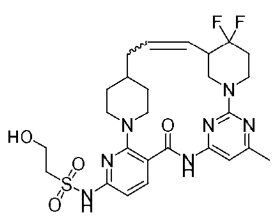
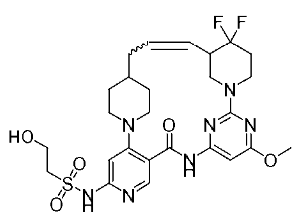

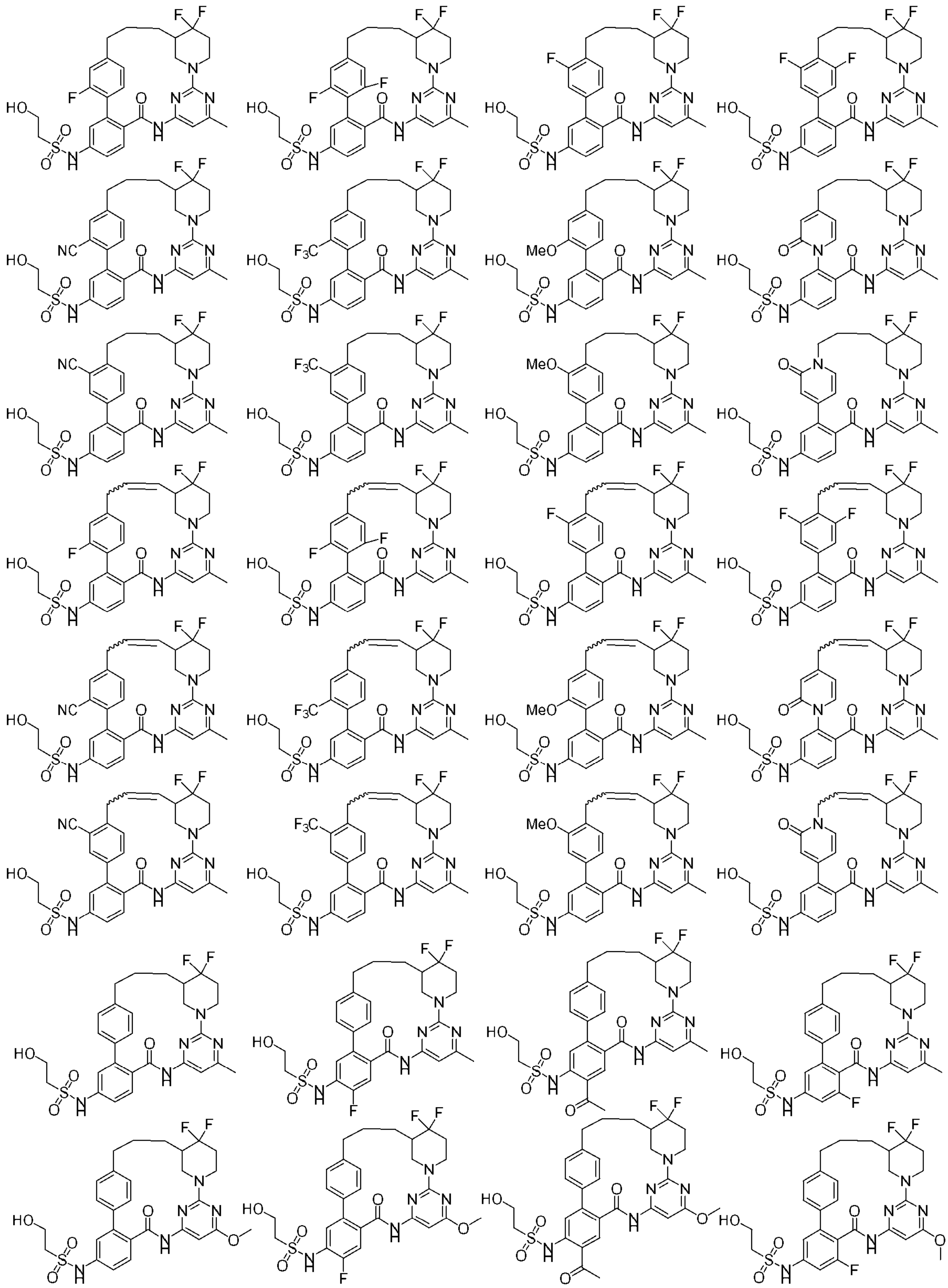

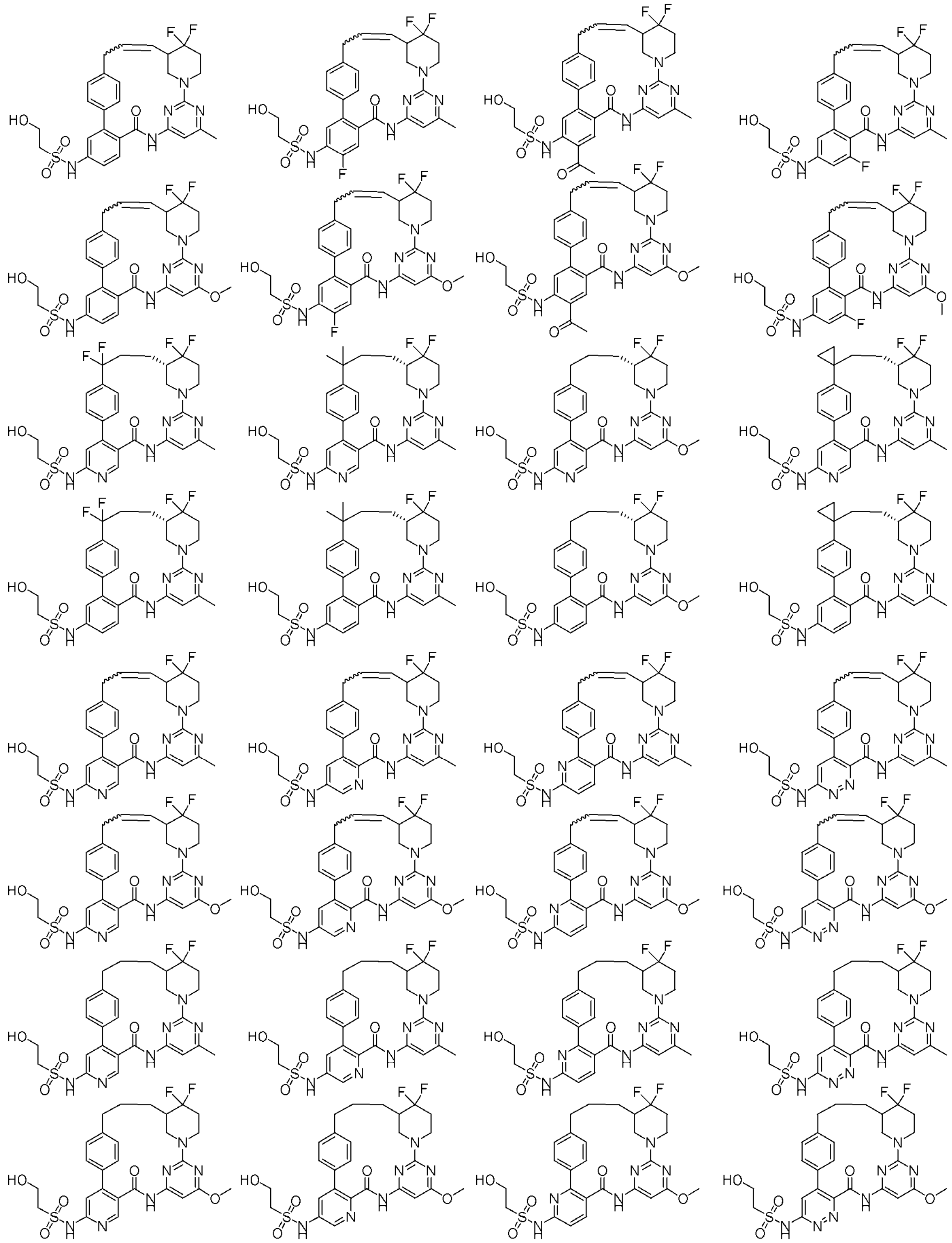

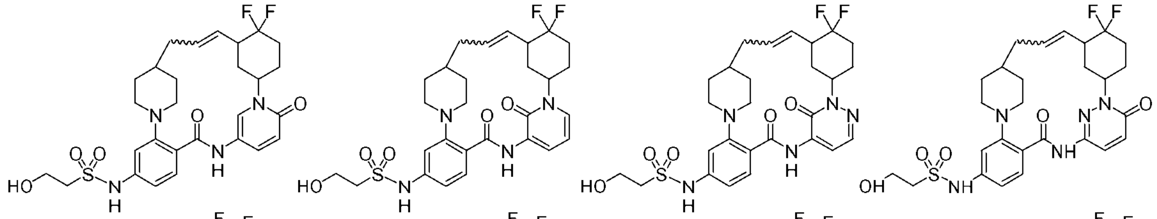

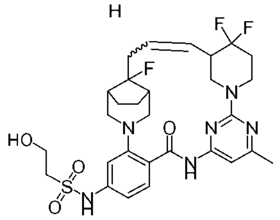
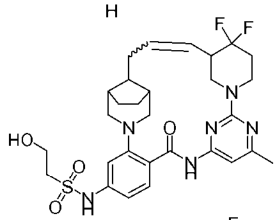
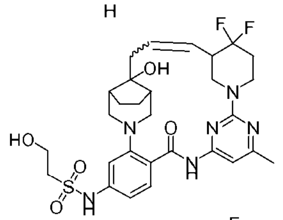
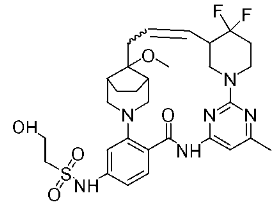

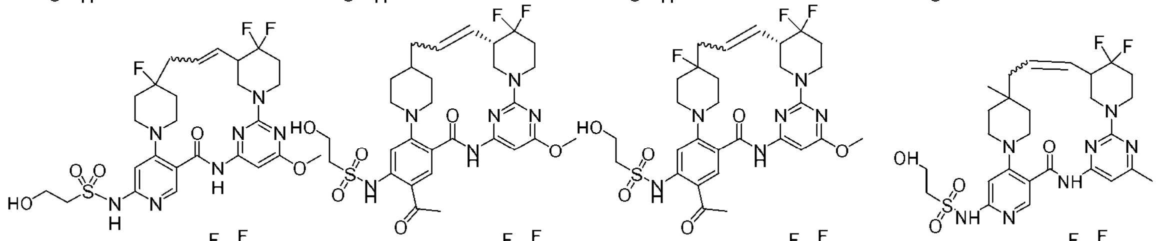

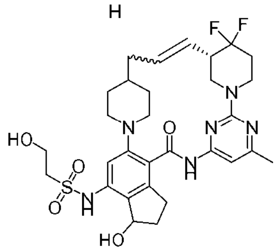
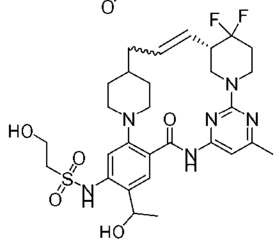
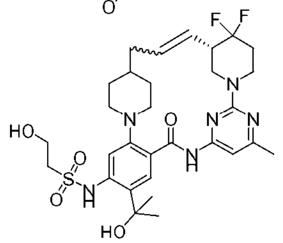
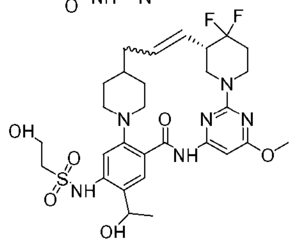

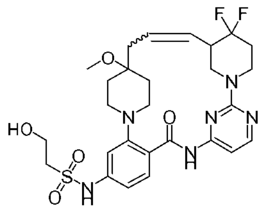
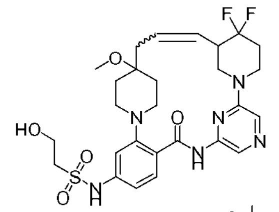
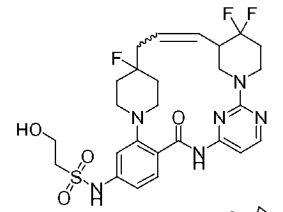
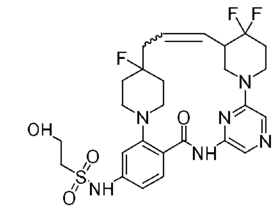

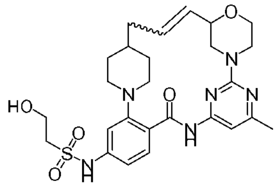
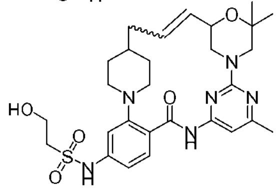
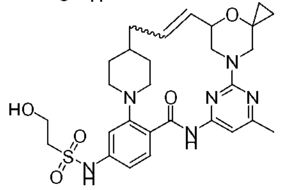
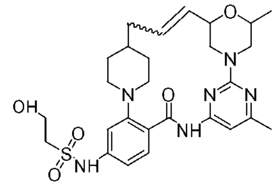

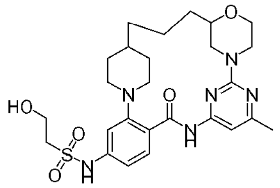
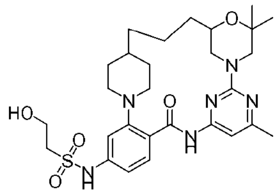
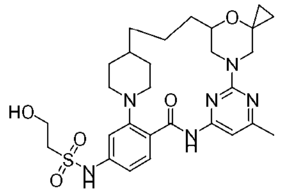
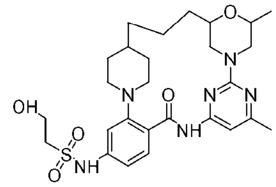

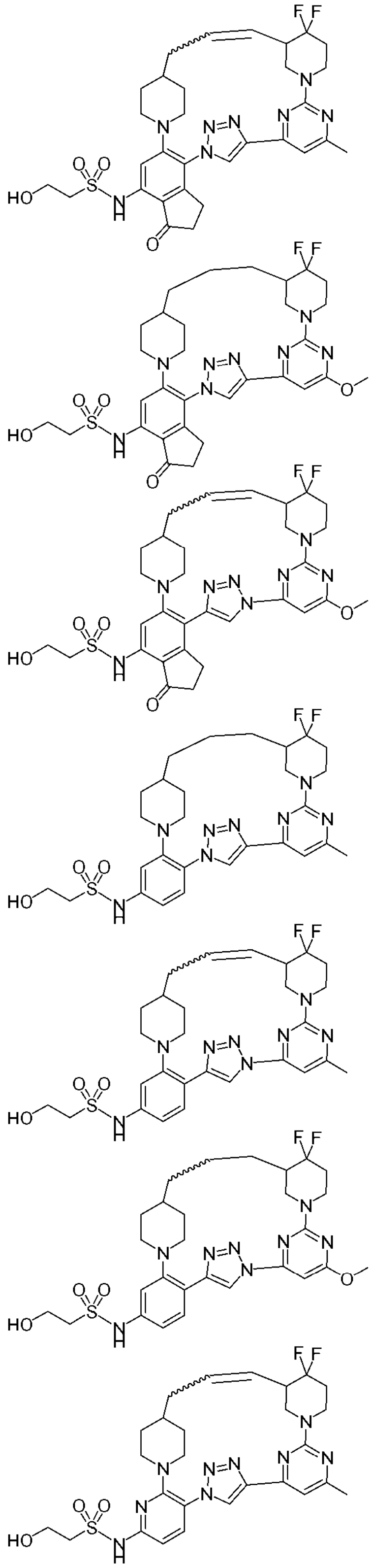
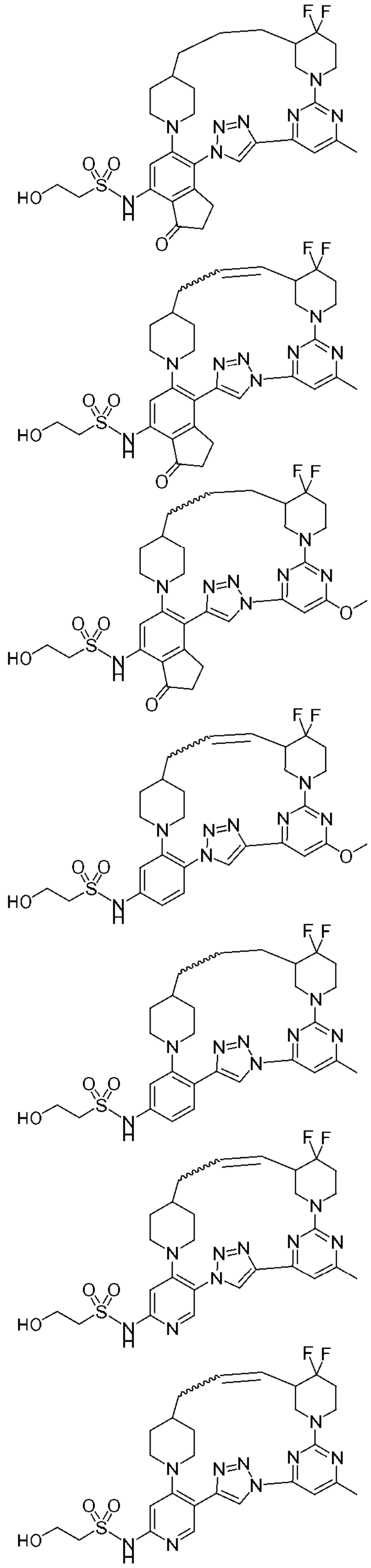
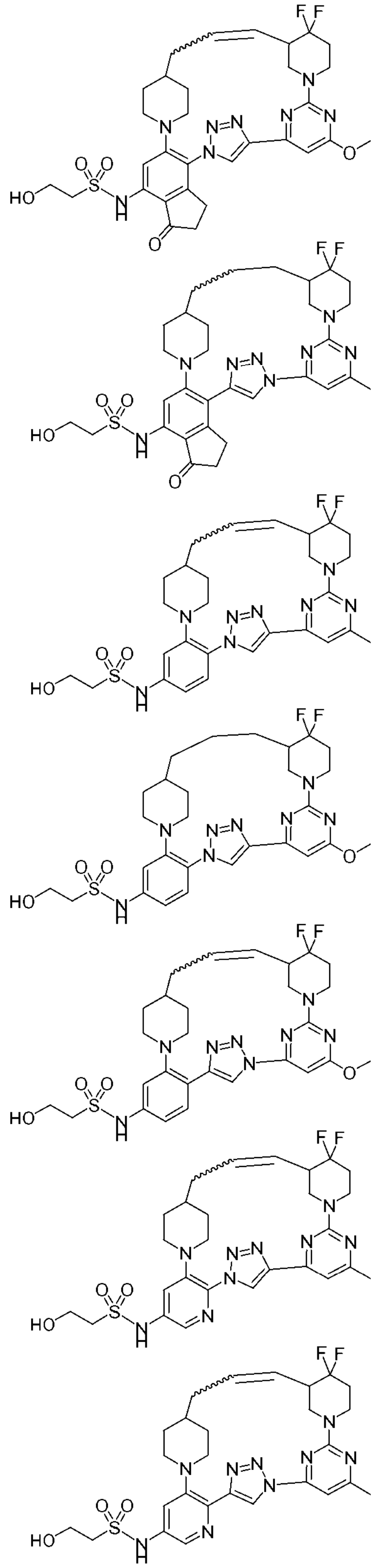

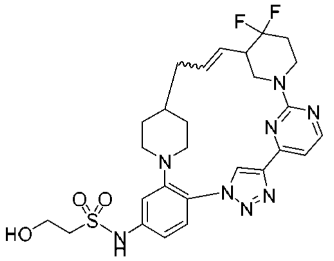
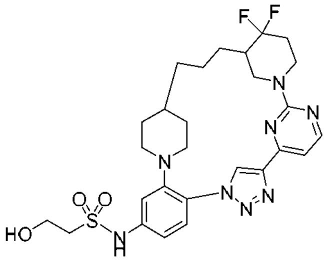
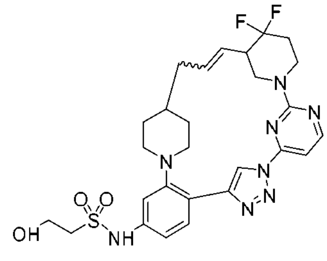

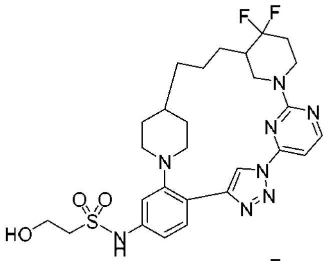
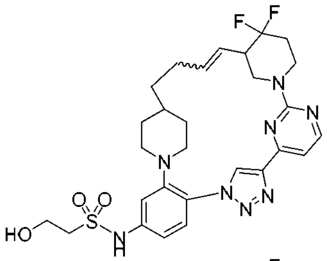
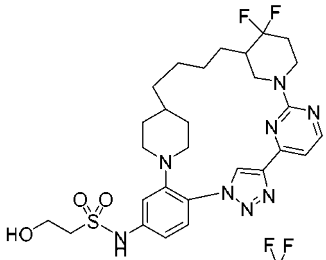

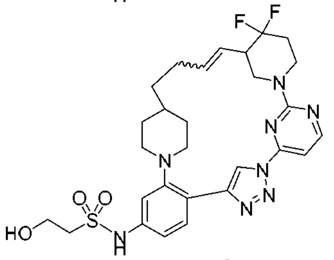
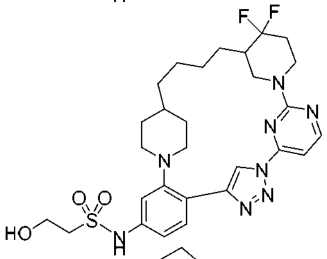
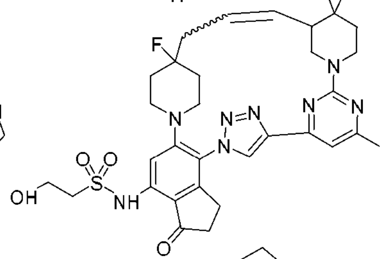

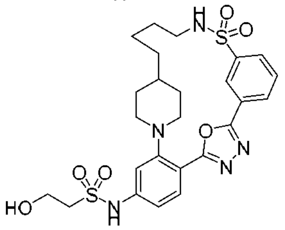
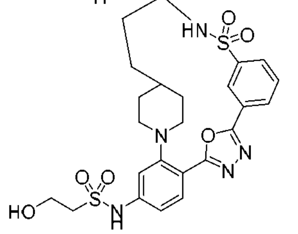
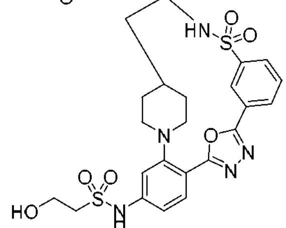

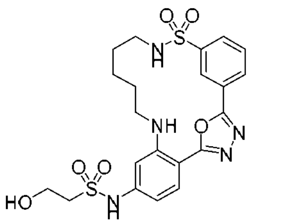
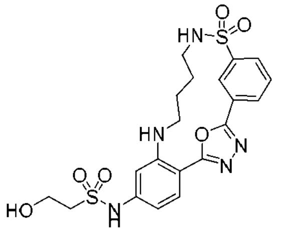
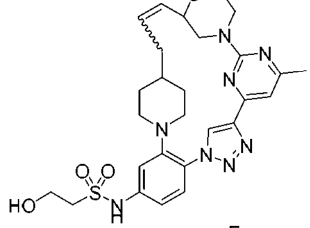

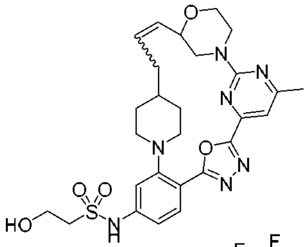
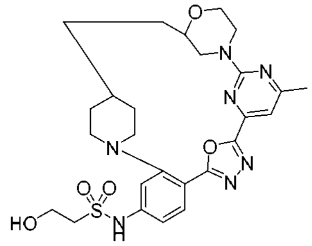
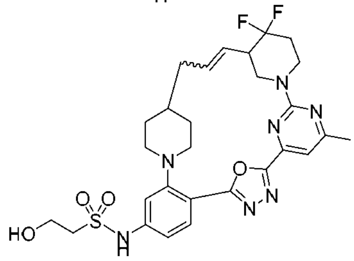

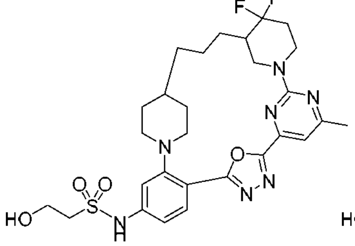
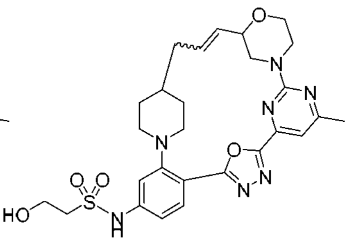
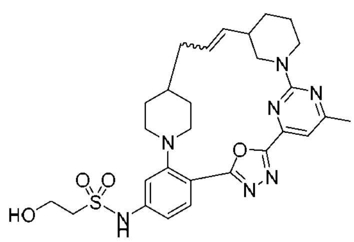

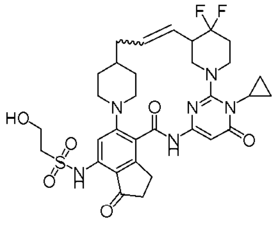
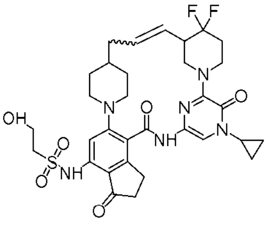
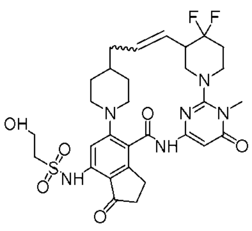
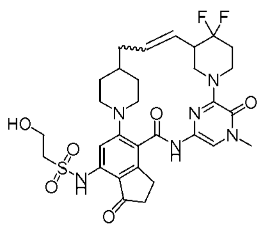

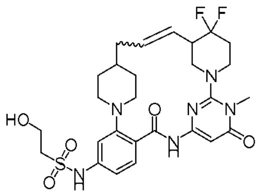
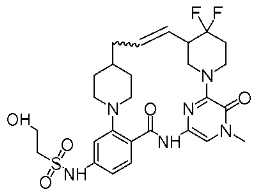
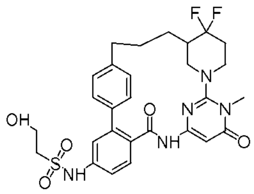
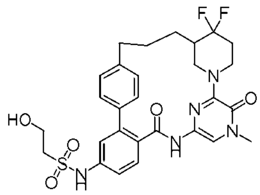

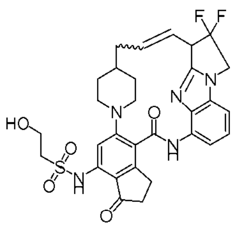
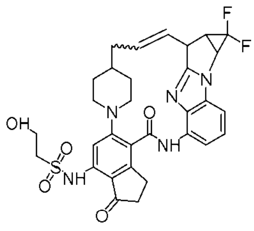
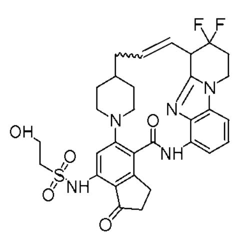
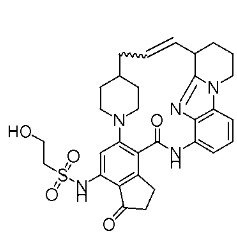

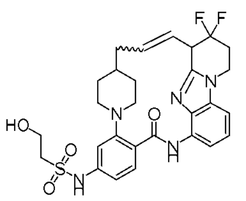
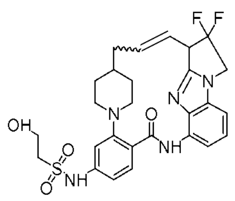
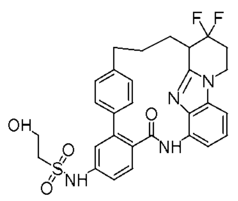
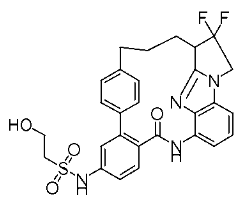

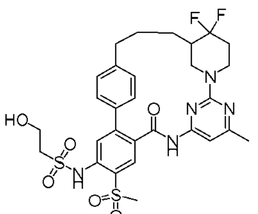
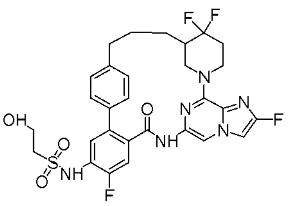
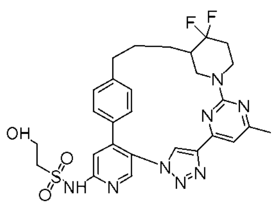
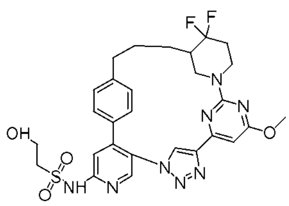

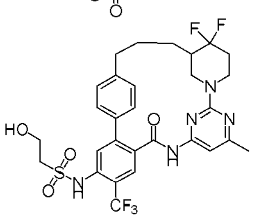
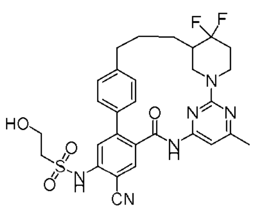
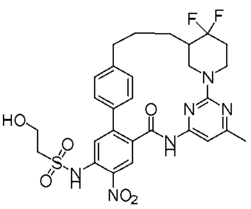
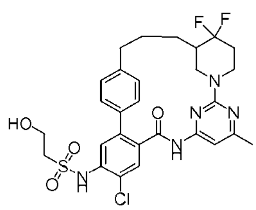

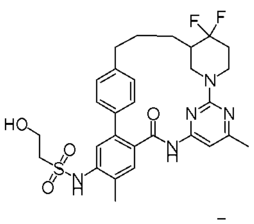
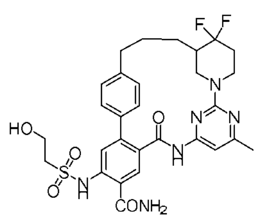
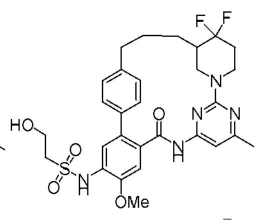
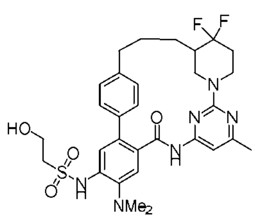

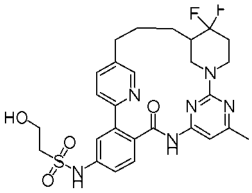
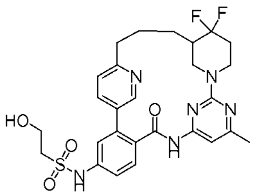
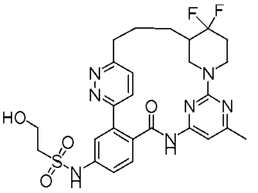
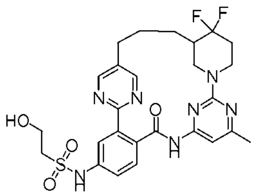

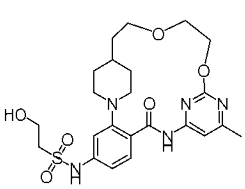
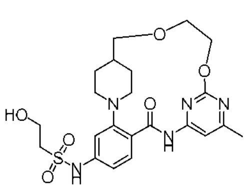
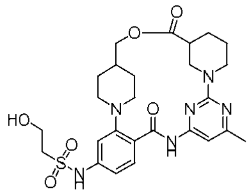
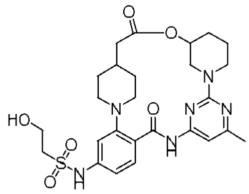

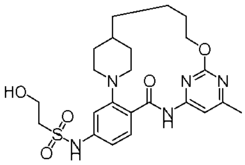
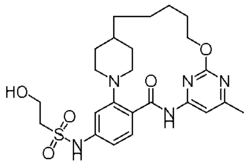
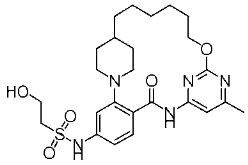
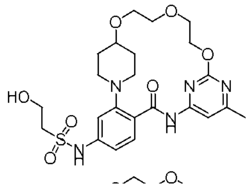

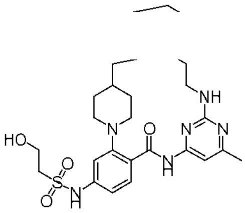
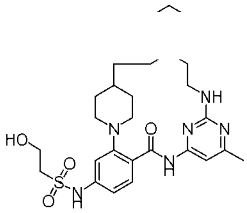
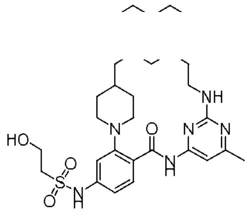
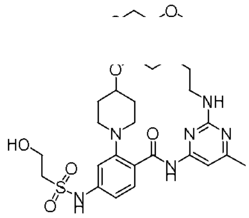

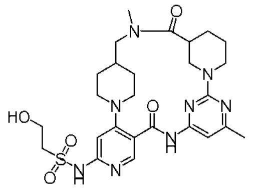
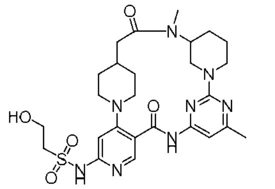
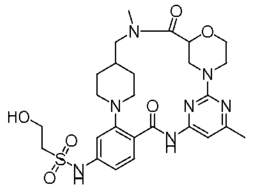
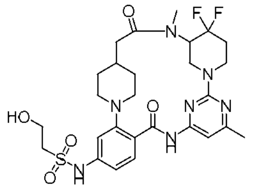
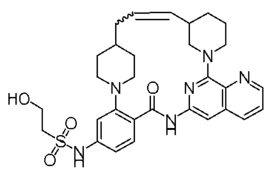
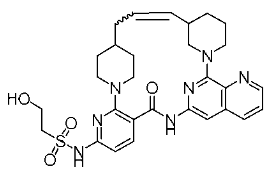
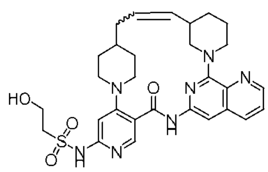
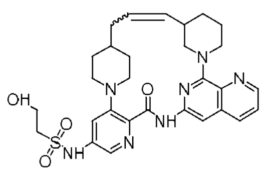
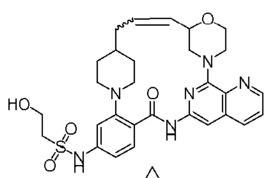
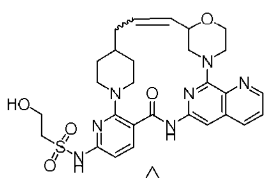
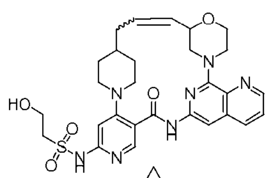
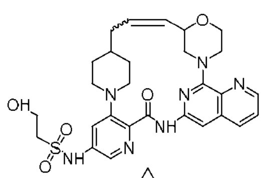
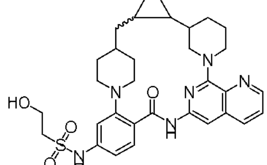
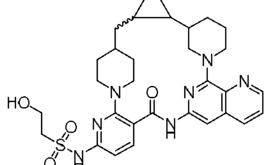
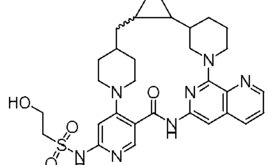
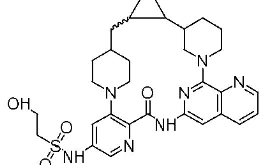
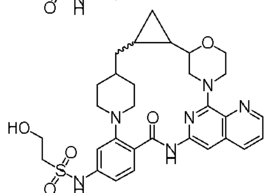
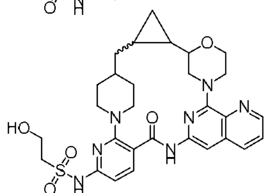
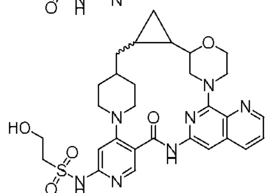
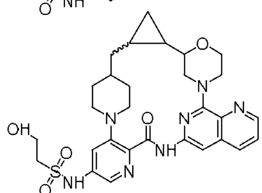
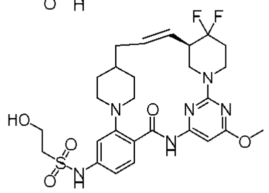
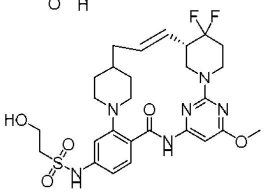
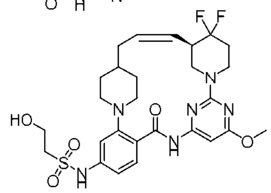
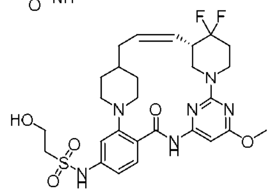
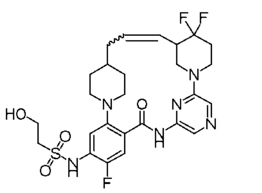
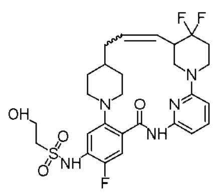
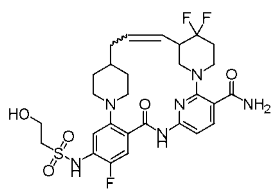
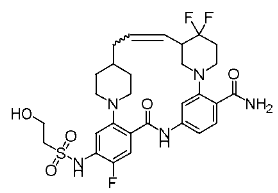
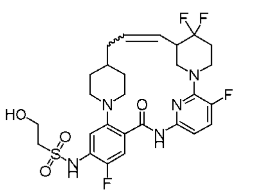
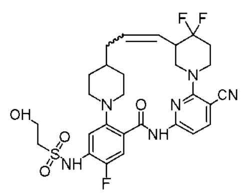
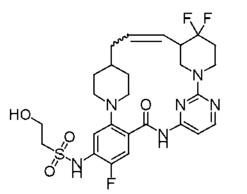
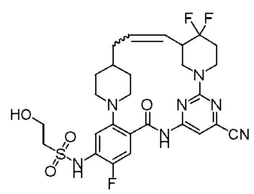
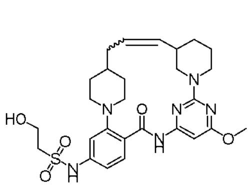
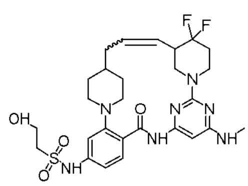
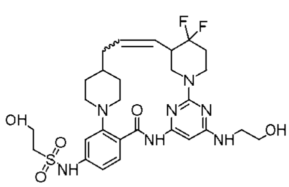
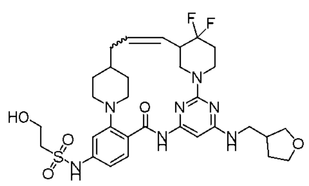

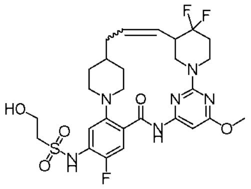
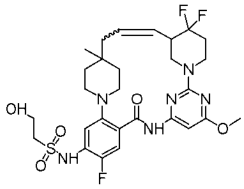
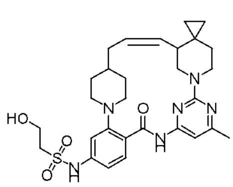
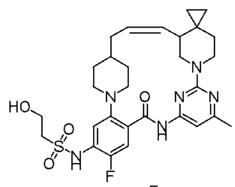
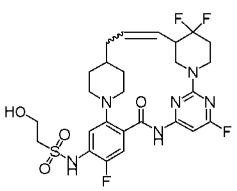
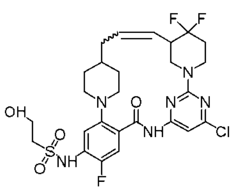
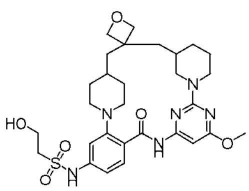
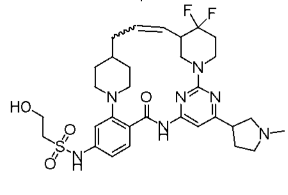
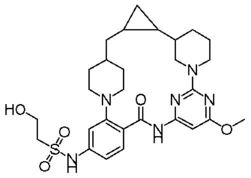
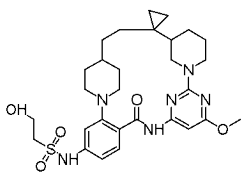
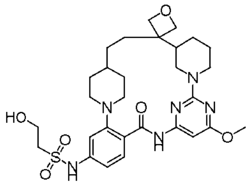
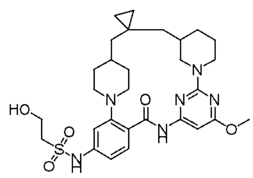
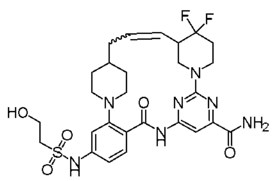
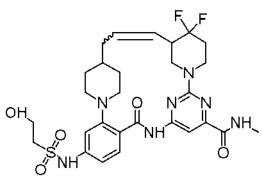
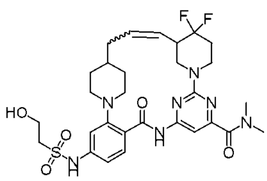
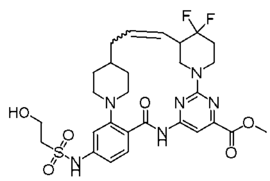
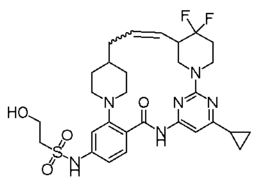
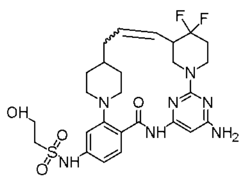
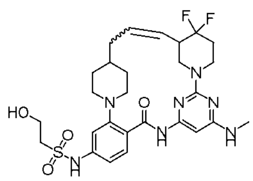
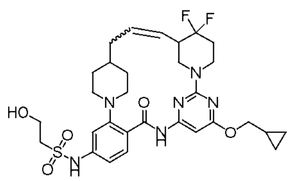
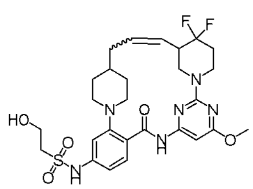
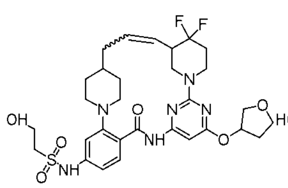
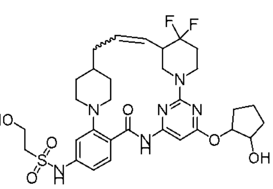
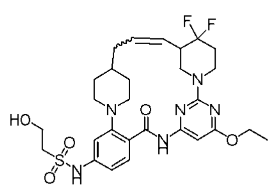
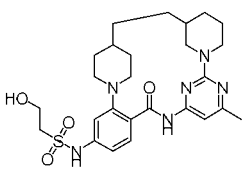
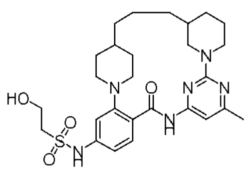
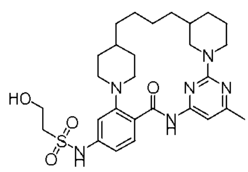
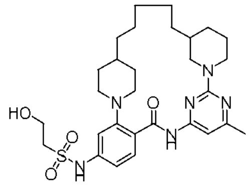

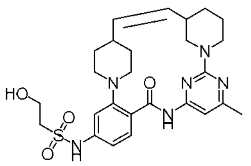
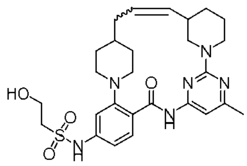
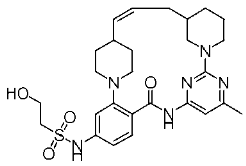
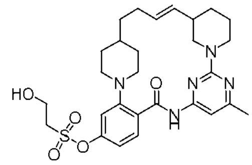
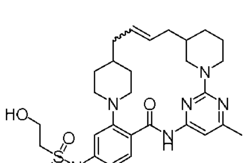
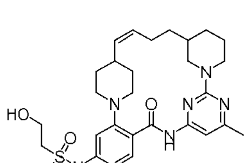
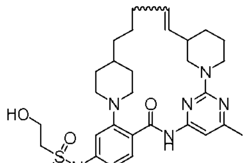
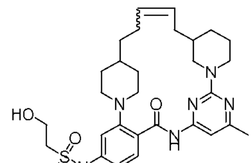
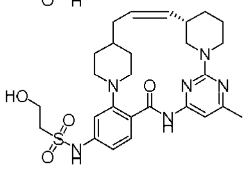
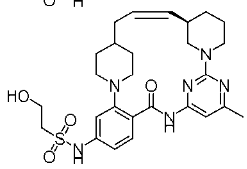
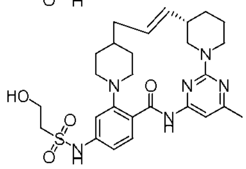
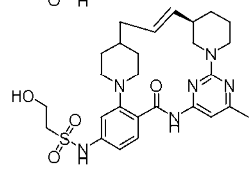
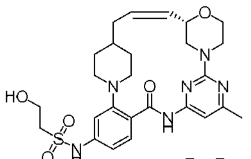
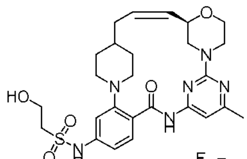
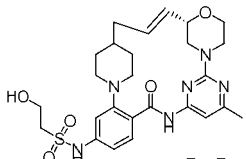
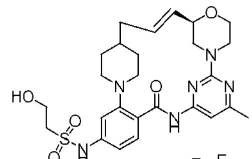
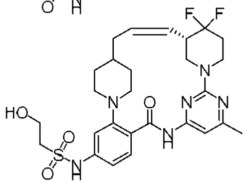
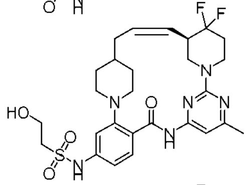
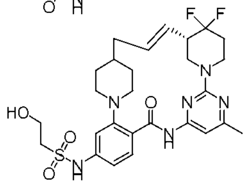
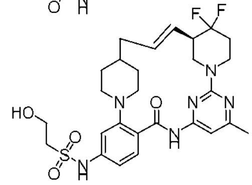
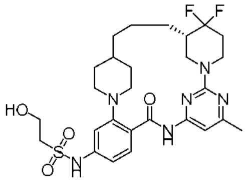
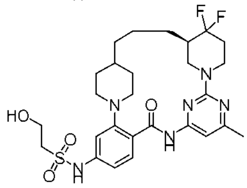
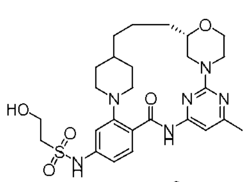
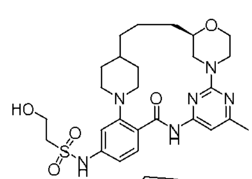
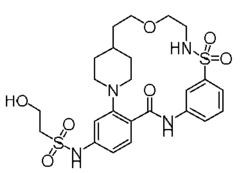
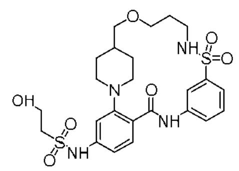
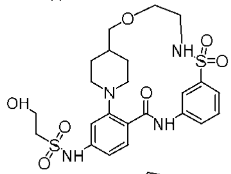
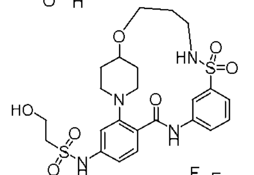
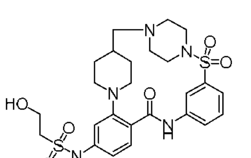
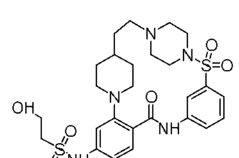
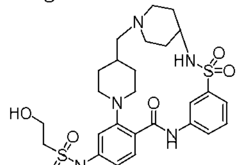
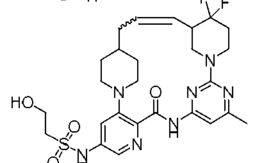
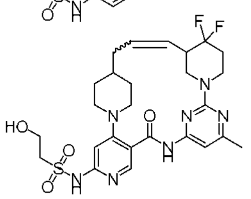
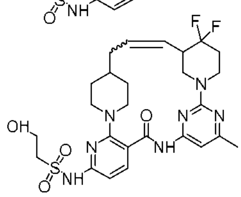
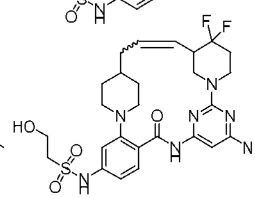
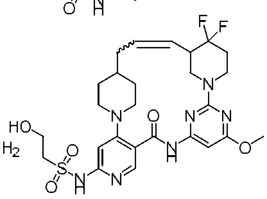
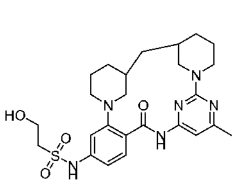
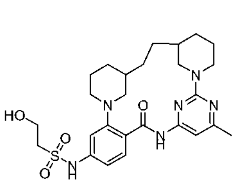
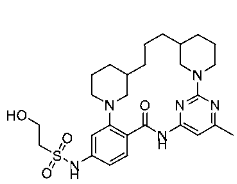
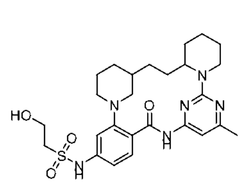

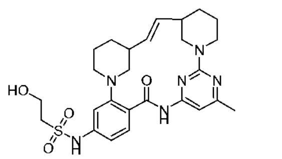
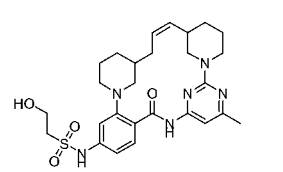
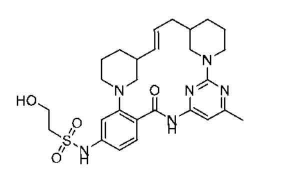
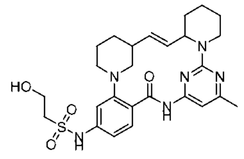

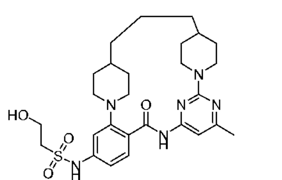
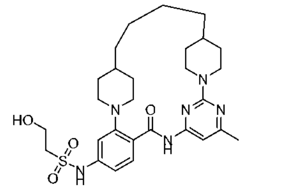
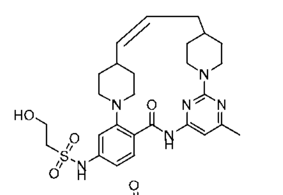
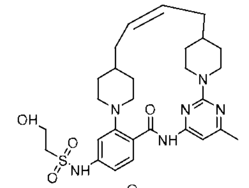

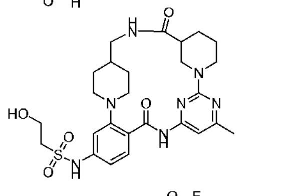
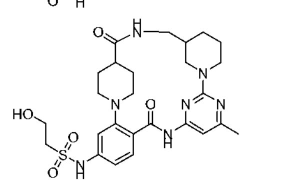
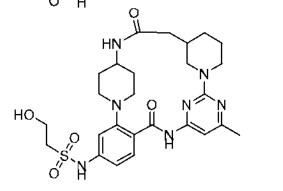
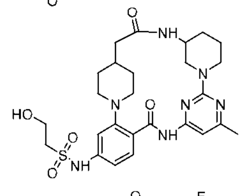

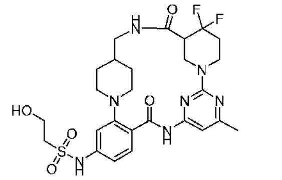
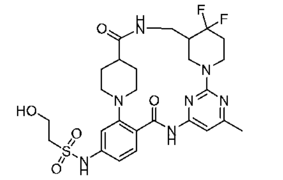
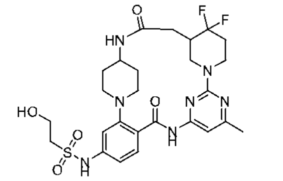
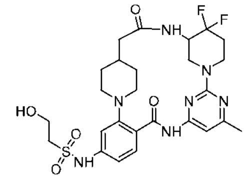

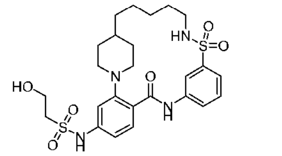
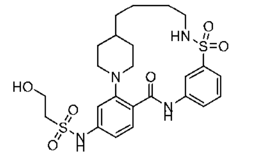
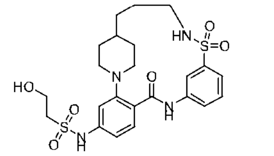
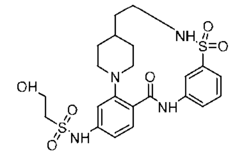

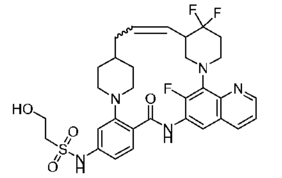
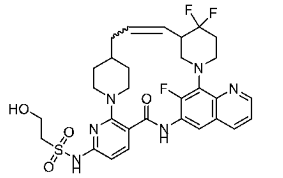
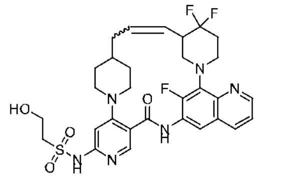
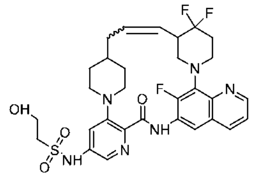

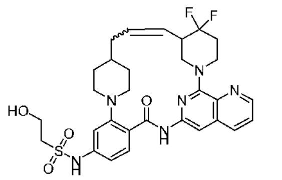
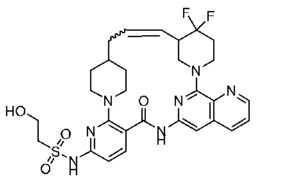
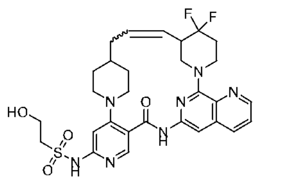
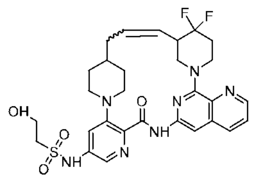

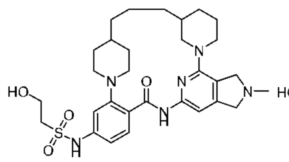
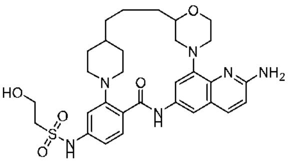
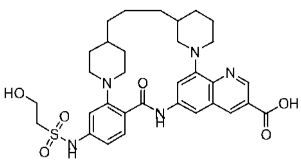
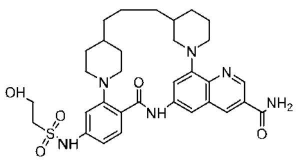

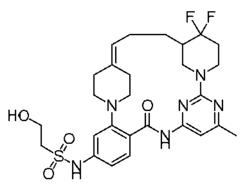
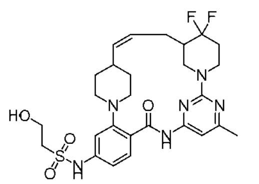
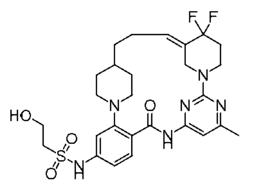
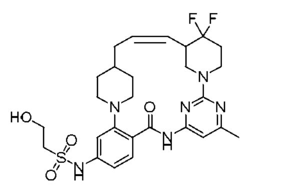

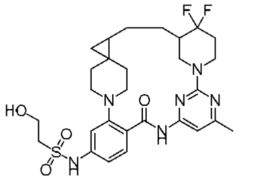
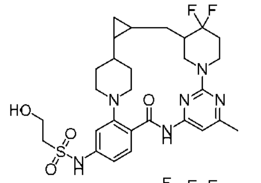
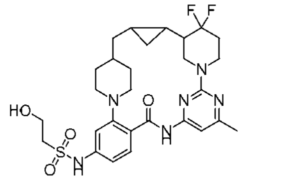
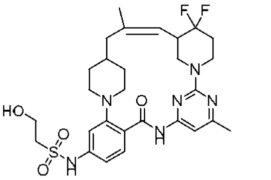
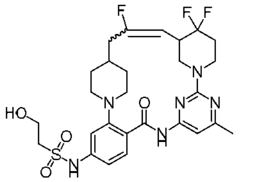
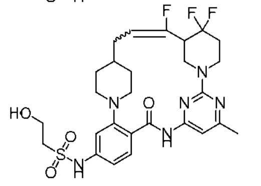
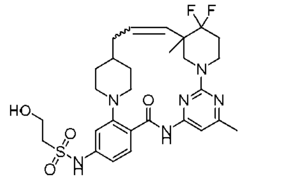
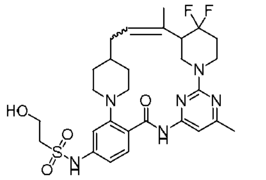
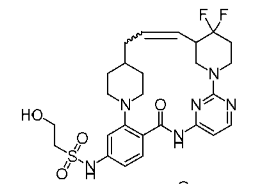
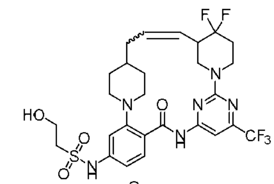
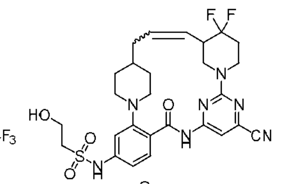
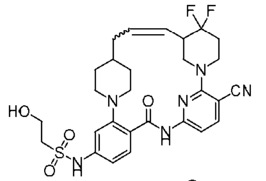
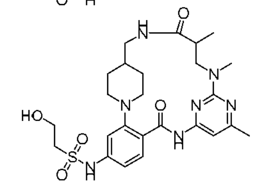
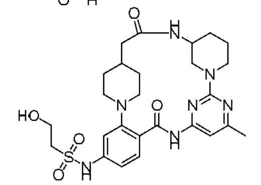
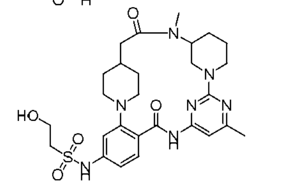
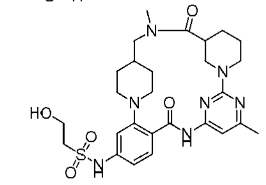
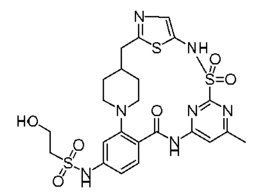
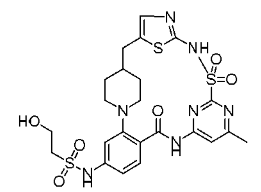
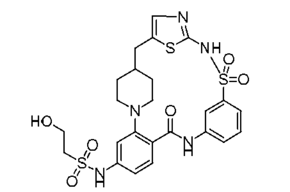
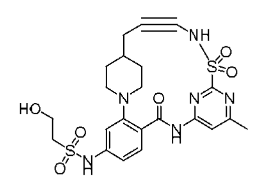
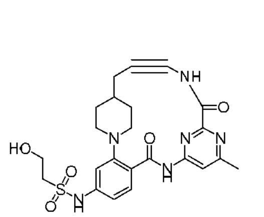
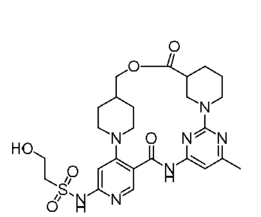
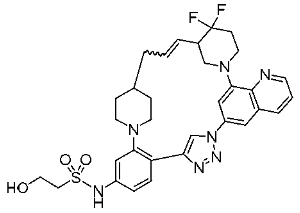
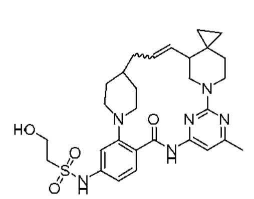
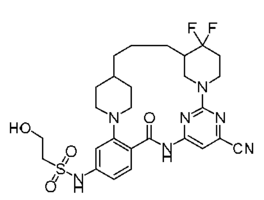
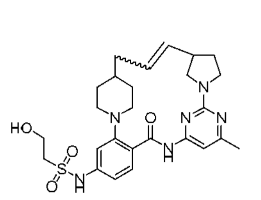
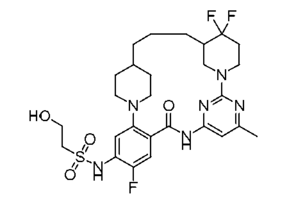
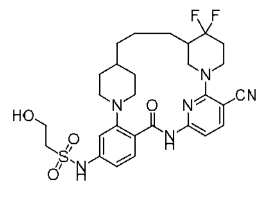
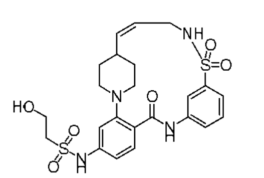
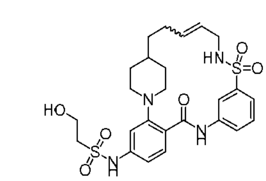
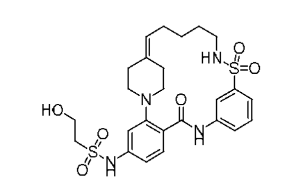
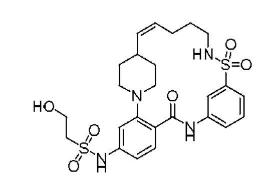

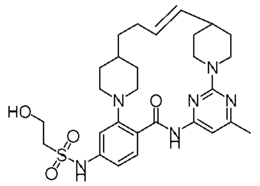
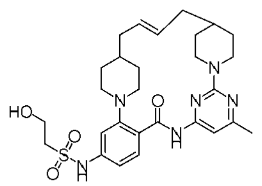
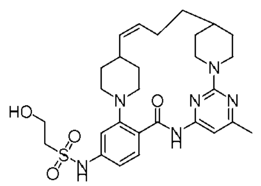
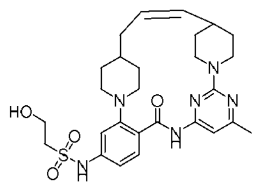
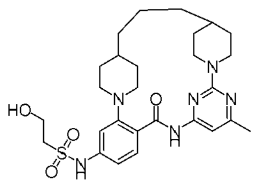
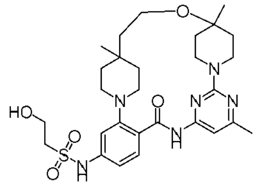
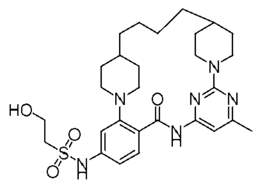
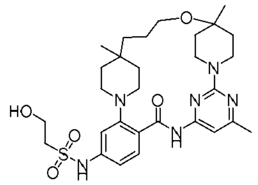
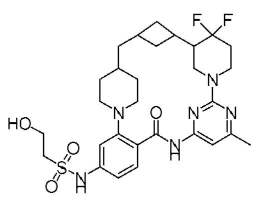
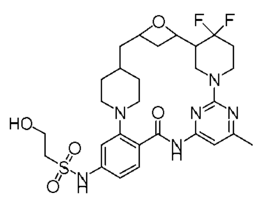
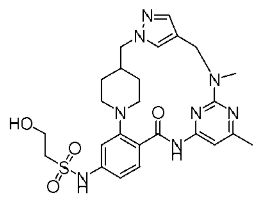
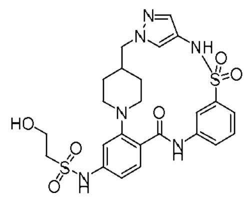
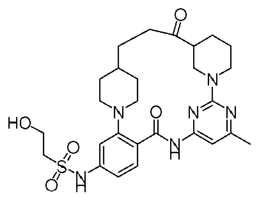
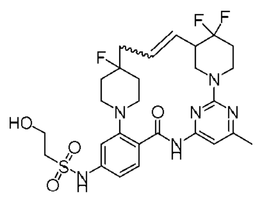
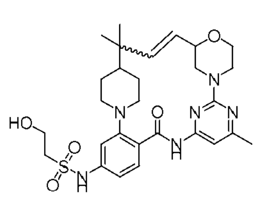
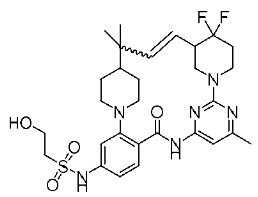
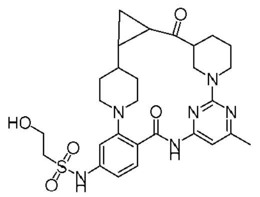
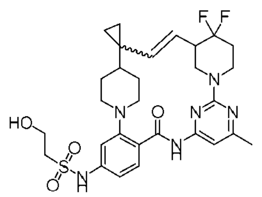
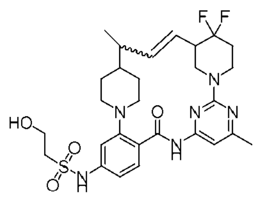
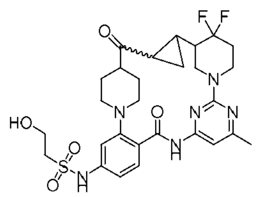
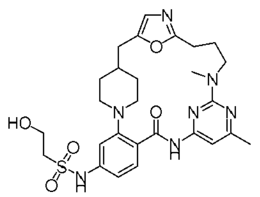
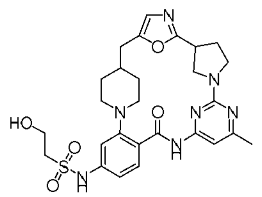
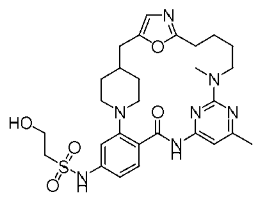
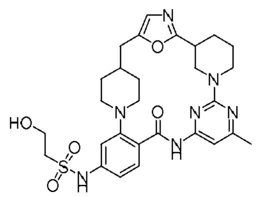
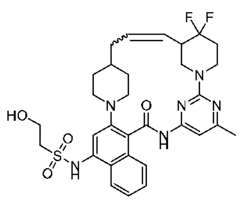
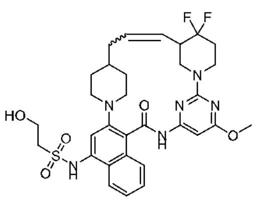
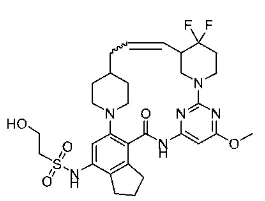
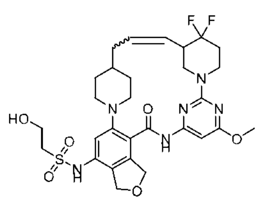

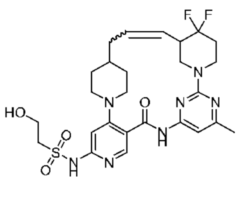 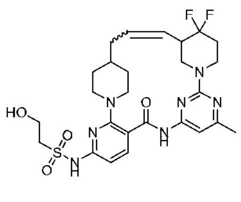 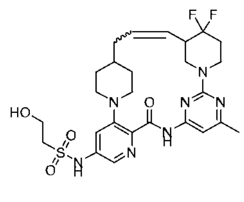 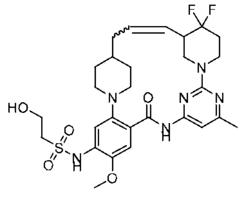

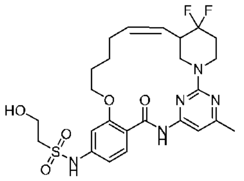 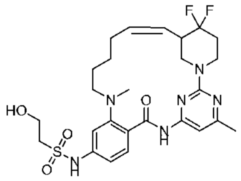 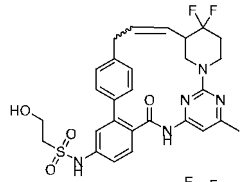 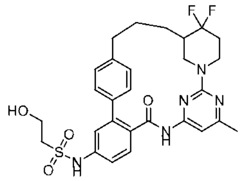

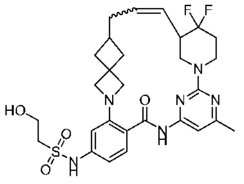 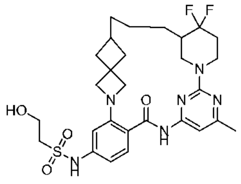 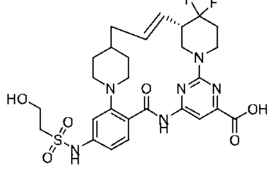 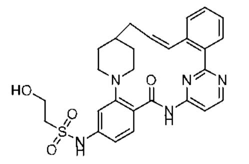

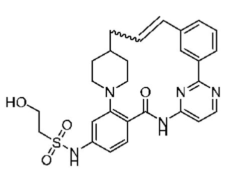 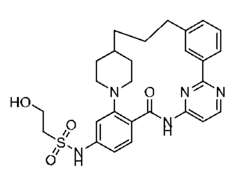 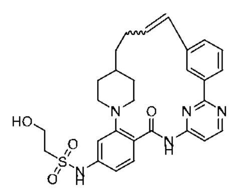 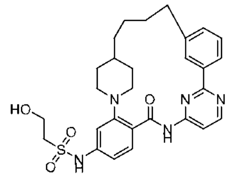

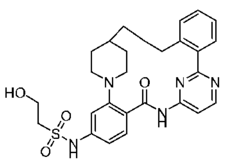 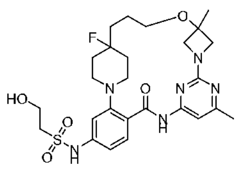 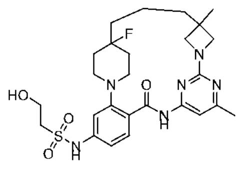 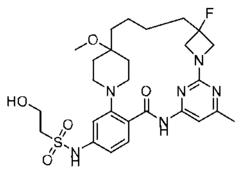

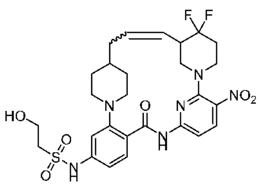 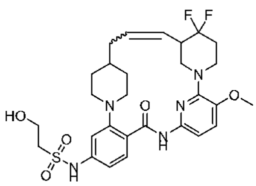 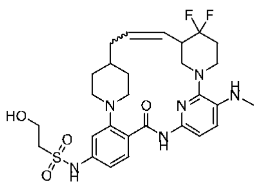 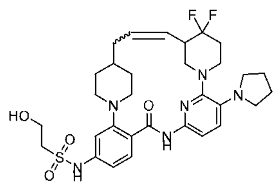

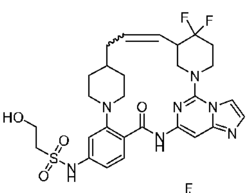 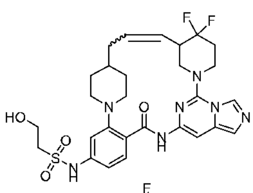 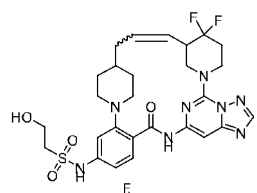 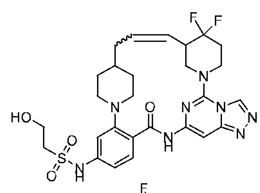

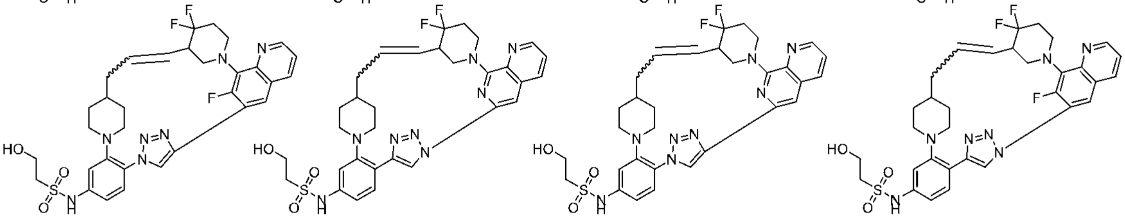

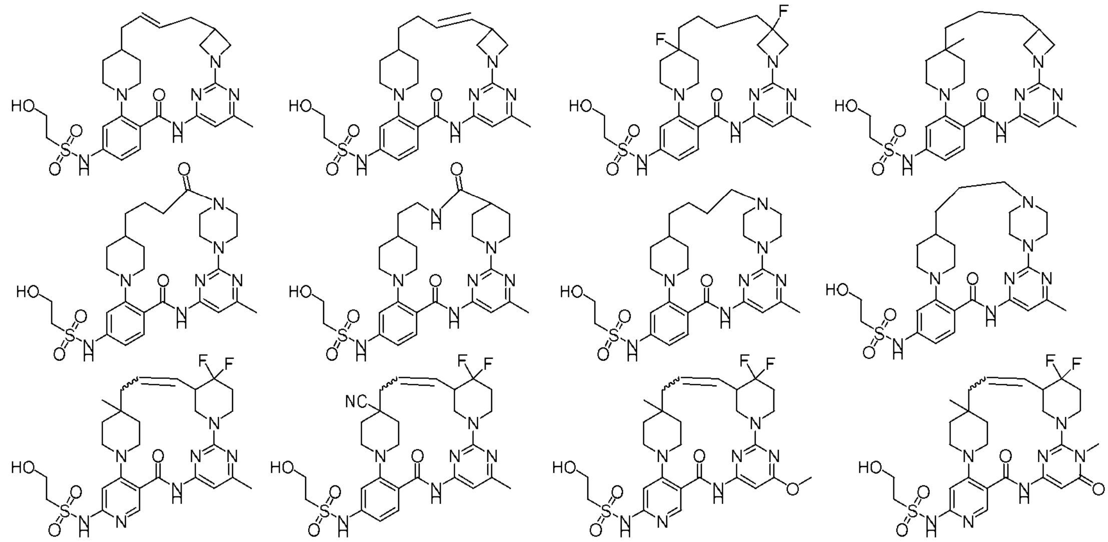

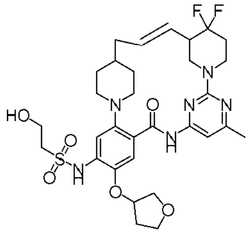
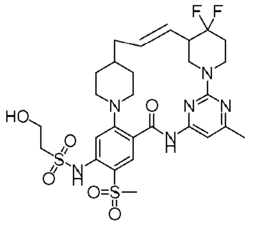
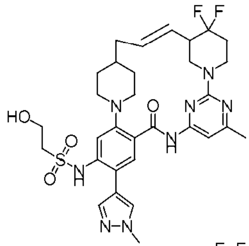
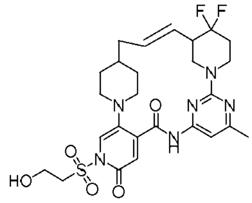

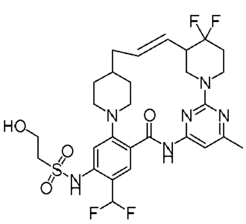
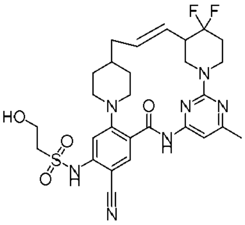
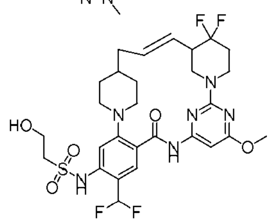
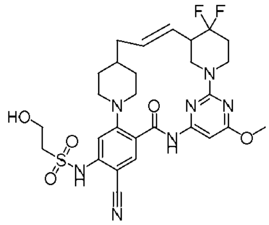

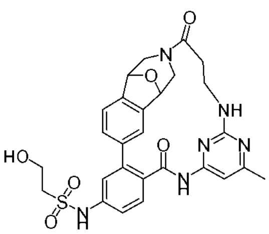
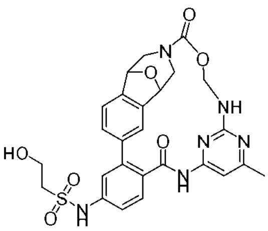
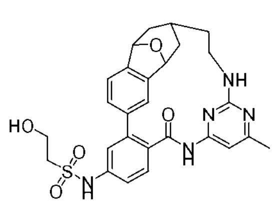
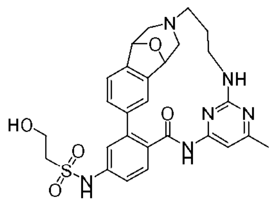

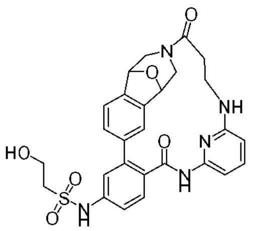
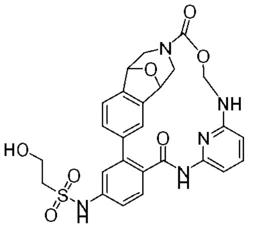
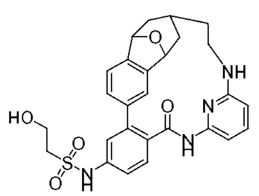
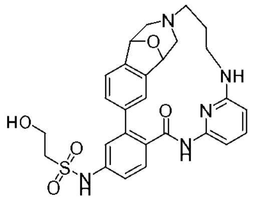

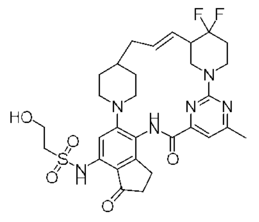
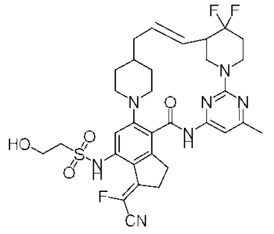
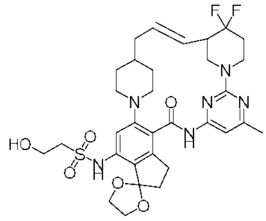
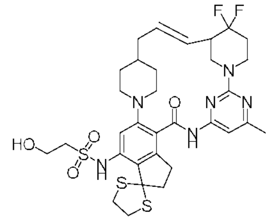

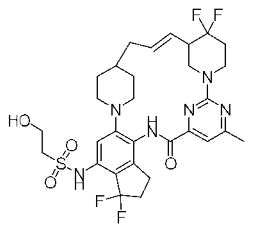
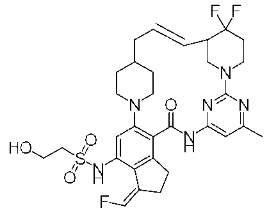
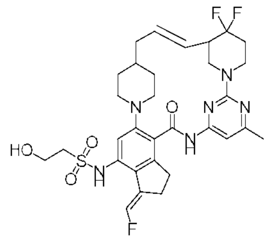
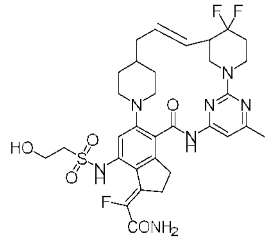

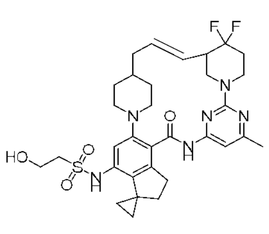
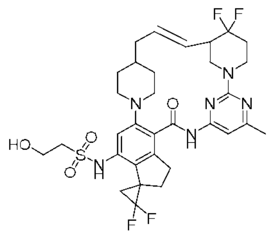
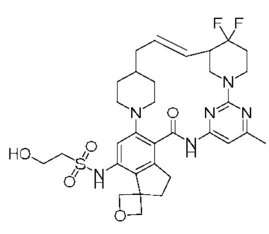
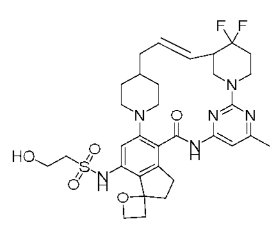

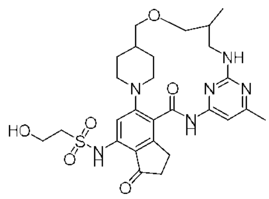
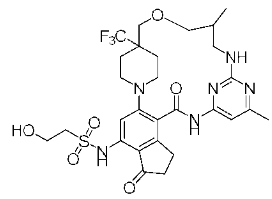
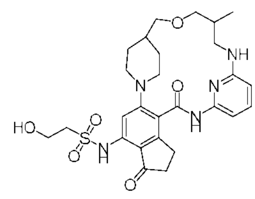
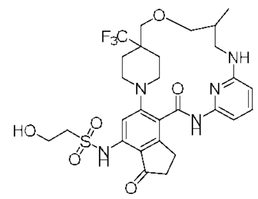

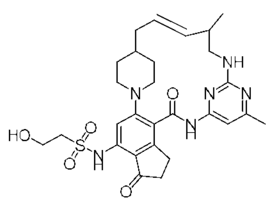
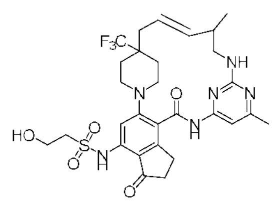
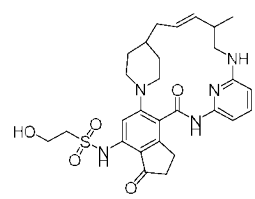
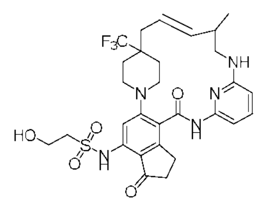

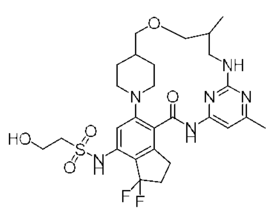
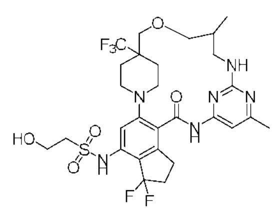
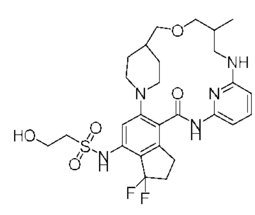
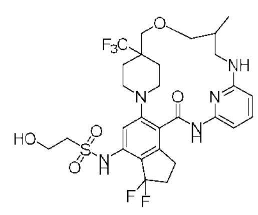

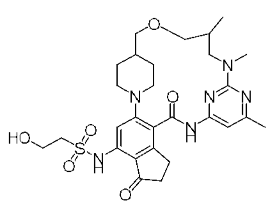
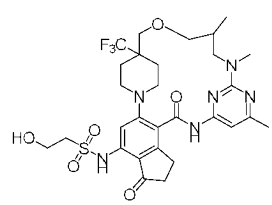
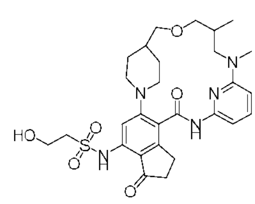
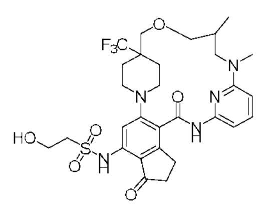

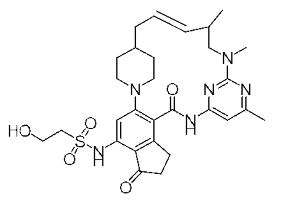
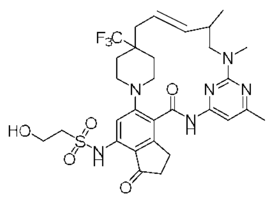
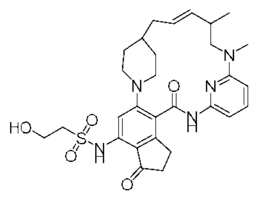
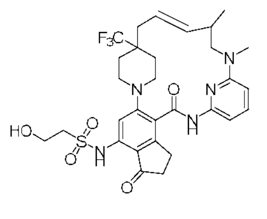

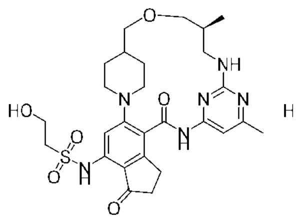
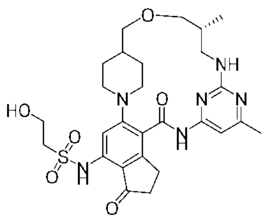
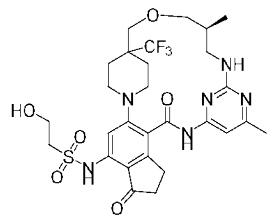
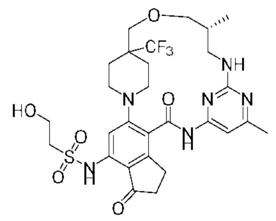

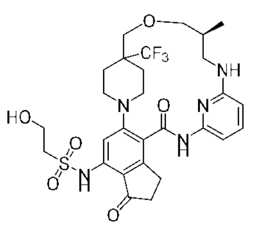
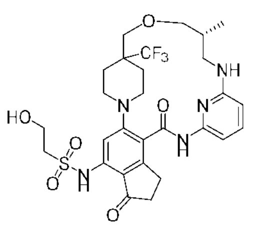
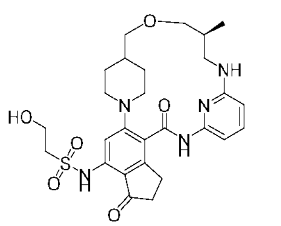
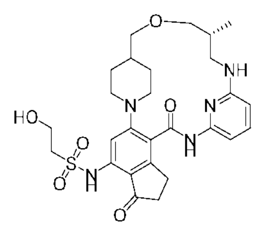

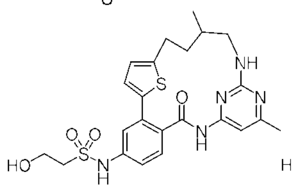
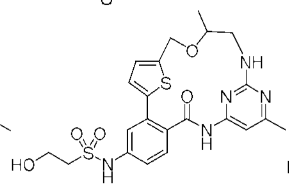
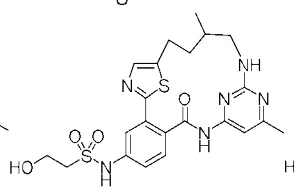
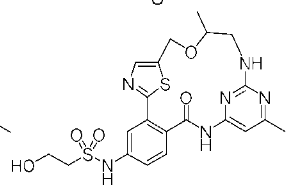

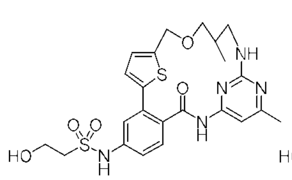
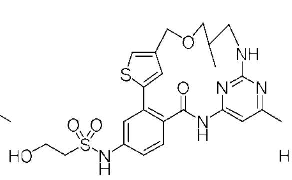
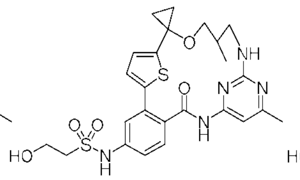
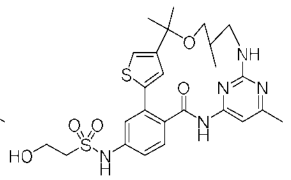

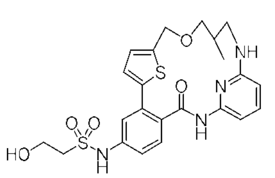
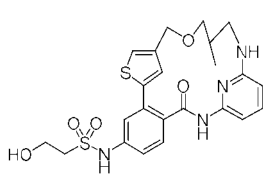
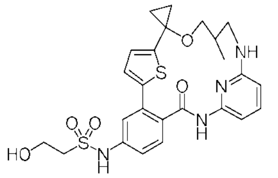
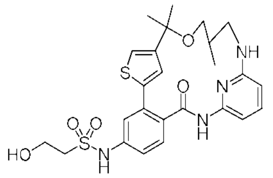

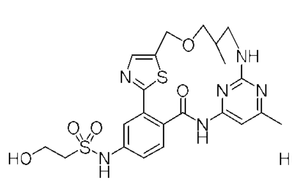
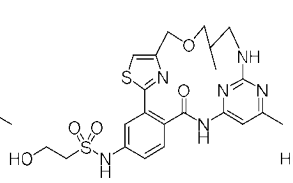
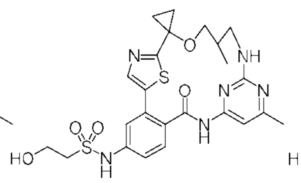
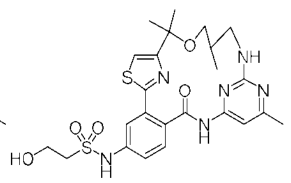

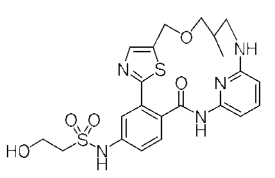
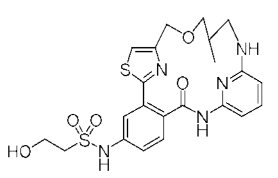
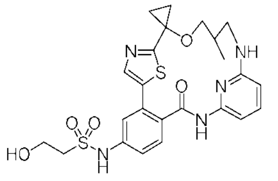
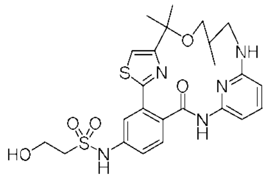

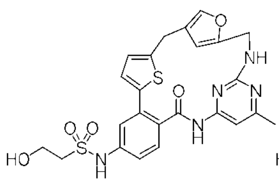 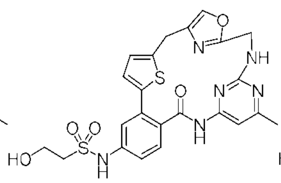 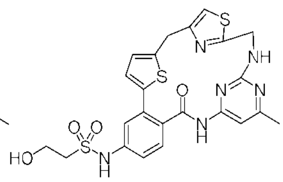 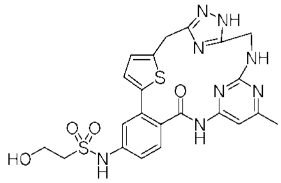

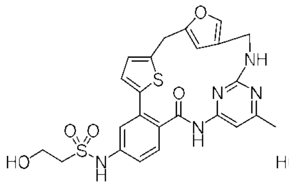 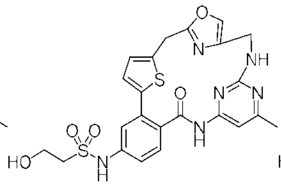 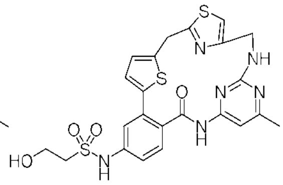 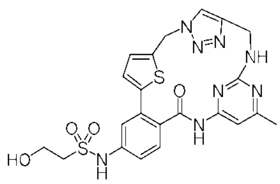

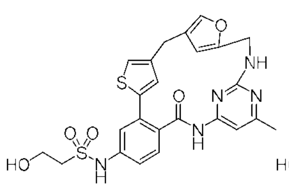 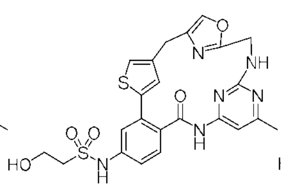 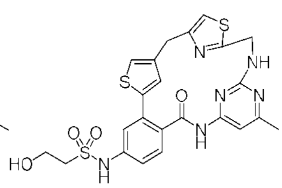 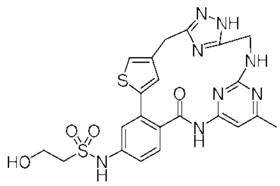

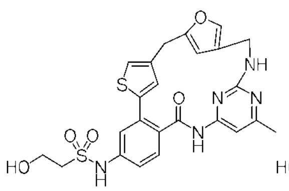 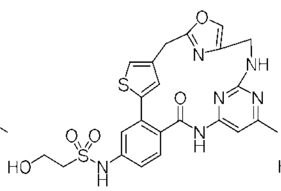 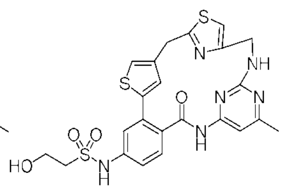 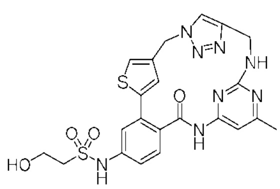

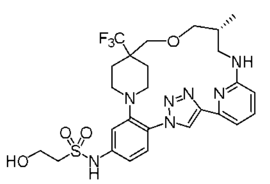 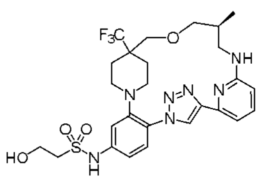 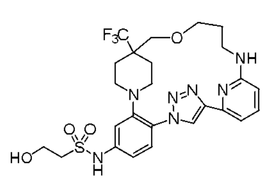 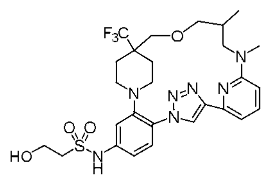

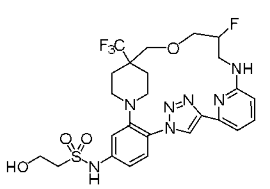 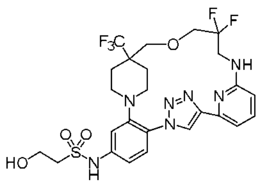 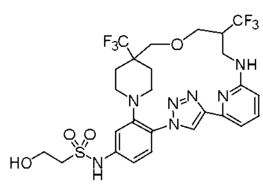 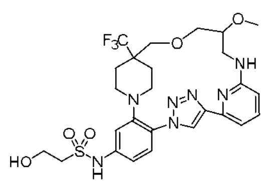

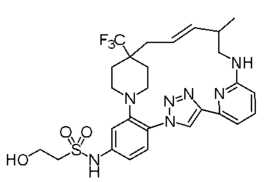 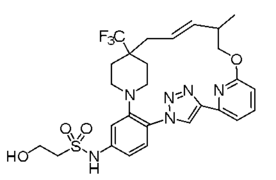 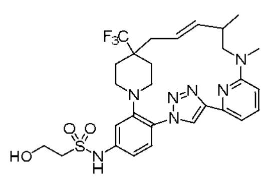 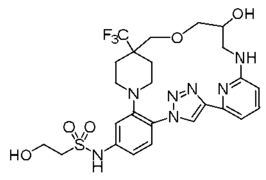

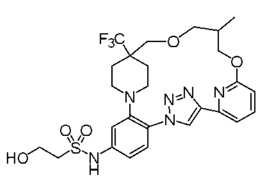 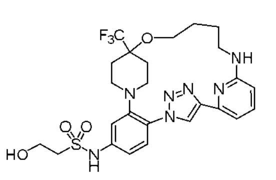 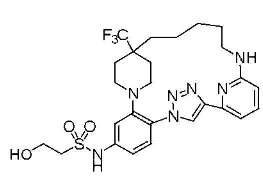 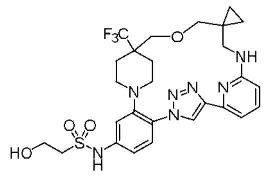

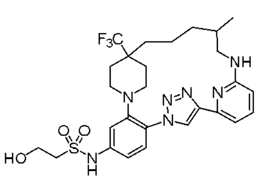 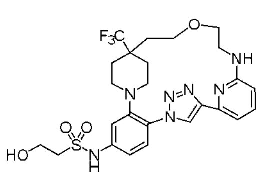 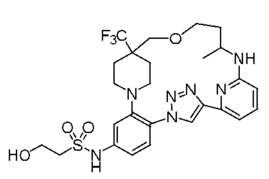 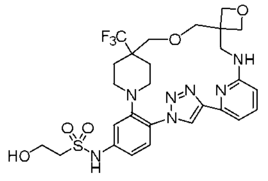

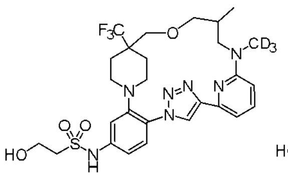 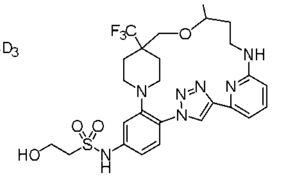 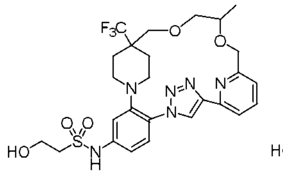 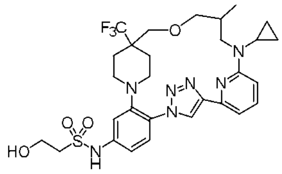

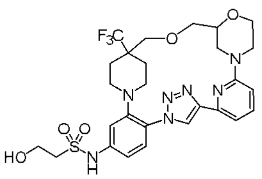 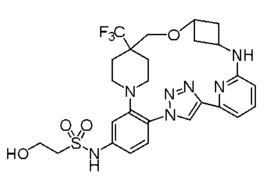 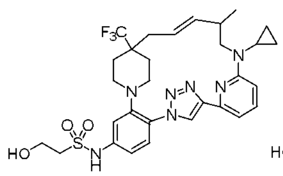 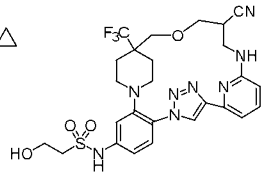

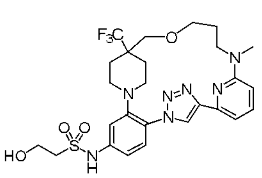 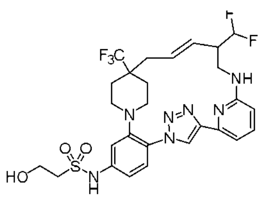 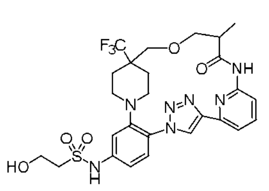 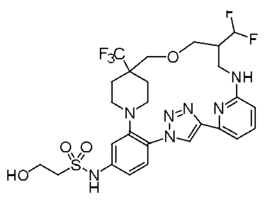

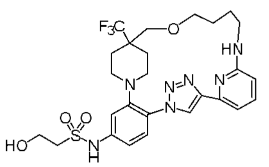 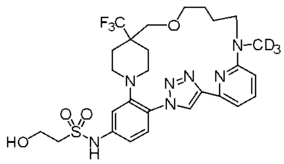 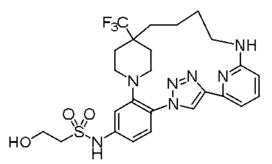 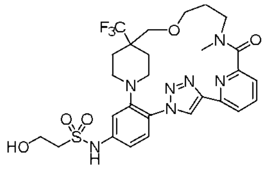

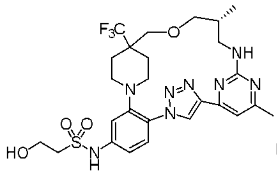 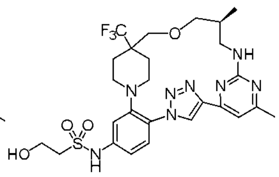 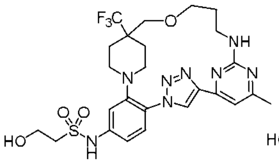 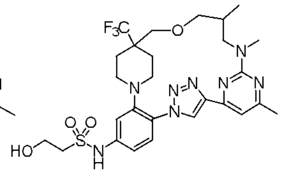

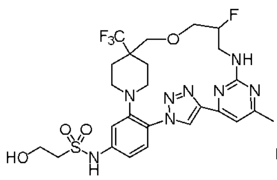 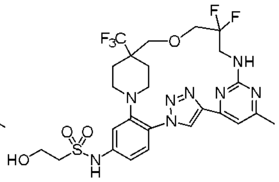 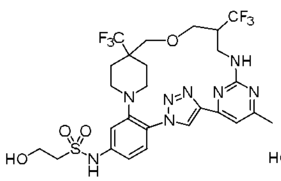 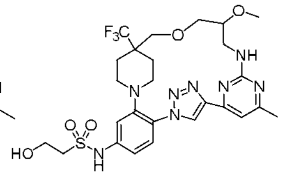

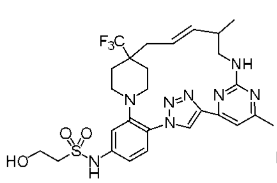 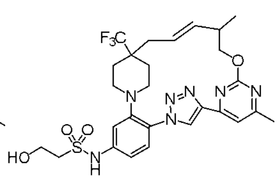 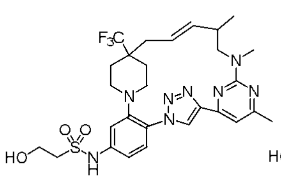 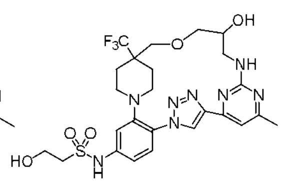

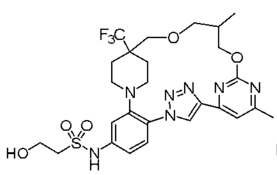
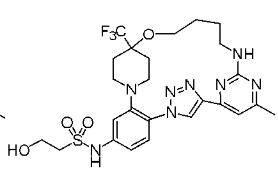
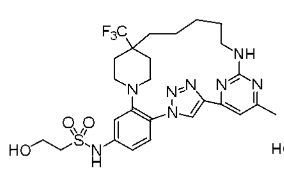
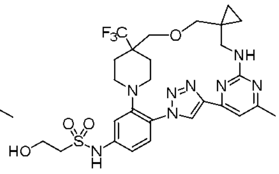

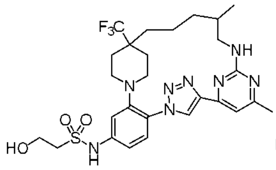
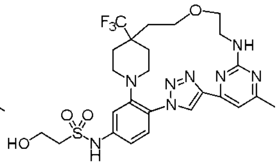
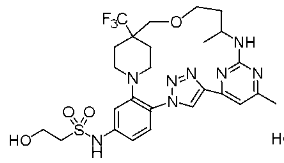
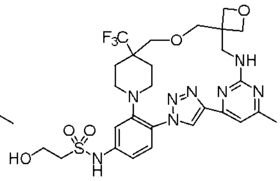

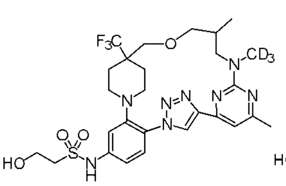
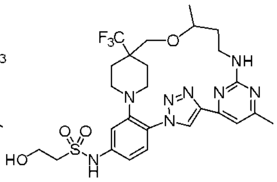
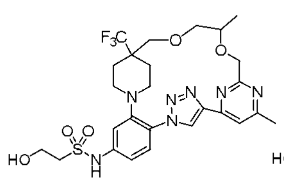
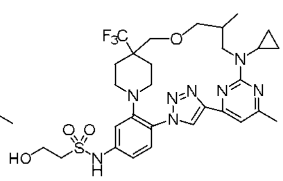

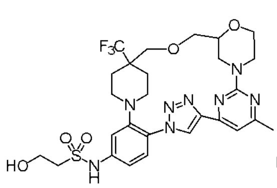
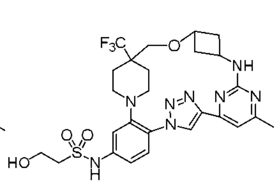
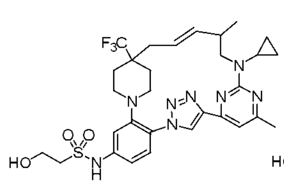
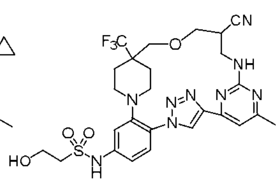

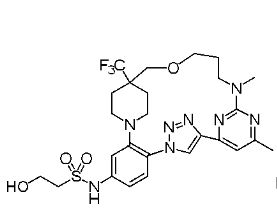
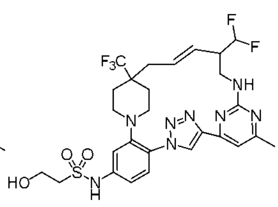
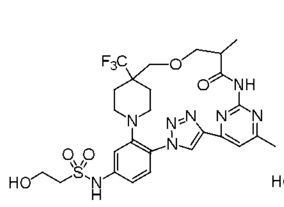
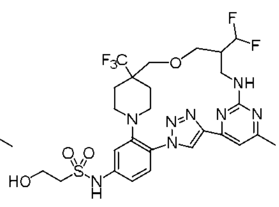

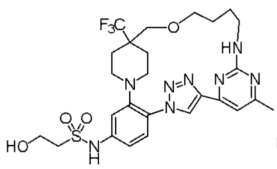
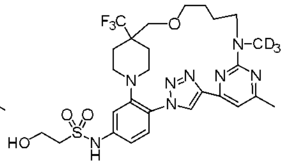
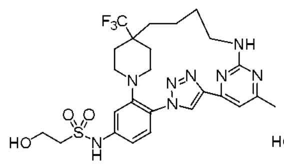
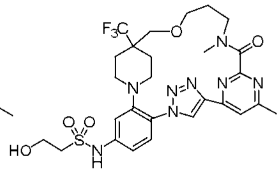

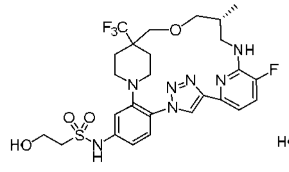
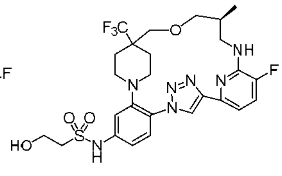
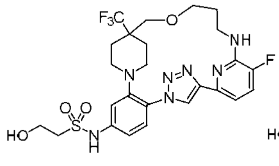
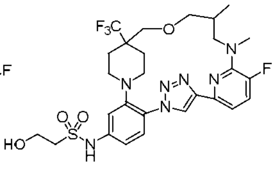

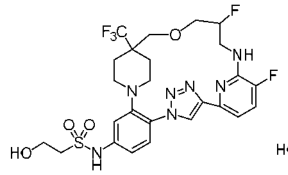
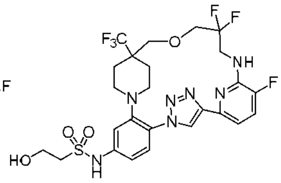
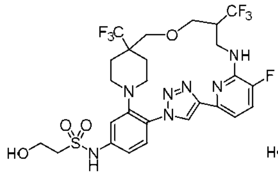
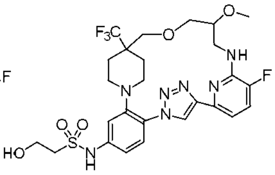

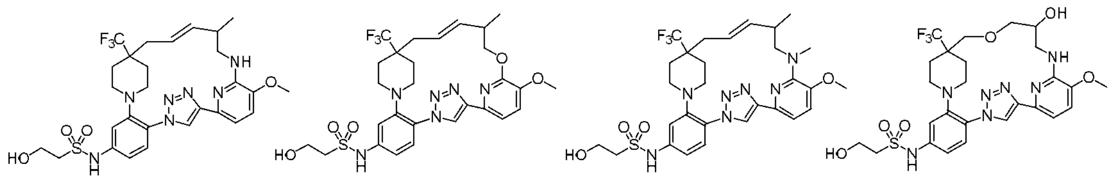

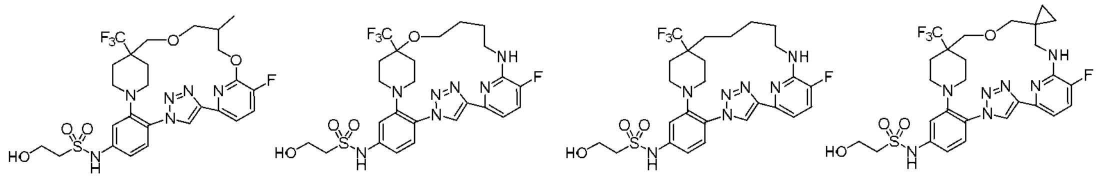

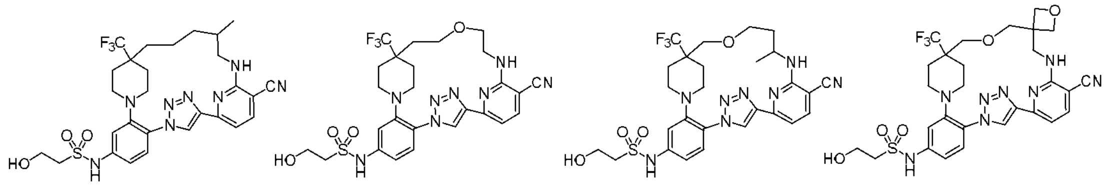

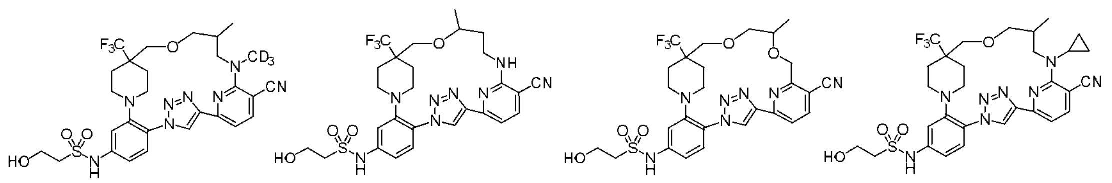

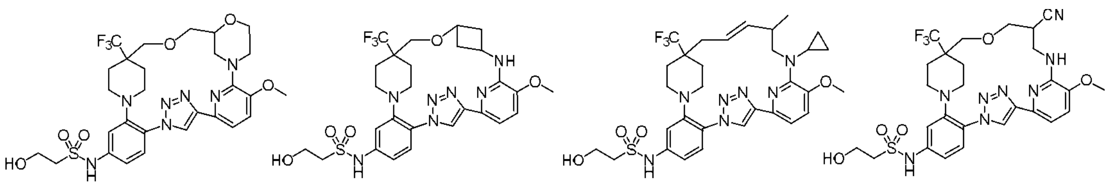

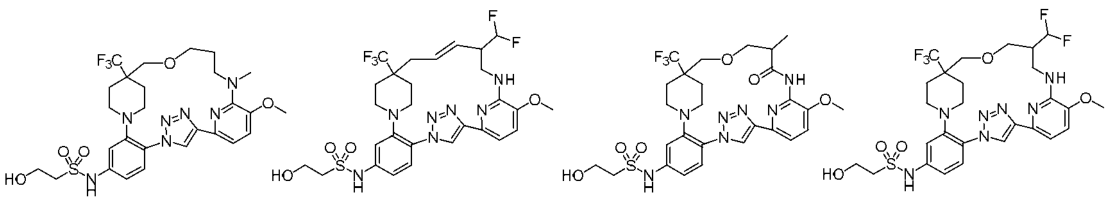

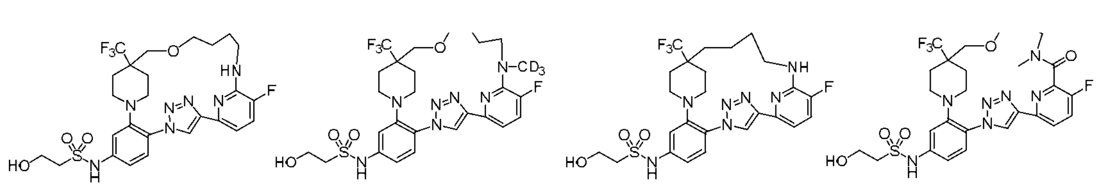

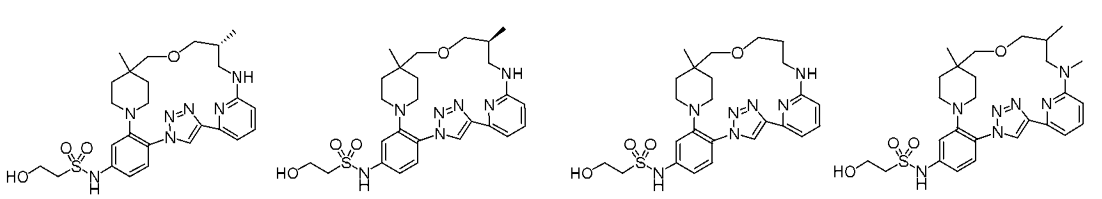

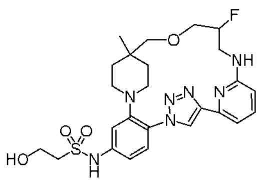
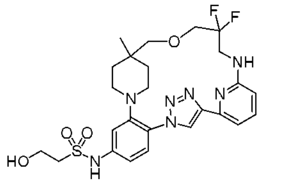
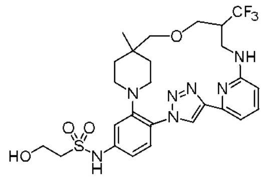
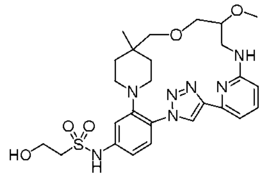
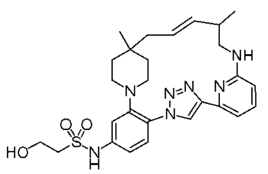
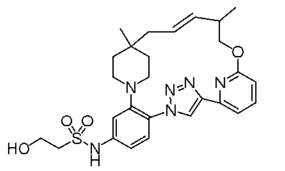
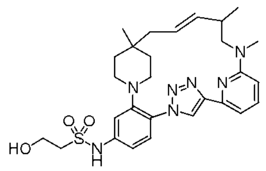
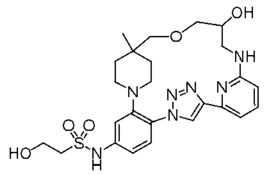
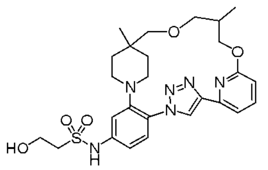
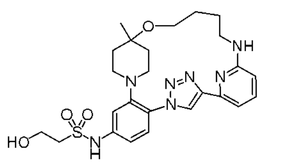
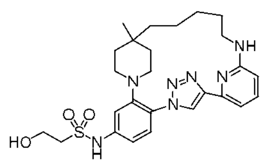
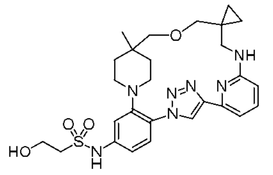
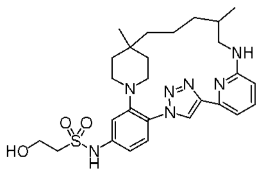
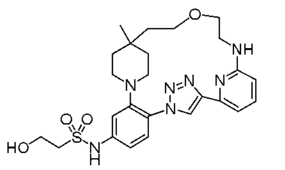
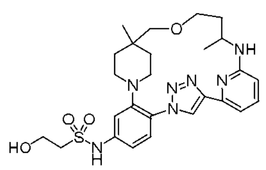
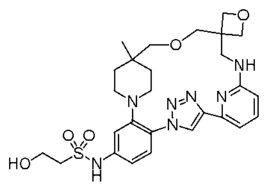
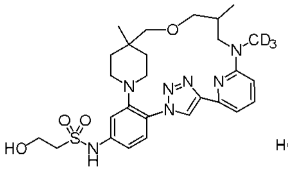
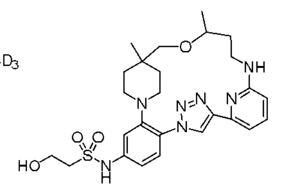
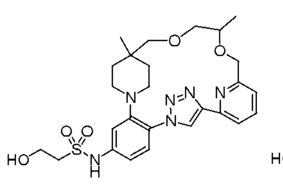
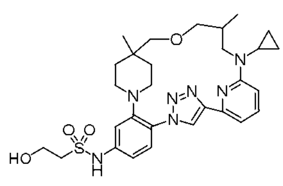
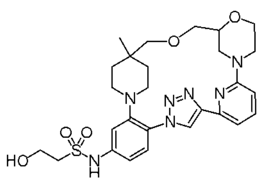
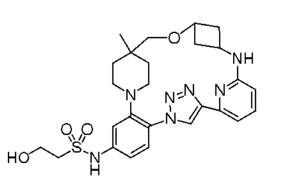
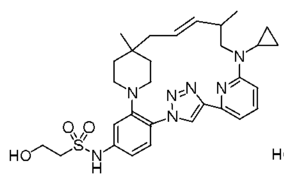
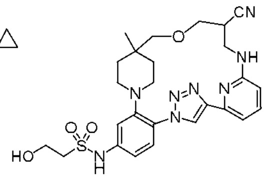
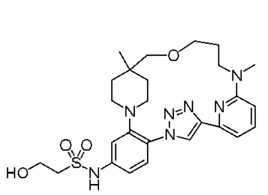
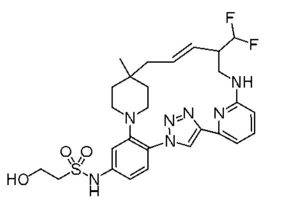
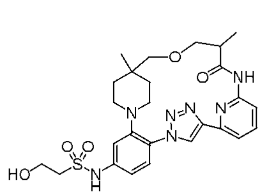
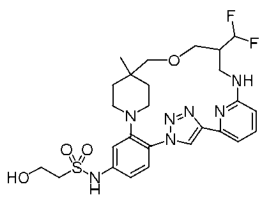
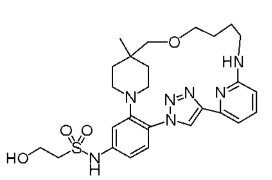
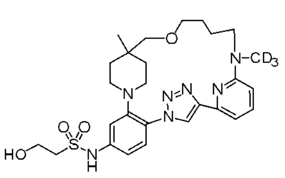
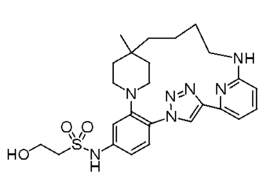
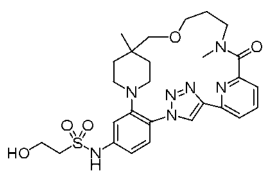

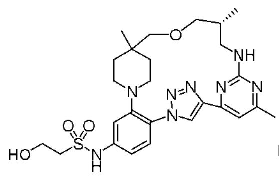
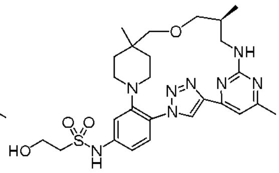
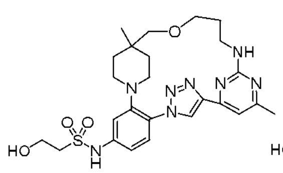
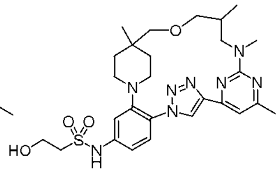

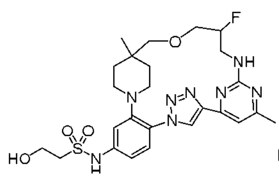
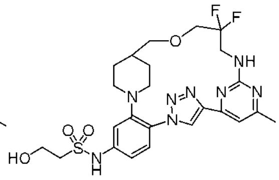
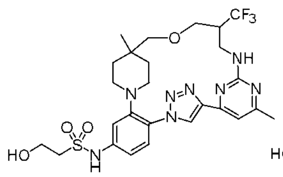
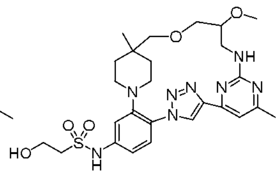

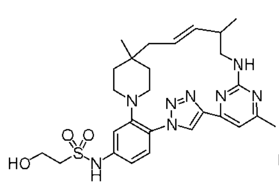
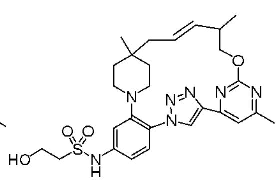
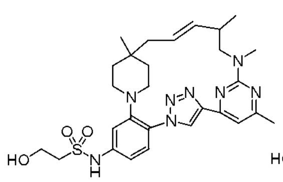
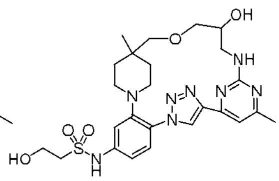

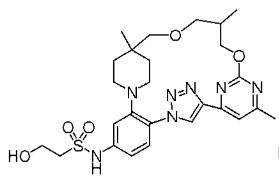
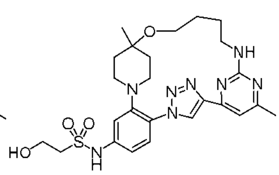
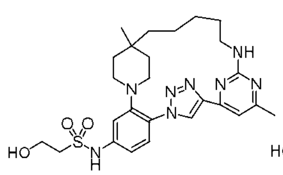
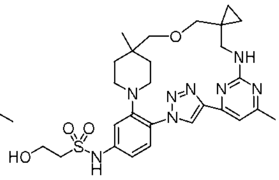

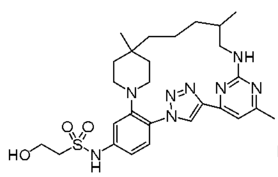
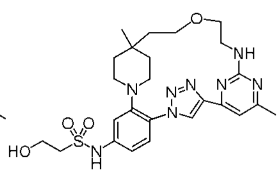
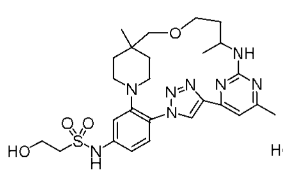
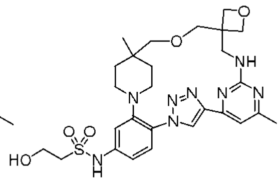

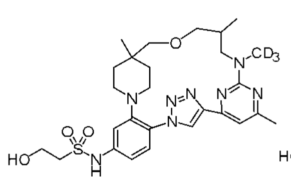
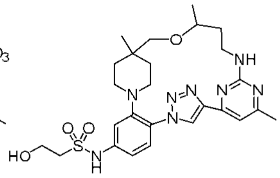
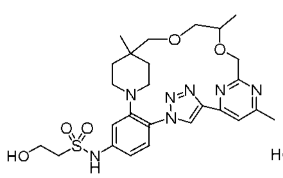
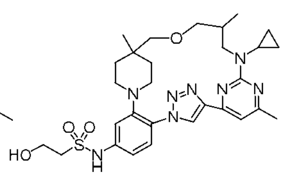

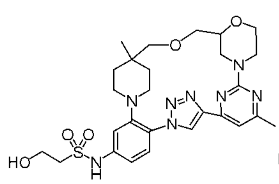
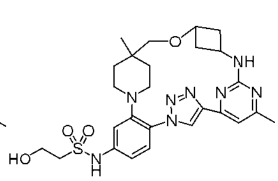
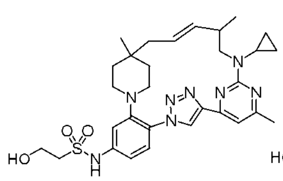
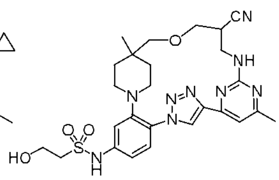

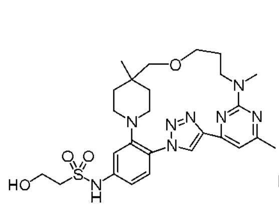
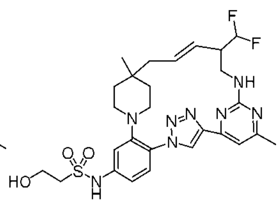
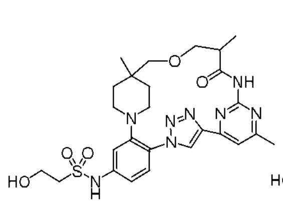
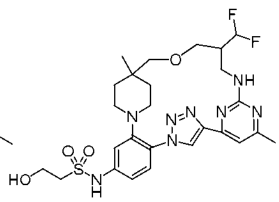

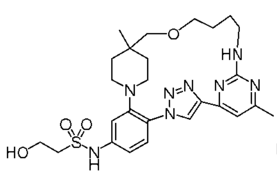
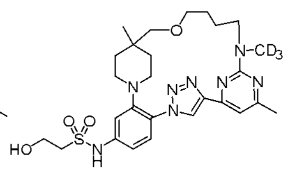
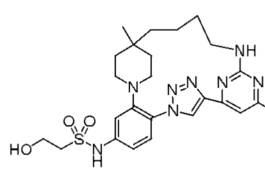
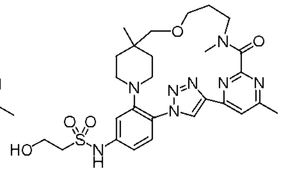

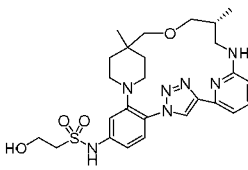
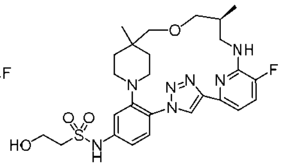
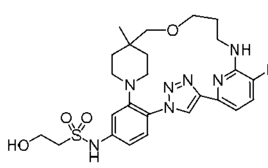
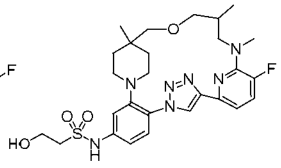

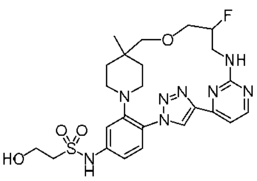
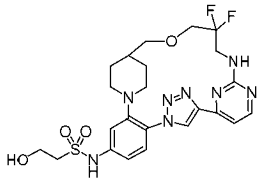
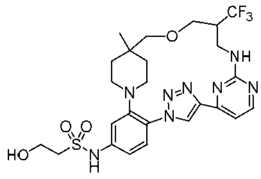
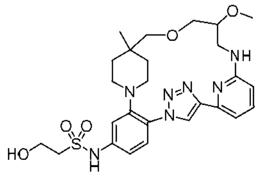

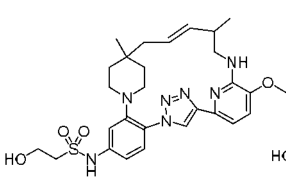
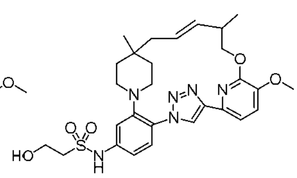
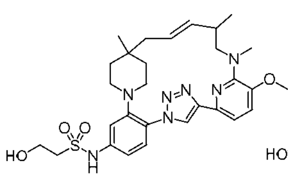
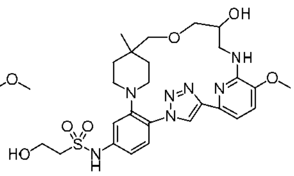

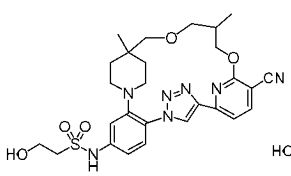
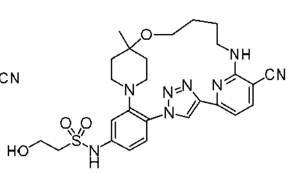
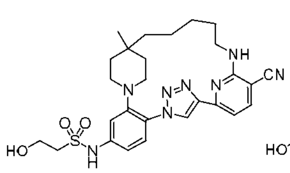
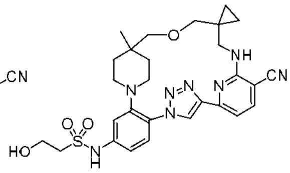

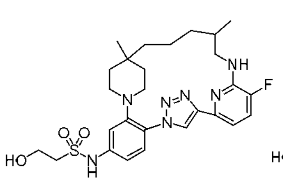
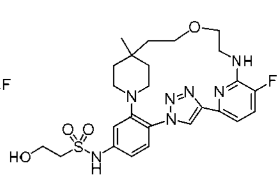
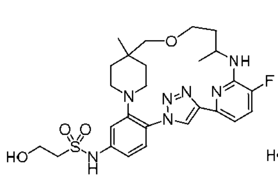
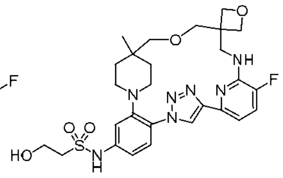

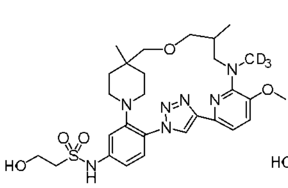
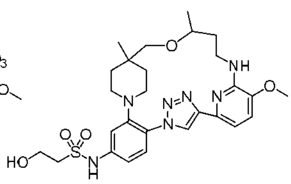
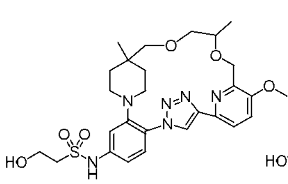
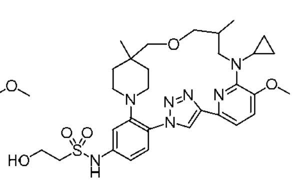

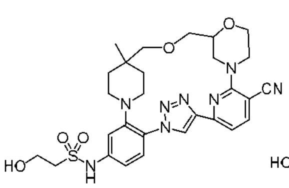
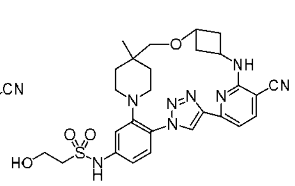
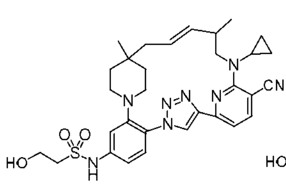
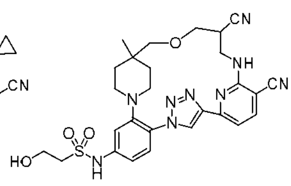

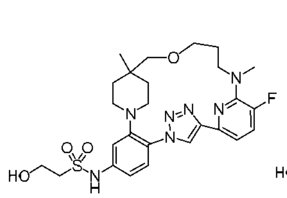 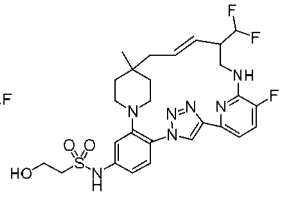 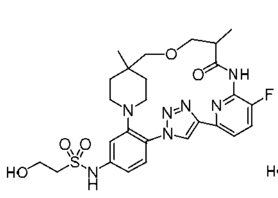 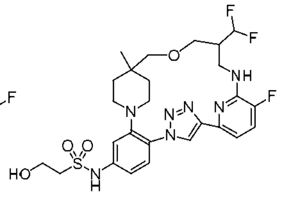

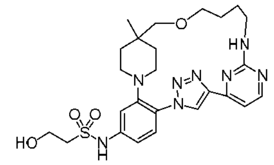 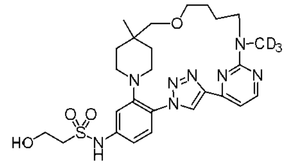 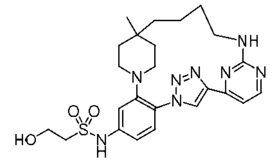 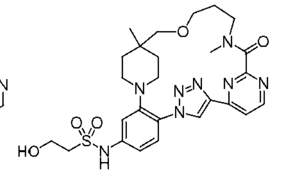

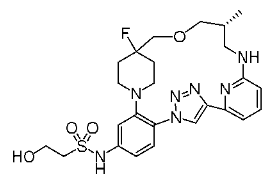 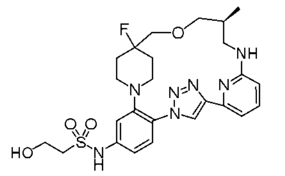 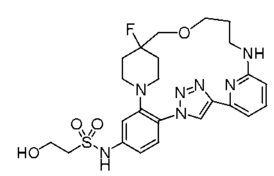 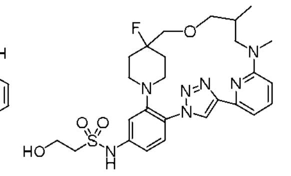

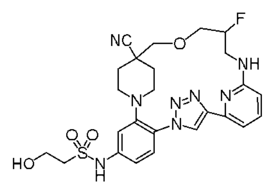 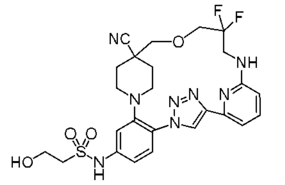 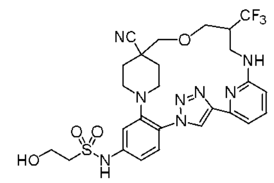 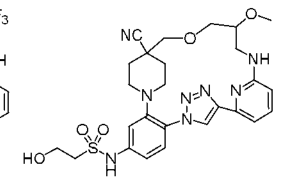

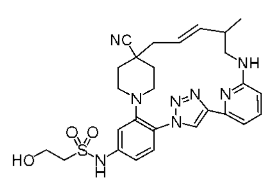 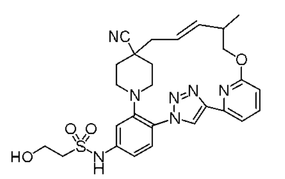 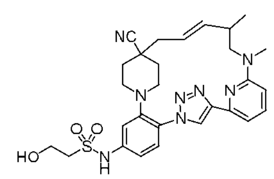 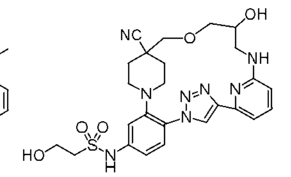

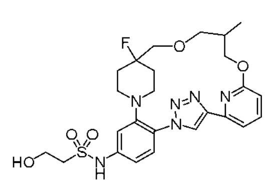 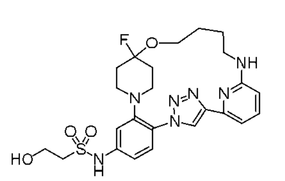 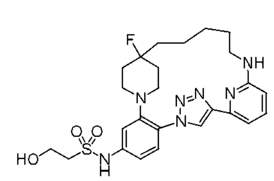 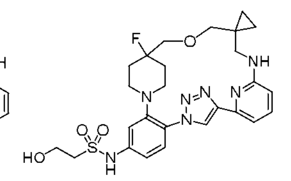

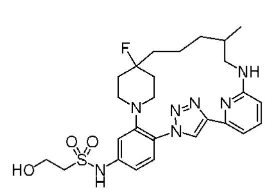 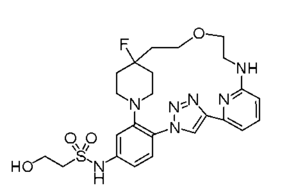 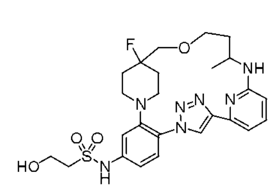 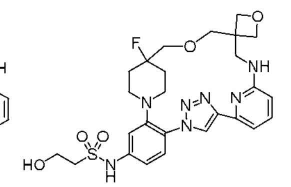

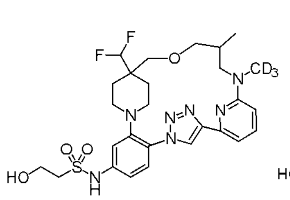 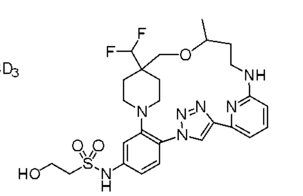 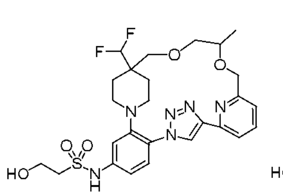 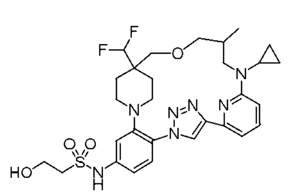

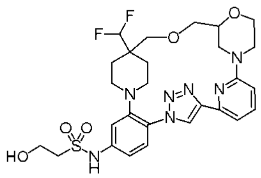
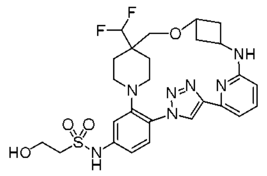
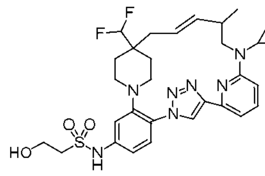
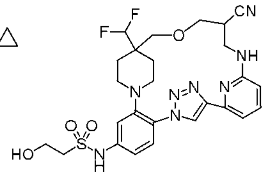

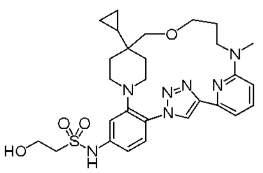
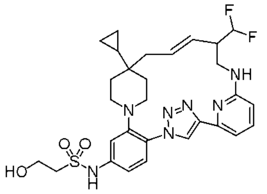
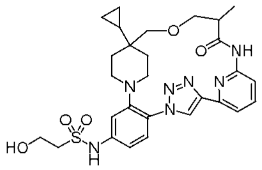
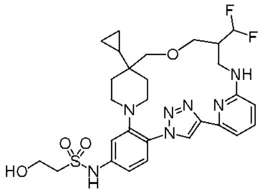

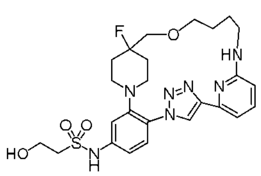
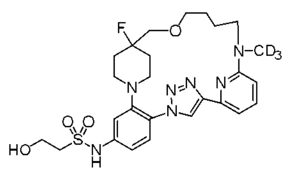
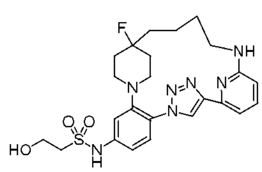
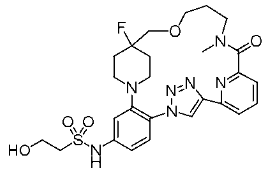

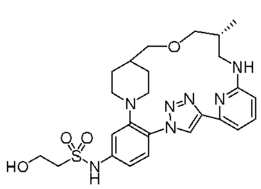
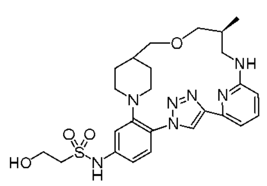
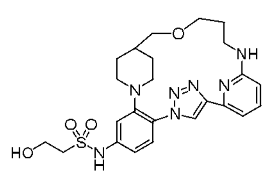
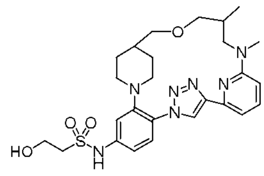

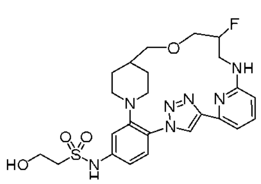
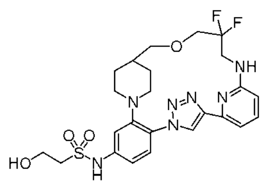
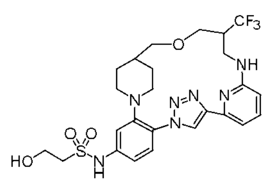
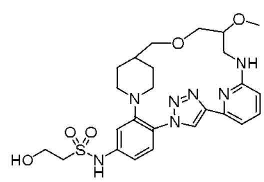

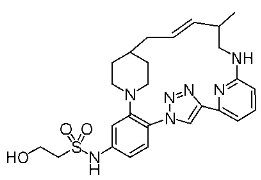
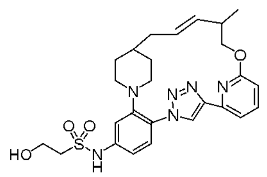
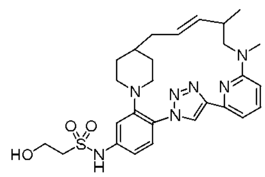
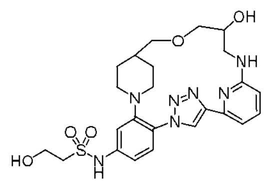

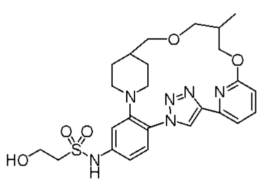
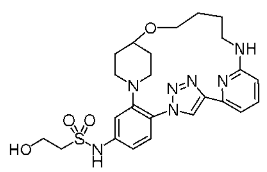
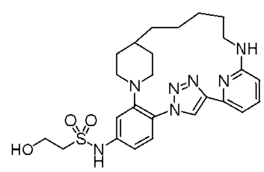
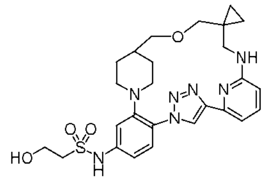

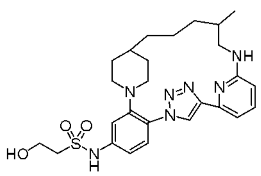
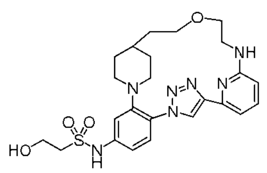
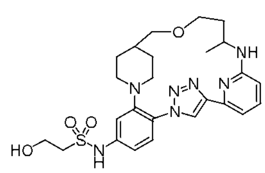
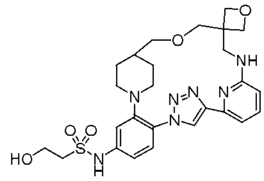

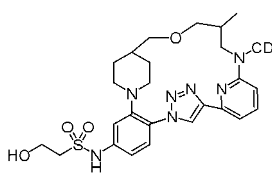
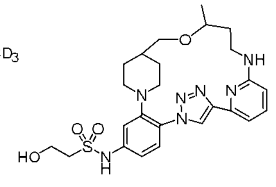
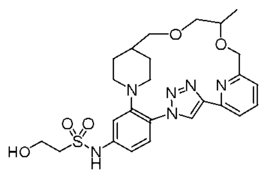
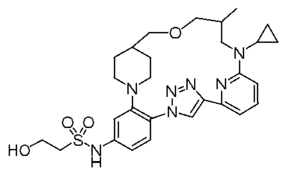
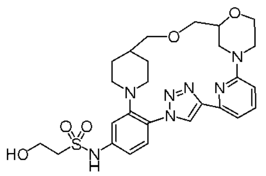
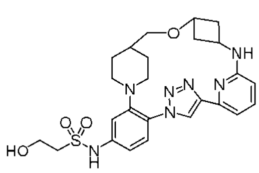
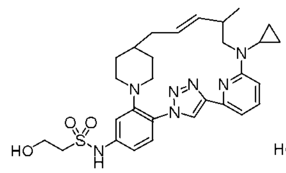
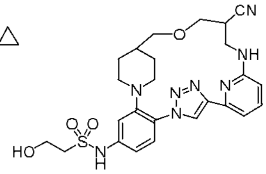
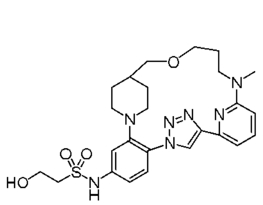
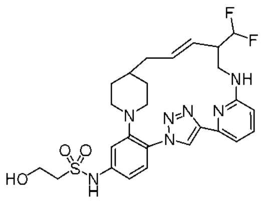
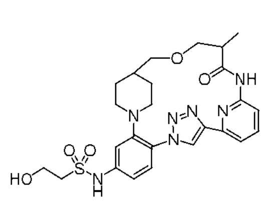
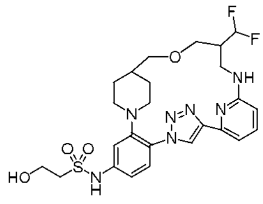
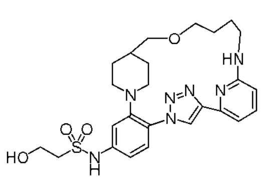
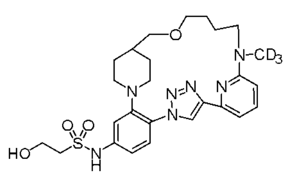
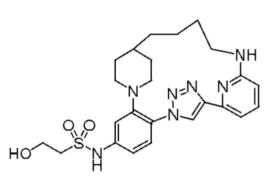
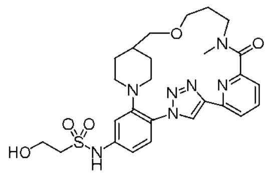
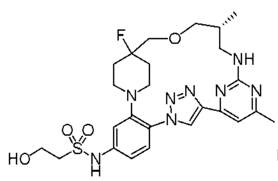
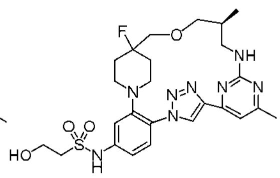
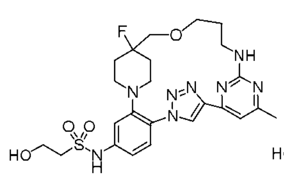
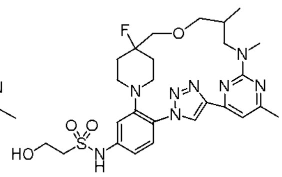
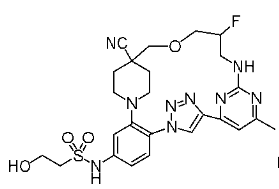
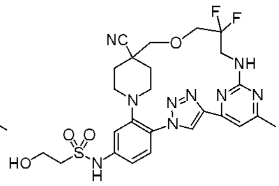
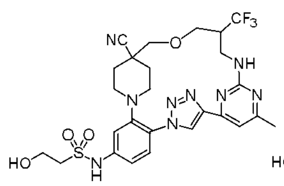
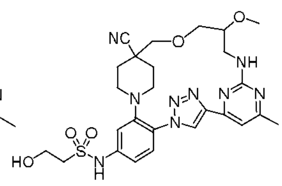
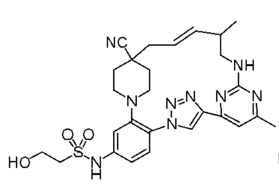
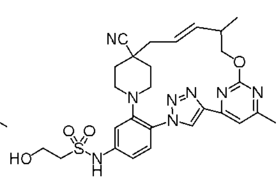
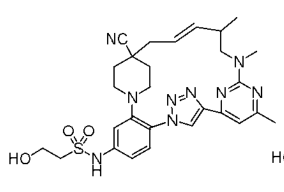
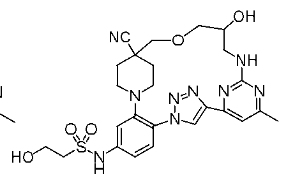
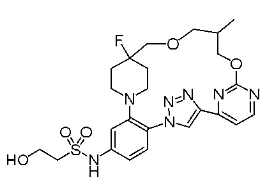
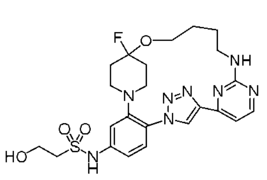
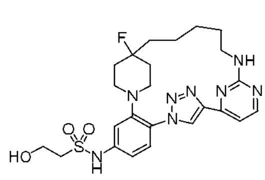
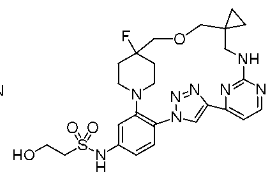

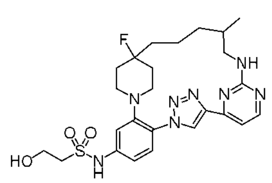
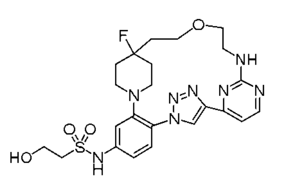
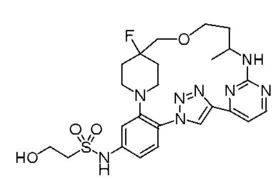
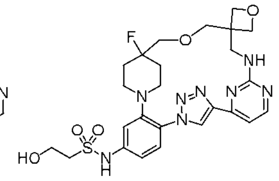
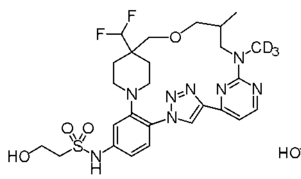
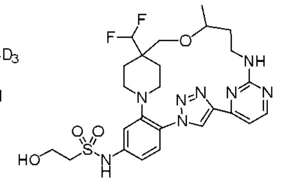
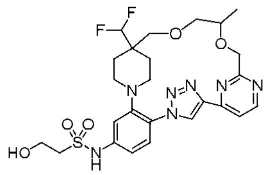
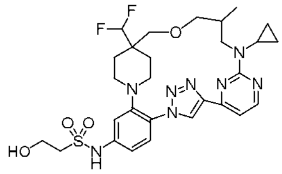
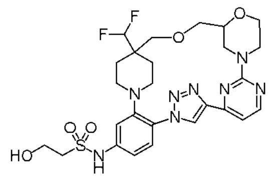
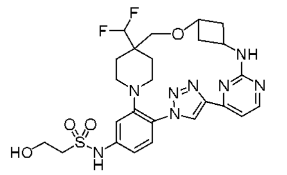
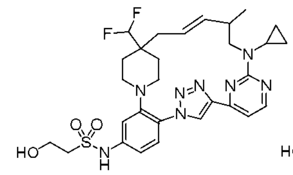
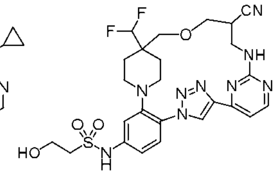
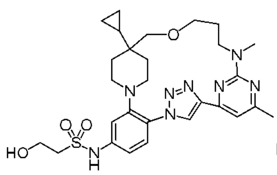
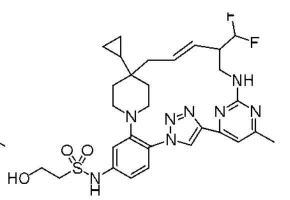
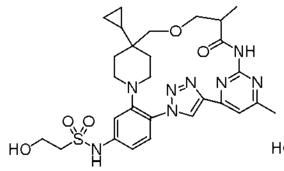
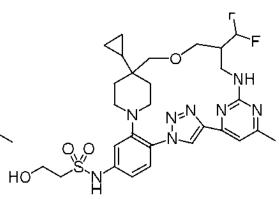
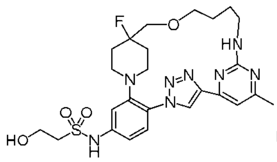
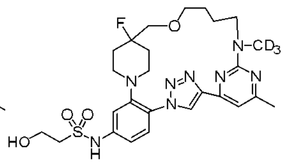
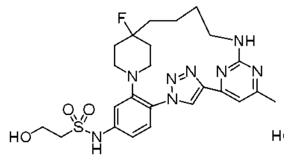
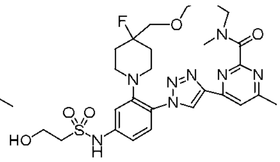
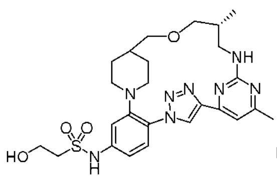
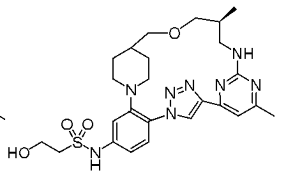
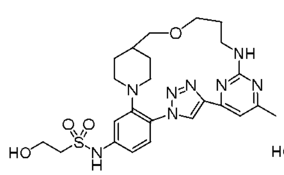
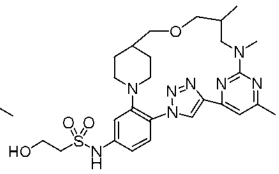
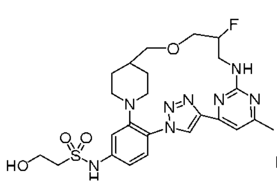
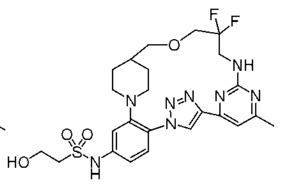
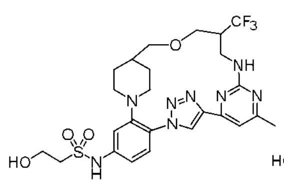
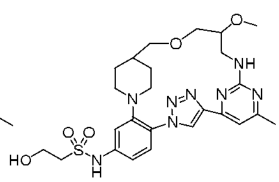
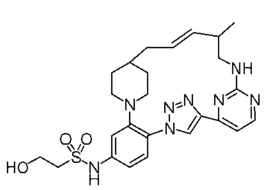
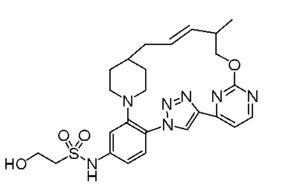
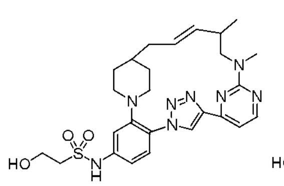
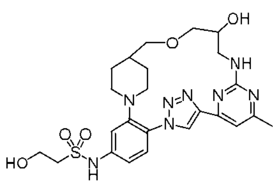

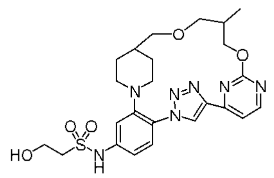
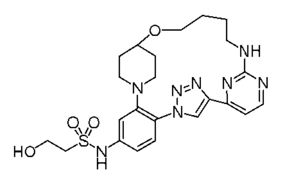
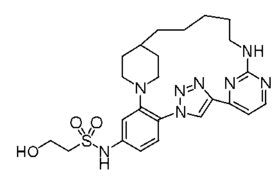
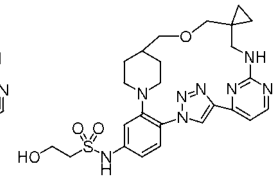
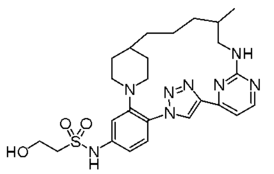
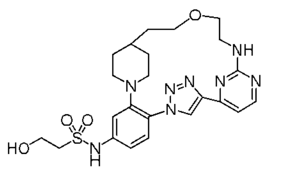
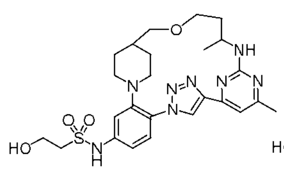
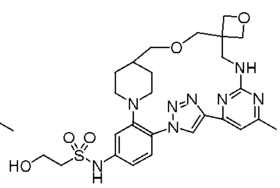
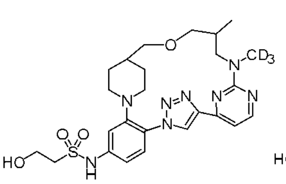
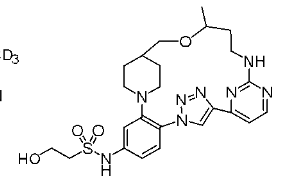
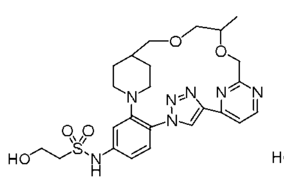
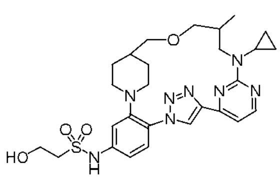
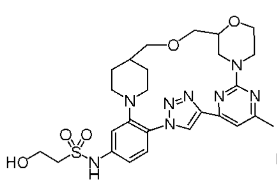
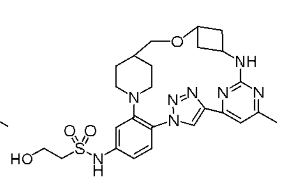
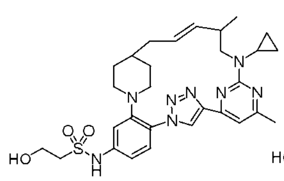
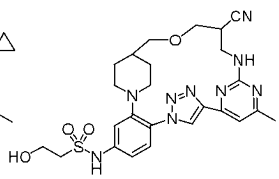
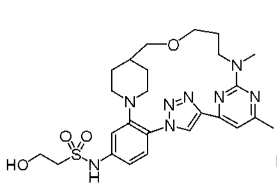
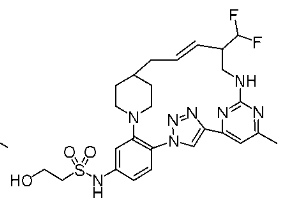
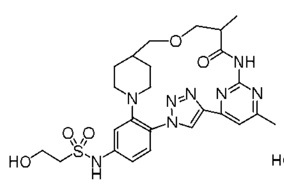
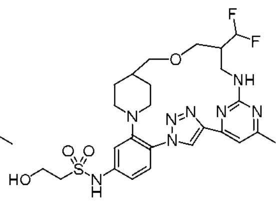
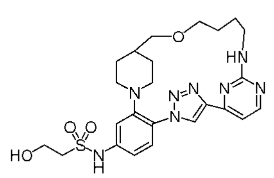
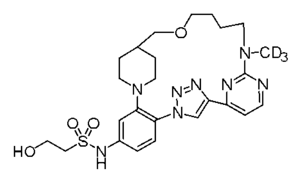
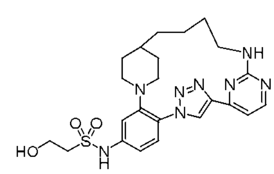
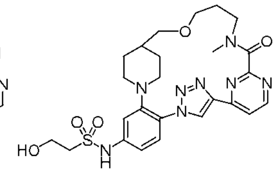
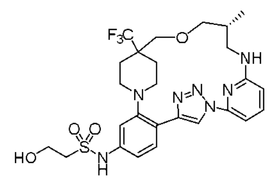
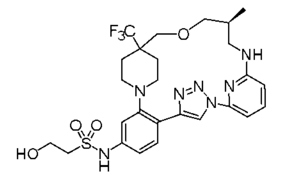
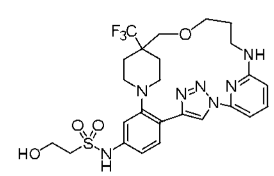
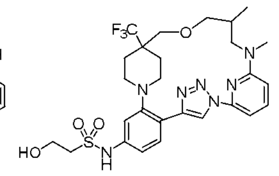
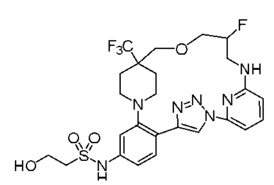
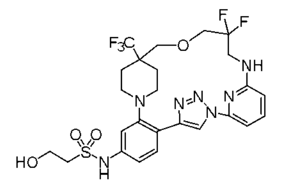
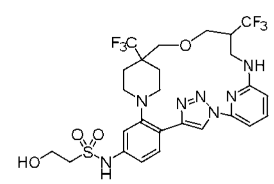
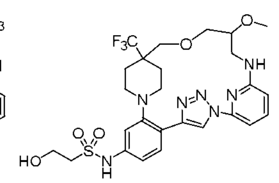

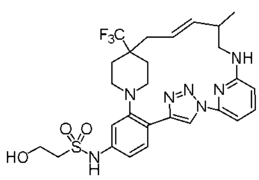
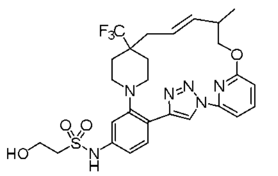
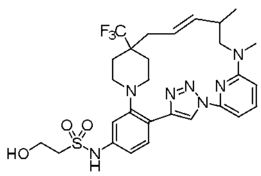
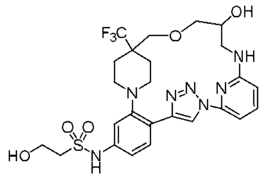
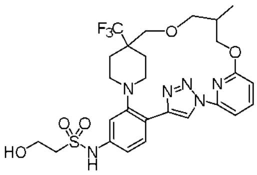
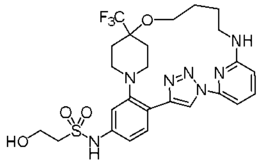
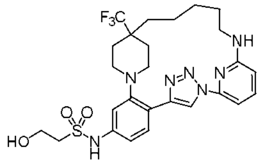
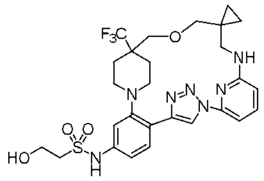
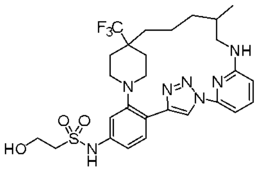
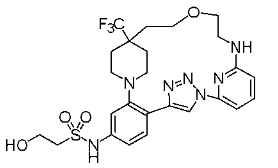
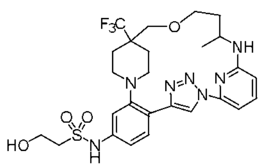
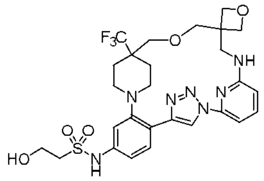
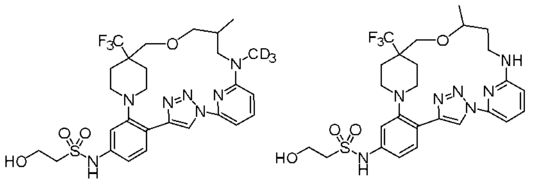
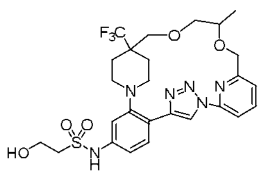
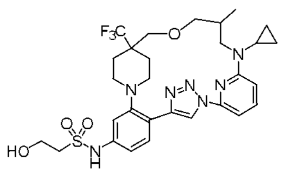
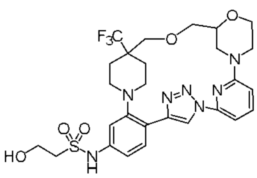
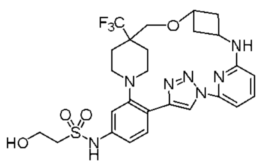
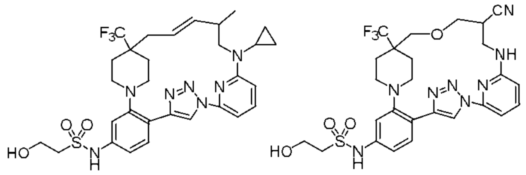
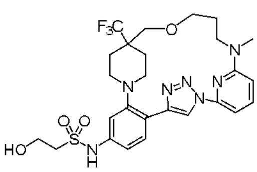
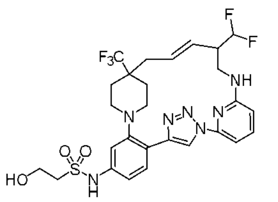
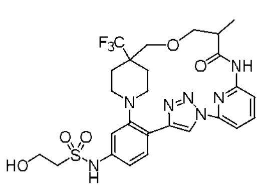
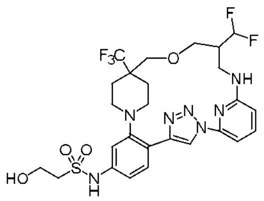
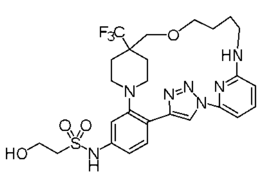
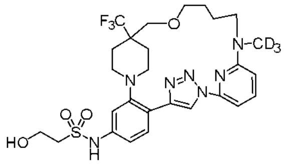
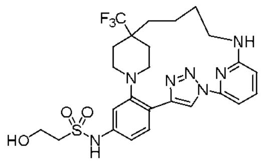
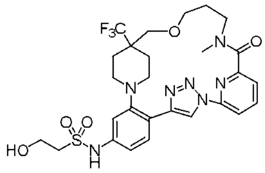
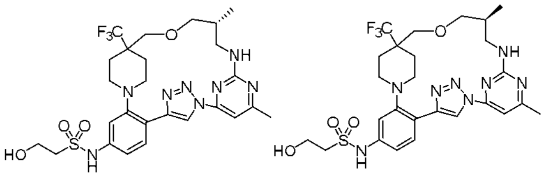
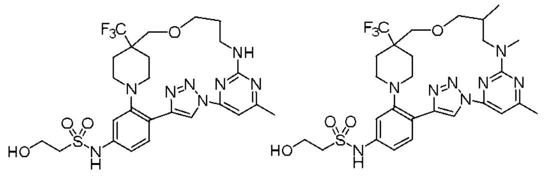

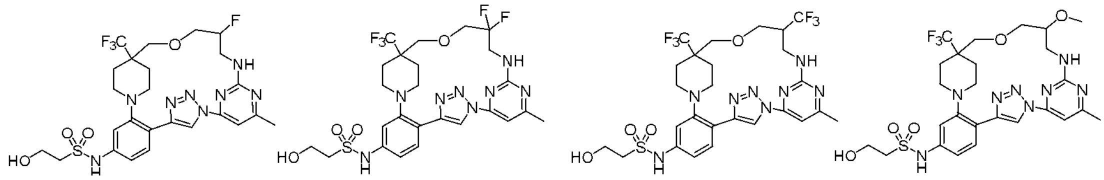

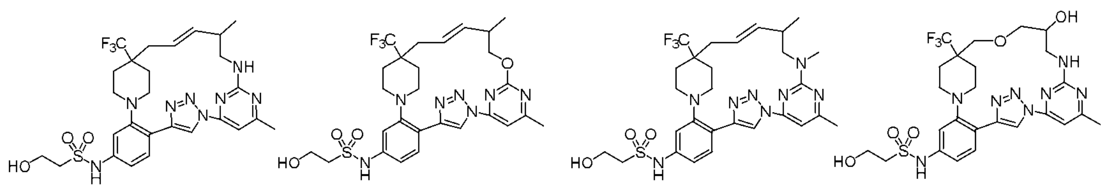

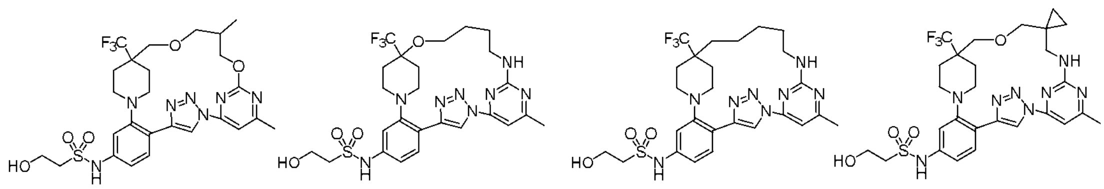

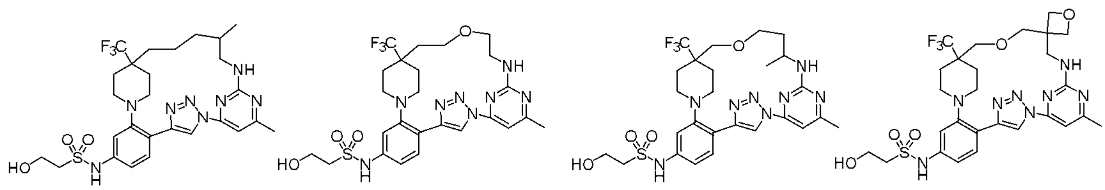

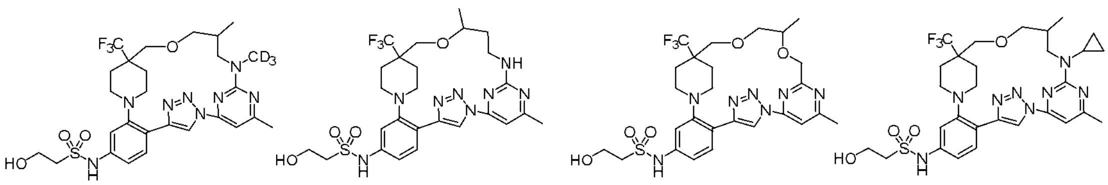

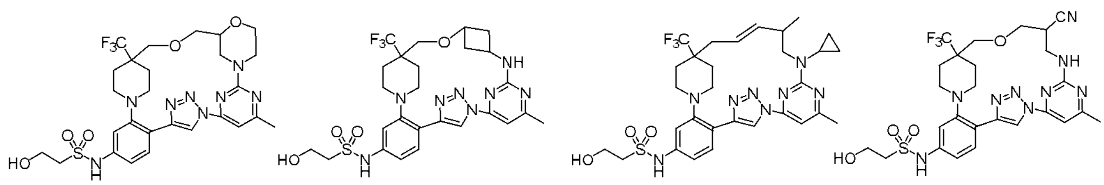

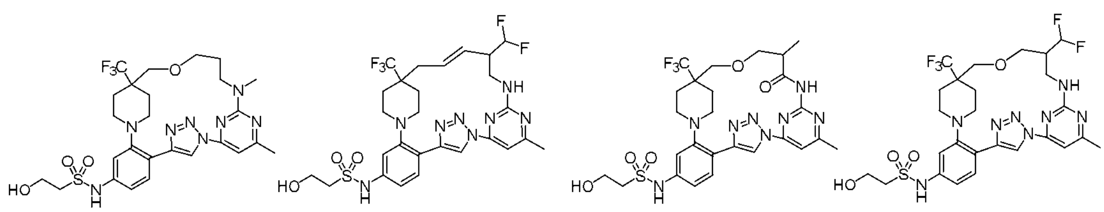

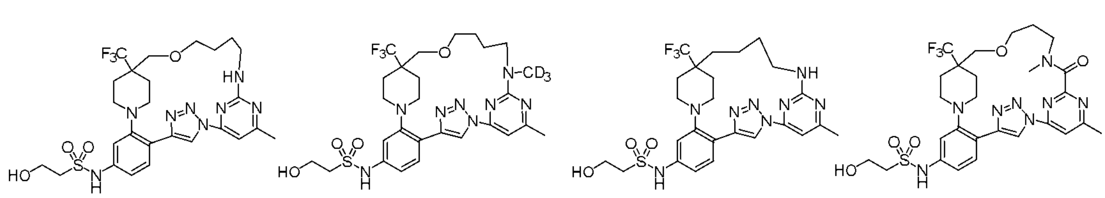

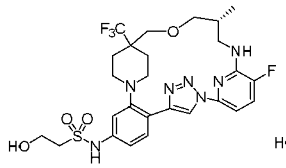
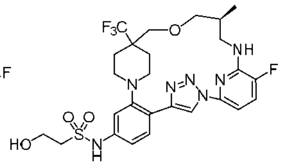
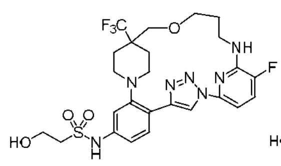
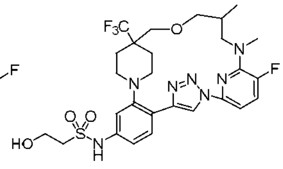
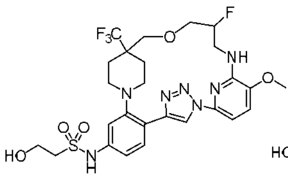
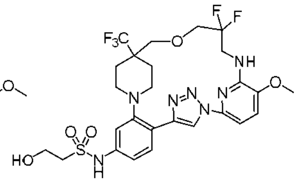
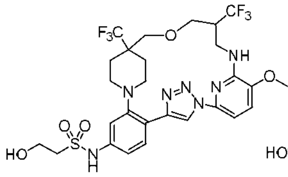
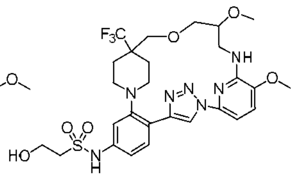
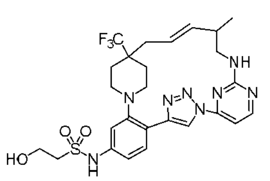
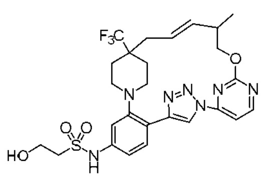
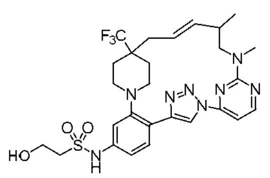
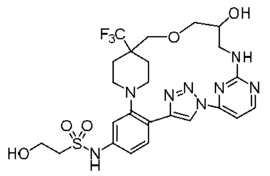
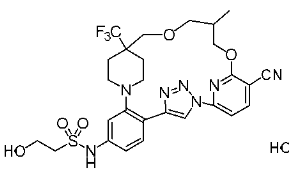
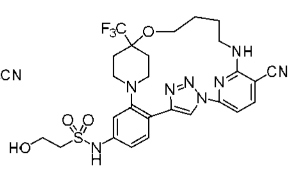
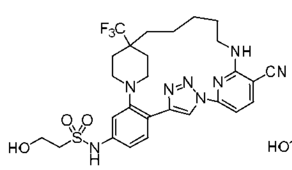
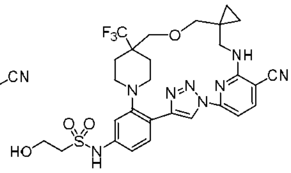
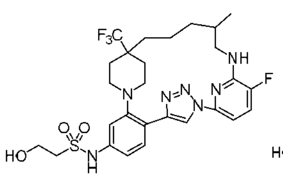
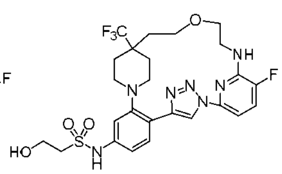
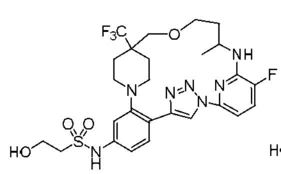
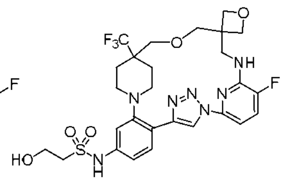
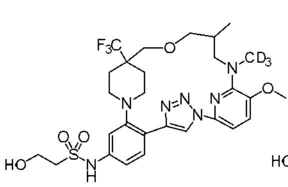
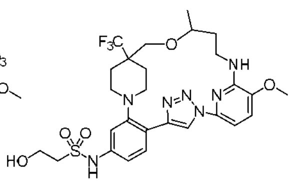
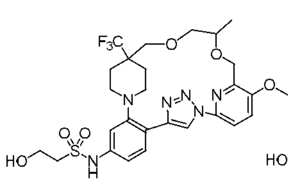
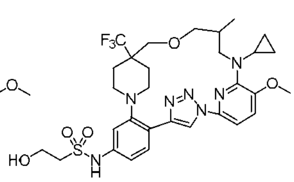
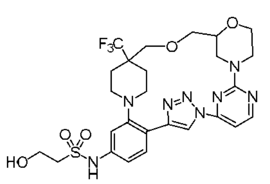
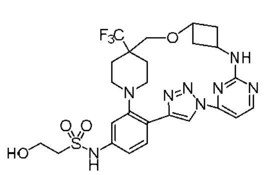
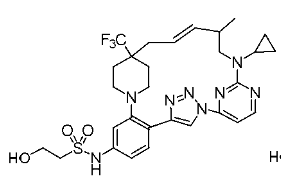
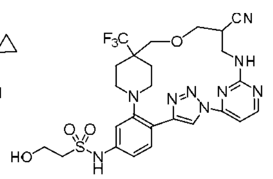
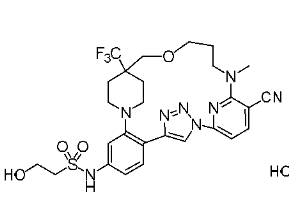
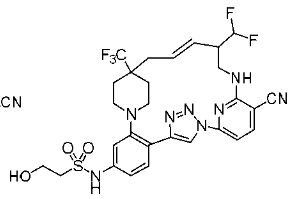
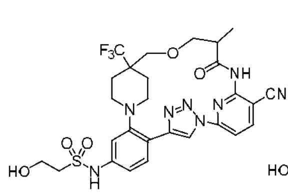
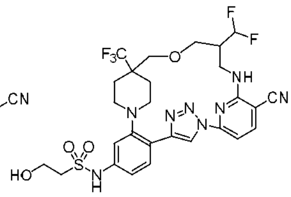

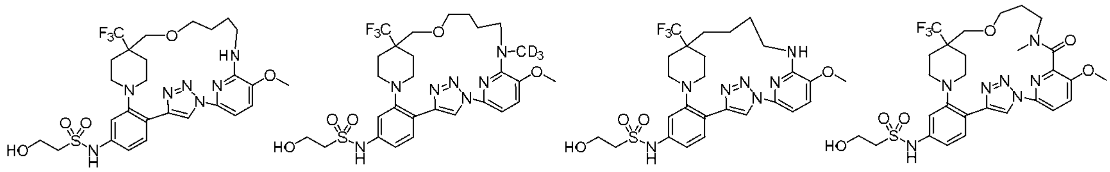

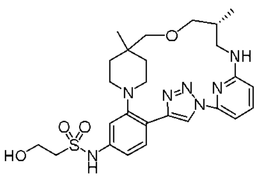

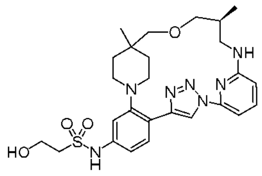

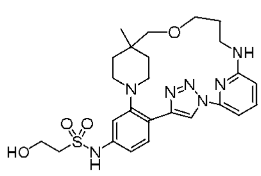

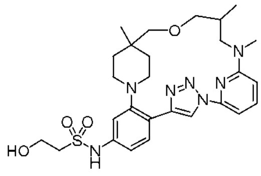

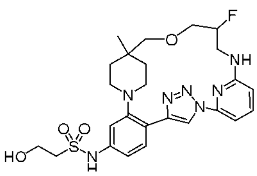

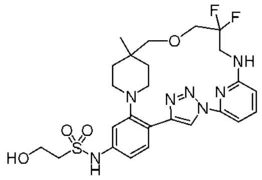

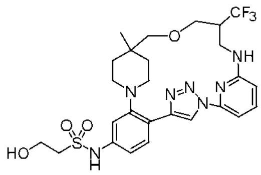

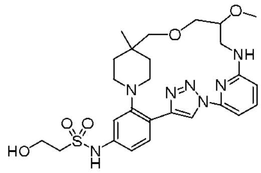

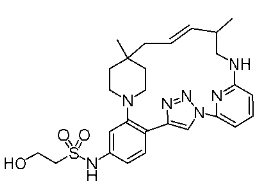

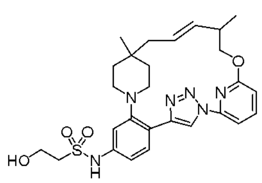

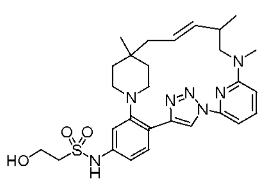

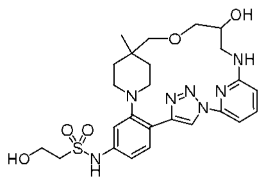

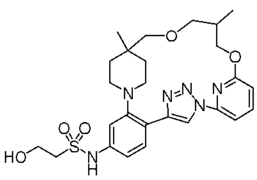

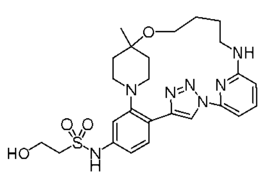

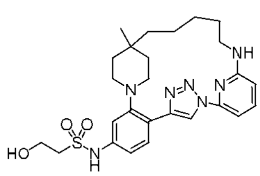

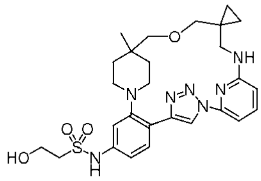

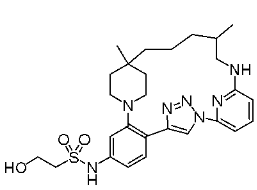

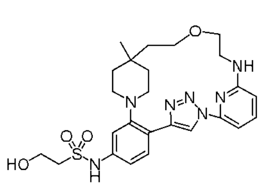

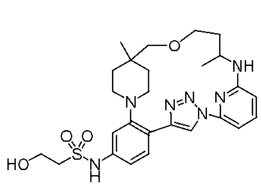

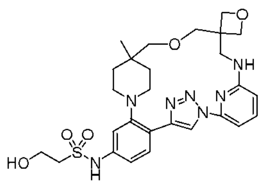

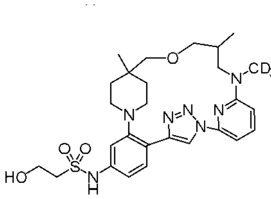

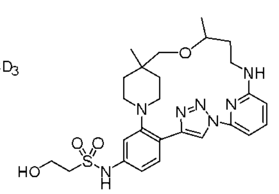

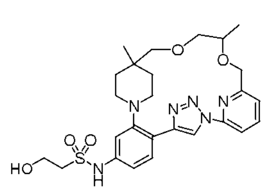

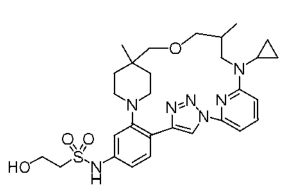

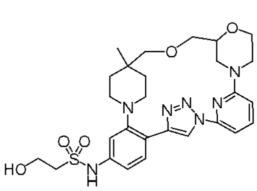

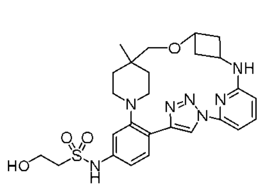

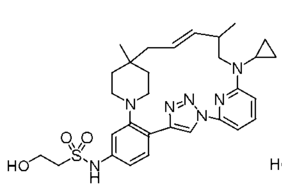

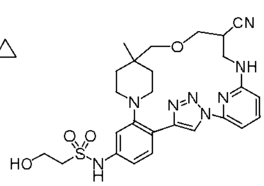

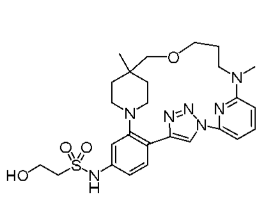
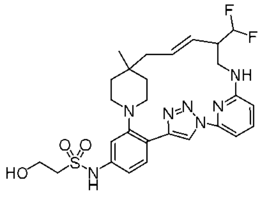
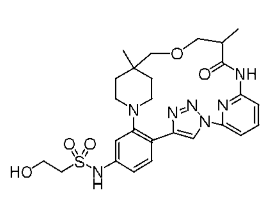
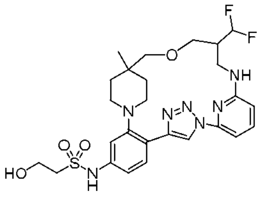

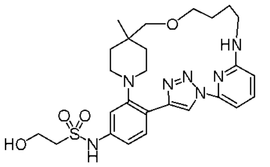
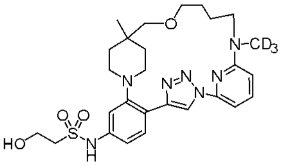
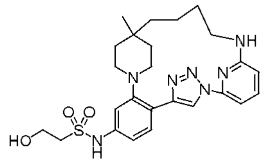
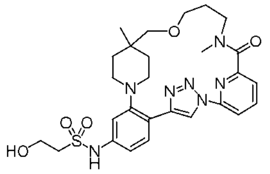

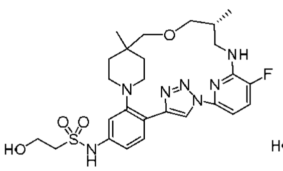
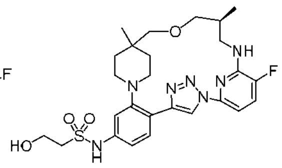
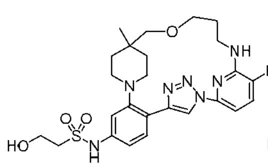
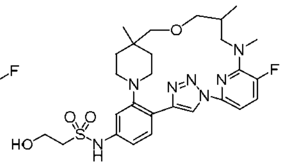

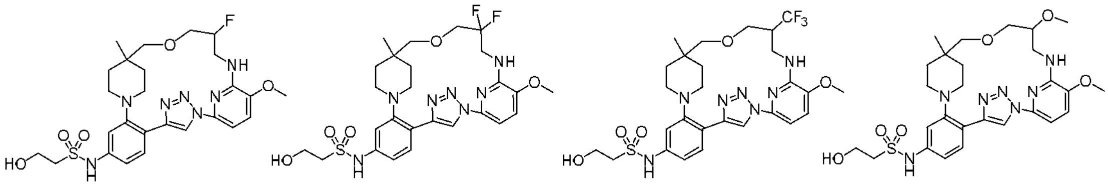

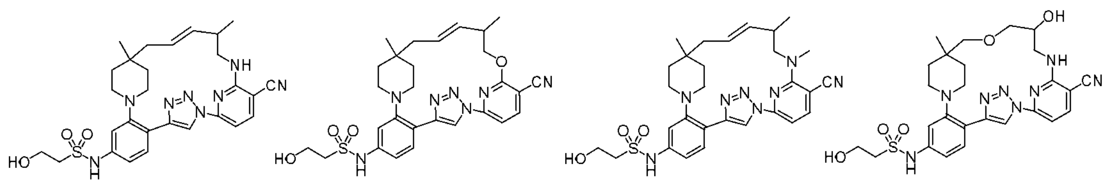

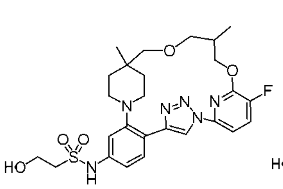
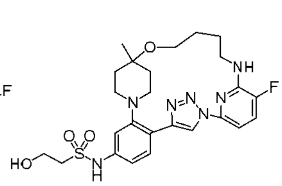
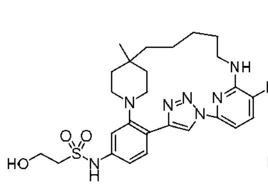
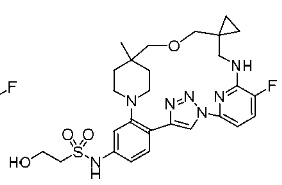

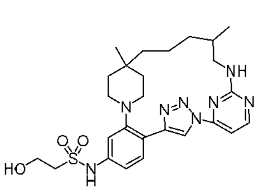
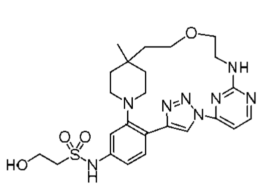
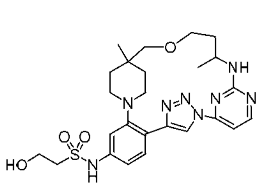
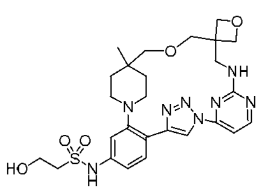

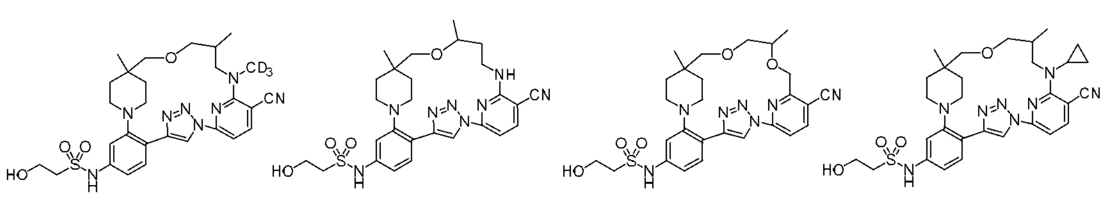

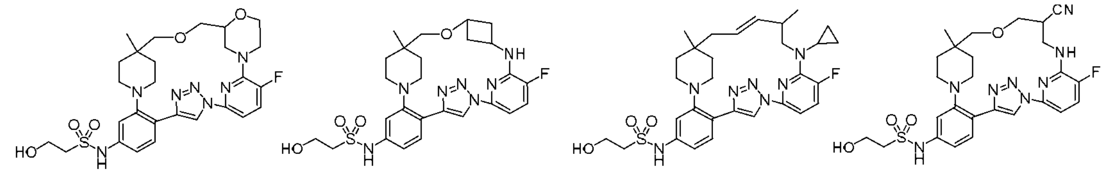

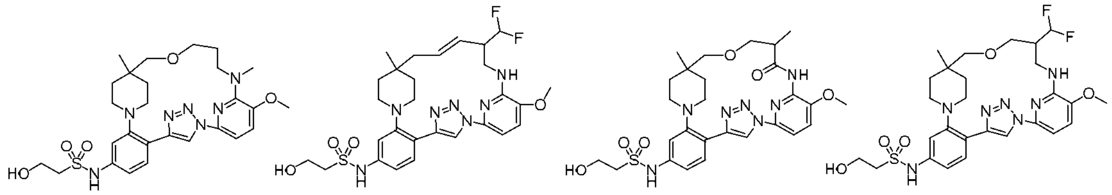

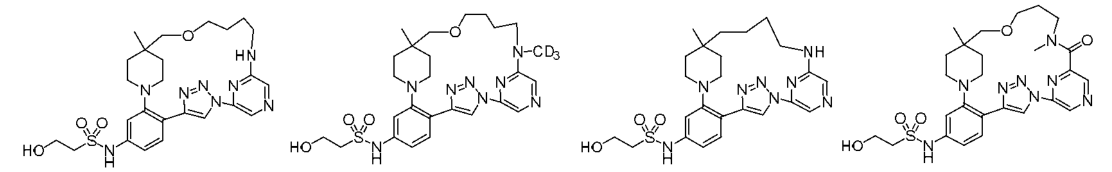

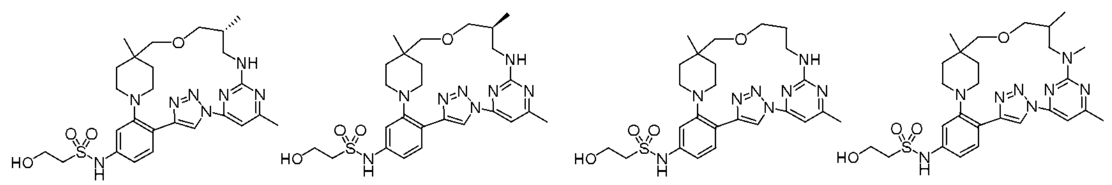

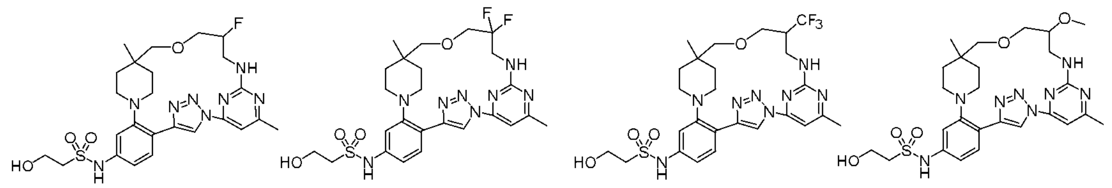

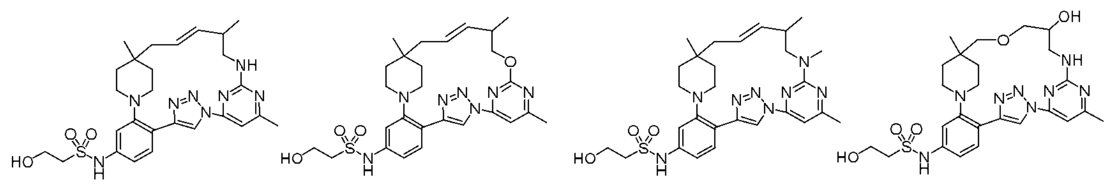

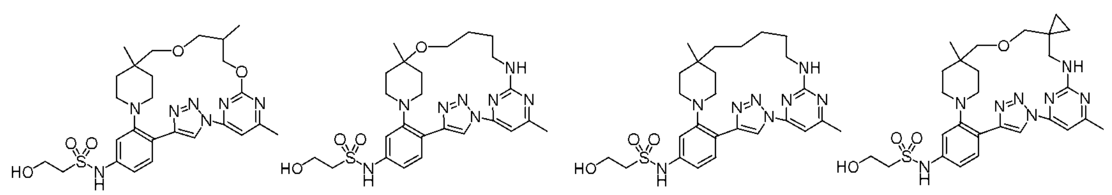

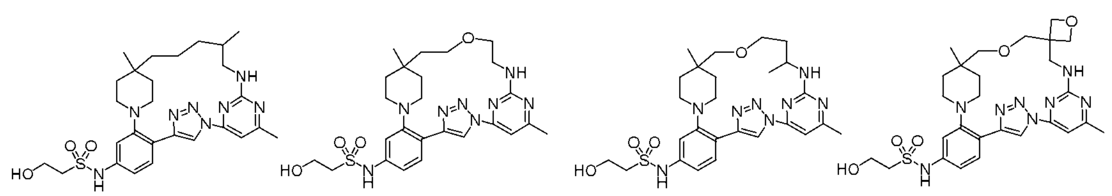

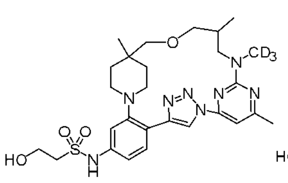 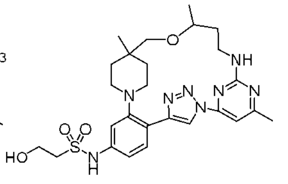 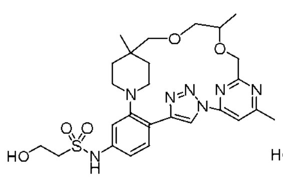 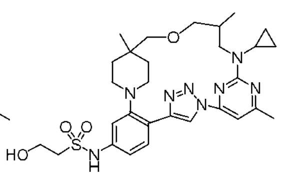

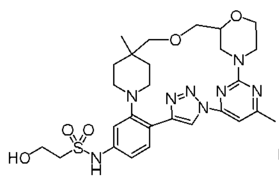 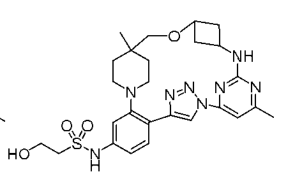 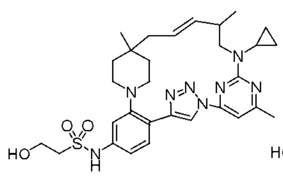 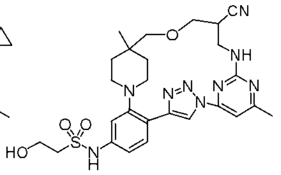

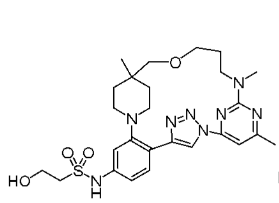 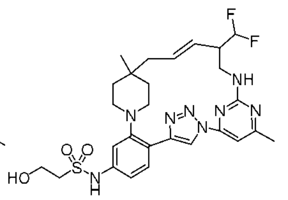 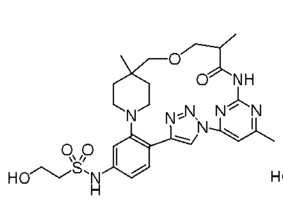 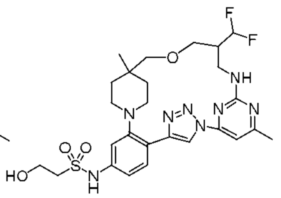

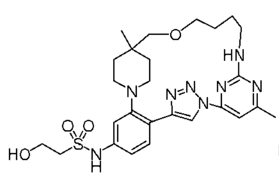 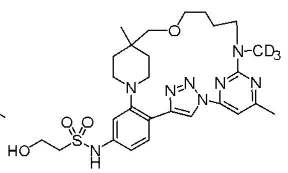 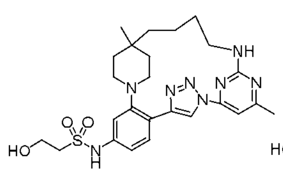 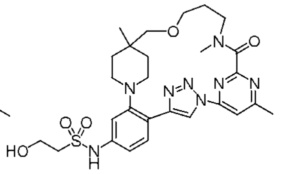

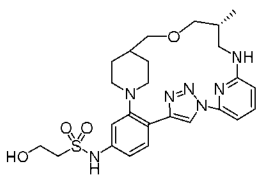 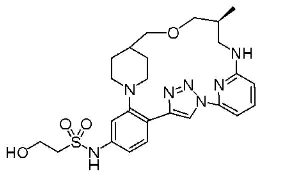 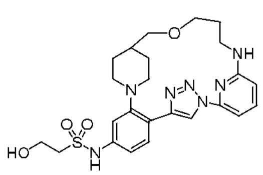 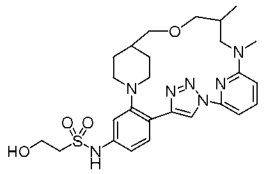

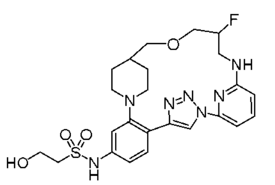 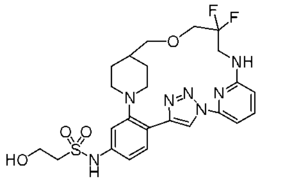 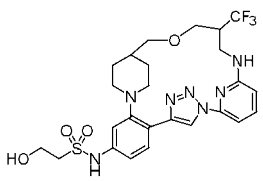 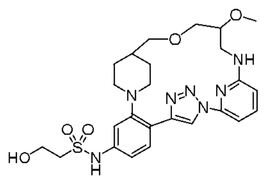

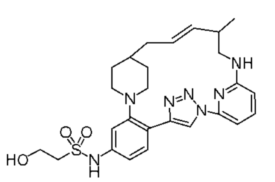 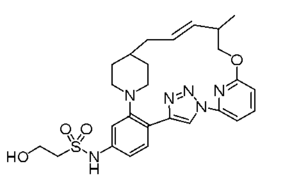 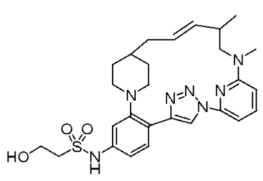 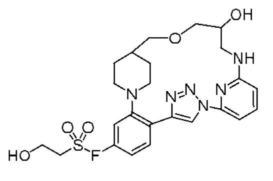

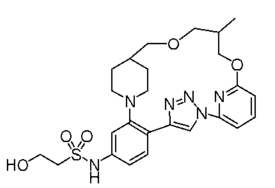 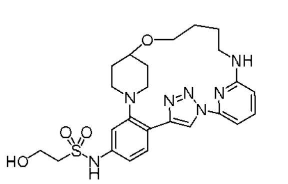 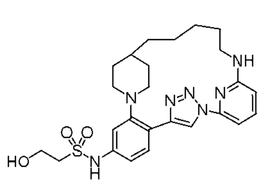 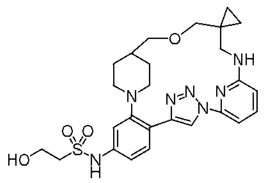

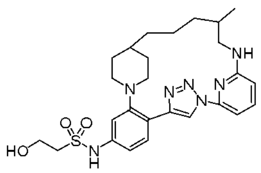
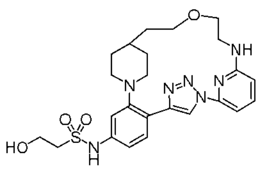
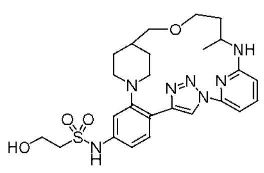
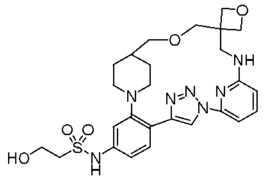

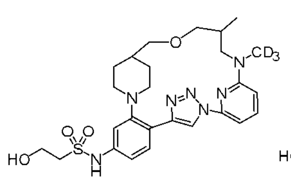
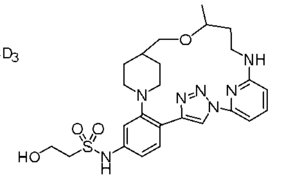
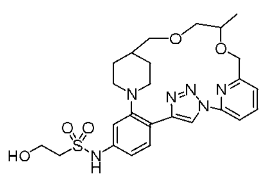
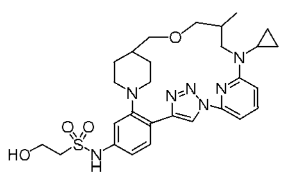

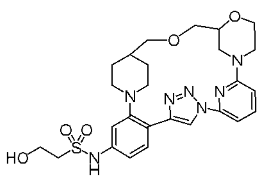
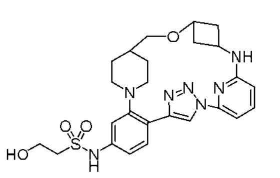
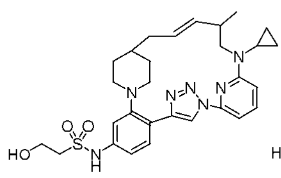
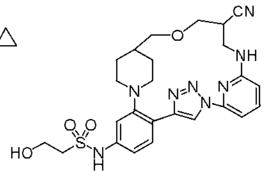

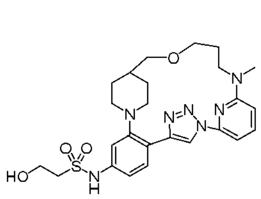
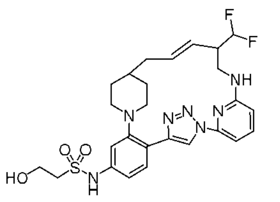
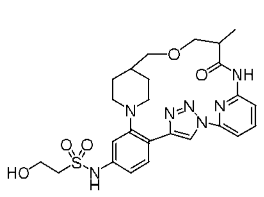
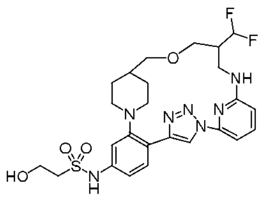

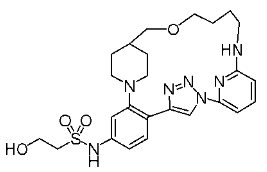
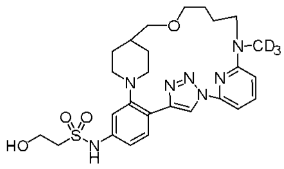
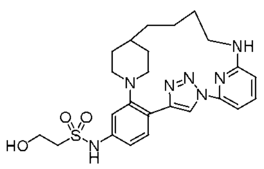
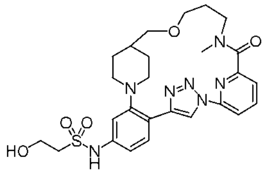

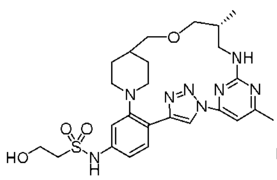
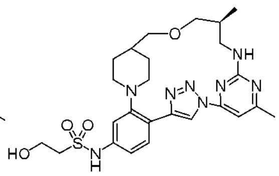
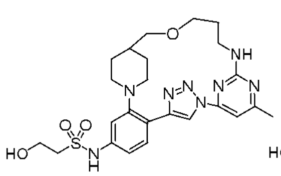
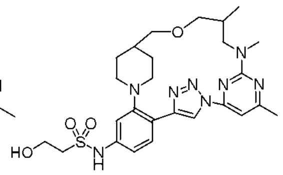

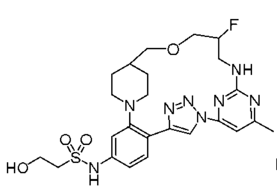
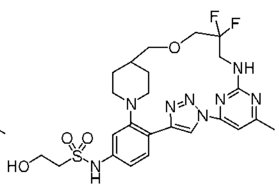
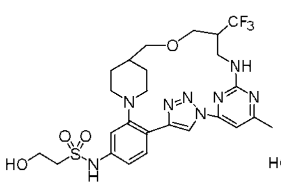
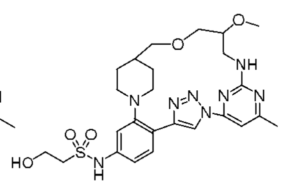

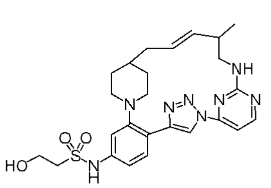
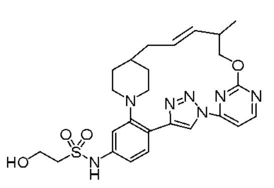
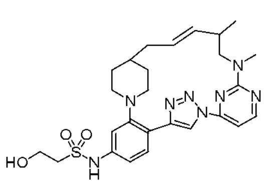
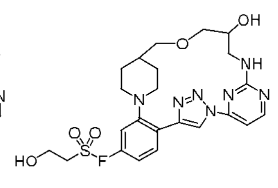

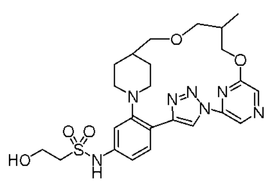
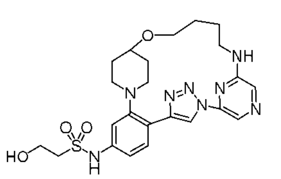
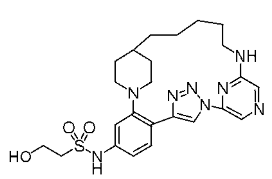
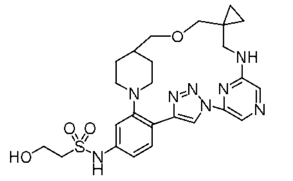

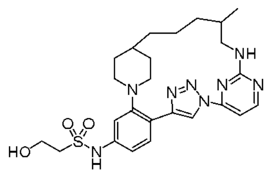
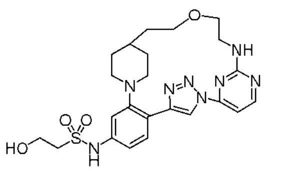
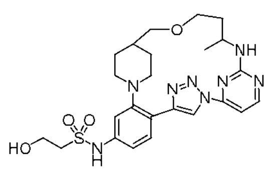
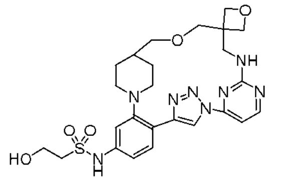

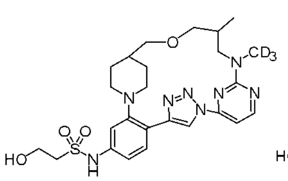
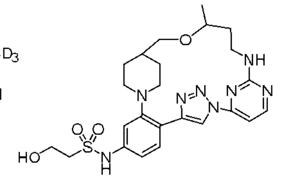
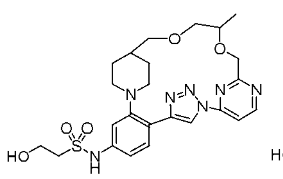
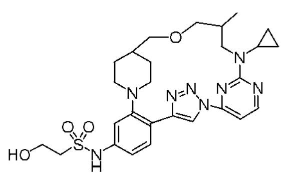

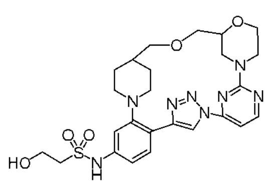
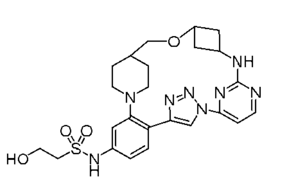
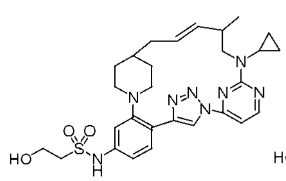
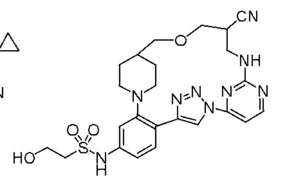

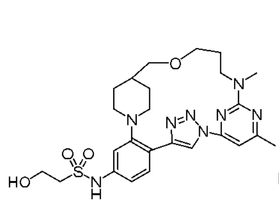
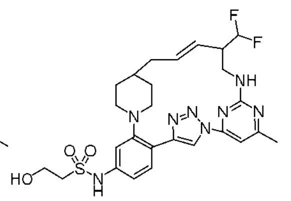
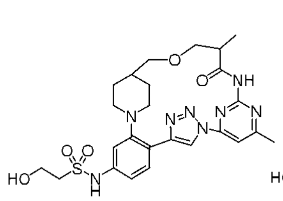
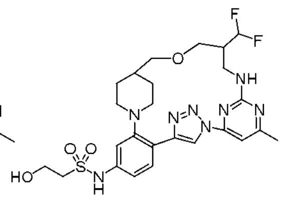

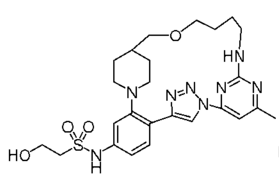
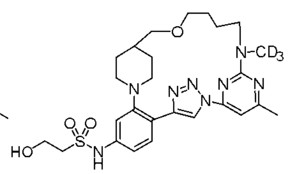
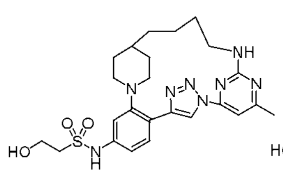
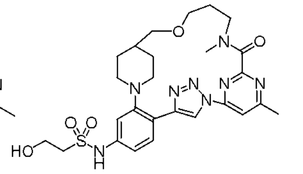

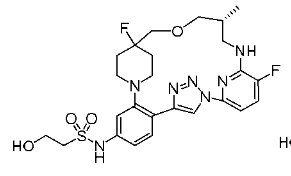
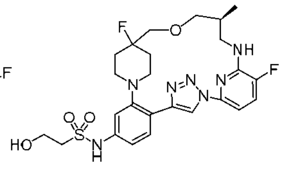
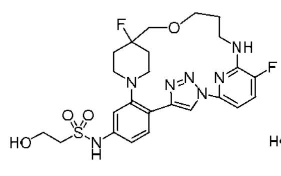
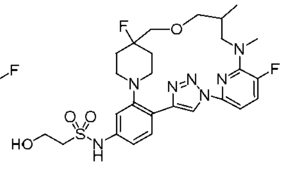

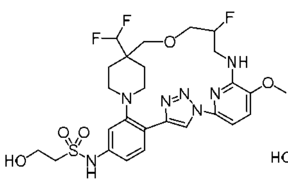
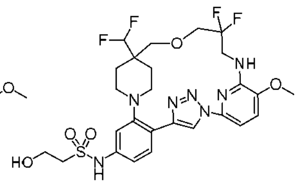
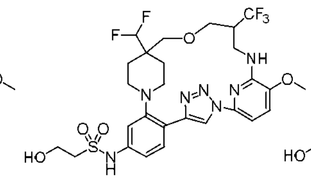
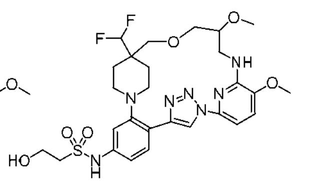

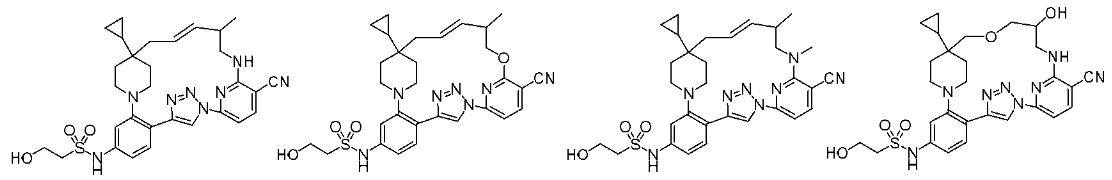

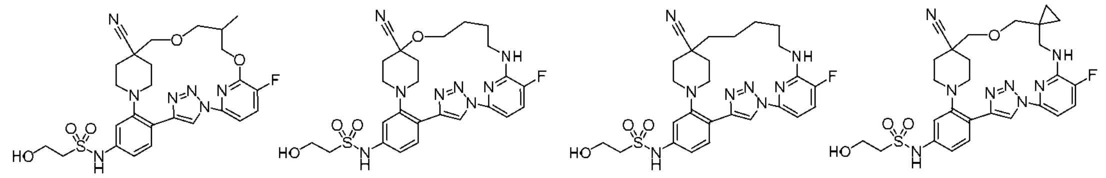

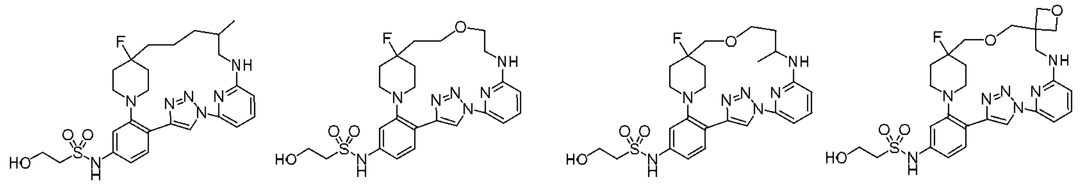

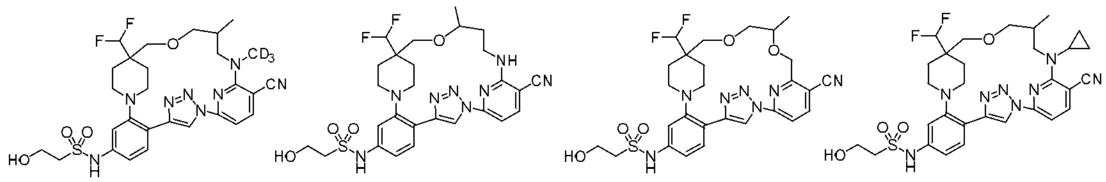

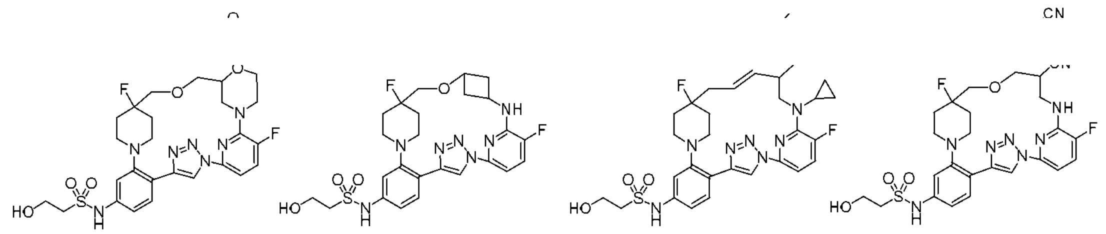

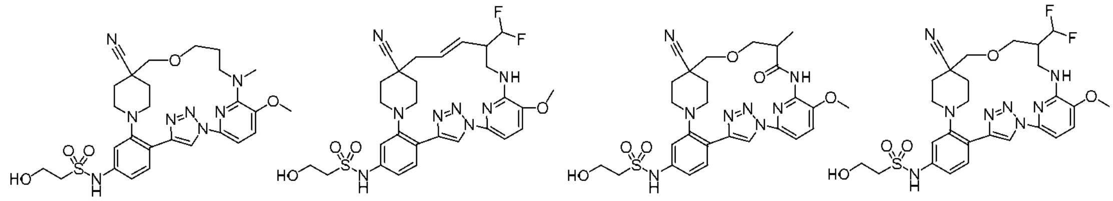

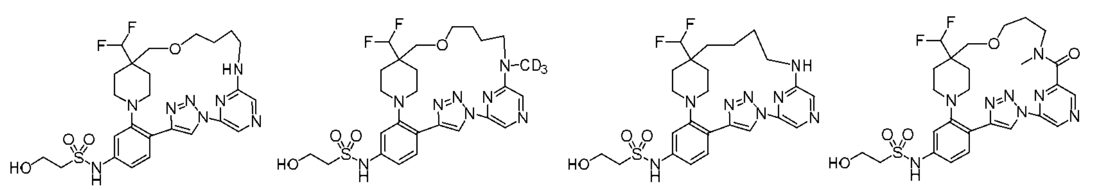

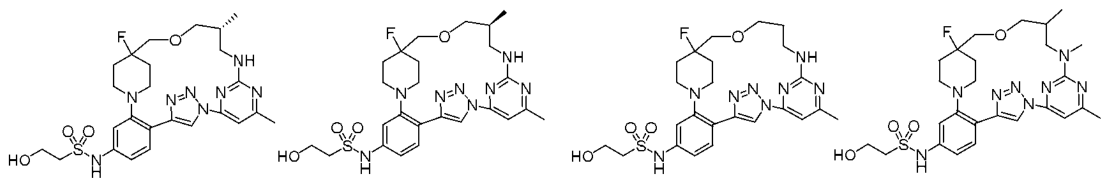

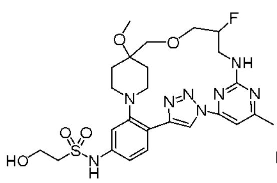 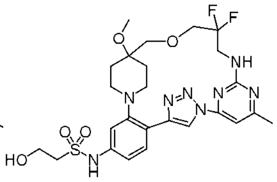 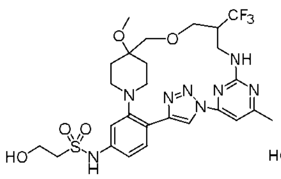 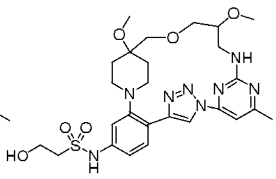

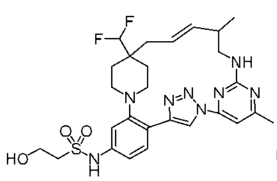 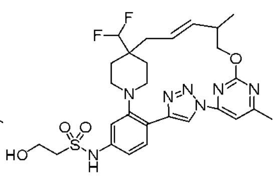 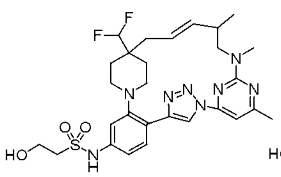 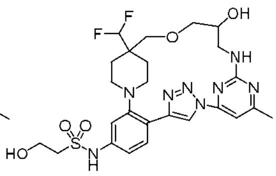

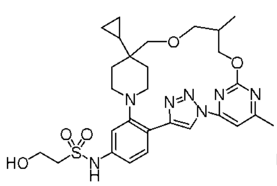 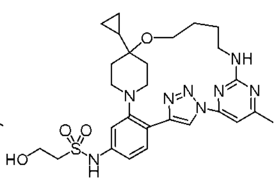 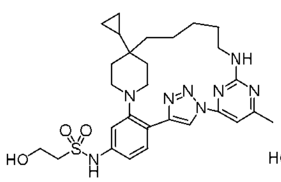 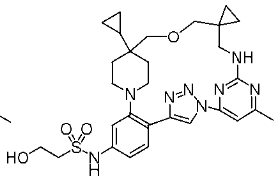

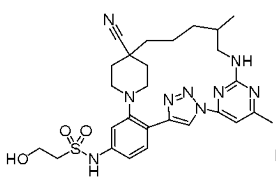 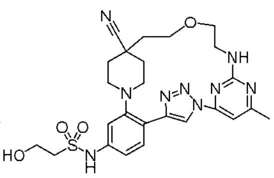 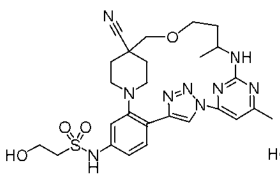 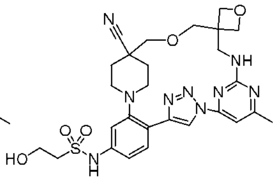

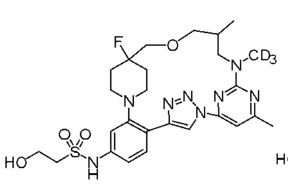 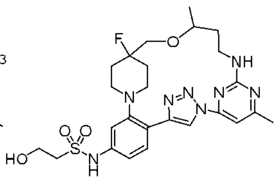 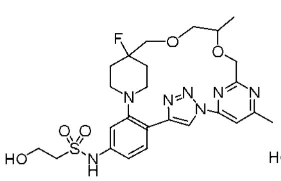 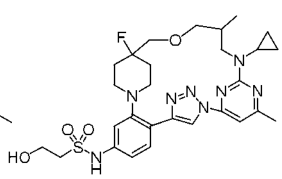

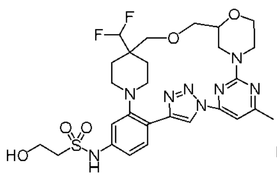 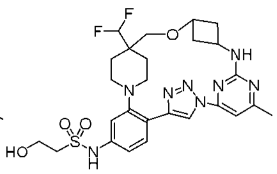 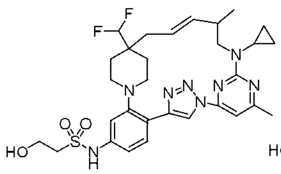 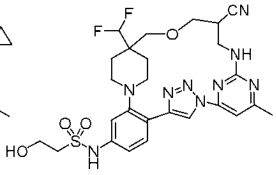

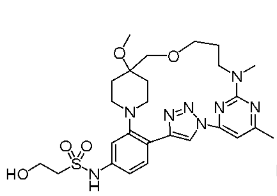 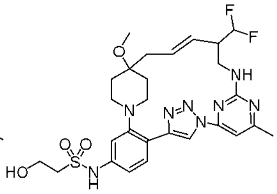 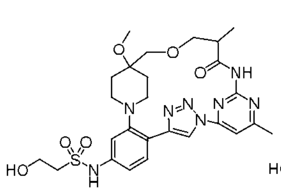 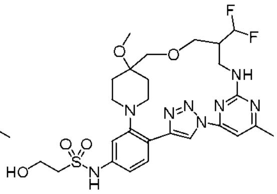

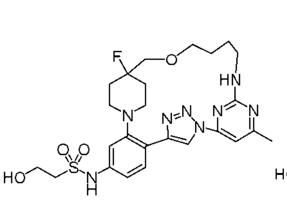 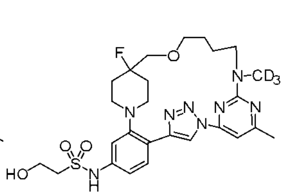 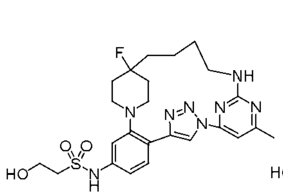 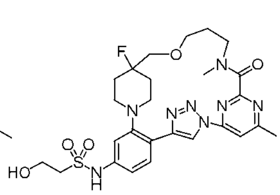

**191.**
A pharmaceutical composition comprising a compound according to any one of claims 1-162, its pharmaceutically acceptable salt, stereoisomer, isotopic isomer and tautomeric form thereof.

## INTERNATIONAL SEARCH REPORT

International application No.
**PCT/CN2024/121098**

### A. CLASSIFICATION OF SUBJECT MATTER

C07D471/22(2006.01)i; C07D471/04(2006.01)i; C07D401/14(2006.01)i; C07D413/14(2006.01)i; C07D405/14(2006.01)i; A61K31/497(2006.01)i; A61K31/444(2006.01)i; A61P35/00(2006.01)i

According to International Patent Classification (IPC) or to both national classification and IPC

### B. FIELDS SEARCHED

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNTXT, ENTXT, CNKI, ISI Web of science, Registry(STN), Caplus(STN), Marpat(STN): 上海湃隆生物科技有限公司, KIF18, 抑制剂, 驱动蛋白, 癌, 肿瘤, inhibitor, kinesin, cancer, tumor, 结构式检索, structural formula search

### C. DOCUMENTS CONSIDERED TO BE RELEVANT

| Category* | Citation of document, with indication, where appropriate, of the relevant passages | Relevant to claim No. |
|---|---|---|
| PX | WO 2024153217 A1 (INSILICO MEDICINE IP LIMITED) 25 July 2024 (2024-07-25) claims 1-34, and description, paragraphs [00170]-[00171] | 1-191 |
| A | CN 113226473 A (AMGEN INC.) 06 August 2021 (2021-08-06) claims 1-42 | 1-191 |
| A | CN 116789637 A (SHANGHAI PAILONG BIOTECHNOLOGY CO., LTD.) 22 September 2023 (2023-09-22) claims 1-10 | 1-191 |
| A | CN 115594664 A (INSILICO MEDICINE LTD.) 13 January 2023 (2023-01-13) claims 1-10 | 1-191 |
| A | WO 2020132651 A1 (AMGEN INC.) 25 June 2020 (2020-06-25) claims 1-35 | 1-191 |
| A | WO 2020132649 A1 (AMGEN INC.) 25 June 2020 (2020-06-25) claims 1-38 | 1-191 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **08 January 2025** | **14 January 2025** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**
**Information on patent family members**

International application No.
**PCT/CN2024/121098**

| Patent document cited in search report | | | Publication date (day/month/year) | Patent family member(s) | | | Publication date (day/month/year) |
|---|---|---|---|---|---|---|---|
| WO | 2024153217 | A1 | 25 July 2024 | TW | 202434237 | A | 01 September 2024 |
| CN | 113226473 | A | 06 August 2021 | UY | 38526 | A | 30 June 2020 |
| | | | | CA | 3123871 | A1 | 25 June 2020 |
| | | | | PE | 20211475 | A1 | 05 August 2021 |
| | | | | SA | 521422303 | B1 | 11 December 2023 |
| | | | | US | 2022106293 | A1 | 07 April 2022 |
| | | | | US | 12054476 | B2 | 06 August 2024 |
| | | | | US | 2020239441 | A1 | 30 July 2020 |
| | | | | US | 11236069 | B2 | 01 February 2022 |
| | | | | CL | 2021001634 | A1 | 10 December 2021 |
| | | | | CR | 20210387 | A | 19 August 2021 |
| | | | | TW | 202034924 | A | 01 October 2020 |
| | | | | TWI | 844602 | B | 11 June 2024 |
| | | | | JP | 2022513972 | A | 09 February 2022 |
| | | | | JP | 7407196 | B2 | 28 December 2023 |
| | | | | IL | 283639 | A | 29 July 2021 |
| | | | | IL | 283639 | B1 | 01 February 2024 |
| | | | | IL | 283639 | B2 | 01 June 2024 |
| | | | | BR | 112021011989 | A2 | 08 September 2021 |
| | | | | AU | 2019403486 | A1 | 24 June 2021 |
| | | | | WO | 2020132648 | A1 | 25 June 2020 |
| | | | | KR | 20210106474 | A | 30 August 2021 |
| | | | | CO | 2021008224 | A2 | 30 July 2021 |
| | | | | MX | 2021007156 | A | 16 August 2021 |
| | | | | US | 2024317715 | A1 | 26 September 2024 |
| | | | | EP | 3897852 | A1 | 27 October 2021 |
| | | | | EA | 202191730 | A1 | 24 August 2021 |
| | | | | AR | 117490 | A1 | 11 August 2021 |
| | | | | MA | 54543 | A | 30 March 2022 |
| | | | | JOP | 20210154 | A1 | 30 January 2023 |
| | | | | JOP | 20210154 | B1 | 17 September 2023 |
| | | | | SG | 11202106520 | VA | 29 July 2021 |
| CN | 116789637 | A | 22 September 2023 | WO | 2023217230 | A1 | 16 November 2023 |
| | | | | CN | 116789637 | B | 02 August 2024 |
| CN | 115594664 | A | 13 January 2023 | CN | 115594664 | B | 24 February 2023 |
| | | | | WO | 2024108765 | A1 | 24 February 2023 |
| WO | 2020132651 | A1 | 25 June 2020 | MA | 54550 | A | 30 March 2022 |
| | | | | US | 2022073504 | A1 | 10 March 2022 |
| | | | | AU | 2019403488 | A1 | 24 June 2021 |
| | | | | ES | 2953821 | T3 | 16 November 2023 |
| | | | | EP | 3897855 | A1 | 27 October 2021 |
| | | | | EP | 3897855 | B1 | 07 June 2023 |
| | | | | JP | 2022514268 | A | 10 February 2022 |
| | | | | CA | 3123042 | A1 | 25 June 2020 |
| | | | | MX | 2021007104 | A | 11 August 2021 |
| WO | 2020132649 | A1 | 25 June 2020 | EP | 3898616 | A1 | 27 October 2021 |
| | | | | EP | 3898616 | C0 | 02 October 2024 |
| | | | | MA | 54546 | A | 30 March 2022 |
| | | | | AU | 2019404576 | A1 | 24 June 2021 |

Form PCT/ISA/210 (patent family annex) (July 2022)

**INTERNATIONAL SEARCH REPORT**
**Information on patent family members**

International application No.
**PCT/CN2024/121098**

| Patent document cited in search report | Publication date (day/month/year) | Patent family member(s) | Publication date (day/month/year) |
|---|---|---|---|
| | | US 2022056015 A1 | 24 February 2022 |
| | | MX 2021007158 A | 16 August 2021 |
| | | CA 3123227 A1 | 25 June 2020 |
| | | JP 2022513967 A | 09 February 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MI MAYR et al.** *Current Biology*, 2007, vol. 17, 488-98
- **ALLEN L. V. JR. et al.** Remington: The Science and Practice of Pharmacy. Pharmaceutical Press, 2012